# EUROPEAN PATENT SPECIFICATION

(11) **EP 2 644 711 B1**
(45) Date of publication and mention of the grant of the patent: **04.07.2018**
(21) Application number: 13174139.9
(22) Date of filing: 23.10.2008
(51) Int. Cl.: C12Q 1/68

(54) **A method of diagnosing neoplasms - II**
Verfahren zur Diagnose von Neoplasmen - II
Procédé permettant de diagnostiquer des néoplasmes - II

(30) Priority: 23.10.2007 US 982115 P
(43) Date of publication of application: 02.10.2013
(62) Divisional of application: 08842023.7
(73) Proprietor: Clinical Genomics Pty Ltd, North Ryde, New South Wales 2113 (AU); Commonwealth Scientific and Industrial Research Organisation, Campbell, ACT 2612 (AU)
(72) Inventor: Lapointe, Lawrence C, West Pennant Hills, New South Wales 2125AU (AU); Dunne, Robert, Darlington, New South Wales 2008 (AU); Young, Graeme P, Malvern, South Australia 5061 (AU); Lockett, Trevor John, Denistone, New South Wales 2114 (AU); Wilson, William J, Hornsby Heights, New South Wales 2077 (AU); Molloy, Peter Laurence, Chatswood, New South Wales 2067 (AU)
(74) Representative: Maschio, Antonio

(56) References cited:
- WO-A2-2005/044990
- INNOC HAN ET AL: "New Insights into Syndecan-2 Expression and Tumourigenic Activity in Colon Carcinoma Cells", THE HISTOCHEMICAL JOURNAL, KLUWER ACADEMIC PUBLISHERS, DO, vol. 35, no. 3, 1 March 2004 (2004-03-01), pages 319-326, XP019244002, ISSN: 1573-6865
- AFFYMETRIX: "Data Sheet GeneChip TM Human Genome Arrays", [Online] 1 January 2004 (2004-01-01), 20040101, XP002712971, Retrieved from the Internet: URL:http://media.affymetrix.com/support/te chnical/datasheets/human_datasheet.pdf> [retrieved on 2013-09-13] * abstract * * page 4 *
- JIAN-HUA LUO ET AL: "Transcriptomic and genomic analysis of human hepatocellular carcinomas and hepatoblastomas", HEPATOLOGY, vol. 44, no. 4, 1 October 2006 (2006-10-01), pages 1012-1024, XP055085794, ISSN: 0270-9139, DOI: 10.1002/hep.21328

## Description

### FIELD OF THE INVENTION

The present disclosure relates generally to nucleic acid molecules in respect of which changes to the DNA or to the RNA or protein expression profiles are indicative of the onset, predisposition to the onset and/or progression of a neoplasm. More particularly, the present disclosure is directed to nucleic acid molecules in respect of which changes to the DNA or to the RNA or protein expression profiles are indicative of the onset and/or progression of a large intestine neoplasm, such as an adenoma or an adenocarcinoma. The DNA or the expression profiles of the present disclosure are useful in a range of applications including, but not limited to, those relating to the diagnosis and/or monitoring of colorectal neoplasms, such as colorectal adenocarcinomas. Accordingly, in a related aspect the present disclosure is directed to a method of screening a subject for the onset, predisposition to the onset and/or progression of a neoplasm by screening for modulation in the DNA or the RNA or protein expression profile of one or more nucleic acid molecule markers. The scope of the present invention is defined by appended claims 1 to 7.

### BACKGROUND OF THE INVENTION

Bibliographic details of the publications referred to by author in this specification are collected alphabetically at the end of the description.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Adenomas are benign tumours, or neoplasms, of epithelial origin which are derived from glandular tissue or exhibit clearly defined glandular structures. Some adenomas show recognisable tissue elements, such as fibrous tissue (fibroadenomas) and epithelial structure, while others, such as bronchial adenomas, produce active compounds that might give rise to clinical syndromes.

Adenomas may progress to become an invasive neoplasm and are then termed adenocarcinomas. Accordingly, adenocarcinomas are defined as malignant epithelial tumours arising from glandular structures, which are constituent parts of many organs of the body. The term adenocarcinoma is also applied to tumours showing a glandular growth pattern. These tumours may be sub-classified according to the substances that they produce, for example mucus secreting and serous adenocarcinomas, or to the microscopic arrangement of their cells into patterns, for example papillary and follicular adenocarcinomas. These carcinomas may be solid or cystic (cystadenocarcinomas). Each organ may produce tumours showing a variety of histological types, for example the ovary may produce both mucinous and cystadenocarcinoma.

Adenomas in different organs behave differently. In general, the overall chance of carcinoma being present within an adenoma (i.e. a focus of cancer having developed within a benign lesion) is approximately 5%. However, this is related to size of an adenoma. For instance, in the large bowel (colon and rectum specifically) occurrence of a cancer within an adenoma is rare in adenomas of less than 1 centimetre. Such a development is estimated at 40 to 50% in adenomas which are greater than 4 centimetres and show certain histopathological change such as villous change, or high grade dysplasia. Adenomas with higher degrees of dysplasia have a higher incidence of carcinoma. In any given colorectal adenoma, the predictors of the presence of cancer now or the future occurrence of cancer in the organ include size (especially greater than 9mm) degree of change from tubular to villous morphology, presence of high grade dysplasia and the morphological change described as "serrated adenoma". In any given individual, the additional features of increasing age, familial occurrence of colorectal adenoma or cancer, male gender or multiplicity of adenomas, predict a future increased risk for cancer in the organ - so-called risk factors for cancer. Except for the presence of adenomas and its size, none of these is objectively defined and all those other than number and size are subject to observer error and to confusion as to precise definition of the feature in question. Because such factors can be difficult to assess and define, their value as predictors of current or future risk for cancer is imprecise.

Once a sporadic adenoma has developed, the chance of a new adenoma occurring is approximately 30% within 26 months.

Colorectal adenomas represent a class of adenomas which are exhibiting an increasing incidence, particularly in more affluent countries. The causes of adenoma, and of progression to adenocarcinoma, are still the subject of intensive research. To date it has been speculated that in addition to genetic predisposition, environmental factors (such as diet) play a role in the development of this condition. Most studies indicate that the relevant environmental factors relate to high dietary fat, low fibre, low vegetable intake, smoking, obesity, physical inactivity and high refined carbohydrates.

Colonic adenomas are localised areas of dysplastic epithelium which initially involve just one or several crypts and may not protrude from the surface, but with increased growth in size, usually resulting from an imbalance in proliferation and/or apoptosis, they may protrude. Adenomas can be classified in several ways. One is by their gross appearance and the major descriptors include degrees of protrusion: flat sessile (i.e. protruding but without a distinct stalk) or pedunculated (i.e. having a stalk). Other gross descriptors include actual size in the largest dimension and actual number in the colon/rectum. While small adenomas (less than say 5 or 10 millimetres) exhibit a smooth tan surface, pedunculated and especially larger adenomas tend to have a cobblestone or lobulated red-brown surface. Larger sessile adenomas may exhibit a more delicate villous surface. Another set of descriptors include the histopathological classification; the prime descriptors of clinical value include degree of dysplasia (low or high), whether or not a focus of invasive cancer is present, degree of change from tubular gland formation to villous gland formation (hence classification is tubular, villous or tubulovillous), presence of admixed hyperplastic change and of so-called "serrated" adenomas and its subgroups. Adenomas can be situated at any site in the colon and/or rectum although they tend to be more common in the rectum and distal colon. All of these descriptors, with the exception of number and size, are relatively subjective and subject to interobserver disagreement.

The various descriptive features of adenomas are of value not just to ascertain the neoplastic status of any given adenomas when detected, but also to predict a person's future risk of developing colorectal adenomas or cancer. Those features of an adenoma or number of adenomas in an individual that point to an increased future risk for cancer or recurrence of new adenomas include: size of the largest adenoma (especially 10mm or larger), degree of villous change (especially at least 25% such change and particularly 100% such change), high grade dysplasia, number (3 or more of any size or histological status) or presence of serrated adenoma features. None except size or number is objective and all are relatively subjective and subject to interobserver disagreement. These predictors of risk for future neoplasia (hence "risk") are vital in practice because they are used to determine the rate and need for and frequency of future colonoscopic surveillance. More accurate risk classification might thus reduce workload of colonoscopy, make it more cost-effective and reduce the risk of complications from unnecessary procedures.

Adenomas are generally asymptomatic, therefore rendering difficult their diagnosis and treatment at a stage prior to when they might develop invasive characteristics and so became cancer. It is technically impossible to predict the presence or absence of carcinoma based on the gross appearance of adenomas, although larger adenomas are more likely to show a region of malignant change than are smaller adenomas. Sessile adenomas exhibit a higher incidence of malignancy than pedunculated adenomas of the same size. Some adenomas result in blood loss which might be observed or detectable in the stools; while sometimes visible by eye, it is often, when it occurs, microscopic or "occult". Larger adenomas tend to bleed more than smaller adenomas. However, since blood in the stool, whether overt or occult, can also be indicative of non-adenomatous conditions, the accurate diagnosis of adenoma is rendered difficult without the application of highly invasive procedures such as colonoscopy combined with tissue acquisition by either removal (i.e. polypectomy) or biopsy and subsequent histopathological analysis.

Accordingly, there is an on-going need to elucidate the causes of adenoma and to develop more informative diagnostic protocols or aids to diagnosis that enable one to direct colonoscopy at people more likely to have adenomas. These adenomas may be high risk, advanced or neither of these, in particular protocols which will enable the rapid, routine and accurate diagnosis of adenoma. Furthermore, it can be difficult after colonoscopy to be certain that all adenomas have been removed, especially in a person who has had multiple adenomas. An accurate screening test may minimise the need to undertake an early second colonoscopy to ensure that the colon has been cleared of neoplasms. Accordingly, the identification of molecular markers for adenomas would provide means for understanding the cause of adenomas and cancer, improving diagnosis of adenomas including development of useful screening tests, elucidating the histological stage of an adenoma, characterising a patient's future risk for colorectal neoplasia on the basis of the molecular state of an adenoma and facilitating treatment of adenomas.

To date, research has focused on the identification of gene mutations which lead to the development of colorectal neoplasms. In work leading up to the present invention, however, it has been determined that changes in the DNA or the RNA or protein expression profiles of genes which are also expressed in healthy individuals are indicative of the development of neoplasms of the large intestine, such as adenomas and adenocarcinomas. It has been further determined that in relation to neoplasms of the large intestine, diagnosis can be made based on screening for one or more of a panel of these differentially expressed genes. In a related aspect, it has still further been determined that to the extent that neoplastic tissue has been identified either by the method of the invention or by some other method, the present invention provides still further means of characterising that tissue as an adenoma or a cancer. In yet another aspect, it has been determined that a proportion of these genes are characterised by gene expression which occurs in the context of non-neoplastic tissue but not in the context of neoplastic tissue, thereby facilitating the development of qualitative analyses which do not require a relative analysis to be performed against a non-neoplastic or normal control reference level. Accordingly, the inventors have identified a panel of genes which facilitate the diagnosis of adenocarcinoma and adenoma development and/or the monitoring of conditions characterised by the development of these types of neoplasms.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

The subject specification contains amino acid and nucleotide sequence information prepared using the programme PatentIn Version 3.4, presented herein after the bibliography. Each amino acid and nucleotide sequence is identified in the sequence listing by the numeric indicator <210> followed by the sequence identifier (eg. <210>1, <210>2, etc). The length, type of sequence (amino acid, DNA, etc.) and source organism for each sequence is indicated by information provided in the numeric indicator fields <211>m <212> and <213>, respectively. Amino acid and nucleotide sequences referred to in the specification are identified by the indicator SEQ ID NO: followed by the sequence identifier (eg. SEQ ID NO:1, SEQ ID NO: 2, etc). The sequence identifier referred to in the specification correlates to the information provided in numeric indicator field <400> in the sequence listing, which is followed by the sequence identifier (eg. <400>1, <400>2, etc). That is SEQ ID NO: 1 as detailed in the specification correlates to the sequence indicated as <400>1 in the sequence listing.

One aspect of the present disclosure is directed to a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:

| | | | |
|---|---|---|---|
| 200600_at | 208399_s_at | 217546_at | 203296_s_at |
| 200621_at | 208450_at | 217757_at | 203343_at |
| 200795_at | 208581_x_at | 217762_s_at | 203474_at |
| 200799_at | 208747_s_at | 217764_s_at | 203638_s_at |
| 200845_s_at | 208763_s_at | 217767_at | 203963_at |
| 200859_x_at | 208788_at | 217897_at | 204018_x_at |
| 200897_s_at | 208789_at | 217967_s_at | 204034_at |
| 200974_at | 208791_at | 218087_s_at | 204036_at |
| 200986_at | 208792_s_at | 218162_at | 204130_at |
| 201041_s_at | 208894_at | 218224_at | 204388_s_at |
| 201058_s_at | 209047_at | 218312_s_at | 204389_at |
| 201061_s_at | 209074_s_at | 218353_at | 204508_s_at |
| 201069_at | 209101_at | 218418_s_at | 204532_x_at |
| 201105_at | 209116_x_at | 218468_s_at | 204607_at |
| 201137_s_at | 209138_x_at | 218469_at | 204673_at |
| 201141_at | 209147_s_at | 218559_s_at | 204818_at |
| 201150_s_at | 209156_s_at | 218756_s_at | 204895_x_at |
| 201289_at | 209167_at | 219014_at | 204897_at |
| 201300_s_at | 209170_s_at | 219087_at | 205112_at |
| 201324_at | 209191_at | 219508_at | 205259_at |
| 201348_at | 209209_s_at | 219607_s_at | 205403_at |
| 201426_s_at | 209210_s_at | 219669_at | 205480_s_at |
| 201427_s_at | 209283_at | 219799_s_at | 205554_s_at |
| 201438_at | 209301_at | 220026_at | 205593_s_at |
| 201496_x_at | 209312_x_at | 220037_s_at | 205892_s_at |
| 201497_x_at | 209335_at | 220376_at | 205929_at |
| 201539_s_at | 209357_at | 220834_at | 206000_at |
| 201540_at | 209373_at | 221541_at | 206094_x_at |
| 201616_s_at | 209436_at | 221667_s_at | 206262_at |
| 201617_x_at | 209457_at | 221747_at | 206377_at |
| 201645_at | 209496_at | 221748_s_at | 206385_s_at |
| 201667_at | 209498_at | 222043_at | 206664_at |
| 201739_at | 209612_s_at | 222162_s_at | 207126_x_at |
| 201743_at | 209613_s_at | 222453_at | 207245_at |
| 201744_s_at | 209621_s_at | 222513_s_at | 207390_s_at |
| 201842_s_at | 209651_at | 222717_at | 207392_x_at |
| 201852_x_at | 209656_s_at | 222722_at | 207432_at |
| 201858_s_at | 209667_at | 223121_s_at | 207761_s_at |
| 201859_at | 209668_x_at | 223122_s_at | 208596_s_at |
| 201865_x_at | 209687_at | 223235_s_at | 208920_at |
| 201893_x_at | 209735_at | 223343_at | 209114_at |
| 201920_at | 209763_at | 223395_at | 209374_s_at |
| 201957_at | 209868_s_at | 223551_at | 209458_x_at |
| 202007_at | 209948_at | 223623_at | 209791_at |
| 202037_s_at | 210084_x_at | 223952_x_at | 210107_at |
| 202069_s_at | 227099_s_at | 224009_x_at | 2t0524_x_at |
| 202133_at | 210133_at | 224352_s_at | 210735_s_at |
| 202222_s_at | 210139_s_at | 224412_s_at | 211372_s_at |
| 202242_at | 210298_x_at | 224480_s_at | 211538_s_at |
| 202274_at | 210299_s_at | 224560_at | 211549_s_at |
| 202283_at | 210302_s_at | 224663_s_at | 211637_x_at |
| 202291_s_at | 210495_x_at | 224694_at | 211699_x_at |
| 202388_at | 210517_s_at | 224823_at | 211745_x_at |
| 202555_s_at | 210764_s_at | 224836_at | 212224_at |
| 202620_s_at | 210809_s_at | 224840_at | 212592_at |
| 202686_s_at | 210946_at | 224959_at | 212741_at |
| 202746_at | 210982_s_at | 224963_at | 212814_at |
| 202760_s_at | 211161_s_at | 224964_s_at | 213317_at |
| 202766_s_at | 211548_s_at | 225207_at | 213451_x_at |
| 202888_s_at | 211596_s_at | 225242_s_at | 213629_x_at |
| 202920_at | 211643_x_at | 225269_s_at | 213921_at |
| 202953_at | 211644_x_at | 225275_at | 213953_at |
| 202957_at | 211645_x_at | 225353_s_at | 214164_x_at |
| 202992_at | 211671_s_at | 225381_at | 214433_s_at |
| 202994_s_at | 211696_x_at | 225442_at | 214598_at |
| 202995_s_at | 211719_x_at | 225575_at | 214916_x_at |
| 203000_at | 211798_x_at | 225602_at | 215125_s_at |
| 203001_s_at | 211813_x_at | 225604_s_at | 215299_x_at |
| 203066_at | 211848_s_at | 225626_at | 215867_x_at |
| 203131_at | 211889_x_at | 225688_s_at | 216336_x_at |
| 203305_at | 211896_s_at | 225710_at | 216491_x_at |
| 203382_s_at | 211959_at | 225720_at | 216510_x_at |
| 203477_at | 211964_at | 225721_at | 217022_s_at |
| 203645_s_at | 211985_s_at | 225782_at | 217109_at |
| 203680_at | 211990_at | 225894_at | 217110 _ s _ at |
| 203729_at | 211991_s_at | 225895_at | 217165_x_at |
| 203748_x_at | 212077_at | 226001_at | 217232_x_at |
| 203766_s_at | 212091_s_at | 226051_at | 217414_x_at |
| 203881_s_at | 212097_at | 226084_at | 218541_s_at |
| 203908_at | 212136_at | 226103_at | 218546_at |
| 203913_s_at | 212158_at | 226303_at | 219059_s_at |
| 203914_x_at | 212185_x_at | 226304_at | 219543_at |
| 203951_at | 212192_at | 226333_at | 219796_s_at |
| 203980_at | 212195_at | 226430_at | 219948_x_at |
| 204069_at | 212230_at | 226492_at | 220075_s_at |
| 204083_s_at | 212233_at | 226682_at | 220266_s_at |
| 204122_at | 212265_at | 226694_at | 220468_at |
| 204135_at | 212288_at | 226818_at | 220645_at |
| 204326_x_at | 212386_at | 226834_at | 220812_s_at |
| 204438_at | 212387_at | 226841_at | 221004_s_at |
| 204457_s_at | 212397_at | 227006_at | 221305_s_at |
| 204570_at | 212414_s_at | 227061_at | 221584_s_at |
| 204688_at | 212419_at | 227235_at | 221841_s_at |
| 204697_s_at | 212464_s_at | 227265_at | 221896_s_at |
| 204719_at | 212667_at | 227404_s_at | 223484_at |
| 204745_x_at | 212671_s_at | 227529_s_at | 223597_at |
| 204834_at | 212713_at | 227561_at | 223754_at |
| 204894_s_at | 212730_at | 227623_at | 224342_x_at |
| 204931_at | 212764_at | 227662_at | 224989_at |
| 204938_s_at | 212859_x_at | 227705_at | 224990_at |
| 204939_s_at | 212956_at | 227727_at | 225458_at |
| 204940_at | 213068_at | 227826_s_at | 225728_at |
| 204955_at | 213071_at | 227827_at | 226147_s_at |
| 205097_at | 213428_s_at | 228202_at | 226302_at |
| 205200_at | 213509_x_at | 228504_at | 226594_at |
| 205267_at | 213624_at | 228507_at | 226654_at |
| 205382_s_at | 213746_s_at | 228640_at | 226811_at |
| 205412_at | 213891_s_at | 228706_s_at | 227052_at |
| 205433_at | 214027_x_at | 228707_at | 227522_at |
| 205464_at | 214038_at | 228750_at | 227682_at |
| 205547_s_at | 214091_s_at | 228766_at | 227725_at |
| 205683_x_at | 214142_at | 228846_at | 227735_s_at |
| 205935_at | 214414_x_at | 228854_at | 227736_at |
| 205950_s_at | 214505_s_at | 228885_at | 228133_s_at |
| 206134_at | 214677_x_at | 229530_at | 228195_at |
| 206143_at | 214696_at | 229839_at | 228232_s_at |
| 206149_at | 214752_x_at | 230087_at | 228241_at |
| 206198_s_at | 214768_x_at | 230264_s_at | 228469_at |
| 206199_at | 214777_at | 230788_at | 228961_at |
| 206208_at | 215049_x_at | 230830_at | 229070_at |
| 206209_s_at | 215076_s_at | 231120_x_at | 229254_at |
| 206422_at | 215118_s_at | 231579_s_at | 229659_s_at |
| 206461_x_at | 215176_x_at | 231773_at | 229831_at |
| 206561_s_at | 215193_x_at | 234764_x_at | 230595_at |
| 206576_s_at | 215382_x_at | 234987_at | 231925_at |
| 206637_at | 215388_s_at | 236300_at | 231975_s_at |
| 206641_at | 215657_at | 236313_at | 233565_s_at |
| 206710_s_at | 216207_x_at | 242317_at | 235146_at |
| 206784_at | 216401_x_at | 200884_at | 235766_x_at |
| 207003_at | 216442_x_at | 201495_x_at | 235849_at |
| 207080_s_at | 216474_x_at | 202266_at | 238143_at |
| 207134_x_at | 216576_x_at | 202350_s_at | 238750_at |
| 207266_x_at | 216834_at | 202731_at | 238751_at |
| 207502_at | 216984_x_at | 202741_at | 239272_at |
| 207961_x_at | 217148_x_at | 202742_s_at | 241994_at |
| 207977_s_at | 217179_x_at | 202768_at | 242447_at |
| 207980_s_at | 217235_x_at | 202838_at | 242601_at |
| 208131_s_at | 217258_x_at | 203058_s_at | 243278_at; and/or |
| 208370_s_at | 217378_x_at | 203060_s_at | |
| 208383_s_at | 217480_x_at | 203240_at | |

**(ii)**

| | | | |
|---|---|---|---|
| CLCA4 | VIM | CA12 | AP1S2 |
| ZG16 | SMPDL3A | FKBP5 | EMP3 |
| CA2 | P2RY14 | HSPB8 | MMP28 |
| CA1 | CHGA | TPSB2 | UGT2A3 |
| MS4A12 | C15orf48 | FGL2 | RGS5 |
| AQP8 | COL3A1 | C1QB | PTGIS |
| SLC4A4 | CYR61 | ANGPTL1 | DUSP5 |
| CEACAM7 | TRPM6 | MEPIA | MFAP4 |
| TAGLN | OSTbeta | GUCY1A3 | UGT1A6 |
| GUCA1B | IGLV1-44 | UGDH | PRKAR2B |
| GCG | VSIG2 | DUSP1 | HHLA2 |
| ADH1B | IGHM | C2orf40 | LOC652128 |
| UGT2B17 | LRRC19 | PLN | C3 |
| ADAMDEC1 | CD163 | UGT2B15 | ATP2B4 |
| MT1M | CEACAM1 | PDLIM3 | HBA1 |
| AKR1B10 | TIMP2 | TP53INP2 | TCF21 |
| FN1 | ENTPD5 | ATP8B1 | PPID |
| MGP | DDR2 | ANK3 | PPAP2B |
| CXCL12 | CHRDL1 | CTGF | SPON1 |
| PDK4 | SRGN | MUCDHL | PHLDB2 |
| CA4 | PDE9A | SDPR | RARRES2 |
| PYY | PMP22 | COL14A1 | ETHE1 |
| IGHA1 | FLNA | DSCR1 | MMP2 |
| TPM2 | STMN2 | CITED2 | SRI |
| C6orf105 | MYL9 | MT1H | CNTN3 |
| HPGD | SEMA6D | NEXN | RGS2 |
| ADH1C | PADI2 | MUC2 | COL6A1 |
| CLCA1 | SEPP1 | NID1 | FBN1 |
| FABP1 | TGFB1I1 | HBB | MXD1 |
| ENAM | SFRP2 | GCNT2 | PLCE1 |
| CFD | UGT1A3 | C20orf118 | KCNMB1 |
| GUCA2B | MS4A7 | SLC20A1 | CALM1 |
| FBLN1 | ALDH1A1 | CD14 | HLA-DPB1 |
| LOC63928 | SGK | KCTD12 | SMOC2 |
| ABCA8 | CFL2 | RBMS1 | LOC285382 |
| POSTN | C1S | PTRF | CLIC5 |
| DCN | SELENBP1 | TSPAN1 | APOE |
| ITLN1 | MT1E | UGT1A9 | SERPINF1 |
| COL6A2 | ADAMTS1 | COX7A1 | PPP1R12B |
| FCGBP | ITM2A | MUC12 | HSPB6 |
| SLC26A2 | POU2AF1 | PDCD4 | FNBP1 |
| PGM5 | FAM55D | CAV1 | C4orf34 |
| DMN | C6orf204 | FAM46C | SORBS2 |
| GPNMB | AKAP12 | LRIG1 | GPA33 |
| IGFBP5 | TUBB6 | HLA-DPA1 | GALNAC4S- |
| CLEC3B | LGALS2 | C1orf115 | 6ST |
| LOC253012 | KIAA0828 | HBA2 | CFHR1 |
| DPT | MGC14376 | EDIL3 | MGC13057 |
| PCK1 | PPP1R14A | DES | C10orf56 |
| CNN1 | MUC4 | MT2A | SULT1A1 |
| HSD17B2 | PKIB | KCNMA1 | TTRAP |
| PLAC8 | PIGR | GAS1 | CCL28 |
| TMEM47 | ASPN | TBC1D9 | IDH3A |
| OGN | A2M | C7 | EDG2 |
| CALD1 | LOC25845 | P2RY1 | UGT1A8 |
| ACTG2 | LGALS1 | NR3C1 | RAB27A |
| MGC4172 | BCHE | STOM | ANTXR1 |
| MAB21L2 | ST6GALNAC1 | CKB | EMP1 |
| RPL24 | GJA1 | CLU | CSRP1 |
| ABCG2 | SCNN1B | SLC26A3 | PLEKHC1 |
| CCDC80 | FABP4 | SDC2 | LOC572558 |
| UGT1A1 | F13A1 | SST | FOXP2 |
| MRC1 | CD36 | HLA-DRA | HSPA2 |
| HSD11B2 | SPARCL1 | TSC22D3 | ATP1A2 |
| ANPEP | ZCWPW2 | IL6ST | TNXB |
| MATN2 | TNC | C1QC | FUCA1 |
| PRNP | MT1A | MT1X | MRGPRF |
| ABI3BP | LOC652745 | AOC3 | HIGD1A |
| HLA-C | MALL | PPAP2A | MFSD4 |
| NDE1 | GNG2 | ZSCAN18 | AXL |
| SRPX | DNASE1L3 | IVD | AQP1 |
| WWTR1 | EGR1 | SFRP1 | MAP1B |
| HMGCS2 | CMBL | COL4A2 | PALLD |
| LOC646627 | GCNT3 | GPM6B | MPEG1 |
| KRT20 | SERPING1 | EPB41L3 | KLHL5 |
| KLF4 | MEIS1 | MAOA | TCEAL7 |
| FHL1 | EDN3 | DMD | FILIP1L |
| ARL14 | MSN | MSRB3 | IQGAP2 |
| LUM | MT1G | PLOD2 | PRDX6 |
| SORBS 1 | TPSAB1 | C9orf19 | RAB31 |
| METTL7A | GPX3 | MIER3 | LOC96610 |
| FAM129A | CDKN2B | XDH | FGFR2 |
| SCARA5 | FOSB | CLDN23 | PAPSS2 |
| SI | HSPA1A | SGCE | XLKD1 |
| ACTA2 | CYBRD1 | FOXF2 | SMTN |
| CD177 | PTGER4 | AGR3 | C8orf4 |
| C10orf99 | MAGI | IGLJ3 | SDCBP2 |
| COL15A1 | BEST2 | QKI | CCL11 |
| NR3C2 | HLA-DQA1 | LOC399959 | ELOVL5 |
| DHRS9 | PRIMA1 | ANKRD25 | FOXF1 |
| LMOD1 | MT1F | CRISPLD2 | RELL1 |
| EFEMP1 | MAFB | ANK2 | PNMA1 |
| GREM1 | FAM107A | LOC283666 | LOC339562 |
| IL1R2 | PRKACB | CRYAB | PALM2- |
| LOC387763 | SELM | ACAT1 | AKAP2 |
| TIMP3 | TYROBP | IGL@ | PAG1 |
| MYLK | TNS1 | PBLD | HCLS1 |
| CLDN8 | MYH11 | CCL8 | RGS1 |
| RDX | ITM2C | LIFR | FXYD6 |
| TSPAN7 | CES2 | HLA-DRB1 | OLFML3 |
| TNFRSF17 | MS4A4A | UGP2 | COL6A3 |
| SYNPO2 | PDGFRA | IGKV1D-13 | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Another aspect of the present disclosure provides a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs:
   220026_at; and/or
(ii) CLCA4
in a biological sample from said individual wherein a lower level of expression of the gene or transcripts of group (i) and/or group (ii) relative to control levels is indicative of a neoplastic large intestine cell or a cell predisposed to the onset of a neoplastic state.

In yet another aspect there is provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs:
   214142_at; and/or
(ii) ZG16
in a biological sample from said individual wherein a lower level of expression of the genes or transcripts of group (i) and/or group (ii) relative to control levels is indicative of a neoplastic large intestine cell or a cell predisposed to the onset of a neoplastic state.

In still another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:

| | |
|---|---|
| 209301_at | 205950_s_at; |

and/or
**(ii)**

| | |
|---|---|
| CA2 | CA1 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In still yet another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   220834_at; and/or
**(ii)** MS4A12
in a biological sample from said individual wherein a lower level of expression of the genes or transcripts of group (i) and/or group (ii) relative to control levels is indicative of a neoplastic large intestine cell or a cell predisposed to the onset of a neoplastic state.

In yet still another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   206784_at; and/or
**(ii)** AQP8
in a biological sample from said individual wherein a lower level of expression of the genes or transcripts of group (i) and/or group (ii) relative to control levels is indicative of a neoplastic large intestine cell or a cell predisposed to the onset of a neoplastic state.

In a further aspect there is provided a method of screening for the onset or predisposition to the onset of a large intestine neoplasm in an individual, said method comprising assessing the level of expression of one or more genes or transcripts selected from:
the gene, genes or transcripts detected by Affymetrix probeset IDs:
**(i)** 203908_at, 206198_s_at, 205547_s_at, 207003_at, 206422_at, 209613_s_at, 207245_at; and/or
**(ii)** SLC4A4, CEACAM7, TAGLN, GUCA1B, GCG, ADH1B, UGT2B17,
in a biological sample from said individual wherein a lower level of expression of the genes or transcripts of group (i) and/or group (ii) relative to control levels is indicative of a neoplastic large intestine cell or a cell predisposed to the onset of a neoplastic state.

In another further aspect there is provided a method of screening for the onset or predisposition to the onset of a large intestine neoplasm in an individual, said method comprising assessing the level of expression of one or more genes or transcripts selected from:
the gene, genes or transcripts detected by Affymetrix probeset IDs:
**(i)** 203908_at, 206198_s_at, 205547_s_at, 207003_at, 206422_at, 209613_s_at, 207245_at; and/or
**(ii)** SLC4A4, CEACAM7, TAGLN, GUCA1B, GCG, ADH1B, UGT2B17,
in a biological sample from said individual wherein a lower level of expression of the genes or transcripts of group (i) and/or group (ii) relative to control levels is indicative of a neoplastic large intestine cell or a cell predisposed to the onset of a neoplastic state.

In still another further aspect there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 225207_at | 211548_s_at | 205382_s_at | 204083_s_at |
| 206208_at | 206262_at | 207502_at | 212592_at |
| 207080_s_at | 210107_at | 202995_s_at | 204719_at |
| 215118_s_at | 205892_s_at | 206149_at | 229070_at; and/or |

**(ii)**

| | | | |
|---|---|---|---|
| PDK4 | HPGD | CFD | TPM2 |
| CA4 | ADH1C | GUCA2B | ENAM |
| PYY | CLCA1 | FBLN1 | ABCA8 |
| IGHA1 | FABP1 | LOC63928 | C6orf105 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In yet still yet another further aspect there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 210809_s_at | 201617_x_at | 202133_at | 217967 _s_at |
| 201893_x_at | 202274_at | 204607_at | 229839_at |
| 223597_at | 218756_s_at | 238143_at | 206664_at |
| 209156_s_at | 210302_s_at | 213953_at | 200974_at |
| 203240_at | 228885_at | 220266_s_at | 219669_at |
| 224963_at | 209735_at | 2t0299_s_at | 227736_at |
| 226303_at | 228504_at | 220468_at | 203477_at |
| 212730_at | 225242_s_at | 201744_s_at | 205259_at |
| 201141_at | 215125_s_at | 218087_s_at | 203951_at |
| 211959_at | 204438_at | 207761_s_at | 204818_at |
| 205200_at | 204130_at | 223395_at | 219014_at |
| 242601_at | 202888_s_at | 2t4768_x_at | 209656_s_at |
| 213068_at | 202350_s_at | 228133_s_at | 222722_at; |
| 208383_s_at | 201300_s_at | 204955_at | and/or |

**(ii)**

| | | | |
|---|---|---|---|
| POSTN | OGN | WWTR1 | FAM129A |
| DCN | CALD1 | HMGCS2 | SCARA5 |
| ITLN1 | ACTG2 | LOC646627 | SI |
| COL6A2 | MGC4172 | KRT20 | ACTA2 |
| FCGBP | MAB21L2 | KLF4 | CD177 |
| SLC26A2 | RPL24 | FHL1 | C10orf99 |
| PGM5 | ABCG2 | ARL14 | COL15A1 |
| DMN | CCDC80 | LUM | NR3C2 |
| GPNMB | UGT1A1 | SORBS1 | CNN1 |
| IGFBP5 | MRC1 | METTL7A | HSD17B2 |
| CLEC3B | HSD11B2 | PRNP | PLAC8 |
| LOC253012 | ANPEP | ABI3BP | TMEM47 |
| DPT | MATN2 | HLA-C | SRPX |
| PCK1 | NDE1 | | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In a related aspect the present disclosure is directed to a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 200600_at | 203680_at | 211959_at | 215076_s_at |
| 200665_s_at | 203729_at | 211964_at | 215193_x_at |
| 200799_at | 203748_x_at | 211985_s_at | 219087_at |
| 200845_s_at | 204069_at | 211990_at | 219607_s_at |
| 200859_x_at | 204122_at | 211991_s_at | 221541_at |
| 200897_s_at | 204135_at | 212077_at | 222043_at |
| 200974_at | 204438_at | 212091_s_at | 222453_at |
| 200986_at | 204457_s_at | 212136_at | 222513_s_at |
| 201041_s_at | 204570_at | 215382_x_at | 223121_s_at |
| 201061_s_at | 204688_at | 215388_s_at | 223122_s_at |
| 201069_at | 205412_at | 216442_x_at | 223235_s_at |
| 201105_at | 205683_x_at | 216474_x_at | 223343_at |
| 201137_s_at | 205935_at | 216834_at | 224560_at |
| 201141_at | 207134_x_at | 217480_x_at | 224694_at |
| 201150_s_at | 207266_x_at | 217757_at | 224840_at |
| 201289_at | 208131_s_at | 217762_s_at | 224964_s_at |
| 201300_s_at | 208370_s_at | 217764_s_at | 225242_s_at |
| 201426_s_at | 208747_s_at | 217767_at | 225269_s_at |
| 201438_at | 208788_at | 217897_at | 225353_s_at |
| 201616_s_at | 208789_at | 218162_at | 225381_at |
| 201617_x_at | 208894_at | 218224_at | 225442_at |
| 201645_at | 209047_at | 218312_s_at | 225602_at |
| 201667_at | 209101_at | 218353_at | 225604_s_at |
| 201743_at | 209138_x_at | 218418_s_at | 225626_at |
| 201744_s_at | 209147_s_at | 218468_s_at | 225688_s_at |
| 201842_s_at | 209156_s_at | 218469_at | 225710_at |
| 201852_x_at | 209191_at | 218559_s_at | 226001_at |
| 201858_s_at | 209209_s_at | 212158_at | 226051_at |
| 201859_at | 209210_s_at | 212185_x_at | 226084_at |
| 201865_x_at | 209312_x_at | 212195_at | 226103_at |
| 201893_x_at | 209335_at | 212230_at | 226430_at |
| 201920_at | 209436_at | 212233_at | 226682_at |
| 202007_at | 209457_at | 212265_at | 226694_at |
| 202069_s_at | 209496_at | 212386_at | 226818_at |
| 202133_at | 209621_s_at | 212387_at | 226834_at |
| 202283_at | 209651_at | 212397_at | 226841_at |
| 202291_s_at | 209656_s_at | 212414_s_at | 227061_at |
| 202403 _s_at | 209868_s_at | 212419_at | 227099_s_at |
| 202620_s_at | 210084_x_at | 212464_s_at | 227235_at |
| 202686_s_at | 210133_at | 212667_at | 227404_s_at |
| 202760_s_at | 210139_s_at | 212671_s_at | 227529_s_at |
| 202766_s_at | 210495_x_at | 212713_at | 227561_at |
| 202953_at | 210517_s_at | 212764_at | 227623_at |
| 202957_at | 210764_s_at | 212956_at | 227705_at |
| 202994_s_at | 210809_s_at | 213428_s_at | 227727_at |
| 202995_s_at | 210982_s_at | 213509_x_at | 228507_at |
| 203066_at | 211161_s_at | 213746_s_at | 228750_at |
| 203131_at | 211596_s_at | 213891_s_at | 228846_at |
| 203305_at | 211671_s_at | 214038_at | 229530_at |
| 203382_s_at | 211719_x_at | 214677_x_at | 230264_s_at |
| 203477_at | 211813_x_at | 214752_x_at | 231579_s_at |
| 203645_s_at | 211896_s_at | 215049_x_at | 234987_at; and/or |

**(ii)**

| | | | |
|---|---|---|---|
| A2M | CYBRD1 | LOC283666 | PTGIS |
| ACAT1 | CYR61 | LOC339562 | PTRF |
| ACTA2 | DCN | LOC387763 | QKI |
| AKAP12 | DDR2 | LOC399959 | RAB31 |
| ANKRD25 | DSCR1 | LRIG1 | RARRES2 |
| ANTXR1 | DUSP1 | LUM | RBMS1 |
| AP1S2 | DUSP5 | MAFB | RDX |
| APOE | EFEMP1 | MAP1B | RELL1 |
| AQP1 | EGR1 | MEIS1 | RGS1 |
| ASPN | ELOVL5 | MFAP4 | RGS5 |
| ATP2B4 | EMP3 | MGP | SDC2 |
| AXL | F13A1 | MMP2 | SELM |
| C10orf56 | FBLN1 | MPEG1 | SEPT6 |
| C1QB | FBN1 | MRC1 | SERPINF1 |
| C1QC | FILIP1L | MRGPRF | SERPING1 |
| C1S | FKBP5 | MS4A4A | SFRP2 |
| C20orf118 | FLNA | MS4A7 | SGCE |
| C3 | FN1 | MSN | SLC20A1 |
| C9orf19 | FOXF1 | MT2A | SMOC2 |
| CALD1 | FXYD6 | MXD1 | SORBS1 |
| CALM1 | GALNAC4S-6ST | NEXN | SPARC |
| CCDC80 | GAS1 | NID1 | SPON1 |
| CCL11 | GJA1 | NR3C1 | SRGN |
| CCL8 | GNG2 | OLFML3 | STOM |
| CD14 | GPNMB | PAG1 | TBC1D9 |
| CD163 | GREM1 | PALLD | TCEAL7 |
| CES2 | GUCY1A3 | PALM2-AKAP2 | TGFB1I1 |
| CFHR1 | HCLS1 | PDGFRA | TIMP2 |
| CLU | HLA-DPA1 | PDLIM3 | TIMP3 |
| COL14A1 | HLA-DPB1 | PHLDB2 | TMEM47 |
| COL15A1 | HLA-DQA1 | PLEKHC1 | TNC |
| COL1A2 | HLA-DRA | PLOD2 | TPSAB1 |
| COL3A1 | HLA-DRB1 | PMP22 | TPSB2 |
| COL4A2 | HSPA1A | PNMA1 | TUBB6 |
| COL6A1 | IDH3A | POSTN | TYROBP |
| COL6A2 | IGFBP5 | PPAP2A | VIM |
| COL6A3 | IGL@ | PPAP2B | WWTR1 |
| COX7A1 | IGLJ3 | PRDX6 | ZSCAN18 |
| CRISPLD2 | IL6ST | PRKAR2B | |
| CTGF | KLHL5 | PRNP | |
| | LGALS1 | | |

in a biological sample from said individual wherein a lower level of expression of the genes or transcripts of group (i) and/or group (ii) relative to control levels is indicative of an adenoma cell or a cell predisposed to the onset of an adenoma state.

In another aspect of the present disclosure there is provided a method of screening for the onset or predisposition to the onset of a large intestine neoplasm in an individual, said method comprising screening the level of expression of one or more genes or transcripts selected from:
**(i)** the gene or genes detected by Affymetrix probeset IDs:

| | | | |
|---|---|---|---|
| 200884_at | 206262_at | 215867_x_at | 227522_at |
| 201495_x_at | 206377_at | 216336_x_at | 227682_at |
| 202266_at | 206385_s_at | 216491_x_at | 227725_at |
| 202350_s_at | 206664_at | 216510_x_at | 227735_s_at |
| 202731_at | 207126_x_at | 217022_s_at | 227736_at |
| 202741_at | 207245_at | 217109_at | 228133_s_at |
| 202742_s_at | 207390_s_at | 217110_s_at | 228195_at |
| 202768_at | 207392_x_at | 217165_x_at | 228232_s_at |
| 202838_at | 207432_at | 217232_x_at | 228241_at |
| 203058_s_at | 207761_s_at | 217414_x_at | 228469_at |
| 203060_s_at | 208596_s_at | 218541_s_at | 228961_at |
| 203240_at | 208920_at | 218546_at | 229070_at |
| 203296_s_at | 209114_at | 219059_s_at | 229254_at |
| 203343_at | 209374_s_at | 219543_at | 229659_s_at |
| 203474_at | 209458_x_at | 219796_s_at | 229831_at |
| 203638_s_at | 209791_at | 219948_x_at | 230595_at |
| 203963_at | 210107_at | 220075_s_at | 231925_at |
| 204018_x_at | 210524_x_at | 220266_s_at | 231975_s_at |
| 204034_at | 210735_s_at | 220468_at | 233565_s_at |
| 204036_at | 211372_s_at | 220645_at | 235146_at |
| 204130_at | 211538_s_at | 220812_s_at | 235766_x_at |
| 204388_s_at | 211549_s_at | 221004_s_at | 235849_at |
| 204389_at | 211637_x_at | 221305_s_at | 238143_at |
| 204508_s_at | 211699_x_at | 221584_s_at | 238750_at |
| 204532_x_at | 211745_x_at | 221841_s_at | 238751_at |
| 204607_at | 212224_at | 221896_s_at | 239272_at |
| 204673_at | 212592_at | 223484_at | 241994_at |
| 204818_at | 212741_at | 223597_at | 242447_at |
| 204895_x_at | 212814_at | 223754_at | 242601_at |
| 204897_at | 213317_at | 224342_x_at | 243278_at; and/or |
| 205112_at | 213451_x_at | 224989_at | |
| 205259_at | 213629_x_at | 224990_at | |
| 205403_at | 213921_at | 225458_at | |
| 205480_s_at | 213953_at | 225728_at | |
| 205554_s_at | 214164_x_at | 226147_s_at | |
| 205593_s_at | 214433_s_at | 226302_at | |
| 205892_s_at | 214598_at | 226594_at | |
| 205929_at | 214916_x_at | 226654_at | |
| 206000_at | 215125_s_at | 226811_at | |
| 206094_x_at | 215299_x_at | 227052_at | |

**(ii)**

| | | | |
|---|---|---|---|
| ADH1C | FOSB | LOC96610 | SDCBP2 |
| AGR3 | FOXF2 | MAOA | SELENBP1 |
| ALDH1A1 | FOXP2 | MATN2 | SI |
| ANK3 | FUCA1 | MEP1A | SMTN |
| ARL14 | GPA33 | METTL7A | SORBS2 |
| ATP1A2 | HBA1 | MFSD4 | SRI |
| ATP8B1 | HBA2 | MGC13057 | SST |
| BEST2 | HBB | MIER3 | ST6GALNAC1 |
| C10orf99 | HHLA2 | MMP28 | SULT1A1 |
| C15orf48 | HIGD1A | MT1A | TNXB |
| Clorf115 | HMGCS2 | MT1F | TSPAN1 |
| C4orf34 | HPGD | MT1M | TTRAP |
| C6orf105 | HSD11B2 | MUC12 | UGDH |
| C8orf4 | HSD17B2 | MUC2 | UGP2 |
| CA12 | HSPA2 | MUC4 | UGT1A1 |
| CCL28 | IGHA1 | MUCDHL | UGT1A3 |
| CKB | IGHM | MYH11 | UGT1A6 |
| CLCA1 | IL1R2 | NDE1 | UGT1A8 |
| CLDN8 | IL8 | NR3C2 | UGT1A9 |
| CLIC5 | IQGAP2 | P2RY1 | UGT2A3 |
| CMBL | ITLN1 | PADI2 | UGT2B15 |
| CNTN3 | ITM2C | PAPSS2 | UGT2B 17 |
| DNASE1L3 | KCNMA1 | PBLD | VSIG2 |
| EDG2 | KIAA0828 | PDCD4 | XDH |
| ENAM | KLF4 | PDE9A | XLKD1 |
| ENTPD5 | KRT20 | PIGR | ZCWPW2 |
| ETHE1 | LOC253012 | PLCE1 | |
| FABP1 | LOC25845 | PPID | |
| FAM46C | LOC285382 | PRKACB | |
| FAM55D | LOC572558 | PTGER4 | |
| FCGBP | LOC646627 | RAB27A | |
| FGFR2 | LOC652128 | SCARA5 | |

in a biological sample from said individual wherein a lower level of expression of the genes or transcripts of group (i) and/or group (ii) relative to control levels is indicative of a cancer cell or a cell predisposed to the onset of a cancerous state.

In still another aspect there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 202920_at | 209613_s_at | 228504_at | 214598_at |
| 203881_s_at | 220037_s_at | 228706_s_at | 219948_x_at |
| 204719_at | 220376_at | 228766_at | 220812_s_at |
| 204931_at | 222717_at | 228854_at | 221305_s_at |
| 204940_at | 224412_s_at | 228885_at | 229831_at |
| 205433_at | 225381_at | 230788_at | 231925_at |
| 206637_at | 225575_at | 231120_x_at | 235146_at |
| 207080_s_at | 227529_s_at | 231773_at | 238751_at |
| 207980_s_at | 227623_at | 203296_s_at | 243278_at; and/or |
| 209170_s_at | 227705_at | 206664_at | |
| 209209_s_at | 227827_at | 211549_s_at | |

**(ii)**

| | | | |
|---|---|---|---|
| ADH1B | CD36 | P2RY1 | PLEKHC1 |
| SORBS2 | BCHE | ANK2 | LRRC19 |
| PYY | TCEAL7 | XLKD1 | LIFR |
| ABCA8 | ANGPTL1 | LOC399959 | ATP1A2 |
| RPL24 | DMD | AKAP12 | HPGD |
| SI | GCNT2 | UGT2A3 | GPM6B |
| CLDN8 | SDPR | HHLA2 | UGT1A8 |
| P2RY14 | PKIB | SORBS2 | FOXP2 |
| PLN | CITED2 | CLDN23 | |
| TRPM6 | TCF21 | CNTN3 | |

in a biological sample from said individual wherein a level of expression of the genes or transcripts of group (i) and/or group (ii) which is not substantially above background levels is indicative of a neoplastic cell or a cell predisposed to the onset of a neoplastic state.

In a further aspect there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:
   209209_s_at, 225381_at, 227529_s_at, 227623_at, 227705_at; and/or
**(ii)** AKAP12, LOC399959, PLEKHC1, TCEAL7,
in a biological sample from said individual wherein a lower level of expression of the genes or transcripts of group (i) and/or group (ii) which is not substantially above background levels is indicative of an adenoma cell or a cell predisposed to the onset of an adenoma state.

In yet still another further aspect there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 203296_s_at | 214598_at | 221305_s_at | 235146_at |
| 206664_at | 219948_x_at | 229831_at | 238751_at |
| 211549_s_at | 220812_s_at | 231925_at | 243278_at; |

and/or
**(ii)**

| | | | |
|---|---|---|---|
| ATP1A2 | FOXP2 | P2RY1 | UGT1A8 |
| CLDN8 | HHLA2 | SI | UGT2A3 |
| CNTN3 | HPGD | SORBS2 | |

in a biological sample from said individual wherein a lower level of expression of the genes or transcripts of group (i) and/or group (ii) which is not substantially above background levels is indicative of a cancer cell or a cell predisposed to the onset of a cancerous state.

In another further aspect there is provided a method of characterising a neoplastic cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | | |
|---|---|---|---|
| 200600_at | 202766_s_at | 210495_x_at | 218469_at |
| 200665_s_at | 202859_x_at | 210511_s_at | 218559_s_at |
| 200832_s_at | 202878_s_at | 210764_s_at | 218638_s_at |
| 200974_at | 202917_s_at | 210809_s_at | 219087_at |
| 200986_at | 202998_s_at | 211161_s_at | 221011_s_at |
| 201058_s_at | 203083_at | 211571_s_at | 221729_at |
| 201069_at | 203325_s_at | 211719_x_at | 221730_at |
| 201105_at | 203382_s_at | 211813_x_at | 221731_x_at |
| 201141_at | 203477_at | 211896_s_at | 37892_at |
| 201147_s_at | 203570_at | 211959_at | 223122_s_at |
| 201150_s_at | 203645_s_at | 211964_at | 223235_s_at |
| 201162_at | 203878_s_at | 211966_at | 224560_at |
| 201163_s_at | 204006_s_at | 211980_at | 224694_at |
| 201185_at | 204051_s_at | 211981_at | 224724_at |
| 201261_x_at | 204122_at | 212077_at | 225664_at |
| 201289_at | 204320_at | 212344_at | 225681_at |
| 201426_s_at | 204475_at | 212353_at | 225710_at |
| 201438_at | 204620_s_at | 212354_at | 225799_at |
| 201616_s_at | 205479_s_at | 212464_s_at | 226237_at |
| 201645_at | 205547_s_at | 212488_at | 226311_at |
| 201667_at | 205828_at | 212489_at | 226694_at |
| 201744_s_at | 207173_x_at | 212667_at | 226777_at |
| 201792_at | 207191_s_at | 213125_at | 226930_at |
| 201842_s_at | 208747_s_at | 213428_s_at | 227099_s_at |
| 201852_x_at | 208782_at | 213524_s_at | 227140_at |
| 201859_at | 208788_at | 213869_x_at | 227566_at |
| 201893_x_at | 208850_s_at | 213905_x_at | 229218_at |
| 202237_at | 208851_s_at | 214247_s_at | 229802_at |
| 202238_s_at | 209101_at | 215049_x_at | 231579_s_at |
| 202283_at | 209156_s_at | 215076_s_at | 231766_s_at |
| 202291_s_at | 209218_at | 215646_s_at | 231879_at |
| 202310_s_at | 209395_at | 216442_x_at | 232458_at |
| 202311_s_at | 209396_s_at | 217430_x_at | 233555_s_at |
| 202403_s_at | 209596_at | 217762_s_at | 234994_at; and/or |
| 202404_s_at | 209875_s_at | 217763_s_at | |
| 202450_s_at | 209955_s_at | 217764_s_at | |
| 202620_s_at | 210095_s_at | 218468_s_at | |

**(ii)**

| | | | |
|---|---|---|---|
| COL1A2 | INHBA | FAP | IGFBP3 |
| CTHRC1 | COL6A2 | VIM | SERPINF1 |
| FN1 | ANTXR1 | TIMP2 | ISLR |
| POSTN | GPNMB | SCD | HNT |
| SPP1 | BGN | TIMP3 | COL5A1 |
| MMP1 | TAGLN | AEBP1 | OLFML2B |
| SPARC | COL4A1 | GJA1 | KIAA1913 |
| LUM | RAB31 | NNMT | PALM2-AKAP2 |
| GREM1 | LGALS1 | COL1A1 | SERPING1 |
| IL8 | ELOVL5 | SULF2 | TYROBP |
| IGFBP5 | MGP | COL6A1 | ACTA2 |
| SFRP2 | MMP2 | SPON2 | COL3A1 |
| SULF1 | LOXL2 | CTSK | PLOD2 |
| ASPN | MYL9 | MXRA5 | MMP11 |
| COL6A3 | DCN | C1S | CD163 |
| COL8A1 | CALD1 | DKK3 | FCGR3B |
| COL12A1 | FBN1 | SRGN | PLAU |
| COL5A2 | MMP3 | LBH | MAFB |
| CDH11 | IGFBP7 | CTGF | LOC541471 |
| THBS2 | FSTL1 | TNC | LOC387763 |
| COL15A1 | COL4A2 | G0S2 | CHI3L1 |
| COL11A1 | VCAN | SQLE | THY1 |
| S100A8 | SMOC2 | EFEMP1 | LOXL1 |
| FNDC1 | HTRA1 | APOE | CD93 |
| SFRP4 | CYR61 | MSN | |

in said cell or cellular population wherein a lower level of expression of the genes of group (i) and/or group (ii) relative to a gastrointestinal cancer control level is indicative of an adenoma cell or a cell predisposed to the onset of an adenoma state.

In still another aspect there is provided a method of characterising a neoplastic cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | |
|---|---|---|
| 200884_at | 214234_s_at | 226248_s_at |
| 203240_at | 214235_at | 226302_at |
| 203963_at | 214433_s_at | 227676_at |
| 204508_s_at | 215125_s_at | 227719_at |
| 204607_at | 215867_x_at | 227725_at |
| 204811_s_at | 217109_at | 228232_s_at |
| 204895_x_at | 217110_s_at | 229070_at |
| 204897_at | 218211_s_at | 231832_at |
| 205259_at | 219543_at | 232176_at |
| 205765_at | 219955_at | 232481_s_at |
| 205927_s_at | 221841_s_at | 235976_at |
| 208063_s_at | 221874_at | 236894_at |
| 208937_s_at | 223969_s_at | 237521_x_at |
| 210107_at | 223970_at | 242601_at; and/or |
| 213106_at | | |

**(ii)**

| | | |
|---|---|---|
| CLCA1 | CTSE | ATP8B1 |
| FCGBP | C6orf105 | CACNA2D2 |
| HMGCS2 | CKB | KLF4 |
| RETNLB | ATP8A1 | CYP3A5P2 |
| L1TD1 | MUC4 | CAPN9 |
| SLITRK6 | UGT1A1 | NR3C2 |
| VSIG2 | SELENBP1 | PBLD |
| LOC253012 | PTGER4 | CA12 |
| ST6GALNAC1 | MLPH | WDR51B |
| ID1 | KIAA1324 | FAM3D |
| CYP3A5 | | |

in a biological sample from said individual wherein a lower level of expression of the genes or transcripts of group (i) and/or group (ii) relative to a gastrointestinal adenoma control level is indicative of a cancer or a cell predisposed to the onset of a cancerous state.

In yet another aspect there is provided a method of characterising a neoplastic cell or cellular population, which cell or cellular population is derived from the large intestine of an individual, said method comprising assessing the level of expression of one or more genes or transcripts selected from:
**(i)** the gene or genes detected by Affymetrix probeset IDs:
   202404_s_at, 212464_s_at, 210809_s_at, 225681_at; and/or
**(ii)** COL1A2, FN1, POSTN, CTHRC1
in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In yet still another aspect, there is provided a method of characterising a neoplastic cell or cellular population, which cell or cellular population is derived from the large intestine of an individual, said method comprising assessing the level of expression of one or more genes selected from:
**(i)** the gene, genes or transcripts detected by Affymetrix probeset IDs:

| | | |
|---|---|---|
| 209875_s_at | 227140_at | 204475_at; |

and/or **(ii)**

| | |
|---|---|
| SPP1 | MMP1 |

in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In still yet another aspect the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | | |
|---|---|---|---|
| 200665_s_at | 219087_at | 37892_at | 201141_at |
| 201744_s_at | 226237_at | 202917_s_at | 213905_x_at |
| 218468_s_at | 225664_at | 226930_at | 205547_s_at |
| 202859_x_at | 221730_at | 204051_s_at | 201438_at; |
| 211959_at | 207173_x_at | 210511_s_at | and/or |
| 223122_s_at | 203083_at | 209156_s_at | |
| 212353_at | 203477_at | 224694_at | |

**(ii)**

| | | | |
|---|---|---|---|
| SPARC | ASPN | COL15A1 | ANTXR1 |
| LUM | COL6A3 | COL11A1 | GPNMB |
| GREM1 | COL8A1 | S100A8 | BGN |
| IL8 | COL12A1 | FNDC1 | TAGLN |
| IGFBP5 | COL5A2 | SFRP4 | |
| SFRP2 | CDH11 | INHBA | |
| SULF1 | THBS2 | COL6A2 | |

in a biological sample from said individual wherein a lower level of expression of the genes or transcripts of group (i) and/or (ii) relative to a gastrointestinal cancer control level is indicative of an adenoma or a cell predisposed to the onset of an adenoma state.

In yet another aspect the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:
   210107_at; and/or
**(ii)** CLCA1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

In still yet another aspect the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | |
|---|---|---|
| 203240_at | 219955_at | 242601_at |
| 204607_at | 232481_s_at | 227725_at |
| 223969_s_at | 228232_s_at; and/or | |

**(ii)**

| | | |
|---|---|---|
| FCGBP | L1TD1 | LOC253012 |
| HMGCS2 | SLITRK6 | ST6GALNAC1 |
| RETNLB | VSIG2 | |

in a biological sample from said individual wherein a lower level of expression of the genes or transcripts of group (i) and/or (ii) relative to a gastrointestinal adenoma control level is indicative of a cancer or a cell predisposed to the onset of a cancerous state.

A further aspect of the present disclosure provides a method of characterising a neoplastic cell or cellular population, which cell or cellular population is derived from the large intestine of an individual, said method comprising assessing the level of expression of one or more genes or transcripts selected from:
**(i)** the gene or genes detected by Affymetrix probeset IDs:

| | | |
|---|---|---|
| 235976_at | 236894_at | 237521; |

and/or **(ii)**

| | |
|---|---|
| SLITRK6 | L1TD1 |

in a biological sample from said individual wherein expression of the genes or transcripts of group (i) and/or (ii) at a level which is not substantially greater than background neoplastic tissue levels is indicative of a cancer or a cell predisposed to the onset of a cancerous state.

A related aspect of the present disclosure provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

### BRIEF DESCRIPTION OF THE DRAWINGS

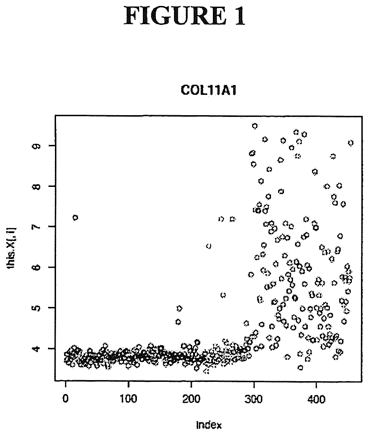

**Figure 1** is a graphical representation of alcohol dehydrogenase IB (class I), beta polypeptide.

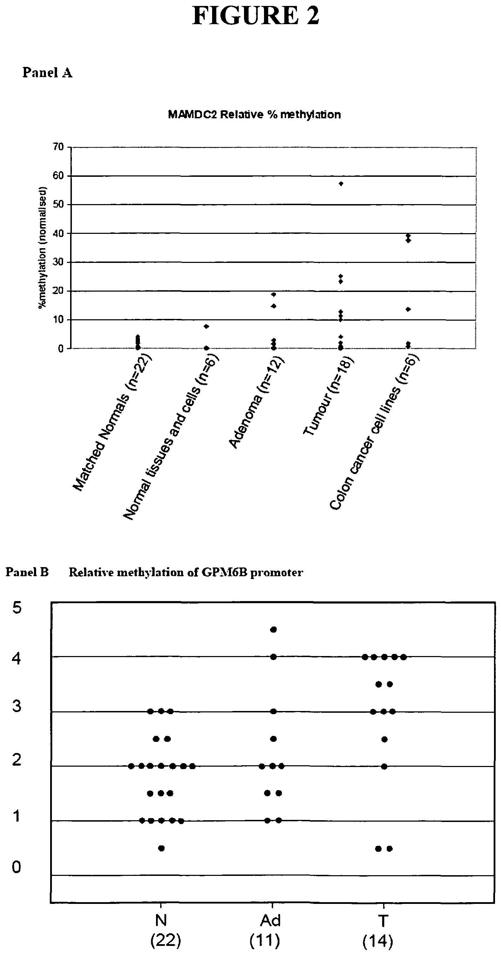

**Figure 2** is a graphical representation of the methylation of MAMDC2 and GPM6B in normal and neoplastic tissues and cell lines. Panel A shows the methylation level of the MAMDC2 gene as assessed by methylation specific PCR, using amplification of the CAGE gene to normalise for input DNA levels. Each point represents an individual tissue sample or cell line. Samples included DNAs from 18 colorectal cancer tissues, 12 colorectal adenomas, 22 matched normal colorectal tissues, 6 other normal tissues and a cell line and 6 colon cancer cell lines. Panel B shows the relative level of methylation of the GPM6B gene assessed by a COBRA assay. Levels of methylation were scored between 0 (no restriction enzyme digestion) and 5 (complete restriction enzyme digestion). Each point represents a single tissue sample. Samples included 14 colorectal cancer tissues, 11 colorectal adenomas and 22 matched normal tissues.

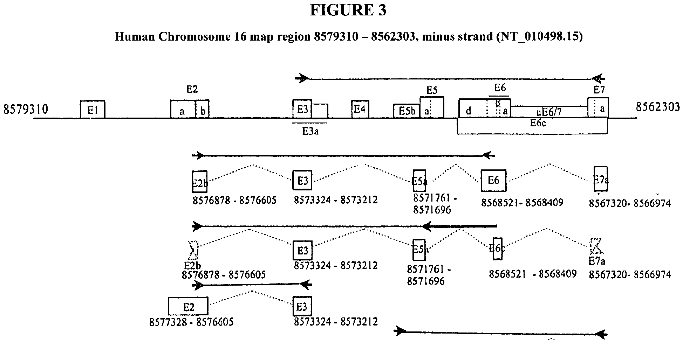

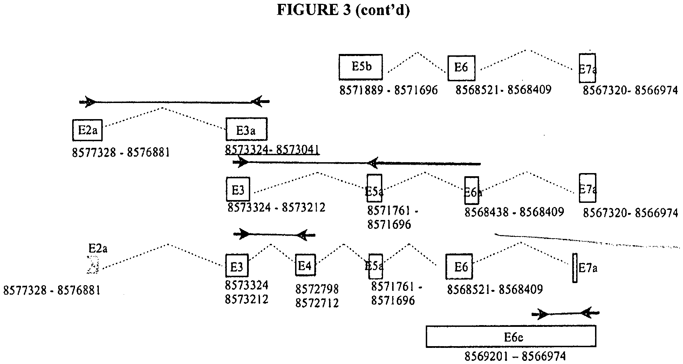

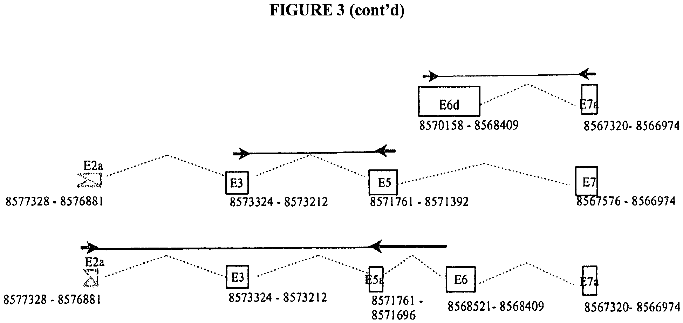

**Figure 3** is a schematic representation of predicted RNA variants derived from hCG_1815491. cDNA clones derived from map region 8579310 to 8562303 on human chromosome 16 were used to locate exon sequences. Arrows: Oligo nucleotide primer sets were designed to allow measurement of individual RNA variants by PCR. Primers covering splice junctions are shown as spanning intron sequences which is not included in the actual oligonucleotide primer sequence.

### DETAILED DESCRIPTION OF THE INVENTION

The present invention is predicated, in part, on the elucidation of gene expression profiles which characterise large intestine cellular populations in terms of their neoplastic state and, more particularly, whether they are malignant or pre-malignant. This finding has now facilitated the development of routine means of screening for the onset or predisposition to the onset of a large intestine neoplasm or characterising cellular populations derived from the large intestine based on screening for downregulation of the expression of these molecules, relative to control expression patterns and levels. To this end, in addition to assessing expression levels of the subject genes relative to normal or non-neoplastic levels, it has been determined that a proportion of these genes are not expressed in the diseased state, thereby facilitating the development of a simple qualitative test based on requiring assessment only relative to test background levels.

In accordance with the present disclosure it has been determined that the genes detailed above are modulated, in terms of differential changes to their levels of expression, depending on whether the cell expressing that gene is neoplastic or not. It should be understood that reference to a gene "expression product" or "expression of a gene" is a reference to either a transcription product (such as primary RNA or mRNA) or a translation product such as protein. In this regard, one can assess changes to the level of expression of a gene either by screening for changes to the level of expression product which is produced (i.e. RNA or protein), changes to the chromatin proteins with which the gene is associated, for example the presence of histone H3 methylated on lysine at amino acid position number 9 or 27 (repressive modifications) or changes to the DNA itself which acts to downregulate expression, such as changes to the methylation of the DNA. These genes and their gene expression products, whether they be RNA transcripts, changes to the DNA which act to downregulate expression or encoded proteins, are collectively referred to as "neoplastic markers".

Accordingly, one aspect of the present disclosure is directed to a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:

| | | | |
|---|---|---|---|
| 200600_at | 208399_s_at | 217546_at | 203296_s_at |
| 200621_at | 208450_at | 217757_at | 203343_at |
| 200795_at | 208581_x_at | 217762_s_at | 203474_at |
| 200799_at | 208747_s_at | 217764_s_at | 203638_s_at |
| 200845_s_at | 208763_s_at | 217767_at | 203963_at |
| 200859_x_at | 208788_at | 217897_at | 204018_x_at |
| 200897_s_at | 208789_at | 217967_s_at | 204034_at |
| 200974_at | 208791_at | 218087_s_at | 204036_at |
| 200986_at | 208792_s_at | 218162_at | 204130_at |
| 201041_s_at | 208894_at | 218224_at | 204388_s_at |
| 201058_s_at | 209047_at | 218312_s_at | 204389_at |
| 201061_s_at | 209074_s_at | 218353_at | 204508_s_at |
| 201069_at | 209101_at | 218418_s_at | 204532_x_at |
| 201105_at | 209116_x_at | 218468_s_at | 204607_at |
| 201137_s_at | 209138_x_at | 218469_at | 204673_at |
| 201141_at | 209147_s_at | 218559_s_at | 204818_at |
| 201150_s_at | 209156_s_at | 218756_s_at | 204895_x_at |
| 201289_at | 209167_at | 219014_at | 204897_at |
| 201300_s_at | 209170_s_at | 219087_at | 205112_at |
| 201324_at | 209191_at | 219508_at | 205259_at |
| 201348_at | 209209_s_at | 219607_s_at | 205403_at |
| 201426_s_at | 209210_s_at | 219669_at | 205480_s_at |
| 201427_s_at | 209283_at | 219799_s_at | 205554_s_at |
| 201438_at | 209301_at | 220026_at | 205593_s_at |
| 201496_x_at | 209312_x_at | 220037_s_at | 205892_s_at |
| 201497_x_at | 209335_at | 220376_at | 205929_at |
| 201539_s_at | 209357_at | 220834_at | 206000_at |
| 201540_at | 209373_at | 221541_at | 206094_x_at |
| 201616_s_at | 209436_at | 221667_s_at | 206262_at |
| 201617_x_at | 209457_at | 221747_at | 206377_at |
| 201645_at | 209496_at | 221748_s_at | 206385_s_at |
| 201667_at | 209498_at | 222043_at | 206664_at |
| 201739_at | 209612_s_at | 222162_s_at | 207126_x_at |
| 201743_at | 209613_s_at | 222453_at | 207245_at |
| 201744_s_at | 209621_s_at | 222513_s_at | 207390_s_at |
| 201842_s_at | 209651_at | 222717_at | 207392_x_at |
| 201852_x_at | 209656_s_at | 222722_at | 207432_at |
| 201858_s_at | 209667_at | 223121_s_at | 207761_s_at |
| 201859_at | 209668_x_at | 223122_s_at | 208596_s_at |
| 201865_x_at | 209687_at | 223235_s_at | 208920_at |
| 201893_x_at | 209735_at | 223343_at | 209114_at |
| 201920_at | 209763_at | 223395_at | 209374_s_at |
| 201957_at | 209868_s_at | 223551_at | 209458_x_at |
| 202007_at | 209948_at | 223623_at | 209791_at |
| 202037_s_at | 210084_x_at | 223952_x_at | 210107_at |
| 202069_s_at | 227099_s_at | 224009_x_at | 210524_x_at |
| 202133_at | 210133_at | 224352_s_at | 210735_s_at |
| 202222_s_at | 210139_s_at | 224412_s_at | 211372_s_at |
| 202242_at | 210298_x_at | 224480_s_at | 211538_s_at |
| 202274_at | 210299_s_at | 224560_at | 211549_s_at |
| 202283_at | 210302_s_at | 224663_s_at | 211637_x_at |
| 202291_s_at | 210495_x_at | 224694_at | 211699_x_at |
| 202388_at | 210517_s_at | 224823_at | 211745_x_at |
| 202555_s_at | 210764_s_at | 224836_at | 212224_at |
| 202620_s_at | 210809_s_at | 224840_at | 212592_at |
| 202686_s_at | 210946_at | 224959_at | 212741_at |
| 202746_at | 210982_s_at | 224963_at | 212814_at |
| 202760_s_at | 211161_s_at | 224964_s_at | 213317_at |
| 202766_s_at | 211548_s_at | 225207_at | 213451_x_at |
| 202888_s_at | 211596_s_at | 225242_s_at | 213629_x_at |
| 202920_at | 211643_x_at | 225269_s_at | 213921_at |
| 202953_at | 211644_x_at | 225275_at | 213953_at |
| 202957_at | 211645_x_at | 225353_s_at | 214164_x_at |
| 202992_at | 211671_s_at | 225381_at | 214433_s_at |
| 202994_s_at | 211696_x_at | 225442_at | 214598_at |
| 202995_s_at | 211719_x_at | 225575_at | 214916_x_at |
| 203000_at | 211798_x_at | 225602_at | 215125_s_at |
| 203001_s_at | 211813_x_at | 225604_s_at | 215299_x_at |
| 203066_at | 211848_s_at | 225626_at | 215867_x_at |
| 203131_at | 211889_x_at | 225688_s_at | 216336_x_at |
| 203305_at | 211896_s_at | 225710_at | 216491_x_at |
| 203382_s_at | 211959_at | 225720_at | 216510_x_at |
| 203477_at | 211964_at | 225721_at | 217022_s_at |
| 203645_s_at | 211985_s_at | 225782_at | 217109_at |
| 203680_at | 211990_at | 225894_at | 217110_s_at |
| 203729_at | 211991_s_at | 225895_at | 217165_x_at |
| 203748_x_at | 212077_at | 226001_at | 217232_x_at |
| 203766_s_at | 212091_s_at | 226051_at | 217414_x_at |
| 203881_s_at | 212097_at | 226084_at | 218541_s_at |
| 203908_at | 212136_at | 226103_at | 218546_at |
| 203913_s_at | 212158_at | 226303_at | 219059_s_at |
| 203914_x_at | 212185_x_at | 226304_at | 219543_at |
| 203951_at | 212192_at | 226333_at | 219796_s_at |
| 203980_at | 212195_at | 226430_at | 219948_x_at |
| 204069_at | 212230_at | 226492_at | 220075_s_at |
| 204083_s_at | 212233_at | 226682_at | 220266_s_at |
| 204122_at | 212265_at | 226694_at | 220468_at |
| 204135_at | 212288_at | 226818_at | 220645_at |
| 204326_x_at | 212386_at | 226834_at | 220812_s_at |
| 204438_at | 212387_at | 226841_at | 221004_s_at |
| 204457_s_at | 212397_at | 227006_at | 221305_s_at |
| 204570_at | 212414_s_at | 227061_at | 221584_s_at |
| 204688_at | 212419_at | 227235_at | 221841_s_at |
| 204697_s_at | 212464_s_at | 227265_at | 221896_s_at |
| 204719_at | 212667_at | 227404_s_at | 223484_at |
| 204745_x_at | 212671_s_at | 227529_s_at | 223597_at |
| 204834_at | 212713_at | 227561_at | 223754_at |
| 204894_s_at | 212730_at | 227623_at | 224342_x_at |
| 204931_at | 212764_at | 227662_at | 224989_at |
| 204938_s_at | 212859_x_at | 227705_at | 224990_at |
| 204939_s_at | 212956_at | 227727_at | 225458_at |
| 204940_at | 213068_at | 227826_s_at | 225728_at |
| 204955_at | 213071_at | 227827_at | 226147_s_at |
| 205097_at | 213428_s_at | 228202_at | 226302_at |
| 205200_at | 213509_x_at | 228504_at | 226594_at |
| 205267_at | 213624_at | 228507_at | 226654_at |
| 205382_s_at | 213746_s_at | 228640_at | 226811_at |
| 205412_at | 213891_s_at | 228706_s_at | 227052_at |
| 205433_at | 214027_x_at | 228707_at | 227522_at |
| 205464_at | 214038_at | 228750_at | 227682_at |
| 205547_s_at | 214091_s_at | 228766_at | 227725_at |
| 205683_x_at | 214142_at | 228846_at | 227735_s_at |
| 205935_at | 214414_x_at | 228854_at | 227736_at |
| 205950_s_at | 214505_s_at | 228885_at | 228133_s_at |
| 206134_at | 214677_x_at | 229530_at | 228195_at |
| 206143_at | 214696_at | 229839_at | 228232_s_at |
| 206149_at | 214752_x_at | 230087_at | 228241_at |
| 206198_s_at | 214768_x_at | 230264_s_at | 228469_at |
| 206199_at | 214777_at | 230788_at | 228961_at |
| 206208_at | 215049_x_at | 230830_at | 229070_at |
| 206209_s_at | 215076_s_at | 231120_x_at | 229254_at |
| 206422_at | 215118_s_at | 231579_s_at | 229659_s_at |
| 206461_x_at | 215176_x_at | 231773_at | 229831_at |
| 206561_s_at | 215193_x_at | 234764_x_at | 230595_at |
| 206576_s_at | 215382_x_at | 234987_at | 231925_at |
| 206637_at | 215388_s_at | 236300_at | 231975_s_at |
| 206641_at | 215657_at | 236313_at | 233565_s_at |
| 206710_s_at | 216207_x_at | 242317_at | 235146_at |
| 206784_at | 216401_x_at | 200884_at | 235766_x_at |
| 207003_at | 216442_x_at | 201495_x_at | 235849_at |
| 207080_s_at | 216474_x_at | 202266_at | 238143_at |
| 207134_x_at | 216576_x_at | 202350_s_at | 238750_at |
| 207266_x_at | 216834_at | 202731_at | 238751_at |
| 207502_at | 216984_x_at | 202741_at | 239272_at |
| 207961_x_at | 217148_x_at | 202742_s_at | 241994_at |
| 207977_s_at | 217179_x_at | 202768_at | 242447_at |
| 207980_s_at | 217235_x_at | 202838_at | 242601_at |
| 208131_s_at | 217258_x_at | 203058_s_at | 243278_at; |
| 208370_s_at | 217378_x_at | 203060_s_at | and/or |
| 208383_s_at | 217480_x_at | 203240_at | |

**(ii)**

| | | | |
|---|---|---|---|
| CLCA4 | VIM | CA12 | AP1S2 |
| ZG16 | SMPDL3A | FKBP5 | EMP3 |
| CA2 | P2RY14 | HSPB8 | MMP28 |
| CA1 | CHGA | TPSB2 | UGT2A3 |
| MS4A12 | C15orf48 | FGL2 | RGS5 |
| AQP8 | COL3A1 | C1QB | PTGIS |
| SLC4A4 | CYR61 | ANGPTL1 | DUSP5 |
| CEACAM7 | TRPM6 | MEP1A | MFAP4 |
| TAGLN | OSTbeta | GUCY1A3 | UGT1A6 |
| GUCA1B | IGLV1-44 | UGDH | PRKAR2B |
| GCG | VSIG2 | DUSP1 | HHLA2 |
| ADH1B | IGHM | C2orf40 | LOC652128 |
| UGT2B17 | LRRC19 | PLN | C3 |
| ADAMDEC1 | CD163 | UGT2B15 | ATP2B4 |
| MT1M | CEACAM1 | PDLIM3 | HBA1 |
| AKR1B10 | TIMP2 | TP53INP2 | TCF21 |
| FN1 | ENTPD5 | ATP8B1 | PPID |
| MGP | DDR2 | ANK3 | PPAP2B |
| CXCL12 | CHRDL1 | CTGF | SPON1 |
| PDK4 | SRGN | MUCDHL | PHLDB2 |
| CA4 | PDE9A | SDPR | RARRES2 |
| PYY | PMP22 | COL14A1 | ETHE1 |
| IGHA1 | FLNA | DSCR1 | MMP2 |
| TPM2 | STMN2 | CITED2 | SRI |
| C6orf105 | MYL9 | MT1H | CNTN3 |
| HPGD | SEMA6D | NEXN | RGS2 |
| ADH1C | PADI2 | MUC2 | COL6A1 |
| CLCA1 | SEPP1 | NID1 | FBN1 |
| FABP1 | TGFB1I1 | HBB | MXD1 |
| ENAM | SFRP2 | GCNT2 | PLCE1 |
| CFD | UGT1A3 | C20orf118 | KCNMB1 |
| GUCA2B | MS4A7 | SLC20A1 | CALM1 |
| FBLN1 | ALDH1A1 | CD14 | HLA-DPB1 |
| LOC63928 | SGK | KCTD12 | SMOC2 |
| ABCA8 | CFL2 | RBMS1 | LOC285382 |
| POSTN | C1S | PTRF | CLIC5 |
| DCN | SELENBP1 | TSPAN1 | APOE |
| ITLN1 | MT1E | UGT1A9 | SERPINF1 |
| COL6A2 | ADAMTS1 | COX7A1 | PPP1R12B |
| FCGBP | ITM2A | MUC12 | HSPB6 |
| SLC26A2 | POU2AF1 | PDCD4 | FNBP1 |
| PGM5 | FAM55D | CAV1 | C4orf34 |
| DMN | C6orf204 | FAM46C | SORBS2 |
| GPNMB | AKAP12 | LRIG1 | GPA33 |
| IGFBP5 | TUBB6 | HLA-DPA1 | GALNAC4S- |
| CLEC3B | LGALS2 | C1orf115 | 6ST |
| LOC253012 | KIAA0828 | HBA2 | CFHR1 |
| DPT | MGC14376 | EDIL3 | MGC13057 |
| PCK1 | PPP1R14A | DES | C10orf56 |
| CNN1 | MUC4 | MT2A | SULT1A1 |
| HSD17B2 | PKIB | KCNMA1 | TTRAP |
| PLAC8 | PIGR | GAS1 | CCL28 |
| TMEM47 | ASPN | TBC1D9 | IDH3A |
| OGN | A2M | C7 | EDG2 |
| CALD1 | LOC25845 | P2RY1 | UGT1A8 |
| ACTG2 | LGALS1 | NR3C1 | RAB27A |
| MGC4172 | BCHE | STOM | ANTXR1 |
| MAB21L2 | ST6GALNAC1 | CKB | EMP1 |
| RPL24 | GJA1 | CLU | CSRP1 |
| ABCG2 | SCNN1B | SLC26A3 | PLEKHC1 |
| CCDC80 | FABP4 | SDC2 | LOC572558 |
| UGT1A1 | F13A1 | SST | FOXP2 |
| MRC1 | CD36 | HLA-DRA | HSPA2 |
| HSD11B2 | SPARCL1 | TSC22D3 | ATP1A2 |
| ANPEP | ZCWPW2 | IL6ST | TNXB |
| MATN2 | TNC | C1QC | FUCA1 |
| PRNP | MT1A | MT1X | MRGPRF |
| ABI3BP | LOC652745 | AOC3 | HIGD1A |
| HLA-C | MALL | PPAP2A | MFSD4 |
| NDE1 | GNG2 | ZSCAN18 | AXL |
| SRPX | DNASE1L3 | IVD | AQP1 |
| WWTR1 | EGR1 | SFRP1 | MAP1B |
| HMGCS2 | CMBL | COL4A2 | PALLD |
| LOC646627 | GCNT3 | GPM6B | MPEG1 |
| KRT20 | SERPING1 | EPB41L3 | KLHL5 |
| KLF4 | MEIS1 | MAOA | TCEAL7 |
| FHL1 | EDN3 | DMD | FILIP1L |
| ARL14 | MSN | MSRB3 | IQGAP2 |
| LUM | MT1G | PLOD2 | PRDX6 |
| SORBS1 | TPSAB1 | C9orf19 | RAB31 |
| METTL7A | GPX3 | MIER3 | LOC96610 |
| FAM129A | CDKN2B | XDH | FGFR2 |
| SCARA5 | FOSB | CLDN23 | PAPSS2 |
| SI | HSPA1A | SGCE | XLKD1 |
| ACTA2 | CYBRD1 | FOXF2 | SMTN |
| CD177 | PTGER4 | AGR3 | C8orf4 |
| C10orf99 | MAGI | IGLJ3 | SDCBP2 |
| COL15A1 | BEST2 | QKI | CCL11 |
| NR3C2 | HLA-DQA1 | LOC399959 | ELOVL5 |
| DHRS9 | PRIMA1 | ANKRD25 | FOXF1 |
| LMOD1 | MT1F | CRISPLD2 | RELL1 |
| EFEMP1 | MAFB | ANK2 | PNMA1 |
| GREM1 | FAM107A | LOC283666 | LOC339562 |
| IL1R2 | PRKACB | CRYAB | PALM2- |
| LOC387763 | SELM | ACAT1 | AKAP2 |
| TIMP3 | TYROBP | IGL@ | PAG1 |
| MYLK | TNS1 | PBLD | HCLS1 |
| CLDN8 | MYH11 | CCL8 | RGS1 |
| RDX | ITM2C | LIFR | FXYD6 |
| TSPAN7 | CES2 | HLA-DRB1 | OLFML3 |
| TNFRSF17 | MS4A4A | UGP2 | COL6A3 |
| SYNPO2 | PDGFRA | IGKV1D-13 | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In one embodiment, said expression is assessed by screening for DNA changes which impact on methylation, in particular hypermethylation. In another embodiment expression is assessed by the association of DNA with chromatin proteins carrying repressive modifications, for example, methylation of lysines 9 or 27 of the histone H3.

Reference to "large intestine" should be understood as a reference to a cell derived from one of the six anatomical regions of the large intestine, which regions commence after the terminal region of the ileum, these being:
(i) the cecum;
(ii) the ascending colon;
(iii) the transverse colon;
(iv) the descending colon;
(v) the sigmoid colon; and
(vi) the rectum.

Reference to "neoplasm" should be understood as a reference to a lesion, tumour or other encapsulated or unencapsulated mass or other form of growth which comprises neoplastic cells. A "neoplastic cell" should be understood as a reference to a cell exhibiting abnormal growth. The term "growth" should be understood in its broadest sense and includes reference to proliferation. In this regard, an example of abnormal cell growth is the uncontrolled proliferation of a cell. Another example is failed apoptosis in a cell, thus prolonging its usual life span. The neoplastic cell may be a benign cell or a malignant cell. In a preferred embodiment, the subject neoplasm is an adenoma or an adenocarcinoma. Without limiting the present invention to any one theory or mode of action, an adenoma is generally a benign tumour of epithelial origin which is either derived from epithelial tissue or exhibits clearly defined epithelial structures. These structures may take on a glandular appearance. It can comprise a malignant cell population within the adenoma, such as occurs with the progression of a benign adenoma to a malignant adenocarcinoma.

Preferably, said neoplastic cell is an adenoma or adenocarcinoma and even more preferably a colorectal adenoma or adenocarcinoma.

Each of the genes and transcripts detailed in sub-paragraphs (i) and (ii), above, would be well known to the person of skill in the art, as would their encoded proteins. The identification of the expression products of these genes and transcripts as markers of neoplasia occurred by virtue of differential expression analysis using Affymetrix HGU133Aor HGU133B gene chips. To this end, each gene chip is characterised by approximately 45,000 probe sets which detect the RNA transcribed from the genome. On average, approximately 11 probe pairs detect overlapping or consecutive regions of the RNA transcript. In general, the genes from which the RNA transcripts described herein are identifiable by the Affymetrix probesets are well known and characterised genes. However, to the extent that some of the probesets detect RNA transcripts which are not yet defined, these transcripts are indicated as "the gene, genes or transcripts detected by Affymetrix probe x". In some cases a number of genes may be detectable by a single probeset. It should be understood, however, that this is not intended as a limitation as to how the expression level of the subject gene or transcript can be detected. In the first instance, it would be understood that the subject gene transcript is also detectable by other probesets which would be present on the Affymetrix gene chip. The reference to a single probeset is merely included as an identifier of the gene transcript of interest. In terms of actually screening for the transcript, however, one may utilise a probe or probeset directed to any region of the transcript and not just to the 3- terminal 600bp transcript region to which the Affymetrix probes are often directed.

Reference to each of the genes and transcripts detailed above and their transcribed and translated expression products should therefore be understood as a reference to all forms of these molecules and to fragments or variants thereof. As would be appreciated by the person of skill in the art, some genes are known to exhibit allelic variation between individuals. Accordingly, the present disclosure should be understood to extend to such variants which, in terms of the present diagnostic applications, achieve the same outcome despite the fact that minor genetic variants between the actual nucleic acid sequences may exist between individuals or that within one individual there may exist 2 or more splice variants of the subject gene. The present invention should therefore be understood to extend to all forms of RNA (eg mRNA, primary RNA transcript, miRNA, etc), cDNA and peptide isoforms which arise from alternative splicing or any other mutation, polymorphic or allelic variation. It should also be understood to include reference to any subunit polypeptides such as precursor forms which may be generated, whether existing as a monomer, multimer, fusion protein or other complex.

To this end, in terms of the genes encompassed by the present disclosure means for determining the existence of such variants, and characterising same, are described in Example 6. To the extent that the genes of the present disclosure are described by reference to an Affymetrix probeset, Table 6 provides details of the nucleic acid sequence to which each probe set is directed. Based on this information, the skilled person could, as a matter of routine procedure, identify the gene in respect of which that sequence forms part. A typical protocol for doing this is also outlined in Example 6.

It should be understood that the "individual" who is the subject of testing may be any human or non-human mammal. Examples of non-human mammals includes primates, livestock animals (e.g. horses, cattle, sheep, pigs, donkeys), laboratory test animals (e.g. mice, rats, rabbits, guinea pigs), companion animals (e.g. dogs, cats) and captive wild animals (e.g. deer, foxes). Preferably the mammal is a human.

The method of the present invention is predicated on the comparison of the level of the neoplastic markers of a biological sample with the control levels of these markers. The "control level" may be either a "normal level", which is the level of marker expressed by a corresponding large intestine cell or cellular population which is not neoplastic.

The normal (or "non-neoplastic") level may be determined using tissues derived from the same individual who is the subject of testing. However, it would be appreciated that this may be quite invasive for the individual concerned and it is therefore likely to be more convenient to analyse the test results relative to a standard result which reflects individual or collective results obtained from individuals other than the patient in issue. This latter form of analysis is in fact the preferred method of analysis since it enables the design of kits which require the collection and analysis of a single biological sample, being a test sample of interest. The standard results which provide the normal level may be calculated by any suitable means which would be well known to the person of skill in the art. For example, a population of normal tissues can be assessed in terms of the level of the neoplastic markers of the present invention, thereby providing a standard value or range of values against which all future test samples are analysed. It should also be understood that the normal level may be determined from the subjects of a specific cohort and for use with respect to test samples derived from that cohort. Accordingly, there may be determined a number of standard values or ranges which correspond to cohorts which differ in respect of characteristics such as age, gender, ethnicity or health status. Said "normal level" may be a discrete level or a range of levels. A decrease in the expression level of the subject genes relative to normal levels is indicative of the tissue being neoplastic.

Without limiting the present disclosure to any one theory or mode of action, although each of the genes or transcripts hereinbefore described is differentially expressed, either singly or in combination, as between neoplastic versus non-neoplastic cells of the large intestine, and is therefore diagnostic of the existence of a large intestine neoplasm, the expression of some of these genes was found to exhibit particularly significant levels of sensitivity, specificity and positive and negative predictive value. Accordingly, in a preferred embodiment one would screen for and assess the expression level of one or more of these genes. To this end, and without limiting the present disclosure to any one theory or mode of action, the following markers were determined to be expressed in neoplastic tissue at a level of 3-11 fold less than non-neoplastic tissue, when assessed by virtue of the method exemplified herein:

| **Fold Decrease** | **Gene, genes or transcripts detected by Affymetrix Probe No:** | **Gene** |
|---|---|---|
| 11 | 220026_at | CLCA4 |
| 10 | 214142_at | ZG16 |
| 9 | 209301_at | CA1 |
| | 205950_s_at | CA2 |
| 8 | 220834_at | MS4A12 |
| 7 | 206784_at | AQP8 |
| 6 | 203908_at | SLC4A4 |
| | 206198_s_at | CEACAM7 |
| | 205547_s_at | TAGLN |
| | 207003_at | GUCA1B |
| | 206422_at | GCG |
| | 209613_s_at | ADH1B |
| | 207245_at | UGT2B 17 |
| 5 | 206134_at | ADAMDEC1 |
| | 217546_at | MT1M |
| | 206561_s_at | AKR1B10 |
| | 211719_x_at | FN1 |
| | 202291_s_at | MGP |
| | 209687_at | CXCL12 |
| | 225207_at | PDK4 |
| | 206208_at | CA4 |
| | 207080_s_at | PYY |
| | 215118_s_at | IGHA1 |
| | 204083_s_at | TPM2 |
| | 229070_at | C60rf105 |
| | 211548_s_at | HPGD |
| | 206262_at | ADH1C |
| 4 | 210107_at | CLCA1 |
| | 205892_s_at | FABP1 |
| | 212592_at | ENAM |
| | 205382_s_at | CFD |
| | 207502_at | GUCA2B |
| | 202995_s_at | FBLN1 |
| | 206149_at | LOC63928 |
| | 204719_at | ABCA8 |
| | 210809_s_at | POSTN |
| | 201893_x_at | DCN |
| | 223597_at | ITLN1 |
| | 209156_s_at | COL6A2 |
| | 203240_at | FCGBP |
| | 224963_at | SLC26A2 |
| | 226303_at | PGM5 |
| | 212730_at | DMN |
| | 201141_at | GPNMB |
| | 211959_at | IGFBP5 |
| | 205200_at | CLEC3B |
| | 242601_at | LOC253012 |
| | 213068_at | DPT |
| | 208383_s_at | PCK1 |
| | 203951_at | CNN1 |
| 3 | 204818_at | HSD17B2 |
| | 219014_at | PLAC8 |
| | 209656_s_at | TMEM47 |
| | 222722_at | OGN |
| | 201617_x_at | CALD1 |
| | 202274_at | ACTG2 |
| | 218756_s_at | MGC4172 |
| | 210302_s_at | MAB21L2 |
| | 228885_at | RPL24 |
| | 209735_at | ABCG2 |
| | 228504_at | CCDC80 |
| | 225242_s_at | UGT1A1 |
| | 215125_s_at | MRC1 |
| | 204438_at | HSD11B2 |
| | 204130_at | ANPEP |
| | 202888_s_at | MATN2 |
| | 202350_s_at | PRNP |
| | 201300_s_at | ABI3BP |
| | 223395_at | HLA-C |
| | 214768_x_at | NDE1 |
| | 228133_s_at | SRPX |
| | 204955_at | WWTR1 |
| | 202133_at | HMGCS2 |
| | 204607_at | LOC646627 |
| | 238143_at | KRT20 |
| | 213953_at | KLF4 |
| | 220266_s_at | FHL1 |
| | 210299_s_at | ARL14 |
| | 220468_at | LUM |
| | 201744_s_at | SORBS1 |
| | 218087_s_at | METTL7A |
| | 207761_s_at | FAM129A |
| | 217967_s_at | SCARA5 |
| | 229839_at | SI |
| | 206664_at | ACTA2 |
| | 200974_at | CD177 |
| | 219669_at | C10orf99 |
| | 227736_at | COL15A1 |
| | 203477_at | NR3C2 |
| | 205259_at | |

There is therefore more particularly provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   220026_at; and/or
**(ii)** CLCA4
in a biological sample from said individual wherein a lower level of expression of the gene or transcripts of group (i) and/or group (ii) relative to control levels is indicative of a neoplastic large intestine cell or a cell predisposed to the onset of a neoplastic state.

Preferably, said control level is a non-neoplastic level.

In another embodiment, there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   214142_at; and/or
**(ii)** ZG16
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet another embodiment there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:

| | |
|---|---|
| 209301_at | 205950_s_at; |

and/or
**(ii)**

| | |
|---|---|
| CA2 | CA1 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still yet another preferred embodiment, there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   220834_at; and/or
**(ii)** MS4A12
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet still another preferred embodiment, there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   206784_at; and/or
**(ii)** AQP8
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In a further embodiment, there is provided a method of screening for the onset or predisposition to the onset of a large intestine neoplasm in an individual, said method comprising assessing the level of expression of one or more genes or transcripts selected from:
**(i) (i)** the gene, genes or transcripts detected by Affymetrix probeset IDs:
   203908_at, 206198_s_at, 205547_s_at, 207003_at, 206422at, 209613_s_at, 207245_at; and/or
**(ii)** SLC4A4, CEACAM7, TAGLN, GUCA1B, GCG, ADH1B, UGT2B17
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In another further embodiment, there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs:
   206134_at, 217546_at, 206561_s_at, 211719_x_at, 202291_s_at, 209687_at; and/or
**(ii)** ADAMDEC1, MT1M, AKR1B10, FN1, MGP, CXCL12
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still another further embodiment, there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | |
|---|---|---|
| 225207_at | 211548_s_at | 205382_s_at |
| 206208_at | 206262_at | 207502_at |
| 207080_s_at | 210107_at | 202995_s_at |
| 215118_s_at | 205892_s_at | 206149_at |
| 204083_s_at | 212592_at | 204719_at |
| 229070_at; and/or | | |

**(ii)**

| | | |
|---|---|---|
| PDK4 | HPGD | CFD |
| CA4 | ADH1C | GUCA2B |
| PYY | CLCA1 | FBLN1 |
| IGHA1 | FABP1 | LOC63928 |
| TPM2 | ENAM | ABCA8 |
| C6orf105 | | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet still yet another further embodiment, there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | |
|---|---|---|
| 210809_s_at | 201617_x_at | 202133_at |
| 201893_x_at | 202274_at | 204607_at |
| 223597_at | 218756_s_at | 238143_at |
| 209156_s_at | 210302_s_at | 213953_at |
| 203240_at | 228885_at | 220266_s_at |
| 224963_at | 209735_at | 210299_s_at |
| 226303_at | 228504_at | 220468_at |
| 212730_at | 225242_s_at | 201744_s_at |
| 201141_at | 215125_s_at | 218087_s_at |
| 211959_at | 204438_at | 207761_s_at |
| 205200_at | 204130_at | 217967_s_at |
| 242601_at | 202888_s_at | 229839_at |
| 213068_at | 202350_s_at | 206664_at |
| 208383_s_at | 201300_s_at | 200974_at |
| 203951_at | 223395_at | 219669_at |
| 204818_at | 214768_x_at | 227736_at |
| 219014_at | 228133_s_at | 203477_at |
| 209656_s_at | 204955_at | 205259_at |
| 222722_at; and/or | | |

**(ii)**

| | | |
|---|---|---|
| POSTN | OGN | WWTR1 |
| DCN | CALD1 | HMGCS2 |
| ITLN1 | ACTG2 | LOC646627 |
| COL6A2 | MGC4172 | KRT20 |
| FCGBP | MAB21L2 | KLF4 |
| SLC26A2 | RPL24 | FHL1 |
| PGM5 | ABCG2 | ARL14 |
| DMN | CCDC80 | LUM |
| GPNMB | UGT1A1 | SORBS1 |
| IGFBP5 | MRC1 | METTL7A |
| CLEC3B | HSD11B2 | FAM129A |
| LOC253012 | ANPEP | SCARA5 |
| DPT | MATN2 | SI |
| PCK1 | PRNP | ACTA2 |
| CNN1 | ABI3BP | CD177 |
| HSD17B2 | HLA-C | C10orf99 |
| PLAC8 | NDE1 | COL15A1 |
| TMEM47 | SRPX | NR3C2 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

According to these aspects of the present disclosure said large intestine tissue is preferably colorectal tissue.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

The detection method of the present invention can be performed on any suitable biological sample. To this end, reference to a "biological sample" should be understood as a reference to any sample of biological material derived from an animal such as, but not limited to, cellular material, biofluids (eg. blood), faeces, tissue specimens (such as biopsy specimens), surgical specimens or fluid which has been introduced into the body of an animal and subsequently removed (such as, for example, the solution retrieved from an enema wash). The biological sample which is tested according to the method of the present invention may be tested directly or may require some form of treatment prior to testing. For example, a biopsy or surgical sample may require homogenisation prior to testing or it may require sectioning for *in situ* testing of the qualitative expression levels of individual genes. Alternatively, a cell sample may require permeabilisation prior to testing. Further, to the extent that the biological sample is not in liquid form, (if such form is required for testing) it may require the addition of a reagent, such as a buffer, to mobilise the sample.

To the extent that the neoplastic marker gene expression product is present in a biological sample, the biological sample may be directly tested or else all or some of the nucleic acid material present in the biological sample may be isolated prior to testing. In yet another example, the sample may be partially purified or otherwise enriched prior to analysis. For example, to the extent that a biological sample comprises a very diverse cell population, it may be desirable to enrich for a sub-population of particular interest. It is within the scope of the present invention for the target cell population or molecules derived therefrom to be pretreated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation. Preferably, said sample is a faecal (stool) sample, enema wash, surgical resection, tissue or blood specimen.

In a related aspect, it has been determined that certain of the markers hereinbefore defined are more indicative of adenoma development versus cancer development or vice versa. This is an extremely valuable finding since it enables one to more specifically characterise the likely nature of a neoplasm which is detected by virtue of the method of the present invention.

Accordingly, in a related aspect the present disclosure is directed to a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 200600_at | 203680_at | 211959_at | 218469_at |
| 200665_s_at | 203729_at | 211964_at | 218559_s_at |
| 200799_at | 203748_x_at | 211985_s_at | 219087_at |
| 200845_s_at | 204069_at | 211990_at | 219607_s_at |
| 200859_x_at | 204122_at | 211991_s_at | 221541_at |
| 200897_s_at | 204135_at | 212077_at | 222043_at |
| 200974_at | 204438_at | 212091_s_at | 222453_at |
| 200986_at | 204457_s_at | 212136_at | 222513_s_at |
| 201041_s_at | 204570_at | 212158_at | 223121_s_at |
| 201061_s_at | 204688_at | 212185_x_at | 223122_s_at |
| 201069_at | 205412_at | 212195_at | 223235_s_at |
| 201105_at | 205683_x_at | 212230_at | 223343_at |
| 201137_s_at | 205935_at | 212233_at | 224560_at |
| 201141_at | 207134_x_at | 212265_at | 224694_at |
| 201150_s_at | 207266_x_at | 212386_at | 224840_at |
| 201289_at | 208131_s_at | 212387_at | 224964_s_at |
| 201300_s_at | 208370_s_at | 212397_at | 225242_s_at |
| 201426_s_at | 208747_s_at | 212414_s_at | 225269_s_at |
| 201438_at | 208788_at | 212419_at | 225353_s_at |
| 201616_s_at | 208789_at | 212464_s_at | 225381_at |
| 201617_x_at | 208894_at | 212667_at | 225442_at |
| 201645_at | 209047_at | 212671_s_at | 225602_at |
| 201667_at | 209101_at | 212713_at | 225604_s_at |
| 201743_at | 209138_x_at | 212764_at | 225626_at |
| 201744_s_at | 209147_s_at | 212956_at | 225688_s_at |
| 201842_s_at | 209156_s_at | 213428_s_at | 225710_at |
| 201852_x_at | 209191_at | 213509_x_at | 226001_at |
| 201858_s_at | 209209_s_at | 213746_s_at | 226051_at |
| 201859_at | 209210_s_at | 213891_s_at | 226084_at |
| 201865_x_at | 209312_x_at | 214038_at | 226103_at |
| 201893_x_at | 209335_at | 214677_x_at | 226430_at |
| 201920_at | 209436 at | 214752_x_at | 226682_at |
| 202007_at | 209457_at | 215049_x_at | 226694_at |
| 202069_s_at | 209496_at | 215076_s_at | 226818_at |
| 202133_at | 209621_s_at | 215193_x_at | 226834_at |
| 202283_at | 209651_at | 215382_x_at | 226841_at |
| 202291_s_at | 209656_s_at | 215388_s_at | 227061_at |
| 202403_s_at | 209868_s_at | 216442_x_at | 227099_s_at |
| 202620_s_at | 210084_x_at | 216474_x_at | 227235_at |
| 202686_s_at | 210133_at | 216834_at | 227404_s_at |
| 202760_s_at | 210139_s_at | 217480_x_at | 227529_s_at |
| 202766_s_at | 210495_x_at | 217757_at | 227561_at |
| 202953_at | 210517_s_at | 217762_s_at | 227623_at |
| 202957_at | 210764_s_at | 217764_s_at | 227705_at |
| 202994_s_at | 210809_s_at | 217767_at | 227727_at |
| 202995_s_at | 210982_s_at | 217897_at | 228507_at |
| 203066_at | 211161_s_at | 218162_at | 228750_at |
| 203131_at | 211596_s_at | 218224_at | 228846_at |
| 203305_at | 211671_s_at | 218312_s_at | 229530_at |
| 203382_s_at | 211719_x_at | 218353_at | 230264_s_at |
| 203477_at | 211813_x_at | 218418_s_at | 231579_s_at |
| 203645_s_at | 211896_s_at | 218468_s_at | 234987_at; |

and/or
**(ii)**

| | | | |
|---|---|---|---|
| A2M | CYBRD1 | LGALS1 | PRKAR2B |
| ACAT1 | CYR61 | LOC283666 | PRNP |
| ACTA2 | DCN | LOC339562 | PTGIS |
| AKAP12 | DDR2 | LOC387763 | PTRF |
| ANKRD25 | DSCR1 | LOC399959 | QKI |
| ANTXR1 | DUSP1 | LRIG1 | RAB31 |
| AP1S2 | DUSP5 | LUM | RARRES2 |
| APOE | EFEMP1 | MAFB | RBMS1 |
| AQP1 | EGR1 | MAP1B | RDX |
| ASPN | ELOVL5 | MEIS1 | RELL1 |
| ATP2B4 | EMP3 | MFAP4 | RGS1 |
| AXL | F13A1 | MGP | RGS5 |
| C10orf56 | FBLN1 | MMP2 | SDC2 |
| C1QB | FBN1 | MPEG1 | SELM |
| C1QC | FILIP1L | MRC1 | SEPT6 |
| C1S | FKBP5 | MRGPRF | SERPINF1 |
| C20orf118 | FLNA | MS4A4A | SERPING1 |
| C3 | FN1 | MS4A7 | SFRP2 |
| C9orf19 | FOXF1 | MSN | SGCE |
| CALD1 | FXYD6 | MT2A | SLC20A1 |
| CALM1 | GALNAC4S-6ST | MXD1 | SMOC2 |
| CCDC80 | GAS1 | NEXN | SORBS1 |
| CCL11 | GJA1 | NIDI | SPARC |
| CCL8 | GNG2 | NR3C1 | SPON1 |
| CD14 | GPNMB | OLFML3 | SRGN |
| CD163 | GREM1 | PAG1 | STOM |
| CES2 | GUCY1A3 | PALLD | TBC1D9 |
| CFHR1 | HCLS1 | PALM2- | TCEAL7 |
| CLU | HLA-DPA1 | AKAP2 | TGFB1I1 |
| COL14A1 | HLA-DPB1 | PDGFRA | TIMP2 |
| COL15A1 | HLA-DQA1 | PDLIM3 | TIMP3 |
| COL1A2 | HLA-DRA | PHLDB2 | TMEM47 |
| COL3A1 | HLA-DRB1 | PLEKHC1 | TNC |
| COL4A2 | HSPA1A | PLOD2 | TPSAB1 |
| COL6A1 | IDH3A | PMP22 | TPSB2 |
| COL6A2 | IGFBP5 | PNMA1 | TUBB6 |
| COL6A3 | IGL@ | POSTN | TYROBP |
| COX7A1 | IGLJ3 | PPAP2A | VIM |
| CRISPLD2 | IL6ST | PPAP2B | WWTR1 |
| CTGF | KLHL5 | PRDX6 | ZSCAN18 |

in a biological sample from said individual wherein a lower level of expression of the genes or transcripts of group (i) and/or group (ii) relative to control levels is indicative of an adenoma cell or a cell predisposed to the onset of an adenoma state.

In another preferred embodiment of this aspect of the present disclosure there is provided a method of screening for the onset or predisposition to the onset of a large intestine neoplasm in an individual, said method comprising screening the level of expression of one or more genes or transcripts selected from:
**(i)** the gene or genes detected by Affymetrix probeset IDs:

| | | | |
|---|---|---|---|
| 200884_at | 206000_at | 214598_at | 226147 s at |
| 201495_x_at | 206094_x_at | 214916_x_at | 226302 at |
| 202266_at | 206262_at | 215125_s_at | 226594_at |
| 202350_s_at | 206377_at | 215299_x_at | 226654_at |
| 202731_at | 206385_s_at | 215867_x_at | 226811_at |
| 202741_at | 206664_at | 216336_x_at | 227052 at |
| 202742_s_at | 207126_x_at | 216491_x_at | 227522_at |
| 202768_at | 207245_at | 216510_x_at | 227682_at |
| 202838_at | 207390_s_at | 217022_s_at | 227725_at |
| 203058_s_at | 207392_x_at | 217109_at | 227735_s_at |
| 203060_s_at | 207432_at | 217110_s_at | 227736_at |
| 203240_at | 207761_s_at | 217165_x_at | 228133_s_at |
| 203296_s_at | 208596_s_at | 217232_x_at | 228195_at |
| 203343_at | 208920_at | 217414_x_at | 228232s_s_at |
| 203474_at | 209114_at | 218541_s_at | 228241_at |
| 203638_s_at | 209374_s_at | 218546_at | 228469_at |
| 203963_at | 209458_x_at | 219059_s_at | 228961_at |
| 204018_x_at | 209791_at | 219543_at | 229070_at |
| 204034_at | 210107_at | 219796_s_at | 229254_at |
| 204036_at | 210524_x_at | 219948_x_at | 229659_s_at |
| 204130_at | 210735_s_at | 220075_s_at | 229831_at |
| 204388_s_at | 211372_s_at | 220266_s_at | 230595_at |
| 204389_at | 211538_s_at | 220468_at | 231925_at |
| 204508_s_at | 211549_s_at | 220645_at | 231975_s_at |
| 204532_x_at | 211637_x_at | 220812_s_at | 233565_s_at |
| 204607_at | 211699_x_at | 221004_s_at | 235146_at |
| 204673_at | 211745_x_at | 221305_s_at | 235766_x_at |
| 204818_at | 212224_at | 221584_s_at | 235849_at |
| 204895_x_at | 212592_at | 221841_s_at | 238143_at |
| 204897_at | 212741_at | 221896_s_at | 238750_at |
| 205112_at | 212814_at | 223484_at | 238751_at |
| 205259_at | 213317_at | 223597_at | 239272_at |
| 205403_at | 213451_x_at | 223754_at | 241994_at |
| 205480_s_at | 213629_x_at | 224342_x_at | 242447_at |
| 205554_s_at | 213921_at | 224989_at | 242601_at |
| 205593_s_at | 213953_at | 224990_at | 243278_at; |
| 205892_s_at | 214164_x_at | 225458_at | and/or |
| 205929_at | 214433_s_at | 225728_at | |

**(ii)**

| | | | |
|---|---|---|---|
| ADH1C | FGFR2 | LOC646627 | PTGER4 |
| AGR3 | FOSB | LOC652128 | RAB27A |
| ALDH1A1 | FOXF2 | LOC96610 | SCARA5 |
| ANK3 | FOXP2 | MAOA | SDCBP2 |
| ARL14 | FUCA1 | MATN2 | SELENBP1 |
| ATP1A2 | GPA33 | MEP1A | SI |
| ATP8B1 | HBA1 | METTL7A | SMTN |
| BEST2 | HBA2 | MFSD4 | SORBS2 |
| C10orf99 | HBB | MGC13057 | SRI |
| C15orf48 | HHLA2 | MIER3 | SST |
| C1orf115 | HIGD1A | MMP28 | ST6GALNAC1 |
| C4orf34 | HMGCS2 | MT1A | SULT1A1 |
| C6orf105 | HPGD | MT1F | TNXB |
| C8orf4 | HSD11B2 | MT1M | TSPAN1 |
| CA12 | HSD17B2 | MUC12 | TTRAP |
| CCL28 | HSPA2 | MUC2 | UGDH |
| CKB | IGHA1 | MUC4 | UGP2 |
| CLCA1 | IGHM | MUCDHL | UGT1A1 |
| CLDN8 | IL1R2 | MYH11 | UGT1A3 |
| CLIC5 | IL8 | NDE1 | UGT1A6 |
| CMBL | IQGAP2 | NR3C2 | UGT1A8 |
| CNTN3 | ITLN1 | P2RY1 | UGT1A9 |
| DNASE1L3 | ITM2C | PADI2 | UGT2A3 |
| EDG2 | KCNMA1 | PAPSS2 | UGT2B 15 |
| ENAM | KIAA0828 | PBLD | UGT2B17 |
| ENTPD5 | KLF4 | PDCD4 | VSIG2 |
| ETHE1 | KRT20 | PDE9A | XDH |
| FABP1 | LOC253012 | PIGR | XLKD1 |
| FAM46C | LOC25845 | PLCE1 | ZCWPW2 |
| FAM55D | LOC285382 | PPID | |
| FCGBP | LOC572558 | PRKACB | |

in a biological sample from said individual wherein a lower level of expression of the genes or transcripts of group (i) and/or group (ii) relative to control levels is indicative of a cancer cell or a cell predisposed to the onset of a cancerous state.

According to these aspects, said control levels are preferably non-neoplastic levels and said large intestine tissue is colorectal tissue. Even more preferably, said biological sample is a stool sample or blood sample.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

In a related aspect, it has been determined that a subpopulation of the markers of the present disclosure are not only expressed at levels lower than normal levels, their expression pattern is uniquely characterised by the fact that expression levels above that of background control levels are not detectable in neoplastic tissue. This determination has therefore enabled the development of qualitative screening systems which are simply designed to detect marker expression relative to a control background level. In accordance with this aspect of the present invention, said "control level" is therefore the "background level".

According to this aspect, there is therefore provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | | |
|---|---|---|---|
| 202920_at | 209613_s_at | 228504_at | 214598_at |
| 203881_s_at | 220037_s_at | 228706_s_at | 219948_x_at |
| 204719_at | 220376_at | 228766_at | 220812_s_at |
| 204931_at | 222717_at | 228854_at | 221305 s_at |
| 204940_at | 224412_s_at | 228885_at | 229831_at |
| 205433_at | 225381_at | 230788_at | 231925_at |
| 206637_at | 225575_at | 231120_x_at | 235146_at |
| 207080_s_at | 227529_s_at | 231773_at | 238751_at |
| 207980_s_at | 227623_at | 203296_s_at | 243278_at; |
| 209170_s_at | 227705_at | 206664_at | |
| 209209_s_at | 227827_at | 211549_s_at | |

and/or
**(ii)**

| | | | |
|---|---|---|---|
| ADH1B | CD36 | P2RY1 | PLEKHC1 |
| SORBS2 | BCHE | ANK2 | LRRC19 |
| PYY | TCEAL7 | XLKD1 | LIFR |
| ABCA8 | ANGPTL1 | LOC399959 | ATP1A2 |
| RPL24 | DMD | AKAP12 | HPGD |
| SI | GCNT2 | UGT2A3 | GPM6B |
| CLDN8 | SDPR | HHLA2 | UGT1A8 |
| P2RY14 | PKIB | SORBS2 | FOXP2 |
| PLN | CITED2 | CLDN23 | |
| TRPM6 | TCF21 | CNTN3 | |

in a biological sample from said individual wherein a level of expression of the genes or transcripts of group (i) and/or group (ii) which is not substantially above background levels is indicative of a neoplastic cell or a cell predisposed to the onset of a neoplastic state.

In a most preferred embodiment, said genes or transcripts are selected from:
**(i)** the gene, genes or transcripts detected by Affymetrix probeset IDs:
   209613_s_at, 227827_at, 204719_at, 228504_at, 228885_at, 206664_at, 207080_s_at; and/or
**(ii)** ADH1B, SORBS2, PYY, ABCA8, RPL24, SI

Preferably, said neoplasm is an adenoma or an adenocarcinoma and said gastrointestinal tissue is colorectal tissue.

In yet another embodiment, it has been determined that a further subpopulation of these markers are more characteristic of adenoma development, while others are more characteristic of cancer development. Accordingly, there is provided a convenient means of qualitatively obtaining indicative information in relation to the characteristics of the subject neoplasm.

According to this embodiment there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:
   209209_s_at, 225381_at, 227529_s_at, 227623_at, 227705_at; and/or
**(ii)** AKAP12, LOC399959, PLEKHC1, TCEAL7
in a biological sample from said individual wherein a lower level of expression of the genes or transcripts of group (i) and/or group (ii) which is not substantially above background levels is indicative of an adenoma cell or a cell predisposed to the onset of an adenoma state.

In yet still another preferred embodiment there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs:

| | | |
|---|---|---|
| 203296_s_at | 219948_x_at | 231925_at |
| 206664_at | 220812_s_at | 235146_at |
| 211549_s_at | 221305_s_at | 238751_at |
| 214598_at | 229831_at | 243278_at; |

and/or
**(ii)**

| | | |
|---|---|---|
| ATP1A2 | HHLA2 | SORBS2 |
| CLDN8 | HPGD | UGT1A8 |
| CNTN3 | P2RY1 | UGT2A3 |
| FOXP2 | SI | |

in a biological sample from said individual wherein a lower level of expression of the genes or transcripts of group (i) and/or group (ii) which is not substantially above background levels is indicative of a cancer cell or a cell predisposed to the onset of a cancerous state.

Preferably, said large intestine tissue is colorectal tissue.

More preferably, said biological sample is a stool sample or a blood sample.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

As detailed hereinbefore, the present invention is designed to screen for a neoplastic cell or cellular population, which is located in the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to "expression" should be understood as a reference to the transcription and/or translation of a nucleic acid molecule. In this regard, the present invention is exemplified with respect to screening for neoplastic marker expression products taking the form of RNA transcripts (eg primary RNA or mRNA). Reference to "RNA" should be understood to encompass reference to any form of RNA, such as primary RNA or mRNA. Without limiting the present invention in any way, the modulation of gene transcription leading to increased or decreased RNA synthesis will also correlate with the translation of some of these RNA transcripts (such as mRNA) to produce a protein product.

Accordingly, the present invention also extends to detection methodology which is directed to screening for modulated levels or patterns of the neoplastic marker protein products as an indicator of the neoplastic state of a cell or cellular population. Although one method is to screen for mRNA transcripts and/or the corresponding protein product, it should be understood that the present invention is not limited in this regard and extends to screening for any other form of neoplastic marker expression product such as, for example, a primary RNA transcript.

In terms of screening for the downregulation of expression of a marker it would also be well known to the person of skill in the art that changes which are detectable at the DNA level are indicative of changes to gene expression activity and therefore changes to expression product levels. Such changes include but are not limited to, changes to DNA methylation. Accordingly, reference herein to "screening the level of expression" and comparison of these "levels of expression" to control "levels of expression" should be understood as a reference to assessing DNA factors which are related to transcription, such as gene/DNA methylation patterns.

It would also be known to a person skilled in the art that changes in the structure of chromatin are indicative of changes in gene expression. Silencing of gene expression is often associated with modification of chromatin proteins, methylation of lysines at either or both positions 9 and 27 of histone H3 being well studied examples, while active chromatin is marked by acetylation of lysine 9 of histone H3. Thus association of gene sequences with chromatin carrying repressive or active modifications can be used to make an assessment of the expression level of a gene.

It is well within the skill of the person of skill in the art to determine the most appropriate screening method for any given situation. To this end, the genes which are known to encode an expression product which is either secreted by the cell or membrane bound is detailed in the table below. It would be appreciated that screening for neoplastic markers which are secreted or membrane bound may provide particular advantages in terms of the design of a diagnostic screening product.

| The gene or genes detected by Affymetrix probe Nos: | | | |
|---|---|---|---|
| 200600_at | 205593_s_at | 212185_x_at | 224480_s_at |
| 200845_s_at | 205765_at | 212192_at | 224663_s_at |
| 200859_x_at | 205892_s_at | 212224_at | 224694_at |
| 200884_at | 205927_s_at | 212230_at | 224823_at |
| 200897_s_at | 205929_at | 212233_at | 224836_at |
| 200974_at | 205935_at | 212265_at | 224840_at |
| 201041_s_at | 205935_at | 212288_at | 224959_at |
| 201058_s_at | 205950_s_at | 212386_at | 224963_at |
| 201061_s_at | 206000_at | 212387_at | 224964_s_at |
| 201069_at | 206094_x_at | 212397_at | 224989_at |
| 201105_at | 206143_at | 212414_s_at | 224990_at |
| 201137_s_at | 206149_at | 212419_at | 225207_at |
| 201300_s_at | 206198_s_at | 212671_s_at | 225242_s_at |
| 201324_at | 206199_at | 212730_at | 225269_s_at |
| 201426_s_at | 206208_at | 212741_at | 225381at |
| 201539_s_at | 206209_s_at | 212764_at | _ 225442_at |
| 201540_at | 206262_at | 212814_at | 225458_at |
| 201616_s_at | 206377_at | 212859_x_at | 225575_at |
| 201617_x_at | 206385_s_at | 212956_at | 225602_at |
| 201667_at | 206461_x_at | 213106_at | 225604_s_at |
| 201739_at | 206561_s_at | 213317_at | 225626_at |
| 201743_at | 206576_s_at | 213509_x_at | 225710_at |
| 201865_x_at | 206637_at | 213629_x_at | 225720_at |
| 201920_at | 206664_at | 213746_s_at | 225721_at |
| 201957_at | 206710_s_at | 213891_s_at | 225782_at |
| 202007_at | 206784_at | 213953_at | 225894_at |
| 202069_s_at | 207126_x_at | 214027_x_at | 225895_at |
| 202133_at | 207245_at | 214234_s_at | 226001_at |
| 202242_at | 207266_x_at | 214235_at | 226051_at |
| 202266_at | 207390_s_at | 214414_x_at | 226084_at |
| 202274_at | 207392_x_at | 214433_s_at | 226103_at |
| 202388_at | 207432_at | 214505_s_at | 226147_s_at |
| 202555_s_at | 207761_s_at | 214598_at | 226248_s_at |
| 202620_s_at | 207980_s_at | 214677_x_at | 226302_at |
| 202686_s_at | 208063_s_at | 214696_at | 226303_at |
| 202731_at | 208131_s_at | 214752_x_at | 226304_at |
| 202741_at | 208370_s_at | 214768_x_at | 226333_at |
| 202742_s_at | 208383_s_at | 214777_at | 226430_at |
| 202746_at | 208450_at | 215049_x_at | 226594_at |
| 202760_s_at | 208581_x_at | 215118_s_at | 226654_at |
| 202768_at | 208596_s_at | 215125_s_at | 226682_at |
| 202888_s_at | 208763_s_at | 215176_x_at | 226694_at |
| 202920_at | 208788_at | 215193_x_at | 226811_at |
| 202957_at | 208789_at | 215299_x_at | 226818_at |
| 202992_at | 208920_at | 215657_at | 226834_at |
| 202994_s_at | 208937_s_at | 216207_x_at | 226841_at |
| 202995_s_at | 209047_at | 216336_x_at | 227006_at |
| 203000_at | 209074_s_at | 216401_x_at | 227052_at |
| 203001_s_at | 209114_at | 216491_x_at | 227061_at |
| 203058_s_at | 209116_x_at | 216576_x_at | 227099_s_at |
| 203060_s_at | 209138_x_at | 216834_at | 227235_at |
| 203066_at | 209147_s_at | 216984_x_at | 227404_s_at |
| 203131_at | 209156_s_at | 217022_s_at | 227522_at |
| 203240_at | 209167_at | 217148_x_at | 227529_s_at |
| 203305_at | 209170_s_at | 217165_x_at | 227561_at |
| 203343_at | 209191_at | 217232_x_at | 227623_at |
| 203382_s_at | 209209_s_at | 217235_x_at | 227662_at |
| 203474_at | 209210_s_at | 217378_x_at | 227682_at |
| 203638_s_at | 209283_at | 217414_x_at | 227705_at |
| 203645_s_at | 209301_at | 217480_x_at | 227719_at |
| 203680_at | 209312_x_at | 217546_at | 227725_at |
| 203729_at | 209357_at | 217762_s_at | 227727_at |
| 203748_x_at | 209373_at | 217764_s_at | 227735_s_at |
| 203766_s_at | 209374_s_at | 217897_at | 227736_at |
| 203908_at | 209457_at | 217967_s_at | 228202_at |
| 203913_s_at | 209458_x_at | 218087_s_at | 228232_s_at |
| 203914_x_at | 209498_at | 218211_s_at | 228469_at |
| 203951_at | 209612_s_at | 218224_at | 228504_at |
| 203980_at | 209613_s_at | 218312_s_at | 228507_at |
| 204018_x_at | 209621_s_at | 218353_at | 228640_at |
| 204034_at | 209651_at | 218418_s_at | 228766_at |
| 204036_at | 209656_s_at | 218546_at | 228846_at |
| 204069_at | 209667_at | 218559_s_at | 228854_at |
| 204083_s_at | 209668_x_at | 219014_at | 228961_at |
| 204122_at | 209868_s_at | 219059_s_at | 229070_at |
| 204130_at | 209948_at | 219508_at | 229254_at |
| 204135_at | 2t0107_at | 219543_at | 229530_at |
| 204326_x_at | 210139_s_at | 219607_s_at | 229659_s_at |
| 204388_s_at | 210298_x_at | 219796_s_at | 229831_at |
| 204389_at | 210299_s_at | 219948_x_at | 229839_at |
| 204438_at | 210302_s_at | 219955_at | 230087_at |
| 204457_s_at | 210517_s_at | 220026_at | 230264_s_at |
| 204532_x_at | 210524_x_at | 220037_s_at | 230595_at |
| 204570_at | 210524_x_at | 220075_s_at | 230788_at |
| 204607_at | 210946_at | 220266_s_at | 230830_at |
| 204688_at | 211372_s_at | 220376_at | 231120_x_at |
| 204697_s_at | 211538_s_at | 220468_at | 231832_at |
| 204719_at | 211548_s_at | 220812_s_at | 231925_at |
| 204745_x_at | 211549_s_at | 220834_at | 231975_s_at |
| 204818_at | 211596_s_at | 221004_s_at | 232176_at |
| 204894_s_at | 211637_x_at | 221305_s_at | 232481_s_at |
| 204897_at | 211643_x_at | 221667_s_at | 233565_s_at |
| 204931_at | 211645_x_at | 221747_at | 234987_at |
| 204938_s_at | 211671_s_at | 221748_s_at | 235146_at |
| 204939_s_at | 211696_x_at | 221841_s_at | 235766_x_at |
| 204940_at | 211699_x_at | 221874_at | 235849_at |
| 204955_at | 211745_x_at | 221896_s_at | 235976_at |
| 205097_at | 211798_x_at | 222513_s_at | 236300_at |
| 205112_at | 211848_s_at | 222717_at | 236313_at |
| 205259_at | 211889_x_at | 223235_s_at | 236894_at |
| 205267_at | 211964_at | 223343_at | 237521_x_at |
| 205403_at | 211985_s_at | 223395_at | 238750_at |
| 205412_at | 211990_at | 223484_at | 241994_at |
| 205433_at | 211991_s_at | 223551_at | 242317_at |
| 205464_at | 212077_at | 223597_at | 242447_at |
| 205480_s_at | 212097_at | 223623_at | 242601_at |
| 205547_s_at | 212136_at | 224352_s_at | 243278_at |
| 205554_s_at | 212158_at | 224412_s_at | |
| ABCA8 | EGR1 | LOC25845 | PPID |
| ABI3BP | ELOVL5 | LOC283666 | PPP1R12B |
| ACAT1 | EMP1 | LOC285382 | PPP1R14A |
| ACTA2 | EMP3 | LOC339562 | PRDX6 |
| ACTG2 | ENTPD5 | LOC387763 | PRIMA1 |
| ADH1B | EPB41L3 | LOC399959 | PRKACB |
| ADH1C | ETHE1 | LOC572558 | PRKAR2B |
| AKAP12 | F13A1 | LOC63928 | PRNP |
| AKR1B10 | FABP1 | LOC652128 | PTGER4 |
| ALDH1A1 | FABP4 | LOC652745 | PTGIS |
| ANK2 | FAM107A | LRIG1 | PTRF |
| ANK3 | FAM129A | LRRC19 | QKI |
| ANKRD25 | FAM46C | MAB21L2 | RAB27A |
| ANPEP | FBLN1 | MAFB | RAB31 |
| ANTXR1 | FCGBP | MAG1 | RBMS1 |
| AOC3 | FGFR2 | MALL | RDX |
| AP1S2 | FHL1 | MAOA | RELL1 |
| APOE | FILIP1L | MAP1B | RGS1 |
| AQP1 | FKBP5 | MEIS1 | RGS2 |
| AQP8 | FLNA | MEP1A | RGS5 |
| ARL14 | FNBP1 | METTL7A | SCARA5 |
| ATP2B4 | FOSB | MFSD4 | SCNN1B |
| ATP8A1 | FOXF1 | MGC14376 | SDC2 |
| AXL | FOXF2 | MIER3 | SDCBP2 |
| BCHE | FOXP2 | MLPH | SDPR |
| BEST2 | FXYD6 | MMP2 | SELENBP1 |
| C10orf56 | GALNAC4S- | MPEG1 | SELM |
| C10orf99 | 6ST | MPEG1 | SGCE |
| C15orf48 | GAS1 | MRC1 | SGK |
| Clorf115 | GCNT2 | MRGPRF | SI |
| C20orf118 | GCNT3 | MS4A12 | SLC20A1 |
| C2orf40 | GJA1 | MS4A4A | SLC26A2 |
| C4orf34 | GNG2 | MS4A7 | SLC26A3 |
| C6orf105 | GPA33 | MSN | SLC4A4 |
| C6orf204 | GPM6B | MSRB3 | SLITRK6 |
| C7 | GUCY1A3 | MT1A | SMOC2 |
| C9orf19 | HBA1 | MT1E | SMTN |
| CA1 | HBA2 | MT1F | SORBS 1 |
| CA2 | HBB | MT1G | SRI |
| CA4 | HCLS1 | MT1H | SRPX |
| CALD1 | HHLA2 | MT1M | ST6GALNAC1 |
| CALM1 | HIGD1A | MT1X | STMN2 |
| CAPN9 | HLA-C | MT2A | STOM |
| CAV1 | HLA-DPA1 | MUC12 | SULT1A1 |
| CCDC80 | HLA-DPB1 | MUCDHL | SYNPO2 |
| CCL28 | HLA-DQA1 | MXD1 | TAGLN |
| CD14 | HLA-DRB1 | MYL9 | TBC1D9 |
| CD163 | HMGCS2 | MYLK | TCEAL7 |
| CD36 | HPGD | NEXN | TCF21 |
| CDKN2B | HSD11B2 | NIDI | TGFB1I1 |
| CEACAM1 | HSD17B2 | NR3C1 | TMEM47 |
| CEACAM7 | HSPA2 | NR3C2 | TNS1 |
| CES2 | HSPB6 | OSTbeta | TP53INP2 |
| CFL2 | HSPB8 | P2RY1 | TPM2 |
| CHGA | ID1 | P2RY14 | TRPM6 |
| CITED2 | IDH3A | PAG1 | TSC22D3 |
| CKB | IGHA1 | PALLD | TSPAN1 |
| CLCA1 | IGHM | PALM2- | TSPAN7 |
| CLCA4 | IGKV1D-13 | AKAP2 | TTRAP |
| CLDN8 | IGL@ | PAPSS2 | TUBB6 |
| CLIC5 | IGLJ3 | PBLD | TYROBP |
| CMBL | IL1R2 | PCK1 | UGDH |
| CNN1 | IQGAP2 | PDCD4 | UGP2 |
| CNTN3 | ITLN1 | PDE9A | UGT1A1 |
| COL4A2 | ITM2A | PDGFRA | UGT1A3 |
| COL6A2 | ITM2C | PDK4 | UGT1A6 |
| COX7A1 | KCNMA1 | PDLIM3 | UGT1A8 |
| CRYAB | KCNMB1 | PGM5 | UGT1A9 |
| CTSE | KCTD12 | PIGR | UGT2A3 |
| CYP3A5 | KIAA0828 | PKIB | UGT2B15 |
| CYP3A5P2 | KIAA1324 | PLAC8 | UGT2B17 |
| DDR2 | KLF4 | PLCE1 | VIM |
| DES | KLHL5 | PLEKHC1 | VSIG2 |
| DMN | KRT20 | PLN | WDR51B |
| DNASE1L3 | L1TD1 | PLOD2 | WWTR1 |
| DSCR1 | LGALS1 | PMP22 | XDH |
| DUSP1 | LGALS2 | PNMA1 | XLKD1 |
| DUSP5 | LIFR | POU2AF1 | ZSCAN18 |
| EDG2 | LMOD1 | PPAP2A | LOC253012 |
| PPAP2B | | | |

Reference to "nucleic acid molecule" should be understood as a reference to both deoxyribonucleic acid molecules and ribonucleic acid molecules and fragments thereof. The present invention therefore extends to both directly screening for mRNA levels in a biological sample or screening for the complementary cDNA which has been reverse-transcribed from an mRNA population of interest. It is well within the skill of the person of skill in the art to design methodology directed to screening for either DNA or RNA. As detailed above, the method of the present invention also extends to screening for the protein product translated from the subject mRNA or the genomic DNA itself.

In one preferred embodiment, the level of gene expression is measured by reference to genes which encode a protein product and, more particularly, said level of expression is measured at the protein level. Accordingly, to the extent that the present disclosure is directed to screening for markers which are detailed in the preceding table, said screening is preferably directed to the encoded protein.
In another particularly preferred embodiment, said gene expression is assessed by analysing genomic DNA methylation. In another embodiment, expression is assessed by the association of DNA with chromatin proteins carrying repressive modifications, for example, methylation of lysines 9 or 27 of histone H3.

As detailed hereinbefore, it should be understood that although the present invention is exemplified with respect to the detection of expressed nucleic acid molecules (e.g. mRNA), it also encompasses methods of detection based on screening for the protein product of the subject genes. The present invention should also be understood to encompass methods of detection based on identifying both proteins and/or nucleic acid molecules in one or more biological samples. This may be of particular significance to the extent that some of the neoplastic markers of interest may correspond to genes or gene fragments which do not encode a protein product. Accordingly, to the extent that this occurs it would not be possible to test for a protein and the subject marker would have to be assessed on the basis of transcription expression profiles or changes to genomic DNA.

The term "protein" should be understood to encompass peptides, polypeptides and proteins (including protein fragments). The protein may be glycosylated or unglycosylated and/or may contain a range of other molecules fused, linked, bound or otherwise associated to the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

The proteins encoded by the neoplastic markers of the present invention may be in multimeric form meaning that two or more molecules are associated together. Where the same protein molecules are associated together, the complex is a homomultimer. An example of a homomultimer is a homodimer. Where at least one marker protein is associated with at least one non-marker protein, then the complex is a heteromultimer such as a heterodimer.

Reference to a "fragment" should be understood as a reference to a portion of the subject nucleic acid molecule or protein. This is particularly relevant with respect to screening for modulated RNA levels in stool samples since the subject RNA is likely to have been degraded or otherwise fragmented due to the environment of the gut. One may therefore actually be detecting fragments of the subject RNA molecule, which fragments are identified by virtue of the use of a suitably specific probe.

Reference to the "onset" of a neoplasm, such as adenoma or adenocarcinoma, should be understood as a reference to one or more cells of that individual exhibiting dysplasia. In this regard, the adenoma or adenocarcinoma may be well developed in that a mass of dysplastic cells has developed. Alternatively, the adenoma or adenocarcinoma may be at a very early stage in that only relatively few abnormal cell divisions have occurred at the time of diagnosis. The present invention also extends to the assessment of an individual's predisposition to the development of a neoplasm, such as an adenoma or adenocarcinoma. Without limiting the present invention in any way, changed levels of the neoplastic markers may be indicative of that individual's predisposition to developing a neoplasia, such as the future development of an adenoma or adenocarcinoma or another adenoma or adenocarcinoma.

In yet another related aspect of the present disclosure markers have been identified which enable the characterisation of neoplastic tissue of the large intestine in terms of whether it is an adenoma or a cancer. This development now provides a simple yet accurate means of characterising tissue using means other than the traditional methods which are currently utilised.

According to this aspect of the present disclosure there is provided a method of characterising a neoplastic cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | | |
|---|---|---|---|
| 200600_at | 202859_x_at | 210764_s_at | 219087 at |
| 200665_s_at | 202878_s_at | 210809_s_at | 221011_s_at |
| 200832_s_at | 202917_s_at | 211161_s_at | 221729_at |
| 200974_at | 202998_s_at | 211571_s_at | 221730_at |
| 200986_at | 203083_at | 211719_x_at | 221731_x_at |
| 201058_s_at | 203325_s_at | 211813_x_at | 37892_at |
| 201069_at | 203382_s_at | 211896_s_at | 223122_s_at |
| 201105_at | 203477_at | 211959_at | 223235_s_at |
| 201141_at | 203570_at | 211964_at | 224560_at |
| 201147_s_at | 203645_s_at | 211966_at | 224694_at |
| 201150_s_at | 203878_s_at | 211980_at | 224724_at |
| 201162_at | 204006_s_at | 211981_at | 225664_at |
| 201163_s_at | 204051_s_at | 212077_at | 225681_at |
| 201185_at | 204122_at | 212344_at | 225710_at |
| 201261_x_at | 204320_at | 212353_at | 225799_at |
| 201289_at | 204475_at | 212354_at | 226237_at |
| 201426_s_at | 204620_s_at | 212464_s_at | 226311_at |
| 201438_at | 205479_s_at | 212488_at | 226694_at |
| 201616_s_at | 205547_s_at | 212489_at | 226777_at |
| 201645_at | 205828_at | 212667_at | 226930_at |
| 201667_at | 207173_x_at | 213125_at | 227099_s_at |
| 201744_s_at | 207191_s_at | 213428_s_at | 227140_at |
| 201792_at | 208747_s_at | 213524_s_at | 227566_at |
| 201842_s_at | 208782_at | 213869_x_at | 229218_at |
| 201852_x_at | 208788_at | 213905_x_at | 229802_at |
| 201859_at | 208850_s_at | 214247_s_at | 231579_s_at |
| 201893_x_at | 20885_1_s_at | 215049_x_at | 231766_s_at |
| 202237_at | 209101_at | 215076_s_at | 231879_at |
| 202238_s_at | 209156_s_at | 215646_s_at | 232458_at |
| 202283_at | 209218_at | 216442_x_at | 233555_s_at |
| 202291_s_at | 209395_at | 217430_x_at | 234994_at; and/or |
| 202310_s_at | 209396_s_at | 217762_s_at | |
| 202311_s_at | 209596_at | 217763_s_at | |
| 202403_s_at | 209875_s_at | 217764_s_at | |
| 202404_s_at | 209955_s_at | 218468_s_at | |
| 202450_s_at | 210095_s_at | 218469_at | |
| 202620_s_at | 210495_x_at | 218559_s_at | |
| 202766_s_at | 210511_s_at | 218638_s_at | |

**(ii)**

| | | | |
|---|---|---|---|
| COL1A2 | INHBA | FAP | IGFBP3 |
| CTHRC1 | COL6A2 | VIM | SERPINF1 |
| FN1 | ANTXR1 | TIMP2 | ISLR |
| POSTN | GPNMB | SCD | HNT |
| SPP1 | BGN | TIMP3 | COL5A1 |
| MMP1 | TAGLN | AEBP1 | OLFML2B |
| SPARC | COL4A1 | GJA1 | KIAA1913 |
| LUM | RAB31 | NNMT | PALM2-AKAP2 |
| GREM1 | LGALS1 | COL1A1 | SERPING1 |
| IL8 | ELOVL5 | SULF2 | TYROBP |
| IGFBP5 | MGP | COL6A1 | ACTA2 |
| SFRP2 | MMP2 | SPON2 | COL3A1 |
| SULF1 | LOXL2 | CTSK | PLOD2 |
| ASPN | MYL9 | MXRA5 | MMP_11 |
| COL6A3 | DCN | C1S | CD163 |
| COL8A1 | CALD1 | DKK3 | FCGR3B |
| COL12A1 | FBN1 | SRGN | PLAU |
| COL5A2 | MMP3 | LBH | MAFB |
| CDH11 | IGFBP7 | CTGF | LOC541471 |
| THBS2 | FSTL1 | TNC | LOC387763 |
| COL15A1 | COL4A2 | G0S2 | CHI3L1 |
| COL11A1 | VCAN | SQLE | THY1 |
| S100A8 | SMOC2 | EFEMP1 | LOXL1 |
| FNDC1 | HTRA1 | APOE | CD93 |
| SFRP4 | CYR61 | MSN | |

in said cell or cellular population wherein a lower level of expression of the genes of group (i) and/or group (ii) relative to a gastrointestinal cancer control level is indicative of an adenoma cell or a cell predisposed to the onset of an adenoma state.

In another aspect there is provided a method of characterising a neoplastic cell or cellular population, which cell or cellular population is derived from the large intestine of an individual, said method comprising assessing the level of expression of one or more genes or transcripts selected from:
(i) the gene, genes or transcripts detected by Affymetrix probeset IDs:

| | | |
|---|---|---|
| 200884_at | 214234_s_at | 226248_s_at |
| 203240_at | 214235_at | 226302_at |
| 203963_at | 214433_s_at | 227676_at |
| 204508_s_at | 215125_s_at | 227719_at |
| 204607_at | 215867_x_at | 227725_at |
| 204811_s_at | 217109_at | 228232_s_at |
| 204895_x_at | 217110_s_at | 229070_at |
| 204897_at | 218211_s_at | 231832_at |
| 205259_at | 219543_at | 232176_at |
| 205765_at | 219955_at | 232481_s_at |
| 205927_s_at | 221841_s_at | 235976_at |
| 208063_s_at | 221874_at | 236894_at |
| 208937_s_at | 223969_s_at | 237521_x_at |
| 210107_at | 223970_at | 242601_at; and/or |
| 213106_at | | |

**(ii)**

| | | | |
|---|---|---|---|
| CLCA1 | ST6GALNAC1 | UGT1A1 | CYP3A5P2 |
| FCGBP | ID1 | SELENBP1 | CAPN9 |
| HMGCS2 | CYP3A5 | PTGER4 | NR3C2 |
| RETNLB | CTSE | MLPH | PBLD |
| L1TD1 | C6orf105 | KIAA1324 | CA12 |
| SLITRK6 | CKB | ATP8B1 | WDR51B |
| VSIG2 | ATP8A1 | CACNA2D2 | FAM3D |
| LOC253012 | MUC4 | KLF4 | |

in a biological sample from said individual wherein a lower level of expression of the genes or transcripts of group (i) and/or group (ii) relative to a gastrointestinal adenoma control level is indicative of a cancer or a cell predisposed to the onset of a cancerous state.

Preferably, said gastrointestinal tissue is colorectal tissue.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

Reference to an "adenoma control level" or "cancer control level" should be understood as a reference to the level of said gene expression in a population of adenoma or cancer gastrointestinal cells, respectively. As discussed hereinbefore in relation to "normal levels", the subject level may be a discrete level or a range of levels. Accordingly, the definition of "adenoma control level" or "cancer control level" should be understood to have a corresponding definition to "normal level", albeit in the context of the expression of genes by a neoplastic population of large intestine cells.

In terms of this aspect of the present invention, the subject analysis is performed on a population of neoplastic cells. These cells may be derived in any manner, such as sloughed off neoplastic cells which have been collected via an enema wash or from a gastrointestinal sample, such as a stool sample. Alternatively, the subject cells may have been obtained via a biopsy or other surgical technique.

Without limiting this aspect of the disclosure in any way, several of the markers of this aspect of the present disclosure have been determined to be expressed at particularly significant levels below those of neoplastic cells. For example, decreased expression levels of 3 to 9 fold have been observed in respect of the following markers which are indicative of gastrointestinal adenomas, when assessed by the method herein exemplified.

| **Fold Decrease** | **Gene, genes or transcripts detected by Affymetrix Probe No:** | **Gene** |
|---|---|---|
| 9 | 202404_s_at | COL1A2 |
| 8 | 225681_at | CTHRC1 |
| 7 | 212464_s_at | FN1 |
| | 210809_s_at | POSTN |
| 6 | 209875 s at | SPP1 |
| 5 | 221740_at | MMP1 |
| | 204475_at | |
| 4 | 200665_s_at | SPARC |
| | 201744_s_at | LUM |
| | 218468_s_at | GREM1 |
| | 202859_x_at | IL8 |
| | 211959_at | IGFBP5 |
| | 223122_s_at | SFRP2 |
| | 212353_at | SULF1 |
| | 219087_at | ASPN |
| | 201438_at | COL6A3 |
| | 226237_at | COL8A1 |
| | 225664_at | COL12A1 |
| | 221730_at | COL5A2 |
| | 207173_x_at | CDH11 |
| | 203083_at | THBS2 |
| | 203477_at | COL15A1 |
| 3 | 37892_at | COL11A1 |
| | 202917_s_at | S100A8 |
| | 226930_at | FNDC1 |
| | 204051_s_at | SFRP4 |
| | 210511_s_at | INHBA |
| | 209156_s_at | COL6A2 |
| | 224694_at | ANTXR1 |
| | 201141_at | GPNMB |
| | 213905_x_at | BGN |
| | 205547_s_at | TAGLN |

In another example, decreased expression levels of between 3 to 5 fold have been observed in respect of the following markers which are indicative of gastrointestinal cancers, when assessed by the method herein exemplified.

| **Fold Decrease** | **Gene, genes or transcripts detected by Affymetrix Probe No:** | **Gene** |
|---|---|---|
| 5 | 210107_at | CLCA1 |
| 3 | 203240_at | FCGBP |
| | 204607_at | HMGCS2 |
| | 223969_s_at | RETNLB |
| | 219955_at | L1TD1 |
| | 232481_s_at | SLITRK6 |
| | 228232_s_at | VSIG2 |
| | 242601_at | LOC253012 |
| | 227725_at | ST6GALNAC1 |

According to this embodiment, there is therefore provided a method of characterising a neoplastic cell or cellular population, which cell or cellular population is derived from the large intestine of an individual, said method comprising assessing the level of expression of one or more genes or transcripts selected from:
**(i)** the gene or genes detected by Affymetrix probeset IDs:
   202404_s_at, 212464_s_at, 210809_s_a, 225681_at;and/or
**(ii)** COL1A2, CTHRC1, FN1, POSTN
in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In another embodiment, there is provided a method of characterising a neoplastic cell or cellular population, which cell or cellular population is derived from the large intestine of an individual, said method comprising assessing the level of expression of one or more genes selected from:
**(i)** the gene, genes or transcripts detected by Affymetrix probeset IDs:

| | | |
|---|---|---|
| 209875_s_at | 227140_at | 204475_at; |

and/or
**(ii)**

| | |
|---|---|
| SPP1 | MMP1 |

in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

Preferably, said gastrointestinal tissue is colorectal tissue.

Still more preferably, said biological sample is a tissue sample.

In another preferred embodiment the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:

| | | | |
|---|---|---|---|
| 200665_s_at | 212353_at | 203083_at | 210511_s_at |
| 201744_s_at | 219087_at | 203477_at | 209156_s_at |
| 218468_s_at | 226237_at | 37892_at | 201438_at; |
| 202859_x_at | 225664_at | 202917_s_at | and/or |
| 211959_at | 221730_at | 226930_at | |
| 223122_s_at | 207173_x_at | 204051_s_at | |

**(ii)**

| | | | |
|---|---|---|---|
| SPARC | ASPN | COL15A1 | ANTXR1 |
| LUM | COL6A3 | COL11A1 | GPNMB |
| GREM1 | COL8A1 | S100A8 | BGN |
| IL8 | COL12A1 | FNDC1 | TAGLN |
| IGFBP5 | COL5A2 | SFRP4 | |
| SFRP2 | CDH11 | INHBA | |
| SULF1 | THBS2 | COL6A2 | |

in a biological sample from said individual wherein a lower level of expression of the genes or transcripts of group (i) and/or (ii) relative to a gastrointestinal cancer control level is indicative of an adenoma or a cell predisposed to the onset of an adenoma state.

In yet another preferred embodiment the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:
   210107_at; and/or
**(ii)** CLCA1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

In still yet another preferred embodiment the present disclosure is directed to a method of characterising a cell or cellular population, which cell or cellular population is derived from the large intestine of an individual, said method comprising assessing the level of expression of one or more genes or transcripts selected from:
**(i)** the gene, genes or transcripts detected by Affymetrix probeset IDs:
   203240_at, 204607at, 223969_s_at, 219955_at, 232481_s_at, 242601_at, 227725_at, 228232_s_at; and/or
**(ii)** FCGBP, HMGCS2, RETNLB, L1TD1, SLITRK6, VSIG2, LOC253012, ST6GALNAC1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

Preferably, said gastrointestinal tissue is colorectal tissue.

Even more preferably, said biological sample is a tissue sample.

In still another related aspect it has been determined that a subset of the markers of this aspect of the present invention are useful as qualitative markers of neoplastic tissue characterisation in that these markers, if not detectable at levels substantially above background levels in neoplastic tissue are indicative of cancerous tissue.

According to this aspect, the present disclosure provides a method of characterising a neoplastic cell or cellular population, which cell or cellular population is derived from the large intestine of an individual, said method comprising assessing the level of expression of one or more genes or transcripts selected from:
**(i)** the gene or genes detected by Affymetrix probeset IDs:

| | | |
|---|---|---|
| 235976_at | 236894_at | 237521; |

and/or
**(ii)**

| | |
|---|---|
| SLITRK6 | L1TD1 |

in a biological sample from said individual wherein expression of the genes or transcripts of group (i) and/or (ii) at a level which is not substantially greater than background neoplastic tissue levels is indicative of a cancer or a cell predisposed to the onset of a cancerous state.

Preferably, said gastrointestinal tissue is colorectal tissue.

Still more preferably, said biological sample is a tissue sample.

In a most preferred embodiment, the methods of the present disclosure are preferably directed to screening for proteins encoded by the markers of the present disclosure or changes to DNA methylation of genomic DNA. In another embodiment, expression is assessed by the association of DNA with chromatin proteins carrying repressive modifications, for example, methylation of lysines 9 or 27 of histone H3.

Although the preferred method is to detect the expression product or DNA changes of the neoplastic markers for the purpose of diagnosing neoplasia development or predisposition thereto, the detection of converse changes in the levels of said markers may be desired under certain circumstances, for example, to monitor the effectiveness of therapeutic or prophylactic treatment directed to modulating a neoplastic condition, such as adenoma or adenocarcinoma development. For example, where reduced expression of the subject markers indicates that an individual has developed a condition characterised by adenoma or adenocarcinoma development, for example, screening for an increase in the levels of these markers subsequently to the onset of a therapeutic regime may be utilised to indicate reversal or other form of improvement of the subject individual's condition.

The method of the present invention is therefore useful as a one off test or as an on-going monitor of those individuals thought to be at risk of neoplasia development or as a monitor of the effectiveness of therapeutic or prophylactic treatment regimes directed to inhibiting or otherwise slowing neoplasia development. In these situations, mapping the modulation of neoplastic marker expression levels in any one or more classes of biological samples is a valuable indicator of the status of an individual or the effectiveness of a therapeutic or prophylactic regime which is currently in use. Accordingly, the method of the present disclosure should be understood to extend to monitoring for increases or decreases in marker expression levels in an individual relative to their normal level (as hereinbefore defined), background control levels, cancer levels, adenoma levels or relative to one or more earlier marker expression levels determined from a biological sample of said individual.

Means of assessing the subject expressed neoplasm markers in a biological sample can be achieved by any suitable method, which would be well known to the person of skill in the art. To this end, it would be appreciated that to the extent that one is examining either a homogeneous cellular population (such as a tumour biopsy or a cellular population which has been enriched from a heterogeneous starting population) or a tissue section, one may utilise a wide range of techniques such as *in situ* hybridisation, assessment of expression profiles by microassays, immunoassays and the like (hereinafter described in more detail) to detect the absence of or downregulation of the level of expression of one or more markers of interest. However, to the extent that one is screening a heterogenous cellular population or a bodily fluid in which heterogeneous populations of cells are found, such as a blood sample, the absence of or reduction in level of expression of a particular marker may be undetectable due to the inherent expression of the marker by non-neoplastic cells which are present in the sample. That is, a decrease in the level of expression of a subgroup of cells may not be detectable. In this situation, a more appropriate mechanism of detecting a reduction in a neoplastic subpopulation of the expression levels of one or more markers of the present invention is via indirect means, such as the detection of epigenetic changes.

Without limiting the present invention to any one theory or mode of action, during development gene expression is regulated by processes that alter the availability of genes for expression in different cell lineages without any alteration in gene sequence, and these states can be inherited through a cell division - a process called epigenetic inheritance. Epigenetic inheritance is determined by a combination of DNA methylation (modification of cytosine to give 5-methyl cytosine, 5meC) and by modifications of the histone chromosomal proteins that package DNA. Thus methylation of DNA at CpG sites and modifications such as deacetylation of histone H3 on lysine 9, and methylation on lysine 9 or 27 are associated with inactive chromatin, while the converse state of a lack of DNA methylation, acetylation of lysine 9 of histone H3 is associated with open chromatin and active gene expression. In cancer, this epigenetic regulation of gene expression is frequently found to be disrupted (Esteller & Herman, 2000; Jones & Baylin, 2002). Genes such as tumour suppressor or metastasis suppressor genes are often found to be silenced by DNA methylation, while other genes may be hypomethylated and inappropriately expressed. Thus, among genes that show a decrease or loss of expression in cancer, this is often characterised by methylation of the promoter or regulatory region of the gene.

A variety of methods are available for detection of aberrantly methylated DNA of a specific gene, even in the presence of a large excess of normal DNA (Clark 2007). Thus, loss of expression of a gene which can be difficult to detect at the protein or RNA level except by immunohistochemistry can often be detected in tumour samples and in bodily fluids of cancer patients by the presence of hypermethylated DNA of the gene's promoter. Similarly DNA hypomethylation may be used for the detection of certain genes whose expression is elevated in cancer. Epigenetic alterations and chromatin changes in cancer are also evident in the altered association of modified histones with specific genes (Esteller, 2007); for example repressed genes are often found associated with histone H3 that is deacetylated and methylated on lysine 9. The use of antibodies targeted to altered histones allows for the isolation of DNA associated with particular chromatin states and its potential use in cancer diagnosis.

Other methods of detecting changes to gene expression levels, particularly where the subject biological sample is not contaminated with high numbers of non-neoplastic cells, include but are not limited to:
(i) *In vivo* detection.
   Molecular Imaging may be used following administration of imaging probes or reagents capable of disclosing altered expression of the markers in the intestinal tissues.
   Molecular imaging (Moore et al., BBA, 1402:239-249, 1988; Weissleder et al., Nature Medicine 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or endoscopy.
(ii) Detection of downregulation of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002).
(iii) Assessment of expression profiles of RNA, for example by array technologies (Alon et al., Proc. Natl. Acad. Sci. USA: 96, 6745-6750, June 1999).
   A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support. As used herein, a DNA microarray is an array of oligonucleotide probes placed onto a chip or other surfaces used to amplify or clone target polynucleotides. Since the position of each particular group of probes in the array is known, the identities of the target polynucleotides can be determined based on their binding to a particular position in the microarray.
   Recent developments in DNA microarray technology make it possible to conduct a large scale assay of a plurality of target nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells or tissues, often a tissue of interest and a control tissue, is compared and any differences in gene expression among the respective tissues are identified. Such information is useful for the identification of the types of genes expressed in a particular tissue type and diagnosis of conditions based on the expression profile.
   In one example, RNA from the sample of interest is subjected to reverse transcription to obtain labelled cDNA. See U.S. Pat. No. 6,410,229 (Lockhart et al.) The cDNA is then hybridized to oligonucleotides or cDNAs of known sequence arrayed on a chip or other surface in a known order. In another example, the RNA is isolated from a biological sample and hybridised to a chip on which are anchored cDNA probes. The location of the oligonucleotide to which the labelled cDNA hybridizes provides sequence information on the cDNA, while the amount of labelled hybridized RNA or cDNA provides an estimate of the relative representation of the RNA or cDNA of interest. See Schena, et al. Science 270:467-470 (1995). For example, use of a cDNA microarray to analyze gene expression patterns in human cancer is described by DeRisi, et al. (Nature Genetics 14:457-460 (1996)).
   In a preferred embodiment, nucleic acid probes corresponding to the subject nucleic acids are made. The nucleic acid probes attached to the biochip are designed to be substantially complementary to the nucleic acids of the biological sample such that specific hybridization of the target sequence and the probes of the present invention occurs. This complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions.
   A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 15 to about 40 bases being particularly preferred. That is, generally entire genes are rarely used as probes. In some embodiments, much longer nucleic acids can be used, up to hundreds of bases. The probes are sufficiently specific to hybridize to a complementary template sequence under conditions known by those of skill in the art. The number of mismatches between the probe's sequences and their complementary template (target) sequences to which they hybridize during hybridization generally do not exceed 15%, usually do not exceed 10% and preferably do not exceed 5%, as-determined by BLAST (default settings).
   Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.
   In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (e.g., U.S. Pat. No. 5,470,967) and polyamide (i.e., peptide nucleic acids). Peptide nucleic acids are described in Nielsen et al. (1991) Science 254: 1497-1500, U.S. Pat. No. 5,714,331, and Nielsen (1999) Curr. Opin. Biotechnol. 10:71-75.
   In certain embodiments, the probe can be a chimeric molecule; i.e., can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind, in a sequence-specific fashion, with its target sequence. A large number of structural modifications, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis is disclosed in U.S. Pat. No. 5,419,966.
   Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target gene are used. That is, two, three, four or more probes, are used to build in a redundancy for a particular target. The probes can be overlapping (i.e. have some sequence in common), or are specific for distinct sequences of a gene. When multiple target polynucleotides are to be detected according to the present invention, each probe or probe group corresponding to a particular target polynucleotide is situated in a discrete area of the microarray.
   Probes may be in solution, such as in wells or on the surface of a micro-array, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. One example comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence. Patterns can be manually or computer analyzed, but it is clear that positional sequencing by hybridization lends itself to computer analysis and automation. In another example, one may use an Affymetrix chip on a solid phase structural support in combination with a fluorescent bead based approach. In yet another example, one may utilise a cDNA microarray. In this regard, the oligonucleotides described by Lockkart *et al* (i.e. Affymetrix synthesis probes *in situ* on the solid phase) are particularly preferred, that is, photolithography.
   As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.
   Nucleic acid probes may be attached to the solid support by covalent binding such as by conjugation with a coupling agent or by covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody F_{c} fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative. Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.
   In general, the probes are attached to the biochip in a wide variety of ways, as will be appreciated by those in the art. As described herein, the nucleic acids can either be synthesized first, with subsequent attachment to the biochip, or can be directly synthesized on the biochip.
   The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated and may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signalling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.
   In one embodiment, the surface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or hetero-bifunctional linkers as are well known. In addition, in some cases, additional linkers, such as alkyl groups (including substituted and heteroalkyl groups) may be used.
   In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.
   The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling). Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al., Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established. See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, *supra*) or by covalent attachment of chemically synthesized oligonucleotides (Guo *et al*., *supra*) in combination with robotic arraying technologies.
   In addition to the solid-phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences. The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of double-stranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR(r) Green 1, is an example of an intercalating dye, that preferentially binds to dsDNA resulting in a concomitant increase in the fluorescent signal. Sequence specific probes, such as used with TaqMan technology, consist of a fluorochrome and a quenching molecule covalently bound to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signalling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer et al., Genome Res. 10:258-266 (2000); Heid et al., Genome Res. 6:986-994 (1996).
(iv) Measurement of altered neoplastic marker protein levels in cell extracts, for example by immunoassay.
   Testing for proteinaceous neoplastic marker expression product in a biological sample can be performed by any one of a number of suitable methods which are well known to those skilled in the art. Examples of suitable methods include, but are not limited to, antibody screening of tissue sections, biopsy specimens or bodily fluid samples.
   To the extent that antibody based methods of diagnosis are used, the presence of the marker protein may be determined in a number of ways such as by Western blotting, ELISA or flow cytometry procedures. These, of course, include both single-site and two-site or "sandwich" assays of the non-competitive types, as well as in the traditional competitive binding assays. These assays also include direct binding of a labelled antibody to a target.
   Sandwich assays are a useful and commonly used assay. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample. Variations on the forward assay include a simultaneous assay, in which both sample and labelled antibody are added simultaneously to the bound antibody. These techniques are well known to those skilled in the art, including any minor variations as will be readily apparent.
   In the typical forward sandwich assay, a first antibody having specificity for the marker or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking, covalently binding or physically adsorbing, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any subunit present in the antibody. Following the incubation period, the antibody subunit solid phase is washed and dried and incubated with a second antibody specific for a portion of the antigen. The second antibody is linked to a reporter molecule which is used to indicate the binding of the second antibody to the antigen.
   An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.
   By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.
   In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. "Reporter molecule" also extends to use of cell agglutination or inhibition of agglutination such as red blood cells on latex beads, and the like.
   Alternately, fluorescent compounds, such as fluorecein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescence and EIA techniques are both very well established in the art and are particularly preferred for the present method. However, other reporter molecules, such as radioisotope, chemiluminescent or bioluminescent molecules, may also be employed.
(v) Determining altered expression of protein neoplastic markers on the cell surface, for example by immunohistochemistry.
(vi) Determining altered protein expression based on any suitable functional test, enzymatic test or immunological test in addition to those detailed in points (iv) and (v) above.

A person of ordinary skill in the art could determine, as a matter of routine procedure, the appropriateness of applying a given method to a particular type of biological sample.

Without limiting the present invention in any way, and as detailed above, gene expression levels can be measured by a variety of methods known in the art. For example, gene transcription or translation products can be measured. Gene transcription products, i.e., RNA, can be measured, for example, by hybridization assays, run-off assays., Northern blots, or other methods known in the art.

Hybridization assays generally involve the use of oligonucleotide probes that hybridize to the single-stranded RNA transcription products. Thus, the oligonucleotide probes are complementary to the transcribed RNA expression product. Typically, a sequence-specific probe can be directed to hybridize to RNA or cDNA. A "nucleic acid probe", as used herein, can be a DNA probe or an RNA probe that hybridizes to a complementary sequence. One of skill in the art would know how to design such a probe such that sequence specific hybridization will occur. One of skill in the art will further know how to quantify the amount of sequence specific hybridization as a measure of the amount of gene expression for the gene was transcribed to produce the specific RNA.

The hybridization sample is maintained under conditions that are sufficient to allow specific hybridization of the nucleic acid probe to a specific gene expression product. "Specific hybridization", as used herein, indicates near exact hybridization (e.g., with few if any mismatches). Specific hybridization can be performed under high stringency conditions or moderate stringency conditions. In one embodiment, the hybridization conditions for specific hybridization are high stringency. For example, certain high stringency conditions can be used to distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. et al., "Current Protocols in Molecular Biology", John Wiley & Sons, (1998)). The exact conditions that determine the stringency of hybridization depend not only on ionic strength (e.g., 0.2.times.SSC, 0.1.times.SSC), temperature (e.g., room temperature, 42°C., 68°C.) and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, equivalent conditions can be determined by varying one or more of these parameters while maintaining a similar degree of identity or similarity between the two nucleic acid molecules. Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions that will allow a given sequence to hybridize (e.g., selectively) with the most complementary sequences in the sample can be determined.
Exemplary conditions that describe the determination of wash conditions for moderate or low stringency conditions are described in Kraus, M. and Aaronson, S., 1991. Methods Enzymol., 200:546-556; and in, Ausubel et al., Current Protocols in Molecular Biology, John Wiley & Sons, (1998). Washing is the step in which conditions are usually set so as to determine a minimum level of complementarity of the hybrids. Generally, starting from the lowest temperature at which only homologous hybridization occurs, each °C. by which the final wash temperature is reduced (holding SSC concentration constant) allows an increase by 1% in the maximum mismatch percentage among the sequences that hybridize. Generally, doubling the concentration of SSC results in an increase in Tₘ of about 17°C. Using these guidelines, the wash temperature can be determined empirically for high, moderate or low stringency, depending on the level of mismatch sought. For example, a low stringency wash can comprise washing in a solution containing 0.2.times.SSC/0.1% SDS for 10 minutes at room temperature; a moderate stringency wash can comprise washing in a pre-warmed solution (42°C) solution containing 0.2.times.SSC/0.1% SDS for 15 minutes at 42°C.; and a high stringency wash can comprise washing in pre-warmed (68°C.) solution containing 0.1.times.SSC/0.1% SDS for 15 minutes at 68°C. Furthermore, washes can be performed repeatedly or sequentially to obtain a desired result as known in the art. Equivalent conditions can be determined by varying one or more of the parameters given as an example, as known in the art, while maintaining a similar degree of complementarity between the target nucleic acid molecule and the primer or probe used (e.g., the sequence to be hybridized).

A related aspect of the present disclosure provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.
Preferably, said percent identity is at least 85%, 90%, 91%, 92%, 93%, 94%, 95%, 96%, 97%, 98% or 99%.
Low stringency includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions. In general, washing is carried out at Tₘ = 69.3 + 0.41 (G + C) % [19] = -12°C. However, the Tₘ of a duplex DNA decreases by 1°C with every increase of 1% in the number of mismatched based pairs (Bonner et al (1973) J. Mol. Biol. 81:123).

Preferably, the subject probes are designed to bind to the nucleic acid or protein to which they are directed with a level of specificity which minimises the incidence of non-specific reactivity. However, it would be appreciated that it may not be possible to eliminate all potential cross-reactivity or non-specific reactivity, this being an inherent limitation of any probe based system.

In terms of the probes which are used to detect the subject proteins, they may take any suitable form including antibodies and aptamers.

A library or array of nucleic acid or protein probes provides rich and highly valuable information. Further, two or more arrays or profiles (information obtained from use of an array) of such sequences are useful tools for comparing a test set of results with a reference, such as another sample or stored calibrator. In using an array, individual probes typically are immobilized at separate locations and allowed to react for binding reactions. Primers associated with assembled sets of markers are useful for either preparing libraries of sequences or directly detecting markers from other biological samples.

A library (or array, when referring to physically separated nucleic acids corresponding to at least some sequences in a library) of gene markers exhibits highly desirable properties. These properties are associated with specific conditions, and may be characterized as regulatory profiles. A profile, as termed here refers to a set of members that provides diagnostic information of the tissue from which the markers were originally derived. A profile in many instances comprises a series of spots on an array made from deposited sequences.

A characteristic patient profile is generally prepared by use of an array. An array profile may be compared with one or more other array profiles or other reference profiles. The comparative results can provide rich information pertaining to disease states, developmental state, receptiveness to therapy and other information about the patient.

Another aspect of the present invention provides a diagnostic kit for assaying biological samples comprising an agent for detecting one or more neoplastic marker reagents useful for facilitating the detection by the agent in the first compartment. Further means may also be included, for example, to receive a biological sample. The agent may be any suitable detecting molecule.

The present invention is further described by the following non-limiting examples:

### EXAMPLE 1

### Methods and Materials

### Affymetrix GeneChip data

Gene expression profiling data and accompanying clinical data was purchased from GeneLogic Inc (Gaithersburg, MD USA). For each tissue analysed, oligonucleotide microarray data for 44,928 probesets (Affymetrix HGU133A & HGU133B, combined), experimental and clinical descriptors, and digitally archived microscopy images of histological preparations were recieved. A quality control analysis was performed to remove arrays not meeting essential quality control measures as defined by the manufacturer.

Transcript expression levels were calculated by both Microarray Suite (MAS) 5.0 (Affymetrix) and the Robust Multichip Average (RMA) normalization techniques (Affymetrix. GeneChip expression data analysis fundamentals. Affymetrix, Santa Clara, CA USA, 2001; Hubbell et al. Bioinformatics, 18:1585-1592, 2002; Irizarry et al. Nucleic Acid Research, 31, 2003)MAS normalized data was used for performing standard quality control routines and the final data set was normalized with RMA for all subsequent analyses.

### Univariate differential expression

Differentially expressed gene transcripts were identified using a moderated t-test implemented in the limma library downloaded from the Bioconductor repository for R. (G. K. Smyth. Statistical Applications in Genetics and Molecular Biology, 3(1):Article 3, 2004; G K Smyth. Bioinformatics and Computational Biology Solutions using R and Bioconductor. Springer, New York, 2005). Significance estimates (p-values) were corrected to adjust for multiple hypothesis testing using the Bonferonni correction.

### Tissue specific expression patterns

To construct a filter for hypothetically 'turned off' gene expression the mean expression level for all 44,928 probesets across the full range of 454 tissues was first estimated. To estimate an expression on/off threshold, the 44,928 mean values were ranked and the expression value equivalent to the 30th percentile across the dataset calculated. This arbitrary threshold was chosen because it was theorized that the majority of transcripts (and presumably more than 30%) in a given specimen should be transcriptionally silenced. Thus this threshold represents a conservative upper bound for what is estimated as non-specific, or background, signal.

### Gene symbol annotations

To map Affymetrix probeset names to official gene symbols the annotation metadata available from Bioconductor was used. Hgu133plus2 library version 1.16.0, which was assembled using Entrez Gene data downloaded on 15 March 2007, was used.

### Estimates of performance characteristics

Diagnostic utility for each table of markers shown herein was estimated including: sensitivity, specificity, positive predictive value, negative predictive value, likelihood ratio positive, likelihood ratio negative. These estimates were calculated in the same data used to discover the markers and will therefore potentially overestimate the performance characteristics in future tissue samples. To improve the generalisabilty of the estimates a modified jackknife resampling technique was used to calculate a less biased value for each characteristic.

### Results

A range of univariate statistical tests were applied on Affymetrix oligonucleotide microarray data to reveal human genes that could be used to discriminate colorectal neoplastic tissues from non-neoplastic tissues. There were further identified a number of gene transcripts that appear to be useful for differentiating colorectal adenomas from colorectal carcinoma. Also identified were a subset of these transcripts that may have particular diagnostic utility due to the protein products being either secreted or displayed on the cell surface of epithelial cells. Finally, there were identified a further subset of transcripts expressed specifically in neoplastic tissues and at low- or near-background levels in non-neoplastic tissues.

### Genes differentially expressed in neoplastic tissues

From a total GeneChip set of 44,928 probesets it was determined that over 11,000 probesets were differentially expressed by moderated t-test using the limma package in BioConductor (G. K. Smyth, 2004 *supra*) employing conservative (Bonferroni) multiple test correction. When this list was further filtered to include only those probesets demonstrating a 2-fold or greater mean expression change between the neoplastic and non-neoplastic tissues, 560 probesets were found to be expressed *lower* in neoplasias relative to normal tissues.

These 560 probesets were annotated using the most recent metadata and annotation packages available for the chips. The 560 underexpressed probesets were mapped to 434 gene symbols.

| Δ-expression | ProbeSet ID | Gene Symbol Maps |
|---|---|---|
| DOWN | 560 | 434 |

### Hypothetical markers specific for colorectal neoplasia

While differential gene expression patterns are useful for diagnostic purposes this project also seeks to identify diagnostic proteins shed into the lumen of the gut by neoplastic colorectal epithelia. To discover candidate proteins, the list of differentially expressed transcripts was filtered with a selection criteria aimed at identifying markers specifically turned off in colorectal neoplasia tissues. To identify 'off' genes the filter criteria were designed to find genes with i) neoplastic expression levels below a theoretical on/off threshold and ii) normal signals at least 2-fold higher. The expression profile of an example transcript that is 'turned-off' in neoplastic tissues is shown in Figure 1.

### EXAMPLE 2

### PROBESETS ELEVATED IN NON-NEOPLASIA RELATIVE TO NEOPLASTIC TISSUES

Differential expression analysis was applied to identify down-regulated probesets in Affymetrix gene chip data measuring RNA concentration in 454 colorectal tissues including 161 adenocarcinoma specimens, 29 adenoma specimens, 42 colitis specimens and 222 non-diseased tissues. Using conservative corrections for multiple hypothesis testing and a 2-fold absolute fold change cut-off it was determined that 560 probesets exhibit a decreased expression level in neoplastic tissues relative to non-neoplastic controls. 560 of these probesets have been mapped to 434 putative gene symbols based on transcript nucleotide sequence.

### Validation/Hypothesis testing

RNA expression levels of these candidates were measured in independently derived clinical specimens. 526 probesets were hybridised to RNA extracts from 68 clinical specimens comprising 19 adenomas, 19 adenocarcinomas, and 30 non-diseased controls using a custom-designed 'Adenoma Gene Chip'. Thirty-four (34) probesets were not tested as they were not included on the custom design. It was confirmed that 459 of 526 of the target probesets (or directly related probesets with the same gene locus target) were likewise differentially expressed (P < 0.05) in these independently-derived tissues. The results of differential expression analysis of these 459 probesets is shown in Table 1.

The 372 of the 434 unique gene loci to which the 560 probesets are understood to hybridise were further tested. The remaining 62 gene symbols were not represented in the validation data. It was observed that 328 of 372 gene symbols were represented in the validation data by at least one differentially expressed probeset and many symbols included multiple probesets against regions across the putative locus. A complete list of probesets that bind to target loci is shown in Table 2.

### Conclusion

The candidate probesets and symbols shown in Tables 1 and 2. respectively, are differentially expressed lower in neoplastic colorectal tissues compared to non-neoplastic controls.

### EXAMPLE 3

### PROBESETS DEMONSTRATING A NON-NEOPLASIA SPECIFIC PROFILE

During analysis of the discovery data, a novel expression profile was observed between neoplastic and non-neoplastic phenotypes. It was hypothesized that a subset of quantitatively differentially expressed probesets are furthermore qualitatively differentially expressed. Such probesets show no evidence of a gene expression activity in neoplastic tissues, i.e. these probesets appear to be expressed above background levels in non-neoplastic tissues only. This observation and the resulting hypothesis are based on two principles:
1. That the majority of human transcripts that are present on a genome-wide GeneChip (e.g. U133) will not likely be expressed in the colorectal mucosa; and
2. That microarray binding intensity for such 'off probesets (to labeled cRNA) will reflect technical assay background, i.e. non-specific oligonucleotide binding.

To generate a list of non-neoplasia specific probesets the neoplastic intensity of differentially expressed probesets were compared with a hypothetical background signal threshold from across all probesets on the chip. We note that, by design, all probesets in the candidate pool from which the 'on' transcripts are chosen are at least two fold over-expressed in the non-diseased tissues relative to diseased tissues. Combined, these criteria yield the subset of differentially expressed transcript species that are specifically expressed in non-neoplastic tissues.

This analysis demonstrated that 42 probesets corresponding to approximately 41 gene loci exhibit a non-neoplasia specific transcription expression profile.

### Validation/Hypothesis Testing

The custom gene chip design precludes testing the non-neoplasia-specific probesets using the same principles as used for discovery. In particular, the custom gene chip (by design) does not contain a large pool of probesets anticipated to hybridise to hypothetically 'off/'non-transcribed' gene transcripts. This is because the custom gene chip design is heavily biased toward differentially expressed transcripts in colorectal neoplastic tissues.

The usual differential expression testing (limma) was therefore applied to these candidate probesets for specifically expressed in non-neoplastic tissues. Of the 37 (of 42) probesets on the custom gene chip, 33 probesets (or probesets which bind to the same locus) were differentially expressed between the 38 neoplastic tissues (adenoma & cancer) and non-neoplastic controls. The results of these validation experiments is shown in Table 3.

It was further aimed to test all probesets which are known to hybridise to the gene loci to which the probesets claimed herein. Of the 41 putative gene loci targeted by the probesets, 33 were present in the validation data. All thirty-three (33) of these 33 (100%) gene symbols demonstrated at least one hybridising probeset which was differentially expressed in the neoplastic tissues. Results for these experiments, including all probesets that bind to each target locus in a differentially expressed manner are shown in Table 4.

### EXAMPLE 4

### MATERIALS AND METHODS FOR EXAMPLES 2 AND 3

Gene expression profiling data measured in 454 colorectal tissue specimens including neoplastic, normal and non-neoplastic disease controls was purchased from GeneLogic Inc (Gaithersburg, MD USA). For each tissue specimen Affymetrix (Santa Clara, CA USA) oligonucleotide microarray data totalling 44,928 probesets (HGU133A & HGU133B, combined), experimental and clinical descriptors, and digitally archived microscopy images of histological preparations was received. Prior to applying discovery methods to these data, extensive quality control methods, including statistical exploration, review of clinical records for consistency and histopathology audit of a random sample of arrays was carried out. Microarrays that did not meet acceptable quality criteria were removed from the analysis.

### Hypothesis testing

Candidate transcription biomarkers were tested using a custom oligonucleotide microarray of 25-mer oligonucleotide probesets designed to hybridise to candidate RNA transcripts identified during discovery. Differential expression hypotheses were tested using RNA extracts derived from independently collected clinical samples comprising 30 normal colorectal tissues, 19 colorectal adenoma tissues, and 19 colorectal adenocarcinoma tissues. Each RNA extract was confirmed to meet strict quality control criteria.

### Colorectal tissue specimens

All tissues used for hypothesis testing were obtained from a tertiary referral hospital tissue bank in metropolitan Adelaide, Australia (Repatriation General Hospital and Flinders Medical Centre). Access to the tissue bank for this research was approved by the Research and Ethics Committee of the Repatriation General Hospital and the Ethics Committee of Flinders Medical Centre. Informed patient consent was received for each tissue studied.

Following surgical resection, specimens were placed in a sterile receptacle and collected from theatre. The time from operative resection to collection from theatre was variable but not more than 30 minutes. Samples, approximately 125mm3 (5x5x5mm) in size, were taken from the macroscopically normal tissue as far from pathology as possible, defined both by colonic region as well as by distance either proximal or distal to the pathology. Tissues were placed in cryovials, then immediately immersed in liquid nitrogen and stored at -150°C until processing.

### RNA extraction

RNA extractions were performed using Trizol(R)reagent (Invitrogen, Carlsbad, CA, USA) as per manufacturer's instructions. Each sample was homogenised in 300µL of Trizol reagent using a modified Dremel drill and sterilised disposable pestles. Additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containing target RNA was obtained by centrifugation at 12,000 rpm for 15 min, 40°C. RNA was then precipitated by incubating samples at RT for 10 min with 250µL of isopropanol. Purified RNA precipitate was collected by centrifugation at 12,000 rpm for 10 minutes, 40°C and supernatants were discarded. Pellets were then washed with 1mL 75% ethanol, followed by vortexing and centrifugation at 7,500g for 8 min, 40°C. Finally, pellets were air-dried for 5 min and resuspended in 80µL of RNase free water. To improve subsequent solubility samples were incubated at 55°C for 10 min. RNA was quantified by measuring the optical density at A260/280 nm. RNA quality was assessed by electrophoresis on a 1.2% agarose formaldehyde gel.

### Gene Chip processing

To test hypotheses related to biomarker candidates for colorectal neoplasia RNA extracts were assayed using a custom GeneChip designed by us in collaboration with Affymertix (Santa Clara, CA USA). These custom GeneChips were processed using the standard Affymetrix protocol developed for the HU Gene ST 1.0 array described in (Affy:WTAssay).

### Statistical software and data processing

The R statistics environment R and BioConductor libraries (BioConductor, www.bioconductor.org) (BIOC) was used for most analyses. To map probeset IDs to gene symbol on the Custom GeneChip hgu133plus2 library version 2.2.0 was used which was assembled using Entrez Gene data downloaded on Apr 18 12:30:55 2008 (BIOC).

### Hypothesis testing of differentially expressed biomarkers

To assess differential expression between tissue classes the Student's t test for equal means between two samples or the robust variant provided by the limma library (Smyth)(limma) was used. To mitigate the impact of false discovery due to multiple hypothesis testing, a Bonferroni adjustment to P values in the discovery process (MHT:Bonf) was applied. For hypotheses testing the slightly less conservative multiple hypothesis testing correction of Benjamini & Hochberg, which aims to control the false discovery rate of solutions(MHT:BH), was applied.

### Discovery of tissue-specific gene expression patterns

Discovery methods using gene expression data often yield numerous candidates, many of which are not suitable for commercial products because they involve subtle gene expression differences that would be difficult to detect in laboratory practice. Pepe *et al*. note that the 'ideal' biomarker is detectable in tumor tissue but not detectable (at all) in non-tumour tissue (Pepe:biomarker:development.) To bias the discovery toward candidates that meet this criteria, an analysis method was developed that aims to enrich the candidates for biomarkers whose qualitative absence or presence measurement is diagnostic for the phenotype of interest. This method attempts to select candidates that show a prototypical 'turned-on' or 'turned-off' pattern relative to an estimate of the background/noise expression across the chip. It is theorized that such RNA transcripts are more likely to correlate with downstream translated proteins with diagnostic potential or to predict upstream genomic changes (e.g. methylation status) that could be used diagnostically. This focus on qualitative rather than quantitative outcomes may simplify the product development process for such biomarkers.

The method is based on the assumption that the pool of extracted RNA species in any given tissue (e.g. colorectal mucosae) will specifically bind to a relatively small subset of the full set of probesets on a GeneChip designed to measure the whole genome. On this assumption, it is estimated that most probesets on a full human gene chip will not exhibit specific, high-intensity signals.

This observation is utilised to approximate the background or 'non-specific binding' across the chip by choosing a theoretical level equal to the value of e.g. lowest 25% quantile of the ranked mean values. This quantile can be arbitrarily set to some level below which there is made a reasonable assumption that the signals do not represent above-background RNA binding. Finally, this background estimate is used as a threshold to estimate the 'OFF' probesets in an experiment for, say, the non-neoplastic tissue specimens.

Conversely, it is further hypothesized that probesets which are 1) expressed above this theoretical threshold level and 2) at differentially higher levels in the tumour specimens may be a tumour specific candidate biomarker. It is noted that in this case the concept of 'fold-change' thresholds can also be conveniently applied to further emphasize the concept of absolute expression increases in a putatively 'ON' probeset.

Given the assumption of low background binding for a sizeable fraction of the measured probesets, this method was only used in the large GeneLogic data and discovery. To construct a filter for hypothetically 'turned on' biomarker in the GeneLogic discovery data, the mean expression level for all 44,928 probesets across the full range of 454 tissues was estimated. The 44,928 mean values were then ranked and the expression value equivalent to the 25th percentile across the dataset calculated. This arbitrary threshold was chosen because the majority of transcripts (and presumably more than 25%) in a given specimen should exhibit low concentration which effectively transcriptional silence. Thus this threshold represents a conservative upper bound for what is estimated is non-specific, or background, expression.

### EXAMPLE 5

### DNA METHYLATION DATA

Assays were developed for detection of methylation in the promoter regions the eight down-regulated genes in Table 5. Methods for bisulphite treatment of DNA and assays for determination of DNA methylation levels, including MSP and COBRA, are described in Clark *et al*.. (2006).

Five MSP assays used the primer pairs shown in TABLE 7. A control PCR for unbiased amplification of the CAGE gene was used to determine the quantity of input DNA to provide a reference for quantification of the level of methylation of each gene. For PCRs, 25 µL reactions in Biorad iQ SyBr Green Super Mix contained 5ng of bisulphite-treated DNAs (1 ng for cell line assays and 6 ng for clinical specimens) and 200nM of forward and reverse primers. PCR cycling conditions were:
95.0°C for 2 min
Followed by 50 cycles of:
   95.0°C/15sec
   Temp°C/30sec
   72.0°C/30sec

Where "Temp" is the re-annealing temperature optimised for each gene as shown in Table yy.

For the DF gene, 3 preliminary cycles were done using a 95.0°C melting temperature, followed by 50 cycles with a lower, 84.0°C melting temperature (to reduce nonspecific amplification).

A standard curve was generated using DNA methylated with M.SssI methylase (100% methylated) and DNA that had been in vitro amplified using Phi29 DNA polymerase (0% methylation).

COBRA assays were developed for three genes as shown in TABLE 8. PCRs were setup as above with cycling conditions:
95.0°C for 2 min
Followed by 50 cycles of:
   95.0°C/15sec
   Temp°C/30sec
   72.0°C/30sec

After PCR, 10 µL of PCR product was digested with the appropriate enzyme (TABLE 8), digestion products analysed by gel electrophoresis and methylation levels determined semiquantitatively.

The methylation state of the eight genes was determined in four colorectal cancer cell lines, Caco2, HCT116, HT29 and SW480 as well as normal blood DNA and the normal lung fibroblast cell line, MRC5. The level of methylation in summarised in Table 5. The promoter regions of all eight genes show strong methylation in 2 or 3 of the four colorectal cancer cell lines tested. All showed a lack or low level of methylation in DNA from normal blood DNA and the fibroblast cell line MRC5, except for methylation of DF in MRC5.

For two of these genes, MAMDC2 and GPM6B analysis has been extended to a set of 12 adenoma, 18 cancer and 22 matched normal tissue samples (Figure 2, A and B).

For MAMDC2 quantitative analysis demonstrated that 2 of 12 adenomas and 6 of 18 cancer samples showed elevated methylation compared with the highest level observed in normal tissue samples. Methylation levels of the GPM6B gene were determined by semiquantitative COBRA assays, scored on a scale of 0 to 5 based on visual inspection of restriction digestions. A clear trend toward increasing promoter methylation in progression from normal to adenoma to cancer was evident (Figure 2, panel B).

These data demonstrate for a number of examples of the down-regulated genes that such downregulation in colorectal cancer cell lines and primary neoplasia tissue may be associated with DNA methylation and that assays of DNA methylation can be used to discriminate cancer and normal tissue.

### EXAMPLE 6

### Determine Gene Identity of a Nucleic Acid Sequence of Interest which is Define by an Affymetrix Probeset

### BLAST the sequence of interest using online available Basic Local Alignment Search Tools [BLAST]. e.g. NCBI/BLAST

(http://blast.ncbi.nlm.nih.gov/Blast.cgi)
(a) Select "Human" in BLAST ASSEMBLED GENOMES on the web page http://blast.ncbi.nlm.nih.gov/Blast.cgi
(b) Leave the default settings, i.e.:
   - Database: Genome (all assemblies)
   - Program: megaBLAST: compare highly related nucleotide sequences
   - Optional parameters: Expect: 0.01, Filter: default, Descriptions: 100, Alignments: 100
(c) Copy/Paste Sequence into the "BLAST" window
(d) Click "Begin Search"
(e) Click "View Report"

### Assessment of the Open BLAST Search Results

Multiple significant sequence alignments may be identified when "blasting" the sequence.

### Identify gene nomenclature of the identified sequence match

(a) Click the link to one of the identified hits
(b) The new page will schematically depict the position of the hit on one chromosome. It will be apparent which gene is hit.
(c) Retrieve the "hit" sequence clicking on the link
(d) Do a search for the gene in the provided "search" window. This provides the gene nucleotide coordinates for the gene.

### Determine promiscuity of Sequence

(a) Open the NCBI/BLAST tool, (http://blast.ncbi.nlm.nih.sov/Blast.cgi)
(b) Click on "nucleotide Blast" under "basic BLAST"
(c) Copy/paste the sequence of interest into the "Query Sequence" window
(d) Click "Blast".

### Assessment of the nBLAST Search Results of the Sequence

(a) The nBLAST exercise with the Sequence may result in multiple Blast hits of which some accession entry numbers are listed in "Description".
(b) These hits should be reviewed.

### Determine location of the Sequence in the Gene

The Ensembl database is an online database, which produces and maintains automatic annotation selected eukaryotic genomes (www.ensembl.orq/index.html)

### Identify location of the Sequence in the Gene

(a) Set "Search" to Homo Sapiens, Type "the gene name" in the provided Search Field Ensemble.org/index.html)
(b) Click "Go"
(c) Click the "vega protein_coding Gene: OTTHUMG000000144184" link to get an annotation report
(d) Click on "Gene DAS Report" to retrieve information regarding Alternative splice site database: Type "the gene name" in search field
   - Click on "the gene entry"
   - Scroll down to "evidences"
   - Review alternative splice sites
   - Click "Confirmed intron/exons" to get a list of coordinates for the exons & introns.

### Alternative splicing and/or transcription

The AceView Database provides curated and non-redundant sequence representation of all public mRNA sequences. The database is available through NCBI:
http://www.ncbi.nlm.nih.gov/IEB/Research/Acembly/

### Further investigation of the Gene mRNA transcripts

(a) Type "the gene name" into the provided "search" field
(b) Click "Go"
(c) The following information is available from the resulting entry in AceView:
   - The number of cDNA clones from which the gene is constructed (ie originated-from experimental work involving isolation of mRNA)
   - The mRNAs predicted to be produced by the gene
   - The existence of non-overlapping alternative exons and validated alternative polyadenylation sites
   - The existence of truncations
   - The possibility of regulated alternate expression
   - Introns recorded as participating in alternatively splicing of the gene
(d) Classic splice site motives

### Application of Method to LOC643911/hCG_1815491

### Materials and Methods

### Extraction of RNA

RNA extractions were performed using Trizol(R) reagent (Invitrogen, Carlsbad, CA, USA) as per manufacturer's instructions. Each sample was homogenised in 300µL of Trizol reagent using a modified dremel drill and sterilised disposable pestles. Additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containg target RNA was obtained by centrifugation at 12,000 rpm for 15 min, 40° C. RNA was then precipitated by incubating samples at RT for 10 min with 250µL of isopropanol. Purified RNA precipitate was collected by centrifugation at 12,000 rpm for 10 minutes, 40° C and supernatants were discarded. Pellets were then washed with 1mL 75% ethanol, followed by vortexing and centrifugation at 7,500g for 8 min, 40° C . Finally, pellets were air-dried for 5 min and resuspended in 80µL of RNase free water. To improve subsequent solubility samples were incubated at 55° C for 10 min. RNA was quantified by measuring the optical density at A260/280 nm. RNA quality was assessed by electrophoresis on a 1.2% agarose formaldehyde gel.

### Gene Chip processing

RNA samples to analyze on Human Exon 1.0 ST GeneChips were processed using the Affymetrix WT target labeling and control kit (part# 900652) following the protocol described in (Affymetrix 2007 P/N 701880 Rev.4). Briefly: First cycle cDNA was synthesized from 100ng ribosomal reduced RNA using random hexamer primers tagged with T7 promoter sequence and SuperScript II (Invitrogen, Carlsbad CA), this was followed by DNA Polymerase I synthesis of the second strand cDNA. Anti-sense cRNA was then synthesized using T7 polymerase. Second cycle sense cDNA was then synthesised using SuperScript II, dNTP+ dUTP, and random hexamers to produce sense strand cDNA incorporating uracil. This single stranded uracil containing cDNA was then fragmented using a combination of uracil DNA glycosylase (UDG) and apurinic/ apyrimidinic endonuclease1 (APE 1). Finally the DNA was biotin labelled using terminal deoxynucleotidyl transferase (TdT) and the Affymetrix proprietary DNA Labeling reagent. Hybridization to the arrays was carried out at 45°C for 16-18hours.

Washing and staining of the hybridized GeneChips was carried out using the Affymetrix Fluidics Station 450 and scanned with the Affymetrix Scanner 3000 following recommended protocols.

### SYBR green based Quantitative Real Time-PCR

Quantitative real time polymerase chain reaction was performed on RNA isolated from clinical samples for the amplification and detection of the various hCG_1815491 transcripts.

Firstly cDNA was synthesized from 2ug of total RNA using the Applied Biosystems High Capacity Reverse transcription Kit (P/N 4368814). After synthesis the reaction was diluted 1:2 with water to obtain a final volume of 40ul and 1ul of this diluted cDNA used in subsequent PCR reactions.

PCR was performed in a 25ul volume using 12.5ul Promega 2x PCR master mix (P/N M7502), 1.5ul 5uM forward primer, 1.5ul 5uM reverse primer, 7.875ul water, 0.625ul of a 1:3000 dilution of 10,000x stock of SYBR green 1 pure dye (Invitrogen P/N S7567), and 1ul of cDNA.

Cycling conditions for amplification were 95° for 2minutes x1 cycle, 95° for 15 seconds and 60° for 1 minute x40 cycles. The amplification reactions were performed in a Corbett Research Rotor-Gene RG3000 or a Roche LightCycler480 real-time PCR machine. When the Roche LightCycler480 real-time PCR machine was used for amplification the reaction volume was reduced to 10ul and performed in a 384 well plate but the relative ratios between all the components remained the same. Final results were calculated using the ΔΔCt method with the expression levels of the various hCG_1815491 transcripts being calculated relative to the expression level of the endogenous house keeping gene HPRT.

### End-point PCR

End point PCR was performed on RNA isolated from clinical samples for the various hCG_1815491 transcripts. Conditions were identical to those described for the SYBR green assay above but with the SYBR green dye being replaced with water. The amplification reactions were performed in a MJ Research PTC-200 thermal cycler. 2.5µl of the amplified products were analysed on 2% agarose E-gel (Invitrogen) along with a 100-base pair DNA Ladder Marker.

### Results

The nucleotide structure and expression levels of transcripts related to hCG_1815491 was analysed based on the identification of diagnostic utility of Affymetrix probesets 238021_s_at and 238022_at from the gene chip analysis.

The gene hCG_1815491 is currently represented in NCBI as a single RefSeq sequence, XM 93911. The RefSeq sequence of hCG_1815491 is based on 89 GenBank accessions from 83 cDNA clones. Prior to March 2006, these clones were predicted to represent two overlapping genes, LOC388279 and LOC650242 (the latter also known as LOC643911). In March 2006, the human genome database was filtered against clone rearrangements, co-aligned with the genome and clustered in a minimal non-redundant way. As a result, LOC388272 and LOC650242 were merged into one gene named hCG_1815491 (earlier references to hCG_1815491 are: LOC388279, LOC643911, LOC650242, XM_944116, AF275804, XM_373688).

It has been determined that the Ref Sequence, which is defined by the genomic coordinates 8579310 to 8562303 on human chromosome 16 as defined by the NCBI contig reference NT_010498.15 | Hs16_10655, NCBI 36 March 2006 genome encompasses hCG_1815491. The 10 predicted RNA variants derived from this gene have been aligned with the genomic nucleotide sequence residing in the map region 8579310 to 8562303. This alignment analysis revealed the existence of at least 6 exons of which several are alternatively spliced. The identified exons are in contrast to the just 4 exons specified in the NCBI hCG_1815491 RefSeq XM_93911. Two additional putative exons were also identified in the Ref Sequence by examination of included probesets on Affymetrix Genechip HuGene Exon 1.0 that target nucleotide sequences embedded in the Ref Sequence. The identified and expanded exon-intron structure of hCG_1815491 have been used to design specific oligonucleotide primers, which allowed measurement of the expression of RNA variants generated from the Ref Sequence by using PCR-based methodology (Figure 4)

### TABLES

The probeset designations include both HG-133plus2 probeset IDs and Human Gene 1.0ST array probe ids. The latter can be conveniently mapped to Transcript Cluster ID using the Human Gene 1.0ST probe tab file provided by Affymetrix (http://www.affymetrix.com/Auth/analysis/downloads/na22/wtgene/HuGene-1_0-st-v1.probe.tab.zip). Using publicly available software such as NetAffx (provided by Affymetrix), the Transcript Cluster ID may be further mapped to gene symbol, chromosomal location, etc.

### Table 1

Probesets demonstrated to be expressed higher in non-neoplastic tissues relative to neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

### Table 2

Evidence of multiple probesets which correspond to gene symbols claimed herein exhibiting RNA concentration differences between non-neoplastic tissues and neoplastic controls. Symbol: gene symbol; ValidPS_DOWN: Affymetrix probeset IDs demonstrating statistically significant overexpression in non-neoplastic RNA extracts relative to neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

### Table 3

Probesets which demonstrate a qualitatively (in addition to quantitative) elevated profile in non-neoplastic tissues relative to neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

### Table 4

Evidence of multiple probesets which correspond to gene symbols claimed herein exhibiting qualitative changes in RNA concentration in non-neoplastic tissues compared to neoplastic tissues. Symbol: gene symbol; ValidPS_DOWN: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**TABLE 1**

| **TargetPS** | **Symbol** | **Signif. FDR** | **D.val5** | **FC** | **Sens-Spec** | **CI (95)** |
|---|---|---|---|---|---|---|
| 230788_at | SPTLC3 :GCNT2 | 2.16E-27 | 3.9512 | 13.36 | 97.6 | 94.2-99.2 |
| 207502_at | GUCA2B | 7.04E-25 | 3.6279 | 51.78 | 96.5 | 92.3-98.6 |
| 207003_at | GUCA1B | 7.32E-25 | 3.565 | 10.7 | 96.3 | 92-98.5 |
| 206208_at | CA4 | 1.73E-24 | 3.6263 | 10.41 | 96.5 | 92.4-98.6 |
| 206209_s_at | CA4 | 1.73E-24 | 3.6265 | 10.41 | 96.5 | 92.3-98.6 |
| 203908_at | LOC727995:SLC4A4:LOC730 895 | 2.23E-23 | 3.6298 | 3.58 | 96.5 | 92.4-98.6 |
| 206784_at | AQP8 | 2.98E-22 | 3.3284 | 10.82 | 95.2 | 90.3-97.9 |
| 205950_s_at | CA1 | 3.15E-22 | 3.368 | 38.27 | 95.4 | 90.6-98 |
| 209735_at | ABCG2 | 1.09E-21 | 3.2584 | 31.53 | 94.8 | 89.7-97.7 |
| 230830_at | OSTbeta | 6.23E-21 | 3.1861 | 7.15 | 94.4 | 89.2-97.5 |
| 223754_at | MGC13057 | 6.94E-21 | 3.2036 | 3.18 | 94.5 | 89.3-97.5 |
| 228195_at | MGC13057 | 6.94E-21 | 3.2023 | 3.18 | 94.5 | 89.4-97.5 |
| 228706_s_at | CLDN23 | 4.27E-20 | 3.0757 | 3.47 | 93.8 | 88.2-97.1 |
| 228707_at | CLDN23 | 4.27E-20 | 3.072 | 3.47 | 93.8 | 88.3-97.1 |
| 223551_at | PKIB | 5.94E-20 | 3.1219 | 3.28 | 94.1 | 88.6-97.3 |
| 231120_x_at | PKIB | 5.94E-20 | 3.1226 | 3.28 | 94.1 | 88.6-97.3 |
| 226492_at | SEMA6D | 6.07E-20 | 3.0898 | 4.27 | 93.9 | 88.4-97.2 |
| 220026_at | CLCA4 | 1.25E-19 | 3.1909 | 16.3 | 94.5 | 89.2-97.5 |
| 224836_at | TP53INP2 | 1.45E-19 | 3.0248 | 3.78 | 93.5 | 87.9-96.9 |
| 220834_at | MS4A12 | 2.13E-19 | 3.29 | 7.15 | 95 | 90-97.8 |
| 224412_s_at | TRPM6 | 2.25E-19 | 3.01 | 7.5 | 93.4 | 87.7-96.8 |
| 220037_s_at | XLKD1 | 3.60E-19 | 2.9412 | 7.44 | 92.9 | 87.1-96.6 |
| 219059_s_at | XLKD1 | 3.60E-19 | 2.942 | 7.44 | 92.9 | 87-96.6 |
| 209612_s_at | ADH1B:ADH1A | 4.70E-19 | 3.0183 | 4.67 | 93.4 | 87.7-96.9 |
| 209613_s_at | ADH1B:ADH1A | 4.70E-19 | 3.0147 | 4.67 | 93.4 | 87.7-96.9 |
| 200845_s_at | LOC389249:PRDX6 | 5.56E-19 | 2.8412 | 2.76 | 92.2 | 86.1-96.1 |
| 209301_at | CA2 | 5.87E-19 | 2.9662 | 9.22 | 93.1 | 87.3-96.7 |
| 208399_s_at | EDN3 | 6.42E-19 | 2.9342 | 11.21 | 92.9 | 87-96.5 |
| 228961_at | MIER3 | 9.14E-19 | 2.9183 | 3.91 | 92.8 | 86.9-96.5 |
| 231975_s_at | MIER3 | 9.14E-19 | 2.9172 | 3.91 | 92.8 | 86.8-96.4 |
| 204719_at | ABCA8 | 1.26E-18 | 2.9172 | 6.82 | 92.8 | 86.8-96.5 |
| 207761_s_at | METTL7A | 1.35E-18 | 3.1247 | 1.93 | 94.1 | 88.6-97.3 |
| 207977_s_at | DPT | 1.53E-18 | 2.8967 | 9.07 | 92.6 | 86.6-96.4 |
| 213068_at | DPT | 1.53E-18 | 2.8973 | 9.07 | 92.6 | 86.7-96.4 |
| 213071_at | DPT | 1.53E-18 | 2.8962 | 9.07 | 92.6 | 86.7-96.4 |
| 218756_s_at | MRM1:MGC4172 | 1.74E-18 | 2.9041 | 3.74 | 92.7 | 86.6-96.4 |
| 212288_at | FNBP1:C1orf85:CCT3 | 1.76E-18 | 3.0475 | 2.04 | 93.6 | 88-97 |
| 204036_at | EDG2 | 2.44E-18 | 2.8853 | 3.19 | 92.5 | 86.4-96.3 |
| 202037_s_at | SFRP1 | 2.77E-18 | 2.8647 | 15.53 | 92.4 | 86.3-96.2 |
| 206143_at | SLC26A3 | 4.18E-18 | 2.9202 | 21.4 | 92.8 | 86.9-96.5 |
| 215657_at | SLC26A3 | 4.18E-18 | 2.9226 | 21.4 | 92.8 | 86.9-96.5 |
| 231773_at | ANGPTL1 | 1.42E-17 | 2.7938 | 4.64 | 91.9 | 85.6-95.9 |
| 205480_s_at | UGP2 | 1.64E-17 | 2.7911 | 2.41 | 91.9 | 85.6-95.9 |
| 204955_at | SRPX | 1.79E-17 | 2.7753 | 3.91 | 91.7 | 85.5-95.8 |
| 222722_at | OGN | 2.25E-17 | 2.7048 | 13.67 | 91.2 | 84.8-95.4 |
| 202555_s_at | MYLK | 2.33E-17 | 2.773 | 3.99 | 91.7 | 85.4-95.8 |
| 224823_at | MYLK | 2.33E-17 | 2.7727 | 3.99 | 91.7 | 85.4-95.8 |
| 225575_at | LIFR | 2.45E-17 | 2.7823 | 6.36 | 91.8 | 85.5-95.8 |
| 214142_at | LOC653808:ZG16 | 2.51E-17 | 2.8119 | 8.14 | 92 | 85.8-95.9 |
| 206710_s_at | EPB41L3 | 2.62E-17 | 2.8298 | 2.51 | 92.1 | 85.9-96.1 |
| 205464_at | SCNN1B | 3.20E-17 | 2.7209 | 16.15 | 91.3 | 84.9-95.5 |
| 220812_s_at | HHLA2 | 3.47E-17 | 2.6717 | 10.02 | 90.9 | 84.4-95.3 |
| 203913_s_at | HPGD | 3.65E-17 | 2.8441 | 3.21 | 92.2 | 86.1-96.1 |
| 203914_x_at | HPGD | 3.65E-17 | 2.8456 | 3.21 | 92.3 | 86.1-96.1 |
| 211548_s_at | HPGD | 3.65E-17 | 2.8446 | 3.21 | 92.3 | 86.1-96.1 |
| 211549_s_at | HPGD | 3.65E-17 | 2.8466 | 3.21 | 92.3 | 86.1-96.1 |
| 206198_s_at | CEACAM7 | 3.96E-17 | 2.7882 | 10.4 | 91.8 | 85.5-95.9 |
| 206199_at | CEACAM7 | 3.96E-17 | 2.7855 | 10.4 | 91.8 | 85.6-95.8 |
| 211848_s_at | CEACAM7 | 3.96E-17 | 2.7882 | 10.4 | 91.8 | 85.6-95.8 |
| 226430_at | SMAD5:RELL1 | 4.10E-17 | 2.6968 | 2.5 | 91.1 | 84.6-95.4 |
| 202992_at | C7 | 4.40E-17 | 2.7163 | 6.67 | 91.3 | 84.9-95.5 |
| 205112_at | PLCE1 | 6.91E-17 | 2.7542 | 2.43 | 91.6 | 85.2-95.7 |
| 229839_at | SCARA5 | 1.25E-16 | 2.6897 | 2.86 | 91.1 | 84.6-95.3 |
| 235849_at | SCARA5 | 1.25E-16 | 2.6918 | 2.86 | 91.1 | 84.5-95.3 |
| 209763_at | CHRDL1 | 1.49E-16 | 2.6093 | 13.88 | 90.4 | 83.7-94.9 |
| 205259_at | NR3C2 | 2.08E-16 | 2.5812 | 3.19 | 90.2 | 83.4-94.7 |
| 202242_at | TSPAN7 | 2.13E-16 | 2.6519 | 3.68 | 90.8 | 84.1-95.1 |
| 203000_at | STMN2 | 2.25E-16 | 2.6115 | 6.13 | 90.4 | 83.7-94.9 |
| 203001_s_at | STMN2 | 2.25E-16 | 2.6141 | 6.13 | 90.4 | 83.7-94.9 |
| 209074_s_at | FAM107A | 2.52E-16 | 2.6171 | 2.92 | 90.5 | 83.8-94.9 |
| 202920_at | ANK2 | 2.59E-16 | 2.6499 | 6.76 | 90.7 | 84.1-95.1 |
| 213317_at | CLIC5 | 3.68E-16 | 2.696 | 2.37 | 91.1 | 84.6-95.4 |
| 204697_s_at | CHGA | 5.54E-16 | 2.6087 | 7.21 | 90.4 | 83.7-94.9 |
| 212814_at | KIAA0828 | 5.58E-16 | 2.5495 | 4.09 | 89.9 | 83-94.5 |
| 225275_at | EDIL3 | 6.21E-16 | 2.6252 | 2.96 | 90.5 | 83.9-95 |
| 208370_s_at | DSCR1 | 6.21E-16 | 2.6146 | 2.14 | 90.4 | 83.7-94.9 |
| 209147_s_at | PPAP2A | 6.43E-16 | 2.5961 | 2.4 | 90.3 | 83.6-94.8 |
| 210946_at | PPAP2A | 6.43E-16 | 2.5967 | 2.4 | 90.3 | 83.5-94.8 |
| 202731_at | PDCD4 | 8.67E-16 | 2.4558 | 2.6 | 89 | 82-94 |
| 219799_s_at | DHRS9:GORASP2 | 1.09E-15 | 2.7368 | 1.51 | 91.4 | 85.1-95.6 |
| 223952_x_at | DHRS9:GORASP2 | 1.09E-15 | 2.7334 | 1.51 | 91.4 | 85-95.6 |
| 224009_x_at | DHRS9:GORASP2 | 1.09E-15 | 2.7359 | 1.51 | 91.4 | 85.1-95.6 |
| 236313_at | CDKN2B | 1.34E-15 | 2.5442 | 10.09 | 89.8 | 83-94.5 |
| 231925_at | P2RY1 | 1.41E-15 | 2.5354 | 3.6 | 89.8 | 82.9-94.4 |
| 238143_at | LOC646627 | 1.58E-15 | 1.9142 | 5.02 | 83.1 | 74.8-89.4 |
| 224480_s_at | LPAAT-THETA | 2.06E-15 | 2.3867 | 2.63 | 88.4 | 81.1-93.5 |
| 212230_at | PPAP2B | 2.44E-15 | 2.5821 | 1.81 | 90.2 | 83.4-94.7 |
| 207080_s_at | PYY | 3.50E-15 | 2.4811 | 11.71 | 89.3 | 82.3-94.1 |
| 205200_at | CLEC3B | 4.65E-15 | 2.4426 | 4.46 | 88.9 | 81.8-93.8 |
| 228133_s_at | NDE1 | 4.84E-15 | 2.514 | 1.99 | 89.6 | 82.7-94.3 |
| 214038_at | CCL8 | 5.75E-15 | 2.4504 | 7.51 | 89 | 81.9-93.9 |
| 219014_at | PLAC8 | 5.76E-15 | 2.4248 | 3.87 | 88.7 | 81.6-93.7 |
| 219796_s_at | MUCDHL | 5.81E-15 | 2.4346 | 3.06 | 88.8 | 81.6-93.8 |
| 220075_s_at | MUCDHL | 5.81E-15 | 2.4354 | 3.06 | 88.8 | 81.7-93.8 |
| 215299_x_at | SULT1A1:SULT1A2 | 7.17E-15 | 2.4231 | 3.37 | 88.7 | 81.5-93.7 |
| 233565_s_at | SDCBP2 | 9.28E-15 | 2.4871 | 2.37 | 89.3 | 82.3-94.1 |
| 228885_at | RPL24:LOC731365 | 1.23E-14 | 2.5555 | 1.63 | 89.9 | 83.1-94.6 |
| 209687_at | CXCL12 | 1.54E-14 | 2.4322 | 3.79 | 88.8 | 81.7-93.8 |
| 218546_at | C1orf115 | 1.95E-14 | 2.4106 | 2.95 | 88.6 | 81.4-93.6 |
| 205097_at | SLC26A2 | 2.08E-14 | 2.4219 | 7.83 | 88.7 | 81.5-93.7 |
| 224959_at | SLC26A2 | 2.08E-14 | 2.4229 | 7.83 | 88.7 | 81.6-93.7 |
| 224963_at | SLC26A2 | 2.08E-14 | 2.4205 | 7.83 | 88.7 | 81.5-93.7 |
| 204069_at | MEIS1 | 2.49E-14 | 2.3759 | 5.44 | 88.3 | 81-93.4 |
| 223121_s_at | SFRP2 | 3.05E-14 | 2.3937 | 7.39 | 88.4 | 81.2-93.5 |
| 223122_s_at | SFRP2 | 3.05E-14 | 2.3967 | 7.39 | 88.5 | 81.2-93.5 |
| 209191_at | TUBB3:MC1 R:TUBB6 | 3.55E-14 | 2.3684 | 5.9 | 88.2 | 80.9-93.3 |
| 201348_at | GPX3 | 5.68E-14 | 2.36 | 2.57 | 88.1 | 80.8-93.2 |
| 214091_s_at | GPX3 | 5.68E-14 | 2.3613 | 2.57 | 88.1 | 80.7-93.3 |
| 228766_at | CD36 | 6.27E-14 | 2.4671 | 1.65 | 89.1 | 82.1-94 |
| 221896_s_at | HIGD1A | 6.29E-14 | 2.3985 | 2.09 | 88.5 | 81.3-93.5 |
| 201865_x_at | NR3C1 | 7.65E-14 | 2.3539 | 2.51 | 88 | 80.6-93.2 |
| 211671_s_at | NR3C1 | 7.65E-14 | 2.3517 | 2.51 | 88 | 80.7-93.2 |
| 206149_at | LOC63928 | 8.09E-14 | 2.3513 | 3.2 | 88 | 80.7-93.2 |
| 228846_at | MXD1 | 8.35E-14 | 2.4074 | 2.11 | 88.6 | 81.4-93.6 |
| 225602_at | C9orf19 | 1.74E-13 | 2.3619 | 1.95 | 88.1 | 80.9-93.3 |
| 225604_s_at | C9orf19 | 1.74E-13 | 2.3646 | 1.95 | 88.1 | 80.8-93.3 |
| 201893_x_at | DCN | 2.05E-13 | 2.311 | 5.62 | 87.6 | 80.2-92.9 |
| 209335_at | DCN | 2.05E-13 | 2.3115 | 5.62 | 87.6 | 80.3-92.9 |
| 211813_x_at | DCN | 2.05E-13 | 2.3145 | 5.62 | 87.6 | 80.2-92.9 |
| 211896_s_at | DCN | 2.05E-13 | 2.3124 | 5.62 | 87.6 | 80.2-92.9 |
| 204818_at | HSD17B2 | 2.32E-13 | 2.196 | 4.76 | 86.4 | 78.7-92 |
| 204931_at | TCF21 | 2.51E-13 | 2.306 | 2.34 | 87.6 | 80.1-92.9 |
| 204438_at | MRC1 | 2.72E-13 | 2.3105 | 2.6 | 87.6 | 80.2-92.9 |
| 206262_at | ADH1A:ADH1C | 2.94E-13 | 2.3719 | 3.1 | 88.2 | 80.9-93.3 |
| 205433_at | BCHE | 3.18E-13 | 2.2514 | 6.21 | 87 | 79.5-92.5 |
| 225242_s_at | CCDC80 | 3.60E-13 | 2.2316 | 3.67 | 86.8 | 79.2-92.3 |
| 207980_s_at | CITED2 | 5.29E-13 | 2.2709 | 1.64 | 87.2 | 79.7-92.6 |
| 209357_at | CITED2 | 5.29E-13 | 2.2704 | 1.64 | 87.2 | 79.7-92.6 |
| 208383_s_at | PCK1 | 5.74E-13 | 2.3242 | 3.6 | 87.7 | 80.4-93 |
| 206385_s_at | ANK3:LOC729184:LOC7311 86 | 7.46E-13 | 2.2305 | 2.45 | 86.8 | 79.2-92.2 |
| 203305_at | F13A1 | 8.82E-13 | 2.1821 | 4.74 | 86.2 | 78.6-91.8 |
| 206134_at | ADAMDEC1 | 9.04E-13 | 2.2607 | 3.19 | 87.1 | 79.5-92.5 |
| 215118_s_at | AHNAK:IGHG1 | 9.41E-13 | 2.3277 | 1.73 | 87.8 | 80.3-93 |
| 217022_s_at | AHNAK:IGHG1 | 9.41E-13 | 2.3254 | 1.73 | 87.8 | 80.4-93 |
| 223395_at | ABI3BP | 9.47E-13 | 2.2207 | 3.62 | 86.7 | 79-92.2 |
| 225626_at | PAG1 | 1.11E-12 | 2.2136 | 2.68 | 86.6 | 78.9-92.1 |
| 213953_at | KRT20 | 1.26E-12 | 2.0964 | 5.49 | 85.3 | 77.4-91.1 |
| 226594_at | ENTPD5 | 1.43E-12 | 2.1687 | 3.01 | 86.1 | 78.4-91.8 |
| 209373_at | MALL | 2.10E-12 | 2.1597 | 3.96 | 86 | 78.3-91.7 |
| 212713_at | MFAP4 | 2.96E-12 | 2.1797 | 2.51 | 86.2 | 78.5-91.9 |
| 208920_at | SRI | 3.03E-12 | 2.0982 | 2.43 | 85.3 | 77.4-91.1 |
| 201739_at | SGK | 3.12E-12 | 2.1221 | 4.08 | 85.6 | 77.8-91.3 |
| 214696_at | MGC14376 | 3.39E-12 | 2.1045 | 2.58 | 85.4 | 77.5-91.2 |
| 204034_at | ETHE1 | 3.46E-12 | 2.2127 | 1.75 | 86.6 | 79-92.1 |
| 209667_at | CES2 | 3.51E-12 | 2.102 | 2.87 | 85.3 | 77.4-91.2 |
| 209668_x_at | CES2 | 3.51E-12 | 2.0978 | 2.87 | 85.3 | 77.4-91.1 |
| 213509_x_at | CES2 | 3.51E-12 | 2.097 | 2.87 | 85.3 | 77.4-91.1 |
| 202291_s_at | MGP:C12orf46 | 3.63E-12 | 2.1448 | 4.68 | 85.8 | 78-91.5 |
| 209167_at | GPM6B | 4.04E-12 | 2.1568 | 3.59 | 86 | 78.2-91.6 |
| 209170_s_at | GPM6B | 4.04E-12 | 2.1581 | 3.59 | 86 | 78.2-91.6 |
| 225720_at | SYNPO2 | 5.07E-12 | 2.1053 | 6.43 | 85.4 | 77.5-91.2 |
| 225721_at | SYNPO2 | 5.07E-12 | 2.1069 | 6.43 | 85.4 | 77.5-91.2 |
| 225894_at | SYNPO2 | 5.07E-12 | 2.1043 | 6.43 | 85.4 | 77.5-91.2 |
| 225895_at | SYNPO2 | 5.07E-12 | 2.1069 | 6.43 | 85.4 | 77.5-91.2 |
| 227662_at | SYNPO2 | 5.07E-12 | 2.1043 | 6.43 | 85.4 | 77.5-91.2 |
| 206422_at | GCG | 5.58E-12 | 2.1185 | 12.56 | 85.5 | 77.7-91.3 |
| 205593_s_at | PDE9A | 6.56E-12 | 2.1443 | 3.91 | 85.8 | 78-91.5 |
| 220376_at | LRRC19 | 6.75E-12 | 2.076 | 4.51 | 85 | 77.1-91 |
| 204130_at | HSD11B2 | 6.97E-12 | 2.0896 | 2.53 | 85.2 | 77.3-91.1 |
| 224964_s_at | GNG2 | 7.25E-12 | 2.1405 | 1.91 | 85.8 | 78-91.5 |
| 219508_at | GCNT3 | 8.39E-12 | 2.0843 | 3.94 | 85.1 | 77.2-91 |
| 211645_x_at | No Symbol | 9.19E-12 | 2.1536 | 1.61 | 85.9 | 78.2-91.6 |
| 212233_at | No Symbol | 9.19E-12 | 2.1541 | 1.61 | 85.9 | 78.1-91.6 |
| 212764_at | No Symbol | 9.19E-12 | 2.1541 | 1.61 | 85.9 | 78.1-91.6 |
| 214777_at | No Symbol | 9.19E-12 | 2.1543 | 1.61 | 85.9 | 78.2-91.6 |
| 217235_x_at | No Symbol | 9.19E-12 | 2.155 | 1.61 | 85.9 | 78.2-91.6 |
| 225710_at | No Symbol | 9.19E-12 | 2.1539 | 1.61 | 85.9 | 78.1-91.6 |
| 226333_at | No Symbol | 9.19E-12 | 2.1529 | 1.61 | 85.9 | 78.1-91.6 |
| 226834_at | No Symbol | 9.19E-12 | 2.154 | 1.61 | 85.9 | 78.1-91.6 |
| 227061_at | No Symbol | 9.19E-12 | 2.152 | 1.61 | 85.9 | 78.1-91.6 |
| 228504_at | No Symbol | 9.19E-12 | 2.1533 | 1.61 | 85.9 | 78.1-91.6 |
| 228507_at | No Symbol | 9.19E-12 | 2.1538 | 1.61 | 85.9 | 78.2-91.6 |
| 228640_at | No Symbol | 9.19E-12 | 2.1532 | 1.61 | 85.9 | 78.2-91.6 |
| 228854_at | No Symbol | 9.19E-12 | 2.1532 | 1.61 | 85.9 | 78.2-91.7 |
| 236300_at | No Symbol | 9.19E-12 | 2.1532 | 1.61 | 85.9 | 78.2-91.6 |
| 242317_at | No Symbol | 9.19E-12 | 2.1529 | 1.61 | 85.9 | 78.1-91.6 |
| 210524_x_at | No Symbol | 9.19E-12 | 2.1554 | 1.61 | 85.9 | 78.2-91.7 |
| 224989_at | No Symbol | 9.19E-12 | 2.1526 | 1.61 | 85.9 | 78.2-91.6 |
| 227052_at | No Symbol | 9.19E-12 | 2.151 | 1.61 | 85.9 | 78.1-91.6 |
| 227682_at | No Symbol | 9.19E-12 | 2.1549 | 1.61 | 85.9 | 78.2-91.6 |
| 235146_at | No Symbol | 9.19E-12 | 2.1527 | 1.61 | 85.9 | 78.2-91.7 |
| 207126_x_at | UGT1A10:UGT1A7:UGT1A8: UGT1A1:UGT1A9:UGT1A6: UGT1A5:UGT1A3:UGT1A4 | 9.98E-12 | 2.0677 | 4.56 | 84.9 | 77-90.9 |
| 206094_x_at | UGT1A10:UGT1A7:UGT1A8: UGT1A1:UGT1A9:UGT1A6: UGT1A5:UGT1A3:UGT1A4 | 1.01E-11 | 2.0693 | 4.56 | 85 | 77-90.8 |
| 208596_s_at | UGT1A10:UGT1A7:UGT1A8: UGT1A1:UGT1A9:UGT1A6: UGT1A5:UGT1A3:UGT1A4 | 1.01E-11 | 2.0662 | 4.56 | 84.9 | 76.9-90.8 |
| 221305_s_at | UGT1A10:UGT1A7:UGT1A8: UGT1A1:UGT1A9:UGT1A6: UGT1A5:UGT1A3:UGT1A4 | 1.03E-11 | 2.0663 | 4.56 | 84.9 | 77-90.9 |
| 204532_x_at | UGT1A10:UGT1A7:UGT1A8: UGT1A1:UGT1A9:UGT1A6: UGT1A5:UGT1A3:UGT1A4 | 1.18E-11 | 2.0678 | 4.56 | 84.9 | 77-90.9 |
| 215125_s_at | UGT1A10:UGT1A7:UGT1A8: UGT1A1:UGT1A9:UGT1A6: UGT1A5:UGT1A3:UGT1A4 | 1.18E-11 | 2.0682 | 4.56 | 84.9 | 77-90.9 |
| 209791_at | PADI2 | 1.46E-11 | 2.0258 | 3.98 | 84.4 | 76.4-90.5 |
| 219669_at | CD177 | 1.49E-11 | 2.0618 | 5.77 | 84.9 | 76.9-90.8 |
| 201539_s_at | FHL1 | 1.63E-11 | 2.0448 | 3.25 | 84.7 | 76.7-90.7 |
| 201540_at | FHL1 | 1.63E-11 | 2.0466 | 3.25 | 84.7 | 76.7-90.7 |
| 210298_x_at | FHL1 | 1.63E-11 | 2.0438 | 3.25 | 84.7 | 76.7-90.6 |
| 210299_s_at | FHL1 | 1.63E-11 | 2.0458 | 3.25 | 84.7 | 76.6-90.6 |
| 214505_s_at | FHL1 | 1.63E-11 | 2.0459 | 3.25 | 84.7 | 76.7-90.6 |
| 206576_s_at | CEACAM1 | 2.66E-11 | 2.0448 | 2.85 | 84.7 | 76.7-90.7 |
| 209498_at | CEACAM1 | 2.66E-11 | 2.0446 | 2.85 | 84.7 | 76.7-90.7 |
| 211889_x_at | CEACAM1 | 2.66E-11 | 2.0471 | 2.85 | 84.7 | 76.7-90.7 |
| 202994_s_at | FBLN1 | 2.82E-11 | 2.0183 | 3.55 | 84.4 | 76.3-90.4 |
| 202995_s_at | FBLN1 | 2.82E-11 | 2.0191 | 3.55 | 84.4 | 76.3-90.4 |
| 201427_s_at | SEPP1 | 2.83E-11 | 2.034 | 4.15 | 84.5 | 76.5-90.6 |
| 212956_at | TBC1D9 | 3.89E-11 | 2.1428 | 1.54 | 85.8 | 78-91.5 |
| 203963_at | CA12 | 4.32E-11 | 2.0024 | 2.65 | 84.2 | 76.1-90.2 |
| 204508_s_at | CA12 | 4.32E-11 | 2.0029 | 2.65 | 84.2 | 76.2-90.3 |
| 210735_s_at | CA12 | 4.32E-11 | 2.0016 | 2.65 | 84.2 | 76.1-90.2 |
| 214164_x_at | CA12 | 4.32E-11 | 2.0016 | 2.65 | 84.2 | 76.1-90.3 |
| 215867_x_at | CA12 | 4.32E-11 | 2.0027 | 2.65 | 84.2 | 76.1-90.3 |
| 203881_s_at | DMD | 5.01E-11 | 2.0159 | 4.74 | 84.3 | 76.2-90.4 |
| 213624_at | SMPDL3A | 5.84E-11 | 1.9409 | 3.14 | 83.4 | 75.2-89.6 |
| 226304_at | HSPB6 | 6.16E-11 | 1.9486 | 6.88 | 83.5 | 75.4-89.8 |
| 206561_s_at | LOC441282:AKR1B10:LOC3 40888 | 7.91E-11 | 1.9478 | 5.17 | 83.5 | 75.3-89.7 |
| 203343_at | UGDH | 8.72E-11 | 1.9619 | 2.4 | 83.7 | 75.6-89.9 |
| 205892_s_at | FABP1 | 9.02E-11 | 1.9694 | 6.54 | 83.8 | 75.6-89.9 |
| 206637_at | P2RY14 | 1.31E-10 | 1.9821 | 2 | 83.9 | 75.8-90.1 |
| 202266_at | TTRAP | 1.59E-10 | 1.9147 | 2.2 | 83.1 | 74.9-89.4 |
| 206000_at | LOC642840:MEP1A:LOC389 747:LOC644777 | 2.07E-10 | 1.9873 | 3.17 | 84 | 75.9-90.1 |
| 201496_x_at | MYH11 | 2.15E-10 | 1.8577 | 5.35 | 82.4 | 74-88.8 |
| 201497_x_at | MYH11 | 2.15E-10 | 1.8599 | 5.35 | 82.4 | 74.1-88.8 |
| 207961_x_at | MYH11 | 2.15E-10 | 1.859 | 5.35 | 82.4 | 74.1-88.9 |
| 201495_x_at | MYH11 | 2.15E-10 | 1.8585 | 5.35 | 82.4 | 74-88.8 |
| 204388_s_at | MAOA | 2.49E-10 | 2.0297 | 2.18 | 84.5 | 76.4-90.5 |
| 204389_at | MAOA | 2.49E-10 | 2.0256 | 2.18 | 84.4 | 76.5-90.5 |
| 212741_at | MAOA | 2.49E-10 | 2.0267 | 2.18 | 84.5 | 76.5-90.5 |
| 214598_at | CLDN8 | 2.92E-10 | 1.862 | 12.96 | 82.4 | 74.2-88.9 |
| 202838_at | FUCA1 | 4.24E-10 | 1.8831 | 2.03 | 82.7 | 74.4-89.1 |
| 217897_at | MB:FXYD6 | 6.39E-10 | 1.8109 | 2.92 | 81.7 | 73.3-88.3 |
| 220468_at | ARL14 | 7.01E-10 | 1.8242 | 3.06 | 81.9 | 73.5-88.4 |
| 201920_at | SLC20A1 | 7.65E-10 | 1.7849 | 4.04 | 81.4 | 72.9-88.1 |
| 210302_s_at | MAB21L2 | 1.05E-09 | 1.7744 | 5.2 | 81.3 | 72.8-87.9 |
| 209114_at | TSPAN1 | 1.16E-09 | 1.7896 | 2.16 | 81.5 | 73-88.1 |
| 220266_s_at | KLF4 | 1.19E-09 | 1.8515 | 2.27 | 82.3 | 73.8-88.7 |
| 221841_s_at | KLF4 | 1.19E-09 | 1.8493 | 2.27 | 82.2 | 73.9-88.8 |
| 209283_at | CRYAB | 1.47E-09 | 1.7693 | 3.91 | 81.2 | 72.7-87.8 |
| 223484_at | C15orf48 | 1.54E-09 | 1.8189 | 3.07 | 81.8 | 73.4-88.4 |
| 205412_at | ACAT1 | 1.70E-09 | 1.8575 | 1.77 | 82.3 | 73.9-88.8 |
| 202888_s_at | ANPEP | 2.04E-09 | 1.7673 | 8.18 | 81.2 | 72.6-87.8 |
| 225458_at | EXOC3 | 2.25E-09 | 1.7957 | 3.17 | 81.5 | 73.1-88.1 |
| 204834_at | FGL2 | 2.64E-09 | 1.8245 | 2.07 | 81.9 | 73.4-88.5 |
| 227265_at | FGL2 | 2.64E-09 | 1.8246 | 2.07 | 81.9 | 73.6-88.4 |
| 228469_at | PPID | 3.34E-09 | 1.8032 | 1.73 | 81.6 | 73.2-88.3 |
| 221004_s_at | ITM2C | 3.87E-09 | 1.7063 | 2.22 | 80.3 | 71.8-87.1 |
| 213921_at | SST | 4.16E-09 | 1.7652 | 6.14 | 81.1 | 72.6-87.8 |
| 230087_at | PRIMA1 | 4.74E-09 | 1.7085 | 2.75 | 80.4 | 71.8-87.2 |
| 201842_s_at | EFEMP1 | 5.80E-09 | 1.8196 | 1.69 | 81.9 | 73.4-88.4 |
| 222162_s_at | ADAMTS1 | 6.42E-09 | 1.7467 | 2.37 | 80.9 | 72.3-87.6 |
| 210517_s_at | AKAP12 | 7.41E-09 | 1.7454 | 2.83 | 80.9 | 72.3-87.6 |
| 227529_s_at | AKAP12 | 7.41E-09 | 1.7437 | 2.83 | 80.8 | 72.3-87.6 |
| 228750_at | COL14A1 | 7.60E-09 | 1.7813 | 1.67 | 81.3 | 72.9-88 |
| 219948_x_at | LOC642329:UGT2A3 | 8.64E-09 | 1.7422 | 4.89 | 80.8 | 72.2-87.5 |
| 208131_s_at | PTGIS | 9.15E-09 | 1.7212 | 3.98 | 80.5 | 71.9-87.3 |
| 207432_at | BTN2A2:BEST2 | 9.19E-09 | 1.7179 | 3.63 | 80.5 | 71.9-87.3 |
| 219607_s_at | MS4A4A:LOC643680 | 9.59E-09 | 1.8197 | 1.55 | 81.9 | 73.4-88.4 |
| 204688_at | SGCE | 1.02E-08 | 1.7438 | 2.03 | 80.8 | 72.3-87.6 |
| 207134_x_at | TPSB2 | 1.24E-08 | 1.3713 | 1.87 | 75.4 | 66.3-82.9 |
| 209156_s_at | COL6A2 | 1.30E-08 | 1.68 | 2.08 | 80 | 71.3-86.8 |
| 202741_at | PRKACB | 1.30E-08 | 1.7217 | 2.18 | 80.5 | 71.9-87.3 |
| 202742_s_at | PRKACB | 1.30E-08 | 1.721 | 2.18 | 80.5 | 71.9-87.3 |
| 200795_at | SPARCL1 | 1.33E-08 | 1.6379 | 2.85 | 79.4 | 70.7-86.3 |
| 219543_at | PBLD | 1.38E-08 | 1.6981 | 2.74 | 80.2 | 71.6-87.1 |
| 225207_at | PDK4 | 1.45E-08 | 1.705 | 3.02 | 80.3 | 71.7-87.2 |
| 202222_s_at | SPEG:LOC729871:DES | 1.47E-08 | 1.7229 | 6.66 | 80.6 | 72-87.3 |
| 214027_x_at | SPEG:LOC729871:DES | 1.47E-08 | 1.7254 | 6.66 | 80.6 | 72-87.4 |
| 239272_at | MMP28 | 1.80E-08 | 1.6622 | 2.47 | 79.7 | 71-86.6 |
| 222453_at | THEM4:CYBRD1 | 1.84E-08 | 1.6934 | 2.7 | 80.1 | 71.6-87 |
| 212195_at | MAGEA4:IL6ST | 1.87E-08 | 1.764 | 1.58 | 81.1 | 72.6-87.8 |
| 203980_at | FABP4 | 2.01E-08 | 1.6762 | 5.55 | 79.9 | 71.3-86.8 |
| 211985_s_at | CALM1 | 2.12E-08 | 1.6839 | 1.72 | 80 | 71.4-86.9 |
| 221747_at | TNS1:AKAP9 | 2.21E-08 | 1.7434 | 2.01 | 80.8 | 72.2-87.5 |
| 221748_s_at | TNS1:AKAP9 | 2.21E-08 | 1.7409 | 2.01 | 80.8 | 72.3-87.5 |
| 201324_at | EMP1 | 2.59E-08 | 1.7149 | 1.95 | 80.4 | 71.9-87.3 |
| 212397_at | LOC643244:RDX | 3.06E-08 | 1.7431 | 1.9 | 80.8 | 72.3-87.6 |
| 205382_s_at | CFD | 3.06E-08 | 1.6462 | 2.81 | 79.5 | 70.8-86.4 |
| 201141_at | GPNMB | 3.11E-08 | 1.6928 | 1.99 | 80.1 | 71.5-87 |
| 205683_x_at | TPSAB1,TPSAB1 | 3.52E-08 | 1.7075 | 1.93 | 80.3 | 71.8-87.1 |
| 210084_x_at | TPSAB1,TPSAB1 | 3.52E-08 | 1.7045 | 1.93 | 80.3 | 71.8-87.1 |
| 215382_x_at | TPSAB1,TPSAB1 | 3.52E-08 | 1.7091 | 1.93 | 80.4 | 71.7-87.2 |
| 216474_x_at | TPSAB1,TPSAB1 | 3.52E-08 | 1.7069 | 1.93 | 80.3 | 71.7-87.2 |
| 227006_at | PPP1R14A | 3.54E-08 | 1.619 | 2.51 | 79.1 | 70.3-86.1 |
| 242601_at | LOC253012 | 3.59E-08 | 1.6517 | 4.46 | 79.6 | 70.8-86.5 |
| 212730_at | DMN | 4.36E-08 | 1.6046 | 4.05 | 78.9 | 70.2-86 |
| 226818_at | MPEG1 | 4.58E-08 | 1.6466 | 1.86 | 79.5 | 70.8-86.5 |
| 226841_at | MPEG1 | 4.58E-08 | 1.6484 | 1.86 | 79.5 | 70.8-86.5 |
| 203474_at | IQGAP2 | 5.06E-08 | 1.6275 | 2.51 | 79.2 | 70.6-86.2 |
| 203766_s_at | LMOD1 | 5.55E-08 | 1.58 | 2.97 | 78.5 | 69.8-85.6 |
| 214916_x_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 5.75E-08 | 1.6352 | 3.1 | 79.3 | 70.6-86.3 |
| 226303_at | PGM5 | 5.87E-08 | 1.6215 | 5.87 | 79.1 | 70.4-86.2 |
| 225442_at | DDR2 | 5.89E-08 | 1.562 | 2.61 | 78.3 | 69.4-85.4 |
| 227561_at | DDR2 | 5.89E-08 | 1.5604 | 2.61 | 78.2 | 69.4-85.5 |
| 202760_s_at | AKAP2:PALM2:PALM2-AKAP2 | 6.28E-08 | 1.6137 | 2.17 | 79 | 70.3-86.1 |
| 226694_at | AKAP2:PALM2:PALM2-AKAP2 | 6.28E-08 | 1.6143 | 2.17 | 79 | 70.2-86.1 |
| 204894_s_at | AOC3 | 6.43E-08 | 1.556 | 2.92 | 78.2 | 69.4-85.4 |
| 203058_s_at | PAPSS2 | 6.43E-08 | 1.6171 | 1.97 | 79.1 | 70.4-86.1 |
| 203060_s_at | PAPSS2 | 6.43E-08 | 1.6159 | 1.97 | 79 | 70.4-86.1 |
| 208763_s_at | TSC22D3 | 7.22E-08 | 1.6098 | 2.76 | 79 | 70.2-86 |
| 222717_at | SDPR | 9.62E-08 | 1.5998 | 2.21 | 78.8 | 70.1-85.9 |
| 203680_at | PRKAR2B | 1.05E-07 | 1.6759 | 2.1 | 79.9 | 71.2-86.8 |
| 201041_s_at | DUSP1 | 1.06E-07 | 1.5295 | 2.44 | 77.8 | 68.9-85 |
| 209374_s_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 1.12E-07 | 1.6333 | 3.1 | 79.3 | 70.6-86.3 |
| 216491_x_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 1.12E-07 | 1.6335 | 3.1 | 79.3 | 70.5-86.3 |
| 229831_at | CNTN3 | 1.20E-07 | 1.6157 | 3.93 | 79 | 70.2-86.1 |
| 235766_x_at | RAB27A | 1.22E-07 | 1.5984 | 1.79 | 78.8 | 70.1-85.9 |
| 229070_at | C6orf105 | 1.38E-07 | 1.6049 | 2.13 | 78.9 | 70.1-86 |
| 226654_at | MUC 12 | 1.53E-07 | 1.4559 | 4.89 | 76.7 | 67.7-84.1 |
| 202686_s_at | AXL | 1.59E-07 | 1.5758 | 1.71 | 78.5 | 69.7-85.6 |
| 205403_at | IL1R2 | 1.73E-07 | 1.5237 | 2.54 | 77.7 | 68.8-84.9 |
| 211372_s_at | IL1R2 | 1.73E-07 | 1.5229 | 2.54 | 77.7 | 68.7-85 |
| 205929_at | GPA33 | 1.86E-07 | 1.7056 | 1.61 | 80.3 | 71.7-87.2 |
| 202069_s_at | IDH3A | 2.08E-07 | 1.5696 | 1.75 | 78.4 | 69.5-85.5 |
| 202350_s_at | MATN2 | 2.11E-07 | 1.506 | 2.23 | 77.4 | 68.4-84.7 |
| 212859_x_at | NUTF2:MT1E:MT1M | 2.14E-07 | 1.6442 | 2.41 | 79.4 | 70.8-86.4 |
| 217546_at | NUTF2:MT1E:MT1M | 2.14E-07 | 1.645 | 2.41 | 79.5 | 70.7-86.5 |
| 216336_x_at | NUTF2:MT1E:MT1M | 2.14E-07 | 1.645 | 2.41 | 79.5 | 70.8-86.4 |
| 221667_s_at | HSPB8 | 2.61E-07 | 1.514 | 3.85 | 77.5 | 68.6-84.8 |
| 217757_at | A2M | 2.72E-07 | 1.598 | 1.64 | 78.8 | 70.1-85.9 |
| 216510_x_at | LOC652128:IGHG1:IGHM:IG HV4-31:LOC647189:IGHV1-69:IGHA1:IL8:EXOC7:SIX6:I GHD:IGH@:IGHG3:CI2orf32 :ZCWPW2:IFI6:IGHG4:IGHA 2:IGHG2:RAC1 | 2.80E-07 | 1.6346 | 3.1 | 79.3 | 70.6-86.4 |
| 223343_at | NYD-SP21:MS4A7 | 2.92E-07 | 1.5396 | 2.27 | 77.9 | 69.1-85.1 |
| 202620_s_at | PLOD2 | 3.06E-07 | 1.5551 | 2.33 | 78.2 | 69.3-85.4 |
| 207245_at | UGT2B17 | 3.75E-07 | 1.4796 | 4.88 | 77 | 68.1-84.5 |
| 210139_s_at | PMP22 | 3.97E-07 | 1.5183 | 2.23 | 77.6 | 68.7-84.9 |
| 204938_s_at | PLN | 4.14E-07 | 1.5454 | 2.01 | 78 | 69.1-85.2 |
| 204939_s_at | PLN | 4.14E-07 | 1.5425 | 2.01 | 78 | 69.1-85.2 |
| 204940_at | PLN | 4.14E-07 | 1.5436 | 2.01 | 78 | 69.1-85.2 |
| 203951_at | CNN1 | 4.77E-07 | 1.4324 | 4.56 | 76.3 | 67.3-83.8 |
| 202746_at | ITM2A | 5.27E-07 | 1.4988 | 2.77 | 77.3 | 68.4-84.6 |
| 221584_s_at | KCNMA1 | 6.86E-07 | 1.4959 | 2.37 | 77.3 | 68.4-84.6 |
| 241994_at | XDH | 8.13E-07 | 1.4709 | 2.03 | 76.9 | 67.9-84.3 |
| 209621_s_at | PDLIM3 | 8.20E-07 | 1.4805 | 2.38 | 77 | 68-84.4 |
| 204326_x_at | LOC645652:MT1X | 9.84E-07 | 1.4427 | 4.35 | 76.5 | 67.4-83.9 |
| 208581_x_at | LOC645652:MT1X | 9.84E-07 | 1.4397 | 4.35 | 76.4 | 67.5-83.9 |
| 201616_s_at | CALD1 | 1.02E-06 | 1.4583 | 3.29 | 76.7 | 67.8-84.1 |
| 201617_x_at | CALD1 | 1.02E-06 | 1.4617 | 3.29 | 76.8 | 67.7-84.1 |
| 212077_at | CALD1 | 1.02E-06 | 1.4581 | 3.29 | 76.7 | 67.8-84.1 |
| 225782_at | MSRB3 | 1.20E-06 | 1.4747 | 2.24 | 77 | 68-84.3 |
| 209436_at | SPON1 | 1.46E-06 | 1.4849 | 1.88 | 77.1 | 68.1-84.4 |
| 223623_at | C2orf40 | 1.69E-06 | 1.416 | 2.97 | 76.1 | 67-83.5 |
| 218087_s_at | SORBS1:KIAA0894 | 2.18E-06 | 1.4259 | 2.54 | 76.2 | 67.3-83.7 |
| 222513_s_at | SORBS1:KIAA0894 | 2.18E-06 | 1.4263 | 2.54 | 76.2 | 67.2-83.7 |
| 224990_at | C4orf34 | 2.55E-06 | 1.3853 | 1.95 | 75.6 | 66.5-83.1 |
| 203131_at | FIP1L1:PDGFRA | 2.94E-06 | 1.4845 | 1.37 | 77.1 | 68.2-84.5 |
| 203638_s_at | FGFR2 | 3.55E-06 | 1.5287 | 1.31 | 77.8 | 68.9-85 |
| 201957_at | PPP1R12B:LOC731632 | 4.26E-06 | 1.3611 | 2.21 | 75.2 | 66.2-82.8 |
| 217967_s_at | FAM129A | 4.59E-06 | 1.4131 | 1.8 | 76 | 67-83.5 |
| 210809_s_at | POSTN | 4.94E-06 | 1.3954 | 2.25 | 75.7 | 66.7-83.2 |
| 226302_at | ATP8B1 | 5.73E-06 | 1.313 | 1.67 | 74.4 | 65.3-82.1 |
| 238750_at | CCL28 | 6.01E-06 | 1.4932 | 1.53 | 77.2 | 68.3-84.6 |
| 226103_at | NEXN | 6.83E-06 | 1.3666 | 2.23 | 75.3 | 66.2-82.8 |
| 209101_at | CTGF | 6.96E-06 | 1.4774 | 1.3 | 77 | 68.1-84.4 |
| 229254_at | MFSD4 | 7.19E-06 | 1.3235 | 2.12 | 74.6 | 65.4-82.3 |
| 219087_at | ASPN | 7.45E-06 | 1.3249 | 2.74 | 74.6 | 65.5-82.3 |
| 209457_at | DUSP5 | 8.60E-06 | 1.3336 | 1.91 | 74.8 | 65.7-82.4 |
| 221541_at | CRISPLD2 | 9.24E-06 | 1.3664 | 1.64 | 75.3 | 66.2-82.9 |
| 206377_at | FOXF2 | 9.50E-06 | 1.3032 | 2.08 | 74.3 | 65.1-82 |
| 207392_x_at | UGT2B11:LOC728160:UGT2 B15,UGT2B11:LOC728160:U GT2B15 | 9.73E-06 | 1.3478 | 5.72 | 75 | 65.8-82.6 |
| 202274_at | ACTG2 | 1.07E-05 | 1.4015 | 1.82 | 75.8 | 66.8-83.4 |
| 227522_at | CMBL | 1.09E-05 | 1.3588 | 1.58 | 75.2 | 66-82.8 |
| 212192_at | KCTD12 | 1.44E-05 | 1.3659 | 1.36 | 75.3 | 66.2-82.8 |
| 227727_at | MRGPRF | 1.52E-05 | 1.2965 | 2.99 | 74.2 | 65-82 |
| 234987_at | C20orf118 | 1.75E-05 | 1.4278 | 1.3 | 76.2 | 67.3-83.7 |
| 209656_s_at | TMEM47 | 1.86E-05 | 1.2983 | 2.02 | 74.2 | 65.1-82 |
| 212265_at | QKI | 1.93E-05 | 1.3443 | 1.31 | 74.9 | 65.9-82.6 |
| 228232_s_at | VSIG2 | 2.01E-05 | 1.2957 | 2.05 | 74.1 | 65-81.9 |
| 200799_at | HSPA1A,HSPA1A | 2.16E-05 | 1.272 | 2.41 | 73.8 | 64.6-81.6 |
| 218312_s_at | ZNF447 | 3.05E-05 | 1.107 | 1.39 | 71 | 61.6-79.1 |
| 230264_s_at | AP1S2 | 3.39E-05 | 1.2546 | 1.92 | 73.5 | 64.3-81.4 |
| 224840_at | FKBP5 | 3.41E-05 | 1.2371 | 1.71 | 73.2 | 63.9-81.1 |
| 209948_at | KCNMB1 | 3.74E-05 | 1.2181 | 2.66 | 72.9 | 63.6-80.8 |
| 201426_s_at | VIM | 4.15E-05 | 1.2221 | 1.86 | 72.9 | 63.7-80.8 |
| 227826_s_at | SORBS2 | 4.23E-05 | 1.3017 | 2.1 | 74.2 | 65.1-82 |
| 227827_at | SORBS2 | 4.23E-05 | 1.3015 | 2.1 | 74.2 | 65.1-82 |
| 225728_at | SORBS2 | 4.23E-05 | 1.3015 | 2.1 | 74.2 | 65.1-82 |
| 238751_at | SORBS2 | 4.23E-05 | 1.3019 | 2.1 | 74.2 | 65.1-82 |
| 228202_at | C6orf204 | 4.90E-05 | 1.2062 | 2.07 | 72.7 | 63.5-80.6 |
| 220645_at | FAM55D | 5.67E-05 | 1.1786 | 3.48 | 72.2 | 63-80.2 |
| 224560_at | TIMP2 | 6.40E-05 | 1.2304 | 1.82 | 73.1 | 63.9-80.9 |
| 231579_s_at | TIMP2 | 6.40E-05 | 1.2286 | 1.82 | 73 | 63.8-81 |
| 208788_at | ELOVL5 | 8.15E-05 | 1.1804 | 2.05 | 72.2 | 63-80.2 |
| 200974_at | ACTA2 | 1.00E-04 | 1.2299 | 1.81 | 73.1 | 63.8-81 |
| 202388_at | RGS2 | 0.0001 | 1.1604 | 1.94 | 71.9 | 62.7-80 |
| 206461_x_at | MT1A:MT1H:LOC645745:LO C727730 | 0.0001 | 1.189 | 4.55 | 72.4 | 63.2-80.4 |
| 208747_s_at | C1S:PRB1:PRB2,C1S:PRB2:P RB1 | 0.0001 | 1.2884 | 1.61 | 74 | 64.9-81.9 |
| 208791_at | CLU | 0.0001 | 1.2303 | 1.42 | 73.1 | 63.9-80.9 |
| 208792_s_at | CLU | 0.0001 | 1.2302 | 1.42 | 73.1 | 63.9-81 |
| 212185_x_at | NUTF2:LOC441019:MT2A:M T1M,NUTF2:LOC441019:MT 2A:MT1M | 0.0001 | 1.1894 | 1.89 | 72.4 | 63.1-80.3 |
| 222043_at | CLU | 0.0001 | 1.2306 | 1.42 | 73.1 | 63.9-81 |
| 227099_s_at | LOC387763 | 0.0001 | 1.1963 | 1.36 | 72.5 | 63.2-80.5 |
| 202766_s_at | FBN1 | 0.0002 | 1.1359 | 1.93 | 71.5 | 62.2-79.6 |
| 204745_x_at | NUTF2:MT1G | 0.0002 | 1.0888 | 2.66 | 70.7 | 61.3-78.9 |
| 218418_s_at | ANKRD25 | 0.0002 | 1.1139 | 1.56 | 71.1 | 61.9-79.3 |
| 201058_s_at | MYL9 | 0.0003 | 1.131 | 2.18 | 71.4 | 62.1-79.5 |
| 205547_s_at | TAGLN | 0.0003 | 1.0914 | 2.12 | 70.7 | 61.4-78.9 |
| 205935_at | FOXF1 | 0.0004 | 1.1743 | 1.28 | 72.1 | 62.9-80.1 |
| 208450_at | LGALS2 | 0.0004 | 1.0845 | 3.07 | 70.6 | 61.4-78.8 |
| 203748_x_at | RBMS1 | 0.0005 | 1.1435 | 1.55 | 71.6 | 62.3-79.7 |
| 207266_x_at | RBMS1 | 0.0005 | 1.1428 | 1.55 | 71.6 | 62.4-79.7 |
| 209868_s_at | RBMS1 | 0.0005 | 1.1437 | 1.55 | 71.6 | 62.3-79.7 |
| 225269_s_at | RBMS1 | 0.0005 | 1.1446 | 1.55 | 71.6 | 62.3-79.6 |
| 212158_at | SDC2 | 0.0006 | 1.0977 | 1.39 | 70.8 | 61.6-79.1 |
| 209209_s_at | PLEKHC1 | 0.0007 | 1.0309 | 1.82 | 69.7 | 60.3-77.9 |
| 209210_s_at | PLEKHC1 | 0.0007 | 1.0325 | 1.82 | 69.7 | 60.3-78 |
| 205554_s_at | ACTB:DNASE1L3 | 0.0007 | 1.0979 | 1.5 | 70.8 | 61.4-78.9 |
| 202283_at | SERPINF1 | 0.0009 | 1.0003 | 1.47 | 69.2 | 59.8-77.5 |
| 209496_at | RARRES2:LOC648925 | 0.0009 | 1.0318 | 1.6 | 69.7 | 60.3-77.9 |
| 224352_s_at | CFL2 | 0.0009 | 1.0586 | 1.75 | 70.2 | 60.8-78.4 |
| 224663_s_at | CFL2 | 0.0009 | 1.0575 | 1.75 | 70.2 | 60.8-78.4 |
| 214433_s_at | SELENBP1 | 0.0009 | 1.0574 | 1.7 | 70.1 | 60.8-78.3 |
| 212592_at | ENAM | 0.0011 | 1.0024 | 2.58 | 69.2 | 59.8-77.5 |
| 213629_x_at | MT1F | 0.0012 | 0.9664 | 2.2 | 68.6 | 59.2-76.9 |
| 217165_x_at | MT1F | 0.0012 | 0.9649 | 2.2 | 68.5 | 59.2-76.9 |
| 203645_s_at | CD163 | 0.0013 | 1.0231 | 1.9 | 69.6 | 60-77.9 |
| 215049_x_at | CD163 | 0.0013 | 1.0215 | 1.9 | 69.5 | 60.1-77.8 |
| 206641_at | TNFRSF17 | 0.0015 | 1.0206 | 2.08 | 69.5 | 60.1-77.8 |
| 227235_at | GUCY1A3 | 0.0015 | 1.0116 | 2.69 | 69.4 | 59.8-77.6 |
| 229530_at | GUCY1A3 | 0.0015 | 1.0115 | 2.69 | 69.3 | 60-77.6 |
| 212097_at | CAV1 | 0.0016 | 1.0199 | 1.58 | 69.5 | 60.1-77.7 |
| 200884_at | CKB | 0.0019 | 0.9758 | 2.27 | 68.7 | 59.2-77 |
| 202133_at | WWTR1 | 0.002 | 0.9917 | 1.48 | 69 | 59.5-77.4 |
| 204607_at | HMGCS2 | 0.0022 | 0.8777 | 2.67 | 67 | 57.5-75.5 |
| 218559_s_at | MAFB | 0.0034 | 0.9595 | 1.66 | 68.4 | 59-76.8 |
| 201061_s_at | STOM | 0.0035 | 1.0165 | 1.21 | 69.4 | 60-77.7 |
| 201743_at | CD14 | 0.0035 | 0.9627 | 1.58 | 68.5 | 59.1-76.9 |
| 204895_x_at | MUC4:TAF5L:LOC650855:L OC645744 | 0.0039 | 1.0448 | 1.2 | 69.9 | 60.6-78.2 |
| 217109_at | MUC4:TAF5L:LOC650855:L OC645744 | 0.0039 | 1.0458 | 1.2 | 69.9 | 60.6-78.1 |
| 217110_s_at | MUC4:TAF5L:LOC650855:L OC645744 | 0.0039 | 1.0453 | 1.2 | 69.9 | 60.5-78.2 |
| 203382_s_at | APOE | 0.0044 | 0.9388 | 1.56 | 68.1 | 58.7-76.5 |
| 206664_at | SI | 0.0054 | 0.9293 | 2.79 | 67.9 | 58.4-76.3 |
| 242447_at | LOC285382 | 0.0058 | 0.7401 | 1.26 | 64.4 | 54.9-73.2 |
| 209312_x_at | HLA-DRB1:HLA-DRB6:LOC731247:LOC73041 5:HLA-DRB4:HLA-DRB5:HLA-DRB3:LOC651845 | 0.0063 | 1.0636 | 1.51 | 70.3 | 60.9-78.5 |
| 215193_x_at | HLA-DRB1:HLA-DRB6:LOC731247:LOC73041 5:HLA-DRB4:HLA-DRB5:HLA-DRB3:LOC651845 | 0.0063 | 1.0646 | 1.51 | 70.3 | 60.9-78.5 |
| 224694_at | ANTXR1 | 0.0071 | 0.9531 | 1.29 | 68.3 | 58.8-76.7 |
| 226811_at | FAM46C | 0.0085 | 0.9163 | 1.38 | 67.7 | 58.1-76.1 |
| 201150_s_at | TIMP3 | 0.0091 | 0.9049 | 1.81 | 67.5 | 57.9-76 |
| 226682_at | LOC283666 | 0.0099 | 0.7784 | 1.22 | 65.1 | 55.7-73.8 |
| 218468_s_at | GREM1 | 0.0106 | 0.8585 | 3.03 | 66.6 | 57.2-75.2 |
| 218469_at | GREM1 | 0.0106 | 0.8579 | 3.03 | 66.6 | 57.1-75.2 |
| 203240_at | FCGBP:LOC651441:LOC652 599 | 0.0121 | 0.7868 | 1.99 | 65.3 | 55.8-73.9 |
| 211538_s_at | HSPA2 | 0.0123 | 0.8916 | 1.43 | 67.2 | 57.7-75.7 |
| 212091_s_at | COL6A1 | 0.0126 | 0.848 | 1.45 | 66.4 | 56.9-74.9 |
| 213428_s_at | COL6A1 | 0.0126 | 0.8474 | 1.45 | 66.4 | 56.9-74.9 |
| 226051_at | SELM | 0.0133 | 0.8606 | 1.32 | 66.7 | 57.1-75.1 |
| 200859_x_at | FLNA:WTAP | 0.0157 | 0.7975 | 1.59 | 65.5 | 55.9-74 |
| 213746_s_at | FLNA:WTAP | 0.0157 | 0.7959 | 1.59 | 65.5 | 56-74.2 |
| 214752_x_at | FLNA:WTAP | 0.0157 | 0.798 | 1.59 | 65.5 | 56-74.1 |
| 204570_at | COX7A1 | 0.0161 | 0.8622 | 1.32 | 66.7 | 57.2-75.2 |
| 203729_at | EMP3 | 0.0164 | 0.8043 | 1.41 | 65.6 | 56.1-74.3 |
| 223597_at | ITLN1 | 0.021 | 0.7148 | 2.54 | 64 | 54.4-72.7 |
| 227735_s_at | C10orf99 | 0.0218 | 0.7254 | 1.76 | 64.2 | 54.6-72.9 |
| 227736_at | C10orf99 | 0.0218 | 0.7259 | 1.76 | 64.2 | 54.6-72.9 |
| 200621_at | CSRP1 | 0.023 | 0.7246 | 1.39 | 64.1 | 54.6-72.9 |
| 204897_at | PTGER4:LOC730002:LOC73 0882 | 0.0237 | 0.8114 | 1.41 | 65.8 | 56.2-74.4 |
| 204083_s_at | PPIL5:TPM2 | 0.026 | 0.7531 | 1.53 | 64.7 | 55.2-73.4 |
| 211643_x_at | HLA-C:HLA-B:LOC730410:LOC652614:L OC732037 | 0.0268 | 0.9237 | 1.24 | 67.8 | 58.3-76.3 |
| 211644_x_at | HLA-C:HLA-B:LOC730410:LOC652614:L OC732037 | 0.0268 | 0.9229 | 1.24 | 67.8 | 58.3-76.2 |
| 214768_x_at | HLA-C:HLA-B:LOC730410:LOC652614:L OC732037 | 0.0268 | 0.9245 | 1.24 | 67.8 | 58.3-76.3 |
| 226147_s_at | PIGR | 0.0278 | 0.7282 | 1.59 | 64.2 | 54.6-73 |
| 229659_s_at | PIGR | 0.0278 | 0.7253 | 1.59 | 64.2 | 54.7-72.9 |
| 201300_s_at | PRNP | 0.0288 | 0.7985 | 1.24 | 65.5 | 56-74.1 |
| 210133_at | CCL11 | 0.0361 | 0.8534 | 1.32 | 66.5 | 57-75.1 |
| 225353_s_at | C1QC | 0.0391 | 0.7457 | 1.36 | 64.5 | 55-73.2 |
| 201289_at | CYR61 | 0.0425 | 0.7849 | 1.47 | 65.3 | 55.8-73.9 |
| 210764_s_at | CYR61 | 0.0425 | 0.7836 | 1.47 | 65.2 | 55.6-73.9 |
| 200986_at | SERPING1 | 0.0503 | 0.7672 | 1.44 | 64.9 | 55.4-73.6 |
| 204122_at | TYROBP:ZNF160 | 0.053 | 0.8181 | 1.17 | 65.9 | 56.4-74.5 |
| 201667_at | GJA1 | 0.0561 | 0.784 | 1.76 | 65.2 | 55.7-73.9 |
| 208789_at | PTRF | 0.0624 | 0.736 | 1.5 | 64.4 | 54.8-73.1 |
| 210107_at | CLCA1 | 0.0681 | 0.7868 | 1.61 | 65.3 | 55.7-73.9 |
| 200665_s_at | SPARC | 0.0684 | 0.6714 | 1.45 | 63.1 | 53.6-72.1 |
| 212667_at | SPARC | 0.0684 | 0.6732 | 1.45 | 63.2 | 53.6-71.9 |
| 211964_at | COL4A2 | 0.0738 | 0.7064 | 1.32 | 63.8 | 54.2-72.6 |
| 201858_s_at | PRG1 | 0.0972 | 0.5559 | 1.25 | 60.9 | 51.3-69.9 |
| 201859_at | PRG1 | 0.0972 | 0.5563 | 1.25 | 61 | 51.4-69.9 |
| 228241_at | BCMP11 | 0.102 | 0.4028 | 1.28 | 58 | 48.4-67.1 |
| 217762_s_at | RAB31 | 0.1041 | 0.8135 | 1.14 | 65.8 | 56.3-74.4 |
| 217764_s_at | RAB31 | 0.1041 | 0.8132 | 1.14 | 65.8 | 56.2-74.5 |
| 202007_at | NID1 | 0.105 | 0.7425 | 1.33 | 64.5 | 55-73.1 |
| 210495_x_at | FN1 | 0.1121 | 0.5756 | 1.73 | 61.3 | 51.8-70.2 |
| 211719_x_at | FN1 | 0.1121 | 0.5767 | 1.73 | 61.3 | 51.8-70.2 |
| 212464_s_at | FN1 | 0.1121 | 0.5765 | 1.73 | 61.3 | 51.6-70.3 |
| 216442_x_at | FN1 | 0.1121 | 0.5745 | 1.73 | 61.3 | 51.7-70.3 |
| 200600_at | MSN | 0.1499 | 0.7232 | 1.15 | 64.1 | 54.6-72.9 |
| 209138_x_at | IGL@:LOC651536:LOC73106 2:CPVL:IGLV2-14:IGLV4-3:IGLV3-25:IGLV3-21:IL8:RPL14 | 0.1551 | 0.681 | 1.26 | 63.3 | 53.8-72.2 |
| 216984_x_at | IGL@:LOC651536:LOC73106 2:CPVL:IGLV2-14:IGLV4-3:IGLV3-25:IGLV3-21:IL8:RPL14 | 0.1551 | 0.6808 | 1.26 | 63.3 | 53.8-72.2 |
| 217148_x_at | IGL@:LOC651536:LOC73106 2:CPVL:IGLV2-14:IGLV4-3:IGLV3-25:IGLV3-21:IL8:RPL14 | 0.1551 | 0.6808 | 1.26 | 63.3 | 53.9-72.1 |
| 201069_at | MMP2 | 0.1755 | 0.6214 | 1.24 | 62.2 | 52.6-71 |
| 202403_s_at | COL1A2:LOC728628 | 0.1891 | 0.6508 | 1.9 | 62.8 | 53.2-71.5 |
| 202768_at | FOSB | 0.2076 | 0.6392 | 2.17 | 62.5 | 52.9-71.5 |
| 209116_x_at | HBB | 0.2357 | 0.6432 | 1.28 | 62.6 | 52.9-71.4 |
| 211696_x_at | HBB | 0.2357 | 0.6436 | 1.28 | 62.6 | 53.1-71.4 |
| 217232_x_at | HBB | 0.2357 | 0.6444 | 1.28 | 62.6 | 53.1-71.5 |
| 205267_at | POU2AF1 | 0.2389 | 0.6573 | 1.18 | 62.9 | 53.3-71.7 |
| 201852_x_at | COL3A1 | 0.2551 | 0.5524 | 1.35 | 60.9 | 51.3-69.8 |
| 211161_s_at | COL3A1 | 0.2551 | 0.5509 | 1.35 | 60.9 | 51.3-69.9 |
| 215076_s_at | COL3A1 | 0.2551 | 0.5513 | 1.35 | 60.9 | 51.3-69.8 |
| 217378_x_at | LOC391427 | 0.2627 | 0.2759 | 1.15 | 55.5 | 45.9-64.8 |
| 223235_s_at | SMOC2 | 0.2701 | 0.656 | 1.34 | 62.9 | 53.2-71.7 |
| 204673_at | MUC5AC:LOC652741:MUC2 | 0.2802 | 0.8531 | 1.22 | 66.5 | 57-75.1 |
| 212414_s_at | Sep-06 | 0.3109 | 0.5714 | 1.21 | 61.2 | 51.7-70.2 |
| 201744_s_at | LUM | 0.3157 | 0.5425 | 1.54 | 60.7 | 51-69.7 |
| 202953_at | C1QB | 0.3668 | 0.4998 | 1.24 | 59.9 | 50.3-68.9 |
| 209651_at | TGFB1I1 | 0.4093 | 0.4932 | 1.35 | 59.7 | 50.2-68.8 |
| 212224_at | ALDH1A1 | 0.4232 | 0.3525 | 1.4 | 57 | 47.4-66.2 |
| 211596_s_at | LRIG1 | 0.4614 | 0.5988 | 1.26 | 61.8 | 52.2-70.7 |
| 218541_s_at | C8orf4 | 0.475 | 0.4838 | 1.44 | 59.6 | 50-68.5 |
| 208894_at | HLA-DRA:HLA-DQA1 ,HLA-DRA:HLA-DQA1, HLA-DRA:HLA-DQA1 | 0.4823 | 0.3234 | 1.29 | 56.4 | 46.8-65.6 |
| 210982_s_at | HLA-DRA:HLA-DQA1,HLA-DRA:HLA-DQA1 ,HLA-DRA:HLA-DQA1 | 0.4823 | 0.3228 | 1.29 | 56.4 | 46.8-65.6 |
| 227404_s_at | EGR1 | 0.4851 | 0.5387 | 1.38 | 60.6 | 51-69.6 |
| 216401_x_at | LOC652745 | 0.4864 | 0.1728 | 1.09 | 53.4 | 43.9-62.8 |
| 216207_x_at | IGKV1D-13 | 0.5177 | 0.16 | 1.1 | 53.2 | 43.6-62.6 |
| 203477_at | COL15A1 | 0.6601 | 0.5385 | 1.22 | 60.6 | 51.1-69.7 |
| 211959_at | IGFBP5 | 0.7663 | 0.3526 | 1.25 | 57 | 47.4-66.2 |
| 201105_at | LGALS1 | 0.8555 | 0.4634 | 1.16 | 59.2 | 49.6-68.2 |
| 201438_at | COL6A3 | 0.8739 | 0.4434 | 1.2 | 58.8 | 49.2-67.8 |
| 212671_s_at | HLA-DQA 1:HLA-DQA2,HLA-DQA 1:LOC731682:HLA-DQA2 | 0.8836 | 0.5398 | 1.28 | 60.6 | 51.1-69.7 |
| 227725_at | ST6GALNAC1 | 0.9452 | 0.2599 | 1.15 | 55.2 | 45.6-64.5 |
| 211990_at | HLA-DPA1,HLA-DPA1 | 0.9664 | 0.417 | 1.39 | 58.3 | 48.7-67.4 |
| 211991_s_at | HLA-DPA1,HLA-DPA1 | 0.9664 | 0.4166 | 1.39 | 58.3 | 48.7-67.4 |
| 217179_x_at | IGLV1-44 | 0.9777 | 0.2852 | 1.07 | 55.7 | 46.1-65 |
| 234764_x_at | IGLV1-44 | 0.9777 | 0.2847 | 1.07 | 55.7 | 46.1-64.9 |
| 224342_x_at | IGLV1-44 | 0.9777 | 0.2848 | 1.07 | 55.7 | 46.1-65 |
| 215176_x_at | NTN2L:IGKC:IGKV1-5:GJB6:HLA-C | 0.9858 | 0.4167 | 1.43 | 58.3 | 48.6-67.3 |
| 216576_x_at | NTN2L:IGKC:IGKV1-5:GJB6:HLA-C | 0.9858 | 0.4171 | 1.43 | 58.3 | 48.6-67.4 |
| 201645_at | TNC | 0.9905 | 0.3687 | 1.09 | 57.3 | 47.7-66.6 |
| 214414_x_at | HBA1:HBA2,HBA1:HBA2 | 0.9979 | 0.4707 | 1.21 | 59.3 | 49.7-68.3 |
| 204018_x_at | HBA1:HBA2,HBA1:HBA2 | 0.9979 | 0.4711 | 1.21 | 59.3 | 49.7-68.3 |
| 209458_x_at | HBA1:HBA2,HBA1:HBA2 | 0.9979 | 0.4727 | 1.21 | 59.3 | 49.7-68.4 |
| 211699_x_at | HBA1:HBA2,HBA1:HBA2 | 0.9979 | 0.4697 | 1.21 | 59.3 | 49.7-68.3 |
| 211745_x_at | HBA1:HBA2,HBA1:HBA2 | 0.9979 | 0.4708 | 1.21 | 59.3 | 49.8-68.3 |
| 217414_x_at | HBA1:HBA2,HBA1:HBA2 | 0.9979 | 0.469 | 1.21 | 59.3 | 49.7-68.4 |

**TABLE 2**

| **Gene Symbol** | **ValidPS_DOWN** | **Signif. FDR** | **D.val5** | **FC** | **Sens-Spec** | **CI (95)** |
|---|---|---|---|---|---|---|
| GCNT2 | 935239-HuGene_st:225205-HuGene_st:1026280-HuGene_st:668101-HuGene_st:1099985-HuGene_st:698568-HuGene_st:134540-HuGene_st:697147-HuGene_st:250092-HuGene_st:611927-HuGene_st:972833-HuGene_st:168891-HuGene_st:990860-HuGene_st:109287-HuGene_st:322116-HuGene_st:231019-HuGene_st:211020_at:959570-HuGene_st:858764-HuGene_st:215593_at:820195-HuGene_st:239606_at:41059-HuGene_st:669940-HuGene_st:215595_x_at:230788_at | 2.16E-27 | 3.9513 | 13.36 | 97.6 | 94.2-99.2 |
| GUCA2B | 276512-HuGene_st:1006871-HuGene_st:948364-HuGene_st:46575-HuGene_st:207502_at:436788-HuGene_st:485636-HuGene_st:60132-HuGene_st:608718-HuGene_st:779511-HuGene_st:132431-HuGene_st:974232-HuGene_st:425596-HuGene_st:308287-HuGene_st:800088-HuGene_st:827119-HuGene_st:233789-HuGene_st:623623-HuGene_st | 7.04E-25 | 3.6268 | 51.78 | 96.5 | 92.4-98.6 |
| GUCA1B | 207003_at | 7.32E-25 | 3.5625 | 10.7 | 96.3 | 91.9-98.5 |
| CA4 | 636392-HuGene_st:206209_s_at:_365396-HuGene_st:978203-HuGene_st:987169-HuGene_st:638557-HuGene_st:206208_at:1033858-HuGene_st:597808-HuGene_st:756864-HuGene_st:326439-HuGene_st:356096-HuGene_st:478435-HuGene_st:31642-HuGene_st:682778-HuGene_st:1006162-HuGene_st:673533-HuGene_st:491473-HuGene_st:822299-HuGene_st:871453-HuGene_st:209666-HuGene_st:465368-HuGene_ st:354896-HuGene_st | 1.73E-24 | 3.6291 | 10.41 | 96.5 | 92.4-98.7 |
| SLC4A4 | 810103-HuGene_st:634869-HuGene_st:210739_x_at:211494_s_at:268 096-HuGene_st:70822-HuGene_st:940785-HuGene_st:484105-HuGene_st:210738_s_at:203908_at:99375 -HuGene_st:238833-HuGene_st:149464-HuGene_st:874564-HuGene_st:161948-HuGene_st:501640-HuGene_st:65687-HuGene_st:886057-HuGene_st:495376-HuGene_st:247215-HuGene_st:847550-HuGene_st:190252-HuGene_st:244077-HuGene_st:477911-HuGene_st:904959-HuGene_st:218417-HuGene_st:1554027_a_at | 2.23E-23 | 3.6301 | 3.58 | 96.5 | 92.4-98.6 |
| AQP8 | 486167-HuGene_st:126752-HuGene_ st:93102-HuGene_st:1090469-HuGene_ st:980308-HuGene_st:107169-HuGene_st:829020-HuGene_st:1053234-HuGene_st:954102-HuGene_st:40331-HuGene_st:206784_at:990703-HuGene_st:459706-HuGene_st:863327-HuGene_st:965233-HuGene_st:459009-HuGene_st:180228-HuGene_st:944315-HuGene_st:814773-HuGene_st:810460-HuGene_ st:228837-HuGene_st | 2.98E-22 | 3.3311 | 10.82 | 95.2 | 90.3-97.9 |
| CA1 | 632246-HuGene_st:205950_s_at:382371-HuGene_st:1004598-HuGene_st:405837-HuGene_st:551543-HuGene_st:242482-HuGene_st:267733-HuGene_st:1074039-HuGene_st:107073-HuGene_st:485365-HuGene_st:254908-HuGene_st:257502-HuGene_st:333301-HuGene_st:180359-HuGene_st:696950-HuGene_st:588557-HuGene_st:381399-HuGene_st:495540-HuGene_ st:384192-HuGene_st:205949_at | 3.15E-22 | 3.3666 | 38.27 | 95.4 | 90.6-98 |
| ABCG2 | 236197-HuGene_st:10623-HuGene_st:1005470-HuGene_st:140860-HuGene_st:492719-HuGene_st:417387-HuGene_st:113831-HuGene_st:507347-HuGene_st:944251-HuGene_st:175319-HuGene_st:784920-HuGene_st:709551-HuGene_st:136344-HuGene_st:689536-HuGene_st:209735_at:167748-HuGene_st:759590-HuGene_st:805222-HuGene_st:945575-HuGene_st:294363-HuGene_st:958524-HuGene_st:402982-HuGene_st:301335-HuGene_st | 1.09E-21 | 3.2599 | 31.53 | 94.8 | 89.8-97.7 |
| OSTbeta | 552746-HuGene_st:57742-HuGene_st:461622-HuGene_st:965982-HuGene_st:413709-HuGene_st:230830_at:232719-HuGene_st:165400-HuGene_st:203597-HuGene_st:1025456-HuGene_st:625341-HuGene_st:881755-HuGene_st:490180-HuGene_st:113547 -HuGene_st:605512-HuGene_st:179662-HuGene_st:280309-HuGene_st:647118-HuGene_st | 6.23E-21 | 3.188 | 7.15 | 94.5 | 89.2-97.5 |
| MGC13057 | 926449-HuGene_st:317301-HuGene_st:834797-HuGene_st:173388-HuGene_st:127937-HuGene_st:182395-HuGene_st:800172-HuGene_st:1041848-HuGene_st:93911-HuGene_st:810617-HuGene_st:1035428-HuGene_st:644866-HuGene_st:343402-HuGene_st:223754_at:228195_at:509933-HuGene_st | 6.94E-21 | 3.2029 | 3.18 | 94.5 | 89.3-97.5 |
| CLDN23 | 403960-HuGene_st:25144-HuGene_st:947653-HuGene_st:228704_s_at:228706_s_at:320 375-HuGene_st:441629-HuGene_st:367414-HuGene_st:855269-HuGene_st:228707_at:788659-HuGene_st:698816-HuGene_st:95789-HuGene_st:270197-HuGene_st:472976-HuGene_st:280539-HuGene_st:1056334-HuGene_st:516288-HuGene_st:579963-HuGene_st | 4.27E-20 | 3.0777 | 3.47 | 93.8 | 88.2-97.1 |
| PKIB | 866170-HuGene_st:1055812-HuGene_st:264946-HuGene_st:684057-HuGene_st:124791-HuGene_st:134561-HuGene_st:1026756-HuGene_st:468593-HuGene_st:1045852-HuGene_st:939917-HuGene_st:110205-HuGene_st:660721-HuGene_st:905229-HuGene_st:223551_at:610426-HuGene_st | 5.94E-20 | 3.1232 | 3.28 | 94.1 | 88.7-97.3 |
| SEMA6D | 855766-HuGene_st:233882_s_at:543564-HuGene_st:277987-HuGene_st:1075165-HuGene_st:220574_at:732853-HuGene_st:410940-HuGene_st:384488-HuGene_st:1041056-HuGene_st:233801_s_at:535378-HuGene_st:477671-HuGene_st:626423-HuGene_st:101599-HuGene_st:714098-HuGene_st:301505-HuGene_st:53682-HuGene_st:177281-HuGene_st:596415-HuGene_st:244746_at:226492_at:699792-HuGene_st:73748-HuGene_st:692437-HuGene_ st | 6.07E-20 | 3.092 | 4.27 | 93.9 | 88.4-97.2 |
| CLCA4 | 220026_at:1000955-HuGene_st:215807-HuGene_st:108406-HuGene_st:538359-HuGene_st:366726-HuGene_st:727431-HuGene_st:240891-HuGene_st:485685-HuGene_st:205213-HuGene_st:476179-HuGene_st:283676-HuGene_st:933390-HuGene_st:601908-HuGene_st:844633-HuGene_st:846667-HuGene_st:99723-HuGene_st:376041-HuGene_st:71188-HuGene_st:261183-HuGene_st:498330-HuGene_st:500064-HuGene_st:785031-HuGene_st | 1.25E-19 | 3.1922 | 16.3 | 94.5 | 89.2-97.5 |
| TP53INP2 | 224836_at:294906-HuGene_st:857693-HuGene_st:852970-HuGene_st:594178-HuGene_st:868173-HuGene_st:353925-HuGene_ st:212485-HuGene_st:804363-HuGene_st:778869-HuGene_st:427072-HuGene_st:451529-HuGene_st:808765-HuGene_st:205314-HuGene_st:415164-HuGene_ st:1048358-HuGene_st | 1.45E-19 | 3.0229 | 3.78 | 93.5 | 87.8-96.9 |
| MS4A12 | 220834_at:582400-HuGene_st:354845-HuGene_st:341876-HuGene_st:769180-HuGene_st:740541-HuGene_st:436753-HuGene_st:570027-HuGene_st:26519-HuGene_st:956581-HuGene_st:335315-HuGene_st:206721-HuGene_st:589444-HuGene_st:16629-HuGene_st:113533-HuGene_st:201614-HuGene_st:774878-HuGene_st:786494-HuGene_st:493550-HuGene_st:403871-HuGene_st:192686-HuGene_st:846528-HuGene_st:249799-HuGene_st | 2.13E-19 | 3.2962 | 7.15 | 95 | 90-97.8 |
| TRPM6 | 767074-HuGene_st:695352-HuGene_st:411125-HuGene_st:221102_s_at:234864_s_at:240 389_at:358229-HuGene_st:755964-HuGene_st:840301-HuGene_st:959234-HuGene_st:782639-HuGene_st:833079-HuGene_st:1066034-HuGene_st:678013-HuGene_st:249083-HuGene_st:143934-HuGene_st:159130-HuGene_st:486486-HuGene_st:185057-HuGene_st:878793-HuGene_st:133981-HuGene_st:224412_s_at:202194-HuGene_st | 2.25E-19 | 3.0106 | 7.5 | 93.4 | 87.7-96.8 |
| XLKD1 | 520080-HuGene_st:1091117-HuGene_st:943125-HuGene_st:444068-HuGene_st:648558-HuGene_st:346991-HuGene_st:1006205-HuGene_st:373107-HuGene_st:682535-HuGene_st:1083245-HuGene_st:863143-HuGene_st:820120-HuGene_st:1044561-HuGene_st:220037_s_at:541228-HuGene_st:220256-HuGene_st:289122-HuGene_st:219059_s_at:246683-HuGene_st:775976-HuGene_st:207399-HuGene_st:1052557-HuGene_st:92121-HuGene_st | 3.60E-19 | 2.94 | 7.44 | 92.9 | 87.1-96.6 |
| ADH1B | 1078343-HuGene_st:512808-HuGene_st:614446-HuGene_st:910188-HuGene_st:422504-HuGene_st:731361-HuGene_st:209612_s_at:258079-HuGene_st:568239-HuGene_st:879930-HuGene_st:420417-HuGene_st:1025048-HuGene_st:908335-HuGene_st:654633-HuGene_st:947292-HuGene_st:1087125-HuGene_st:1004870-HuGene_st:209613_s_at:579636-HuGene_st:681018-HuGene_st:822774-HuGene_ st | 4.70E-19 | 3.0172 | 4.67 | 93.4 | 87.7-96.9 |
| PRDX6 | 353187-HuGene_st:520885-HuGene_st:919334-HuGene_st:646105-HuGene_st:390352-HuGene_st:200844_s_at:855125-HuGene_st:200845_s_at:54231-HuGene_st:612931-HuGene_st:837652-HuGene_st:538086-HuGene_st:796167-HuGene_st:208992-HuGene_st:39945-HuGene_st:489353-HuGene_st:963499-HuGene_st:376244-HuGene_st:297536-HuGene_st:495777-HuGene_st:1005253-HuGene_ st:833460-HuGene_st | 5.56E-19 | 2.8414 | 2.76 | 92.2 | 86.1-96.1 |
| CA2 | 762141-HuGene_st:111686-HuGene_st:512916-HuGene_st:9132-HuGene_st:995083-HuGene_st:209301_at:578483-HuGene_st:583428-HuGene_st:961832-HuGene_st:11748-HuGene_st:1056641-HuGene_st:545067-HuGene_st:952781-HuGene_st:689246-HuGene_st:246103-HuGene_st:803284-HuGene_st:828842-HuGene_ st:694989-HuGene_st:808226-HuGene_st:199030-HuGene_st | 5.87E-19 | 2.9625 | 9.22 | 93.1 | 87.2-96.7 |
| EDN3 | 314103-HuGene_st:217154_s_at:820613-HuGene_st:1050996-HuGene_st:348157-HuGene_st:338856-HuGene_st:594687-HuGene_st:38595-HuGene_st:270770-HuGene_st:817067-HuGene_st:766407-HuGene_st:256284-HuGene_st:208399_s_at:200417-HuGene_st:496738-HuGene_st:590141-HuGene_st:137170-HuGene_st:499250-HuGene_st:478524-HuGene_st:720694-HuGene_ st:543565-HuGene_st | 6.42E-19 | 2.937 | 11.21 | 92.9 | 87-96.5 |
| MIER3 | 884917-HuGene_st:740856-HuGene_st:107094-HuGene_st:408774-HuGene_st:461523-HuGene_st:153511-HuGene_st:207042-HuGene_st:737492-HuGene_st:43443-HuGene_st:614740-HuGene_st:1559652_at:783045-HuGene_st:887606-HuGene_st:996253-HuGene_st:1041635-HuGene_st:142612-HuGene_st:1553336_a_at:220555-HuGene_st:975347-HuGene_st:470750-HuGene_st:1554450_s_at:804524-HuGene_st:265536-HuGene_st:774142-HuGene_st:231975_s_at:34726-HuGene_st:1554449_at:228961_at | 9.14E-19 | 2.9162 | 3.91 | 92.8 | 86.8-96.5 |
| ABCA8 | 57305-HuGene_st:111752-HuGene_st:61357-HuGene_st:123451-HuGene_st:740746-HuGene_st:512280-HuGene_st:389185-HuGene_st:427721-HuGene_st:346028-HuGene_st:224566-HuGene_st:149653-HuGene_st:680699-HuGene_st:76772-HuGene_st:742091-HuGene_st:423333-HuGene_st:559944-HuGene_st:341399-HuGene_st:1565780_at:863400-HuGene_st:921748-HuGene_st:623719-HuGene_st:204719_at:123143-HuGene_ st:1077391-HuGene_st | 1.26E-18 | 2.9165 | 6.82 | 92.8 | 86.8-96.5 |
| METTL7A | 278779-HuGene_st:481332-HuGene_st:787584-HuGene_st:989793-HuGene_st:278513-HuGene_st:424901-HuGene_st:551005-HuGene_st:921169-HuGene_st:125176-HuGene_st:277465-HuGene_st:560742-HuGene_st:207761_s_at:367459-HuGene_st:224782-HuGene_st:660194-HuGene_st:266654-HuGene_st:1086300-HuGene_st:134633-HuGene_st:211424_x_at:273973-HuGene_st:947408-HuGene_st:209703_x_at:617184-HuGene_st:864197-HuGene_st:10061-HuGene_st | 1.35E-18 | 3.1231 | 1.93 | 94.1 | 88.6-97.3 |
| DPT | 876245-HuGene_st:1036210-HuGene_st:214758-HuGene_st:1005554-HuGene_st:178625-HuGene_st:701967-HuGene_st:35076-HuGene_st:33432-HuGene_st:897771-HuGene_st:304494-HuGene_st:445757-HuGene_st:491297-HuGene_st:646408-HuGene_st:842542-HuGene_st:87795-HuGene_st:213068_at:207977_s_at:55338 9-HuGene_st:477075-HuGene_st:213071_at:1046282-HuGene_st | 1.53E-18 | 2.8977 | 9.07 | 92.6 | 86.7-96.4 |
| MGC4172 | 339712-HuGene_st:1089815-HuGene_st:265870-HuGene_st:895908-HuGene_st:226292-HuGene_st:322910-HuGene_st:575887-HuGene_st:416485-HuGene_st:1055581-HuGene_st:1088692-HuGene_st:442194-HuGene_st:218756_s_at:775684-HuGene_st:773137-HuGene_st:373028-HuGene_st:512667 HuGene_st:164027-HuGene_st:541185-HuGene_st:593729-HuGene_st:506796-HuGene_st | 1.74E-18 | 2.9044 | 3.74 | 92.7 | 86.7-96.4 |
| FNBP1 | 728822-HuGene_st:845505-HuGene_st:1054016-HuGene_st:383098-HuGene_st:230389_at:31865-HuGene_st:981305-HuGene_st:213940_s_at:755179-HuGene_st:839717-HuGene_st:230086_at:239453_at:985213-HuGene_st:125814-HuGene_st:451336-HuGene_st:22306-HuGene_st:540957-HuGene_st:277486-HuGene_st:314000-HuGene_st:212288_at:662225-HuGene_st:524650-HuGene_st:605840-HuGene_st:180978-HuGene_st:604436-HuGene_ st | 1.76E-18 | 3.0483 | 2.04 | 93.6 | 88-97 |
| EDG2 | 816816-HuGene_st:309471-HuGene_st:77020-HuGene_st:289373-HuGene_st:10014-HuGene_st:981213-HuGene_st:204038_s_at:1035537-HuGene_st:75833-HuGene_st:781880-HuGene_st:494373-HuGene_st:745317-HuGene_st:497715-HuGene_st:693528-HuGene_st:806817-HuGene_st:812294-HuGene_st:204036_at:241773_at:176887-HuGene_st:441141-HuGene_st:189399-HuGene_st:723788-HuGene_st:204037_at:485543-HuGene_st:1014086-HuGene_st:232716_at | 2.44E-18 | 2.8838 | 3.19 | 92.5 | 86.5-96.3 |
| SFRP1 | 722739-HuGene_st:1095144-HuGene_st:1039611-HuGene_st:836419-HuGene_st:34446-HuGene_st:50826-HuGene_st:483033-HuGene_st:202036_s_at:988375-HuGene_st:774922-HuGene_st:228413_s_at:665988-HuGene_st:302344-HuGene_st:202035_s_at:412438-HuGene_st:995086-HuGene_st:202037_s_at:181713-HuGene_st:527962-HuGene_st:880207-HuGene_st:100260-HuGene_st | 2.77E-18 | 2.8631 | 15.53 | 92.4 | 86.3-96.2 |
| SLC26A3 | 206143_at:172542-HuGene_st:768419-HuGene_st:820082-HuGene_st:263997-HuGene_st:681117-HuGene_st:581666-HuGene_st:180553-HuGene_st:356894-HuGene_st:240715-HuGene_st:718555-HuGene_st:517735-HuGene_st:339388-HuGene_st:989337-HuGene_st:1511-HuGene_st:228629-HuGene_st:354149-HuGene_st:603695-HuGene_st:791438-HuGene_st:154 730-HuGene_st:905529-HuGene_st:248690-HuGene_st:431441-HuGene_st:215657_at | 4.18E-18 | 2.9214 | 21.4 | 92.8 | 86.8-96.5 |
| ANGPTL1 | 572942-HuGene_st:891312-HuGene_st:953040-HuGene_st:232844-HuGene_st:145730-HuGene_st:142205-HuGene_st:227771-HuGene_st:80584-HuGene_st:982090-HuGene_st:999640-HuGene_st:672931-HuGene_st:148578-HuGene_st:224339_s_at:1046706-HuGene_st:239183_at:155600-HuGene_st:284674-HuGene_st:231773_at:818064-HuGene_st:978991-HuGene_st:728775-HuGene_ st | 1.42E-17 | 2.7929 | 4.64 | 91.9 | 85.6-95.9 |
| UGP2 | 795967-HuGene_st:561220-HuGene_st:754258-HuGene_st:846757-HuGene_st:868436-HuGene_st:321605-HuGene_st:299097-HuGene_st:7536-HuGene_st:216268-HuGene_st:925542-HuGene_st:967807-HuGene_st:205480_s_at:639621-HuGene_st:177627-HuGene_st:894271-HuGene_st:617530-HuGene_st:231698_at:971369-HuGene_st:132663-HuGene_st:215198-HuGene_st:647604-HuGene_st:537579-HuGene_st:232180_at:1056881-HuGene_st | 1.64E-17 | 2.7904 | 2.41 | 91.9 | 85.6-95.9 |
| SRPX | 195599-HuGene_st:541428-HuGene_st:143706-HuGene_st:728558-HuGene_st:190938-HuGene_st:455214-HuGene_st:522280-HuGene_st:966576-HuGene_st:650551-HuGene_st:810331-HuGene_st:396246-HuGene_st:133110-HuGene_st:232310-HuGene_st:475511-HuGene_st:225561-HuGene_st:351281-HuGene_st:204955_at | 1.79E-17 | 2.7749 | 3.91 | 91.7 | 85.5-95.8 |
| OGN | 955964-HuGene_st:775073-HuGene_st:678354-HuGene_st:238500-HuGene_st:1019070-HuGene_st:614445-HuGene_st:23784-HuGene_st:658925-HuGene_st:757128-HuGene_st:632689-HuGene_st:989474-HuGene_st:233177-HuGene_st:650498-HuGene_st:950000-HuGene_st:751146-HuGene_st:67232-HuGene_st:997991-HuGene_st:218730_s_at:222722_at:82148 8-HuGene_st | 2.25E-17 | 2.7078 | 13.67 | 91.2 | 84.7-95.4 |
| MYLK | 527465-HuGene_st:617817-HuGene_st:716971-HuGene_st:942711-HuGene_st:230460_at:161532-HuGene_st:767103-HuGene_st:1041083-HuGene_st:202167-HuGene_st:738247-HuGene_st:505966-HuGene_st:62701-HuGene_st:776564-HuGene_st:679137-HuGene_st:551306-HuGene_st:202555_s_at:224823_at:98631 3-HuGene_st:324943-HuGene_st:218172-HuGene_st:351524-HuGene_st:1568770_at:280094-HuGene_st:1017324-HuGene_st:1563466_at | 2.33E-17 | 2.7729 | 3.99 | 91.7 | 85.4-95.8 |
| LIFR | 275506-HuGene_st:323055-HuGene_st:444251-HuGene_st:1056178-HuGene_st:398104-HuGene_st:917434-HuGene_st:1044918-HuGene_st:167500-HuGene_st:423760-HuGene_st:837336-HuGene_st:321505-HuGene_st:918321-HuGene_st:252278-HuGene_st:884504-HuGene_st:124845-HuGene_st:499777-HuGene_st:969722-HuGene_st:709439-HuGene_st:611505-HuGene_st:227771_at:287217-HuGene_st:205876_at:225571_at:229185_ at:233367_at:1093011-HuGene_st | 2.45E-17 | 2.7826 | 6.36 | 91.8 | 85.6-95.8 |
| ZG16 | 287680-HuGene_st:1704-HuGene_st:61566-HuGene_st:964936-HuGene_st:9737-HuGene_st:620497-HuGene_st:785765-HuGene_st:214142_at:323025-HuGene_st:1093257-HuGene_st:398415-HuGene_st:942991-HuGene_st:871899-HuGene_st:548837-HuGene_st:605328-HuGene_st:231918-HuGene_st:461816-HuGene_st:604283-HuGene_st:971003-HuGene_st:750108-HuGene_st:927723-HuGene_st:782940-HuGene_st:98669-HuGene_ st | 2.51E-17 | 2.8097 | 8.14 | 92 | 85.8-96 |
| EPB41L3 | 275431-HuGene_st:540074-HuGene_st:731277-HuGene_st:211776_s_at:218157-HuGene_st:328922-HuGene_st:384314-HuGene_st:272486-HuGene_st:196590-HuGene_st:294230-HuGene_st:206710_s_at:747631-HuGene_st:52743-HuGene_st:1089957-HuGene_st:423337-HuGene_st:915755-HuGene_st:623682-HuGene_st:130815-HuGene_st:497574-HuGene_st:212681_at:77106-HuGene_ st:874781-HuGene_st | 2.62E-17 | 2.8312 | 2.51 | 92.2 | 86-96.1 |
| SCNN1B | 773390-HuGene_st:467376-HuGene_st:411583-HuGene_st:433511-HuGene_st:142579-HuGene_st:99655-HuGene_st:278054-HuGene_st:371417-HuGene_st:99189-HuGene_st:491965-HuGene_st:490957-HuGene_st:916581-HuGene_st:285916-HuGene_st:359156-HuGene_st:977951-HuGene_st:291765-HuGene_st:767266-HuGene_st:206091-HuGene_st:348267-HuGene_st:205464_at:838576-HuGene_st | 3.20E-17 | 2.7219 | 16.15 | 91.3 | 84.9-95.5 |
| HHLA2 | 371335-HuGene_st:978721-HuGene_st:1065567-HuGene_st:282548-HuGene_st:240410-HuGene_st:170899-HuGene_st:947848-HuGene_st:438234-HuGene_st:220812_s_at:927495-HuGene_st:351364-HuGene_st:234673_at:993142-HuGene_st:1009637-HuGene_st:335000-HuGene_st:285313-HuGene_st:533646-HuGene_st:234624_at:458597-HuGene_st:104838-HuGene_st:26687-HuGene_st:258409-HuGene_st:493304-HuGene_st:378019-HuGene_st:576796-HuGene_st | 3.47E-17 | 2.6741 | 10.02 | 90.9 | 84.4-95.2 |
| HPGD | 291863-HuGene_st:375608-HuGene_st:793406-HuGene_st:436293-HuGene_st:75568-HuGene_st:211549_s_at:684728-HuGene_st:674596-HuGene_st:527856-HuGene_ st:329920-HuGene_st:748432-HuGene_st:259392-HuGene_st:769902-HuGene_st:620673-HuGene_st:450707-HuGene_st:203913_s_at:304752-HuGene_st:447604-HuGene_st:170968-HuGene_st:852359-HuGene_st:836377-HuGene_st:242733_at:243846-HuGene_st:136281-HuGene_st:203914_x_at:211548_s_at:288 252-HuGene_st | 3.65E-17 | 2.8433 | 3.21 | 92.2 | 86.1-96.1 |
| CEACAM7 | 206199_at:105665-HuGene_st:481561-HuGene_st:211848_s_at:574369-HuGene_st:104615-HuGene_st:242258-HuGene_st:740634-HuGene_st:439249-HuGene_st:15044-HuGene_st:424954-HuGene_st:1002484-HuGene_st:206198_s_at:1067871-HuGene_st:751891-HuGene_st:36594-HuGene_st:298155-HuGene_st:1024423-HuGene_st:384378-HuGene_st:1093665-HuGene_ st:985998-HuGene_st:325578-HuGene_st:596079-HuGene_st:89770-HuGene_st:864758-HuGene_st:205004-HuGene_st:374052-HuGene_st:133822-HuGene_st:1093942-HuGene_st:780375-HuGene_ st:338636-HuGene_st | 3.96E-17 | 2.785 | 10.4 | 91.8 | 85.5-95.8 |
| RELL1 | 753672-HuGene_st:871189-HuGene_st:202462-HuGene_st:615056-HuGene_st:639180-HuGene_st:4103-HuGene_st:778017-HuGene_st:236549-HuGene_st:86799-HuGene_st:908328-HuGene_st:813998-HuGene_st:859771-HuGene_st:254041-HuGene_st:787272-HuGene_st:916269-HuGene_st:885786-HuGene_st:1554714_at:645386-HuGene_st:777480-HuGene_st:927821-HuGene_ st:226430_at | 4.10E-17 | 2.6983 | 2.5 | 91.1 | 84.6-95.4 |
| C7 | 893214-HuGene_st:54339-HuGene_st:501090-HuGene_st:659367-HuGene_st:536407-HuGene_st:598145-HuGene_st:444111-HuGene_st:643669-HuGene_st:191981-HuGene_st:832001-HuGene_st:313065-HuGene_st:612836-HuGene_st:673870-HuGene_st:1005162-HuGene_st:206652-HuGene_st:1021871-HuGene_st:489813-HuGene_st:350161-HuGene_st:766550-HuGene_st:2377-HuGene_st:202992_at:578639-HuGene_st | 4.40E-17 | 2.7158 | 6.67 | 91.3 | 84.9-95.5 |
| PLCE1 | 1562826_at:1563103_at:699801-HuGene_st:353862-HuGene_st:187026-HuGene_st:475246-HuGene_st:819995-HuGene_st:477396-HuGene_st:920273-HuGene_st:1566739_at:158053-HuGene_st:554291-HuGene_st:854670-HuGene_st:950080-HuGene_st:1064466-HuGene_st:1017434-HuGene_st:987146-HuGene_st:137607-HuGene_st:833052-HuGene_st:205112_at:987189-HuGene_st:1092203-HuGene_st:449587-HuGene_st:205111_s_at:1566740_at:9284 26-HuGene_st:649071-HuGene_st | 6.91E-17 | 2.7528 | 2.43 | 91.6 | 85.2-95.7 |
| SCARA5 | 734536-HuGene_st:1080104-HuGene_st:796100-HuGene_st:258146-HuGene_st:670462-HuGene_st:362431-HuGene_st:1019057-HuGene_st:287988-HuGene_st:235849_at:222306-HuGene_st:495319-HuGene_st:84286-HuGene_st:47679-HuGene_st:855551-HuGene_st:229839_at:999202-HuGene_st:982358-HuGene_st:86142-HuGene_ st:568080-HuGene_st:109115-HuGene_st:590235-HuGene_st:1554705_at:800351-HuGene_st | 1.25E-16 | 2.6912 | 2.86 | 91.1 | 84.5-95.3 |
| CHRDL1 | 335657-HuGene_st:1058673-HuGene_st:166016-HuGene_st:825273-HuGene_st:35456-HuGene_st:91115-HuGene_st:303433-HuGene_st:209044-HuGene_st:1081642-HuGene_st:135625-HuGene_st:284037-HuGene_st:452295-HuGene_st:313790-HuGene_st:912814-HuGene_st:712513-HuGene_st:1078238-HuGene_st:721877-HuGene_ st:209763_at:657006-HuGene_st | 1.49E-16 | 2.6092 | 13.88 | 90.4 | 83.7-94.9 |
| NR3C2 | 748063-HuGene_st:177629-HuGene_st:964944-HuGene_st:346404-HuGene_st:120283-HuGene_st:646104-HuGene_st:731385-HuGene_st:493776-HuGene_st:440054-HuGene_st:286582-HuGene_st:1097927-HuGene_st:804888-HuGene_st:234142_at:49341-HuGene_st:370558-HuGene_st:22007-HuGene_st:981978-HuGene_st:100953-HuGene_st:239673_at:149241-HuGene_st:940356-HuGene_st:628366-HuGene_st:205259_at:1564236_at:328672 -HuGene_st | 2.08E-16 | 2.5811 | 3.19 | 90.2 | 83.3-94.7 |
| TSPAN7 | 703402-HuGene_st:745610-HuGene_st:1018812-HuGene_st:742943-HuGene_st:168712-HuGene_st:234246_at:902239-HuGene_st:285968-HuGene_st:883914-HuGene_st:113610-HuGene_st:236336-HuGene_st:511310-HuGene_st:40850-HuGene_st:346422-HuGene_st:189042-HuGene_st:745521-HuGene_st:996022-HuGene_st:202242_at:195303-HuGene_st:70610-HuGene_st:31995-HuGene_st:234245_at | 2.13E-16 | 2.651 | 3.68 | 90.7 | 84.2-95.1 |
| STMN2 | 459270-HuGene_st:611227-HuGene_st:295241-HuGene_st:935618-HuGene_st:161827-HuGene_st:17520-HuGene_st:337557-HuGene_st:16478-HuGene_st:1015231-HuGene_st:790658-HuGene_st:892767-HuGene_st:343589-HuGene_st:1047491-HuGene_st:454759-HuGene_st:9820-HuGene_st:203001_s_at:472072-HuGene_st:203000_at:691598-HuGene_st:1064573-HuGene_st:811444-HuGene_ st:485134-HuGene_st | 2.25E-16 | 2.6147 | 6.13 | 90.4 | 83.8-94.9 |
| FAM107A | 315531-HuGene_st:294482-HuGene_st:764207-HuGene_st:809908-HuGene_st:209074_s_at:341591-HuGene_st:948705-HuGene_st:207547_s_at:415418-HuGene_st:215297-HuGene_st:332641-HuGene_st | 2.52E-16 | 2.6168 | 2.92 | 90.5 | 83.8-94.9 |
| ANK2 | 314086-HuGene_st:282874-HuGene_st:382431-HuGene_st:229308-HuGene_st:779600-HuGene_st:297624-HuGene_st:385943-HuGene_st:730140-HuGene_st:442277-HuGene_st:699309-HuGene_st:182816-HuGene_st:202921_s_at:799860-HuGene_st:868462-HuGene_st:634421-HuGene_st:571536-HuGene_st:1050903-HuGene_st:649509-HuGene_st:239935-HuGene_ st:202920_at | 2.59E-16 | 2.6501 | 6.76 | 90.7 | 84.1-95.1 |
| CLIC5 | 217628_at:219866_at:116794-HuGene_st:114619-HuGene_st:895974-HuGene_st:749788-HuGene_st:485909-HuGene_st:326032-HuGene_st:1083118-HuGene_st:213317_at:507492-HuGene_st:480370-HuGene_st:160114-HuGene_st:637800-HuGene_st:33914-HuGene_st:1044626-HuGene_st:243917_at:664038-HuGene_st:867787-HuGene_st:261656-HuGene_st:399156-HuGene_st:558641-HuGene_ st | 3.68E-16 | 2.6963 | 2.37 | 91.1 | 84.6-95.4 |
| CHGA | 152790-HuGene_st:354161-HuGene_st:365802-HuGene_st:813261-HuGene_st:40004-HuGene_st:504502-HuGene_st:624128-HuGene_st:719186-HuGene_st:916335-HuGene_st:921714-HuGene_st:489360-HuGene_st:849455-HuGene_st:231475-HuGene_st:294992-HuGene_st:204697_s_at:659997-HuGene_st:521835-HuGene_st:659283-HuGene_st:890388-HuGene_st:786316-HuGene_st | 5.54E-16 | 2.6097 | 7.21 | 90.4 | 83.6-94.9 |
| KIAA0828 | 613787-HuGene_st:478932-HuGene_st:67325-HuGene_st:668897-HuGene_st:160683-HuGene_st:1031978-HuGene_st:1018444-HuGene_st:237998-HuGene_st:892138-HuGene_st:262221-HuGene_st:864937-HuGene_st:917541-HuGene_st:593865-HuGene_st:55857-HuGene_st:215672_s_at:509888-HuGene_st:302988-HuGene_st:668914-HuGene_ st:507489-HuGene_st:212814_at | 5.58E-16 | 2.5514 | 4.09 | 89.9 | 83-94.5 |
| EDIL3 | 436107-HuGene_st:1558643_s_at:214345_at:6687 80-HuGene_st:545521 HuGene_st:641822-HuGene_st:207379_at:411378-HuGene_st:485618-HuGene_st:576523-HuGene_st:843058-HuGene_st:910789-HuGene_st:165720-HuGene_st:79965-HuGene_st:533665-HuGene_st:237605-HuGene_st:593615-HuGene_st:396108-HuGene_st:701416-HuGene_st:325630-HuGene_st:912197-HuGene_st:224797-HuGene_st:724765-HuGene_st:167229-HuGene_st:233668_at:225275_at:16759-HuGene_st:233875_at | 6.21E-16 | 2.6259 | 2.96 | 90.5 | 83.8-95 |
| DSCR1 | 535011-HuGene_st:789105-HuGene_st:215253_s_at:838760-HuGene_st:617570-HuGene_st:419279-HuGene_st:1015938-HuGene_st:208370_s_at:131478-HuGene_st:228884-HuGene_st:685397-HuGene_st:68677-HuGene_st:215254_at:656173-HuGene_st:971107-HuGene_st:1039926-HuGene_st:206974-HuGene_st:232227-HuGene_st:546248-HuGene_st:175597-HuGene_ st:1558181_at | 6.21E-16 | 2.6122 | 2.14 | 90.4 | 83.7-94.9 |
| PPAP2A | 282276-HuGene_st:1062765-HuGene_st:952258-HuGene_st:214647-HuGene_st:153982-HuGene_st:678140-HuGene_st:209147_s_at:1083041-HuGene_st:459000-HuGene_st:505597-HuGene_st:1035745-HuGene_st:576550-HuGene_st:243715_at:180473-HuGene_st:527974-HuGene_st:821572-HuGene_st:260389-HuGene_st:1042377-HuGene_st:138155-HuGene_st:210946_at:243442_x_at | 6.43E-16 | 2.596 | 2.4 | 90.3 | 83.6-94.8 |
| PDCD4 | 681770-HuGene_st:1029160-HuGene_st:594740-HuGene_st:247013-HuGene_st:845755-HuGene_st:813933-HuGene_st:367555-HuGene_st:456148-HuGene_st:295280-HuGene_st:144200-HuGene_st:620666-HuGene_st:212593_s_at:590149-HuGene_st:87_471-HuGene_st:189624-HuGene_st:202730_s_at:44562-HuGene_st:641673-HuGene_st:577621-HuGene_st:102842-HuGene_st:202731_at:776615-HuGene_st:615983-HuGene_st:212594_at:1557166_at | 8.67E-16 | 2.4578 | 2.6 | 89 | 82-93.9 |
| DHRS9 | 413956-HuGene_st:371161-HuGene_st:224009_x_at:61832-HuGene_st:874913-HuGene_st:321055-HuGene_st:223952_x_at:916038-HuGene_st:313731-HuGene_st:629747-HuGene_st:836048-HuGene_st:369374-HuGene_st:157673-HuGene_st:194386-HuGene_st:1089433-HuGene_st:352973-HuGene_st:637905-HuGene_st:1010893-HuGene_st:219799_s_at:361862-HuGene_st:800910-HuGene_st:1012296-HuGene_st | 1.09E-15 | 2.7371 | 1.51 | 91.4 | 85.1-95.6 |
| CDKN2B | 252481-HuGene_st:1000015-HuGene_st:172198-HuGene_st:967979-HuGene_st:675431-HuGene_st:605224-HuGene_st:277126-HuGene_st:846115-HuGene_st:452433-HuGene_st:332731-HuGene_st:563208-HuGene_st:527577-HuGene_st:207530_s_at:891201-HuGene_st:512260-HuGene_st:236313_at:26244-HuGene_st:61586-HuGene_st | 1.34E-15 | 2.5433 | 10.09 | 89.8 | 82.9-94.5 |
| P2RY1 | 628249-HuGene_st:113454-HuGene_st:627857-HuGene_st:461281-HuGene_st:207455_at:259065-HuGene_st:797734-HuGene_st:135788-HuGene_st:42916-HuGene_st:315405-HuGene_st:340050-HuGene_st:173225-HuGene_st:919818-HuGene_st:591228-HuGene_st:899117-HuGene_st:785070-HuGene_ st:286200-HuGene_st:231925_at | 1.41E-15 | 2.5341 | 3.6 | 89.7 | 82.8-94.4 |
| LOC64662 7 | 238143_at | 1.58E-15 | 1.9131 | 5.02 | 83.1 | 74.9-89.4 |
| PPAP2B | 484735-HuGene_st:860271-HuGene_st:501067-HuGene_st:180731-HuGene_st:919245-HuGene_st:209355_s_at:322730-HuGene_st:830995-HuGene_st:212226_s_at:353268-HuGene_st:989778-HuGene_st:279549-HuGene_st:583834-HuGene_st:1035367-HuGene_st:155537-HuGene_st:782552-HuGene_st:867734-HuGene_st:267847-HuGene_st:212230_at:619833-HuGene_st:232324_x_at | 2.44E-15 | 2.5819 | 1.81 | 90.2 | 83.4-94.7 |
| PYY | 656845-HuGene_st:816022-HuGene_st:633572-HuGene_st:20355-HuGene_st:240779-HuGene_st:638358-HuGene_st:879780-HuGene_st:207080_s_at:211253_x_at:368 591-HuGene_st | 3.50E-15 | 2.4793 | 11.71 | 89.2 | 82.2-94 |
| CLEC3B | 794375-HuGene_st:894763-HuGene_st:152328-HuGene_st:669650-HuGene_st:1090081-HuGene_st:251162-HuGene_st:264101-HuGene_st:367416-HuGene_st:984078-HuGene_st:512272-HuGene_st:166348-HuGene_st:181414-HuGene_st:995862-HuGene_st:603678-HuGene_st:205200_at:56968-HuGene_ st:542483-HuGene_st | 4.65E-15 | 2.4437 | 4.46 | 88.9 | 81.7-93.8 |
| NDE1 | 228133_s_at:218414_s_at:555730-HuGene_st:620739-HuGene_st:111363-HuGene_st | 4.84E-15 | 2.5125 | 1.99 | 89.5 | 82.6-94.3 |
| CCL8 | 402294-HuGene_st:1098932-HuGene_st:930533-HuGene_st:524795-HuGene_st:574692-HuGene_st:314202-HuGene_st:719831-HuGene_st:410906-HuGene_st:643479-HuGene_st:911966-HuGene_st:31200-HuGene_st:547634-HuGene_st:39456-HuGene_st:527508-HuGene_st:1057270-HuGene_st:1062460-HuGene_st:214038_at | 5.75E-15 | 2.4512 | 7.51 | 89 | 81.9-93.9 |
| PLAC8 | 219014_at:739479-HuGene_st:354260-HuGene_st:994047-HuGene_st:456534-HuGene_st:236727_at:330382-HuGene_st:118286-HuGene_st:940467-HuGene_st:493579-HuGene_st:37786-HuGene_st:776403-HuGene_st:295172-HuGene_st:271859-HuGene_st:438305-HuGene_st:770531-HuGene_st:982881-HuGene_st:543535-HuGene_st:554805-HuGene_st:667817-HuGene_st:519806-HuGene_st:1065533-HuGene_st:345940-HuGene st | 5.76E-15 | 2.4228 | 3.87 | 88.7 | 81.5-93.7 |
| MUCDHL | 690807-HuGene_st:11068-HuGene_st:955242-HuGene_st:969280-HuGene_st:220075_s_at:625411-HuGene_st:789310-HuGene_st:444568-HuGene_st:589749-HuGene_st:265998-HuGene_st:275576-HuGene_st:219796_s_at:625643-HuGene_st:452184-HuGene_st:692224-HuGene_st:1009431-HuGene_st:311533-HuGene_st:289109-HuGene_st:101091-HuGene_ st:220074_at:307121-HuGene_st | 5.81E-15 | 2.4358 | 3.06 | 88.8 | 81.7-93.8 |
| SULT1A1 | 322199-HuGene_st:1021081-HuGene_st:372215-HuGene_st:689177-HuGene_st:215299_x_at:369102-HuGene_st:203615_x_at:761577-HuGene_st:744847-HuGene_st:691837-HuGene_st:2491-HuGene_st:742631-HuGene_st:1037597-HuGene_st:805936-HuGene st | 7.17E-15 | 2.422 | 3.37 | 88.7 | 81.6-93.7 |
| SDCBP2 | 233565_s_at:263537-HuGene_st:901885-HuGene_st:421400-HuGene_st:835589-HuGene_st:70935-HuGene_st:462061-HuGene_st:702702-HuGene_st:719818-HuGene_st:766065-HuGene_st:612065-HuGene_st:825595-HuGene_st:1004265-HuGene_st:206488-HuGene_st:867231-HuGene_st:858503-HuGene_st:29674-HuGene_st:26237-HuGene_st:351356-HuGene_st:52388-HuGene_st:192518-HuGene_ st | 9.28E-15 | 2.4869 | 2.37 | 89.3 | 82.3-94.1 |
| RPL24 | 1559655_at:1559656_a_at:228885_at | 1.23E-14 | 2.5579 | 1.63 | 90 | 83.1-94.6 |
| CXCL12 | 567389-HuGene_st:886750-HuGene_st:815979-HuGene_st:1018943-HuGene_st:209687_at:800403-HuGene_st:500331-HuGene_st:369373-HuGene_st:1050367-HuGene_st:952256-HuGene_st:1066302-HuGene_st:448047-HuGene_st:186655-HuGene_st:771363-HuGene_st:103922-HuGene_st:1025591-HuGene_st:47442-HuGene_st:624854-HuGene st:211406-HuGene st:255389-HuGene_st | 1.54E-14 | 2.4343 | 3.79 | 88.8 | 81.6-93.7 |
| C1orf115 | 293810-HuGene_st:439869-HuGene_st:640378-HuGene_st:226391-HuGene_st:88026-HuGene_st:230159_at:709535-HuGene_st:687885-HuGene_st:218546_at:628297-HuGene_st:695536-HuGene_st:230774-HuGene_st:884109-HuGene_st:517531-HuGene_st:472518-HuGene_st:8635-HuGene_ st:158841-HuGene_st | 1.95E-14 | 2.4078 | 2.95 | 88.6 | 81.4-93.6 |
| SLC26A2 | 224963_at:861453-HuGene_st:175674-HuGene_st:658865-HuGene_st:979969-HuGene_st:1053352-HuGene_st:224959_at:509013-HuGene_st:755618-HuGene_st:879968-HuGene_st:205097_at:901728-HuGene_st:975514-HuGene_st:407147-HuGene_st:891478-HuGene_st:196344-HuGene_st:1015698-HuGene_st:955403-HuGene_st:430227-HuGene_st:1075936-HuGene_st:1080935-HuGene_st:18346-HuGene_st:341007-HuGene_st:471383-HuGene_st | 2.08E-14 | 2.4223 | 7.83 | 88.7 | 81.5-93.7 |
| MEIS1 | 868613-HuGene_st:1072855-HuGene_st:139075-HuGene_st:721526-HuGene_st:1559477_s_at:381698-HuGene_st:641395-HuGene_st:494458-HuGene_st:183063-HuGene_st:212378-HuGene_st:579377-HuGene_st:1006382-HuGene_st:753093-HuGene_st:863677-HuGene_st:517122-HuGene_st:777085-HuGene_st:386678-HuGene_st:204069_at:1005832-HuGene_st:395572-HuGene_st:231941-HuGene_st:568986-HuGene_st:108139-HuGene_st | 2.49E-14 | 2.3774 | 5.44 | 88.3 | 81-93.4 |
| SFRP2 | 144787-HuGene_st:337246-HuGene_st:97123-HuGene_st:614920-HuGene_st:223121_s_at:236674-HuGene_st:602699-HuGene_st:370588-HuGene_st:794693-HuGene_st:252957-HuGene_st:870849-HuGene_st:279759-HuGene_st:367084-HuGene_st:691080-HuGene_st:223122_s_at:466709-HuGene_st | 3.05E-14 | 2.3944 | 7.39 | 88.4 | 81.2-93.5 |
| TUBB6 | 209191_at:238151_at:267951-HuGene_st:578028-HuGene_st: 862750-HuGene_st | 3.55E-14 | 2.3694 | 5.9 | 88.2 | 80.9-93.3 |
| GPX3 | 623237-HuGene_st:40650-HuGene_st:134873-HuGene_st:952394-HuGene_st:644367-HuGene_st:428176-HuGene_st:201348_at:214091_s_at:37706 0-HuGene_st:674301-HuGene_st:142325-HuGene_st:905944-HuGene_st:439631-HuGene_st:1064572-HuGene_st:587834-HuGene_st:1040504-HuGene_st:250733-HuGene_st:199701-HuGene_st:784815-HuGene_ st:652659-HuGene_st | 5.68E-14 | 2.3613 | 2.57 | 88.1 | 80.8-93.2 |
| CD36 | 392196-HuGene_st:274514-HuGene_st:477005-HuGene_st:691585-HuGene_ st:872909-HuGene_st:543050-HuGene_st:603343-HuGene_st:514557-HuGene_st:296850-HuGene_st:945913-HuGene_st:495755-HuGene_st:206488_s_at:1035854-HuGene_st:887301-HuGene_st:836370-HuGene_st:209555_s_at:939919-HuGene_st:507440-HuGene_st:151788-HuGene_st:146280-HuGene_st:360545-HuGene_st:1051486-HuGene_ st:228766_at:512885-HuGene_st | 6.27E-14 | 2.4677 | 1.65 | 89.1 | 82.1-94 |
| HIGD1A | 936261-HuGene_st:824654-HuGene_st:165981-HuGene_st:225595-HuGene_st:290747-HuGene_st:641007-HuGene_st:1073341-HuGene_st:761357-HuGene_st:818902-HuGene_st:1038575-HuGene_st:102828-HuGene_st:533707-HuGene_st:414059-HuGene_st:217845_x_at:852712-HuGene_st:221896_s_at:291015-HuGene_st:295866-HuGene_st:520869-HuGene_st:155393-HuGene_st:460566-HuGene_st:969731-HuGene_st:170508-HuGene_st:744650-HuGene_st:309982-HuGene_st:795342-HuGene_st:387733-HuGene_st:324047-HuGene_st:239559-HuGene_st:1055553-HuGene_st:827869-HuGene_st:1058468-HuGene_st | 6.29E-14 | 2.4011 | 2.09 | 88.5 | 81.3-93.5 |
| NR3C1 | 111450-HuGene_st:434945-HuGene_st:1054045-HuGene_st:201866_s_at:98168-HuGene_st:402326-HuGene_st:154154-HuGene_st:117026-HuGene_st:1027472-HuGene_st:517524-HuGene_st:540910-HuGene_st:538776-HuGene_st:550290-HuGene_st:460796-HuGene_st:211671_s_at:216321_s_at:201 865_x_at:649431-HuGene_st:461795-HuGene st | 7.65E-14 | 2.3495 | 2.51 | 88 | 80.6-93.2 |
| LOC63928 | 206149_at:681928-HuGene_st:1004459-HuGene_st:249694-HuGene_st:80283-HuGene_st:323497-HuGene_st:650243-HuGene_st:872231-HuGene_st:253533-HuGene_st:270238-HuGene_st:282291-HuGene st:268597-HuGene st:913922-HuGene_st:416350-HuGene_st:992808-HuGene_st:472127-HuGene_st:988731-HuGene_st:243977-HuGene_st:802623-HuGene_ st:314649-HuGene_st | 8.09E-14 | 2.352 | 3.2 | 88 | 80.6-93.2 |
| MXD1 | 226275_at:206877_at:602969-HuGene_st:127172-HuGene_st:577694-HuGene_st:53257-HuGene_st:228846_at:669193-HuGene_st:37027-HuGene_st:458872-HuGene_st:518444-HuGene_st:410159-HuGene_st:77113-HuGene_st:280760-HuGene_st:229252-HuGene_st:318164-HuGene_st:393130-HuGene_st:172809-HuGene_st:1020416-HuGene_st:1050501-HuGene_st:269397-HuGene_st:68122-HuGene_st:423400-HuGene_st:103208-HuGene st:936311-HuGene_st | 8.35E-14 | 2.4077 | 2.11 | 88.6 | 81.3-93.6 |
| C9orf19 | 268503-HuGene_st:209268-HuGene_st:225604_s_at:518559-HuGene_st:1007612-HuGene_st:499070-HuGene_st:225602_at:291028-HuGene_st:615472-HuGene_st:3136-HuGene_st:926635-HuGene_st:219003-HuGene_st:396117-HuGene_st:94077-HuGene_st:747558-HuGene_st:226948-HuGene_st:940376-HuGene_st:977640-HuGene_st:316643-HuGene_st:382182-HuGene_ st:745966-HuGene_st | 1.74E-13 | 2.3659 | 1.95 | 88.2 | 80.9-93.3 |
| DCN | 6640-HuGene_st:201893_x_at:211813_x_at:311 031-HuGene_st:211896_s_at:505982-HuGene_st:219729-HuGene_st:1067446-HuGene_st:538017-HuGene_st:13085-HuGene_st:960643-HuGene_st:132704-HuGene_st:253410-HuGene_st:54359-HuGene_st:1020632-HuGene_st:973501-HuGene_st:483405-HuGene_st:252884-HuGene_st:274013-HuGene_st:664014-HuGene_st:522817-HuGene_st:745324-HuGene_st:209335_at:339470-HuGene_ st:916917-HuGene_st | 2.05E-13 | 2.3127 | 5.62 | 87.6 | 80.3-92.9 |
| HSD17B2 | 281748-HuGene_st:142500-HuGene_st:602472-HuGene_st:437526-HuGene_st:1057583-HuGene_st:999857-HuGene_st:915850-HuGene_st:1097510-HuGene_st:127085-HuGene_st:1088665-HuGene_st:194898-HuGene_st:834134-HuGene_st:360927-HuGene_st:1029542-HuGene_st:422429-HuGene_st:573141-HuGene_st:204818_at:918446-HuGene_st:386936-HuGene_st:613850-HuGene_st:489258-HuGene_st | 2.32E-13 | 2.1956 | 4.76 | 86.4 | 78.7-92 |
| TCF21 | 804657-HuGene_st:106365-HuGene_st:710514-HuGene_st:299556-HuGene_st:608149-HuGene_st:242233-HuGene_st:655881-HuGene_st:356773-HuGene_st:788445-HuGene_st:709897-HuGene_st:605488-HuGene_st:652466-HuGene_st:204931_at:830709-HuGene_st:273418-HuGene_st:1004754-HuGene_st:990861-HuGene_st:238739-HuGene_st:836241-HuGene_st:110045-HuGene_st:229529_at:1001969-HuGene_ st | 2.51E-13 | 2.3047 | 2.34 | 87.5 | 80.1-92.8 |
| MRC1 | 467197-HuGene_st:758500-HuGene_st:600690-HuGene_st:270842-HuGene_st:787475-HuGene_st:710055-HuGene_st:295499-HuGene_st:757670-HuGene_st:994062-HuGene_st:587369-HuGene_st:224499-HuGene_st:428438-HuGene_st:577964-HuGene_st:159156-HuGene_st:298439-HuGene_st:1088275-HuGene_st:715300-HuGene_st:1627-HuGene_st:513230-HuGene_st:106268-HuGene_st:204438_at | 2.72E-13 | 2.3106 | 2.6 | 87.6 | 80.2-92.9 |
| ADH1C | 661427-HuGene_st:243544_at:634797-HuGene_st:291687-HuGene_st:972177-H uGene_st:316339-HuGene_st:580686-HuGene_st:206262_at:211851-HuGene_st:641619-HuGene_st:142959-HuGene_st:705200-HuGene_st:1063529-HuGene_st:916148-HuGene_st:901368-HuGene_st:43611-HuGene_st:789755-HuGene_st:410220-HuGene_st:289785-HuGene_st:155724-HuGene_st:289958-HuGene_ st:232595-HuGene_st | 2.94E-13 | 2.3724 | 3.1 | 88.2 | 81-93.3 |
| BCHE | 228090-HuGene_st:752051-HuGene_st:800167-HuGene_st:221362-HuGene_st:155900-HuGene_st:717363-HuGene_st:536584-HuGene_st:146780-HuGene_st:302487-HuGene_st:508472-HuGene_st:516293-HuGene_st:968992-HuGene_st:666625-HuGene_st:923158-HuGene_st | 3.18E-13 | 2.2535 | 6.21 | 87 | 79.4-92.4 |
| CCDC80 | 1072039-HuGene_st:973747-H uGene_st:580839-HuGene_st:583669-HuGene_st:290210-HuGene_st:308086-HuGene_st:679211-HuGene_st:43315-HuGene_st:1080603-HuGene_st:852141-HuGene_st:569200-HuGene_st:92231-HuGene_st:535306-HuGene_st:427093-HuGene_st:1003098-HuGene_st:408541-HuGene_st:135243-HuGene_st:225298-HuGene_st:225242_s_at:225241_at:63307 9-HuGene_st:43353-HuGene_st:642388-HuGene_ st:243864_at | 3.60E-13 | 2.2322 | 3.67 | 86.8 | 79.2-92.3 |
| CITED2 | 125201-HuGene_st:410723-HuGene_st:463405-HuGene_st:401168-HuGene_st:1012057-HuGene_st:235057-HuGene_st:361772-HuGene_st:207980_s_at:1091907-HuGene_st:985355-HuGene_st:175990-HuGene_st:227287_at:48433-HuGene_ st:209357_at:477746-HuGene_st:243264-HuGene_st:904401-HuGene_st:328536-HuGene_st:1095110-HuGene_st:89784-HuGene_st:206734-HuGene_st:927615-HuGene_st | 5.29E-13 | 2.2712 | 1.64 | 87.2 | 79.7-92.6 |
| PCK1 | 208383_s_at:221954-HuGene_st:772877-HuGene_st:518166-HuGene_st:1098164-HuGene_st:241175-HuGene_st:111987-HuGene_st:395761-HuGene_st:846308-HuGene_st:584532-HuGene_st:547720-HuGene_st:1018765-HuGene_st:817976-HuGene_st:454213-HuGene_st:66958-HuGene_st:301878-HuGene_st:804419-HuGene_st:442879-HuGene_st:211870-HuGene_st:638998-HuGene_st:448080-HuGene_st:948845-HuGene_st:313770-HuGene_st | 5.74E-13 | 2.3236 | 3.6 | 87.7 | 80.4-93 |
| ANK3 | 472262-HuGene_st:337923-HuGene_st:715126-HuGene_st:416201-HuGene_st:942091-HuGene_st:364373-HuGene_st:207950_s_at:209442_x_at:410 536-HuGene_st:206385_s_at:845022-HuGene_st:400240-HuGene_st:651770-HuGene_st:29904-HuGene_st:215314_at | 7.46E-13 | 2.2325 | 2.45 | 86.8 | 79.2-92.3 |
| F13A1 | 412872-HuGene_st:755197-HuGene_st:1023946-HuGene_st:591121-HuGene_st:912274-HuGene_st:465239-HuGene_st:1002989-HuGene_st:541053-HuGene_st:1052450-HuGene_st:204339-HuGene_st:176807-HuGene_st:58339-HuGene_st:42656-HuGene_st:965853-HuGene_st:1096456-HuGene_st:365069-HuGene_st:300786-HuGene_st:739769-HuGene_st:70605-HuGene_st:174382-HuGene_ st:203305_at:783884-HuGene_st | 8.82E-13 | 2.1855 | 4.74 | 86.3 | 78.6-91.9 |
| ADAMDE C1 | 893574-HuGene_st:1087946-HuGene_st:453119-HuGene_st:995166-HuGene_st:660805-HuGene_st:162940-HuGene_st:402188-HuGene_st:782577-HuGene_st:89831-HuGene_st:11993-HuGene_st:1020874-HuGene_st:997719-HuGene_st:268541-HuGene_st:41641-HuGene_st:1004377-HuGene_st:53970-HuGene_st:206134_at:904943-HuGene_st:644140-HuGene_st:332803-HuGene_st:16627-HuGene_st:1555-HuGene_st | 9.04E-13 | 2.2604 | 3.19 | 87.1 | 79.5-92.5 |
| ABI3BP | 801423-HuGene_st:1065486-HuGene_st:692353-HuGene_st:456260-HuGene_st:686533-HuGene_st:68008-HuGene_st:317350-HuGene_st:239950-HuGene_st:604210-HuGene_st:20407-HuGene_st:1099546-HuGene_st:395466-HuGene_st:548603-HuGene_st:931079-HuGene_st:159325-HuGene_st:251067-HuGene_st:1056116-HuGene_st:224253-HuGene_st:885069-HuGene_st:1075816-HuGene_st:592859-HuGene_st | 9.47E-13 | 2.2176 | 3.62 | 86.6 | 79-92.2 |
| PAG1 | 572231-HuGene_st:431317-HuGene_st:342766-HuGene_st:1080197-HuGene_st:225622_at:81123-HuGene_st:859237-HuGene_st:287765-HuGene_st:225626_at:743424-HuGene_st:759835-HuGene_st:622195-HuGene_st:208082-HuGene_st:768207-HuGene_st:657358-HuGene_st:506609-HuGene_st:226184-HuGene_st:540710-HuGene_st:236888-HuGene_st:36408-HuGene_st:546382-HuGene_st:498489-HuGene_st:227354_at | 1.11E-12 | 2.215 | 2.68 | 86.6 | 79-92.1 |
| KRT20 | 11827-HuGene_st:50958-HuGene_st:684746-HuGene_st:574501-HuGene_st:270813-HuGene_st:421213-HuGene_st:213953_at:225474-HuGene_st:310071-HuGene_st:356429-HuGene_st:997077-HuGene_st:963515-HuGene_st:944854-HuGene_st:178191-HuGene_st:613655-HuGene_st:337174-HuGene_st:268017-HuGene_st:81041-HuGene_st:85506-HuGene_st:798272-HuGene_st:925916-HuGene_st:780714-HuGene_ st:138025-HuGene_st | 1.26E-12 | 2.0981 | 5.49 | 85.3 | 77.4-91.1 |
| ENTPD5 | 760608-HuGene_st:231676_s_at:831252-HuGene_st:497317-HuGene_st:712688-HuGene_st:259180-HuGene_st:832617-HuGene_st:785663-HuGene_st:205757_at:553118-HuGene_st:384386-HuGene_st:911183-HuGene_st:440835-HuGene_st:150070-HuGene_st:821276-HuGene_st:14461-HuGene_st:520930-HuGene_st:181407-HuGene_st:294287-HuGene_st:226594_at:551172-HuGene_st:1040872-HuGene_st:1048020-HuGene_st | 1.43E-12 | 2.1686 | 3.01 | 86.1 | 78.3-91.7 |
| MALL | 209373_at:628866-HuGene_st:1092892-HuGene_st:488187-HuGene_st:515617-HuGene_st:649198-HuGene_st:991653-HuGene_st:301852-HuGene_st:1067925-HuGene_st:451072-HuGene_st:102723-HuGene_st:670639-HuGene_st:848482-HuGene_st:928502-HuGene_st:787192-HuGene_st:916920-HuGene_st:959742-HuGene_st:711672-HuGene_st:20252-HuGene_st:225337-HuGene_st | 2.10E-12 | 2.1585 | 3.96 | 86 | 78.2-91.7 |
| MFAP4 | 308095-HuGene_st:1004622-HuGene_st:754788-HuGene_st:212713_at:911082-HuGene_st:767889-HuGene_st:5569-HuGene_st:308458-HuGene_st:699815-HuGene_st:658908-HuGene_st:73048-HuGene_st:515283-HuGene_st:671512-HuGene_st:792571-HuGene_st:689375-HuGene_st:590963-HuGene_st:821074-HuGene_st:132543-HuGene_st:655171-HuGene_st:341975-HuGene_st:1048103-HuGene_st | 2.96E-12 | 2.1813 | 2.51 | 86.2 | 78.5-91.9 |
| SRI | 1018107-HuGene_st:929936-HuGene_st:596109-HuGene_st:469173-HuGene_st:235900-HuGene_st:303619-HuGene_st:792372-HuGene_st:1034405-HuGene_st:145638-HuGene_st:76828-HuGene_st:103032-HuGene_st:788729-HuGene_st:262807-HuGene_st:452805-HuGene_st:208921_s_at:863794-HuGene_st:172167-HuGene_st:309679-HuGene_st:1058573-HuGene_st:208920_at:235325-HuGene_st:813384-HuGene_st | 3.03E-12 | 2.0985 | 2.43 | 85.3 | 77.4-91.1 |
| SGK | 425210-HuGene_st:201739_at:137804-HuGene_st:182666-HuGene_st:271994-HuGene_st:629671-HuGene_st:350855-HuGene_st:179953-HuGene_st:1039396-HuGene_st:664449-HuGene_st:1049246-HuGene_st:1100037-HuGene_st:1094410-HuGene_st:677142-HuGene_st:231137-HuGene_st:393473-HuGene_st | 3.12E-12 | 2.1186 | 4.08 | 85.5 | 77.7-91.4 |
| IGHM | 1001583-HuGene_st:162285-HuGene_st:1061429-HuGene_st:359832-HuGene_st:60182-HuGene_st:1002550-HuGene_st:311771-HuGene_st:790352-HuGene_st:977059-HuGene_st:587401-HuGene_st:343597-HuGene_st:25657-HuGene_st:640259-HuGene_st:184414-HuGene_st:336659-HuGene_st:1008406-HuGene_st:1044209-HuGene_st:519020-HuGene_st:427467-HuGene_st:669553-HuGene_st:722843-HuGene_st:126162-HuGene_st:654578-HuGene_st:146515-HuGene_st:1100721-HuGene_st:823307-HuGene_st:899494-HuGene_st:952685-HuGene_st:215118_s_at:235399-HuGene_st:802521-HuGene_st:126175-HuGene_st:910847-HuGene_st:212827_at:722700-HuGene_st:173623-HuGene_st:1042953-HuGene_st:38807-HuGene_st:408026-HuGene_st | 3.25E-12 | 2.3285 | 1.73 | 87.8 | 80.4-93 |
| ZCWPW2 | 1001583-HuGene_st:162285-HuGene_st:1061429-HuGene_st:359832-HuGene_st:60182-HuGene_st:1002550-HuGene_st:311771-HuGene_st:790352-HuGene_st:977059-HuGene_st:587401-HuGene_st:343597-HuGene_st:25657-HuGene_st:640259-HuGene_st:184414-HuGene_st:336659-HuGene_st:1008406-HuGene_st:1044209-HuGene_ st:519020-HuGene_st:427467-HuGene_st:669553-HuGene_st:722843-HuGene_st:126162-HuGene_st:654578-HuGene_st:146515-HuGene_st:1100721-HuGene_st:823307-HuGene_st:899494-HuGene_st:952685-HuGene_st:215118_s_at:235399-HuGene_st:802521-HuGene_st:126175-HuGene_st:910847-HuGene_st:212827_at:722700-HuGene_st:173623-HuGene_st:1042953-HuGene_st:38807-HuGene_st:408026-HuGene_st | 3.25E-12 | 2.3264 | 1.73 | 87.8 | 80.4-93 |
| IL8 | 1001583-HuGene_st:162285-HuGene_st:1061429-HuGene_st:359832-HuGene_st:60182-HuGene_st:1002550-HuGene_st:311771-HuGene_st:790352-HuGene_st:977059-HuGene_st:587401-HuGene_st:343597-HuGene_st:25657-HuGene_st:640259-HuGene_st:184414-HuGene_st:336659-HuGene_st:1008406-HuGene_st:1044209-HuGene_st:519020-HuGene_st:427467-HuGene_st:669553-HuGene_st:722843-HuGene_st:126162-HuGene_st:654578-HuGene_st:146515-HuGene_st:1100721-HuGene_st:823307-HuGene_st:899494-HuGene_st:952685-HuGene_st:215118_s_at:235399-HuGene_st:802521-HuGene_st:126175-HuGene_st:910847-HuGene_st:212827_at:722700-HuGene_st:173623-HuGene_st:1042953-HuGene_st:38807-HuGene_st:408026-HuGene_st | 3.28E-12 | 2.3266 | 1.73 | 87.8 | 80.4-93 |
| IGHA1 | 1001583-HuGene_st:162285-HuGene_st:1061429-HuGene_st:359832-HuGene_st:60182-HuGene_st:1002550-HuGene_st:311771-HuGene_st:790352-HuGene_st:977059-HuGene_st:587401-HuGene_st:343597-HuGene_st:25657-HuGene_st:640259-HuGene_st:184414-HuGene_st:336659-HuGene_st:1008406-HuGene_st:235364_at:1044209-HuGene_st:519020-HuGene_st:427467-HuGene_st:669553-HuGene_st:722843-HuGene_st:208763_s_at:207001_x_at:126 162-HuGene_st:654578-HuGene_st:146515-HuGene_st:1100721-HuGene_st:823307-HuGene_st:899494-HuGene_st:952685-HuGene_st:215118_s_at:235399-HuGene_st:802521-HuGene_st:126175-HuGene_st:910847-HuGene_st:212827_at:722700-HuGene_st:173623-HuGene_st:1042953-HuGene_st:38807-HuGene_st:408026-HuGene_st | 3.28E-12 | 2.3276 | 1.73 | 87.8 | 80.4-93 |
| MGC14376 | 20793-HuGene_st:228915-HuGene_st:630688-HuGene_st:117325-HuGene_st:532108-HuGene_st:373670-HuGene_st:552164-HuGene_st:1004615-HuGene_st:527421-HuGene_st:880155-HuGene_st:10973-HuGene_st:395595-HuGene_st:312241-HuGene_st:644241-HuGene_st:214696_at:234825-HuGene_st:1002029-HuGene_st:1032648-HuGene_ st | 3.39E-12 | 2.1014 | 2.58 | 85.3 | 77.5-91.2 |
| ETHE1 | 1008653-HuGene_st:507181-HuGene_st:435886-HuGene_st:296660-HuGene_st:764234-HuGene_st:918966-HuGene_st:1078682-HuGene_st:789091-HuGene_st:100698-HuGene_st:204034_at:301334-HuGene_st:869871-HuGene_st:841094-HuGene_st:612751-HuGene_st:43763-HuGene_st:853527-HuGene_st:593768-HuGene_st:52787-HuGene_st:555225-HuGene_st:1028299-HuGene_st | 3.46E-12 | 2.2097 | 1.75 | 86.5 | 78.9-92.1 |
| CES2 | 817786-HuGene_st:63720-HuGene_st:527565-HuGene_st:678893-HuGene_st:209668_x_at:825633-HuGene_st:336873-HuGene_st:409854-HuGene_st:213509_x_at:134988-HuGene_st:446296-HuGene_st:842160-HuGene_st:827636-HuGene_st:209667_at:882043-HuGene_st:279172-HuGene_st:1081267-HuGene_st:756682-HuGene_st:90632-HuGene_ st | 3.51E-12 | 2.0993 | 2.87 | 85.3 | 77.4-91.1 |
| GPM6B | 599862-HuGene_st:754598-HuGene_st:503642-HuGene_st:224935-HuGene_ _st:754449-HuGene_st:577489-HuGene_st:1073242-HuGene_st:560873-HuGene_st:1003662-HuGene_st:430217-HuGene_st:903323-HuGene_st:231962-HuGene_ _st:244945-HuGene_st:562460-HuGene_ st:583561-HuGene_st:209168_at | 4.04E-12 | 2.1563 | 3.59 | 86 | 78.2-91.6 |
| SYNPO2 | 800385-HuGene_st:938576-HuGene_st:54552-HuGene_st:946854-HuGene_st:278208-HuGene_st:170777-HuGene_st:796143-HuGene_st:544243-HuGene_st:469048-HuGene_st:364454-HuGene_st:400491-HuGene_st:22204-HuGene_st:315299-HuGene_st:526384-HuGene_st:841867-HuGene_st:244108_at:2158-HuGene_st:283531-HuGene_st:407855-HuGene_st:225721_at:225720_at:225895_ at:225894_at:1017102-HuGene_st:227662_at:232119_at | 5.07E-12 | 2.1029 | 6.43 | 85.3 | 77.5-91.2 |
| GCG | 1044997-HuGene_st:682227-HuGene_st:368693-HuGene_st:25836-HuGene_st:228935-HuGene_st:1030502-HuGene_st:12466-HuGene_st:1098133-HuGene_st:994392-HuGene_st:581476-HuGene_st:799068-HuGene_st:350795-HuGene_st:705389-HuGene_st:981135-HuGene_st:736837-HuGene_st:253854-HuGene_st:1032660-HuGene_st:273941-HuGene_st:206422_at:940866-HuGene_ st:514731-HuGene_st | 5.58E-12 | 2.1165 | 12.56 | 85.5 | 77.7-91.3 |
| MGP | 696658-HuGene_st:618386-HuGene_st:202291_s_at:195765-HuGene_st:3486-HuGene_st:829319-HuGene_st:731815-HuGene_st:829950-HuGene_ st:601468-HuGene_st:366689-HuGene_st:460503-HuGene_st:712045-HuGene_st:545937-HuGene_st:148839-HuGene_st:704906-HuGene_st:612318-HuGene_st:708651-HuGene_st:722064-HuGene_st:846497-HuGene_st:1084237-HuGene_ st:854168-HuGene_st | 6.11E-12 | 2.1428 | 4.68 | 85.8 | 78-91.5 |
| PDE9A | 779446-HuGene_st:768516-HuGene_st:609563-HuGene_st:387613-HuGene_st:594122-HuGene_st:774367-HuGene_st:221476-HuGene_st:140410-HuGene_st:812534-HuGene_st:581440-HuGene_st:205593_s_at:142832-HuGene_st:767027-HuGene_st:464973-HuGene_st:97182-HuGene_st:128487-HuGene_st:206471-HuGene_st:878522-HuGene_st:237139_at:988884-HuGene_st:237283_at | 6.56E-12 | 2.145 | 3.91 | 85.8 | 78-91.5 |
| LRRC19 | 177641-HuGene_st:1070020-HuGene_st:1055140-HuGene_st:525999-HuGene_st:937256-HuGene_st:620791-HuGene_st:891251-HuGene_st:707559-HuGene_st:892056-HuGene_st:764919-HuGene_st:382143-HuGene_st:52584-HuGene_st:920414-HuGene_st:1028155-HuGene_st:755055-HuGene_st:678651-HuGene_st:1080156-HuGene_st:530282-HuGene_st:523877-HuGene_st:335198-HuGene_st:787709-HuGene_st:153175-HuGene_st:220376_at | 6.75E-12 | 2.0763 | 4.51 | 85 | 77.1-91 |
| HSD11B2 | 78582-HuGene_st:653519-HuGene_st:630341-HuGene_st:919091-HuGene_st:132633-HuGene_st:907106-HuGene_st:621993-HuGene_st:1089280-HuGene_ st:34729-HuGene_st:342165-HuGene_st:887157-HuGene_st:323862-HuGene_st:870050-HuGene_st:204130_at:635792-HuGene_st:606173-HuGene_st:867938-HuGene_st:113467-HuGene_st:806665-HuGene_ st | 6.97E-12 | 2.0895 | 2.53 | 85.2 | 77.3-91.1 |
| GNG2 | 1555766_a_at:224965_at:1011730-HuGene_st:77527-HuGene_st:669616-HuGene_st:223943_s_at:938512-HuGene_st:450519-HuGene_st:1025129-H uGene_st:30927-HuGene_st:382458-HuGene_st:330996-HuGene_st:545415-HuGene_st:774093-HuGene_st:758152-HuGene_st:176111-HuGene_st:743969-HuGene_ st | 7.25E-12 | 2.1437 | 1.91 | 85.8 | 78-91.5 |
| GCNT3 | 230376_at:254999-HuGene_st:476622-HuGene_st:219508_at:452671-HuGene_st:732602-HuGene_st:306264-HuGene_st:696871-HuGene_st:721027-HuGene_st:235453-HuGene_st:676136-HuGene_st:777254-HuGene_st:506598-HuGene_st:598423-HuGene_st:699538-HuGene_st:220940-HuGene_st:804174-HuGene_st:12527-HuGene_st:802770-HuGene_st:403287-HuGene_st:351721-HuGene_st:581784-HuGene_st:700724-HuGene_ st:398939-HuGene_st | 8.39E-12 | 2.0861 | 3.94 | 85.2 | 77.2-91 |
| UGT1A6 | 116025-HuGene_st:6488-HuGene_st:881135-HuGene_st:230953_at:221304_at:221305_ s_at:511516-HuGene_st:594963-HuGene_st:396121-HuGene_st:123777-HuGene_st:1016481-HuGene_st:204532_x_at:683377-HuGene_st:1055169-HuGene_st:1035103-HuGene_st:1088102-HuGene_st:207126_x_at:42874-HuGene_st:215125_s_at:206094_x_at:208 596_s_at:97211-HuGene_st:1009861-HuGene_st:603368-HuGene_st:232654_s_at:625897-HuGene_st:998604-HuGene_st | 1.32E-11 | 2.07 | 4.56 | 85 | 77-90.9 |
| UGT1A8 | 116025-HuGene_st:6488-HuGene_st:881135-HuGene_st:230953_at:221304_at:221305_ s_at:511516-HuGene_st:594963-HuGene_st:396121-HuGene_st:123777-HuGene_st:1016481-HuGene_st:204532_x_at:683377-HuGene_st:1055169-HuGene_st:1035103-HuGene_st:1088102-HuGene_st:207126_x_at:42874-HuGene_st:215125_s_at:206094_x_at:208 596_s_at:97211-HuGene_st:1009861-HuGene_ _st:603368-HuGene_st:232654_s_at:625897-HuGene_ st:998604-HuGene_st | 1.32E-11 | 2.0687 | 4.56 | 85 | 77-90.9 |
| UGT1A1 | 116025-HuGene_st:6488-HuGene_st:881135-HuGene_st:230953_at:221304_at:221305_ s_at:511516-HuGene_st:594963-HuGene_st:396121-HuGene_st:123777-HuGene_st:1016481-HuGene_st:204532_x_at:683377-HuGene_st:1055169-HuGene_st:1035103-H uGene_st:1088102-HuGene_st:207126_x_at:42874-HuGene_st:215125_s_at:206094_x_at:208 596_s_at:97211-HuGene_st:1009861-HuGene_st:603368-HuGene_st:232654_s_at:625897-HuGene_ st:998604-HuGene_st | 1.32E-11 | 2.0661 | 4.56 | 84.9 | 77-90.8 |
| UGT1A3 | 116025-HuGene_st:6488-HuGene_st:881135-HuGene_st:230953_at:221304_at:221305_ s_at:511516-HuGene_st:594963-HuGene_st:396121-HuGene_st:123777-HuGene_st:1016481-HuGene_st:204532_x_at:683377-HuGene_st:1055169-HuGene_st:1035103-HuGene_st:1088102-HuGene_st:207126_x_at:42874-HuGene_st:215125_s_at:206094_x_at:208 596_s_at:97211-HuGene_st:1009861-HuGene_st:603368-HuGene_st:232654_s_at:625897-HuGene_st:998604-HuGene_st | 1.32E-11 | 2.07 | 4.56 | 85 | 77-90.9 |
| UGT1A9 | 116025-HuGene_st:6488-HuGene_st:881135-HuGene_st:230953_at:221304_at:221305_ s_at:511516-HuGene_st:594963-HuGene_st:396121-HuGene_st:123777-HuGene_st:1016481-HuGene_st:204532_x_at:683377-HuGene_st:1055169-HuGene_st:1035103-HuGene_st:1088102-HuGene_st:207126_x_at:42874-HuGene_st:215125_s_at:206094_x_at:208 596_s_at:97211-HuGene_st:1009861-HuGene_st:603368-HuGene_st:232654_s_at:625897-HuGene_ st:998604-HuGene_st | 1.32E-11 | 2.0667 | 4.56 | 84.9 | 77-90.8 |
| PADI2 | 391522-HuGene_st:918392-HuGene_st:10163-HuGene_st:399093-HuGene_st:704572-HuGene_st:1554385_a_at:366338-HuGene_st:440876-HuGene_st:1075540-HuGene_st:963259-HuGene_st:994995-HuGene_st:315189-HuGene_st:272079-HuGene_st:243230-HuGene_st:96325-HuGene_st:793116-HuGene-st:362093-HuGene_st:390381-HuGene_st:690679-HuGene_st:209791_at:173109-HuGene_ st:339627-HuGene_st:1554384_at | 1.46E-11 | 2.0234 | 3.98 | 84.4 | 76.4-90.5 |
| CD177 | 993284-HuGene_st:444696-HuGene_st:79609-HuGene_st:969709-HuGene_st:369998-HuGene_st:219669_at:572266-HuGene_st:536281-HuGene_st:90319-HuGene_st:699790-HuGene_st:531312-HuGene_st:707358-HuGene_st:180891-HuGene_st:426820-HuGene_st:1006900-HuGene_st:638144-HuGene_st:232715-HuGene_st:134551-HuGene_st:609683-HuGene_st | 1.49E-11 | 2.0611 | 5.77 | 84.9 | 76.9-90.8 |
| FHL1 | 72610-HuGene_st:539308-HuGene_st:951657-HuGene_st:902973-HuGene_st:1001828-HuGene_st:286356-HuGene_st:899294-HuGene_st:25935-HuGene_st:214505_s_at:210298_x_at:210 299_s_at:602362-HuGene_st:1055343-HuGene_st:201680-HuGene_st:319870-HuGene_st:21197-HuGene_st:36473-HuGene_st:689087-HuGene_st:528922-HuGene_st:201539_s_at:1022628-HuGene_st:201540_at:437953-HuGene_st:746514-HuGene_st:829623-HuGene_st | 1.63E-11 | 2.0432 | 3.25 | 84.7 | 76.7-90.6 |
| CEACAM1 | 209498_at:206576_s_at:211889_x_at:864 113-HuGene_st:14136-HuGene_st:361816-HuGene_st:959455-HuGene_st:428485-HuGene_st:747162-HuGene_st:647202-HuGene_st:472587-HuGene_st:344791-HuGene_st:508072-HuGene_st:762013-HuGene_st:546799-HuGene_st:213733-HuGene_st:652484-HuGene_st:872278-HuGene_st:211883_x_at:309256-HuGene_st:699246-HuGene_st:698844-HuGene_st:677813-HuGene_st:26592-HuGene_st:210610_at:276021-HuGene_st:873164-HuGene_st | 2.66E-11 | 2.0448 | 2.85 | 84.7 | 76.7-90.6 |
| FBLN1 | 546550-HuGene_st:562183-HuGene_st:202995_s_at:507438-HuGene_st:361582-HuGene_st:134034-HuGene_st:804953-HuGene_st:122878-HuGene_st:331523-HuGene_st:1089938-HuGene_st:453797-HuGene_st:106596-HuGene_st:862139-HuGene_st:972691-HuGene_st:201787_at:687992-HuGene_st:478818-HuGene_st:991293-HuGene_st:328594-HuGene_st:844984-HuGene_st:202994_s_at:675821-HuGene_st:207835_at:55816-HuGene_st | 2.82E-11 | 2.0164 | 3.55 | 84.3 | 76.3-90.4 |
| SEPP1 | 803943-HuGene_st:717194-HuGene_st:113867-HuGene_st:286524-HuGene_st:228585-HuGene_st:824292-HuGene_st:754251-HuGene_st:150171-HuGene_st:904197-HuGene_st:116381-HuGene_st:267171-HuGene_st:8541 08-HuGene_st:201427_s_at:231669_at:32788 4-HuGene_st:136636-HuGene_st:164967-HuGene_st:1037558-HuGene_st:26213-HuGene_st:456616-HuGene_st:1000147-HuGene_st:748769-HuGene_st:658219-HuGene_st:242519_at:229620_at:237475_ x_at | 2.83E-11 | 2.0327 | 4.15 | 84.5 | 76.6-90.6 |
| TBC1D9 | 237091_at:730535-HuGene_st:659317-HuGene_st:68121-HuGene_st:899166-HuGene_st:785719-HuGene_st:138865-HuGene_st:953028-HuGene_st:20928-HuGene_st: 969812-HuGene_st:591384-HuGene_st:953516-HuGene_st:643103-HuGene_st:821110-HuGene_st:988068-HuGene_st:623144-HuGene_st:381615-HuGene_ st:708956-HuGene_st:212956_at | 3.89E-11 | 2.1454 | 1.54 | 85.8 | 78-91.5 |
| CA12 | 89078-HuGene_st:363685-HuGene_st:814108-HuGene_st:125567-HuGene_st:226479-HuGene_st:495486-HuGene_st:692924-HuGene_st:203963_at:65565-HuGene_st:963408-HuGene_st:1017708-HuGene_st:210735_s_at:1009602-HuGene_st:875965-HuGene_st:215867_x_at:749017-HuGene_st:638145-HuGene_st:204508_s_at:214164_x_at:104 9350-HuGene_st:189118-HuGene_st:698714-HuGene_st:1013062-HuGene_st:280470-HuGene_st:439438-HuGene_st:1049334-HuGene_st:204509_at:233388_at | 4.32E-11 | 2.0009 | 2.65 | 84.1 | 76-90.3 |
| DMD | 945600-HuGene_st:12596-HuGene_st:434657-HuGene_st:676962-HuGene_st:1020969-HuGene_st:170680-HuGene_st:855670-HuGene_st:909848-HuGene_st:134644-HuGene_st:887814-HuGene_st:649184-HuGene_st:110990-HuGene_st:914217-HuGene_st:961622-HuGene_st:514011-HuGene_st:842792-HuGene_st:445483-HuGene_st:208086_s_at:987362-HuGene_ st:997308-HuGene_st | 5.01E-11 | 2.0152 | 4.74 | 84.3 | 76.3-90.3 |
| SMPDL3A | 734740-HuGene_st:406928-HuGene_st:871227-HuGene_st:30922-HuGene_st:771860-HuGene_st:376657-HuGene_st:539013-HuGene_st:900092-HuGene_st:253658-HuGene_st:696711-HuGene_st:284449-HuGene_st:324159-HuGene_st:70962-HuGene_st:161952-HuGene_ st:873946-HuGene_st:588668-HuGene_st:681531-HuGene_st:195216-HuGene_st:213624_at:471149-HuGene_st:1562512_at | 5.84E-11 | 1.941 | 3.14 | 83.4 | 75.2-89.7 |
| HSPB6 | 438264-HuGene_st:771601-HuGene_st:17884-HuGene_st:587849-HuGene_st:923983-HuGene_st:214767_s_at:226304_at:76350 5-HuGene_st:835176-HuGene_st:868647-HuGene_st:511313-HuGene_st:848921-HuGene_st | 6.16E-11 | 1.9492 | 6.88 | 83.5 | 75.4-89.8 |
| AKR1B10 | 1017995-HuGene_st:244038-HuGene_st:943992-HuGene_st:690334-HuGene_st:486646-HuGene_st:234015-HuGene_st:599681-HuGene_ _st:206561_s_at:347314-HuGene_st:827689-HuGene_st:212880-HuGene_st:516961-HuGene_st:638468-HuGene_ st | 7.91E-11 | 1.9484 | 5.17 | 83.5 | 75.3-89.7 |
| UGDH | 961600-HuGene_st:1046173-HuGene_st:509128-HuGene_st:428595-HuGene_st:363704-HuGene_st:519385-HuGene_st:319601-HuGene_st:653273-HuGene_st:121518-HuGene_st:786461-HuGene_st:470924-HuGene_st:530032-HuGene_st:810299-HuGene_st:954191-HuGene_st:203343_at:855306-HuGene_st:1075627-HuGene_st:184297-HuGene_st:775350-HuGene_st:170400-HuGene_st:374055-HuGene_st:186342-HuGene_st | 8.72E-11 | 1.9628 | 2.4 | 83.7 | 75.5-89.9 |
| FABP1 | 825235-HuGen-HuGene_st:626063-HuGene_st:625600-HuGene_st:909598-HuGene_st:350768-HuGene_st:760377-HuGene_st:52477-HuGene_st:421055-HuGene_st:205892_s_at:231693_at:69993 2-HuGene_st:561606-HuGene_st:791463-HuGene_st:495764-HuGene_st:703121-HuGene_st:1025066-HuGene_st:392859-HuGene_st:902525-HuGene_st:81171-HuGene_st:1073289-HuGene_st:219434-HuGene_st:735424-HuGene_st:297976-HuGene_ st | 9.02E-11 | 1.968 | 6.54 | 83.7 | 75.6-89.9 |
| P2RY14 | 528057-HuGene_st:780310-HuGene_st:235178-HuGene_st:352954-HuGene_st:699489-HuGene_st:38001-HuGene_st:637791-HuGene_st:25606-HuGene_st:40647-HuGene_st:896487-HuGene_st:672149-HuGene_st:863820-HuGene_st:352427-HuGene_st:632821-HuGene_st:116148-HuGene_st:561792-HuGene_st:840910-HuGene_st:296420-HuGene_st:974643-HuGene_st:206637_at | 1.31E-10 | 1.9821 | 2 | 83.9 | 75.8-90 |
| TTRAP | 369720-HuGene_st:914515-HuGene_st:43073-HuGene_st:85920-HuGene_st:278231-HuGene_st:345627-HuGene_st:358211-HuGene_st:331406-HuGene_st:748071-HuGene_st:840774-HuGene_st:384150-HuGene_st:528293-HuGene_st:202266_at:784674-HuGene_st:370526-HuGene_st:1031392-HuGene_st:258400-HuGene_st:1084398-HuGene_st:223012-HuGene_st:1033688-HuGene_ st:232744-HuGene_st | 1.59E-10 | 1.9196 | 2.2 | 83.1 | 74.9-89.4 |
| MEP1A | 854525-HuGene_st:233471-HuGene_st:521357-HuGene_st:398055-HuGene_st:479853-HuGene_st:686103-HuGene_st:711452-HuGene_st:984096-HuGene_st:1083396-HuGene_st:1058253-HuGene_st:951000-HuGene_st:984505-HuGene_st:390982-HuGene_st:289221-HuGene_st:1044210-HuGene_st:700879-HuGene_st:368627-HuGene_st:1081805-HuGene_st:206000_at:54151-HuGene_st:558849-HuGene_st:200136-HuGene_ st:519266-HuGene_st | 2.07E-10 | 1.9887 | 3.17 | 84 | 76-90.1 |
| MYH11 | 628303-HuGene_st:20510-HuGene_st:77517-HuGene_st:847369-HuGene_st:396579-HuGene_st:751338-HuGene_st:56832-HuGene_st:227843_at:643005-HuGene_st:410559-HuGene_st:618203-HuGene_st:64921-HuGene_st:1083141-HuGene_st:724450-HuGene_st:755245-HuGene_st:947574-HuGene_st:201496_x_at:482335-HuGene_st:456670-HuGene_st:974777-HuGene_st:86441-HuGene_st:1066402-HuGene_st:289331-HuGene_st:888083-HuGene_st:201495_x_at:201497_x_at:207 961_x_at:1568760_at:239307_at | 2.15E-10 | 1.8602 | 5.35 | 82.4 | 74-88.8 |
| MAOA | 234534_at:353863-HuGene_st:1038534-HuGene_st:732300-HuGene_st:204389_at:204388_s_at:21167 0-HuGene_st:360481-HuGene_st:151427-HuGene_st:347487-HuGene_st:791116-HuGene_st:212741_at:1003275-HuGene_st:24543-HuGene_st:126920-HuGene_st:500217-HuGene_st:434659-HuGene_st:73861-HuGene_st:382884-HuGene_st:675946-HuGene_st:108162-HuGene_st:119160-HuGene_st | 2.49E-10 | 2.0268 | 2.18 | 84.5 | 76.5-90.5 |
| CLDN8 | 1018006-HuGene_st:190634-HuGene_st:590280-HuGene_st:186468-HuGene_st:954438-HuGene_st:428391-HuGene_st:480543-HuGene_st:944337-HuGene_st:179725-HuGene_st:508584-HuGene_st:1009114-HuGene_st:948216-HuGene_st:658285-HuGene_st:1022600-HuGene_st:737498-HuGene_st:470015-HuGene_st:103315-HuGene_st:699348-HuGene_st:89877-HuGene_st:56937-HuGene_st:862663-HuGene_st:504945-HuGene_st:214598_at | 2.92E-10 | 1.8632 | 12.96 | 82.4 | 74-88.9 |
| FUCA1 | 295335-HuGene_st:550769-HuGene_st:674252-HuGene_st:343732-HuGene_st:10170-HuGene_st:1012064-HuGene_st:296930-HuGene_st:179394-HuGene_st:587765-HuGene_st:229137_at: 142430-HuGene_st:801038-HuGene_st:447607-HuGene_st:66284-HuGene_st:937530-HuGene_st:884152-HuGene_st:674608-HuGene_st:202838_at:106313-HuGene_st:602308-HuGene_st:887004-HuGene_st:206251-HuGene_st:357516-HuGene_st | 4.24E-10 | 1.8845 | 2.03 | 82.7 | 74.4-89.1 |
| FXYD6 | 138547-HuGene_st:814211-HuGene_st:185591-HuGene_st:992039-HuGene_st:549291-HuGene_st:921510-HuGene_st:163300-HuGene_st:4792-HuGene_st:369594-HuGene_st:209348-HuGene_st:1028937-HuGene_st:77232-HuGene_st:529384-HuGene_st:217897_at:877189-HuGene_st:973770-HuGene_st:1054980-HuGene_ st:240323_at:466048-HuGene_st | 6.39E-10 | 1.8087 | 2.92 | 81.7 | 73.3-88.3 |
| ARL14 | 220468_at:471121-HuGene_st:690873-HuGene_st:468858-HuGene_st:750196-HuGene_st:62407-HuGene_st:159605-HuGene_st:979866-HuGene_st:1032832-HuGene_st:446532-HuGene_st:861783-HuGene_st:116203-HuGene_st:991610-HuGene_st:515168-HuGene_st:1073679-HuGene_st:439428-HuGene_st:976681-HuGene_st:452044-HuGene_st:197209-HuGene_st:12441-HuGene_st | 7.01E-10 | 1.8255 | 3.06 | 81.9 | 73.5-88.4 |
| SLC20A1 | 335219-HuGene_st:230038-HuGene_st:1078924-HuGene_st:520074-HuGene_st:769870-HuGene_st:175687-HuGene_st:541149-HuGene_st:973433-HuGene_st:643658-HuGene_st:220784-HuGene_st:175599-HuGene_st:482373-HuGene_st:1003908-HuGene_st:337454-HuGene_st:655800-HuGene_st:321368-HuGene_st:230494_at:178021-HuGene_st:201920_at:448729-HuGene_st:896684-HuGene_st:363789-HuGene_st:130920-HuGene_st:786196-HuGene_st | 7.65E-10 | 1.7854 | 4.04 | 81.4 | 72.9-88.1 |
| MAB21L2 | 338297-HuGene_st:994188-HuGene_st:109548-HuGene_st:395419-HuGene_st:922092-HuGene_st:737843-HuGene_st:6731-HuGene_st:708698-HuGene_ st:352943-HuGene_st:485256-HuGene_st:1023066-HuGene_st:825747-HuGene_st:210303_at:309088-HuGene_st: 1031483-HuGene_st:441727-HuGene_st:751700-HuGene_st:974270-HuGene_st:210302_s_at:254681-HuGene _st:982880-HuGene_st | 1.05E-09 | 1.7739 | 5.2 | 81.2 | 72.7-87.9 |
| TSPAN1 | 833585-HuGene_st:209114_at:664359-HuGene_st:307816-HuGene_st:111939-HuGene_st:39185-HuGene_st:496300-HuGene_st:1038310-HuGene_st:401084-HuGene_st:632040-HuGene_st:1076526-HuGene_st:488552-HuGene_st:35199-HuGene_st:611793-HuGene_st:1007567-HuGene_st:305523-HuGene_st:1033895-HuGene_st:145680-HuGene_st:812712-HuGene_st:929225-HuGene_st:641156-HuGene_ st:116814-HuGene_st | 1.16E-09 | 1.7875 | 2.16 | 81.4 | 73-88.1 |
| KLF4 | 310345-HuGene_st:421025-HuGene_st:853068-HuGene_st:1040228-HuGene_st:1040166-HuGene_st:549197-HuGene_st:257340-HuGene_st:95161-HuGene_st:166058-HuGene_st:749339-HuGene_st:212619-HuGene_st:220266_s_at:1078952-HuGene_st:289946-HuGene_st:824845-HuGene_st:965293-HuGene_st:467978-HuGene_st:287320-HuGene_st:334405-HuGene_st:234619-HuGene_st:221841_s_at:145954-HuGene_st:733888-HuGene_st | 1.19E-09 | 1.8535 | 2.27 | 82.3 | 74-88.8 |
| CRYAB | 581894-HuGene_st:42986-HuGene_st:626903-HuGene_st:612490-HuGene_st:1017597-HuGene_st:473983-HuGene_st:380047-HuGene_st:25982-HuGene_st:298978-HuGene_st:485492-HuGene_st:413012-HuGene_st:944836-HuGene_st:209283_at:615857-HuGene_st:303036-HuGene_st:592729-HuGene_st:349645-HuGene_st:226566-HuGene_st:215779-HuGene_st:702453-HuGene_st:95542-HuGene_st:697816-HuGene_ st | 1.47E-09 | 1.768 | 3.91 | 81.2 | 72.7-87.9 |
| C15orf48 | 661133-HuGene_st:240581-HuGene_st:302164-HuGene_st:262469-HuGene_st:512593-HuGene_st:122711-HuGene_st:269066-HuGene_st:246950-HuGene_st:483493-HuGene_st:352750-HuGene_st:645087-HuGene_st:775549-HuGene_st:223484_at:521885-HuGene_st:1004430-HuGene_st:885617-HuGene_st:166185-HuGene_st:752633-HuGene_st:1056973-HuGene_st:60479-HuGene_ st | 1.54E-09 | 1.8166 | 3.07 | 81.8 | 73.5-88.4 |
| ACAT1 | 205412_at:877718-HuGene_st:697995-HuGene_st:244911-HuGene_st:705306-HuGene_st:110401-HuGene_st:31594-HuGene_st:19333-HuGene_st:499096-HuGene_st:942019-HuGene_st:618952-HuGene_st:933332-HuGene_st:715946-HuGene_st:676239-HuGene_st:172044-HuGene_st:828361-HuGene_st:321829-HuGene_st:897946-HuGene_st:331003-HuGene_st:1559239_s_at:814756-HuGene_st | 1.70E-09 | 1.8589 | 1.77 | 82.4 | 74-88.8 |
| ANPEP | 42832-HuGene_st:567567-HuGene_st:992744-HuGene_st:524001-HuGene_st:229640-HuGene_st:372330-HuGene_st:842255-HuGene_st:42234-HuGene_st:640878-HuGene_st:702098-HuGene_st:917110-HuGene_st:25968-HuGene_st:850385-HuGene_st:503143-HuGene_st:202888_s_at:429651-HuGene_st:409574-HuGene_st:60717-HuGene_st:998436-HuGene_st:618326-HuGene_st:1067290-HuGene_st:333846-HuGene_st | 2.04E-09 | 1.7687 | 8.18 | 81.2 | 72.7-87.9 |
| FGL2 | 884418-HuGene_st:249194-HuGene_st:463016-HuGene_st:194197-HuGene_st:789123-HuGene_st:998752-HuGene_st:596140-HuGene_st:896312-HuGene_st:762195-HuGene_st:909927-HuGene_st:686836-HuGene_st:381278-HuGene_st:463952-HuGene_st:757331-HuGene_st:206261-HuGene_st:706108-HuGene_st:202575-HuGene_st:148017-HuGene_st: 199906-HuGene_st:707754-HuGene_st:204834_at | 2.64E-09 | 1.8254 | 2.07 | 81.9 | 73.5-88.5 |
| PPID | 204185_x_at:204186_s_at | 3.34E-09 | 1.8025 | 1.73 | 81.6 | 73.2-88.2 |
| ITM2C | 83688-HuGene_st:398134-HuGene_st:972817-HuGene_st:698719-HuGene_st:369206-HuGene_st:377066-HuGene_st:23436-HuGene_st:593996-HuGene_st:987556-HuGene_st:221004_s_at:465490-HuGene_st:776424-HuGene_st:1052550-HuGene_st:344231-HuGene_st:1039973-HuGene_st:127312-HuGene_st:577346-HuGene_st:394326-HuGene_st:848902-HuGene_st | 3.87E-09 | 1.7063 | 2.22 | 80.3 | 71.7-87.2 |
| SST | 477599-HuGene_st:1101191-HuGene_st:474819-HuGene_st:144116-HuGene_st:76078-HuGene_st:698582-HuGene_st:600708-HuGene_st:98570-HuGene_st:4754-HuGene_st:675107-HuGene_st:213921_at:115911-HuGene_st:235647-HuGene_st:131210-HuGene_st:502176-HuGene_st:63243-HuGene_st | 4.16E-09 | 1.7627 | 6.14 | 81.1 | 72.6-87.8 |
| PRIMA1 | 1084922-HuGene_st:1008125-HuGene_st:22062-HuGene_st:182482-HuGene_st:225291-HuGene_st:566243-HuGene_st:50975-HuGene_st:230087_at:787213-HuGene_st:661160-HuGene_st:256004-HuGene_st:6276-HuGene_st:44738-HuGene_st | 4.74E-09 | 1.7053 | 2.75 | 80.3 | 71.7-87.1 |
| EFEMP1 | 201842_s_at:1073952-HuGene_st:228421_s_at:520435-HuGene_st:551581-HuGene_st:909695-HuGene_st:539520-HuGene_st:719105-HuGene_st:1046788-HuGene_st:728978-HuGene_st:956186-HuGene_st:89258-HuGene_st:155486-HuGene_st:986491-HuGene_st:207603-HuGene_st:243191-HuGene_st:842290-HuGene_st:757697-HuGene_st:784694-HuGene_st:132382-HuGene_st | 5.80E-09 | 1.8208 | 1.69 | 81.9 | 73.4-88.4 |
| ADAMTS1 | 432578-HuGene_st:139364-HuGene_st:423315-HuGene_st:578666-HuGene_st:419311-HuGene_st:928538-HuGene_st:344696-HuGene_st:617885-HuGene_st:136304-HuGene_st:75724-HuGene_st:412640-HuGene_st:368117-HuGene_st:97806-HuGene_st:641285-HuGene_st:222486_s_at:711563-HuGene_st:1006281-HuGene_st:222162_s_at:32418-HuGene_st | 6.42E-09 | 1.7467 | 2.37 | 80.9 | 72.4-87.6 |
| AKAP12 | 212419-HuGene_st:231067_s_at:379659-HuGene_st:1010338-HuGene_st:1075094-HuGene_st:42401-HuGene_st:522584-HuGene_st:480972-HuGene_st:948623-HuGene_st:701945-HuGene_st:276784-HuGene_st:64858-HuGene_st:210517_s_at:874382-HuGene_st:909976-HuGene_st:182037-HuGene_st:417182-HuGene_st:722881-HuGene_st | 7.41E-09 | 1.744 | 2.83 | 80.8 | 72.2-87.5 |
| COL14A1 | 216866_s_at:309808-HuGene_st:532905-HuGene_st:938881-HuGene_st:184937-HuGene_st:332362-HuGene_st:431855-HuGene_st:295715-HuGene_st:813208-HuGene_st:216865_at:426456-HuGene_st:212865_s_at:513900-HuGene_st:1054393-HuGene_st:947013-HuGene _st:966609-HuGene_st:929330-HuGene_st:283165-HuGene_st:67940-HuGene_st:655615-HuGene_st:236228-HuGene_ st | 7.60E-09 | 1.7817 | 1.67 | 81.3 | 72.9-88 |
| UGT2A3 | 149647-HuGene_st:860083-HuGene_st:922544-HuGene_st:244206-HuGene_st:503323-HuGene_st:353576-HuGene_st:603619-HuGene_st:787458-HuGene_st:219796-HuGene_st:333564-HuGene_st:257402-HuGene_st:366699-HuGene_st:461685-HuGene_st:891681-HuGene_st:644952-HuGene_st:621618-HuGene_st:737617-HuGene_st:88682-HuGene_st:529761-HuGene_st:895203-HuGene_st:658594-HuGene_st:455115-HuGene_st | 8.64E-09 | 1.7414 | 4.89 | 80.8 | 72.2-87.5 |
| PTGIS | 162553-HuGene_st:277727-HuGene_st:426160-HuGene_st:49754-HuGene_st:491989-HuGene_st:846155-HuGene_st:214744-HuGene_st:81045-HuGene_st:757700-HuGene_st:713439-HuGene_st:765856-HuGene_st:211892_s_at:424645-HuGene_st:805861-HuGene_st:16678-HuGene_st:49009-HuGene_st:210702_s_at:33677-HuGene_st:757535-HuGene_st:1065183-HuGene_ st:949066-HuGene_st | 9.15E-09 | 1.7213 | 3.98 | 80.5 | 72-87.3 |
| BEST2 | 242542-HuGene_st:368041-HuGene_st:316182-HuGene_st:417689-HuGene_st:953359-HuGene_st:196857-HuGene_st:2545-HuGene_st:207432_at:371039-HuGene_st:105864-HuGene_st:946835-HuGene_st:371107-HuGene_st:170027-HuGene_st:238631-HuGene_st:515622-HuGene_st:886418-HuGene_st:396311-HuGene_st:735639-HuGene_st:947978-HuGene_st:882286-HuGene_st:894968-HuGene_st | 9.19E-09 | 1.7181 | 3.63 | 80.5 | 71.9-87.3 |
| MS4A4A | 651445-HuGene_st:308636-HuGene_st:174209-HuGene_st:486453-HuGene_st:219607_s_at:1041327-HuGene_st:84116-HuGene_st:161192-HuGene_st:491070-HuGene_st:1555728_a_at:142937-HuGene_st:581150-HuGene_st:579786-HuGene_st:588041-HuGene_st:224357_s_at:886807-HuGene_st:122093-HuGene_st | 9.59E-09 | 1.8179 | 1.55 | 81.8 | 73.4-88.4 |
| SGCE | 976641-HuGene_st:204688_at:241763-HuGene_st:83484-HuGene_st:281261-HuGene_st:324304-HuGene_st:683152-HuGene_st:850448-HuGene_st:254097-HuGene_st:992961-HuGene_st:69099-HuGene_st:781384-HuGene_st:899200-HuGene_st:974761-HuGene_st:417302-HuGene_st:211350-HuGene_st:198065-HuGene_ st | 1.02E-08 | 1.7444 | 2.03 | 80.8 | 72.3-87.6 |
| TPSB2 | 207134_x_at:207741_x_at | 1.24E-08 | 1.3708 | 1.87 | 75.3 | 66.3-83 |
| COL6A2 | 919359-HuGene_st:40510-HuGene_st:375216-HuGene_st:1086501-HuGene_st:160736-HuGene_st:602554-HuGene_st:148827-HuGene_st:794188-HuGene_st:213290_at:536399-HuGene_st:501950-HuGene_st:404866-HuGene_st:209156_s_at:813295-HuGene_st | 1.30E-08 | 1.6771 | 2.08 | 79.9 | 71.2-86.8 |
| PRKACB | 202741_at:795011-HuGene_st:360410-HuGene_st:970814-HuGene_st:70528-HuGene_st:1042799-HuGene_st:202742_s_at:964845-HuGene_st:563484-HuGene_st:142353-HuGene_st:597859-HuGene_st:660092-HuGene_st:948901-HuGene_st:301751-HuGene_st:294241-HuGene_st:694446-HuGene_st:309819-HuGene_st:668357-HuGene_ st:589422-HuGene_st | 1.30E-08 | 1.7234 | 2.18 | 80.6 | 72-87.3 |
| SPARCL1 | 246509-HuGene_st:300147-HuGene_st:525246-HuGene_st:380181-HuGene_st:257526-HuGene_st:585652-HuGene_st:733855-HuGene_st:517768-HuGene_st:853991-HuGene_st:854537-HuGene_st:1023002-HuGene_st:55936-HuGene_st:371227-HuGene_st:782475-HuGene_st:670571-HuGene_st:1026998-HuGene_st:894310-HuGene_st:948826-HuGene_st:302303-HuGene_st:151789-HuGene_st:905519-HuGene_ st:200795_at:602848-HuGene_st | 1.33E-08 | 1.634 | 2.85 | 79.3 | 70.5-86.3 |
| PBLD | 1003993-HuGene_st:308979-HuGene_st:622498-HuGene_st:840594-HuGene_st:84006-HuGene_st:636001-HuGene_st:529256-HuGene_st:651369-HuGene_st:379972-HuGene_st:492235-HuGene_st:584905-HuGene_st:222771-HuGene_st:383791-HuGene_st:1016249-HuGene_st:819013-HuGene_st:1555175_a_at:115400-HuGene_st:969544-HuGene_st:949581-HuGene_st:897360-HuGene_ st:219543_at:548506-HuGene_st | 1.38E-08 | 1.6983 | 2.74 | 80.2 | 71.5-87 |
| PDK4 | 806272-HuGene_st:44901-HuGene_st:272445-HuGene_st:650209-HuGene_st:796256-HuGene_st:342230-HuGene_st:648024-HuGene_st:205960_at:25700-HuGene_st:152414-HuGene_st:360382-HuGene_st:471160-HuGene_st:219862-HuGene_st:584279-HuGene_st:225207_at:498670-HuGene_st:405050-HuGene_st:546964-HuGene_st:735039-HuGene_st:1562321_at:348010-HuGene_st:1073661-HuGene_st:578954-HuGene_st:966364-HuGene_st:115766-HuGene_st | 1.45E-08 | 1.7059 | 3.02 | 80.3 | 71.8-87.1 |
| DES | 680953-HuGene_st:710186-HuGene_st:224168-HuGene_st:720121-HuGene_st:632911-HuGene_st:14146-HuGene _st:472740-HuGene_st:452392-HuGene_st:70983-HuGene_st:626407-HuGene_st:330060-HuGene_st:678707-HuGene_st:202222_s_at:830447-HuGene_ st:466888-HuGene_st:832644-HuGene_st:190691-HuGene_st:1043063-HuGene_ st:476251-HuGene_st:856127-HuGene_st:680072-HuGene_st:1053701-HuGene_ st:490364-HuGene_st:954860-HuGene_st:7755-HuGene_st:856708-HuGene_st:272796-HuGene_st:377421-HuGene_st:214027_x_at:458352-HuGene_st:104906-HuGene_st:276614-HuGene_ st | 1.47E-08 | 1.7257 | 6.66 | 80.6 | 72-87.4 |
| MMP28 | 578497-HuGene_st:31358-HuGene_st:283053-HuGene_st:152786-HuGene_st:519390-HuGene_st:239273_s_at:599795-HuGene_st:800954-HuGene_st:219909_at:190189-HuGene_st:222937_s_at:1084226-HuGene_st:315451-HuGene_st:100436-HuGene_st:794275-HuGene_st:256212-HuGene_st:8583-HuGene_st:987411-HuGene_st:239272_at:224207_x_at:21241 8-HuGene_st:319017-HuGene_st:604727-HuGene _st | 1.80E-08 | 1.663 | 2.47 | 79.7 | 71.1-86.7 |
| CYBRD1 | 580256-HuGene_st:931026-HuGene_st:412808-HuGene_st:138769-HuGene_st:199471-HuGene_st:675528-HuGene_st:217889_s_at:570877-HuGene_st:438204-HuGene_st:836676-HuGene_st:251906-HuGene_st:71842-HuGene_st:140118-HuGene_st:266609-HuGene_st:132102-HuGene_st:774519-HuGene_st:1068423-HuGene_st:995512-HuGene_st:298248-HuGene_st:1099283-HuGene_st:222189-HuGene_ st:222453_at:232459_at | 1.84E-08 | 1.6939 | 2.7 | 80.1 | 71.6-87 |
| IL6ST | 1054392-HuGene_st:234474_x_at:953995-HuGene_st:234967_at:48974-HuGene_st:325364-HuGene_st:32344-HuGene_st:821422-HuGene_st:298043-HuGene_st:606460-HuGene_st:1074930-HuGene_st:44790-HuGene_st:204864_s_at:1059165-HuGene_st:288593-HuGene_st:39514-HuGene_st:204863_s_at:175414-HuGene_st:293958-HuGene_st:212196_at:148922-HuGene_st:211000_s_at:648367-HuGene_st:240067-HuGene_st:212195_at:822552-HuGene_ st:523608-HuGene_st:474929-HuGene st | 1.87E-08 | 1.7642 | 1.58 | 81.1 | 72.6-87.8 |
| FABP4 | 547124-HuGene_st:96292-HuGene_st:545014-HuGene_st:22370-HuGene_st:138079-HuGene_st:1064936-HuGene_st:804933-HuGene_st:1087992-HuGene_st:20784-HuGene_st:671128-HuGene_st:328798-HuGene_st:594739-HuGene_st:1047009-HuGene_st:825613-HuGene_st:637074-HuGene_st:759940-HuGene_st:754024-HuGene_st:88372-HuGene_st | 2.01E-08 | 1.6776 | 5.55 | 79.9 | 71.2-86.8 |
| CALM1 | 196751-HuGene_st:1094382-HuGene_st:211985_s_at:445194-HuGene_st:211984_at:213688_at:213710_ s_at:444099-HuGene_st:708994-HuGene_st:418220-HuGene_st:241614_at:96889-HuGene_st:50211-HuGene_st:200653_s_at:767359-HuGene_st:200655_s_at:616208-HuGene_st:1019255-HuGene_st:209563_x_at:715468-HuGene_st:18183-HuGene_st:544779-HuGene_st:239705-HuGene_st:1084911-HuGene_st:98189-HuGene_st | 2.12E-08 | 1.6819 | 1.72 | 80 | 71.3-86.9 |
| TNS1 | 766751-HuGene_st:503077-HuGene_st:455229-HuGene_st:905238-HuGene_st:129966-HuGene_st:750627-HuGene_st:989199-HuGene_st:61598-HuGene_st:671515-HuGene_st:129638-HuGene_st:1088728-HuGene_st:693249-HuGene_st:418601-HuGene_st:168879-HuGene_st:220476-HuGene_st:23579-HuGene_st:731710-HuGene_st:476750-HuGene_st:295235-HuGene_st:218863_s_at:665955-HuGene_st:221747_at:105442-HuGene_st:676236-HuGene_st:124848-HuGene_st:474353-HuGene_st:218864_at:925862-HuGene_st:279934-HuGene_st:1008295-HuGene_st:221246_x_at:384388-HuGene_st:522313-HuGene_st:754212-HuGene_st:221748_s_at:420410-HuGene_st:250619-HuGene_st | 2.21E-08 | 1.7412 | 2.01 | 80.8 | 72.3-87.5 |
| EMP1 | 201324_at:311769-HuGene_st:201325_s_at:506469-HuGene_st:861706-HuGene_st:46080-HuGene_st:236046-HuGene_st:548197-HuGene_st:100442-HuGene_st:1096474-HuGene_st:717809-HuGene_st:596930-HuGene_st:468199-HuGene_st:751888-HuGene_st:166375-HuGene_st:213895_at:871061-HuGene_st:164793-HuGene_st:509462-HuGene_st:182838-HuGene _st:1564796_at:446286-HuGene_st | 2.59E-08 | 1.7156 | 1.95 | 80.4 | 71.9-87.3 |
| RDX | 350620-HuGene_st:1005467-HuGene_st:284799-HuGene_st:78392-HuGene_st:923159-HuGene_st:204969_s_at:935366-HuGene_st:603982-HuGene_st:418137-HuGene_st:304555-HuGene_st:174237-HuGene_st:140908-HuGene_st:1034349-HuGene_st:212398_at:977130-HuGene_st:6485-HuGene_st | 3.06E-08 | 1.7426 | 1.9 | 80.8 | 72.2-87.6 |
| CFD | 396618-HuGene_st:672277-HuGene_st:290815-HuGene_st:304988-HuGene_st:580394-HuGene_st:605542-HuGene_st:698498-HuGene_st:580177-HuGene_st:633095-HuGene_st:35339-HuGene_st:530155-HuGene_st:584306-HuGene_st:205382_s_at:915940-HuGene_st:859510-HuGene_st:978939-HuGene_st:59414-HuGene_st:557142-HuGene_ st:104453-HuGene_st | 3.06E-08 | 1.6465 | 2.81 | 79.5 | 70.8-86.4 |
| GPNMB | 201141_at:25823-HuGene_st:926046-HuGene_st:724736-HuGene_st:591503-HuGene_st:660677-HuGene_st:701912-HuGene_st:267933-HuGene_st:799615-HuGene_st:569803-HuGene_st:979125-HuGene_st:38037-HuGene_st:89805-HuGene_st:335128-HuGene_st:131027-HuGene_st:908272-HuGene_st:950837-HuGene_st:39441-HuGene_st:1056618-HuGene_ st:860466-HuGene_st | 3.11E-08 | 1.6924 | 1.99 | 80.1 | 71.5-87 |
| TPSAB1 | 350712-HuGene_st:134899-HuGene_st:122333-HuGene_st:1098415-HuGene_st:665622-HuGene_st:701849-HuGene_st:535697-HuGene_st:163189-HuGene_st:744603-HuGene_st:153098-HuGene_st:216485_s_at:216474_x_at:205 683_x_at:416078-HuGene_st:162760-HuGene_st:217023_x_at:520284-HuGene_st:215382_x_at:210084_x_at:567 472-HuGene_st:519234-HuGene_st:154829-HuGene_st:247406-HuGene_st:331572-HuGene_st:264147-HuGene_st:462114-HuGene_st:880961-HuGene_ st:705156-HuGene_st | 3.52E-08 | 1.7066 | 1.93 | 80.3 | 71.7-87.2 |
| PPP1R14A | 374630-HuGene_st:551938-HuGene_st:783588-HuGene_st:44913-HuGene_st:966135-HuGene_st:227006_at:537429-HuGene_st:815370-HuGene_st:1040980-HuGene_ st:76662-HuGene_st | 3.54E-08 | 1.6167 | 2.51 | 79.1 | 70.3-86.1 |
| LOC25301 2 | 873053-HuGene_st:945097-HuGene_st:896826-HuGene_st:363568-HuGene_st:162938-HuGene_st:441915-HuGene_st:1070854-HuGene_st:463072-HuGene_st:199784-HuGene_st:304834-HuGene_st:381523-HuGene_st:568837 - HuGene_st:39629-HuGene_st:1026726-HuGene_st:1014415-HuGene_st:51876-HuGene_st:749900-HuGene_st:804632-HuGene_st | 3.59E-08 | 1.6527 | 4.46 | 79.6 | 70.9-86.5 |
| DMN | 207872-HuGene_st:407332-HuGene_st:828944-HuGene_st:469719-HuGene_st:166409-HuGene_st:430636-HuGene_st:87850-HuGene_st:1061724-HuGene_st:1040362-HuGene_st:336607-HuGene_st:799683-HuGene_st:504888-HuGene_st:212730_at:872910-HuGene_st:861909-HuGene_st:406723-HuGene_st:530704-HuGene_st:214304_x_at | 4.36E-08 | 1.6041 | 4.05 | 78.9 | 70-86 |
| MPEG1 | 226818_at:918920-HuGene_st:283447-HuGene_st:449729-HuGene_st:301732-HuGene_st:915566-HuGene_st:854136-HuGene_st:226841_at:477225-HuGene_st: 1084905-HuGene_st:104883-HuGene_st:342839-HuGene_st:863106-HuGene_st:504453-HuGene_st:44663-HuGene_st:607415-HuGene_st:968224-HuGene_st:990725-HuGene_st:735251-HuGene_ st | 4.58E-08 | 1.6464 | 1.86 | 79.5 | 70.8-86.4 |
| IQGAP2 | 185028-HuGene_st: 763806-HuGene_st:88647-HuGene_st:93358-HuGene_st:393573-HuGene_st:743251-HuGene_st:1010599-HuGene_st:786301-HuGene_st:1066864-HuGene_st:231977-HuGene_st:370908-HuGene_st:937713-HuGene_st:201743-HuGene_st:243728_at:899180-HuGene_st:981926-HuGene_st:897914-HuGene_st:1095716-HuGene_st:802231-HuGene_st:263364-HuGene_st:986453-HuGene_st:203474_at | 5.06E-08 | 1.6277 | 2.51 | 79.2 | 70.5-86.2 |
| LMOD1 | 611871-HuGene_st:53373-HuGene_st:380885-HuGene_st:958079-HuGene_st:986515-HuGene_st:173411-HuGene_st:616270-HuGene_st:517377-HuGene_st:433277-HuGene_st:287901-HuGene_st:1071756-HuGene_st:180631-HuGene_st:52042-HuGene_st:203766_s_at:732981-HuGene_st:211562_s_at:999216-HuGene_st:993253-HuGene_st:589530-HuGene_st:675886-HuGene_st:945700-HuGene_ st | 5.55E-08 | 1.5812 | 2.97 | 78.5 | 69.8-85.6 |
| PGM5 | 810480-HuGene_st:111685-HuGene_st:338926-HuGene_st:81611-HuGene_st:658883-HuGene_st:803066-HuGene_st:208491_s_at:956998-HuGene_st:1095193-HuGene_st:1066912-HuGene_st:1079743-HuGene_st:743259-HuGene_st:429274-HuGene_st:259184-HuGene_st:376761-HuGene_st:249074-HuGene_st:226303_at | 5.87E-08 | 1.6218 | 5.87 | 79.1 | 70.4-86.2 |
| DDR2 | 857174-HuGene_st:297630-HuGene_st:198601-HuGene_st:951531-HuGene_st:341474-HuGene_st:1008685-HuGene_st:1000005-HuGene_st:712988-HuGene_st:263417-HuGene_st:511597-HuGene_st:348176-HuGene_st:928611-HuGene_st:318480-HuGene_st:801516-HuGene_st:849806-HuGene_st:510631-HuGene_st:225442_at:87448-HuGene_st:704299-HuGene_st:227561_at | 5.89E-08 | 1.5612 | 2.61 | 78.2 | 69.5-85.4 |
| PALM2-AKAP2 | 202759_s_at:202760_s_at | 6.28E-08 | 1.613 | 2.17 | 79 | 70.3-86.1 |
| AOC3 | 557248-HuGene_st:209280-HuGene_st:588332-HuGene_st:108003-HuGene_st:960783-HuGene_st:407777-HuGene_st:147438-HuGene_st:548345-HuGene_st:721301-HuGene_st:128882-HuGene_st:340215-HuGene_st:631235-HuGene_st:641187-HuGene_st:117366-HuGene_st:949088-HuGene_st:204894_s_at | 6.43E-08 | 1.5565 | 2.92 | 78.2 | 69.3-85.4 |
| PAPSS2 | 1040734-HuGene_st:893182-HuGene_st:355010-HuGene_st:808055-HuGene_st:449559-HuGene_st:203059_s_at:710965-HuGene_st:891049-HuGene_st:1050455-HuGene_st:59122-HuGene_st:203058_s_at:203060_s_at:968 581-HuGene_st:932781-HuGene_st:291649-HuGene_st:600968-HuGene_st:413041-HuGene_st:247101-HuGene_st:1087256-HuGene_st:237496_at: 15324-HuGene_st:675829-HuGene_st:771003-HuGene_st:343109-HuGene st | 6.43E-08 | 1.6163 | 1.97 | 79 | 70.4-86.1 |
| SDPR | 878908-HuGene_st:781527-HuGene_st:331976-HuGene_st:238150-HuGene_st:306039-HuGene_st:535903-HuGene_st: 302361-HuGene_st:1005813-HuGene_st:71118-HuGene_st:992629-HuGene_st:218711_s_at:293110-HuGene_st:779040-HuGene_st:222717_at:970479-HuGene_ st:581654-HuGene_st | 9.62E-08 | 1.6006 | 2.21 | 78.8 | 70-85.9 |
| DUSP1 | 308761-HuGene_st:904724-HuGene_st:371253-HuGene_st:201041_s_at:918293-HuGene_st:636896-HuGene_st:793780-HuGene_st:714382-HuGene_st:83208-HuGene_st:504846-HuGene_st:1046152-HuGene_st:936429-HuGene_st:226578_s_at:367434-HuGene_st:676144-HuGene_st:57046-HuGene_st:466265-HuGene_st:775780-HuGene_st:747888-HuGene_st:554753-HuGene_st:101136-HuGene_st:829116-HuGene_st:201044_x_at:571046-HuGene_ st | 1.06E-07 | 1.527 | 2.44 | 77.7 | 68.8-85 |
| PRKAR2B | 101664-HuGene_st:162600-HuGene_st:426444-HuGene_st:988677-HuGene_st:546073-HuGene_st:714756-HuGene_st:683742-HuGene_st:422832-HuGene_st:509601-HuGene_st:898244-HuGene_st:904472-HuGene_st:705909-HuGene_st:348626-HuGene_st:699010-HuGene_st:742973-HuGene_st:283465-HuGene_st:720328-HuGene_st:320328-HuGene_st:210153_s_at:688661 - HuGene_st:478012-HuGene_st:373286-HuGene_st:484605-HuGene_st:989810-HuGene_st:280047-HuGene_st:473253-HuGene_st:630331-HuGene_st:1003297-HuGene_st:209397_at:203680_at | 1.09E-07 | 1.677 | 2.1 | 79.9 | 71.2-86.9 |
| CNTN3 | 267567-HuGene_st:782053-HuGene_st:550157-HuGene_st:541394-HuGene_st:989173-HuGene_st: 15899-HuGene_st:78622-HuGene_st:339738-HuGene_st:585282-HuGene_st:661814-HuGene_st:360715-HuGene_st:695033-HuGene_st:483058-HuGene_st:555394-H uGene_st:315011-HuGene_st:905374-HuGene_st:1067212-HuGene_st:557263-HuGene_st:233502_at:811729-HuGene_ st:87414-HuGene_st | 1.20E-07 | 1.6161 | 3.93 | 79 | 70.3-86.1 |
| RAB27A | 339168-HuGene_st:340479-HuGene_st:1057756-HuGene_st:99120-HuGene_st:301308-HuGene_st:309278-HuGene_st:641167-HuGene_st:900707-HuGene_st:961788-HuGene_st:210951_x_at:209515_s_at:924 247-HuGene_st:209514_s_at:2432-HuGene_st:405271-HuGene_st:758428-HuGene_st:235766_x_at:391537-HuGene_st:983411-HuGene_ st:222294_s_at | 1.22E-07 | 1.5976 | 1.79 | 78.8 | 70-85.9 |
| C6orf105 | 669403-HuGene_st:937383-HuGene_st:344202-HuGene_st:229070_at:1088682-HuGene_st:215100_at:287254-HuGene_st:34196-HuGene_st:701140-HuGene_st:461427-HuGene_st:1032543-HuGene_st:237434-HuGene_st:542250-HuGene_st:316541-HuGene_st:179382-HuGene_st:148807-HuGene_st:1029919-HuGene_st:58955-HuGene_st:48477-HuGene_st:869984-HuGene_st:667357-HuGene_st:697774-HuGene_st:164714-HuGene_st:105735-HuGene_st | 1.38E-07 | 1.6043 | 2.13 | 78.9 | 70.1-85.9 |
| MUC12 | 412725-HuGene_st: 1083259-HuGene_st:437412-HuGene_st:751451-HuGene_st:498196-HuGene_st:1087628-HuGene_st:214378-HuGene_st:61291-HuGene_st:661525-HuGene_st:678015-HuGene_st:468158-HuGene_st:991377-HuGene_st:280731-HuGene_st:790312-HuGene_st:770658-HuGene_st:56500-HuGene_st:181935-HuGene_st:1013137-HuGene_st:1557906_at:226654_at:155790 7_x_at:153751-HuGene_st:765891-HuGene_st:946487-HuGene_st:231814_at:42037-HuGene_st | 1.53E-07 | 1.4566 | 4.89 | 76.7 | 67.7-84.1 |
| AXL | 321003-HuGene_st:546377-HuGene_st:429002-HuGene_st:790260-HuGene_st:796222-HuGene_st:59559-HuGene_st:221741-HuGene_st:669140-HuGene_st:822162-HuGene_st:521036-HuGene_st:202685_s_at:501383-HuGene_st:883192-HuGene_st | 1.59E-07 | 1.5776 | 1.71 | 78.5 | 69.7-85.6 |
| IL1R2 | 377084-HuGene_st:278685-HuGene_st:472031-HuGene_st:792041-HuGene_st:1012886-HuGene_st:568945-HuGene_st:80054-HuGene_st:866918-HuGene_st:866883-HuGene_st:17623-HuGene_st:314768-HuGene_st:326744-HuGene_st:211372_s_at:888405-HuGene_st:205403_at:834475-HuGene_st:386354-HuGene_st:411355-HuGene_st:862981-HuGene_st:70227-HuGene_st | 1.73E-07 | 1.5226 | 2.54 | 77.7 | 68.7-84.9 |
| TSC22D3 | 235364_at:1044209-HuGene_st:519020-HuGene_st:427467-HuGene_st:669553-HuGene_st:722843-HuGene_st:208763_s_at:126162-HuGene_st:207001_x_at:654578-HuGene_st:146515-HuGene_st:1100721-HuGene_st:823307-HuGene_st:899494-HuGene_st:952685-HuGene_st:235399-HuGene_st:802521-HuGene_st:126175-HuGene_ st:722700-HuGene_st | 1.85E-07 | 1.6337 | 3.1 | 79.3 | 70.6-86.3 |
| GPA33 | 169349-HuGene_st:772424-HuGene_st:482659-HuGene_st:1053088-HuGene_st:221229-HuGene_st:205929_at:21623-HuGene_st:352510-HuGene_st:431411-HuGene_st:507225-HuGene_st:362352-HuGene_st:492326-HuGene_st:555302-HuGene_st:812966-HuGene_st:207883-HuGene_st:401745-HuGene_st:630242-HuGene_st:212295-HuGene_st:1097025-HuGene_st:157847-HuGene_st | 1.86E-07 | 1.7041 | 1.61 | 80.3 | 71.7-87.2 |
| IDH3A | 202069_s_at:300578-HuGene_st:202070_s_at:342767-HuGene_st:8965-HuGene_st:1079553-HuGene_st:221816-HuGene_st:1042110-HuGene_st:647088-HuGene_st:670662-HuGene_st:53656-HuGene_st:572196-HuGene_st:607148-HuGene_st:463534-HuGene_st:784198-HuGene_st:859733-HuGene_st:221939-HuGene_st:606301-HuGene_st:546210-HuGene_st:475783-HuGene_st:1084692-HuGene_st:538386-HuGene_ st | 2.08E-07 | 1.567 | 1.75 | 78.3 | 69.6-85.5 |
| MATN2 | 902099-HuGene_st: 1065921-HuGene_st:452682-HuGene_st:1012649-HuGene_st:1019476-HuGene_st:88797-HuGene_st:986521-HuGene_st:181168-HuGene_st:102298-HuGene_st:757359-HuGene_st:859886-HuGene_st:540941-HuGene_st:202350_s_at:326901-HuGene_st:781583-HuGene_st:498255-HuGene_st:212041-HuGene_st | 2.11E-07 | 1.5084 | 2.23 | 77.5 | 68.6-84.8 |
| MT2A | 723245-HuGene_st:71 0161-HuGene_st:133506-HuGene_st:90770-HuGene_st:309654-HuGene_st:194391-HuGene_st:692585-HuGene_st:117983-HuGene_st:858756-HuGene_st:123483-HuGene_st:296152-HuGene_st:217546_at:239766-HuGene_st:46711-HuGene_st:1057926-HuGene_st:611729-HuGene_st:1048755-HuGene_st:519540-HuGene_st:284543-HuGene_st:847898-HuGene_st:648660-HuGene_st:977125-HuGene_st:743580-HuGene_st:327541-HuGene_st:948505-HuGene_ st:6546-HuGene_st:84521-HuGene_st:907619-HuGene_st:939779-HuGene_st:561550-HuGene_st:76641-HuGene_st:1000258-HuGene_st:447330-HuGene_st:983649-HuGene_st:613324-HuGene_st:846448-HuGene_st:304444-HuGene_st:212859_x_at:40069-HuGene_st:212884-HuGene_st:575774-HuGene_st:427914-HuGene_st:56303-HuGene_st:213349-HuGene_st:721776-HuGene_st:212185_x_at:739002-HuGene_st:902404-HuGene_st:483985-HuGene_st:91794-HuGene_st:137880-HuGene_st:317864-HuGene_st:203291-HuGene_st:66799-HuGene_st:160991-HuGene_st:216336_x_at:310066-HuGene_st:1079767-HuGene_st:781831-HuGene_ st:1095705-HuGene_st:534398-HuGene_st:467500-HuGene_st:491335-HuGene_st:66800-HuGene_st:485318-HuGene_st:90966-HuGene_st:365104-HuGene_st:79259-HuGene_st:495714-HuGene_st:1095744-HuGene_st:102946-HuGene_st:477858-HuGene_st:771030-HuGene_st:675707-HuGene_st:854293-HuGene_st:160013-HuGene_st:320514-HuGene_st:1047433-HuGene_st:890901-HuGene_st:230953-HuGene_st:749925-HuGene_st:19613-HuGene_st:166551-H uGene_st:574267-HuGene_st:623210-HuGene_st:1006466-HuGene_st:900368-HuGene_st:760559-HuGene_st:446223-HuGene_st:205427-HuGene_st:983813-HuGene_st:435034-HuGene_st:250747-HuGene_ st | 2.49E-07 | 1.6459 | 2.41 | 79.5 | 70.8-86.5 |
| MT1M | 723245-HuGene_st:710161-HuGene_st:133506-HuGene_st:90770-HuGene_st:309654-HuGene_st: 194391-HuGene_st:692585-HuGene_st:117983-HuGene_st:858756-HuGene_st:123483-HuGene_st:296152-HuGene_st:217546_at:239766-HuGene_st:46711-HuGene_st:1057926-HuGene_st:611729-HuGene_st:1048755-HuGene_st:519540-HuGene_st:284543-HuGene_st:847898-HuGene_st:648660-HuGene_st:977125-HuGene_st:743580-HuGene_st:327541-HuGene_st:948505-HuGene_st:6546-HuGene_st:84521-HuGene_st:907619-HuGene_st:939779-HuGene_st:561550-HuGene_st:76641-HuGene_st: 1000258-HuGene_st:447330-HuGene_st:983649-HuGene_st:613324-HuGene_st:846448-HuGene_st:304444-HuGene_st:212859_x_at:40069-HuGene_st:212884-HuGene_st:575774-HuGene_st:427914-HuGene_st:56303-HuGene_st:213349-HuGene_st:721776-HuGene_st:212185_x_at:739002-HuGene_st:902404-HuGene_st:483985-HuGene_st:91794-HuGene_st:137880-HuGene_st:317864-HuGene_st:203291-HuGene_st:66799-HuGene_st:160991-HuGene_st:216336_x_at:310066-HuGene_st:1079767-HuGene_st:781831-HuGene_st:1095705-HuGene_st:534398-HuGene_st:467500-HuGene_st:491335-HuGene_st:66800-HuGene_st:485318-HuGene_st:90966-HuGene_st:365104-HuGene_ _st:79259-HuGene_st:495714-HuGene_st: 1095744-HuGene_st:102946-HuGene_st:477858-HuGene_st:771030-HuGene_st:675707-HuGene_st:854293-HuGene_st: 160013-HuGene_st:320514-HuGene_st:1047433-HuGene_st:890901-HuGene_ st:230953-HuGene st:749925-HuGene_st:19613-HuGene_st:166551-HuGene_st:574267-HuGene_st:623210-HuGene_st:1006466-HuGene_st:900368-HuGene_st:760559-HuGene_st:446223-HuGene_st:205427-HuGene_st:983813-HuGene_st:435034-HuGene_st:250747-HuGene_ st | 2.49E-07 | 1.6419 | 2.41 | 79.4 | 70.7-86.4 |
| IGL@ | 173623-HuGene_st:408026-HuGene_ st:38807-HuGene st | 2.50E-07 | 1.6337 | 3.1 | 79.3 | 70.7-86.3 |
| HSPB8 | 688528-HuGene_st:311091-HuGene_st:415722-HuGene_st:56376-HuGene_st:79205-HuGene_st:499751-HuGene_st:214231-HuGene_st:225808-HuGene_st:174359-HuGene_st:1083550-HuGene_st:221667_s_at:662744-HuGene_ st:346589-HuGene_st | 2.61E-07 | 1.5121 | 3.85 | 77.5 | 68.7-84.8 |
| A2M | 897456-HuGene_st:217757_at:164005-HuGene_st:848096-HuGene_st:244657-HuGene_st:317437-HuGene_st:330214-HuGene_st:814658-HuGene_st:1100520-HuGene_st:451567-HuGene_st:559737-HuGene_st:600508-HuGene_st:730036-HuGene_st:385395-HuGene_st:833129-HuGene_st: 101038-HuGene_st:195639-HuGene_st:371583-HuGene_st:808161-HuGene_st:893977-HuGene_st:1558450_at:167145-HuGene_st:660806-HuGene_st | 2.72E-07 | 1.5968 | 1.64 | 78.8 | 69.9-85.9 |
| MS4A7 | 803709-HuGene_st:372940-HuGene_st:558657-HuGene_st:758990-HuGene_st:279234-HuGene_st:716878-HuGene_st:228770-HuGene_st:77553-HuGene_st:212226-HuGene_st:148024-HuGene_st:727350-HuGene_st:223344_s_at:223343_at:22435 8_s_at:932003-HuGene_st:584291-HuGene_st:16857-HuGene_st:751363-HuGene_st:120594-HuGene_st:66810-HuGene_st:473741-HuGene_st | 2.92E-07 | 1.5398 | 2.27 | 77.9 | 69.1-85.2 |
| PLOD2 | 366970-HuGene_st:202619_s_at:557917-HuGene_st:371377-HuGene_st:606385-HuGene_st:54079-HuGene_st:976248-HuGene_st:572307-HuGene_st:716158-HuGene_st:754684-HuGene_st:255595-HuGene_st: 17343-HuGene_st:124945-HuGene_st:770320-HuGene_st:704827-HuGene_st:202620_s_at;309852-HuGene_st:509273-HuGene_st:703772-HuGene_st:1082989-HuGene_st:550147-HuGene_st:740582-HuGene_st:6711-HuGene_ st | 3.06E-07 | 1.5569 | 2.33 | 78.2 | 69.3-85.4 |
| UGT2B17 | 357315-HuGene_st:493733-HuGene_st:353886-HuGene_ _st:207245_at:59042-HuGene_ st:1025125-HuGene_st | 3.75E-07 | 1.4818 | 4.88 | 77.1 | 68.2-84.5 |
| PMP22 | 1086412-HuGene_st:139615-HuGene_st:864327-HuGene_st: 1565637_at:493430-HuGene_st:640953-HuGene_st:309874-HuGene_st:1072798-HuGene_st:761816-HuGene_st: 136620-HuGene_st:441104-HuGene_st:97712-HuGene_st:800086-HuGene_st:352501-HuGene_st:363233-HuGene_st:210139_s_at:48107-HuGene_st:338800-HuGene_st:532682-HuGene_st:988715-HuGene_st:853970-HuGene_st | 3.97E-07 | 1.5173 | 2.23 | 77.6 | 68.8-84.9 |
| PLN | 204938_s_at:418214-HuGene_st:769837-HuGene_st:603329-HuGene_st:1048096-HuGene_st:944510-HuGene_st:430402-HuGene_st:86633-HuGene_st:795093-HuGene_st:377757-HuGene_st:770145-HuGene_st:920628-HuGene_st:594048-HuGene_st:800583-HuGene_st:204939_s_at:556723-HuGene_st:818339-HuGene_st:175524-HuGene_st:498924-HuGene_st:204940_at | 4.14E-07 | 1.5464 | 2.01 | 78 | 69.2-85.2 |
| CNN1 | 337187-HuGene_st:787686-HuGene_st:532503-HuGene_st:41547-HuGene_st:236587-HuGene_st:809046-HuGene_st:674638-HuGene_st:1055006-HuGene_st:617683-HuGene_st:769218-HuGene_st:373491-HuGene_st:609613-HuGene_st:402708-HuGene_st:871546-HuGene_st:203951_at:880804-HuGene_st:1091224-HuGene_st:911860-HuGene_ st:1090431-HuGene_st | 4.77E-07 | 1.4318 | 4.56 | 76.3 | 67.2-83.8 |
| ITM2A | 391687-HuGene_st:1059695-HuGene_st:12009-HuGene_st:677746-HuGene_st:258271-HuGene_st:676659-HuGene_st:496836-HuGene_st:452608-HuGene_st:1059257-HuGene_st:47720-HuGene_st:105265-HuGene_st:657686-HuGene_st:68702-HuGene_st:115530-HuGene_st:202747_s_at:202746_at | 5.27E-07 | 1.4958 | 2.77 | 77.3 | 68.3-84.6 |
| KCNMA 1 | 554163-HuGene_st:716361-HuGene_st:403072-HuGene_st:63347-HuGene_st:587079-HuGene_st:256038-HuGene_st: 172619-HuGene_st:918468-HuGene_st:94420-HuGene_st:399013-HuGene_st:414900-HuGene_st:307320-HuGene_st:906761-HuGene_st:115093-HuGene_st:221583_s_at:745071-HuGene_st:915037-HuGene_st:343951-HuGene_st:1569763_at:446685-HuGene_st:652192-HuGene_st | 6.86E-07 | 1.497 | 2.37 | 77.3 | 68.4-84.6 |
| XDH | 919455-HuGene_st:105269-HuGene_st:106986-HuGene_st:972900-HuGene_st:210301_at:716491-HuGene_st:667307-HuGene_st:790507-HuGene_st:800140-HuGene_st:328962-HuGene st:355792-HuGene_st:707858-HuGene_st:731429-HuGene_st:403069-HuGene_st:215338-HuGene_st:101916-HuGene_st:48855-HuGene_st:895641-HuGene_st:68212-HuGene_st:287011-HuGene_st:1082168-HuGene_st:241994_at:709200-HuGene_st | 8.13E-07 | 1.4727 | 2.03 | 76.9 | 67.9-84.3 |
| PDLIM3 | 659306-HuGene_st:9491-HuGene_st:787864-HuGene_st:482305-HuGene_st:810161-HuGene_st:1037974-HuGene_st:353413-HuGene_st:731754-HuGene_st:733690-HuGene_st:209621_s_at:394037-HuGene_st:957822-HuGene_st:67576-HuGene_st:369461-HuGene_st:768470-HuGene_st:489488-HuGene_st:711969-HuGene_st: 1569564_at:1077054-HuGene_st:210170_at:238592_at | 8.20E-07 | 1.4799 | 2.38 | 77 | 68.2-84.4 |
| MT1X | 788358-HuGene_st:20578-HuGene_st:92311-HuGene_st:501820-HuGene_st:208581_x_at:73687-HuGene_st:905260-HuGene_st:1087816-HuGene_st:204326_x_at:96216-HuGene_st:764373-HuGene_st:103649-HuGene_st:40373-HuGene_st:198342-HuGene_ st | 9.84E-07 | 1.4411 | 4.35 | 76.4 | 67.5-83.9 |
| CALD1 | 796040-HuGene_st:839187-HuGene_st:412659-HuGene_st:1023569-HuGene_st:450656-HuGene_st:212077_at:201616_s_at:81643 9-HuGene_st:165931-HuGene_st:201617_x_at:576686-HuGene_st:1094139-HuGene_st:201615_x_at:519079-HuGene_st:558226-HuGene_st:755661-HuGene_st:243084_at:318906-HuGene st:688034-HuGene_st:215199_at | 1.02E-06 | 1.4584 | 3.29 | 76.7 | 67.7-84.1 |
| MSRB3 | 483115-HuGene_st:439736-HuGene_st:1096860-HuGene_st:644381-HuGene_st:225790_at:306630-HuGene_st:1073255-HuGene_st:121556-HuGene_st:126154-HuGene_st:72517-HuGene_st:480208-HuGene_st:331986-HuGene_st:294511-HuGene_st:204701-HuGene_st: 1554127_s_at:42731-HuGene_st:675396-HuGene_st:174560-HuGene_st:1566481_at | 1.20E-06 | 1.4754 | 2.24 | 77 | 68-84.4 |
| SPON1 | 209437_s_at:209436_at:892181-HuGene_st:960315-HuGene_st:377887-HuGene_st:253667-HuGene_st:48054-HuGene_st:1008441-HuGene_st:979120-HuGene_st:213994_s_at:811254-HuGene_st:928583-HuGene_st:405680-HuGene_st:963535-HuGene_st:836638-HuGene_st:1033364-HuGene_st:965345-HuGene_st:936102-HuGene_st:555640-HuGene_st:22053-HuGene_st:234367-HuGene st:224488_s_at:327210-HuGene_st:481375-HuGene_st:213993_at:1013182-HuGene_st | 1.46E-06 | 1.4862 | 1.88 | 77.1 | 68.2-84.5 |
| C2orf40 | 1011921-HuGene_st:304065-HuGene_st:441303-HuGene_st:1001854-HuGene_st:999754-HuGene_st:893886-HuGene_st:951843-HuGene_st:375926-HuGene_st | 1.69E-06 | 1.4159 | 2.97 | 76.1 | 67-83.6 |
| SORBS1 | 1041567-HuGene_st:83451-HuGene_st:29118-HuGene_st:222513_s_at:617821-HuGene_st:56685-HuGene_st:280855-HuGene_st:67922-HuGene_st:1028489-HuGene_st:611267-HuGene_st:931477-HuGene_st:66752-HuGene_st:618734-HuGene_st:624287-HuGene_st:211819_s_at:581936-HuGene_st:218087_s_at:1039879-HuGene_st:45815-HuGene_st:186556-HuGene_ st:628496-HuGene_st:242736_at | 2.18E-06 | 1.4269 | 2.54 | 76.2 | 67.2-83.7 |
| C4orf34 | 576095-HuGene_st:1061109-HuGene_st:698934-HuGene_st:634224-HuGene_st:854845-HuGene_st:18233-HuGene_st:275684-HuGene_st:784949-HuGene_st:346086-HuGene_st:91468-HuGene_st:936558-HuGene_st:163564-HuGene_st:614699-HuGene_st:428090-HuGene_st:340671-HuGene_st:90795-HuGene_st:823553-HuGene_st:431110-HuGene_st:719502-HuGene_st:850208-HuGene_st:224990_at:542653-HuGene_st | 2.55E-06 | 1.3851 | 1.95 | 75.6 | 66.5-83.1 |
| PDGFRA | 782752-HuGene_st:789846-HuGene_st:116091-HuGene_st:860506-HuGene_st:537532-HuGene_st:112478-HuGene_st:97681-HuGene_st:514113-HuGene_st:561375-HuGene_st:115277-HuGene_st:240764-HuGene_st:578952-HuGene_st:723105-HuGene_st:230638-HuGene_st:544163-HuGene_st:50957-HuGene_st:468599-HuGene_st:328136-HuGene_st:346391-HuGene_st:203131_at:853019-HuGene_st | 2.94E-06 | 1.4819 | 1.37 | 77.1 | 68.1-84.4 |
| FGFR2 | 476373-HuGene_st:92078-HuGene_st:969020-HuGene_st:1022426-HuGene_st:203639_s_at:24514-HuGene_st:614945-HuGene_st:211398_at:1097953-HuGene_st:117962-HuGene_st:777874-HuGene_st:829764-HuGene_st:868500-HuGene_st:211401_s_at:403546-HuGene_st:850416-HuGene_st:208234_x_at:537762-HuGene_st: 132866-HuGene_st:426700-HuGene_st:208228_s_at:831045-HuGene_st:203638_s_at:240913_at:23084 2_at | 3.55E-06 | 1.527 | 1.31 | 77.7 | 68.9-85 |
| PPP1R12B | 23543-HuGene_st:654289-HuGene_st:947526-HuGene_st:714709-HuGene_st:544138-HuGene_st:1557553_at:227411-HuGene_st:201958_s_at:7483 8-HuGene_st:201957_at:233700_at:67323-HuGene_st:767707-HuGene_st:224270_at:50561-HuGene_st:233359-HuGene_st:565179-HuGene_st:317428-HuGene_st:442074-HuGene_st:591561-HuGene_st | 4.26E-06 | 1.3592 | 2.21 | 75.2 | 66.1-82.8 |
| FAM129A | 148319-HuGene_st:19042-HuGene_st:1075897-HuGene_st:49110-HuGene_st:245230-HuGene_st:154650-HuGene_st:203917-HuGene_st:503184-HuGene_st:373453-HuGene_st:432126-HuGene_st:786874-HuGene_st:648379-HuGene_st:1033222-HuGene_st:217966_s_at:574775-HuGene_st:217967_s_at:156986-HuGene_st:9160-HuGene_st:212626-HuGene_st:935377-HuGene_st:921695-HuGene st:1053262-HuGene_st | 4.59E-06 | 1.4144 | 1.8 | 76 | 67-83.5 |
| POSTN | 210809_s_at:297712-HuGene_st:1555778_a_at:608435-HuGene_st:743877-HuGene_st:753556-HuGene_st:388659-HuGene_st:954838-HuGene_st:724846-HuGene_st:588010-HuGene_st:713459-HuGene_st:713707-HuGene_st:106590-HuGene_st:538299-HuGene_st:649547-HuGene_st:13993-HuGene_st:417058-HuGene_st:636479-HuGene_st:228481_at:874100-HuGene_st:776445-HuGene_st:779754-HuGene_st | 4.94E-06 | 1.3944 | 2.25 | 75.7 | 66.7-83.3 |
| ATP8B1 | 1088077-HuGene_st:699379-HuGene_st:81949-HuGene_st:120481-HuGene_st:1006809-HuGene_st:960479-HuGene_st:1033226-HuGene_st:854521-HuGene_st:258436-HuGene_st:224444-HuGene_ st:958640-HuGene_st:670830-HuGene_st:734950-HuGene_st:61644-HuGene_st:354636-HuGene_st:308159-HuGene_st:645299-HuGene_st:306422-HuGene_st:223980-HuGene_st:772656-HuGene_st:226302_at:745109-HuGene_ st:1011843-HuGene_st | 5.73E-06 | 1.3113 | 1.67 | 74.4 | 65.3-82.1 |
| CCL28 | 224240_s_at:155321-HuGene_st:780816-HuGene_st:224027_at:356805-HuGene_st:120679-HuGene_st:525328-HuGene_st:623557-HuGene_st:238750_at:519318-HuGene_st:956859-HuGene_st:251110-HuGene_st:331562-HuGene_st:224868-HuGene_st:28476-HuGene_st:153501-HuGene_st:242217-HuGene_st:821675-HuGene_st:451844-HuGene_st:513548-HuGene_st:773827-HuGene_st:398738-HuGene_st | 6.0 1E-06 | 1.494 | 1.53 | 77.2 | 68.4-84.6 |
| NEXN | 184663-HuGene_st:720053-HuGene_st:539444-HuGene_st:311368-HuGene_st:260384-HuGene_st:56754-HuGene_st: 1040436-HuGene_st:1064626-HuGene_st:292849-HuGene_st:453309-HuGene_st:804402-HuGene_st:500301-HuGene_st:248505-HuGene_st:402104-HuGene_st:1552309_a_at:941387-HuGene_ st:709246-HuGene_st:226103_at | 6.83E-06 | 1.363 | 2.23 | 75.2 | 66.1-82.9 |
| CTGF | NA | 6.96E-06 | 1.4786 | 1.3 | 77 | 68.1-84.4 |
| MFSD4 | 15050-HuGene_st:785959-HuGene_st:884375-HuGene_st:75016-HuGene_st:235772-HuGene_st:185211-HuGene_st:539224-HuGene_st:871027-HuGene_st:855473-HuGene_st:728207-HuGene_st:242782-HuGene_st:711626-HuGene_st:484086-HuGene_st:242372_s_at:238862_at:22925 4_at:656926-HuGene_st:767273-HuGene_st:184295-HuGene_st:697889-HuGene_st:1566569_at:708165-HuGene_st | 7.19E-06 | 1.3233 | 2.12 | 74.6 | 65.5-82.3 |
| ASPN | 787638-HuGene_st:567513-HuGene_st:499513-HuGene_st:546047-HuGene_st:1055545-HuGene_st:720939-HuGene_st:835521-HuGene_st:640448-HuGene_st:673620-HuGene_st:821057-HuGene_st:248224-HuGene_st:447994-HuGene_st:407869-HuGene_st:510872-HuGene_st:766560-HuGene_st | 7.45E-06 | 1.3261 | 2.74 | 74.6 | 65.5-82.3 |
| DUSP5 | 25091-HuGene_st:352326-HuGene_st:119170-HuGene_st:292093-HuGene_st:209457_at:899442-HuGene_st:171695-HuGene_st:331265-HuGene_st:991652-HuGene_st:242476-HuGene_st:430239-HuGene_st:1070934-HuGene_st:555959-HuGene_st | 8.60E-06 | 1.334 | 1.91 | 74.8 | 65.6-82.4 |
| CRISPLD2 | 314440-HuGene_st:395147-HuGene_st:429916-HuGene_st:763823-HuGene_st:729858-HuGene_st: 1555809_at:909486-HuGene_st:239091-HuGene_st:418565-HuGene_st:949339-HuGene_st:658945-HuGene_st:276173-HuGene_st:96079-HuGene_st | 9.24E-06 | 1.3669 | 1.64 | 75.3 | 66.2-82.9 |
| FOXF2 | 589126-HuGene_st:856761-HuGene_st:145881-HuGene_st:915587-HuGene_st:1088669-HuGene_st:81821-HuGene_st:206377_at:575658-HuGene_st:494266-HuGene_st:813114-HuGene_st:613721-HuGene_st | 9.50E-06 | 1.3047 | 2.08 | 74.3 | 65.2-82.1 |
| UGT2B15 | 500573-HuGene_st:352298-HuGene_st:770027-HuGene_st:207392_x_at:31342-HuGene_st:829850-HuGene_st:217175_at:57112-HuGene_st:1062564-HuGene_st:951114-HuGene_st:844819-HuGene_st:841767-HuGene_st:57590-HuGene_st:504268-HuGene_st:27324-HuGene_st:340207-HuGene_st: 102754-HuGene_st:1055412-HuGene_st | 9.73E-06 | 1.3469 | 5.72 | 75 | 65.9-82.6 |
| ACTG2 | 667818-HuGene_st:500390-HuGene_st:979342-HuGene_st:560814-HuGene_st:801310-HuGene_st:552015-HuGene_st:972136-HuGene_st:617230-HuGene_st:409424-HuGene_st:15155-HuGene_st:416391-HuGene_st:800450-HuGene_st:902303-HuGene_st:647647-HuGene_st: 123624-HuGene_st:202274_at:586898-HuGene_st:431150-HuGene_st:247321-HuGene_st:735136-HuGene_st:986398-HuGene_st:261974-HuGene st:241148_at:575416-HuGene_st | 1.07E-05 | 1.3992 | 1.82 | 75.8 | 66.8-83.4 |
| CMBL | 157156-HuGene_st:305010-HuGene_st:227522_at:553921-HuGene_st:529224-HuGene_st:1032780-HuGene_st:204886-HuGene_st:623086-HuGene_st:451862-HuGene_st:537678-HuGene_st:918595-HuGene_st:178105-HuGene_st:417861-HuGene_st:230995_at:986212-HuGene_st:405624-HuGene_st:424469-HuGene_st:836099-HuGene_st:665079-HuGene_ st:234981_x_at | 1.09E-05 | 1.3583 | 1.58 | 75.1 | 66.1-82.8 |
| KCTD12 | 509699-HuGene_st:77199-HuGene_st:212188_at:229632-HuGene_st:502611-HuGene_st:931430-HuGene_st:908582-HuGene_st:769157-HuGene_st:312044-HuGene_st:212192_at:43415-HuGene_st:412026-HuGene_st:247270-HuGene_st:770750-HuGene_st:827060-HuGene_ st:714432-HuGene_st | 1.44E-05 | 1.3636 | 1.36 | 75.2 | 66.2-82.8 |
| MRGPRF | 717973-HuGene_st:226058-HuGene_st:541082-HuGene_st:60861-HuGene_st:1024643-HuGene_st:354954-HuGene_ st:1099895-HuGene_st:672489-HuGene_st:489062-HuGene_st:435421-HuGene_st:227727_at:99658-HuGene_st | 1.52E-05 | 1.2986 | 2.99 | 74.2 | 65.1-82 |
| C20orf118 | 235964_x_at:234987_at:235529_x_at:174 500-HuGene_st:168513-HuGene_st:312879-HuGene_st:57294-HuGene_st:748530-HuGene_st:24012-HuGene_st:219008-HuGene_st:471274-HuGene_st: 1086281-HuGene_st:740018-HuGene_ st:233829_at | 1.75E-05 | 1.4284 | 1.3 | 76.2 | 67.3-83.8 |
| TMEM47 | 20487-HuGene_st:587019-HuGene_st:458329-HuGene_st:944333-HuGene_st:292459-HuGene_st:436914-HuGene_st:632184-HuGene_st:209655_s_at:885991-HuGene_st: 33524-HuGene_st:528167-HuGene_st:555631-HuGene_st:595266-HuGene_st:503408-HuGene_st:296998-HuGene_st:672458-HuGene_st:64704-HuGene_st:285813-HuGene_st:865055-HuGene_ st:734911-HuGene_st | 1.86E-05 | 1.301 | 2.02 | 74.2 | 65.1-82 |
| QKI | 214543_x_at:212262_at:696914-HuGene_st:363144-HuGene_st:820387-HuGene_st:646624-HuGene_st:979853-HuGene_st:658669-HuGene_st:748520-HuGene_st:656477-HuGene_st:296134-HuGene_st:1555154_a_at:212636_at:2344 92_at:462894-HuGene_st:1055543-HuGene_st | 1.93E-05 | 1.3452 | 1.31 | 74.9 | 65.8-82.6 |
| VSIG2 | 985113-HuGene_st:555767-HuGene_st:391438-HuGene_st:1093892-HuGene_st:675062-HuGene_st:788466-HuGene_st:826720-HuGene_st:997596-HuGene_st:1069591-HuGene_st:228232_s_at:223925-HuGene_st:637781-HuGene_st:265221-HuGene_st:652010-HuGene_st:265186-HuGene_st: 343187-HuGene_st:1095607-HuGene_st | 2.01E-05 | 1.2959 | 2.05 | 74.1 | 65.1-81.9 |
| HSPA1A | 979726-HuGene_st:1069731-HuGene_st:50191-HuGene_st:799087-HuGene_st:200799_at:539596-HuGene_st:150764-HuGene_st:200800_s_at:492579-HuGene_st | 2.16E-05 | 1.2699 | 2.41 | 73.7 | 64.6-81.5 |
| AP1S2 | 230413_s_at:230264_s_at:99873-HuGene_st:57979-HuGene_st:985324-HuGene_st:203300_x_at:84828-HuGene_ st:910415-HuGene_st | 3.39E-05 | 1.2572 | 1.92 | 73.5 | 64.3-81.3 |
| FKBP5 | 204560_at:224856_at:246403-HuGene_st:513199-HuGene_st:69253-HuGene_st:642461-HuGene_st:467598-HuGene_st:1038165-HuGene_st:512075-HuGene_st:224840_at:797941-HuGene_st:633285-HuGene_st:511644-HuGene_st:469540-HuGene_st:209829-HuGene_ st:800516-HuGene_st:247764-HuGene_st:468711-HuGene_st:112353-HuGene_st:746630-HuGene_st:337035-HuGene_st:1014328-HuGene_st:292916-HuGene_st | 3.41E-05 | 1.2369 | 1.71 | 73.2 | 64-81 |
| KCNMB1 | 17348-HuGene_st:414025-HuGene_st:792493-HuGene_st:131717-HuGene_st:569127-HuGene_st:17962-HuGene_st:504462-HuGene_st:758432-HuGene_st:209948_at:772102-HuGene_st:518039-HuGene_st | 3.74E-05 | 1.2189 | 2.66 | 72.9 | 63.7-80.8 |
| VIM | 564251-HuGene_st:201426_s_at:234475-HuGene_st:319318-HuGene_st:339807-HuGene_st:527110-HuGene_st:994975-HuGene_st:837477-HuGene_st:302722-HuGene_st:516025-HuGene_st:1079757-HuGene_st:398387-HuGene_st:155888-HuGene_st:139661-HuGene_st:192324-HuGene_st:1093618-HuGene_st:364983-HuGene_st:436158-HuGene_st:410777-HuGene_ st:1555938_x_at | 4.15E-05 | 1.2225 | 1.86 | 72.9 | 63.7-80.9 |
| SORBS2 | 238751_at:805920-HuGene_st | 4.23E-05 | 1.3007 | 2.1 | 74.2 | 65.1-82 |
| C6orf204 | 486932-HuGene_st:573436-HuGene_st:400510-HuGene_st:228202_at:123307-HuGene_st:181714-HuGene_st:206020-HuGene_st:949623-HuGene_st:814579-HuGene_st:515524-HuGene_st:514175-HuGene_st | 4.90E-05 | 1.2042 | 2.07 | 72.6 | 63.5-80.6 |
| FAM55D | 633727-HuGene_st:966568-HuGene_st:625133-HuGene_st:858568-HuGene_st:220645_at:201856-HuGene_st:1013940-HuGene_st:316604-HuGene_st:450702-HuGene_st:1028406-HuGene_st:1090862-HuGene_st:741930-HuGene_st:981211-HuGene_st:970069-HuGene_st:259706-HuGene_st:291282-HuGene_st:56493-HuGene_st:888970-HuGene_st:821065-HuGene_st:53354-HuGene_st:752570-HuGene_st:539086-HuGene_ st:204142-HuGene_st | 5.67E-05 | 1.1792 | 3.48 | 72.2 | 62.9-80.2 |
| TIMP2 | 1087828-HuGene_st:231579_s_at:436546-HuGene_st:799495-HuGene_st:563754-HuGene_st:914626-HuGene_st:526357-HuGene_st:203167_at:760416-HuGene_st:651774-HuGene_st:965142-HuGene_st:224540-HuGene_st:690107-HuGene_st:224560_at:319958-HuGene_st:437652-HuGene_st:43390-HuGene_st:727465-HuGene_st:310783-HuGene_st:750028-HuGene_st:957648-HuGene_st:106688-HuGene_st:289430-HuGene_st:740790-HuGene_st | 6.40E-05 | 1.2298 | 1.82 | 73.1 | 63.8-80.9 |
| ELOVL5 | NA | 8.15E-05 | 1.1814 | 2.05 | 72.3 | 63-80.3 |
| ACTA2 | 215787_at:743686-HuGene_st:200974_at:1070983-HuGene_st:120087-HuGene_st:980364-HuGene_st:529704-HuGene_st:855050-HuGene_st:530590-HuGene_st:692252-HuGene_st:404109-HuGene_st:630797-HuGene_st:670805-HuGene_st:293590-HuGene_st:757648-HuGene_st:445454-HuGene_st:479447-HuGene_st:548186-HuGene_st:237159-HuGene_st:90357-HuGene_st:313690-HuGene_st:183075-HuGene_st:785232-HuGene_st | 1.00E-04 | 1.2318 | 1.81 | 73.1 | 63.9-80.9 |
| C1S | 208747_s_at:233042_at:620011-HuGene_st:1555229_a_at:245571-HuGene_st:426716-HuGene_st:866948-HuGene_st:705447-HuGene_st:947234-HuGene_st:682561-HuGene_st:332123-HuGene_st | 0.0001 | 1.2896 | 1.61 | 74 | 64.8-81.9 |
| LOC38776 3 | 1062323-HuGene_st | 0.0001 | 1.1943 | 1.36 | 72.5 | 63.2-80.5 |
| CLU | 222043_at:939366-HuGene_st:832450-HuGene_st:208792_s_at:125360-HuGene_st:216446-HuGene_st:208791_at:1054768-HuGene_st:1034873-HuGene_st:1056536-HuGene_st:787017-HuGene_st:1060096-HuGene_st:591687-HuGene_st:1014914-HuGene_st:213483-HuGene_st:459630-HuGene_st:1036064-HuGene_st:972503-HuGene_st:492827-HuGene_st:485686-HuGene_st | 0.0001 | 1.2285 | 1.42 | 73 | 63.8-81 |
| MT1H | 197989-HuGene_st:589780-HuGene_st:800631-HuGene_st:854292-HuGene_st:644419-HuGene_st:851562-HuGene_st:590830-HuGene_st:727551-HuGene_st:51225-HuGene_st:206461_x_at:1099945-HuGene_ _st:334778-HuGene_st:6545-HuGene_st:451479-HuGene_st:621584-HuGene_st:885933-HuGene_st:73199-HuGene_ st:882631-HuGene_st | 0.0001 | 1.1885 | 4.55 | 72.4 | 63.1-80.4 |
| RGS2 | 331021-HuGene_st:202388_at:895469-HuGene_st:962521-HuGene_st:843420-HuGene_st:767847-HuGene_st:1022759-HuGene_st:507303-HuGene_st:121934-HuGene_st:1094262-HuGene_st:1067241-HuGene_st:111860-HuGene_st:661918-HuGene_st | 0.0001 | 1.1617 | 1.94 | 71.9 | 62.6-79.9 |
| FBN1 | 192231-HuGene_st:794561-HuGene_st:1094040-HuGene_st:427611-HuGene_st:394963-HuGene_st:828273-HuGene_st:202765_s_at:162818-HuGene_st:169932-HuGene_st:192029-HuGene_st:288955-HuGene_st:600623-HuGene_st:941525-HuGene_st:971683-HuGene_st:82155-HuGene_st:359130-HuGene_st:404656-HuGene_st:921008-HuGene_st:999573-HuGene_st:112593-HuGene_st | 0.0002 | 1.1368 | 1.93 | 71.5 | 62.2-79.6 |
| ANKRD25 | 808074-HuGene_st:932564-HuGene_st:143731-HuGene_st:292383-HuGene_st:393569-HuGene_st:201770-HuGene_st:973796-HuGene_st:824703-HuGene_st:109143-HuGene_st:132685-HuGene_st:698165-HuGene_st:218418_s_at | 0.0002 | 1.1162 | 1.56 | 71.2 | 61.9-79.3 |
| MT1G | 226585-HuGene_st:495514-HuGene_st:159768-HuGene_st:186270-HuGene_st:855342-HuGene_st:449379-HuGene_st:1100995-HuGene_st:617733-HuGene_st:914660-HuGene_st:884883-HuGene_st:903179-HuGene_st:311881-HuGene_st:977536-HuGene_st:210472_at:1061010-HuGene_st:415267-HuGene_st:1048754-HuGene_st:1011704-HuGene_st:59546-HuGene_st:917923-HuGene_st:204745_x_at | 0.0002 | 1.0893 | 2.66 | 70.7 | 61.3-78.8 |
| MYL9 | 201058_s_at:543240-HuGene_st:923797-HuGene_st:173220-HuGene_st:874422-HuGene_st:925187-HuGene_st:979054-HuGene_st:676360-HuGene_st:117460-HuGene_st:1092848-HuGene_st:608837-HuGene_st:404703-HuGene_st:50995-HuGene_st:206106-HuGene_st:995028-HuGene_st | 0.0003 | 1.1288 | 2.18 | 71.4 | 62-79.4 |
| TAGLN | 279405-HuGene_st:205547_s_at:695669-HuGene_st:902958-HuGene_st:304704-HuGene_st:741240-HuGene_st:898711-HuGene_st:1555724_s_at:26929-HuGene_st:66114-HuGene_st:505391-HuGene_st:613243-HuGene_st:73428-HuGene_st:983072-HuGene_st:543865-HuGene_st:179049-HuGene_st:458482-HuGene_st:823959-HuGene_st:931316-HuGene_st:556028-HuGene_st:565900-HuGene_st:278667-HuGene_ st:226523_at:232248_at | 0.0003 | 1.0901 | 2.12 | 70.7 | 61.4-78.8 |
| FOXF1 | 21663-HuGene_st:832719-HuGene_st:174216-HuGene_st:337755-HuGene_ st:775422-HuGene_st:205935_at | 0.0004 | 1.1745 | 1.28 | 72.1 | 62.9-80.1 |
| LGALS2 | 204754-HuGene_st:455546-HuGene_st:428640-HuGene_st:228729-HuGene_st:450048-HuGene_st:1087923-HuGene_st:616130-HuGene_st:818575-HuGene_st:699850-HuGene_st:820908-HuGene_st:850596-HuGene_st:751645-HuGene_st:1028381-HuGene_st:667147-HuGene_st:245361-HuGene_st:754620-HuGene_ st | 0.0004 | 1.0851 | 3.07 | 70.6 | 61.2-78.8 |
| SDC2 | 1019857-HuGene_st:730583-HuGene_st:33355-HuGene_st:717018-HuGene_st:212158_at:272847-HuGene_st:458585-HuGene_st:212154_at:446134-HuGene_st:1096317-HuGene_st:815650-HuGene_st:590116-HuGene_st:367740-HuGene_st:1074315-HuGene_st | 0.0006 | 1.099 | 1.39 | 70.9 | 61.5-79 |
| DNASE1L 3 | 867148-HuGene_st:588394-HuGene_st:205554_s_at:616846-HuGene_st:58544-HuGene_st:277847-HuGene_st:18171-HuGene_st:429485-HuGene_st:960168-HuGene_st:981221-HuGene_st:23306-HuGene_st:991254-HuGene_st | 0.0007 | 1.0972 | 1.5 | 70.8 | 61.6-79 |
| PLEKHC1 | 831786-HuGene_st:88016-HuGene_st:1087035-HuGene_st:530331-HuGene_st:209209_s_at:184398-HuGene_st:904923-HuGene_st:193550-HuGene_st:468805-HuGene_st:104495-HuGene_st:604317-HuGene_st:501652-HuGene_st:118096-HuGene_st:321532-HuGene_st:675257-HuGene_st:79496-HuGene_st:590867-HuGene_st:147248-HuGene_st:627402-HuGene_st:377514-HuGene_st | 0.0007 | 1.0318 | 1.82 | 69.7 | 60.3-78 |
| SERPINF1 | 546682-HuGene_st:814007-HuGene_st:202283_at:898654-HuGene_st:884794-HuGene_st:264195-HuGene_st:787378-HuGene_st:1036417-HuGene_st | 0.0009 | 1.0012 | 1.47 | 69.2 | 59.8-77.4 |
| RARRES2 | 1098138-HuGene_st:515302-HuGene_st:849018-HuGene_st:209496_at:741143-HuGene_st:903829-HuGene_st:305131-HuGene_st:715557-HuGene_st:373579-HuGene_st:177871-HuGene_st:106318-HuGene_st:662795-HuGene_st:38090-HuGene_st | 0.0009 | 1.0325 | 1.6 | 69.7 | 60.3-78 |
| CFL2 | 545956-HuGene_st:833414-HuGene_st:214239-HuGene_st: 192253-HuGene_st:224663_s_at:694174-HuGene_st:233496_s_at:992798-HuGene_st:328584-HuGene_st:950857-HuGene_st:612593-HuGene_st:95686-HuGene_st:177218-HuGene_st:451953-HuGene_st:800232-HuGene_st:575557-HuGene_st:794701-HuGene_st | 0.0009 | 1.0592 | 1.75 | 70.2 | 60.8-78.4 |
| RBMS1 | 375424-HuGene_st:87825-HuGene_st:1046943-HuGene_st:209868_s_at:207266_x_at:225 269_s_at:238185_at | 0.0009 | 1.1417 | 1.55 | 71.6 | 62.3-79.7 |
| SELENBP1 | 914892-HuGene_st:582091-HuGene_st:643114-HuGene_st:532506-HuGene_st:823159-HuGene_st:171447-HuGene_st:189960-HuGene_st:75763-HuGene_st:834358-HuGene_st:245623-HuGene_st:214433_s_at:233267_at: 56418 6-HuGene_st:637748-HuGene_st:200643-HuGene_st:781415-HuGene_st:208204-HuGene_st:730504-HuGene_st:1008117-HuGene_st:801694-HuGene_st:433986-HuGene_st:183676-HuGene_st:634656-HuGene_ st:1087258-HuGene_st | 0.0009 | 1.0563 | 1.7 | 70.1 | 60.7-78.4 |
| ENAM | 212592_at | 0.0011 | 1.0044 | 2.58 | 69.2 | 59.9-77.5 |
| MT1F | 987574-HuGene_st:213629_x_at:171840-HuGene_st:217165_x_at:867979-HuGene_st:354284-HuGene_st:424055-HuGene_st:998184-HuGene_st:616693-HuGene_st:459005-HuGene_st:221141-HuGene_st:338518-HuGene_st:951452-HuGene_st:915306-HuGene_st:506126-HuGene_st:845635-HuGene_st:1048483-HuGene_st:1094672-HuGene_st:576368-HuGene_ st:414800-HuGene_st | 0.0012 | 0.9654 | 2.2 | 68.5 | 59.1-76.9 |
| CD163 | 620892-HuGene_st:215049_x_at:32161-HuGene_st:867013-HuGene_st:899841-HuGene_st:662901-HuGene_st:97726-HuGene_st:180883-HuGene_st:706228-HuGene_st:849505-HuGene_st:217692-HuGene_st:566183-HuGene_st:154492-HuGene_st:737668-HuGene_st:203645_s_at:948100-HuGene_st:900552-HuGene_st:288236-HuGene_ st | 0.0013 | 1.0234 | 1.9 | 69.6 | 60.2-77.9 |
| GUCY1A3 | 560981-HuGene_st:1088572-HuGene_st:692186-HuGene_st:648114-HuGene_st:783434-HuGene_st:221942_s_at:1026405-HuGene_st:256104-HuGene_st:965063-HuGene_st:889044-HuGene_st:752296-HuGene_st:239580_at:185668-HuGene_st:120195-HuGene_st:324658-HuGene_st:229530_at | 0.0015 | 1.0136 | 2.69 | 69.4 | 60-77.7 |
| TNFRSF17 | 1062688-HuGene_st:38682-HuGene_st:558270-HuGene_st:631025-HuGene_st:24518-HuGene_st:240754-HuGene_st:193693-HuGene_st:255074-HuGene_st:493368-HuGene_st:191710-HuGene_st:610232-HuGene_st:544680-HuGene_st:205576-HuGene_st:738267-HuGene_st:746498-HuGene_st | 0.0015 | 1.0199 | 2.08 | 69.5 | 60.1-77.7 |
| CAV1 | 793412-HuGene_st:1007317-HuGene_st:212097_at:203065_s_at:81283 2-HuGene_st:505037-HuGene_st:819580-HuGene_st:348883-HuGene_st:659416-HuGene_st:698493-HuGene_st:861877-HuGene_ st:518081-HuGene_st:35773-HuGene_st | 0.0016 | 1.0215 | 1.58 | 69.5 | 60.1-77.8 |
| CKB | 718720-HuGene_st:769422-HuGene_st:581051-HuGene_st:549987-HuGene_st:24890-HuGene_st:1041238-HuGene_st:16603-HuGene_st:480007-HuGene_st:435665-HuGene_st:25470-HuGene_st:40899-HuGene_st:896708-HuGene_st:373774-HuGene_st:963331-HuGene_st:23457-HuGene_st:766369-HuGene_st:200884_at:405417-HuGene_st:888623-HuGene_st:396007-HuGene_ st | 0.0019 | 0.9741 | 2.27 | 68.7 | 59.2-77 |
| WWTR1 | 202132_at:249804-HuGene_st:735408-HuGene_st:925151-HuGene_st:213564-HuGene_st:561456-HuGene_st:125734-HuGene_st:367743-HuGene_st:153369-HuGene_st:628871-HuGene_st:942493-HuGene_st:949013-HuGene_st:202134_s_at:426662-HuGene_st:586249-HuGene_st:228629_s_at:506281-HuGene_st | 0.002 | 0.9904 | 1.48 | 69 | 59.6-77.3 |
| HMGCS2 | 729816-HuGene_st:658722-HuGene_st:253113-HuGene_st:900260-HuGene_st:253100-HuGene_st:283650-HuGene_st:624250-HuGene_st:439478-HuGene_st:407398-HuGene_st:171889-HuGene_st:397691-HuGene_st:448587-HuGene_st:593619-HuGene_st:371076-HuGene_st:228587-HuGene_st:888308-HuGene_st:538023-HuGene_st:204607_at:43162-HuGene_st:865416-HuGene_st:619083-HuGene_st:789982-HuGene_st:616916-HuGene_st | 0.0022 | 0.8767 | 2.67 | 66.9 | 57.5-75.5 |
| MAFB | 876871-HuGene_st:600037-HuGene_st:177002-HuGene_st:3125-HuGene_st:1057503-HuGene_st:274807-HuGene_st | 0.0034 | 0.9563 | 1.66 | 68.4 | 59-76.8 |
| STOM | 201060_x_at:669723-HuGene_st:1099166-HuGene_st:201061_s_at:22513-HuGene_st:430393-HuGene_st:486266-HuGene_ st:47888-HuGene_st | 0.0035 | 1.0176 | 1.21 | 69.5 | 60.1-77.7 |
| CD14 | 327635-HuGene_st:201743_at:822666-HuGene_st:999890-HuGene_st:753790-HuGene_st:1080129-HuGene_st:532035-HuGene_st:1087885-HuGene_st:528527-HuGene_st:851691-HuGene_st:905038-HuGene_st:437599-HuGene_st:31858-HuGene_st | 0.0035 | 0.9646 | 1.58 | 68.5 | 59-76.9 |
| MUC4 | 1061780-HuGene_st:43545-HuGene_st:217110_s_at:47402-HuGene_st:1042572-HuGene_st:548777-HuGene_st:612341-HuGene_st:711231-HuGene_st:217109_at:278229-HuGene_st:536580-HuGene_st:235055_x_at:743233-HuGene_st:223515-HuGene_st:204895_x_at:670488-HuGene_st:106413-HuGene_st:35359-HuGene_st:344313-HuGene_st:284609-HuGene_st:882714-HuGene_st:512794-HuGene_ st | 0.0039 | 1.0448 | 1.2 | 69.9 | 60.5-78.2 |
| APOE | 520118-HuGene_st:886930-HuGene_st:771679-HuGene_st:944600-HuGene_st:169546-HuGene_st:483757-HuGene_st:16650-HuGene_st:511913-HuGene_st:1099818-HuGene_st:33219-HuGene_st:663513-HuGene_st | 0.0044 | 0.9406 | 1.56 | 68.1 | 58.7-76.6 |
| SI | 9605-HuGene_st:514814-HuGene_st:809314-HuGene_st:576613-HuGene_st:45502-HuGene_st:369676-HuGene_st:438636-HuGene_st:267204-HuGene_st:326716-HuGene_st:897825-HuGene_st:377666-HuGene_st:519273-HuGene_st:325741-HuGene_st:245381-HuGene_st:108368-HuGene_st:464198-HuGene_st:679193-HuGene_st:168180-HuGene_st | 0.0054 | 0.9299 | 2.79 | 67.9 | 58.5-76.4 |
| LOC28538 2 | 242447_at | 0.0058 | 0.7422 | 1.26 | 64.5 | 55-73.2 |
| HLA-DRB1 | 971813-HuGene_st | 0.0063 | 1.0668 | 1.51 | 70.3 | 60.9-78.6 |
| ANTXR1 | 241549_at:232956_at | 0.0071 | 0.9507 | 1.29 | 68.3 | 58.8-76.7 |
| FAM46C | 197110-HuGene_st:220306_at:110466-HuGene_st:988119-HuGene_st:1098424-HuGerie_st:560529-HuGene_st:387944-HuGene_st:38645-HuGene_st:221689-HuGene_ st:322082-HuGene_st | 0.0085 | 0.9172 | 1.38 | 67.7 | 58.2-76.1 |
| TIMP3 | 767021-HuGene_st:201150_s_at:201148_s_at:228 752-HuGene_st:241221-HuGene_st:201147_s_at:125730-HuGene_st | 0.0091 | 0.9064 | 1.81 | 67.5 | 58-76 |
| LOC28366 6 | 235567_at:236266_at:1562682_at:240951 _at | 0.0099 | 0.7796 | 1.22 | 65.2 | 55.6-73.8 |
| GREM1 | 569365-HuGene_st:407017-HuGene_st:218468_s_at:292333-HuGene_st:330742-HuGene_st:546941-HuGene_st:556201-HuGene_st:484435-HuGene_st:326978-HuGene_st:144073-HuGene_st:411208-HuGene_st:1081939-HuGene_st:345681-HuGene_st:2445-HuGene_st:864620-HuGene_st | 0.0106 | 0.8579 | 3.03 | 66.6 | 57-75.1 |
| FCGBP | 22847-HuGene_st:203240_at:338164-HuGene_st:516715-HuGene_st:88809-HuGene_st:948682-HuGene_st:311904-HuGene_st:997435-HuGene_st:841000-HuGene _st:508230-HuGene_st:226428-HuGene_st:634071-HuGene_st:948120-HuGene_st:1079875-HuGene_st:71797-HuGene_st:781083-HuGene_st:426005-HuGene_st:27264-HuGene_st:16108-HuGene_st:63672-HuGene_st:1085208-HuGene_ st:708701-HuGene_st | 0.0121 | 0.7889 | 1.99 | 65.3 | 55.7-74 |
| HSPA2 | 831351-HuGene_st:270747-HuGene_st:720913-HuGene_st:660812-HuGene_st:939000-HuGene_st:920196-HuGene_ st | 0.0123 | 0.8908 | 1.43 | 67.2 | 57.7-75.7 |
| COL6A1 | 138366-HuGene_st:528191-HuGene_st:884210-HuGene_st:57954-HuGene_st:737256-HuGene_st:858575-HuGene_st:212937_s_at:214200_s_at:190 064-HuGene_st:216904_at | 0.0126 | 0.8452 | 1.45 | 66.4 | 56.9-75 |
| SELM | NA | 0.0133 | 0.8596 | 1.32 | 66.6 | 57.2-75.2 |
| FLNA | 296693-HuGene_st:685285-HuGene_st:704065-HuGene_st:445061-HuGene_st:11735-HuGene_st:1066792-HuGene_st:214752_x_at:213746_s_at:871 038-HuGene_st:396236-HuGene_st:200859_x_at:786282-HuGene_st:136680-HuGene_st:50466-HuGene_st:955232-HuGene_st:588605-HuGene_st:40025-HuGene_st:1002306-HuGene_st:833338-HuGene_st:86435-HuGene_ st:172457-HuGene_st | 0.0157 | 0.7967 | 1.59 | 65.5 | 55.9-74.1 |
| COX7A1 | 386303-HuGene_st:354428-HuGene_st | 0.0161 | 0.8613 | 1.32 | 66.7 | 57.2-75.3 |
| EMP3 | 736604-HuGene_st:155271-HuGene_st:584704-HuGene_st | 0.0164 | 0.8053 | 1.41 | 65.6 | 56.1-74.2 |
| ITLN1 | 389198-HuGene_st:496499-HuGene_st:48547-HuGene_st:552029-HuGene_st:126777-HuGene_st:223597_at:281671-HuGene_st:45052-HuGene_st:573120-HuGene_st:137624-HuGene_st:948945-HuGene_st:527196-HuGene_st:358648-HuGene_st:45640-HuGene_st:512550-HuGene_st:839317-HuGene_st:634036-HuGene_st:101083-HuGene_st:857541-HuGene_ st:404069-HuGene_st | 0.021 | 0.7137 | 2.54 | 63.9 | 54.4-72.7 |
| C10orf99 | 227735_s_at:563767-HuGene_st:963471-HuGene_st:965947-HuGene_st:1081062-HuGene_st:354432-HuGene_st:602661-HuGene_st:227736_at:1009594-HuGene_st:969063-HuGene_st:77052-HuGene_st:483367-HuGene_st:50662-HuGene_st:738457-HuGene_st:69019-HuGene_st:215415-HuGene_st:1003926-HuGene_st:676577-HuGene_st:676855-HuGene_st:225297-HuGene_st:560897-HuGene_st:886384-HuGene_st:303754-HuGene_st | 0.0218 | 0.724 | 1.76 | 64.1 | 54.7-72.9 |
| CSRP1 | 453033-HuGene_st:673637-HuGene_st:607761-HuGene_st:582630-HuGene_st:654799-HuGene_st:258474-HuGene_st:200621_at:1028504-HuGene_st:802909-HuGene_st:339914-HuGene_st:533385-HuGene_st:256461-HuGene_st:1043113-HuGene_st:72101-HuGene_st:105671-HuGene_st:339691-HuGene_st:108781-HuGene_st:133369-HuGene_st:614016-HuGene_st:629648-HuGene_ st:81550-HuGene_st | 0.023 | 0.7234 | 1.39 | 64.1 | 54.6-72.9 |
| PTGER4 | 450915-HuGene_st:1067155-HuGene_st:812085-HuGene_st:1943-HuGene_st:109314-HuGene_st:204897_at:252442-HuGene_st:800125-HuGene_st:965220-HuGene_st:446764-HuGene_st:204896_s_at:23686-HuGene_st:548266-HuGene_st:392964-HuGene_st:986807-HuGene_st:619166-HuGene_st | 0.0237 | 0.8101 | 1.41 | 65.7 | 56.2-74.4 |
| TPM2 | 828216-HuGene_st:373854-HuGene_st:981165-HuGene_st:191586-HuGene_st:445912-HuGene_st:446500-HuGene_st:922931-HuGene_st:336603-HuGene_st:272114-HuGene_st:375166-HuGene_st:8388-HuGene_st:204083_s_at:544548-HuGene_st:945443-HuGene_st:628440-HuGene_st:763118-HuGene_st:538951-HuGene_st | 0.026 | 0.7539 | 1.53 | 64.7 | 55.2-73.4 |
| PIGR | 748831-HuGene_st:226147_s_at:166398-HuGene_st:593192-HuGene_st:204213_at:557727-HuGene_st:229659_s_at:242106-HuGene_st:146237-HuGene_st:883137-HuGene_st:691362-HuGene_st:201991-HuGene_st:1045753-HuGene_st:589879-HuGene_st:1017162-HuGene_st:325979-HuGene_st:105903-HuGene_st:1063932-HuGene_st:207526-HuGene_st:566905-HuGene_st | 0.0278 | 0.7282 | 1.59 | 64.2 | 54.7-73 |
| HLA-C | 754900-HuGene_st | 0.0279 | 0.9253 | 1.24 | 67.8 | 58.3-76.2 |
| PRNP | 178399-HuGene_st:201300_s_at:386578-HuGene_ st:783439-HuGene_st | 0.0288 | 0.798 | 1.24 | 65.5 | 56-74.2 |
| CCL11 | 495934-HuGene_st | 0.0361 | 0.8547 | 1.32 | 66.5 | 57.1-75 |
| C1QC | 137801-HuGene_st:851445-HuGene_st | 0.0391 | 0.7457 | 1.36 | 64.5 | 54.9-73.3 |
| CYR61 | 736150-HuGene_st:385534-HuGene_st:48239-HuGene_st:242630-HuGene_st | 0.0425 | 0.7875 | 1.47 | 65.3 | 55.7-73.9 |
| SERPING1 | NA | 0.0503 | 0.7659 | 1.44 | 64.9 | 55.4-73.6 |
| TYROBP | NA | 0.053 | 0.8176 | 1.17 | 65.9 | 56.3-74.5 |
| GJA1 | NA | 0.0561 | 0.7839 | 1.76 | 65.2 | 55.6-73.9 |
| PTRF | NA | 0.0624 | 0.7354 | 1.5 | 64.3 | 54.8-73.1 |
| CLCA1 | NA | 0.0681 | 0.7855 | 1.61 | 65.3 | 55.8-74 |
| SPARC | NA | 0.0684 | 0.6702 | 1.45 | 63.1 | 53.5-71.9 |
| COL4A2 | NA | 0.0738 | 0.7081 | 1.32 | 63.8 | 54.3-72.5 |
| RAB31 | NA | 0.1041 | 0.8159 | 1.14 | 65.8 | 56.3-74.4 |
| NID1 | NA | 0.105 | 0.7428 | 1.33 | 64.5 | 54.9-73.2 |
| FN1 | NA | 0.1121 | 0.5769 | 1.73 | 61.3 | 51.7-70.2 |
| MSN | NA | 0.1499 | 0.7215 | 1.15 | 64.1 | 54.6-72.9 |
| MMP2 | NA | 0.1755 | 0.6219 | 1.24 | 62.2 | 52.5-71.2 |
| COL1A2 | NA | 0.1891 | 0.65 | 1.9 | 62.7 | 53.2-71.6 |
| FOSB | NA | 0.2076 | 0.6389 | 2.17 | 62.5 | 52.9-71.4 |
| HBB | NA | 0.2357 | 0.6434 | 1.28 | 62.6 | 53-71.5 |
| POU2AF1 | NA | 0.2389 | 0.6564 | 1.18 | 62.9 | 53.3-71.7 |
| COL3A1 | NA | 0.2551 | 0.5538 | 1.35 | 60.9 | 51.3-69.9 |
| SMOC2 | NA | 0.2701 | 0.6533 | 1.34 | 62.8 | 53.2-71.7 |
| MUC2 | NA | 0.2802 | 0.8523 | 1.22 | 66.5 | 57-75 |
| Sep-06 | NA | 0.3109 | 0.5713 | 1.21 | 61.2 | 51.7-70.2 |
| LUM | NA | 0.3157 | 0.5442 | 1.54 | 60.7 | 51.1-69.7 |
| C1QB | NA | 0.3668 | 0.5005 | 1.24 | 59.9 | 50.4-69 |
| TGFB1I1 | NA | 0.4093 | 0.4951 | 1.35 | 59.8 | 50.1-68.8 |
| ALDH1A1 | NA | 0.4232 | 0.354 | 1.4 | 57 | 47.4-66.2 |
| LRIG1 | NA | 0.4614 | 0.5998 | 1.26 | 61.8 | 52.2-70.7 |
| C8orf4 | NA | 0.475 | 0.483 | 1.44 | 59.5 | 49.9-68.5 |
| EGR1 | NA | 0.4851 | 0.5374 | 1.38 | 60.6 | 51-69.6 |
| LOC65274 5 | NA | 0.4864 | 0.1722 | 1.09 | 53.4 | 43.8-62.8 |
| IGKV1D-13 | NA | 0.5177 | 0.1597 | 1.1 | 53.2 | 43.7-62.5 |
| COL15A1 | NA | 0.6601 | 0.54 | 1.22 | 60.6 | 51-69.6 |
| IGFBP5 | NA | 0.7663 | 0.3504 | 1.25 | 57 | 47.4-66.3 |
| LGALS1 | NA | 0.8555 | 0.4626 | 1.16 | 59.1 | 49.5-68.3 |
| COL6A3 | NA | 0.8739 | 0.4412 | 1.2 | 58.7 | 49.1-67.9 |
| HLA-DQA1 | NA | 0.9084 | 0.5417 | 1.28 | 60.7 | 51.1-69.6 |
| HLA-DRA | NA | 0.9084 | 0.5391 | 1.28 | 60.6 | 51-69.6 |
| ST6GALN AC1 | NA | 0.9452 | 0.2594 | 1.15 | 55.2 | 45.6-64.5 |
| HLA-DPA1 | NA | 0.9664 | 0.4166 | 1.39 | 58.3 | 48.7-67.4 |
| IGLV1-44 | NA | 0.9777 | 0.2865 | 1.07 | 55.7 | 46.1-64.9 |
| TNC | NA | 0.9905 | 0.3682 | 1.09 | 57.3 | 47.7-66.5 |
| HBA1 | NA | 0.9979 | 0.47 | 1.21 | 59.3 | 49.6-68.4 |
| HBA2 | NA | 0.9979 | 0.4705 | 1.21 | 59.3 | 49.6-68.3 |

**TABLE 3**

| **TargetPS** | **Symbol** | **Signif. FDR** | **D.val5** | **FC** | **Sens-Spec** | **CI (95)** |
|---|---|---|---|---|---|---|
| 230788_at | SPTLC3:GCNT2 | 2.1587E-27 | 3.9562 | 13.36 | 97.6 | 94.2-99.2 |
| 228706_s_at | CLDN23 | 4.2694E-20 3.0727 | | 3.47 | 93.8 | 88.3-97.1 |
| 231120_x_at | PKIB | 5.9397E-20 | 3.1191 | 3.28 | 94.1 | 88.6-97.3 |
| 224412_s_at | TRPM6 | 2.2514E-19 | 3.0115 | 7.5 | 93.4 | 87.7-96.8 |
| 220037_s_at | XLKD1 | 3.5991E-19 | 2.9405 | 7.44 | 92.9 | 87-96.5 |
| 209613_s_at | ADH1B:ADH1A | 4.6957E-19 | 3.0158 | 4.67 | 93.4 | 87.7-96.9 |
| 204719_at | ABCA8 | 1.2559E-18 | 2.9171 | 6.82 | 92.8 | 86.8-96.5 |
| 231773_at | ANGPTL1 | 1.4172E-17 | 2.7946 | 4.64 | 91.9 | 85.6-95.9 |
| 225575_at | LIFR | 2.4532E-17 | 2.7797 | 6.36 | 91.8 | 85.5-95.8 |
| 220812_s_at | HHLA2 | 3.4739E-17 | 2.6718 | 10.02 | 90.9 | 84.4-95.2 |
| 211549_s_at | HPGD | 3.6502E-17 | 2.8451 | 3.21 | 92.3 | 86.1-96.1 |
| 202920_at | ANK2 | 2.5934E-16 | 2.6488 | 6.76 | 90.7 | 84.2-95.1 |
| 231925_at | P2RY1 | 1.4091E-15 | 2.5329 | 3.6 | 89.7 | 82.8-94.4 |
| 207080_s_at | PYY | 3.5022E-15 | 2.4783 | 11.71 | 89.2 | 82.2-94 |
| 228885_at | RPL24:LOC731365 | 1.2312E-14 | 2.5575 | 1.63 | 90 | 83.1-94.6 |
| 228766_at | CD36 | 6.2677E-14 | 2.4687 | 1.65 | 89.1 | 82.1-94 |
| 204931_at | TCF21 | 2.5147E-13 | 2.3036 | 2.34 | 87.5 | 80.1-92.8 |
| 205433_at | BCHE | 3.1756E-13 | 2.2538 | 6.21 | 87 | 79.5-92.5 |
| 207980_s_at | CITED2 | 5.2905E-13 | 2.2709 | 1.64 | 87.2 | 79.7-92.6 |
| 209170_s_at | GPM6B | 4.0429E-12 | 2.1588 | 3.59 | 86 | 78.1-91.6 |
| 220376_at | LRRC19 | 6.7548E-12 | 2.0763 | 4.51 | 85 | 77.1-90.9 |
| 228504_at | No Symbol | 9.1926E-12 | 2.152 | 1.61 | 85.9 | 78.2-91.6 |
| 228854_at | No Symbol | 9.1926E-12 | 2.1538 | 1.61 | 85.9 | 78.2-91.6 |
| 235146_at | No Symbol | 9.1926E-12 | 2.1537 | 1.61 | 85.9 | 78.2-91.6 |
| 221305_s_at | UGT1A10:UGT1A7:UGT1A8:UGT1 A1:UGT1A9:UGT1A6:UGT1A5:UG T1A3:UGT1A4 | 1.0319E-11 | 2.0677 | 4.56 | 84.9 | 77.1-90.9 |
| 203881_s_at | DMD | 5.0063E-11 | 2.0137 | 4.74 | 84.3 | 76.3-90.4 |
| 206637_at | P2RY14 | 1.3104E-10 | 1.9836 | 2 | 83.9 | 75.8-90.1 |
| 214598_at | CLDN8 | 2.9225E-10 | 1.8624 | 12.96 | 82.4 | 74.1-88.9 |
| 227529_s_at | AKAP12 | 7.4077E-09 | 1.7434 | 2.83 | 80.8 | 72.3-87.5 |
| 219948_x_at | LOC642329:UGT2A3 | 8.638E-09 | 1.741 | 4.89 | 80.8 | 72.3-87.6 |
| 222717_at | SDPR | 9.6207E-08 | 1.5993 | 2.21 | 78.8 | 70.1-85.9 |
| 229831_at | CNTN3 | 1.1953E-07 | 1.6145 | 3.93 | 79 | 70.3-86.1 |
| 204940_at | PLN | 4.1416E-07 | 1.5439 | 2.01 | 78 | 69.2-85.2 |
| 227827_at | SORBS2 | 4.2323E-05 | 1.3016 | 2.1 | 74.2 | 65.1-81.9 |
| 238751_at | SORBS2 | 4.2323E-05 | 1.3015 | 2.1 | 74.2 | 65.1-82 |
| 209209_s_at | PLEKHC1 | 0.0007 | 1.0304 | 1.82 | 69.7 | 60.3-78 |
| 206664_at | SI | 0.0054 | 0.9301 | 2.79 | 67.9 | 58.4-76.3 |

**TABLE 4**

| **Gene Symbol** | **ValidPS_DOWN** | **Signif. FDR** | **D.val 5** | **FC** | **Sens-Spec** | **CI (95)** |
|---|---|---|---|---|---|---|
| ANK2 | 314086-HuGene_st:282874-HuGene_st:382431-HuGene_st:229308-HuGene_st:779600-HuGene_st:297624-HuGene_st:385943-HuGene_st:730140-HuGene_st:442277-HuGene_st:699309-HuGene_st:182816-HuGene_st:202921_s_at:799860-HuGene_st:868462-HuGene_st:634421-HuGene_st:571536-HuGene_st:1050903-HuGene_st:649509-HuGene_st:239935-HuGene_st:202920_at | 2.59E-16 | 2.6515 | 6.76 | 90.8 | 84.1-95.1 |
| DMD | 945600-HuGene_st:12596-HuGene_st:434657-HuGene_st:676962-HuGene_st:1020969-HuGene_st:170680-HuGene_st:855670-HuGene_st:909848-HuGene_st:134644-HuGene_st:887814-HuGene_st:649184-HuGene_st:110990-HuGene_st:914217-HuGene_st:961622-HuGene_st:514011-HuGene_st:842792-HuGene_st:445483-HuGene_st:208086_s_at:987362-HuGene_ st:997308-HuGene_st | 5.01E-11 | 2.0162 | 4.74 | 84.3 | 76.3-90.4 |
| ABCA8 | 57305-HuGene_st:111752-HuGene_st:61357-HuGene_st:123451-HuGene_st:740746-HuGene_st:512280-HuGene_st:389185-HuGene_st:427721-HuGene_st:346028-HuGene_st:224566-HuGene_st:149653-HuGene_st:680699-HuGene_st:76772-HuGene_st:742091-HuGene_st:423333-HuGene_st:559944-HuGene_st:341399-HuGene_st:1565780_at:863400-HuGene_st:921748-HuGene_st:623719-HuGene_st:204719_at:123143-HuGene_ st:1077391-HuGene_st | 1.26E-18 | 2.9182 | 6.82 | 92.8 | 86.8-96.5 |
| TCF21 | 804657-HuGene_st:106365-HuGene_st:710514-HuGene_st:299556-HuGene_st:608149-HuGene_st:242233-HuGene_st:655881-HuGene_st:356773-HuGene_st:788445-HuGene_st:709897-HuGene_st:605488-HuGene_st:652466-HuGene_st:204931_at:830709-HuGene_st:273418-HuGene_st:1004754-HuGene_st:990861-HuGene_st:238739-HuGene_st:836241-HuGene_st:110045-HuGene_st:229529_at:1001969-HuGene_st | 2.51E-13 | 2.3037 | 2.34 | 87.5 | 80.1-92.9 |
| PLN | 204938_s_at:418214-HuGene_st:769837-HuGene_st:603329-HuGene_st:1048096-HuGene_st:944510-HuGene_st:430402-HuGene_st:86633-HuGene_st:795093-HuGene_st:377757-HuGene_st:770145-HuGene_st:920628-HuGene_st:594048-HuGene_st:800583-HuGene_st:204939_s_at:556723-HuGene_st:818339-HuGene_st:175524-HuGene_st:498924-HuGene_st:204940_at | 4.14E-07 | 1.5399 | 2.01 | 77.9 | 69.2-85.2 |
| BCHE | 228090-HuGene_st:752051-HuGene_st:800167-HuGene_st:221362-HuGene_st:155900-HuGene_st:717363-HuGene_st:536584-HuGene_st:146780-HuGene_st:302487-HuGene_st:508472-HuGene_st:516293-HuGene_st:968992-HuGene_st:666625-HuGene_ st:923158-HuGene_st | 3.18E-13 | 2.2544 | 6.21 | 87 | 79.4-92.5 |
| P2RY14 | 528057-HuGene_st:780310-HuGene_st:235178-HuGene_st:352954-HuGene_st:699489-HuGene_st:38001-HuGene_st:637791-HuGene_st:25606-HuGene_st:40647-HuGene_st:896487-HuGene_st:672149-HuGene_st:863820-HuGene_st:352427-HuGene_st:632821-HuGene_st:116148-HuGene_ st:561792-HuGene_st:840910-HuGene_st:296420-HuGene_st:974643-HuGene_st:206637_at | 1.31E-10 | 1.9805 | 2 | 83.9 | 75.9-90 |
| PYY | 656845-HuGene_st:816022-HuGene_st:633572-HuGene_st:20355-HuGene_st:240779-HuGene_st:638358-HuGene_st:879780-HuGene_st:207080_s_at:211253_x_at:368591-HuGene_ st | 3.50E-15 | 2.4828 | 11.71 | 89.3 | 82.2-94 |
| CITED2 | 125201-HuGene_st:410723-HuGene_st:463405-HuGene_st:401168-HuGene_st:1012057-HuGene_st:235057-HuGene_st:361772-HuGene_st:207980_s_at:1091907-HuGene_st:985355-HuGene_st:175990-HuGene_st:227287_at:48433-HuGene_st:209357_at:477746-HuGene_st:243264-HuGene_st:904401-HuGene_st:328536-HuGene_st:1095110-HuGene_st:89784-HuGene_st:206734-HuGene_st:927615-HuGene_st | 5.29E-13 | 2.269 | 1.64 | 87.2 | 79.7-92.6 |
| GPM6B | 599862-HuGene_st:754598-HuGene_st:503642-HuGene_st:224935-HuGene_st:754449-HuGene_st:577489-HuGene_st:1073242-HuGene_st:560873-HuGene_st:1003662-HuGene_st:430217-HuGene_st:903323-HuGene_st:231962-HuGene_st:244945-HuGene_st:562460-HuGene_st:583561-HuGene_ st:209168_at | 4.04E-12 | 2.1547 | 3.59 | 85.9 | 78.2-91.7 |
| PLEKH C1 | 831786-HuGene_st:88016-HuGene_st:1087035-HuGene_st:530331-HuGene_st:209209_s_at:184398-HuGene_st:904923-HuGene_st:193550-HuGene_st:468805-HuGene_st:104495-HuGene_st:604317-HuGene_st:501652-HuGene_st:118096-HuGene_st:321532-HuGene_st:675257-HuGene_st:79496-HuGene_st:590867-HuGene_st:147248-HuGene_st:627402-HuGene st:377514-HuGene_st | 7.00E-04 | 1.0307 | 1.82 | 69.7 | 60.4-78 |
| ADH1B | 1078343-HuGene_st:512808-HuGene_st:614446-HuGene_st:910188-HuGene_st:422504-HuGene_st:731361-HuGene_st:209612_s_at:258079-HuGene_st:568239-HuGene_st:879930-HuGene_st:420417-HuGene_st: 1025048-HuGene_st:908335-HuGene_st:654633-HuGene_st:947292-HuGene_st:1087125-HuGene_st:1004870-HuGene_st:209613_s_at:579636-HuGene_ st:681018-HuGene_st:822774-HuGene_st | 4.70E-19 | 3.0174 | 4.67 | 93.4 | 87.7-96.9 |
| XLKD1 | 520080-HuGene_st:1091117-HuGene_st:943125-HuGene_st:444068-HuGene_st:648558-HuGene-st:346991-HuGene-st:1006205-HuGene_st:373107-HuGene_st:682535-HuGene_st:1083245-HuGene_st:863143-HuGene_st:820120-HuGene_st:1044561-HuGene_st:220037_s_at:541228-HuGene_st:220256-HuGene_st:289122-HuGene_st:219059_s_at:246683-HuGene_st:775976-HuGene_st:207399-HuGene_st:1052557-HuGene_st:92121-HuGene_st | 3.60E-19 | 2.9461 | 7.44 | 93 | 87.1-96.6 |
| LRRC19 | 177641-HuGene_st:1070020-HuGene_st:1055140-HuGene_st:525999-HuGene_st:937256-HuGene_st:620791-HuGene_st:891251-HuGene_st:707559-HuGene_st:892056-HuGene_st:764919-HuGene_st:382143-HuGene_st:52584-HuGene_st:920414-HuGene_st:1028155-HuGene_st:755055-HuGene_st:678651-HuGene_st:1080156-HuGene_st:530282-HuGene_st:523877-HuGene_st:335198-HuGene_st:787709-HuGene_st:153175-HuGene st:220376_at | 6.75E-12 | 2.0779 | 4.51 | 85.1 | 77.1-91 |
| SDPR | 878908-HuGene_st:781527-HuGene_st:331976-HuGene_st:238150-HuGene_st:306039-HuGene_st:535903-HuGene_st:302361-HuGene_st:1005813-HuGene_st:71118-HuGene_st:992629-HuGene_st:218711_s_at:293110-HuGene_st:779040-HuGene_st:222717_at:970479-HuGene_st:581654-HuGene_st | 9.62E-08 | 1.6036 | 2.21 | 78.9 | 70-85.9 |
| TRPM6 | 767074-HuGene_st:695352-HuGene_st:411125-HuGene_st:221102_s_at:234864_s_at:240389_at:3 58229-HuGene_st:755964-HuGene_st:840301-HuGene_st:959234-HuGene_st:782639-HuGene_st:833079-HuGene_st:1066034-HuGene_st:678013-HuGene_st:249083-HuGene_st:143934-HuGene_st:159130-HuGene_st:486486-HuGene_st:185057-HuGene_st:878793-HuGene_st:133981-HuGene_st:224412_s_at:202194-HuGene_st | 2.25E-19 | 3.0111 | 7.5 | 93.4 | 87.7-96.8 |
| LIFR | 275506-HuGene_st:323055-HuGene_st:444251-HuGene_st:1056178-HuGene_st:3981_04-HuGene_st:917434-HuGene_st:1044918-HuGene_st: 167500-HuGene_st:423760-HuGene_st:837336-HuGene_st:321505-HuGene_st:918321-HuGene_st:252278-HuGene_st:884504-HuGene_st:124845-HuGene_st:499777-HuGene_st:969722-HuGene_st:709439-HuGene_st:611505-HuGene_st:227771_at:287217-HuGene_st:205876_at:225571_at:229185_at:23336 7_at:1093011-HuGene_st | 2.45E-17 | 2.7814 | 6.36 | 91.8 | 85.5-95.8 |
| AKAP12 | 212419-HuGene_st:231067_s_at:379659-HuGene_st:1010338-HuGene_st:1075094-HuGene_st:42401-HuGene_st:522584-HuGene_st:480972-HuGene_st:948623-HuGene_st:701945-HuGene_st:276784-HuGene_st:64858-HuGene_st:210517_s_at:874382-HuGene_st:909976-HuGene_st:182037-HuGene_st:417182-HuGene_st:722881-HuGene_st | 7.41E-09 | 1.7456 | 2.83 | 80.9 | 72.4-87.6 |
| CLDN23 | 403960-HuGene_st:25144-HuGene_st:947653-HuGene_st:228704_s_at:228706_s_at:320375-HuGene_st:441629-HuGene_st:367414-HuGene_st:855269-HuGene_st:228707_at:788659-HuGene_st:698816-HuGene_st:95789-HuGene_st:270197-HuGene_st:472976-HuGene_st:280539-HuGene_st:1056334-HuGene_ st:516288-HuGene_st:579963-HuGene_st | 4.27E-2C | 3.0748 | 3.47 | 93.8 | 88.2-97.1 |
| CD36 | 392196-HuGene_st:274514-HuGene_st:477005-HuGene_st:691585-HuGene_st:872909-HuGene_st:543050-HuGene_st:603343-HuGene_st:514557-HuGene_st:296850-HuGene_st:945913-HuGene_st:495755-HuGene_st:206488_s_at:1035854-HuGene_st:887301-HuGene_st:836370-HuGene_st:209555_s_at:939919-HuGene_st:507440-HuGene_st:151788-HuGene_st:146280-HuGene_st:360545-HuGene_st:1051486-HuGene_st:228766_at:512885-HuGene_st | 6.27E-14 | 2.4696 | 1.65 | 89.2 | 82.1-94 |
| RPL24 | 1559655_at:1559656_a_at:228885_at | 1.23E-14 | 2.5568 | 1.63 | 89.9 | 183.1-94.6 |
| GCNT2 | 935239-HuGene_st:225205-HuGene_st:1026280-HuGene_st:668101-HuGene_st:1099985-HuGene_st:698568-HuGene_st:134540-HuGene_st:697147-HuGene_st:250092-HuGene_st:611927-HuGene_st:972833-HuGene_st: 168891-HuGene_st:990860-HuGene_st:109287-HuGene_st:322116-HuGene_st:231019-HuGene_st:211020_at:959570-HuGene_st:858764-HuGene_st:215593_at:820195-HuGene_st:239606_at:41059-HuGene_st:669940-HuGene_st:215595_x_at:230788_at | 2.16E-27 | 3.9536 | 13.36 | 97.6 | 94.1-99.2 |
| PKIB | 866170-HuGene_st:1055812-HuGene_st:264946-HuGene_st:684057-HuGene_st:124791-HuGene_st:134561-HuGene_st:1026756-HuGene_st:468593-HuGene_st:1045852-HuGene_st:939917-HuGene_st:110205-HuGene_st:660721-HuGene_st:905229-HuGene_ st:223551_at:610426-HuGene_st | 5.94E-20 | 3.1204 | 3.28 | 94.1 | 88.6-97.3 |
| ANGPT L1 | 572942-HuGene_st:891312-HuGene_st:953040-HuGene_st:232844-HuGene_st:145730-HuGene_st:142205-HuGene_st:227771-HuGene_st:80584-HuGene_st:982090-HuGene_st:999640-HuGene_st:672931-HuGene_st:148578-HuGene_st:224339_s_at:1046706-HuGene_st:239183_at:155600-HuGene_st:284674-HuGene_st:231773_at:818064-HuGene_st:978991-HuGene_st:728775-HuGene_st | 1.42E-17 | 2.7964 | 4.64 | 91.9 | 85.6-95.9 |
| SI | 9605-HuGene_st:514814-HuGene_st:809314-HuGene_st:576613-HuGene_st:45502-HuGene_st:369676-HuGene_st:438636-HuGene_st:267204-HuGene_st:326716-HuGene_st:897825-HuGene_st:377666-HuGene_st:519273-HuGene_st:325741-HuGene_st:245381-HuGene_st:108368-HuGene_st:464198-HuGene_st:679193-HuGene_st:168180-HuGene_st | 5.40E-03 | 0.9299 | 2.79 | 67.9 | 58.4-76.4 |
| HPGD | 291863-HuGene_st:375608-HuGene_st:793406-HuGene_st:436293-HuGene_st:75568-HuGene_st:211549_s_at:684728-HuGene_st:674596-HuGene_st:527856-HuGene_st:329920-HuGene_st:748432-HuGene_st:259392-HuGene_st:769902-HuGene_st:620673-HuGene_st:450707-HuGene_st:203913_s_at:304752-HuGene_st:447604-HuGene_st:170968-HuGene_ _st:852359-HuGene_st:836377-HuGene_st:242733_at:243846-HuGene_st:136281-HuGene_st:203914_x_at:211548_s_at:288252-HuGene_ st | 3.65E-17 | 2.8462 | 3.21 | 92.3 | 86.2-96.1 |
| CLDN8 | 1018006-HuGene_st:190634-HuGene_st:590280-HuGene_ st:186468-HuGene_st:954438-HuGene_st:428391-HuGene_st:480543-HuGene_st:944337-HuGene_st:179725-HuGene_st:508584-HuGene_st:1009114-HuGene_st:948216-HuGene_st:658285-HuGene_st:1022600-HuGene_st:737498-HuGene_st:470015-HuGene_st:103315-HuGene_st:699348-HuGene_st:89877-HuGene_st:56937-HuGene_st:862663-HuGene_ st:504945-HuGene_st:214598_at | 2.92E-10 | 1.8639 | 12.96 | 82.4 | 74.1-88.9 |
| UGT2A3 | 149647-HuGene_st:860083-HuGene_st:922544-HuGene_st:244206-HuGene_st:503323-HuGene_st:353576-HuGene_st:603619-HuGene_st:787458-HuGene_st:219796-HuGene_st:333564-HuGene_st:257402-HuGene_st:366699-HuGene_st:461685-HuGene_st:891681-HuGene_st:644952-HuGene_st:621618-HuGene_st:737617-HuGene_st:88682-HuGene_st:529761-HuGene_st:895203-HuGene_st:658594-HuGene_ st:455115-HuGene st | 8.64E-09 | 1.7422 | 4.89 | 80.8 | 72.3-87.5 |
| HHLA2 | 371335-HuGene_st:978721-HuGene_st:1065567-HuGene_st:282548-HuGene_st:240410-HuGene_st:170899-HuGene_st:947848-HuGene_st:438234-HuGene_st:220812_s_at:927495-HuGene_st:351364-HuGene_st:234673_at:993142-HuGene_st:1009637-HuGene_st:335000-HuGene_st:285313-HuGene_st:533646-HuGene_st:234624_at:458597-HuGene_st:104838-HuGene_st:26687-HuGene_st:258409-HuGene_st:493304-HuGene_st:378019-HuGene_st:576796-HuGene_st | 3.47E-17 | 2.6743 | 10.02 | 90.9 | 84.3-95.2 |
| UGT1A8 | 116025-HuGene_st:6488-HuGene_st:881135-HuGene_st:230953_at:221304_at:221305_s_at:511 516-HuGene_st:594963-HuGene_st:396121-HuGene_st:123777-HuGene_st:1016481-HuGene_st:204532_x_at:683377-HuGene_st:1055169-HuGene_st:1035103-HuGene_st:1088102-HuGene_st:207126_x_at:42874-HuGene_st:215125_s_at:206094_x_at:208596_s_a t:97211-HuGene_st:1009861-HuGene_st:603368-HuGene_st:232654_s_at:625897-HuGene_ st:998604-HuGene_st | 1.32E-11 | 2.0703 | 4.56 | 85 | 77.1-90.9 |
| CNTN3 | 267567-HuGene_st:782053-HuGene_st:550157-HuGene_st:541394-HuGene_st:989173-HuGene_st:15899-HuGene_st:78622-HuGene_st:339738-HuGene_st:585282-HuGene_st:661814-HuGene_st:360715-HuGene_st:695033-HuGene_st:483058-HuGene_st:555394-HuGene_st:315011-HuGene_st:905374-HuGene_st:1067212-HuGene_st:557263-HuGene_st:233502_at:811729-HuGene_ st:87414-HuGene_st | 1.20E-07 | 1.6155 | 3.93 | 79 | 70.3-86.1 |
| P2RY1 | 328249-HuGene_st:113454-HuGene_st:627857-HuGene_st:461281-HuGene_st:207455_at:259065-HuGene_st:797734-HuGene_st:135788-HuGene_st:42916-HuGene_st:315405-HuGene_st:340050-HuGene_st: 173225-HuGene_st:919818-HuGene_st:591228-HuGene_st:899117-HuGene_st:785070-HuGene_ st:286200-HuGene_st:231925_at | 1.41E-15 | 2.5326 | 3.6 | 89.7 | 82.8-94.4 |
| SORBS2 | 238751_at:805920-HuGene_st | 4.23E-05 | 1.3026 | 2.1 | 74.3 | 65.1-82 |

**TABLE 5**

| **Gene** | **Normal Blood** | **MRC5** | **CaCo2** | **HCT116** | **HT29** | **SW480** |
|---|---|---|---|---|---|---|
| **CXCL12** | Unmeth | low | unmeth | meth | meth | unmeth |
| **DF** | Low | meth | meth | meth | unmeth | meth |
| **MAMDC2** | Unmeth | unmeth | meth | unmeth | unmeth | meth |
| **CA4** | unmeth | unmeth | meth | meth | meth | unmeth |
| **MT1M** | low | low | low | meth | low | meth |
| **P2RY14** | unmeth | Low | Meth | Meth | Unmeth | Meth |
| **GPM6B** | Low | Low | Low | Meth | Meth | Meth |
| ADAMDEC1 | low | Unmeth | Meth | Meth | unmeth | meth |

**TABLE 6**

| | Probe Set ID | Target Sequence |
|---|---|---|
| **1** | 200600 _at | |
| **2** | 200621 _at | |
| **3** | 200665 _s_at | |
| **4** | 200795 _at | |
| **5** | 200799 _at | |
| **6** | 200845 _s_at | |
| **7** | 200859 _x_at | |
| **8** | 200884 _at | |
| **9** | 200897 _s_at | |
| **10** | 200974 _at | |
| | | |
| **11** | 200986 _at | |
| **12** | 201041 _s_at | |
| **13** | 201058 _s_at | |
| **14** | 201061 _s_at | |
| **15** | 201069 _at | |
| **16** | 201105 _at | |
| **17** | 201137 _s_at | |
| **18** | 201141 _at | |
| **19** | 201150 _s_at | |
| **20** | 201289 _at | |
| | | |
| **21** | 201300 _s_at | |
| **22** | 201324 _at | |
| **23** | 201348 _at | |
| **24** | 201426 _s_at | |
| **25** | 201427 _s_at | |
| **26** | 201438 _at | |
| **27** | 201495 _x_at | |
| **28** | 201496 _x_at | |
| **29** | 201497 _x_at | |
| **30** | 201539 _s_at | |
| **31** | 201540 _at | |
| **32** | 201616 _s_at | |
| **33** | 201617 _x_at | |
| **34** | 201645 _at | |
| **35** | 201667 _at | |
| **36** | 201739 _at | |
| **37** | 201743 _at | |
| **38** | 201744 _s_at | |
| **39** | 201842 _s_at | |
| **40** | 201852 _x_at | |
| **41** | 201858 _s_at | |
| **42** | 201859 _at | |
| **43** | 201865 _x_at | |
| **44** | 201893 _x_at | |
| **45** | 201920 _at | |
| **46** | 201957 _at | |
| **47** | 202007 _at | |
| **48** | 202037 _s_at | |
| **49** | 202069 _s_at | |
| **50** | 202133 _at | |
| **51** | 202222 _s_at | |
| **52** | 202242 _at | |
| **53** | 202266 _at | |
| **54** | 202274 _at | |
| **55** | 202283 _at | |
| **56** | 202291 _s_at | |
| **57** | 202350 _s_at | |
| **58** | 202388 _at | |
| **59** | 202403 _s_at | |
| **60** | 202555 _s_at | |
| **61** | 202620 _s_at | |
| | | |
| **62** | 202686 _s_at | |
| **63** | 202731 _at | |
| **64** | 202741 _at | |
| **65** | 202742 _s_at | |
| **66** | 202746 _at | |
| **67** | 202760 _s_at | |
| **68** | 202766 _s_at | |
| **69** | 202768 _at | |
| **70** | 202838 _at | |
| **71** | 202888 _s_at | |
| **72** | 202920 _at | |
| **73** | 202953 _at | |
| **74** | 202957 _at | |
| **75** | 202992 _at | |
| **76** | 202994 _s_at | |
| **77** | 202995 _s_at | |
| **78** | 203000 _at | |
| **79** | 203001 _s_at | |
| **80** | 203058 _s_at | |
| | | |
| **81** | 203060 _s_at | |
| **82** | 203066 _at | |
| **83** | 203131 _at | |
| **84** | 203240 _at | |
| **85** | 203296 _s_at | |
| **86** | 203305 _at | |
| **87** | 203343 _at | |
| **88** | 203382 _s_at | |
| **89** | 203474 _at | |
| | | |
| **90** | 203477 _at | |
| **91** | 203638 _s_at | |
| **92** | 203645 _s_at | |
| **93** | 203680 _at | |
| **94** | 203729 _at | |
| **95** | 203748 _x_at | |
| **96** | 203766 _s_at | |
| **97** | 203881 _s_at | |
| **98** | 203908 _at | |
| **99** | 203913 _s_at | |
| | | |
| **100** | 203914 _x_at | |
| **101** | 203951 _at | |
| **102** | 203963 _at | |
| **103** | 203980 _at | |
| **104** | 204018 _x_at | |
| **105** | 204034 _at | |
| **106** | 204036 _at | |
| **107** | 204069 _at | |
| **108** | 204083 _s_at | |
| **109** | 204122 _at | |
| **110** | 204130 _at | |
| **111** | 204135 _at | |
| **112** | 204326 _x_at | |
| **113** | 204388 _s_at | |
| **114** | 204389 _at | |
| **115** | 204438 _at | |
| **116** | 204457 _s_at | |
| **117** | 204508 _s_at | |
| **118** | 204532 _x_at | |
| **119** | 204570 _at | |
| **120** | 204607 _at | |
| | | |
| **121** | 204673 _at | |
| **122** | 204688 _at | |
| **123** | 204697 _s_at | |
| **124** | 204719 _at | |
| **125** | 204745 _x_at | |
| **126** | 204818 _at | |
| **127** | 204834 _at | |
| **128** | 204894 _s_at | |
| **129** | 204895 _x_at | |
| **130** | 204897 _at | |
| | | |
| **131** | 204931 _at | |
| **132** | 204938 _s_at | |
| **133** | 204939 _s_at | |
| **134** | 204940 _at | |
| **135** | 204955 _at | |
| **136** | 205097 _at | |
| **137** | 205112 _at | |
| **138** | 205200 _at | |
| **139** | 205259 _at | |
| **140** | 205267 _at | |
| **141** | 205382 _s_at | |
| **142** | 205403 _at | |
| **143** | 205412 _at | |
| | | |
| **144** | 205433 _at | |
| **145** | 205464 _at | |
| **146** | 205480 _s_at | |
| **147** | 205547 _s_at | |
| **148** | 205554 _s_at | |
| **149** | 205593 _s_at | |
| **150** | 205683 _x_at | |
| **151** | 205892 _s_at | |
| **152** | 205929 _at | |
| | | |
| **153** | 205935 _at | |
| **154** | 205950 _s_at | |
| **155** | 206000 _at | |
| **156** | 206094 _x_at | |
| **157** | 206134 _at | |
| **158** | 206143 _at | |
| **159** | 206149 _at | |
| **160** | 206198 _s_at | |
| **161** | 206199 _at | |
| **162** | 206208 _at | |
| **163** | 206209 _s_at | |
| **164** | 206262 _at | |
| **165** | 206377 _at | |
| **166** | 206385 _s_at | |
| **167** | 206422 _at | |
| **168** | 206461 _x_at | |
| **169** | 206561 _s_at | |
| **170** | 206576 _s_at | |
| **171** | 206637 _at | |
| **172** | 206641 _at | |
| **173** | 206664 _at | |
| | | |
| **174** | 206710 _s_at | |
| **175** | 206784 _at | |
| **176** | 207003 _at | |
| **177** | 207080 _s_at | |
| **178** | 207126 _x_at | |
| **179** | 207134 _x_at | |
| **180** | 207245 _at | |
| **181** | 207266 _x_at | |
| **182** | 207390 _s_at | |
| **183** | 207392 _x_at | |
| **184** | 207432 _at | |
| | | |
| **185** | 207502 _at | |
| **186** | 207761 _s_at | |
| **187** | 207961 _x_at | |
| **188** | 207977 _s_at | |
| **189** | 207980 _s_at | |
| **190** | 208131 _s_at | |
| **191** | 208370 _s_at | |
| **192** | 208383 _s_at | |
| **193** | 208399 _s_at | |
| **194** | 208450 _at | |
| | | |
| **195** | 208581 _x_at | |
| **196** | 208596 _s_at | |
| **197** | 208747 _s_at | |
| **198** | 208763 _s_at | |
| **199** | 208788 _at | |
| **200** | 208789 _at | |
| **201** | 208791 _at | |
| **202** | 208792 _s_at | |
| **203** | 208894 _at | |
| **204** | 208920 _at | |
| **205** | 209047 _at | |
| | | |
| **206** | 209074 _s_at | |
| **207** | 209101 _at | |
| **208** | 209114 _at | |
| **209** | 209116 _x_at | |
| **210** | 209138 _x_at | |
| **211** | 209147 _s_at | |
| **212** | 209156 _s_at | |
| **213** | 209167 _at | |
| **214** | 209170 _s_at | |
| **215** | 209191 _at | |
| | | |
| **216** | 209209 _s_at | |
| **217** | 209210 _s_at | |
| **218** | 209283 _at | |
| **219** | 209301 _at | |
| **220** | 209312 _x_at | |
| **221** | 209335 _at | |
| **222** | 209357 _at | |
| **223** | 209373 _at | |
| **224** | 209374 _s_at | |
| **225** | 209436 _at | |
| **226** | 209457 _at | |
| | | |
| **227** | 209458 _x_at | |
| **228** | 209496 _at | |
| **229** | 209498 _at | |
| **230** | 209612 _s_at | |
| **231** | 209613 _s_at | |
| **232** | 209621 _s_at | |
| **233** | 209651 _at | |
| **234** | 209656 _s_at | |
| **235** | 209667 _at | |
| **236** | 209668 _x_at | |
| **237** | 209687 _at | |
| **238** | 209735 _at | |
| **239** | 209763 _at | |
| **240** | 209791 _at | |
| **241** | 209868 _s_at | |
| **242** | 209948 _at | |
| **243** | 210084 _x_at | |
| **244** | 210107 at | |
| **245** | 210133 _at | |
| **246** | 210139 _s_at | |
| | | |
| **247** | 210298 _x_at | |
| **248** | 210299 _s_at | |
| **249** | 210302 s at | |
| **250** | 210495 _x_at | |
| **251** | 210517 _s_at | |
| **252** | 210524 _x_at | |
| **253** | 210735 _s_at | |
| **254** | 210764 _s_at | |
| **255** | 210809 _s_at | |
| **256** | 210946 _at | |
| **257** | 210982 _s_at | |
| **258** | 211161 _s_at | |
| **259** | 211372 _s_at | |
| **260** | 211538 _s_at | |
| **261** | 211548 _s_at | |
| **262** | 211549 _s_at | |
| **263** | 211596 _s_at | |
| **264** | 211637 _x_at | |
| **265** | 211643 _x_at | |
| **266** | 211644 _x_at | |
| **267** | 211645 _x_at | |
| **268** | 211671 _s_at | |
| **269** | 211696 _x_at | |
| **270** | 211699 _x_at | |
| **271** | 211719 _x_at | |
| **272** | 211745 _x_at | |
| **273** | 211798 _x_at | |
| **274** | 211813 _x_at | |
| **275** | 211848 _s_at | |
| **276** | 211889 _x_at | |
| **277** | 211896 _s_at | |
| **278** | 211959 _at | |
| **279** | 211964 _at | |
| **280** | 211985 _s_at | |
| **281** | 211990 _at | |
| **282** | 211991 _s_at | |
| **283** | 212077 at | |
| **284** | 212091 _s_at | |
| **285** | 212097 _at | |
| **286** | 212136 _at | |
| **287** | 212158 _at | |
| **288** | 212185 _x_at | |
| **289** | 212192 _at | |
| **290** | 212195 _at | |
| | | |
| **291** | 212224 _at | |
| **292** | 212230 _at | |
| **293** | 212233 _at | |
| **294** | 212265 _at | |
| **295** | 212288 _at | |
| **296** | 212386 _at | |
| **297** | 212387 _at | |
| **298** | 212397 _at | |
| **299** | 212414 _s_at | |
| **300** | 212419 _at | |
| **301** | 212464 _s_at | |
| **302** | 212592 _at | |
| **303** | 212667 _at | |
| **304** | 212671 _s_at | |
| **305** | 212713 _at | |
| **306** | 212730 _at | |
| **307** | 212741 _at | |
| **308** | 212764 _at | |
| **309** | 212814 _at | |
| **310** | 212859 _x_at | |
| **311** | 212956 _at | |
| **312** | 213068 _at | |
| **313** | 213071 _at | |
| **314** | 213317 _at | |
| **315** | 213428 _s_at | |
| **316** | 213451 _x_at | |
| **317** | 213509 _x_at | |
| **318** | 213624 _at | |
| **319** | 213629 _x_at | |
| **320** | 213746 _s_at | |
| **321** | 213891 _s_at | |
| **322** | 213921 _at | |
| **323** | 213953 _at | |
| | | |
| **324** | 214027 _x_at | |
| **325** | 214038 _at | |
| **326** | 214091 _s_at | |
| **327** | 214142 _at | |
| **328** | 214164 _x_at | |
| **329** | 214414 _x_at | |
| **330** | 214433 _s_at | |
| **331** | 214505 _s_at | |
| **332** | 214598 _at | |
| **333** | 214677 _x_at | |
| **334** | 214696 _at | |
| | | |
| **335** | 214752 _x_at | |
| **336** | 214768 _x_at | |
| **337** | 214777 _at | |
| **338** | 214916 _x_at | |
| **339** | 215049 _x_at | |
| **340** | 215076 _s_at | |
| **341** | 215118 _s_at | |
| **342** | 215125 _s_at | |
| **343** | 215176 _x_at | |
| **344** | 215193 _x_at | |
| **345** | 215299 _x_at | |
| **346** | 215382 _x_at | |
| **347** | 215388 _s_at | |
| **348** | 215657 _at | |
| **349** | 215867 _x_at | |
| **350** | 216207 _x_at | |
| **351** | 216336 _x_at | |
| **352** | 216401 _x_at | |
| **353** | 216442 _x_at | |
| **354** | 216474 _x_at | |
| **355** | 216491 _x_at | |
| **356** | 216510 _x_at | |
| **357** | 216576 _x_at | |
| **358** | 216834 _at | |
| **359** | 216984 _x_at | |
| **360** | 217022 _s_at | |
| **361** | 217109 _at | |
| **362** | 217110 _s_at | |
| **363** | 217148 _x_at | |
| **364** | 217165 _x_at | |
| **365** | 217179 _x_at | |
| **366** | 217232 _x_at | |
| **367** | 217235 _x_at | |
| **368** | 217258 _x_at | |
| **369** | 217378 _x_at | |
| **370** | 217414 _x_at | |
| **371** | 217480 _x_at | |
| **372** | 217546 _at | |
| | | |
| **373** | 217757 _at | |
| **374** | 217762 _s_at | |
| **375** | 217764 _s_at | |
| **376** | 217767 _at | |
| **377** | 217897 _at | |
| **378** | 217967 _s_at | |
| **379** | 218087 _s_at | |
| **380** | 218162 _at | |
| **381** | 218224 _at | |
| **382** | 218312 _s_at | |
| **383** | 218353 _at | |
| **384** | 218418 _s_at | |
| **385** | 218468 _s_at | |
| **386** | 218469 _at | |
| **387** | 218541 _s_at | |
| **388** | 218546 _at | |
| **389** | 218559 _s_at | |
| **390** | 218756 _s_at | |
| **391** | 219014 _at | |
| | | |
| **392** | 219059 _s_at | |
| **393** | 219087 _at | |
| **394** | 219508 _at | |
| **395** | 219543 _at | |
| **396** | 219607 _s_at | |
| **397** | 219669 _at | |
| **398** | 219796 _s_at | |
| **399** | 219799 _s_at | |
| **400** | 219948 _x_at | |
| **401** | 220026 _at | |
| **402** | 220037 _s_at | |
| | | |
| **403** | 220075 _s_at | |
| **404** | 220266 _s_at | |
| **405** | 220376 _at | |
| **406** | 220468 _at | |
| **407** | 220645 _at | |
| **408** | 220812 _s_at | |
| **409** | 220834 _at | |
| **410** | 221004 _s_at | |
| **411** | 221305 _s_at | |
| **412** | 221541 _at | |
| **413** | 221584 _s_at | |
| **414** | 221667 _s_at | 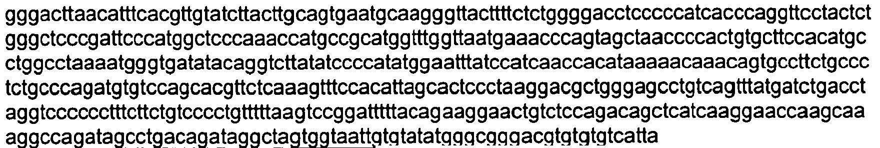 |
| **415** | 221747 _at | 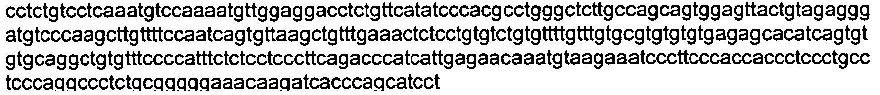 |
| **416** | 221748 _s_at | 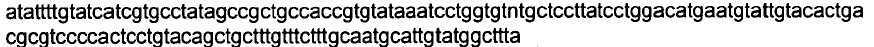 |
| **417** | 221841 _s_at | 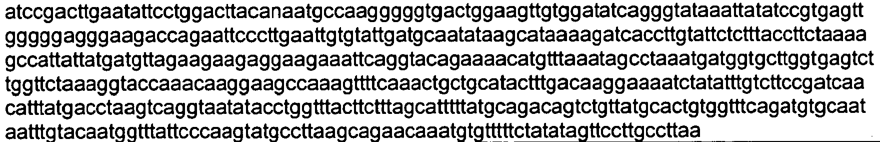 |
| **418** | 221896 _s_at | 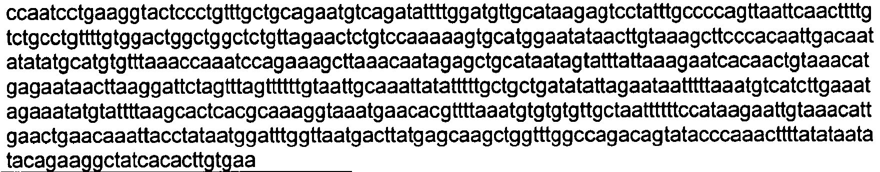 |
| **419** | 222043 _at | 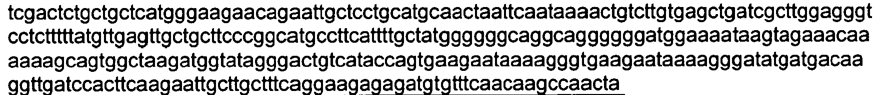 |
| **420** | 222162 _s_at | 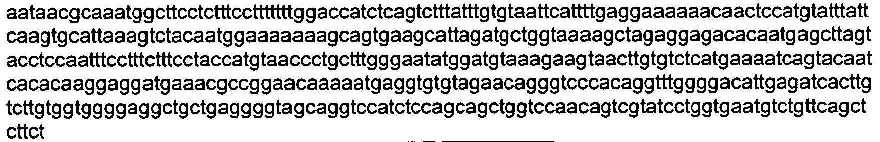 |
| **421** | 222453 _at | 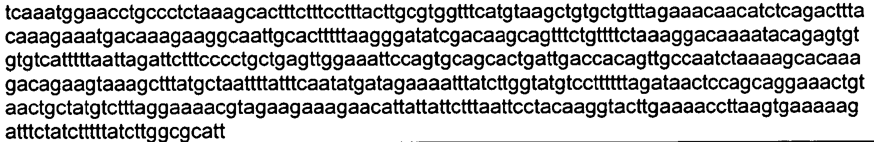 |
| **422** | 222513 _s_at | 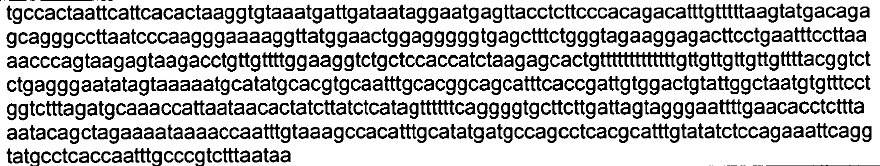 |
| **423** | 222717 _at | 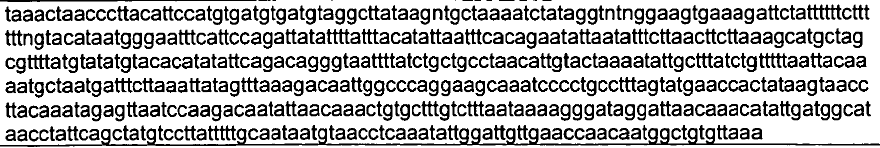 |
| **424** | 222722 _at | 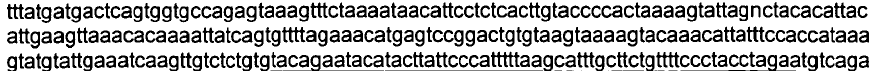 |
| | | |
| **425** | 223121 _s_at | |
| **426** | 223122 _s_at | |
| **427** | 223235 _s_at | |
| **428** | 223343 _at | |
| **429** | 223395 _at | |
| **430** | 223484 _at | |
| **431** | 223551 _at | |
| **432** | 223597 _at | |
| **433** | 223623 _at | |
| **434** | 223754 _at | |
| **435** | 223952 _x_at | |
| **436** | 224009 _x_at | |
| **437** | 224342 _x_at | |
| **438** | 224352 _s_at | |
| **439** | 224412 _s_at | |
| **440** | 224480 _s_at | |
| **441** | 224560 _at | |
| **442** | 224663 _s_at | |
| **443** | 224694 _at | |
| **444** | 224823 _at | |
| | | |
| **445** | 224836 _at | |
| **446** | 224840 _at | |
| **447** | 224959 _at | |
| **448** | 224963 _at | |
| **449** | 224964 _s_at | |
| **450** | 224989 _at | |
| **451** | 224990 _at | |
| **452** | 225207 _at | |
| **453** | 225242 _s_at | |
| **454** | 225269 _s_at | |
| | | |
| **455** | 225275 _at | |
| **456** | 225353 _s_at | |
| **457** | 225381 _at | |
| **458** | 225442 _at | |
| **459** | 225458 _at | |
| **460** | 225575 _at | |
| **461** | 225602 _at | |
| **462** | 225604 _s_at | |
| **463** | 225626 _at | |
| **464** | 225688 _s_at | |
| | | |
| **465** | 225710 _at | |
| **466** | 225720 _at | |
| **467** | 225721 _at | |
| **468** | 225728 _at | |
| **469** | 225782 _at | |
| **470** | 225894 _at | |
| **471** | 225895 _at | |
| **472** | 226001 _at | |
| **473** | 226051 _at | |
| **$47 4.00** | 226084 _at | |
| | | |
| **475** | 226103 _at | |
| **476** | 226147 _s_at | |
| **477** | 226302 _at | |
| **478** | 226303 at | |
| **479** | 226304 _at | |
| **480** | 226333 _at | |
| **481** | 226430 _at | |
| **482** | 226492 _at | |
| **483** | 226594 _at | |
| **484** | 226654 _at | |
| | | |
| **485** | 226682 _at | |
| **486** | 226694 _at | |
| **487** | 226811 _at | |
| **488** | 226818 at | |
| **489** | 226834 _at | |
| **490** | 226841 _at | |
| **491** | 227006 at | |
| **492** | 227052 _at | |
| **493** | 227061 _at | |
| **494** | 227099 _s_at | |
| | | |
| **495** | 227235 _at | |
| **496** | 227265 _at | |
| **497** | 227404 _s_at | |
| **498** | 227522 _at | |
| **499** | 227529 _s_at | |
| **500** | 227561 _at | |
| **501** | 227623 _at | |
| **502** | 227662 _at | |
| **503** | 227682 _at | |
| **504** | 227705 _at | |
| **505** | 227725 _at | |
| | | |
| **506** | 227727 _at | |
| **507** | 227735 _ s_at | |
| **508** | 227736 _at | |
| **509** | 227826 s at | |
| **510** | 227827 _at | |
| **511** | 228133 _s_at | |
| **512** | 228195 _at | |
| **513** | 228202 _at | |
| **514** | 228232 _s_at | |
| **515** | 228241 _at | |
| **516** | 228469 _at | |
| **517** | 228504 at | |
| **518** | 228507 _at | |
| **519** | 228640 at | |
| **520** | 228706 _s_at | |
| **521** | 228707 _at | |
| **522** | 228750 _at | |
| **523** | 228766 _at | |
| **524** | 228846 _at | |
| **525** | 228854 _at | |
| **526** | 228885 _at | |
| **527** | 228961 _at | |
| **528** | 229070 _at | |
| **529** | 229254 _at | |
| | | |
| **530** | 229530 _at | |
| **531** | 229659 _s_at | |
| **532** | 229831 at | |
| **533** | 229839 _at | |
| **534** | 230087 _at | |
| **535** | 230264 _s_at | |
| **536** | 230595 _at | |
| **537** | 230788 _at | |
| **538** | 230830 _at | |
| **539** | 231120 _x_at | |
| **540** | 231579 _s_at | |
| | | |
| **541** | 231773 _at | |
| **542** | 231925 _at | |
| **543** | 231975 _s_at | |
| **544** | 233565 _s_at | |
| **545** | 234764 _x_at | |
| **546** | 234987 _at | |
| **547** | 235146 at | |
| **548** | 235766 _x_at | |
| **549** | 235849 _at | |
| **550** | 236300 _at | |
| **551** | 236313 _at | |
| | | |
| **552** | 238143 _at | |
| **553** | 238750 _at | |
| **554** | 238751 at | |
| **555** | 239272 _at | |
| **556** | 241994 _at | |
| **557** | 242317 at | |
| **558** | 242447 _at | |
| **559** | 242601 _at | |
| **560** | 243278 _at | |
| **561** | 200832 _s_at | |
| **562** | 201147_s_at | |
| | | |
| **563** | 201162 _at | |
| **564** | 201163 _s_at | |
| **565** | 201185 _at | |
| **566** | 201261 _x_at | |
| **567** | 201792 _at | |
| **568** | 202237 _at | |
| **569** | 202238 _s_at | |
| **570** | 202310 _s_at | |
| **571** | 202311 _s_at | |
| **572** | 202404 _s_at | |
| **573** | 202450 _s_at | |
| | | |
| **574** | 202859 _x_at | |
| **575** | 202878 _s_at | |
| **576** | 202917 _s_at | |
| **577** | 202998 _s_at | |
| **578** | 203083 _at | |
| **579** | 203325 _s_at | |
| **580** | 203570 _at | |
| **581** | 203878 _s_at | |
| **582** | 204006 _s_at | |
| **583** | 204051 _s_at | |
| | | |
| **584** | 204320 _at | |
| **585** | 204475 _at | |
| **586** | 204620 _s_at | |
| **587** | 204811 _s_at | |
| **588** | 205479 _s_at | |
| **589** | 205765 _at | |
| **590** | 205828 _at | |
| **591** | 205927 _s_at | |
| **592** | 207173 _x_at | |
| **593** | 207191 _s_at | |
| | | |
| **594** | 208063 _s_at | |
| **595** | 208782 _at | |
| **596** | 208850 _s_at | |
| **597** | 208851 _s_at | |
| **598** | 208937 _s_at | |
| **599** | 209218 _at | |
| **600** | 209395 _at | |
| **601** | 209396 _s_at | |
| **602** | 209596 _at | |
| **603** | 209875 _s_at | |
| | | |
| **604** | 209955 _s_at | |
| **605** | 210095 _s_at | |
| **606** | 210511 _s_at | |
| **607** | 211571 _s_at | |
| **608** | 211966 _at | |
| **609** | 211980 _at | |
| **610** | 211981 _at | |
| **611** | 212344 _at | |
| **612** | 212353 _at | |
| | | |
| **613** | 212354 _at | |
| **614** | 212488 _at | |
| **615** | 212489 _at | |
| **616** | 213106 _at | |
| **617** | 213125 _at | |
| **618** | 213524 _s_at | |
| **619** | 213869 _x_at | |
| **620** | 213905 _x_at | |
| **621** | 214234 _s_at | |
| **622** | 214235 _at | |
| **623** | 214247_s_at | |
| | | |
| **624** | 215646 _s_at | |
| **625** | 217430 _x_at | |
| **626** | 217763 _s_at | |
| **627** | 218211 _s_at | |
| **628** | 218638 _s_at | |
| **629** | 219955 _at | |
| **630** | 221011 _s_at | |
| **631** | 221729 _at | |
| **632** | 221730 _at | |
| **633** | 221731 _x_at | |
| **634** | 221874_at | |
| | | |
| **635** | 223969 _s_at | |
| **636** | 223970 _at | |
| **637** | 224724 _at | |
| **638** | 225664 _at | |
| **639** | 225681 _at | |
| **640** | 225799 _at | |
| **641** | 226237 _at | |
| **642** | 226248 _s_at | |
| **643** | 226311 _at | |
| **644** | 226777 at | |
| | | |
| **645** | 226930 _at | |
| **646** | 227140 _at | |
| **647** | 227566 _at | |
| **648** | 227676 _at | |
| **649** | 227719 _at | |
| **650** | 229218 _at | |
| **651** | 229802 _at | |
| **652** | 231766 _s_at | |
| **653** | 231832 _at | |
| **654** | 231879 _at | |
| **655** | 232176 _at | |
| **656** | 232458 _at | |
| **657** | 232481 _s_at | |
| **658** | 233555 _s_at | |
| **659** | 234994 _at | |
| **660** | 235976 _at | |
| **661** | 236894 _at | |
| **662** | 237521 _x_at | |
| **663** | 37892_ at | |

**TABLE 7: MSP primers and PCR conditions**

| **Gene** | **Probe set** | **Gene Name** | **Forward primer 5'- 3'** | **Reverse primer 5' - 3'** | **Re-anneal Temp** |
|---|---|---|---|---|---|
| CA4 | 206208_at | Carbonic anhydrase IV | | GCGCACCGAAAAAACCG (SEQ ID NO:665) | 61.0°C |
| CXCL1 2 | 209687_at | Chemokine,CXC Motif, Ligand 12 | | | 64.0°C |
| DF | 205382_s _at | complement factor D | | AAACGAACCGCTCCCCG (SEQ ID NO:669) | 64.0°C |
| MAMD C2 | 228885_at | MAM domain-containing protein 2 precursor | | | 60.0°C |
| MT1M | 217546_at | Metallothionein-1 M | | GCTTACACCCGCCCGACTA (SEQ ID NO:673) | 62.0°C |
| CAGE | | Control unbiased amplification assay | | | 61.0°C |

**TABLE 8: COBRA Primers and PCR conditions**

| **Gene** | **Probe set** | **Gene Name** | **Forward primer 5'- 3'** | **Reverse primer 5' - 3'** | **Re-anneal Temp, Enzyme** |
|---|---|---|---|---|---|
| ADAM DECI | 206134_at | ADAM-like, decysin 1 | | | 61.0°C |
| | | | | | BstU I |
| GPM6B | 209170_s _at | Neuronal membrane glycoprotein M6-b | | | 60.0°C |
| | | | | | Hinf I |
| P2RY14 | 206637_at | Purinergic receptor P2Y, G-protein coupled, 14 | | | 60.0°C |
| | | | | | Acl I |

### BIBLIOGRAPHY

Affymetrix. GeneChip expression data analysis fundamentals. Affymetrix, Santa Clara, CA USA, 2001.
Alon et al., Proc. Natl. Acad. Sci. USA: 96, 6745-6750, June 1999
Ausubel, F. et al., "Current Protocols in Molecular Biology", John Wiley & Sons, (1998)
Bonner et al (1973) J. Mol. Biol. 81:123
Clark et al. 2006, Nature Protocols I:2353-2364
DeRisi, et al., Nature Genetics 14:457-460 (1996)
Germer et al., Genome Res. 10:258-266 (2000)
Guo et al., Nucleic Acids Res. 22:5456-5465 (1994)
Heid et al., Genome Res. 6:986-994 (1996)
Hubbell E.W., W. M. Liu, and R. Mei. Robust estimators for expression analysis. Bioinformatics, 18:1585-1592, 2002.
Irizarry R.W., B. M. Bolstad, F. Collin, L. M. Cope, B. Hobbs, and T. P. Speed. Summaries of affymetrix genechip probe level data. Nucleic Acid Research, 31, 2003.
Kraus, M. and Aaronson, S., 1991. Methods Enzymol., 200:546-556
Maskos and Southern, Nuc. Acids Res. 20:1679-84, 1992
Moore et al., BBA, 1402:239-249, 1988
Nielsen (1999) Curr. Opin. Biotechnol. 10:71-75
Nielsen et al. (1991) Science 254: 1497-1500
Pease et al., Proc. Natl. Acad. Sci. USA 91(11):5022-5026 (1994)
Pevzner et al., J. Biomol. Struc. & Dyn. 9:399-410, 1991
Schena, et al. Science 270:467-470 (1995)
Smith et al., Science 258:1122-1126 (1992)
Smyth G.K. Bioinformatics and Computational Biology Solutions using R and Bioconductor. Springer, New York, 2005.
Smyth G.K. Linear models and empirical bayes methods for assessing differential expression in microarray experiments. Statistical Applications in Genetics and Molecular Biology, 3(1):Article 3, 2004.
T. Sano and C. R. Cantor, Bio/Technology 9:1378-81 (1991)
Urdea et al., Nucleic Acids Symp. Ser., 24:197-200 (1991)
Wedemeyer et al., Clinical Chemistry 48:9 1398-1405, 2002)
Weissleder et al., Nature Medicine 6:351-355, 2000

## Claims

1. A method of screening for the onset or predisposition to the onset of a large intestine neoplasm or monitoring the progress of a large intestine neoplasm in an individual, said method comprising assessing the level of expression of one or more genes or transcripts selected from:
**(i)** the gene, genes or transcripts detected by Affymetrix probeset IDs:
212158_at; and/or
**(ii)** SDC2
in a biological sample from said individual wherein a lower level of expression of the genes or transcripts of group (i) and/or group (ii) relative to control levels is indicative of a neoplastic large intestine cell or a cell predisposed to the onset of a neoplastic state.

2. The method according to claim 1, wherein said control level is a non-neoplastic level.

3. The method according to claim 1 or claim 2, wherein said cell is a colorectal cell.

4. The method according to any preceding claim, wherein said biological sample is a faecal sample, enema wash, surgical resection, tissue biopsy or blood sample.

5. The method according to any preceding claim, wherein said level of expression is assessed by screening for changes to mRNA expression, protein expression genomic DNA or the chromatin protein with which said gene is associated.

6. The method according to claim 5 wherein said changes to genomic DNA are changes to DNA methylation.

7. The method according to any preceding claim, wherein said individual is a human.

## Patentansprüche

1. Verfahren zum Screening des Beginns oder der Prädisposition des Beginns von einem Dickdarm-Neoplasma oder Verfolgen des Fortschritts von einem Dickdarm-Neoplasma bei einem Individuum, wobei das Verfahren Bewerten des Expressionsspiegels von einem oder mehreren Genen oder Transkripten umfasst, ausgewählt aus:
(i) dem Gen, Genen oder Transkripten, nachgewiesen durch Affymetrix Sondensatz IDs: 212158_at; und/oder
(ii) SDC2
in einer biologischen Probe von dem Individuum, wobei ein geringerer Expressionsspiegel der Gene oder Transkripte von Gruppe (i) und/oder Gruppe (ii), bezogen auf die Kontrollspiegel, auf eine neoplastische Dickdarmzelle oder eine für den Beginn eines neoplastischen Zustands prädisponierte Zelle hinweist.

2. Verfahren nach Anspruch 1, wobei der Kontrollspiegel ein nicht-neoplastischer Spiegel ist.

3. Verfahren nach Anspruch 1 oder Anspruch 2, wobei die Zelle eine kolorektale Zelle ist.

4. Verfahren nach einem vorstehenden Anspruch, wobei die biologische Probe eine Fäkalprobe, Klistierwaschung, chirurgische Resektion, Gewebebiopsie oder Blutprobe ist.

5. Verfahren nach einem vorstehenden Anspruch, wobei der Expressionsspiegel durch Screening auf Änderungen der mRNA-Expression, Proteinexpression genomischer DNA oder des Chromatinproteins, womit das Gen assoziiert ist, bewertet wird.

6. Verfahren nach Anspruch 5, wobei die Änderungen der genomischen DNA Änderungen der DNA-Methylierung sind.

7. Verfahren nach einem vorstehenden Anspruch, wobei das Individuum ein Mensch ist.

## Revendications

1. Procédé de criblage pour l'apparition ou la prédisposition à l'apparition d'un néoplasme du gros intestin ou de surveillance de la progression d'un néoplasme du gros intestin chez un individu, ledit procédé comprenant l'estimation du taux d'expression d'un ou de plusieurs gènes ou transcrits choisis parmi :
(i) le gène, les gènes ou les transcrits détectés par l'identifiant d'ensemble de sondes Affymetrix : 212158_at ; et/ou
(ii) SDC2
dans un échantillon biologique provenant dudit individu dans lequel un taux inférieur d'expression des gènes ou des transcrits du groupe (i) et/ou du groupe (ii) par rapport à des taux témoins indique une cellule néoplasique du gros intestin ou une cellule prédisposée à l'apparition d'un état néoplasique.

2. Procédé selon la revendication 1, dans lequel ledit taux témoin est un taux non néoplasique.

3. Procédé selon la revendication 1 ou la revendication 2, dans lequel ladite cellule est une cellule colorectale.

4. Procédé selon l'une quelconque des revendications précédentes, dans lequel ledit échantillon biologique est un échantillon fécal, un liquide de lavement, une résection chirurgicale, une biopsie tissulaire ou un échantillon de sang.

5. Procédé selon l'une quelconque des revendications précédentes, dans lequel ledit taux d'expression est estimé par criblage pour des variations d'expression d'ARNm, d'expression de protéines, d'ADN génomique ou de la protéine chromatine avec laquelle ledit gène est associé.

6. Procédé selon la revendication 5 dans lequel lesdites variations d'ADN génomique sont des variations de méthylation d'ADN.

7. Procédé selon l'une quelconque des revendications précédentes, dans lequel ledit individu est un être humain.


### SEQUENCE LISTING

<110> CLINICAL GENOMICS PTY. LTD. COMMONWEALTH SCIENTIFIC AND INDUSTRIAL RESEARCH ORGANISATION LaPointe, Lawrence Dunne, Rob Young, Graeme Lockett, Trevor Wilson, William Molloy, Peter
<120> A method of diagnosing neoplasms - II
<130> 30637603/TDO
<150> US 60/982115
   <151> 2007-10-23
<160> 681
<170> PatentIn version 3.4
<210> 1
   <211> 544
   <212> DNA
   <213> human
<400> 1
   tagtcttggg gcaggatgat tttggcctca ttactttacc acccccacac ctggaaagca     60
   tatactatat tacaaaatga cattttgcca aaattattaa tataagaagc tttcagtatt    120
   agtgatgtca tctgtcacta taggtcatac aatccattct taaagtactt gttatttgtt    180
   tttattatta ctgtttgtct tctccccagg gttcagtccc tcaaggggcc atcctgtccc    240
   accatgcagt gccccctagc ttagagcctc cctcaattcc ccctggccac caccccccac    300
   tctgtgcctg accttgagga gtcttgtgtg cattgctgtg aattagctca cttggtgata    360
   tgtcctatat tggctaaatt gaaacctgga attgtggggc aatctattaa tagctgcctt    420
   aaagtcagta acttaccctt agggaggctg ggggaaaagg ttagattttg tattcagggg    480
   ttttttgtgt actttttggg tttttaaaaa attgtttttg gaggggttta tgctcaatcc    540
   atgt                                                                 544
<210> 2
   <211> 530
   <212> DNA
   <213> human
<400> 2
   gatagcctcc cactaggact gggaggagaa taacccaggt cttaaggacc ccaaagtcag     60
   gatgttgttt gatcttctca aacatctagt tccctgcttg atgggaggat cctaatgaaa    120
   tacctgaaac atatattggc atttatcaat ggctcaaatc ttcatttatc tctggcctta    180
   accctggctc ctgaggctgc ggccagcaga gcccaggcca gggctctgtt cttgccacac    240
   ctgcttgatc ctcagatgtg gagggaggta ggcactgcct cagtcttcat ccaaacacct    300
   ttccctttgc cctgagacct cagaatcttc cctttaaccc aagaccctgc ctcttccact    360
   ccacccttct ccagggaccc ttagatcaca tcactccacc cctgccaggc cccaggttag    420
   gaatagtggt gggaggaagg ggaaagggct gggcctcacc gctcccagca actgaaagga    480
   caacactatc tggagccacc cactgaaagg gctgcaggca tgggctgtac               530
<210> 3
   <211> 416
   <212> DNA
   <213> human
<400> 3
   gttggttcaa acttttggga gcacggactg tcagttctct gggaagtggt cagcgcatcc     60
   tgcagggctt ctcctcctct gtcttttgga gaaccagggc tcttctcagg ggctctaggg    120
   actgccaggc tgtttcagcc aggaaggcca aaatcaagag tgagatgtag aaagttgtaa    180
   aatagaaaaa gtggagttgg tgaatcggtt gttctttcct cacatttgga tgattgtcat    240
   aaggttttta gcatgttcct ccttttcttc accctcccct ttgttcttct attaatcaag    300
   agaaacttca aagttaatgg gatggtcgga tctcacaggc tgagaactcg ttcacctcca    360
   agcatttcat gaaaaagctg cttcttatta atcatacaaa ctctcaccat gatgtg        416
<210> 4
   <211> 511
   <212> DNA
   <213> human
<400> 4
   aacactgcat aacccgtttc tttgaggagt gtgaccccaa caaggataag cacatcaccc     60
   tgaaggagtg gggccactgc tttggaatta aagaagagga catagatgaa aatctcttgt    120
   tttgaacgaa gattttaaag aactcaactt tccagcatcc tcctctgttc taaccacttc    180
   agaaatatat gcagctgtga tacttgtaga tttatattta gcaaaatgtt agcatgtatg    240
   acaagacaat gagagtaatt gcttgacaac aacctatgca ccaggtattt aacattaact    300
   ttggaaacaa aaatgtacaa ttaagtaaag tcaacatatg caaaatactg tacattgtga    360
   acagaagttt aattcatagt aatttcactc tctgcattga cttatgagat aattaatgat    420
   taaactatta atgataaaaa taatgcattt gtattgttca taatatcatg tgcacttcaa    480
   gaaaatggaa tgctactctt ttgtggttta c                                   511
<210> 5
   <211> 456
   <212> DNA
   <213> human
<400> 5
   ggccgacaag aagaaggtgc tggacaagtg tcaagaggtc atctcgtggc tggacgccaa     60
   caccttggcc gagaaggacg agtttgagca caagaggaag gagctggagc aggtgtgtaa    120
   ccccatcatc agcggactgt accagggtgc cggtggtccc gggcctgggg gcttcggggc    180
   tcagggtccc aagggagggt ctgggtcagg ccccaccatt gaggaggtag attaggggcc    240
   tttccaagat tgctgttttt gttttggagc ttcaagactt tgcatttcct agtatttctg    300
   tttgtcagtt ctcaatttcc tgtgtttgca atgttgaaat tttttggtga agtactgaac    360
   ttgctttttt tccggtttct acatgcagag atgaatttat actgccatct tacgactatt    420
   tcttcttttt aatacactta actcaggcca tttttt                              456
<210> 6
   <211> 524
   <212> DNA
   <213> human
<400> 6
   tcctgtcacc cattttgaag agtggcagaa cttgaagttc aacttcctct gtaaatatcc     60
   aagtataaag cccaggaact tctagaataa cccagatgcg ctttaatttt ttttaatatg    120
   ttttgatcac agaacttcta gaataaccca gatgctcttt catattcttt taatacatct    180
   tgatcacagc tgggggaaaa aaagcttttt aattctgtac cttcctagta gataagtgaa    240
   gagcagggaa agagaccttt aaatattttg ctataaaaaa atttgtgata agtttctatc    300
   aaaatgggga gattgcagaa aaggcttccc ttggctccca aggaggtgta gcaggtgtga    360
   gcaatattag tgccatgtgc ctttcacaca gggtttgcat ttatcagtct gttttccgat    420
   gatgtgtaca tgaaagagta caccatgtga agagaagaga gaatgattga aaatgtttta    480
   gtatagaact cttcttgcag tgggttgcta ttttctagat ttta                     524
<210> 7
   <211> 562
   <212> DNA
   <213> human
<400> 7
   gtttgtagac tctctgacca aggccacctg tgccccccag catggggccc cgggtcctgg     60
   gcctgctgac gccagcaagg tggtggccaa gggcctgggg ctgagcaagg cctacgtagg    120
   ccagaagagc agcttcacag tagactgcag caaagcaggc aacaacatgc tgctggtggg    180
   ggttcatggc ccaaggaccc cctgcgagga gatcctggtg aagcacgtgg gcagccggct    240
   ctacagcgtg tcctacctgc tcaaggacaa gggggagtac acactggtgg tcaaatgggg    300
   gcacgagcac atcccaggca gcccctaccg cgttgtggtg ccctgagtct ggggcccgtg    360
   ccagccggca gcccccaagc ctgccccgct acccaagcag ccccgccctc ttcccctcaa    420
   ccccggccca ggccgccctg gccgcccgcc tgtcactgca gctgcccctg ccctgtgccg    480
   tgctgcgctc acctgcctcc ccagccagcc gctgacctct cggctttcac ttgggcagag    540
   ggagccattt ggtggcgctg ct                                             562
<210> 8
   <211> 521
   <212> DNA
   <213> human
<400> 8
   cctcacccag attgaaactc tcttcaagtc taaggactat gagttcatgt ggaaccctca     60
   cctgggctac atcctcacct gcccatccaa cctgggcacc gggctgcggg caggtgtcga    120
   tatcaagctg cccaacctgg gcaagcatga gaagttctcg gaggtgctta agcggctgcg    180
   acttcagaag cgaggcacag gcggtgtgga cacggctgcg gtgggcgggg tcttcgacgt    240
   ctccaacgct gaccgcctgg gcttctcaga ggtggagctg gtgcagatgg tggtggacgg    300
   agtgaagctg ctcatcgaga tggaacagcg gctggagcag ggccaggcca tcgacgacct    360
   catgcctgcc cagaaatgaa gcccggccca cacccgacac cagccctgct gcttcctaac    420
   ttattgcctg cagtgcccac catgcacccc tcgatgttgc cgtctggcga gcccttagcc    480
   ttgctgtaag gaaggcttcc gtcacccttg gtagagttta t                        521
<210> 9
   <211> 381
   <212> DNA
   <213> human
<400> 9
   ctctcttagc tcagttactc aattcatacg tagtattttt taaaataatt ttatatctgt     60
   gtaccacccc atatatttca tattactgtt tcacatgtac agctttctac ttctttgtaa    120
   gaacaccaac caaccaaggt ttaagtgatt aataggcttg agcaccgggt ggcagatgtt    180
   ctatgcagtg tggttcaagt ttctttgacc gcacttatat gcattgctaa tatggaattt    240
   aagataccat acacagtctc tcatggacct atctctattg tagaattatg acttatgtct    300
   tacttggcaa atttttctga atgtgacctt tttttgctga tttgctgggt ttgggattaa    360
   ctagcattat tttgccacct t                                              381
<210> 10
   <211> 523
   <212> DNA
   <213> human
<400> 10
   aagagttacg agttgcctga tgggcaagtg atcaccatcg gaaatgaacg tttccgctgc     60
   ccagagaccc tgttccagcc atccttcatc gggatggagt ctgctggcat ccatgaaacc    120
   acctacaaca gcatcatgaa gtgtgatatt gacatcagga aggacctcta tgctaacaat    180
   gtcctatcag ggggcaccac tatgtaccct ggcattgccg accgaatgca gaaggagatc    240
   acggccctag cacccagcac catgaagatc aagatcattg cccctccgga gcgcaaatac    300
   tctgtctgga tcggtggctc catcctggcc tctctgtcca ccttccagca gatgtggatc    360
   agcaaacagg aatacgatga agccgggcct tccattgtcc accgcaaatg cttctaaaac    420
   actttcctgc tcctctctgt ctctagcaca caactgtgaa tgtcctgtgg aattatgcct    480
   tcagttcttt tccaaatcat tcctagccaa agctctgact cgt                      523
<210> 11
   <211> 513
   <212> DNA
   <213> human
<400> 11
   gacgaccagc caggatatgc tctcaatcat ggagaaattg gaattcttcg atttttctta     60
   tgaccttaac ctgtgtgggc tgacagagga cccagatctt caggtttctg cgatgcagca    120
   ccagacagtg ctggaactga cagagactgg ggtggaggcg gctgcagcct ccgccatctc    180
   tgtggcccgc accctgctgg tctttgaagt gcagcagccc ttcctcttcg tgctctggga    240
   ccagcagcac aagttccctg tcttcatggg gcgagtatat gaccccaggg cctgagacct    300
   gcaggatcag gttagggcga gcgctacctc tccagcctca gctctcagtt gcagccctgc    360
   tgctgcctgc ctggacttgc ccctgccacc tcctgcctca ggtgtccgct atccaccaaa    420
   agggctcctg agggtctggg caagggacct gcttctatta gcccttctcc atggccctgc    480
   catgctctcc aaaccacttt ttgcagcttt ctc                                 513
<210> 12
   <211> 360
   <212> DNA
   <213> human
<400> 12
   gcaataactc tgggaggggc tcgagagggc tggtccttat ttatttaact tcacccgagt     60
   tcctctgggt ttctaagcag ttatggtgat gacttagcgt caagacattt gctgaactca    120
   gcacattcgg gaccaatata tagtgggtac atcaagtcca tctgacaaaa tggggcagaa    180
   gagaaaggac tcagtgtgtg atccggtttc tttttgctcg cccctgtttt ttgtagaatc    240
   tcttcatgct tgacatacct accagtatta ttcccgacga cacatataca tatgagaata    300
   taccttattt atttttgtgt aggtgtctgc cttcacaaat gtcattgtct actcctagaa    360
<210> 13
   <211> 453
   <212> DNA
   <213> human
<400> 13
   gcatcctcaa acatggcgcc aaggataaac acgactaggc catccccagc cccctgacac     60
   ccagcccccg ccagtcaccc ctccccgcac acacccgtcc ataccagctc cctgcccatg    120
   accctcgctc agggatcccc ctttgagggt tagggtccca gttcccagtg gaagaaacag    180
   gccaggagag tgcgtgccga gctgaggcag atgttcccac agtgacccca gagccctggg    240
   ctatagtctc tgacccctcc aaggaaagac caccttctgg ggacatgggc tggagggcag    300
   gacctagagg caccaaggga accgcattcc ggggctgttc cccgaggagg aagggaagcc    360
   tctgtgtgcc ccccaggagg aagaggccct gagtcctggg atcagacacc ccttcacgtg    420
   tatcccacac aaatgcaagc tcaccaaggt ccc                                 453
<210> 14
   <211> 436
   <212> DNA
   <213> human
<400> 14
   tatagctctc tttagctcaa ccactctgtc catccagcca atggatgtcc ttccctgtac     60
   ccaattcaag cttattttag ggaagccttg aaactaccat gtatctggct ctagctgagt    120
   tattgaggat tgagccagtg caacgttaaa ctcagtgcac ttacatttga tttaaatgat    180
   ggttttatct gttgtgtgaa gtggttcacc cttgaggacc aggagcctcc atatcctgac    240
   tgaaaacctt ttctgagact tagagtaaca gtacttttgg ttccttgagt tctcctgtct    300
   ccagatacct aaatgacctt gacttttctg ccttgtgaat tcgtagtcca atcagctgaa    360
   attaaatcac ttgggaggga cgcatagaag gagctctagg aacacagtgc cagtgcagaa    420
   gtttctccag gtggcc                                                    436
<210> 15
   <211> 525
   <212> DNA
   <213> human
<400> 15
   ctcagagcca cccctaaaga gatcctttga tattttcaac gcagccctgc tttgggctgc     60
   cctggtgctg ccacacttca ggctcttctc ctttcacaac cttctgtggc tcacagaacc    120
   cttggagcca atggagactg tctcaagagg gcactggtgg cccgacagcc tggcacaggg    180
   cagtgggaca gggcatggcc aggtggccac tccagacccc tggcttttca ctgctggctg    240
   ccttagaacc tttcttacat tagcagtttg ctttgtatgc actttgtttt tttctttggg    300
   tcttgttttt tttttccact tagaaattgc atttcctgac agaaggactc aggttgtctg    360
   aagtcactgc acagtgcatc tcagcccaca tagtgatggt tcccctgttc actctactta    420
   gcatgtccct accgagtctc ttctccactg gatggaggaa aaccaagccg tggcttcccg    480
   ctcagccctc cctgcccctc ccttcaacca ttccccatgg gaaat                    525
<210> 16
   <211> 398
   <212> DNA
   <213> human
<400> 16
   ctcctggact caatcatggc ttgtggtctg gtcgccagca acctgaatct caaacctgga     60
   gagtgccttc gagtgcgagg cgaggtggct cctgacgcta agagcttcgt gctgaacctg    120
   ggcaaagaca gcaacaacct gtgcctgcac ttcaaccctc gcttcaacgc ccacggcgac    180
   gccaacacca tcgtgtgcaa cagcaaggac ggcggggcct gggggaccga gcagcgggag    240
   gctgtctttc ccttccagcc tggaagtgtt gcagaggtgt gcatcacctt cgaccaggcc    300
   aacctgaccg tcaagctgcc agatggatac gaattcaagt tccccaaccg cctcaacctg    360
   gaggccatca actacatggc agctgacggt gacttcaa                            398
<210> 17
   <211> 489
   <212> DNA
   <213> human
<400> 17
   ggcagcattc aagtccgatg gttcctgaat ggacaggagg aaacagctgg ggtcgtgtcc     60
   accaacctga tccgtaatgg agactggacc atccagatcc tggtgatgct ggaaatgacc    120
   ccccagcagg gagacgtcta catctgccaa gtggagcaca ccagcctgga tagttccgtc    180
   accgtggagt ggaaggcaca gtctgattct gcccagagta agacattgac gggagctggg    240
   ggcttcgtgc tggggctcat catctgtgga gtgggactct tcatgcacag gaggagcaag    300
   aaagttcaac gaggatctgc ataaacaggg ttcctgacct caccgaaaag actaatgtgc    360
   cttagaacaa gcatttgctg tgttttgtta acacctggtt ccaggacaga ccctcagctt    420
   cccaagagga tactgctgcc aagaagttgc tctgaagtca gtttctatcg ttctgctctt    480
   tgattcaaa                                                            489
<210> 18
   <211> 459
   <212> DNA
   <213> human
<400> 18
   agaggcggga tactttcagc tttccatgta actgtatgca taaagccaat gtagtccagt     60
   ttctaagatc atgttccaag ctaactgaat cccacttcaa tacacactca tgaactcctg    120
   atggaacaat aacaggccca agcctgtggt atgatgtgca cacttgctag actcagaaaa    180
   aatactactc tcataaatgg gtgggagtat tttggtgaca acctactttg cttggctgag    240
   tgaaggaatg atattcatat attcatttat tccatggaca tttagttagt gctttttata    300
   taccaggcat gatgctgagt gacactcttg tgtatatttc caaatttttg tatagtcgct     360
   gcacatattt gaaatcatat attaagactt tccaaagatg aggtccctgg tttttcatgg    420
   caacttgatc agtaaggatt tcacctctgt ttgtaacta                           459
<210> 19
   <211> 493
   <212> DNA
   <213> human
<400> 19
   gactttttgg aatagccctg tctagggcaa actgtggccc ccaggagaca ctacccttcc     60
   atgccccaga cctctgtctt gcatgtgaca attgacaatc tggactaccc caagatggca    120
   cccaagtgtt tggcttctgg ctacctaagg ttaacatgtc actagagtat ttttatgaga    180
   gacaaacatt ataaaaatct gatggcaaaa gcaaaacaaa atggaaagta ggggaggtgg    240
   atgtgacaac aacttccaaa ttggctcttt ggaggcgaga ggaaggggag aacttggaga    300
   atagtttttg ctttgggggt agaggcttct tagattctcc cagcatccgc ctttcccttt    360
   agccagtctg ctgtcctgaa acccagaagt gatggagaga aaccaacaag agatctcgaa    420
   ccctgtctag aaggaatgta tttgttgcta aatttcgtag cactgtttac agttttcctc    480
   catgttattt atg                                                       493
<210> 20
   <211> 300
   <212> DNA
   <213> human
<400> 20
   gccacgattg gagaatactt tgcttcatag tattggagca catgttactg cttcattttg     60
   gagcttgtgg agttgatgac tttctgtttt ctgtttgtaa attatttgct aagcatattt    120
   tctctaggct tttttccttt tggggttcta cagtcgtaaa agagataata agattagttg    180
   gacagtttaa agcttttatt cgtcctttga caaaagtaaa tgggagggca ttccatccct    240
   tcctgaaggg ggacactcca tgagtgtctg tgagaggcag ctatctgcac tctaaactgc    300
<210> 21
   <211> 511
   <212> DNA
   <213> human
<400> 21
   ttaggtcaag ttcatagttt ctgtaattgg cttttgaatc aaagaatagg gagacaatct     60
   aaaaaatatc ttaggttgga gatgacagaa atatgattga tttgaagtgg aaaaagaaat    120
   tctgttaatg ttaattaaag taaaattatt ccctgaattg tttgatattg tcacctagca    180
   gatatgtatt acttttctgc aatgttatta ttggcttgca ctttgtgagt atctatgtaa    240
   aaatatatat gtatataaaa tatatattgc ataggacaga cttaggagtt ttgtttagag    300
   cagttaacat ctgaagtgtc taatgcatta acttttgtaa ggtactgaat acttaatatg    360
   tgggaaaccc ttttgcgtgg tccttaggct tacaatgtgc actgaatcgt ttcatgtaag    420
   aatccaaagt ggacaccatt aacaggtctt tgaaatatgc atgtacttta tattttctat    480
   atttgtaact ttgcatgttc ttgttttgtt a                                   511
<210> 22
   <211> 299
   <212> DNA
   <213> human
<400> 22
   caccaaatta cctaggctga ggttagagag attggccagc aaaaactgtg ggaagatgaa     60
   ctttgtcatt atgatttcat tatcacatga ttatagaagg ctgtcttagt gcaaaaaaca    120
   tacttacatt tcagacatat ccaaagggaa tactcacatt ttgttaagaa gttgaactat    180
   gactggagta aaccatgtat tcccttatct tttacttttt ttctgtgaca tttatgtctc    240
   atgtaatttg cattactctg gtggattgtt ctagtactgt attgggcttc ttcgttaat     299
<210> 23
   <211> 280
   <212> DNA
   <213> human
<400> 23
   accagctcta ggtccaattg ttctgctcta actgatacct caaccttggg gccagcatct     60
   cccactgcct ccaaatatta gtaactatga ctgacgtccc cagaagtttc tgggtctacc    120
   acactcccca accccccact cctacttcct gaagggccct cccaaggcta catccccacc    180
   ccacagttct ccctgagaga gatcaacctc cctgagatca accaaggcag atgtgacagc    240
   aagggccacg gaccccatgg caggggtggc gtcttcatga                          280
<210> 24
   <211> 428
   <212> DNA
   <213> human
<220>
   <221> misc_feature
   <222> (110)..(110)
   <223> n is a, c, g, or t
<220>
   <221> misc_feature
   <222> (143)..(143)
   <223> n is a, c, g, or t
<220>
   <221> misc_feature
   <222> (146)..(146)
   <223> n is a, c, g, or t
<220>
   <221> misc_feature
   <222> (166)..(166)
   <223> n is a, c, g, or t
<220>
   <221> misc_feature
   <222> (179)..(179)
   <223> n is a, c, g, or t
<400> 24
   tgtggatgtt tccaagcctg acctcacggc tgccctgcgt gacgtacgtc agcaatatga     60
   aagtgtggct gccaagaacc tgcaggaggc agaagaatgg tacaaatccn agtttgctga    120
   cctctctgag gctgccaacc ggnacnatga cgccctgcgc caggcnaagc aggagtccnc    180
   tgagtaccgg agacaggtgc agtccctcac ctgtgaagtg gatgccctta aaggaaccaa    240
   tgagtccctg gaacgccaga tgcgtgaaat ggaagagaac tttgccgttg aagctgctaa    300
   ctaccaagac actattggcc gcctgcagga tgagattcag aatatgaagg aggaaatggc    360
   tcgtcacctt cgtgaatacc aagacctgct caatgttaag atggcccttg acattgagat    420
   tgccacct                                                             428
<210> 25
   <211> 362
   <212> DNA
   <213> human
<400> 25
   gatccagaaa tacttaacac gtgaatattt tgctaaaaaa gcatatataa ctattttaaa     60
   tatccattta tcttttgtat atctaagact catcctgatt tttactatca cacatgaata    120
   aaggcctttg tatctttctt tctctaatgt tgtatcatac tcttctaaaa cttgagtggc    180
   tgtcttaaaa gatataaggg gaaagataat attgtctgtc tctatattgc ttagtaagta    240
   tttccatagt caatgatggt ttaataggta aaccaaaccc tataaacctg acctccttta    300
   tggttaatac tattaagcaa gaatgcagta cagaattgga tacagtacgg atttgtccaa    360
   at                                                                   362
<210> 26
   <211> 456
   <212> DNA
   <213> human
<400> 26
   ctccagcagt tctctcgaat actttgaatg ttgtgtaaca gttagccact gctggtgttt     60
   atgtgaacat tcctatcaat ccaaattccc tctggagttt catgttatgc ctgttgcagg    120
   caaatgtaaa gtctagaaaa taatgcaaat gtcacggcta ctctatatac ttttgcttgg    180
   ttcatttttt ttccctttta gttaagcatg actttagatg ggaagcctgt gtatcgtgga    240
   gaaacaagag accaactttt tcattccctg cccccaattt cccagactag atttcaagct    300
   aattttcttt ttctgaagcc tctaacaaat gatctagttc agaaggaagc aaaatccctt    360
   aatctatgtg caccgttggg accaatgcct taattaaaga atttaaaaaa gttgtaatag    420
   agaatatttt tggcattcct ctcaatgttg tgtgtt                              456
<210> 27
   <211> 421
   <212> DNA
   <213> human
<400> 27
   caagtgcagc gatggggagc gggcccgggc ggagctcaat gacaaagtcc acaagctgca     60
   gaatgaagtt gagagcgtca cagggatgct taacgaggcc gaggggaagg ccattaagct    120
   ggccaaggac gtggcgtccc tcagttccca gctccaggac acccaggagc tgcttcaaga    180
   agaaacccgg cagaagctca acgtgtctac gaagctgcgc cagctggagg aggagcggaa    240
   cagcctgcaa gaccagctgg acgaggagat ggaggccaag cagaacctgg agcgccacat    300
   ctccactctc aacatccagc tctccgactc gaagaagaag ctgcaggact ttgccagcac    360
   cgtggaagct ctggaagagg ggaagaagag gttccagaag gagatcgaga acctcaccca    420
   g                                                                    421
<210> 28
   <211> 563
   <212> DNA
   <213> human
<220>
   <221> misc_feature
   <222> (297)..(298)
   <223> n is a, c, g, or t
<400> 28
   tcaagtccaa gttcaagtcc accatcgcgg cgctggaggc caagattgca cagctggagg     60
   agcaggtcga gcaggaggcc agagagaaac aggcggccac caagtcgctg aagcagaaag    120
   acaagaagct gaaggaaatc ttgctgcagg tggaggacga gcgcaagatg gccgagcagt    180
   acaaggagca ggcagagaaa ggcaatgcca gggtcaagca gctcaagagg cagctggagg    240
   aggcagagga ggagtcccag cgcatcaacg ccaaccgcag gaagctgcag cgggagnngg    300
   atgaggccac ggagagcaac gaggccatgg gccgcgaggt gaacgcactc aagagcaagc    360
   tcagagggcc ccccccacag gaaacttcgc agtgatgcac caggcgagga aacgagacct    420
   ctttcgttcc ttctagaagg tctggaggac gtagagttat tgaaaatgca gatggttctg    480
   aggaggaaac ggacactcga gacgcagact tcaatggaac caaggccagt gaataagcaa    540
   ctttctacag ttttgcacca cgg                                            563
<210> 29
   <211> 539
   <212> DNA
   <213> human
<400> 29
   tgagccagga agaacgctcc agcccaggac ttcgaggctg caatgagcta taattgcatc     60
   attgcactcc agcctgggca acagagaccc tgtctcaacc accaccacca ccaccacccc    120
   tactacccct gtattcaagg taaaaattga agtttgtatg atgtaagaga tgagaaaaac    180
   ccaacaggaa acacagacac atcctccagt tctatcaatg gattgtgcag acactgagtt    240
   tttagaaaaa catatccacg gtaaccggtc cctggcaatt ctgtttacat gaaatgggga    300
   gaaagtcacc gaaatgggtg ccgccggccc ccactcccaa ttcattccct aacctgcaaa    360
   cctttccaac ttctcacgtc aggcctttga gaattctttc cccctctcct ggtttccaca    420
   cctcagacac gcacagttca ccaagtgcct tctgtagtca catgaattga aaaggagacg    480
   ctgctcccac ggaggggagc aggaatgctg cactgtttac accctgactg tgcttaaaa     539
<210> 30
   <211> 202
   <212> DNA
   <213> human
<400> 30
   tgaagtgcaa caaggccatc acatctggag gaatcactta ccaggatcag ccctggcatg     60
   ccgattgctt tgtgtgtgtt acctgctcta agaagctggc tgggcagcgt ttcaccgctg    120
   tggaggacca gtattactgc gtggattgct acaagaactt tgtggccaag aagtgtgctg    180
   gatgcaagaa ccccatcact gg                                              202
<210> 31
   <211> 357
   <212> DNA
   <213> human
<400> 31
   cagggctgtc atcaacatgg atatgacatt tcacaacagt gactagttga atcccttgta     60
   acgtagtagt tgtctgctct ttgtccatgt gttaatgagg actgcaaagt cccttctgtt    120
   gtgattccca ggacttttcc tcaagaggaa atctggattt ccacctaccg cttacctgaa    180
   atgcaggatc acctacttac tgtattctac attattatat gacatagtat aatgagacaa    240
   tatcaaaagt aaacatgtaa tgacaataca tactaacatt cttgtaggag tggttagaga    300
   agctgatgcc tcatttctac attctgtcat tagctattat catctaacgt ttcagtg       357
<210> 32
   <211> 445
   <212> DNA
   <213> human
<400> 32
   cttgagacca ggagacgtat ccagcaagcg gaacctctgg gaaaagcaat ctgtggataa     60
   ggtcacttcc cccactaagg tttgagacag ttccagaaag aacccaagct caagacgcag    120
   gacgagctca gttgtagagg gctaattcgc tctgttttgt atttatgttg atttactaaa    180
   ttgggttcat tatcttttat ttttcaatat cccagtaaac ccatgtatat tatcactata    240
   tttaataatc acagtctaga gatgttcatg gtaaaagtac tgcctttgca caggagcctg    300
   tttctaaaga aacccatgct gtgaaataga gacttttcta ctgatcatca taactctgta    360
   tctgagcagt gataccaacc acatctgaag tcaacagaag atccaagttt aaaattgcct    420
   gcggaatgtg tgcagtatct agaaa                                          445
<210> 33
   <211> 496
   <212> DNA
   <213> human
<400> 33
   tgttgtttct gcactttata ataaagcatg gaagaaatta tcttagtagg caattgtaac     60
   actttttgaa agtaacccat ttcagatttg aaatactgca ataatggttg tctttaaaaa    120
   aaaaaagaat gtactgttaa ggtattactt tttttcatgc tgatgattca tatctaaatt    180
   acattattat gttagctgac agtggtactg attttttagg ttggttgttt tgtggatttc   240
   tttagtagtg atagtagcct gaaccacatt ttagataact caattatgta tgtatgtgca    300
   tacacatata caaacacact aatggtagaa tgctttttta tgtgctagac tattatattt    360
   agtagtatgt cattgtaact agccaatatc acagcttttg aaaaattaaa aaatcatcat    420
   cactataata tttcatattt gccaacagaa acatggcaga taggtatcaa tatgttttca    480
   atgcctgatg acctat                                                   496
<210> 34
   <211> 535
   <212> DNA
   <213> human
<400> 34
   ttttaccaaa gcatcaatac aaccagccca accatcggtc cacacctggg catttggtga     60
   gaatcaaagc tgaccatgga tccctggggc caacggcaac agcatgggcc tcacctcctc    120
   tgtgatttct ttctttgcac caaagacatc agtctccaac atgtttctgt tttgttgttt    180
   gattcagcaa aaatctccca gtgacaacat cgcaatagtt ttttacttct cttaggtggc    240
   tctgggatgg gagaggggta ggatgtacag gggtagtttg ttttagaacc agccgtattt    300
   tacatgaagc tgtataatta attgtcatta tttttgttag caaagattaa atgtgtcatt    360
   ggaagccatc ccttttttta catttcatac aacagaaacc agaaaagcaa tactgtttcc    420
   attttaagga tatgattaat attattaata taataatgat gatgatgatg atgaaaacta    480
   aggatttttc aagagatctt tctttccaaa acatttctgg acagtacctg attgt         535
<210> 35
   <211> 436
   <212> DNA
   <213> human
<400> 35
   gagtggacta ttaaatgtgc ctaaatgaat tttgcagtaa ctggtattct tgggttttcc     60
   tacttaatac acagtaattc agaacttgta ttctattatg agtttagcag tcttttggag    120
   tgaccagcaa ctttgatgtt tgcactaaga ttttatttgg aatgcaagag aggttgaaag    180
   aggattcagt agtacacata caactaattt atttgaacta tatgttgaag acatctacca    240
   gtttctccaa atgccttttt taaaactcat cacagaagat tggtgaaaat gctgagtatg    300
   acacttttct tcttgcatgc atgtcagcta cataaacagt tttgtacaat gaaaattact    360
   aatttgtttg acattccatg ttaaactacg gtcatgttca gcttcattgc atgtaatgta    420
   gacctagtcc atcaga                                                   436
<210> 36
   <211> 534
   <212> DNA
   <213> human
<400> 36
   tgctgtgtga accgtcgtgt gagtgtggta tgcctgatca cagatggatt ttgttataag     60
   catcaatgtg acacttgcag gacactacaa cgtgggacat tgtttgtttc ttccatattt    120
   ggaagataaa tttatgtgta gacttttttg taagatacgg ttaataacta aaatttattg   180
   aaatggtctt gcaatgactc gtattcagat gcctaaagaa agcattgctg ctacaaatat    240
   ttctattttt agaaagggtt tttatggacc aatgccccag ttgtcagtca gagccgttgg   300
   tgtttttcat tgtttaaaat gtcacctgta aaatgggcat tatttatgtt tttttttttg    360
   cattcctgat aattgtatgt attgtataaa gaacgtctgt acattgggtt ataacactag    420
   tatatttaaa cttacaggct tatttgtaat gtaaaccacc attttaatgt actgtaatta    480
   acatggttat aatacgtaca atccttccct catcccatca cacaactttt tttg         534
<210> 37
   <211> 551
   <212> DNA
   <213> human
<400> 37
   ccatccagaa tctagcgctg cgcaacacag gaatggagac gcccacaggc gtgtgcgccg     60
   cactggcggc ggcaggtgtg cagccccaca gcctagacct cagccacaac tcgctgcgcg    120
   ccaccgtaaa ccctagcgct ccgagatgca tgtggtccag cgccctgaac tccctcaatc    180
   tgtcgttcgc tgggctggaa caggtgccta aaggactgcc agccaagctc agagtgctcg    240
   atctcagctg caacagactg aacagggcgc cgcagcctga cgagctgccc gaggtggata    300
   acctgacact ggacgggaat cccttcctgg tccctggaac tgccctcccc cacgagggct    360
   caatgaactc cggcgtggtc ccagcctgtg cacgttcgac cctgtcggtg ggggtgtcgg     420
   gaaccctggt gctgctccaa ggggcccggg gctttgccta agatccaaga cagaataatg    480
   aatggactca aactgccttg gcttcagggg agtcccgtca ggacgttgag gacttttcga    540
   ccaattcaac c                                                         551
<210> 38
   <211> 305
   <212> DNA
   <213> human
<400> 38
   caaatgatgt gcaaaacctt ttactggttg catggaaatc agccaagttt tataatcctt     60
   aaatcttaat gttcctcaaa gcttggatta aatacatatg gatgttactc tcttgcacca    120
   aattatcttg atacattcaa atttgtctgg taaaaaaata ggtggtagat attgaggcca    180
   agaatattgc aaaatacatg aagcttcatg cacttaaaga agtattttta gaataagaat    240
   ttgcatactt acctagtgaa acttttctag aattattttt cactctaagt catgtatgtt    300
   tctct                                                                305
<210> 39
   <211> 456
   <212> DNA
   <213> human
<400> 39
   tcagcagtat agggaccttc cgcacaagct ctgtgttaag attgacaata atagtggggc     60
   cattttcatt ttagtctttt ctaagagtca accacaggca tttaagtcag ccaaagaata    120
   ttgttacctt aaagcactat tttatttata gatatatcta gtgcatctac atctctatac    180
   tgtacactca cccataattc aaacaattac accatggtat aaagtgggca tttaatatgt    240
   aaagattcaa agtttgtctt tattactata tgtaaattag acattaatcc actaaactgg    300
   tcttcttcaa gagagctaag tatacactat ctggtgaaac ttggattctt tcctataaaa    360
   gtgggaccaa gcaatgatga tcttctgtgg tgcttaagga aacttactag agctccacta    420
   acagtctcat aaggaggcag ccatcataac cattga                              456
<210> 40
   <211> 476
   <212> DNA
   <213> human
<400> 40
   gtgccaatcc tttgaatgtt ccacggaaac actggtggac agattctagt gctgagaaga      60
   aacacgtttg gtttggagag tccatggatg gtggttttca gtttagctac ggcaatcctg     120
   aacttcctga agatgtcctt gatgtgcagc tggcattcct tcgacttctc tccagccgag     180
   cttcccagaa catcacatat cactgcaaaa atagcattgc atacatggat caggccagtg     240
   gaaatgtaaa gaaggccctg aagctgatgg ggtcaaatga aggtgaattc aaggctgaag     300
   gaaatagcaa attcacctac acagttctgg aggatggttg cacgaaacac actggggaat     360
   ggagcaaaac agtctttgaa tatcgaacac gcaaggctgt gagactacct attgtagata     420
   ttgcacccta tgacattggt ggtcctgatc aagaatttgg tgtggacgtt ggccct          476
<210> 41
   <211> 376
   <212> DNA
   <213> human
<400> 41
   cctggttctg gaatcctcag ttcaaggtta tcctacgcag agagccaggt accaatgggt      60
   gcgctgcaat ccagacagta attctgcaaa ctgccttgaa gaaaaaggac caatgttcga     120
   actacttcca ggtgaatcca acaagatccc ccgtctgagg actgaccttt ttccaaagac     180
   gagaatccag gacttgaatc gtatcttccc actttctgag gactactctg gatcaggctt     240
   cggctccggc tccggctctg gatcaggatc tgggagtggc ttcctaacgg aaatggaaca     300
   ggattaccaa ctagtagacg aaagtgatgc tttccatgac aaccttaggt ctcttgacag     360
   gaatctgccc tcagac                                                     376
<210> 42
   <211> 482
   <212> DNA
   <213> human
<400> 42
   tatgctttaa tgctgttatc tatcttattg ttcttgaaaa tacctgcatt ttttggtatc      60
   atgttcaacc aacatcatta tgaaattaat tagattccca tggccataaa atggctttaa     120
   agaatatata tatattttta aagtagcttg agaagcaaat tggcaggtaa tatttcatac     180
   ctaaattaag actctgactt ggattgtgaa ttataatgat atgccccttt tcttataaaa     240
   acaaaaaaaa aataatgaaa cacagtgaat ttgtagagtg ggggtatttg acatatttta     300
   cagggtggag tgtactatat actattacct ttgaatgtgt ttgcagagct agtggatgtg     360
   tttgtctaca agtatgattg ctgttacata acaccccaaa ttaactccca aattaaaaca     420
   cagttgtgct gtcaatacct catactgctt tacctttttt tcctggatat ctgtgtattt     480
   tc                                                                    482
<210> 43
   <211> 570
   <212> DNA
   <213> human
<400> 43
   gttgagtcgt catcactttt cagtgatggg agagtagatg gtgaaattta ttagttaata      60
   tatcccagaa attagaaacc ttaatatgtg gacgtaatct ccacagtcaa agaaggatgg     120
   cacctaaacc accagtgccc aaagtctgtg tgatgaactt tctcttcata ctttttttca    180
   cagttggctg gatgaaattt tctagacttt ctgttggtgt atcccccccc tgtatagtta     240
   ggatagcatt tttgatttat gcatggaaac ctgaaaaaaa gtttacaagt gtatatcaga     300
   aaagggaagt tgtgcctttt atagctatta ctgtctggtt ttaacaattt cctttatatt     360
   tagtgaacta cgcttgctca tttttcttta cataattttt tattcaagtt attgtacagc     420
   tgtttaagat gggcagctag ttcgtagctt tcccaaataa actctaaaca ttaatcaatc     480
   atctgtgtga aaatgggttg gtgcttctaa cctgatggca cttagctatc agaagaccac     540
   aaaaattgac tcaaatctcc agtattcttg                                      570
<210> 44
   <211> 508
   <212> DNA
   <213> human
<400> 44
   tctcctacat ccgcattgct gataccaata tcaccagcat tcctcaaggt cttcctcctt     60
   cccttacgga attacatctt gatggcaaca aaatcagcag agttgatgca gctagcctga    120
   aaggactgaa taatttggct aagttgggat tgagtttcaa cagcatctct gctgttgaca    180
   atggctctct ggccaacacg cctcatctga gggagcttca cttggacaac aacaagctta    240
   ccagagtacc tggtgggctg gcagagcata agtacatcca ggttgtctac cttcataaca    300
   acaatatctc tgtagttgga tcaagtgact tctgcccacc tggacacaac accaaaaagg    360
   cttcttattc gggtgtgagt cttttcagca acccggtcca gtactgggag atacagccat    420
   ccaccttcag atgtgtctac gtgcgctctg ccattcaact cggaaactat aagtaattct    480
   caagaaagcc ctcattttta taacctgg                                      508
<210> 45
   <211> 499
   <212> DNA
   <213> human
<400> 45
   gtatcaggct tcaattccat tatgttttaa tgttgtctct gaagatgact tgtgattttt     60
   ttttcttttt tttaaaccat gaagagccgt ttgacagagc atgctctgcg ttgttggttt    120
   caccagcttc tgccctcaca tgcacaggga tttaacaaca aaaatataac tacaacttcc    180
   cttgtagtct cttatataag tagagtcctt ggtactctgc cctcctgtca gtagtggcag    240
   gatctattgg catattcggg agcttcttag agggatgagg ttctttgaac acagtgaaaa    300
   tttaaattag taactttttt gcaagcagtt tattgactgt tattgctaag aagaagtaag    360
   aaagaaaaag cctgttggca atcttggtta tttctttaag atttctggca gtgtgggatg    420
   gatgaatgaa gtggaatgtg aactttgggc aagttaaatg ggacagcctt ccatgttcat    480
   ttgtctacct cttaactga                                                499
<210> 46
   <211> 453
   <212> DNA
   <213> human
<400> 46
   gttgtgccta ccactggctg gcacaccagg gcaatgattt ccctgcagaa ggaaggaaag     60
   aatgtttcac ccttgcatcc ttcttggaga agctacagcc tgtgctcagt tgagtggttc    120
   acactcagac tttggcttta tggttttcct tcctccttgt ctttgccctg accttgatca    180
   acaggggtga aaagaaccac cctgaggttt ccatgcctct cccattttag tggtagcatt    240
   ttgtgtcttt actccaccct tcaccctagt tccaccaagg ttcacacacc agatgtaact    300
   gtttttcagc tgagttgtat ggattaactt cagtccactg taaatacacc tgggatgggg    360
   tggggttggg gttgtttagg gagaagcagc cagacttgct ttgtgaactg aatgtatttt    420
   tatgcaattt tgagtggcct ttcaacccta aga                                453
<210> 47
   <211> 594
   <212> DNA
   <213> human
<220>
   <221> misc_feature
   <222> (42)..(42)
   <223> n is a, c, g, or t
<400> 47
   tatcagtagc tcgtgttatc tttttatcaa ctgcttccca gngtcctaaa acaatagaaa     60
   ttttggattg aaaagttcag cataaggagt ttgagtcagt aaaggatggg ataaaggagt    120
   cgagatgatt caatgaaaag tatcacaaaa aagagattga tcaacaagag aaataaaaaa    180
   gcccaagagg aagtggtagg ggaaggaatt taagaacagc aataagtaaa actcttaagt    240
   aactccaaaa agaaaatggt acattttgcc aaagaccact tatacttgag aacatggaag    300
   aatttgcctg atactctctt tggggaaaag agtctctcct cttttcctca aaccccagta    360
   cactcagcct ctctgcccca ccttctcctg actttgtcct cacttgcttc tgcagtacat    420
   tggaacctga attgaaagaa agtcttcctt gaataattgg agtttgtctt gagaggcaaa    480
   tatagcccca agaatcacaa gattcgagga ccatgtaggt cttttacgta gcccaaatcc    540
   ataaattagt ctcacttttt gtatttatcg tttcatatta aaccctctat atca          594
<210> 48
   <211> 335
   <212> DNA
   <213> human
<400> 48
   taacacttgg ctcttggtac ctgtgggtta gcatcaagtt ctccccaggg tagaattcaa     60
   tcagagctcc agtttgcatt tggatgtgta aattacagta atcccatttc ccaaacctaa    120
   aatctgtttt tctcatcaga ctctgagtaa ctggttgctg tgtcataact tcatagatgc    180
   aggaggctca ggtgatctgt ttgaggagag caccctaggc agcctgcagg gaataacata    240
   ctggccgttc tgacctgttg ccagcagata cacaggacat ggatgaaatt cccgtttcct    300
   ctagtttctt cctgtagtac tcctctttta gatcc                               335
<210> 49
   <211> 464
   <212> DNA
   <213> human
<400> 49
   cagtcactct aaatggacac cacatgaacc tctgtttaga atacctacgt atgtatgcat     60
   tggtttgctt gtttcttgac agtacatttt tagatctggc cttttcttaa caaaatctgt    120
   gcaaaagatg caggtggatg tccctaggtc tgttttcaaa gaactttttc caagtgcttg    180
   ttttatttat taagtgtcta cctggtaaat gttttttttg taaactctga gtggactgta    240
   tcatttgcta ttctaaacca ttttacactt aagttaaaat agtttctctt cagctgtaaa    300
   taacaggata cagaattaac aagagaaaat gtctaacttt ttaagaaaaa ccttattttc    360
   ttcggttttt gaaaaacata atggaaataa aacaggatat tgacataata gcacaaaatg    420
   acactcttct aaaactaaat gggcacaaga gaattttcct ggga                     464
<210> 50
   <211> 499
   <212> DNA
   <213> human
<400> 50
   gtcaaaacct aattgcaatt ctgttaaatc taagtaattt ttagacagtg tttcaccgta     60
   ttatttagga tgtgaaatgc catttctttc actgattaca ccatatacag gaaacaggta    120
   aaacagtgaa aactttattg tgctggttga tgccaacttg gttgaaaagc tctctgcaga    180
   agaagtgatc tagactgaca gaagtgttgc taattacaag ttgtgttctc atgacgtaat    240
   tagaaagtaa cttctcaaag tacaactttt atgaaaaaaa taagctgtta aaaaaaggaa    300
   atcgtaggtt aatttaattg ggaaaatggg caattgacag agaccatttt cctaacacat    360
   atatgtgcta gtactttaac tttttaaaat tttacttcta cgttttgtaa tataaaaatt    420
   tctattttaa gtttagaatg ttatacgtac cgaaagtatg cagccaaatc gatcagatca    480
   aaccatttta cctggagtt                                                 499
<210> 51
   <211> 174
   <212> DNA
   <213> human
<400> 51
   agctgtgagc cttggctgtt ggcagtgagt gagcctggct cttgtgctgg atggagccca     60
   ggcgggagcg gtggccctgt ccctcccacc tctgtgacct gaggcctacg ctttggctct    120
   ggagatagcc ccagagcagg gtgttgggat actgcagggc caggactgag cccc          174
<210> 52
   <211> 531
   <212> DNA
   <213> human
<400> 52
   ctgttagctc ctcactgtgg taaatgccac acacctttaa gtagataagc agacgatagt     60
   tatctgttct tttgacttaa tctcatttgg tttgattttc cctctactaa ggctttccta    120
   ccttcttcag gctgcctaag acatgtaagc gaaacacttc aataattgtc catgaggaga    180
   aaaaaagcat tgtcatgcat gaaggaaact gaacttgagg tggcctcctt gcttgttaca    240
   tacctgggta tgtgtaggca gtttagtgca tctttgcctc tcagttgaaa cctgtataac    300
   cctgttacaa agctgtgttg ttgcttcttg tgaaggccat gatattttgt tttttcccca    360
   attaattgct attgtgttat tttactaact tctctctgta ttttttcttg cattgacatt    420
   atagacattg aggacctcat ccaaacaatt taaaaatgag tgtgaagggg gaacaagtca    480
   aaatattttt aaaagatctt caaaaataat gcctctgtct agcatgccaa c             531
<210> 53
   <211> 467
   <212> DNA
   <213> human
<400> 53
   tgtcagagtt ttcaacggtg cttatagctg ccagctggat tccaaacagg taccccattg     60
   tctctgagct aatgtttata tttttccatt caggcaccga aatagttaat atttaaaata    120
   agtcttcaaa agaaaacata agagattatt gagttcttgg gactggatcc tttatttcat    180
   aagttcagat catcttaaat gaaaatgcca tgattatctg cagttaagta gatgacagct    240
   attctacatc agacttgatt tttgtcagct aattacataa ttggtaagct ataattgaaa    300
   ccttatggct taaaattcct taactccttt ttgattcatg tttgtagtca tgttgtcaac    360
   agaggcaaag ttaagcttga tgatggttaa aatcggtttg atagcaccat gggacatttt    420
   tctaacaaaa ataaatgcat gaagagacat agccttttag ttttgct                  467
<210> 54
   <211> 445
   <212> DNA
   <213> human
<400> 54
   aagagctatg agctgccaga tgggcaggtt atcaccattg gcaatgagcg cttccgctgc     60
   cctgagaccc tcttccagcc ttcctttatt ggcatggagt ccgctggaat tcatgagaca    120
   acctacaatt ccatcatgaa gtgtgacatt gacatccgta aggacttata tgccaacaat    180
   gtcctctctg ggggcaccac catgtaccct ggcattgctg acaggatgca gaaggagatc    240
   acagccctgg cccccagcac catgaagatc aagattattg ctcccccaga gcggaagtac    300
   tcagtctgga tcgggggctc tatcctggcc tctctctcca ccttccagca gatgtggatc    360
   agcaagcctg agtatgatga ggcagggccc tccattgtcc acaggaagtg cttctaaagt    420
   cagaacaggt tctccaagga tcccc                                          445
<210> 55
   <211> 525
   <212> DNA
   <213> human
<400> 55
   caactgccct tgaccggaag catgagtatc atcttcttcc tgcccctgaa agtgacccag     60
   aatttgacct tgatagagga gagcctcacc tccgagttca ttcatgacat agaccgagaa    120
   ctgaagaccg tgcaggcggt cctcactgtc cccaagctga agctgagtta cgaaggcgaa    180
   gtcaccaagt ccctgcagga gatgaagctg caatccttgt ttgattcacc agactttagc    240
   aagatcacag gcaaacccat caagctgact caggtggaac accgggctgg ctttgagtgg    300
   aacgaggatg gggcgggaac cacccccagc ccagggctgc agcctgccca cctcaccttc    360
   ccgctggact atcaccttaa ccagcctttc atcttcgtac tgagggacac agacacaggg    420
   gcccttctct tcattggcaa gattctggac cccaggggcc cctaatatcc cagtttaata    480
   ttccaatacc ctagaagaaa acccgaggga cagcagattc cacag                    525
<210> 56
   <211> 444
   <212> DNA
   <213> human
<400> 56
   ctgacctgca ggacgaaacc atgaagagcc tgatccttct tgccatcctg gccgccttag     60
   cggtagtaac tttgtgttat gaatcacatg aaagcatgga atcttatgaa cttaatccct    120
   tcattaacag gagaaatgca aataccttca tatcccctca gcagagatgg agagctaaag    180
   tccaagagag gatccgagaa cgctctaagc ctgtccacga gctcaatagg gaagcctgtg    240
   atgactacag actttgcgaa cgctacgcca tggtttatgg atacaatgct gcctataatc    300
   gctacttcag gaagcgccga ggggccaaat gagactgagg gaagaaaaaa aatctctttt    360
   tttctggagg ctggcacctg attttgtatc cccctgtagc agcattactg aaatacatag    420
   gcttatatac aatgcttctt tcct                                           444
<210> 57
   <211> 444
   <212> DNA
   <213> human
<400> 57
   gaagccctgg aaaatcgcct gagatacaga tgaagattag aaatcgcgac acatttgtag     60
   tcattgtatc acggattaca atgaacgcag tgcagagccc caaagctcag gctattgtta    120
   aatcaataat gttgtgaagt aaaacaatca gtactgagaa acctggtttg ccacagaaca    180
   aagacaagaa gtatacacta acttgtataa atttatctag gaaaaaaatc cttcagaatt    240
   ctaagatgaa tttaccaggt gagaatgaat aagctatgca aggtattttg taatatactg    300
   tggacacaac ttgcttctgc ctcatcctgc cttagtgtgc aatctcattt gactatacga    360
   taaagtttgc acagtcttac ttctgtagaa cactggccat aggaaatgct gtttttttgt    420
   actggacttt accttgatat atgt                                           444
<210> 58
   <211> 450
   <212> DNA
   <213> human
<400> 58
   gctggtatca gaacagcttc cctcactgtg tacagaacgc aagaagggaa taggtggtct     60
   gaacgtggtg tctcactctg aaaagcagga atgtaagatg atgaaagaga caatgtaata    120
   ctgttggtcc aaaagcattt aaaatcaata gatctgggat tatgtggcct taggtagctg    180
   gttgtacatc tttccctaaa tcgatccatg ttaccacata gtagttttag tttaggattc    240
   agtaacagtg aagtgtttac tatgtgcaag ggtattgaag ttcttatgac cacagatcat    300
   cagtactgtt gtctcatgta atgctaaaac tgaaatggtc cgtgtttgca ttgttaaaaa    360
   tgatgtgtga aatagaatga gtgctatggt gttgaaaact gcagtgtccg ttatgagtgc    420
   caaaaatctg tcttgaaggc agctacactt                                     450
<210> 59
   <211> 474
   <212> DNA
   <213> human
<400> 59
   aacctgaaaa catcccagcc aagaactggt ataggagctc caaggacaag aaacacgtct    60
   ggctaggaga aactatcaat gctggcagcc agtttgaata taatgtagaa ggagtgactt   120
   ccaaggaaat ggctacccaa cttgccttca tgcgcctgct ggccaactat gcctctcaga   180
   acatcaccta ccactgcaag aacagcattg catacatgga tgaggagact ggcaacctga   240
   aaaaggctgt cattctacag ggctctaatg atgttgaact tgttgctgag ggcaacagca   300
   ggttcactta cactgttctt gtagatggct gctctaaaaa gacaaatgaa tggggaaaga   360
   caatcattga atacaaaaca aataagccat cacgcctgcc cttccttgat attgcacctt   420
   tggacatcgg tggtgctgac caggaattct ttgtggacat tggcccagtc tgtt         474
<210> 60
   <211> 421
   <212> DNA
   <213> human
<400> 60
   gagccattgg aagactgtcc tctatggcaa tgatctcagg gctcagtggc aggaaatcct    60
   caacagggtc accaaccagc ccgctcaatg cagaaaaact agaatctgaa gatgtgtccc   120
   aagctttcct tgaggctgtt gctgaggaaa agcctcatgt aaaaccctat ttctctaaga   180
   ccattcgcga tttagaagtt gtggagggaa gtgctgctag atttgactgc aagattgaag   240
   gatacccaga ccccgaggtt gtctggttca aagatgacca gtcaatcagg gagtcccgcc   300
   acttccagat agactacgat gaggacggga actgctcttt aattattagt gatgtttgcg   360
   gggatgacga tgccaagtac acctgcaagg ctgtcaacag tcttggagaa gccacctgca   420
   c                                                                   421
<210> 61
   <211> 502
   <212> DNA
   <213> human
<400> 61
   ttatcaagtg tcaagatcag caagtgtcct taagtcaaat aggttttttt ttgttggtgg    60
   ttgtgcttgc tttccttttt tagaaagttc tagaaaatag gaaaacgaaa aatttcattg   120
   agatgagtag tgcatttaat tattttttaa aaactttttt aagtacttga attttatatc    180
   aggaaaacaa agttgttgag ccttgcttct tccgttttgc cctttgtctc gctccttatt   240
   cttttttggg gggagggtta tttgcttttt tatcttcctg gcataatttc cattttattc  300
   ttctgagtgt ctatgttaac ttccctctat cccgcttata aaaaaattct ccaacaaaaa   360
   tacttgttga cttgatgttt tatcacttct ctaagtaagg ttgaaatatc cttattgtag   420
   ctactgtttt taatgtaaag gttaaacttg aaaagaaatt cttaatcacg gtgccaaaat   480
   tcattttcta acaccatgtg tt                                            502
<210> 62
   <211> 541
   <212> DNA
   <213> human
<220>
   <221> misc_feature
   <222> (163)..(164)
   <223> n is a, c, g, or t
<400> 62
   agattctaac ggtctgttct gtttcaaggc actctagatt ccattggtcc aagattccgg     60
   atcctaagca tctaagttat aagactctca cactcagttg tgactaacta gacaccaaag    120
   ttctaataat ttctaatgtt ggacaccttt aggttctttg ctnnattctg cctctctagg    180
   accatggtta agagtccaag aatccacatt tctaaaatct tatagttcta ggcactgtag    240
   ttctaagact caaatgttct aagtttctaa gattctaaag gtccacaggt ctagactatt    300
   aggtgcaatt tcaaggttct aaccctatac tgtagtattc tttggggtgc ccctctcctt    360
   cttagctatc attgcttcct cctccccaac tgtgggggtg tgcccccttc aagcctgtgc    420
   aatgcattag ggatgcctcc tttccgcagg ggatggacga tctcccacct ttcgggccat    480
   gttgcccccg tgagccaatc cctcaccttc tgagtacaga gtgtggactc tggtgcctcc    540
   a                                                                     541
<210> 63
   <211> 496
   <212> DNA
   <213> human
<400> 63
   ggctctggag gtgggcagca atctgtcaat caccttgtta aagagattga tatgctgctg     60
   aaagaatatt tactctctgg agacatatct gaagctgaac attgccttaa ggaactggaa    120
   gtacctcatt ttcaccatga gcttgtatat gaagctatta taatggtttt agagtcaact    180
   ggagaaagta catttaagat gattttggat ttattaaagt ccctttggaa gtcttctacc    240
   attactgtag accaaatgaa aagaggttat gagagaattt acaatgaaat tccggacatt    300
   aatctggatg tcccacattc atactctgtg ctggagcggt ttgtagaaga atgttttcag    360
   gctggaataa tttggaaaca actcagagat ctttgtcctt caaggggcag aaagcgtttt    420
   gtaagcgaag gagatggagg tcgtcttaaa ccagagagct actgaatata agaactcttg    480
   cagtcttaga tgttat                                                    496
<210> 64
   <211> 507
   <212> DNA
   <213> human
<400> 64
   gtaagattcc tcctaacttt cacagtcgat gacaagattg tctttttatc tgatattttg     60
   aagggtatat tgctttgaag taagtctcaa taaggcaata tattttaggg catctttctt    120
   cttatctctg acagtgttct taaaattatt tgaatatcat aagagccttg gtgtctgtcc    180
   taattccttt ctcactcacc gatgctgaat acccagttga atcaaactgt caacctacca    240
   aaaacgatat tgtggcttat gggtattgct gtctcattct tggtatattc ttgtgttaac    300
   tgcccattgg cctgaaaata ctcattgtaa gcctgaaaaa aaaaatcttt cccactgttt    360
   tttctgcttg ttgtaagaat caaatgaaat aatgtatgtg aaagcacctt gtaaactgta    420
   acctatcaat gtaaaatgtt aaggtgtgtt gttatttcat taattacttc tttgtttaga    480
   atggaatttc ctatgcacta ctgtagc                                        507
<210> 65
   <211> 450
   <212> DNA
   <213> human
<400> 65
   tacacttttt tttgccaact gacttaacaa cattgctgtc aggtggaaat ttcaagcact     60
   tttgcacatt tagttcagtg tttgttgaga atccatggct taacccactt gttttgctat    120
   ttttttcttt gcttttaatt ttccccatct gattttatct ctgcgtttca gtgacctacc    180
   ttaaaacaac acacgagaag agttaaactg ggttcatttt aatgatcaat ttacctgcat    240
   ataaaattta tttttaatca agctgatctt aatgtatata atcattctat ttgctttatt    300
   atcggtgcag gtaggtcatt aacaccactt cttttcatct gtaccacacc ctggtgaaac    360
   ctttgaagac ataaaaaaaa cctgtctgag atgttctttc taccaatcta tatgtctttc    420
   ggttatcaag tgtttctgca tggtaatgtc                                     450
<210> 66
   <211> 549
   <212> DNA
   <213> human
<220>
   <221> misc_feature
   <222> (76).. (76)
   <223> n is a, c, g, or t
<400> 66
   aaactactaa ccactgcaag ctcttgtcaa attttagttt aattggcatt gcttgttttt     60
   tgaaactgaa attacntgag tttcattttt tctttgaatt tatagggttt agatttctga    120
   aagcagcatg aatatatcac ctaacatcct gacaataaat tccatccgtt gttttttttg    180
   tttgtttgtt ttttcttttc ctttaagtaa gctctttatt catcttatgg tgcagcaatt    240
   ttaaaatttg aaatatttta aattgttttt gaactttttg tgtaaaatat atcagatctc   300
   aacattgttg gtttcttttg tttttcattt tgtacaactt tcttgaattt agaaattaca    360
   tctttgcagt tctgttaggt gctctgtaat taacctgact tatatgtgaa caattttcat    420
   gagacagtca tttttaacta atgcagtgat tctttctcac tactatctgt attgtggaat    480
   gcacaaaatt gtgtaggtgc tgaatgctgt aaggagttta ggttgtatga attctacaac    540
   cctataata                                                            549
<210> 67
   <211> 489
   <212> DNA
   <213> human
<400> 67
   gagatactat ttttgctgag caaccagtgt gtttcaggat gatacaaaga aaaatataga     60
   atagaaataa gtgcaggctt ggaatcagct acaaatccta aagatggggt gtgtgtggat    120
   gtgtgtgtgt gtgtgtgtac accattgtgt gtttgtaaaa tgtgtatgtt catgagtaag    180
   ggtgtgtgtg tgtgtgtatt aaaattccag agtgaccgtg gcacttgggt gtacaggtaa    240
   ttcctccaga gctgtttgct ggcttcagga gtggagtgag aatttctttt ttatgaaaag    300
   ggatataaag gcaccgagct gatgcagtat ttgtaatatt aagttgacct aacaaggtat    360
   ttgcatgagt cacaattaca aagttttgag cggttttgta atttgacatt taggaaagtc    420
   tcctatttat tctcatactt tacattcatg cttagtatac tatagaggat gccagcttta    480
   atctttctg                                                            489
<210> 68
   <211> 543
   <212> DNA
   <213> human
<400> 68
   gctttcaatt gatggactac tctatttttt gcaaatttgt aaactttgct tctccaaata      60
   caagtactag gttgtccatt tatggtacct atttggtgct agtaaatttt caaactagat     120
   ttataaatgc actgtaatat gtacacaact tagaaaccaa attacaagta ttcagttcca     180
   atacttcatt aatttcaatc aaccaaagtt agttcagtag cttatctcag ttatgagtat     240
   aatacattac atgtaaatta agtgtgtgta tactgtaatc gtgctatttt ttatcattga     300
   aacatttata aactagaata ataatgccct taatgtgagg gtttgtaatg gtgcttatta     360
   agaccaaaga cttgttaaat gtatacacca agtggtaatg aaatttcgtg actggcccac     420
   acgtgcatag aggtctggga ggaccaggaa acagcctcag tggccagagg atcaccagtg     480
   catccttcat cacagcatgt gcaatatgcc aagattaccc tcggtcattc ctgtcaacaa     540
   ggg                                                                   543
<210> 69
   <211> 500
   <212> DNA
   <213> human
<400> 69
   gcgtgagtgt gtgagcgctt ctgcagcctc ggcctaggtc acgttggccc tcaaagcgag      60
   ccgttgaatt ggaaactgct tctagaaact ctggctcagc ctgtctcggg ctgacccttt     120
   tctgatcgtc tcggcccctc tgattgttcc cgatggtctc tctccctctg tcttttctcc     180
   tccgcctgtg tccatctgac cgttttcact tgtctccttt ctgactgtcc ctgccaatgc     240
   tccagctgtc gtctgactct gggttcgttg gggacatgag attttatttt ttgtgagtga     300
   gactgaggga tcgtagattt ttacaatctg tatctttgac aattctgggt gcgagtgtga     360
   gagtgtgagc agggcttgct cctgccaacc acaattcaat gaatccccga cccccctacc     420
   ccatgctgta cttgtggttc tctttttgta ttttgcatct gaccccgggg ggctgggaca     480
   gattggcaat gggccgtccc                                                 500
<210> 70
   <211> 482
   <212> DNA
   <213> human
<400> 70
   agaaagaggc gctgctcact gttttcctgc ttcagttttt ctcttatagt accatcacta      60
   taatcaacga acttctcttc tccacccaga gatggctttt ccaacacatt ttaattaaag     120
   gaactgagta cattaccctg atgtctaaat ggaccaaaga tctgagatcc attgtgatta     180
   tatctgtatc aggtcagcag aagaaggaac tgagcagttg aactctgagt tcatcaattc     240
   taatatttgg aaattatcta caatggaatc ttccctctgt tctctgataa cctacttgct     300
   tactcaatgc ctttaagcca agtcaccctg ttgcctatgg gaggaggtgg aaggatttgg     360
   caagctcaac cacatgctat ttagttagca tcagttgtca ccaacagtct ttctgcaaag     420
   ggcaggagag ctttggggga aaggaaaagg cttaccaggc tgctatggtc aactcttcag     480
   aa                                                                    482
<210> 71
   <211> 508
   <212> DNA
   <213> human
<400> 71
   agccctggag aagacgaaag ccaacatcaa gtgggtgaag gagaacaagg aggtggtgct      60
   ccagtggttc acagaaaaca gcaaatagtc cccagccctt gaagtcaccc ggccccgatg     120
   caaggtgccc acatgtgtcc atcccagcgg ctggtgcagg gcctccattc ctggagcccg 180
   aggcaccagt gtcctcccct caaggacaaa gtctccagcc cacgttctct ctgcctgtga 240
   gccagtctag ttcctgatga cccaggctgc ctgagcacct cccagcccct gcccctcatg 300
   ccaaccccgc cctaggcctg gcatggcacc tgtcgcccag tgccctgggg ctgatctcag 360
   ggaagcccag ctccagggcc agatgagcag aagctctcga tggacaatga acggccttgc 420
   tgggggccgc cctgtaccct ctttcacctt tccctaaaga ccctaaatct gaggaatcaa 480
   cagggcagca gatctgtata tttttttc 508
<210> 72
   <211> 460
   <212> DNA
   <213> human
<400> 72
   attatctggt cttttcctgt tgtgcaaaaa tgactcattg ctccgaatgt caaaaacaaa 60
   tgcgacaaac aatggcactt catcatttaa agtaatgttg ccaagagaaa aaatttcctg 120
   ggagggaggt ttcccacaag ccaaatctcc taagcctcaa atgctagcac tttttggcag 180
   ttggatagga aatgagacat tctttggcag ccaaaataag agaggccgat ggtgaaactt 240
   tttgagacac cctatggcct tcttgtcaaa accttcactg gagctcaaga aaagcatttc 300
   tgttgtgtta tttgcagtgc agatgatgtc tgtgtaacaa cataatggtt attcaccttt 360
   ttttgatttt gatttttgct gtgttatcaa aaacttgaat actgtgagaa gaagtgaatt 420
   ttcagttgac gaatcagcat cttgttccca tggtgataac 460
<210> 73
   <211> 551
   <212> DNA
   <213> human
<400> 73
   cttcgaccac gtgatcacca acatgaacaa caattatgag ccccgcagtg gcaagttcac 60
   ctgcaaggtg cccggtctct actacttcac ctaccacgcc agctctcgag ggaacctgtg 120
   cgtgaacctc atgcgtggcc gggagcgtgc acagaaggtg gtcaccttct gtgactatgc 180
   ctacaacacc ttccaggtca ccaccggtgg catggtcctc aagctggagc agggggagaa 240
   cgtcttcctg caggccaccg acaagaactc actactgggc atggagggtg ccaacagcat 300
   cttttccggg ttcctgctct ttccagatat ggaggcctga cctgtgggct gcttcacatc 360
   caccccggct ccccctgcca gcaacgctca ctctaccccc aacaccaccc cttgcccagc 420
   caatggacac agtagggctt ggtgaatgct gctgagtgaa tgagtaaata aactcttcaa 480
   ggccaaggaa cagtggtcta attcaactct gtgtcccagc actggcacac cagaagtgcc 540
   atgctcagaa a 551
<210> 74
   <211> 542
   <212> DNA
   <213> human
<400> 74
   tgatgagctt tcctttgatc cggacgacgt aatcactgac attgagatgg tggacgaggg 60
   ctggtggcgg ggacgttgcc atggccactt tggactcttc cctgcaaatt atgtcaagct 120
   tctggagtga ctagagctca ctgtctactg caactgtgat ttcccatgtc caaagtggct 180
   ctgctccacc ccctccctat tcctgatgca aatgtctaac cagatgagtt tctggacaga 240
   cttccctctc ctgcttcatt aagggcttgg ggcagagaca gcatggggaa ggaggtcccc 300
   ttccccaaga gtcctctcta tcctggatga gctcatgaac atttctcttg tgttcctgac 360
   tccttcccaa tgaacacctc tctgccaccc caagctctgc tctcctcctc tgtgagctct 420
   gggcttccca gtttgtttac ccgggaaagt acgtctagat tgtgtggttt gcctcattgt 480
   gctatttgcc cactttcctt ccctgaagaa atatctgtga accttctttc tgttcagtcc 540
   ta 542
<210> 75
   <211> 436
   <212> DNA
   <213> human
<400> 75
   gctggaatac ttactcttgt cgggagattg aaccactaaa atgttagagc agaattcatt     60
   atgctgtggt cacaggggtg tcttgtctga gaacaaatac aattcagtct tctctttggg    120
   gttttagtat gtgtcaaaca taggactgga agtttgcccc tgttcttttt tcttttgaaa    180
   gaacatcagt tcatgcctga ggcatgagtg actgtgcatt tgagatagtt ttccctattc    240
   tgtggataca gtcccagagt tttcagggag tacacaggta gattagtttg aagcattgac    300
   cttttattta ttccttattt ctctttcatc aaaacaaaac agcagctgtg ggaggagaaa    360
   tgagagggct taaatgaaat ttaaaataag ctatattata caaatactat ctctgtattg    420
   ttctgaccct ggtaaa                                                    436
<210> 76
   <211> 379
   <212> DNA
   <213> human
<220>
   <221> misc_feature
   <222> (170)..(170)
   <223> n is a, c, g, or t
<400> 76
   aatgcgaagg ctaagtgtca ccccctttct ctgcctctgg ctgggccttg ctaagggcca     60
   aggaaagaaa gacatttttt agggggcagc cagtccaaat gccaaaagaa gaccagttct    120
   tgccctgatt gtatgaaatt tgacattttg gcactttttt tttttttttn ggccaatcag    180
   attttctatg ttctaaggac atggctgctg tagaatagca cagacgtgga tgataaatta    240
   tccccagaag cagcatgaca gaatgcctcg gggagcactt ggaagggaaa ttgcagttct    300
   gttgaaatag aggaaaatcc cttggtaaag acacagcctg ttaggctcgt gtgggcctcc    360
   agtatgttca ccaggggaa                                                 379
<210> 77
   <211> 451
   <212> DNA
   <213> human
<400> 77
   aacgatgtca catgcgtgtt cgaccccgtg cacaccatct cccacaccgt catctcgctg     60
   cctaccttcc gcgagttcac ccgccctgaa gagatcatct tcctccgggc catcacgcca    120
   ccgcatcctg ccagccaggc taacatcatc ttcgacatca cggaagggaa cctgcgggac    180
   tcttttgaca tcatcaagcg ttacatggac ggcatgaccg tgggtgtcgt gcgccaggtg    240
   cggcccatcg tgggcccatt tcatgccgtc ctgaagctgg agatgaacta tgtggtcggg    300
   ggcgtggttt cccaccgaaa tgttgtcaac gtccgcatct tcgtctctga gtactggttc    360
   tgagggctgg tctgccgcac agccgcaggt gcacctccag gccaaatcat tgctgccagt    420
   gactgtggtc tgtacttgtt tataccctca g                                   451
<210> 78
   <211> 500
   <212> DNA
   <213> human
<220>
   <221> misc_feature
   <222> (410)..(411)
   <223> n is a, c, g, or t
<400> 78
   gacagacctg agaccaatct gggtagaagc aaaaagttga accttttaaa gtgctgaaca     60
   caaatccaaa ttcgaatggt tcaagcagcc gtgaaatcgc tcttcataaa gtgggcttaa    120
   ttctctagtt taagttcttt tgatggaatg aattaattaa tgtgtcaggt ggcttatttg    180
   tggatgccat gattgatgat gttcatttta agctcttacc tatagtacaa gtacatgatg    240
   ctactgaata tttttccact tggaaactgt gagctggttg ttgcattaaa acacacatac    300
   aaacaaaatc aaaaacactg cggactttca ctcaagctgg tctttcttcc ccagtgtaag    360
   gcaatcctgc ctactaacaa caccaacaac aaaacactcc atctgtgaan ntgacgcagt    420
   taagggggct aggcagggca tttgtgccaa ctaagaatca ccagataccc accataagta    480
   cctatcgcag ttttgaagtc                                                500
<210> 79
   <211> 480
   <212> DNA
   <213> human
<400> 79
   acctcgcaac atcaacatct atacttacga tgatatggaa gtgaagcaaa tcaacaaacg     60
   tgcctctggc caggcttttg agctgatctt gaagccacca tctcctatct cagaagcccc    120
   acgaacttta gcttctccaa agaagaaaga cctgtccctg gaggagatcc agaagaaact    180
   ggaggctgca ggggaaagaa gaaagtctca ggaggcccag gtgctgaaac aattggcaga    240
   gaagagggaa cacgagcgag aagtccttca gaaggctttg gaggagaaca acaacttcag    300
   caagatggcg gaggaaaagc tgatcctgaa aatggaacaa attaaggaaa accgtgaggc    360
   taatctagct gctattattg aacgtctgca ggaaaaggag aggcatgctg cggaggtgcg    420
   caggaacaag gaactccagg ttgaactgtc tggctgaagc aagggagggt ctggcacgcc    480
<210> 80
   <211> 510
   <212> DNA
   <213> human
<400> 80
   atggcctctg tgaataatgt aactccagtt acacggtgac ttttaatagc atacagtgat     60
   ttgatgaaag gacgtcaaac aatgtggcga tgtcgtggaa agttatcttt cccgctcttt    120
   gctgtggtca ttgtgtcttg cagaaaggat ggccctgatg cagcagcagc gccagctgta    180
   ataaaaaata attcacacta tcagactagc aaggcactag aactggaaaa gaccacagaa    240
   aacaaagaat ccaacccttt catcttacag gtgaacaaac tgtgatgatg cacatgtatg    300
   tgttttgtaa gctgtgagca ccgtaacaaa atgtaaattt gccattatta ggaaagtgct    360
   ggtggcagtg aagaagcacc caggccactt gactcccagt ctggtgccct gtctacacca    420
   gacaacacag gagctgggtc agattcccct cagctgctta acaaagttcc tcgaacagaa    480
   agtgcttaca aagctgcctt ctcggatact                                     510
<210> 81
   <211> 560
   <212> DNA
   <213> human
<400> 81
   agctgccttc tcggatactg aaaggtcgag ttttctgaac tgcactgatt ttattgcagt     60
   tgaaaaaccc aaagctattc caaagatttc aagctgttct gagacatctt ctgatggctt    120
   tacttcctga gaggcaatgt ttttacttta tgcataattc attgttgcca aggaataaag    180
   tgaagaaaca gcaccttttt aatatatagg tctctctgga agagacctaa atttagaaag    240
   agaaaactgt gacaattttc atattctcat tcttaaaaaa cactaatctt aactaacaaa    300
   agttcttttg agaataagtt acacacaatg gccacagcag tttgtcttta atagtatagt    360
   gcctatactc atgtaatcgg ttactcacta ctgcctttaa aaaaaaccag catatttatt    420
   gaaaacatga gacaggatta tagtgcctta accgatatat tttgtgactt aaaaaataca    480
   tttaaaactg ctcttctgct ctagtaccat gcttagtgca aatgattatt tctatgtaca    540
   actgatgctt gttcttattt                                                560
<210> 82
   <211> 561
   <212> DNA
   <213> human
<400> 82
   ttcagttgct aatgaggctc ctccgctctg gacacaaccc ttttatagat taatttctct     60
   gccaattaac ttgtcatttt cagtacatat tttactattc cacaccaacc ataattacaa    120
   caagggattt ttcttatgca ctcctatgca tgtgaataac atgtggtgta attctgcttc    180
   ttacagaagt attactgaag gtattatttc caatattatt tggtttatta tgcggatctt    240
   ttttatatat gcagtcccat cccttctgtg ccactcaatg ccatccagac atggtttttc    300
   cctccagggg cctttctctc cagagggcac ttcggctgcc tctgcttcct ctcattcgag    360
   gcccggctct tgctgacaga ataggttccg ttctgggcgg tggttctcga gcctgccatt    420
   caaaaccaaa gcaaattgga gcatttctca caacatggta ttgaagttcc tttttgttct    480
   caaaagttgt gaccgtgtta aattgtactc ccttagtcct gtaaggtatg ttaagtgaat    540
   cgcagttacg ctgtactttt a                                              561
<210> 83
   <211> 458
   <212> DNA
   <213> human
<400> 83
   agaaaatttg ccaatctttc ctactttcta tttttatgat gacaatcaaa gccggcctga     60
   gaaacactat ttgtgacttt ttaaacgatt agtgatgtcc ttaaaatgtg gtctgccaat    120
   ctgtacaaaa tggtcctatt tttgtgaaga gggacataag ataaaatgat gttatacatc    180
   aatatgtata tatgtatttc tatatagact tggagaatac tgccaaaaca tttatgacaa    240
   gctgtatcac tgccttcgtt tatatttttt taactgtgat aatccccaca ggcacattaa    300
   ctgttgcact tttgaatgtc caaaatttat attttagaaa taataaaaag aaagatactt    360
   acatgttccc aaaacaatgg tgtggtgaat gtgtgagaaa aactaacttg atagggtcta    420
   ccaatacaaa atgtattacg aatgcccctg ttcatgtt                            458
<210> 84
   <211> 543
   <212> DNA
   <213> human
<400> 84
   ccaggactac agaataccat cccctggtac cgtgtagttg ccgaagtcca gatctgccat     60
   ggcaaaacgg aggctgtggg ccaggtccac atcttcttcc aggatgggat ggtgacgttg    120
   actccaaaca agggtgtgtg ggtgaatggt ctccgagtgg atctcccagc tgagaagtta    180
   gcatctgtgt ccgtgagtcg tacacctgat ggctccctgc tagtccgcca gaaggcaggg    240
   gtccaggtgt ggcttggagc caatgggaag gtggctgtga ttgtcagcaa tgaccatgct    300
   gggaaactgt gtggggcctg tggaaacttt gacggggacc agaccaatga ttggcatgac    360
   tcccaggaga agccagcgat ggagaaatgg agagcgcagg acttctcccc atgttatggc    420
   tgatcagtca tccaccagga acgaagattt cctgaagaag acctggtccc tctggaggtt    480
   gcggtggctg aaggatgcat catgtgctcc taccctgctc taccgctttt ctgggtcaca    540
   gag                                                                  543
<210> 85
   <211> 401
   <212> DNA
   <213> human
<400> 85
   aaagacaagc attgggtcag acccataaac cacctcccaa aggctgtcat ttcattgcac     60
   tggaattttg ctttatcaga agcaaggaag taagggagtc attgccttgg gcctgggaat    120
   ctaagtggga gacaatatta atttggatcc gattaattgg agattactaa ctgtggacaa    180
   aagtttatct ttgcacaatc aataaaaatg gcattttttt agtaaattaa gagcataaac    240
   aatattgcta gaggtggcat gtttagtcta ccaaaaacaa tacttttcag gcactttaga    300
   aatatccttt tagaagcagc gagtgcatgg gctaattatc atcaatcttt atgtatttgt    360
   taaagaaaca tctacaggat ctttattggt gaccttttgt a                        401
<210> 86
   <211> 454
   <212> DNA
   <213> human
<400> 86
   gtccttcaca tcaccatttt gagacctcag cttggcactc aggtgctgaa gggtaatatg     60
   gactcagcct tgcaaatagc cagtgctagt tctgacccaa ccacagagga tgctgacatc    120
   atttgtatta tgttccaagg ctactacaga gaaggctgcc tgctatgtat ttgcaaggct    180
   gatttatggt cagaatttcc ctctgatatg tctagggtgt gatttaggtc agtagactgt    240
   gattcttagc aaaaaatgaa cagtgataag tatactgggg gcaaaatcag aatggaatgc    300
   tctggtctat ataaccacat ttctgagcct ttgagactgt tcctgagcct tcagcactaa    360
   cctatgaggg tgagctggtc ccctctatat atacatcata cttaacttta ctaagtaatc    420
   tcacagcatt tgccaagtct cccaatatcc aatt                                454
<210> 87
   <211> 492
   <212> DNA
   <213> human
<400> 87
   atgtgtcact gattttttag ctcaaaatca tcactgttaa tttccagtca ccccaaatat     60
   ggttaaaaga tttttttttt taatcatgaa gagaaaatta gtagcatttc tttctctccc    120
   cattatttat tggttttcct cactaatctt ttttttttta gtccaaaagc caaaaatatt    180
   tatcttggtt ttacatttta atttccattc ttaattgtaa tttttttctt taaataagga    240
   aaccaatata atctcatgta taaaaactta aatattttac aagttacata tagcatcatt    300
   ctaaaataag aatttttttt gttttctgtc tgcttttttc ttatgtctct tgttgagttt    360
   tatattttca gtggttattt ttgcttgtgt tagatcatta ttaaaatata tccaatgtcc    420
   ctttgatact tgtgctctgc tgagaatgta cagtttgcat taaacatccc aggtctcatc    480
   cttcaggaat tt                                                        492
<210> 88
   <211> 444
   <212> DNA
   <213> human
<400> 88
   gaagcgcctg gcagtgtacc aggccggggc ccgcgagggc gccgagcgcg gcctcagcgc     60
   catccgcgag cgcctggggc ccctggtgga acagggccgc gtgcgggccg ccactgtggg    120
   ctccctggcc ggccagccgc tacaggagcg ggcccaggcc tggggcgagc ggctgcgcgc    180
   gcggatggag gagatgggca gccggacccg cgaccgcctg gacgaggtga aggagcaggt    240
   ggcggaggtg cgcgccaagc tggaggagca ggcccagcag atacgcctgc aggccgaggc    300
   cttccaggcc cgcctcaaga gctggttcga gcccctggtg gaagacatgc agcgccagtg    360
   ggccgggctg gtggagaagg tgcaggctgc cgtgggcacc agcgccgccc ctgtgcccag    420
   cgacaatcac tgaacgccga agcc                                           444
<210> 89
   <211> 500
   <212> DNA
   <213> human
<400> 89
   actgtgatat aggtactctg atttaaaact ttggacatcc tgtgatctgt tttaaagttg     60
   gggggtggga aatttagctg actagggaca aacatgtaaa cctattttcc tatgaaaaaa    120
   gttttaaatg tcccacttga ataacgtaat tcttcatagt ttttttaatc tatggataaa    180
   tggaaaccta attatttgta atgaattatt tagacagttc taagccctgt cttctgggag    240
   ttatcaattt taaagagaac ttttgtgcaa ttcaaatgaa gtttttataa gtaattgaaa    300
   atgacaacac aataacactt tctgtataaa agtatatatt ttatgtgatt tattcctact    360
   aaatgaaagt gcactactgc ctcatgtaaa gactcttgca cgcagagcct ttaagtgact    420
   aaggaacaac atagatagtg agcatagtcc ccacctccac ccctcacaat ttatttgaat    480
   acttcaattg tgcctctcaa                                                500
<210> 90
   <211> 444
   <212> DNA
   <213> human
<400> 90
   ttcagacagt tatatcctcc ttttaaacca ttgttgttga gtgtaagatg tccttcatgt     60
   tttcttataa agtcagtgtt tagaaatgtt accctttcta agttatatac agatcaaatg    120
   cttttttctt tcacgtacat ccatcatttg caactgctgt tcgtacacag aaacaggact    180
   gctcaaatga tcctatttgt attttctgat gctatcagac tctaatgttt ttttccctaa    240
   aatattattg ccatcatgct ttaggaattt ttatattttt acacaatcat attttagtat    300
   ggtgtctgtt tatgtaactc tgacttgctg gaaaagttga aactccaaat aatctgaaac    360
   tagaaaagaa atagcacata attactacct tccccttggc ggctctcctc ccccaacccc    420
   caccccacaa ttttatgact tcca                                           444
<210> 91
   <211> 435
   <212> DNA
   <213> human
<400> 91
   caacgtctaa ctggacttcc caagataaat ggtaccagcg tcctcttaaa agatgcctta     60
   atccattcct tgaggacaga ccttagttga aatgatagca gaatgtgctt ctctctggca    120
   gctggccttc tgcttctgag ttgcacatta atcagattag cctgattctc ttcagtgaat    180
   tttgataatg gcttccagac tctttgcgtt ggagacgcct gttaggatct tcaagtccca    240
   tcatagaaaa ttgaaacaca gagttgttct gctgatagtt ttggggatac gtccatcttt    300
   ttaagggatt gctttcatct aattctggca ggacctcacc aaaagatcca gcctcatacc    360
   tacatcagac aaaatatcgc cgttgttcct tctgtactaa agtattgtgt tttgctttgg    420
   aaacacccac tcact                                                     435
<210> 92
   <211> 486
   <212> DNA
   <213> human
<400> 92
   gccagacgct ggggccatag tgagtgtggg cacaaggaag acgctgcagt gaattgcaca     60
   gatatttcag tgcagaaaac cccacaaaaa gccacaacag gtcgctcatc ccgtcagtca    120
   tcctttattg cagtcgggat ccttggggtt gttctgttgg ccattttcgt cgcattattc    180
   ttcttgacta aaaagcgaag acagagacag cggcttgcag tttcctcaag aggagagaac    240
   ttagtccacc aaattcaata ccgggagatg aattcttgcc tgaatgcaga tgatctggac    300
   ctaatgaatt cctcaggagg ccattctgag ccacactgaa aaggaaaatg ggaatttata    360
   acccagtgag ttcagccttt aagatacctt gatgaagacc tggactattg aatggagcag    420
   aaattcacct ctctcactga ctattacagt tgcattttta tggagttctt cttctcctag    480
   gattcc                                                               486
<210> 93
   <211> 522
   <212> DNA
   <213> human
<400> 93
   ttttcattgt tcattgatat gctcagtatg ctgccacata agatgaattt aattatattc     60
   aaccaaagca atatactctt acatgatttc taggccccat gacccagtgt ctagagacat    120
   taattctaac cagttgtttg cttttaaatg agtgatttca ttttgggaaa caggtttcaa    180
   atgaatatat atacatgggt aaaattactc tgtgctagtg tagtcttact agagaatgtt    240
   tatggtccca cttgtatatg aaaatgtggt tagaatgtta attggataat gtatatataa    300
   gaagttaaag tatgtaaagt ataacttcag ccacattttt agaacactgt ttaacatttt    360
   tgcaaaacct tcttgtagga aaagagagct ctctacatga agatgacttg ttttatattt    420
   cagattttat tttaaaagcc atgtctgtta aacaagaaaa aacacaaaag aactccagat    480
   tcctggttca tcattctgta ttcttactca ctttttcaag tt                       522
<210> 94
   <211> 465
   <212> DNA
   <213> human
<400> 94
   atcctcattc ttatactgct tttcgtggcc actttggaca agtcctggtg gactctccct     60
   gggaaagagt ccctgaatct ctggtacgac tgcacgtgga acaacgacac caaaacatgg    120
   gcctgcagta atgtcagcga gaatggctgg ctgaaggcgg tgcaggtcct catggtgctc    180
   tccctcattc tctgctgtct ctccttcatc ctgttcatgt tccagctcta caccatgcga    240
   cgaggaggtc tcttctatgc caccggcctc tgccagcttt gcaccagcgt ggcggtgttt    300
   actggcgcct tgatctatgc cattcacgcc gaggagatcc tggagaagca cccgcgaggg    360
   ggcagcttcg gatactgctt cgccctggcc tgggtggcct tccccctcgc cctggtcagc    420
   ggcatcatct acatccacct acggaagcgg gagtgagcgc cccgc                    465
<210> 95
   <211> 560
   <212> DNA
   <213> human
<400> 95
   caagtatcgg ggctctgcta tcaaggtgca aagtccttcg tggatgcaac ctcaaccata     60
   tattctacag caccctggtg ccgtgttaac tccctcaatg gagcacacca tgtcactaca    120
   gcccgcatca atgatcagcc ctctggccca gcagatgagt catctgtcac taggcagcac    180
   cggaacatac atgcctgcaa cgtcagctat gcaaggagcc tacttgccac agtatgcaca    240
   tatgcagacg acagcggttc ctgttgagga ggcaagtggt caacagcagg tggctgtcga    300
   gacgtctaat gaccattctc catatacctt tcaacctaat aagtaactgt gagatgtaca    360
   gaaaggtgtt cttacatgaa gaagggtgtg aaggctgaac aatcatggat ttttctgatc     420
   aattgtgctt taggaaatta ttgacagttt tgcacaggtt cttgaaaacg ttatttataa    480
   tgaaatcaac taaaactatt tttgctataa gttctataag gtgcataaaa cccttaaatt    540
   catctagtag ctgttccccc                                                560
<210> 96
   <211> 442
   <212> DNA
   <213> human
<400> 96
   caccttgggt ctgtgtcact tgtaggtttc tctgcctcca ggttgcctca acagcaggag      60
   gcacagcagt ttcaccatct ttgaggtgag ggtggggtgc cccagctagg aagcaagatc     120
   gctgtgctag gtctgaccaa aaccagaggg cagtctagtc ctgggggtaa agccctcaga     180
   tcccagggta cactcttctc cattccctcc acccacttgc ctgtcacccc agtcacctaa     240
   gcaatcactg ggcccagagg agaggagaca gacacacact ggctcctgga cctaaagggt     300
   atgagctgga gctaaggcca gctagagctt ccactgtcag ccctcactgt cagccccact    360
   gcacccccct gtgcctgctg ggcactgggc actagctaga tgctttaggt tgcttcagct     420
   gatccttcaa ctctgtgagg tg                                              442
<210> 97
   <211> 484
   <212> DNA
   <213> human
<400> 97
   tatgtgacgc tggacctttt ctttacccaa ggatttttaa aactcagatt taaaacaagg      60
   ggttacttta catcctacta agaagtttaa gtaagtaagt ttcattctaa aatcagaggt     120
   aaatagagtg cataaataat tttgttttaa tctttttgtt tttcttttag acacattagc     180
   tctggagtga gtctgtcata atatttgaac aaaaattgag agctttattg ctgcatttta     240
   agcataatta atttggacat tatttcgtgt tgtgttcttt ataaccaccg agtattaaac     300
   tgtaaatcat aatgtaactg aagcataaac atcacatggc atgttttgtc attgttttca     360
   ggtactgagt tcttacttga gtatcataat atattgtgtt ttaacaccaa cactgtaaca     420
   tttacgaatt atttttttaa acttcagttt tactgcattt tcacaacata tcagacttca     480
   ccaa                                                                  484
<210> 98
   <211> 509
   <212> DNA
   <213> human
<400> 98
   tgtgtaatac gccaaccagt caagttgtgt tttggccaga gatttagata tgtccaattt      60
   cctggctcat ttcattgtgc tctatgggta cgtataaaaa gcaagaattc tgtttcctag     120
   gcaaacattg caactcaggg ctaaagtcat ccagtgaaac ttttagagcc agaagtaact     180
   ttgtcccagt cctacaatgt gaaaagagtg aatagttgcc tctttttagc cattttcatg     240
   gctggtacat attcgtacgc attacttttc agaatcaata cgcactttca gatattctta     300
   tttttattct cttaagtctt tattaacttt ggagagagaa atgatgcatc tttttatttt     360
   aaatgaagta gatcaacatg gtggaacaaa atgataaaga acagaaaaca tttcaatata     420
   ttactaataa ctttttccaa tataaatcct aaaattccta taacatagta ttttacagtt     480
   ttatgaagct ttctattgtg acttttatg                                       509
<210> 99
   <211> 453
   <212> DNA
   <213> human
<220>
   <221> misc_feature
   <222> (250)..(250)
   <223> n is a, c, g, or t
<400> 99
   aacactcaca tacaagttca tactttaaaa gaggaaagct acttaacaat gacaaatatt 60
   tcacaataat aatttttact tatataccat ctttcaactg aacatttcag ttcttccaag 120
   agcttcttag agtagtatat tttgggggca gtcaaggaat aaactacagt gtaaacatat 180
   cccagatgaa aactgctgta tggaaaaatg acagaaagta actgattgac actgttgatt 240
   cacagttcan gcctcctatc tgggaaagac atttctttcc tctgctcact ttaagaactt 300
   ttaccgactc caaaaatctc aggaattaaa cttttaacag ttacagcaat aaagaatagt 360
   tagtactcca aaaatattat atttaagatg ctcaacaaga aaaaaatgca aatgtaatat 420
   ttttttcaaa ttacttcttt attgacttgt cca 453
<210> 100
   <211> 461
   <212> DNA
   <213> human
<400> 100
   gatttccaaa tcagtcgttt tctcaaaaaa atatcgtata agtgactcat cctgtctgct 60
   aactccagac ctcccagctt gaagccaaat ctttccatgt gagattgata tggatttcct 120
   agaagtactg gaatgttgtc atatcttgcc ctattttaat tctgctatag aaaacaattg 180
   ccttcacttt taaggagtaa tttgaatatt aataactctg gtctagattt tcatataatg 240
   tattaaagac aaagtagtga acatcaatga acatctgata gagataaact gtaatcaggc 300
   ataagcttgt ttgtatgttc tggcagtgac taatcagtaa atgatgtcgg tttgcccagt 360
   atcacttatc ttctgtattt ttcctctgtc gtgtaaatag tataaccttt tcatttatgg 420
   acaatttttt ggactagtag ccttcaatat acattctgct t 461
<210> 101
   <211> 470
   <212> DNA
   <213> human
<400> 101
   acgcacacaa ctactacaat tccgcctagg tccacaaggc cttcactgtt ttccccccaa 60
   gggaggctgc tgctgctctt ggctggagcc agccagggcc agccgacccc ctctccctgc 120
   atggcatcct ccagcccctg tagaactcaa cctctacagg gttagagttt ggagagagca 180
   gactggcggg gggcccattg gggggaaggg gaccctccgc tctgtagtgc tacagggtcc 240
   aacatagaac agggtgtccc caacagcgcc caaaggacgc actgagcaac gctattccag 300
   ctgtcccccc actccctcac aagtgggtac ccccaggacc agaagctccc ccagcaaagc 360
   ccccagagcc caggctcggc ctgcccccac cccattcccg cagtgggagc aaactgcatg 420
   cccagagacc cagcggacac acgcggtttg gtttgcagcg actggcatac 470
<210> 102
   <211> 476
   <212> DNA
   <213> human
<400> 102
   ccaaatttca tttcagccac ttctgcagga tccctactgc caacctggaa tggagacttt 60
   tatctacttc tctctctctg aagatgtcaa atcgtggttt agatcaaata tatttcaagc 120
   tataaaagca ggaggttatc tgtgcagggg gctggcatca tgtatttagg ggcaagtaat 180
   aatggaatgc tactaagata ctccatattc ttccccgaat cacacagaca gtttctgaca 240
   ggcgcaactc ctccattttc ctcccgcagg tgagaaccct gtggagatga gtcagtgcca 300
   tgactgagaa ggaaccgacc cctagttgag agcaccttgc agttccccga gaactttctg 360
   attcacagtc tcattttgac agcatgaaat gtcctcttga agcatagctt tttaaatatc 420
   tttttccttc tactcctccc tctgactcta agaattctct cttctggaat cgcttg 476
<210> 103
   <211> 501
   <212> DNA
   <213> human
<400> 103
   gtgtgatgct tttgtaggta cctggaaact tgtctccagt gaaaactttg atgattatat     60
   gaaagaagta ggagtgggct ttgccaccag gaaagtggct ggcatggcca aacctaacat    120
   gatcatcagt gtgaatgggg atgtgatcac cattaaatct gaaagtacct ttaaaaatac    180
   tgagatttcc ttcatactgg gccaggaatt tgacgaagtc actgcagatg acaggaaagt    240
   caagagcacc ataaccttag atgggggtgt cctggtacat gtgcagaaat gggatggaaa    300
   atcaaccacc ataaagagaa aacgagagga tgataaactg gtggtggaat gcgtcatgaa    360
   aggcgtcact tccacgagag tttatgagag agcataagcc aagggacgtt gacctggact    420
   gaagttcgca ttgaactcta caacattctg tgggatatat tgttcaaaaa gatattgttg    480
   ttttccctga tttagcaagc a                                              501
<210> 104
   <211> 462
   <212> DNA
   <213> human
<400> 104
   ggtccccaca gactcagaga gaacccacca tggtgctgtc tcctgccgac aagaccaacg     60
   tcaaggccgc ctggggtaag gtcggcgcgc acgctggcga gtatggtgcg gaggccctgg    120
   agaggatgtt cctgtccttc cccaccacca agacctactt cccgcacttc gacctgagcc    180
   acggctctgc ccaggttaag ggccacggca agaaggtggc cgacgccctg accaacgccg    240
   tggcgcacgt ggacgacatg cccaacgcgc tgtccgccct gagcgacctg cacgcgcaca    300
   agcttcgggt ggacccggtc aacttcaagc tcctaagcca ctgcctgctg gtgaccctgg    360
   ccgcccacct ccccgccgag ttcacccctg cggtgcacgc ctccctggac aagttcctgg    420
   cttctgtgag caccgtgctg acctccaaat accgttaagc tg                       462
<210> 105
   <211> 520
   <212> DNA
   <213> human
<400> 105
   gcccaggctg acttacacat tgaggatgga gactccatcc gcttcgggcg cttcgcgttg     60
   gagaccaggg ccagccctgg ccacacccca ggctgtgtca ccttcgtcct gaatgaccac    120
   agcatggcct tcactggaga tgccctgttg atccgtgggt gtgggcggac agacttccag    180
   caaggctgtg ccaagacctt gtaccactcg gtccatgaaa agatcttcac acttccagga    240
   gactgtctga tctaccctgc tcacgattac catgggttca cagtgtccac cgtggaggag    300
   gagaggactc tgaaccctcg gctcaccctc agctgtgagg agtttgtcaa aatcatgggc    360
   aacctgaact tgcctaaacc tcagcagata gactttgctg ttccagccaa catgcgctgt    420
   ggggtgcaga cacccactgc ctgatctcac ttctgtcaga tgctcccatc cactattaat    480
   gcactaggtg ggaggagagg gcggcaatga cactgcacct                          520
<210> 106
   <211> 515
   <212> DNA
   <213> human
<220>
   <221> misc_feature
   <222> (89)..(89)
   <223> n is a, c, g, or t
<220>
   <221> misc_feature
   <222> (110)..(110)
   <223> n is a, c, g, or t
<220>
   <221> misc_feature
   <222> (227)..(227)
   <223> n is a, c, g, or t
<220>
   <221> misc_feature
   <222> (250)..(250)
   <223> n is a, c, g, or t
<220>
   <221> misc_feature
   <222> (276)..(276)
   <223> n is a, c, g, or t
<400> 106
   aattgccaca ttttcttatg gcattaaaaa ttttacaaaa acataatttt aatggctata     60
   ttatattcca tttaatggat gcaactcang tttatttaac cattcccatn gttgttaact    120
   atttaggttg tttctaattt tcattattat aaagttgcag aaatttggtg tacataaaac    180
   tgtctccata taattgatta ttaggatata ttcccatgaa ggattcnttt ttttaaaaaa    240
   atgtgaaatn tcatcttgta cttacacctt tcatgnaaag ggatttcctg cttttgtact    300
   gcatgggtgg cagttgtgag gaaaagccag tcaaatgacc tttttacaaa agaaatgcag    360
   tggtcacttc agttgagagt gactttttaa tacaacaaga tcaactagaa gaattcaact    420
   gtctcaagaa tcaaggtacc ccaatatatc tcgcaattcc aaactttgtt tgagggactc    480
   gttatccagc tcttggtagc cacacctgca atgta                               515
<210> 107
   <211> 532
   <212> DNA
   <213> human
<400> 107
   aagccttaca gttatcctgc aagggacagg aaggtctgat ttgcaggatt tttagagcat     60
   taaaataact atcaggcaga agaatctttc ttctcgccta ggatttcagc catgcgcgcg    120
   ctctctctct ttctctctct tttcctctct ctccctcttt ctagcctggg gcttgaattt    180
   gcatgtctaa ttcatttact caccatattt gaattggcct gaacagatgt aaatcgggaa    240
   ggatgggaaa aactgcagtc atcaacaatg attaatcagc tgttgcaggc agtgtcttaa    300
   ggagactggt aggaggaggc atggaaacca aaaggccgtg tgtttagaag cctaattgtc    360
   acatcaagca tcattgtccc catgcaacaa ccaccacctt atacatcact tcctgtttta    420
   agcagctcta aaacatagac tgaagattta tttttaatat gttgacttta tttctgagca    480
   aagcatcggt catgtgtgta ttttttcata gtcccacctt ggagcattta tg            532
<210> 108
   <211> 415
   <212> DNA
   <213> human
<400> 108
   gaaggcggct gatgagagcg agagaggaat gaaggtcatc gaaaaccggg ccatgaagga     60
   tgaggagaag atggaactgc aggagatgca gctgaaggag gccaagcaca tcgctgagga    120
   ttcagaccgc aaatatgaag aggtggccag gaagctggtg atcctggaag gagagctgga    180
   gcgctcggag gagagggctg aggtggccga gagccgagcc agacagctgg aggaggaact    240
   tcgaaccatg gaccaggccc tcaagtccct gatggcctca gaggaggagt attccaccaa    300
   agaagataaa tatgaagagg agatcaaact gttggaggag aagctgaagg aggctgagac    360
   ccgagcagag tttgccgaga ggtctgtggc aaagttggag aaaaccatcg atgac         415
<210> 109
   <211> 366
   <212> DNA
   <213> human
<400> 109
   catgggggga cttgaaccct gcagcaggct cctgctcctg cctctcctgc tggctgtaag     60
   tggtctccgt cctgtccagg cccaggccca gagcgattgc agttgctcta cggtgagccc    120
   gggcgtgctg gcagggatcg tgatgggaga cctggtgctg acagtgctca ttgccctggc    180
   cgtgtacttc ctgggccggc tggtccctcg gggcgagggg gctgcggagg cagcgacccg    240
   gaaacagcgt atcactgaga ccgagtcgcc ttatcaggag ctccagggtc agaggtcgga    300
   tgtctacagc gacctcaaca cacagaggcc gtattacaaa tgagcccgaa tcatgacagt    360
   cagcaa                                                               366
<210> 110
   <211> 480
   <212> DNA
   <213> human
<400> 110
   tccccagcag tggctcggtg agccatgtgc acctatggcc cagccactgc agcacaggag     60
   gctccgtgag ccttggttcc tccccgaaaa cccccagcat tacgatcccc caagtgtcct    120
   ggaccctggc ctaaagaatc ccacccccac ttcatgccca ctgccgatgc ccaatccagg    180
   cccggtgagg ccaaggtttc ccagtgagcc tctgcgcctc tccactgttt catgagccca    240
   aacaccctcc tggcacaacg ctctaccctg cagcttggag aactccgctg gatgggagtc    300
   tcatgcaaga cttcactgca gcctttcaca ggactctgca gatagtgcct ctgcaaacta    360
   aggagtgact aggtgggttg gggaccccct caggattgtt tctcggcacc agtgcctcag    420
   tgctgcaatt gagggctaaa tcccaagtgt ctcttgactg gctcaagaat tagggcccca    480
<210> 111
   <211> 475
   <212> DNA
   <213> human
<400> 111
   cagaccggca gtcatcatgg cagtttcagc gttcaaacag caatagctca agtgtgataa     60
   ctactgagga taataaaatc cacattcact taggaagtcc ttacatgcaa gctgtagcca    120
   gcccttcagc accactgcag gataaccgaa ctcaaggctt aattaacggg gcactaaaca    180
   aaacaaccaa taaagtcacc agcagtatta ctatcacacc aacagccaca cctcttcctc    240
   gacaatcaca aattacagta agtaatatat ataactgacc acgctcaccc tcatccagtc    300
   catactgata tttttgcaag gaactcaatc cttttttaat catccctcca tatcccccaa    360
   gactgactga actcgtactt tgggaaggtt tgtgcatgaa ctatacaaga gtatctgaaa    420
   ctaactgttg cctgcatagt catatcgagt gtgcacttac tgtatatctt ttcat         475
<210> 112
   <211> 344
   <212> DNA
   <213> human
<220>
   <221> misc_feature
   <222> (173)..(173)
   <223> n is a, c, g, or t
<400> 112
   gcgtgttttc ctcttgatcg ggaactcctg cttctccttg cctcgaaatg gaccccaact 60
   gctcctgctc gcctgttggc tcctgtgcct gtgccggctc ctgcaaatgc aaagagtgca 120
   aatgcacctc ctgcaagaag agctgctgct cctgctgccc tgtgggctgt gcnaagtgtg 180
   cccagggctg catctgcaaa gggacgtcag acaagtgcag ctgctgtgcc tgatgccagg 240
   acagctgtgc tctcagatgt aaatagagca acctatataa acctggattt tttttttttt 300
   ttttttgtac aaccctgacc cgtttgctac atcttttttt ctat 344
<210> 113
   <211> 318
   <212> DNA
   <213> human
<400> 113
   ttccacatca gtaactgccc tggggtttgt gctgtacaaa tacaagctcc tgccacggtc 60
   ttgaagttct gttcttatgc tctctgctca ctggttttca ataccaccaa gaggaaaata 120
   ttgacaagtt taaaggctgt gtcattgggc catgtttaag tgtactggat ttaactacct 180
   ttggcttaat tccaatcatt gttaaagtaa aaacaattca aagaatcacc taattaattt 240
   cagtaagatc aagctccatc ttatttgtca gtgtagatca actcatgtta attgatagaa 300
   taaagccttg tgatcact 318
<210> 114
   <211> 411
   <212> DNA
   <213> human
<400> 114
   ctttgcgggc acagagactg ccacaaagtg gagcggctac atggaagggg cagttgaggc 60
   tggagaacga gcagctaggg aggtcttaaa tggtctcggg aaggtgaccg agaaagacat 120
   ctgggtacaa gaacctgaat caaaggacgt tccagcggta gaaatcaccc acaccttctg 180
   ggaaaggaac ctgccctctg tttctggcct gctgaagatc attggatttt ccacatcagt 240
   aactgccctg gggtttgtgc tgtacaaata caagctcctg ccacggtctt gaagttctgt 300
   tcttatgctc tctgctcact ggttttcaat accaccaaga ggaaaatatt gacaagttta 360
   aaggctgtgt cattgggcca tgtttaagtg tactggattt aactaccttt g 411
<210> 115
   <211> 454
   <212> DNA
   <213> human
<400> 115
   taattgcttg ttttctagcc tggcaagata ttttcataaa agagggataa caatgctgat 60
   tactaccttt taaaatattt tagataaatg cacagcacca cagcaccaca tctaagcatt 120
   agtgatgggt agctgatgtc agcttcatgt ggattttaag cactctagaa acaatgaagc 180
   ttcttggcat attttaagga gctcccaaaa tgtgttacct attaaattgt aactcagcaa 240
   gtagaagacc atttgaaaag tcaggtacaa atttcctcaa gtggcataaa aatgtagtca 300
   gttttctctt ttaccagttt ttatttccac tccaattatt tagaacttta tttgtacatg 360
   tgcagaagaa taaggcagct gagaatcttg tttcccccaa gagagtttta caggctgagt 420
   gttgcaaatg tgttctttgt cctgttatat gtat 454
<210> 116
   <211> 482
   <212> DNA
   <213> human
<400> 116
   gcactgccat gtatgaaagt ctctttatga tgtttgtttt tttgtcattt ttgttcttta     60
   catcaagaaa ttttatgttt aaatatgcgg agaatgtata ttgcctctgc tcctatcagg    120
   gttgctaaac cctggtacat cgtatataaa atgtattaaa actggggttt gttaccagtt    180
   gctgtacttt gtatatagaa tttttataaa ttgtatgctt cagaaataat ttatttttaa    240
   aaagaaatta aaagttttaa actcacatcc atattacacc tttcccccct gaaatgtata    300
   gaatccattt gtcatcagga atcaaaaccc acagtccatt gtgaagtgtg ctatatttag    360
   aacagtctta aaatgtacag tgtattttat agaattgaag ttaacattct tattttcaag    420
   agaatttatg gacgttgtag aaatgtacaa atgcatttcc aaactgcctt aaacgttgta    480
   tt                                                                   482
<210> 117
   <211> 422
   <212> DNA
   <213> human
<400> 117
   gggactgtct gtcaaaagac tctgtatatc ttttgtggat gagttttgtg agagaacaga     60
   gagaccattg tacctggcac aagggctctt catgaaaagg gagacttact gggaggtgca    120
   agacagtggc atttctcctc tcctcttgct gctcagcaca gccctggatt gcagccccga    180
   ggctgagacc agacaaagcc cgggaggcag aaagatgctc caagaaccaa cactatcaat    240
   gtctttgcaa atcctcacag gattcctgtg ggtccagctt tggaactggg aaacctttct    300
   tcggatccgc actcattcca ctgatgccag ctgcccctga aggatgccag tactgtggtg    360
   tgtgagtctc agcagccgcc cacacgctcc taactctgct gcatggcaga tgcctaggtg    420
   ga                                                                   422
<210> 118
   <211> 357
   <212> DNA
   <213> human
<400> 118
   attaatcagc cccagagtgc tttaaaaaat tctcttaaat aaaaataata gactcgctag     60
   tcagtaaaga tatttgaata tgtatcgtgc cccctccggt gtctttgatc aggatgacat    120
   gtgccatttt tcagaggacg tgcagacagg ctggcattct agattacttt tcttactctg    180
   aaacatggcc tgtttgggag tgcgggattc aaaggtggtc ccaccgctgc ccctactgca    240
   aatggcagtt ttaatcttat cttttggctt ctgcagatgg ttgcaattga tccttaacca    300
   ataatggtca gtcctcatct ctgtcctgct tcataggtgc caccttgtgt gtttaaa       357
<210> 119
   <211> 328
   <212> DNA
   <213> human
<400> 119
   ctacgcgtgt ccttgggcgg agaagggagg tgactccggc ggaagaggac aaggcagaat     60
   gcaggccctt cgggtgtccc aggcgctgat ccgctccttc agctccaccg cccggaaccg    120
   ctttcagaac cgagtgcgcg agaaacagaa gctcttccag gaggacaatg acatcccgtt    180
   gtacctgaag ggcggcatcg ttgacaacat cctgtaccga gtgacaatga cgctgtgtct    240
   gggcggcact gtctacagct tgtactccct tggctgggcc tccttcccca ggaattaaga    300
   ccaagaagcc tggggggcct gagagact                                        328
<210> 120
   <211> 417
   <212> DNA
   <213> human
<400> 120
   tggtgacaca aacagccttt tcccaggtac ttggtacctg gagcgagtgg acgagcagca 60
   tcgccgaaag tatgcccggc gtcccgtcta aaggtgttct gcagatccat ggaaagcttc 120
   ctgggaaacg tatgctagca gagcttctcc ccgtgaatca tatttttaag atcccactct 180
   tagctggtaa atgaatttga atcgacatag tagccccata agcatcagcc ctgtagagtg 240
   aggagccatc tctagcgggc ccttcattcc tctccatgct gcaatcactg tcctgggctt 300
   atggtgccta tggactaggg gtcctttgtg aaagagcaag atggagcaat ggagagaaga 360
   cctcttcctg aatcactgga ctccagaaat gtgcatgcag atcagctgtt gccttca 417
<210> 121
   <211> 533
   <212> DNA
   <213> human
<400> 121
   ttgatgccag catttgcatc ccgggctcca tcacattcat gcccaatgga tgctgcaaga 60
   cctgcacccc tcgcaatgag accagggtgc cctgctccac cgtccccgtc accacggagg 120
   tttcgtacgc cggctgcacc aagaccgtcc tcatgaatca ttgctccggg tcctgcggga 180
   catttgtcat gtactcggcc aaggcccagg ccctggacca cagctgctcc tgctgcaaag 240
   aggagaaaac cagccagcgt gaggtggtcc tgagctgccc caatggcggc tcgctgacac 300
   acacctacac ccacatcgag agctgccagt gccaggacac cgtctgcggg ctccccaccg 360
   gcacctcccg ccgggcccgg cgctccccta ggcatctggg gagcgggtga gcggggtggg 420
   cacagccccc ttcactgccc tcgacagctt tacctccccc ggaccctctg agcctcctaa 480
   gctcggcttc ctctcttcag atatttattg tctgagtctt tgttcagtcc ttg 533
<210> 122
   <211> 417
   <212> DNA
   <213> human
<400> 122
   cttgcttata tcatgtgctg ccgacgggaa ggcgtggaaa agagaaacat gcaaacacca 60
   gacatccaac tggtccatca cagtgctatt cagaaatcta ccaaggagct tcgagacatg 120
   tccaagaata gagagatagc atggcccctg tcaacgcttc ctgtgttcca ccctgtgact 180
   ggggaaatca tacctccttt acacacagac aactatgata gcacaaacat gccattgatg 240
   caaacgcagc agaacttgcc acatcagact cagattcccc aacagcagac tacaggtaaa 300
   tggtatccct gaagaaagaa aactgactga agcaatgaat ttataatcag acaatatagc 360
   agttacatca catttctttt ctcttccaat aatgcatgag cttttctggc atatgtt 417
<210> 123
   <211> 542
   <212> DNA
   <213> human
<400> 123
   cctcccggga ggacagcctt gaggcgggcc tgcccctcca ggtccgaggc taccccgagg 60
   agaagaaaga ggaggagggc agcgcaaacc gcagaccaga ggaccaggag ctggagagcc 120
   tgtcggccat tgaagcagag ctggagaaag tggcccacca gctgcaggca ctacggcggg 180
   gctgagacac cggctggcag ggctggcccc agggcaccct gtggccctgg ctctgctgtc 240
   cccttggcag gtcctggcca gatggcccgg acgctgcttc cggtagggag gcagcctcca 300
   gcctgcccaa gcccaggcca ccctatcgcc ccctacgcgc cttgtctcct actcctgact 360
   cctacctgcc ctggaacatc ctttgcaggg cagccccaca actttaaaca ttgacgattc 420
   cttctctgaa cacaggcagc tttctagaag tttcccttcc tccatcctat ccactgggca 480
   caactgcaat aacttctgac cttttggtga aagctgagaa ctcctgactg taacatattc 540
   tg 542
<210> 124
   <211> 537
   <212> DNA
   <213> human
<400> 124
   gcaggccata ctggttccat tgttctgtat aatactgaat aaataaattt acttttacat     60
   gatcgtataa gtttctagat aagataaaca aattctgttt aaattttttt aataaaaatc    120
   ttaaaacact ttttttctaa cctagactga gaaattcatg tttacttttc taggtgtatg    180
   atactttgta aagttgatac tttcctaaga atttaacatg tcatattttt gaaatagatt    240
   taagtgtgct tcttattgct aaaaatacta aatgtcatgg gtcatagtat ctgatatcaa    300
   tatcgttgat aacatatcca caggtaacac catgatgtag gcataaatgg aaaacaaaaa    360
   ccctactatt tcaaatatat tgtacttttt tatttctgta agccaactgt gtgccatttt    420
   cactggactt ttaaatctag actttagtga tgtctacatt gtaaatgatc ttttgtggat    480
   atttgtcact tggtttcaga aagttcacaa atgtagcaac agctcacatg actgagt       537
<210> 125
   <211> 312
   <212> DNA
   <213> human
<400> 125
   tcccttctcg cttgggaact ctagtctcgc ctcgggttgc aatggacccc aactgctcct     60
   gtgccgctgg tgtctcctgc acctgcgcca gctcctgcaa gtgcaaagag tgcaaatgca    120
   cctcctgcaa gaagagctgc tgctcctgct gccctgtggg ctgtgccaag tgtgcccaag    180
   gctgcatctg caaaggggca tcggagaagt gcagctgctg cgcctgatgt cgggacagcc    240
   ctgctcccaa gtacaaatag agtgacccgt aaaatctagg attttttgtt ttttgctaca    300
   atcttgaccc ct                                                        312
<210> 126
   <211> 393
   <212> DNA
   <213> human
<400> 126
   aaaggctggc atcttatggc tcatcaaagg cggctgtgac catgttctca tcagttatga     60
   gactggagct ttccaagtgg ggaattaaag ttgcttccat ccaacctgga ggcttcctaa    120
   caaatatcgc aggcaccagt gacaagtggg aaaagctgga gaaggacatt ctggaccacc    180
   tccccgctga ggtacaggaa gactacggcc aggactacat cttagcacag cggaatttcc    240
   tcctattgat caactcgtta gccagcaagg acttctctcc ggtgctgcgg gacatccagc    300
   atgctatctt ggcgaagagc ccttttgcct attacacgcc agggaaaggc gcttacttgt    360
   ggatctgcct tgctcactat ttgcctattg gca                                 393
<210> 127
   <211> 460
   <212> DNA
   <213> human
<400> 127
   ggtgtcgaac tatatgcctt gtatgatcag ttttatgtgg ctaatgagtt tctcaaatat     60
   cgtttacacg ttggtaacta taatggcaca gctggagatg cattacgttt caacaaacat    120
   tacaaccacg atctgaagtt tttcaccact ccagataaag acaatgatcg atatccttct    180
   gggaactgtg ggctgtacta cagttcaggc tggtggtttg atgcatgtct ttctgcaaac    240
   ttaaatggca aatattatca ccaaaaatac agaggtgtcc gtaatgggat tttctggggt    300
   acctggcctg gtgtaagtga ggcacaccct ggtggctaca agtcctcctt caaagaggct    360
   aagatgatga tcagacccaa gcactttaag ccataaatca ctctgttcat tcctccaggt    420
   attcgttatc taatagggca attaattcct tcagcacttt                          460
<210> 128
   <211> 525
   <212> **DNA**
   <213> human
<400> 128
   ccaggagggg ctgtccaatc acattcaggc atgcgaatga gctgggccct gggtgaggtg     60
   ggggtctggc ctagtgggga ggggcctggc ctgggtgggg cagggcctgg cctggtccag    120
   gcttgggctc cattcccatc actgctgtcc ctcctgaggt ctggattggg gatggggaca    180
   aagaaatagc aagagatgag aaacaacaga aacttttttc tctaaaggac tggttaaatc    240
   aattctgata cagccttaca atacaatagt atgcagctaa aaaataattg tatgtcttta    300
   tatactaata tgtaataatc ttcaggtgaa aaaggcaagc cacagaaatg tgtatagcgc    360
   acttcccatt tgtgtttcag aaaggagtag aatataaaca cataattgct tatgtatgcc    420
   tattcagaat aaatgggtaa cactgattac ttttgggagg ggaaccagta ggttgaggac    480
   aggagaggga agggtcttaa cacttacacc cttttgtaca ttttg                     525
<210> 129
   <211> 515
   <212> DNA
   <213> human
<400> 129
   aggagcccag gaacgacgtg gtcttccagc ccatttccgg ggaagacgtg cgcgatgtga     60
   cagccctgaa cgtgagcacg ctgaaggctt acttcagatg cgatggctac aagggctacg    120
   acctggtcta cagcccccag agcggcttca cctgcgtgtc cccgtgcagt aggggctact    180
   gtgaccatgg aggccagtgc cagcacctgc ccagtgggcc ccgctgcagc tgtgtgtcct    240
   tctccatcta cacggcctgg ggcgagcact gtgagcacct gagcatgaaa ctcgacgcgt    300
   tcttcggcat cttctttggg gccctgggcg gcctcttgct gctgggggtc gggacgttcg    360
   tggtcctgcg cttctggggt tgctccgggg ccaggttctc ctatttcctg aactcagctg    420
   aggccttgcc ttgaaggggc agctgtggcc taggctacct caagactcac ctcatcctta    480
   ccgcacattt aaggcgccat tgcttttggg agact                                515
<210> 130
   <211> 339
   <212> DNA
   <213> human
<400> 130
   agcagcttat tgtttctctg aaagtgtgtg tagttttact ttcctaagga attaccaaga     60
   atatccttta aaatttaaaa ggatggcaag ttgcatcaga aagctttatt ttgagatgta    120
   aaaagattcc caaacgtggt tacattagcc attcatgtat gtcagaagtg cagaattggg    180
   gcacttaatg gtcaccttgt aacagttttg tgtaactccc agtgatgctg tacacatatt    240
   tgaagggtct ttctcaaaga aatattaagc atgttttgtt gctcagtgtt tttgtgaatt    300
   gcttggttgt aattaaattc tgagcctgat attgatatg                            339
<210> 131
   <211> 377
   <212> DNA
   <213> human
<400> 131
   gagaacgggt acattcaccc ggtcaacctg acgtggccct ttatggtggc cgggaaaccc     60
   gagagtgacc tgaaagaagt ggtgaccgcg agccgcttat gtggaaccac cgcgtcctga    120
   ccttggaggt gcgagtctgg gaaaggcgcg ctcccggggg gagcgggccc cgggaaggcg    180
   acccctgccc tcagtgctct ctgtctctgc ttccccctcg caatgctcct ctctctgtcc    240
   caccccgcga gaacacttta caacgacgag gagattcgtt tccaaaccag aggagatcaa    300
   ttgtacttac aaagattccc atctatttaa ctttattaac ttctaccgtg aatgactctg    360
   caagccttgc tggtcca                                                    377
<210> 132
   <211> 252
   <212> DNA
   <213> human
<400> 132
   ttctctcgac cacttaaaac ttcagacttc ctgtcctgct ggtatcatgg agaaagtcca     60
   atacctcact cgctcagcta taagaagagc ctcaaccatt gaaatgcctc aacaagcacg    120
   tcaaaagcta cagaatctat ttatcaattt ctgtctcatc ttaatatgtc tcttgctgat    180
   ctgtatcatc gtgatgcttc tctgaagttc tgctacaacc tctagatctg cagcttgcca    240
   catcagctta aa                                                        252
<210> 133
   <211> 191
   <212> DNA
   <213> human
<400> 133
   tggcccatct attacatcta cagctgaccc ttgaacatgg gggttagggg agctgacaat     60
   tcgtgggtcc gcaaaatctt aactacctaa tagcctacta ttgaccataa accttactga    120
   taacataaac agtaaattaa cacatatttt gcgtgttata tgtattatac actatattcc    180
   tacaataaag t                                                         191
<210> 134
   <211> 371
   <212> DNA
   <213> human
<400> 134
   aaatctgttc taagacatat gatcaacaga tgagaactgg tggttaatat gtgacagtga     60
   gattagtcat atcactaata tactaacaac agaatctaat cttcatttaa ggcactgtag    120
   tgaattatct gagctagagt tacctagctt accatactat atctttggaa tcatgaaacc    180
   ttaagacttc agaatgattt tgcaggttgt cttccattcc agcctaacat ccaatgcagg    240
   caaggaaaat aaaagatttc cagtgacaga aaaatatatt atctcaagta ttttttaaaa    300
   atatatgaat tctctctcca aatattaact aattattaga ttatattttg aaatgaactt    360
   gttggcccat c                                                         371
<210> 135
   <211> 441
   <212> DNA
   <213> human
<400> 135
   ggacaaagag cgctatgtct ccctggtgat gcctgtggcc ctgttcaacc tgattgacac     60
   ttttcccttg agaaaagaag agatggtcct acaagccgaa atgagccaga cctgtaacac    120
   ctgacatgat ggttcctctc ttggcaattc ctcttcattg tctacatagt gacatgcaca    180
   cgggaaagcc ttaaaaatat ccttgatgta cagattttat ttgtaatttt aaaagtctat    240
   tttattatga gctttctttg cacttaaaaa ttagcatgct gctttttgta cttggaagtg    300
   tttcaaaaaa ttatatgacc atatttactc tttctaacct ttctttactc catcatggct    360
   ggttgatttg tagagaaatt agaacccata accatacaca ggctatcaac atgttattca    420
   atgtgacacc taactctttt c                                              441
<210> 136
   <211> 402
   <212> DNA
   <213> human
<220>
   <221> misc_feature
   <222> (353)..(353)
   <223> n is a, c, g, or t
<400> 136
   tactcatgcc tttttgttta ggataaatag gtaagcacaa agagctcttc aaaatcagaa     60
   aaaacaatag gagtccttcc ttgtcttttc tgtgatctct gtccttgttt ctgagacttt    120
   ctctaccatt aagctctatt ttagctttca gttattctag tttgtttccc atggaatctg    180
   tcctaaactg gtgtttttgt cagtgacagt cttgccagtc agcaatttct aacagcattt    240
   taaatgagtt tgatgtacag taaatattga tgacaatgac agcttttaac tcttcaagtc    300
   acctaaagct attatgcagg aggatttaga agtcacattc ataaaaccca agngctatgg    360
   gtgtattatt catgatagct ggcccacagg tcatgaattg ag                       402
<210> 137
   <211> 174
   <212> DNA
   <213> human
<400> 137
   agaagctcac caagtcaact aaacagcccc gaggacttac atcaccttct cagctcttga     60
   cctcagaaag tatccaaacc aaggaggaga aacctgtggg tggcttgtcc tccagtgaca    120
   caatggatta ccgacagtga ctaagggcag catgtttaac ccaggtgaag atct          174
<210> 138
   <211> 371
   <212> DNA
   <213> human
<400> 138
   tttctggcct tcacccagac gaagaccttc cacgaggcca gcgaggactg catctcgcgc     60
   gggggcaccc tgagcacccc tcagactggc tcggagaacg acgccctgta tgagtacctg    120
   cgccagagcg tgggcaacga ggccgagatc tggctgggcc tcaacgacat ggcggccgag    180
   ggcacctggg tggacatgac cggcgcccgc atcgcctaca agaactggga gactgagatc    240
   accgcgcaac ccgatggcgg caagaccgag aactgcgcgg tcctgtcagg cgcggccaac    300
   ggcaagtggt tcgacaagcg ctgccgcgat cagctgccct acatctgcca gttcgggatc    360
   gtgtagccgg c                                                         371
<210> 139
   <211> 406
   <212> DNA
   <213> human
<400> 139
   gcagactggg agttgctagc aaacaaatgg cttacttaca aaagcagctt ttagttcaga     60
   cttagttttt ataaaatgag aattctgact tacttaacca ggtttgggat ggagatggtc    120
   tgcatcagct ttttgtatta acaaagttac tggctctttg tgtgtctcca ggtaactttg     180
   cttgattaaa cagcaaagcc atattctaaa ttcactgttg aatgcctgtc ccagtccaaa    240
   ttgtctgtct gctcttattt ttgtaccata ttgctcttaa aaatcttggt ttggtacagt    300
   tcataattca ccaaaaagtt catataattt aaagaaacac taaattagtt taaaatgaag    360
   caatttatat ctttatgcaa aaacatatgt ctgtctttgc aaagga                   406
<210> 140
   <211> 388
   <212> DNA
   <213> human
<400> 140
   gcacccagcc ttttaccatg tgggtttctt tagtgtctta aaagcgtcca taagccacca    60
   ttctgtggaa ccaaggcccc ctccacgcaa acaccctccc tcctggggac ctctggagcc   120
   tcagccagaa gtaccattag gtttaatttt aatttgtttt gctggagaaa catcaggttt   180
   gtaggagact gagttgttag caggtgtgct tagctcttga tagtgaacgt gtaccttggg   240
   aactggctca cccacctgct aatagcacca tcgtcactat taagcagaca tttcagttgg   300
   tagaatccat gtagaagtca tggacttttc tgggaaatga cttttctggg aaatgacagt   360
   ttctttgaca tattttcttt gcccactt                                       388
<210> 141
   <211> 317
   <212> DNA
   <213> human
<400> 141
   cgccatcacc gagcgcttga tgtgcgcgga gagcaatcgc cgggacagct gcaagggtga    60
   ctccgggggc ccgctggtgt gcgggggcgt gctcgagggc gtggtcacct cgggctcgcg   120
   cgtttgcggc aaccgcaaga agcccgggat ctacacccgc gtggcgagct atgcggcctg   180
   gatcgacagc gtcctggcct agggtgccgg ggcctgaagg tcagggtcac ccaagcaaca   240
   aagtcccgag caatgaagtc atccactcct gcatctggtt ggtctttatt gagcacctac   300
   tatatgcaga aggggag                                                  317
<210> 142
   <211> 317
   <212> DNA
   <213> human
<400> 142
   gggccacgcc aggaatattc agaaaataat gagaactaca ttgaagtgcc attgattttt    60
   gatcctgtca caagagagga tttgcacatg gattttaaat gtgttgtcca taataccctg   120
   agttttcaga cactacgcac cacagtcaag gaagcctcct ccacgttctc ctggggcatt   180
   gtgctggccc cactttcact ggccttcttg gttttggggg gaatatggat gcacagacgg   240
   tgcaaacaca gaactggaaa agcagatggt ctgactgtgc tatggcctca tcatcaagac   300
   tttcaatcct atcccaa                                                  317
<210> 143
   <211> 456
   <212> DNA
   <213> human
<400> 143
   gctgctctgg ttctcatgac ggcagatgca gcgaagaggc tcaatgttac accactggca    60
   agaatagtag catttgctga cgctgctgta gaacctattg attttccaat tgctcctgta   120
   tatgctgcat ctatggttct taaagatgtg ggattgaaaa aagaagatat tgcaatgtgg   180
   gaagtaaatg aagcctttag tctggttgta ctagcaaaca ttaaaatgtt ggagattgat   240
   ccccaaaaag tgaatatcaa tggaggagct gtttctctgg gacatccaat tgggatgtct   300
   ggagccagga ttgttggtca tttgactcat gccttgaagc aaggagaata cggtcttgcc   360
   agtatttgca atggaggagg aggtgcttct gccatgctaa ttcagaagct gtagacaacc   420
   tctgctattt aaggagacaa ccctatgtga ccagaa                             456
<210> 144
   <211> 507
   <212> DNA
   <213> human
<400> 144
   ggaaagcagg attccatcgc tggaacaatt acatgatgga ctggaaaaat caatttaacg 60
   attacactag caagaaagaa agttgtgtgg gtctctaatt aatagattta ccctttatag 120
   aacatatttt cctttagatc aaggcaaaaa tatcaggagc ttttttacac acctactaaa 180
   aaagttatta tgtagctgaa acaaaaatgc cagaaggata atattgattc ctcacatctt 240
   taacttagta ttttacctag catttcaaaa cccaaatggc tagaacatgt ttaattaaat 300
   ttcacaatat aaagttctac agttaattat gtgcatatta aaacaatggc ctggttcaat 360
   ttctttcttt ccttaataaa tttaagtttt ttccccccaa aattatcagt gctctgcttt 420
   tagtcacgtg tattttcatt accactcgta aaaaggtatc ttttttaaat gaattaaata 480
   ttgaaacact gtacaccata gtttaca 507
<210> 145
   <211> 489
   <212> DNA
   <213> human
<400> 145
   gagggtgatg ccatctaacc ctgcccctgt ccaccccggg tgggtgaaac tcactgagca 60
   gccaagactg ttgcccgagg actcactgta tggtgccctc tccaaagggt cgggagggta 120
   gctctccagg ccagagcttg tgtccttcaa cagagaggcc agcggcaact ggtccgttac 180
   tggccaaggg ctctgaagaa tcaacggtgc tggtacagga tacaggaata aattgtatct 240
   tcacctggtt cctaccctcg tccctacctg tcctgatcct ggtcctgaag acccctcgga 300
   acaccctctc ctggtggcag gccacttccc tcccagtgcc agtctccatc caccccagag 360
   aggaacaggc gggtgggcca tgtggttttc tccttcctgg ccttggctgg cctctggggc 420
   aggggtggtg gagagatgga agggcatcag gtgtagggac cctgccaagt ggcacctgat 480
   ttactctag 489
<210> 146
   <211> 480
   <212> DNA
   <213> human
<400> 146
   ttaatgtgcc aaggagccgt tttctgcctg tcaaaaccac atcagatctc ttgctggtga 60
   tgtcaaacct ctatagtctt aatgcaggat ctctgacaat gagtgaaaag cgggaatttc 120
   ctacagtgcc cttggttaaa ttaggcagtt cttttacgaa ggttcaagat tatctaagaa 180
   gatttgaaag tataccagat atgcttgaat tggatcacct cacagtttca ggagatgtga 240
   catttggaaa aaatgtttca ttaaagggaa cggttatcat cattgcaaat catggtgaca 300
   gaattgatat cccacctgga gcagtattag agaacaagat agtgtctgga aaccttcgca 360
   tcttggacca ctgaaatgaa aaatactgtg gacacttaaa taatgggcta gtttcttaca 420
   atgaaatgtt ctctaggatt taggcactaa aaggtacttt actatgttac tgtaccctgc 480
<210> 147
   <211> 529
   <212> DNA
   <213> human
<400> 147
   caagaatgat gggcactacc gtggagatcc caactggttt atgaagaaag cgcaggagca 60
   taagagggaa ttcacagaga gccagctgca ggagggaaag catgtcattg gccttcagat 120
   gggcagcaac agaggggcct cccaggccgg catgacaggc tacggacgac ctcggcagat 180
   catcagttag agcggagagg gctagccctg agcccggcgc tcccccagct ccttggctgc 240
   agccatcccg cttagcctgc ctcacccaca cccgtgtggt accttcagcc ctggccaagc 300
   tttgaggctc tgtcactgag caatggtaac tgcacctggg cagctcctcc ctgtgccccc 360
   agcctcagcc caacttctta cccgaaagca tcactgcctt ggcccctccc tcccggcggc 420
   ccccatcacc tctactgtct cctccctggg ctaagcaggg gagaagcggg ctgggggtag 480
   cctggatgtg ggcgaagtcc actgtcctcc ttggcggcaa aagcccatt 529
<210> 148
   <211> 525
   <212> DNA
   <213> human
<400> 148
   tcaaagactt cgtgattatc cccctgcaca ccaccccaga gacatccgtt aaggagatcg     60
   atgagttggt tgaggtctac acggacgtga aacaccgctg gaaggcggag aatttcattt    120
   tcatgggtga cttcaatgcc ggctgcagct acgtccccaa gaaggcctgg aagaacatcc    180
   gcttgaggac tgaccccagg tttgtttggc tgatcgggga ccaagaggac accacggtga    240
   agaagagcac caactgtgca tatgacagga ttgtgcttag aggacaagaa atcgtcagtt    300
   ctgttgttcc caagtcaaac agtgtttttg acttccagaa agcttacaag ctgactgaag    360
   aggaggccct ggatgtcagc gaccactttc cagttgaatt taaactacag tcttcaaggg    420
   ccttcaccaa cagcaaaaaa tctgtcactc taaggaagaa aacaaagagc aaacgctcct    480
   agacccaagg gtctcatctt attaaccatt tcttgcctct aaata                    525
<210> 149
   <211> 559
   <212> DNA
   <213> human
<400> 149
   aacgaggagc acatgaccct gctgaagatg attttgataa aatgctgtga tatctctaac     60
   gaggtccgtc caatggaagt cgcagagcct tgggtggact gtttattaga ggaatatttt    120
   atgcagagcg accgtgagaa gtcagaaggc cttcctgtgg caccgttcat ggaccgagac    180
   aaagtgacca aggccacagc ccagattggg ttcatcaagt ttgtcctgat cccaatgttt    240
   gaaacagtga ccaagctctt ccccatggtt gaggagatca tgctgcagcc actttgggaa    300
   tcccgagatc gctacgagga gctgaagcgg atagatgacg ccatgaaaga gttacagaag    360
   aagactgaca gcttgacgtc tggggccacc gagaagtcca gagagagaag cagagatgtg    420
   aaaaacagtg aaggagactg tgcctgagga aagcgggggg cgtggctgca gttctggacg    480
   ggctggccga gctgcgcggg atccttgtgc agggaagagc tgccctgggc acctggcacc    540
   acaagaccat gttttctaa                                                 559
<210> 150
   <211> 542
   <212> DNA
   <213> human
<400> 150
   tgacgcaaaa taccaccttg gcgcctacac gggagacgac gtccgcatcg tccgtgacga     60
   catgctgtgt gccgggaaca cccggaggga ctcatgccag ggcgactccg gagggcccct    120
   ggtgtgcaag gtgaatggca cctggctgca ggcgggcgtg gtcagctggg gcgagggctg    180
   tgcccagccc aaccggcctg gcatctacac ccgtgtcacc tactacttgg actggatcca    240
   ccactatgtc cccaaaaagc cgtgagtcag gcctgggttg gccacctggg tcactggagg    300
   accaacccct gctgtccaaa acaccactgc ttcctaccca ggtggcgact gccccccaca     360
   ccttccctgc cccgtcctga gtgccccttc ctgtcctaag ccccctgctc tcttctgagc     420
   cccttcccct gtcctgagga cccttcccta tcctgagccc ccttccctgt cctaagcctg    480
   acgcctgcac cgggccctcc agccctcccc tgcccagata gctggtggtg ggcgctaatc    540
   ct                                                                   542
<210> 151
   <211> 220
   <212> DNA
   <213> human
<400> 151
   gaagagggag ctctattgcc accatgagtt tctccggcaa gtaccaactg cagagccagg      60
   aaaactttga agccttcatg aaggcaatcg gtctgccgga agagctcatc cagaagggga     120
   aggatatcaa gggggtgtcg gaaatcgtgc agaatgggaa gcacttcaag ttcaccatca    180
   ccgctgggtc caaagtgatc caaaacgaat tcacggtggg                          220
<210> 152
   <211> 524
   <212> DNA
   <213> human
<400> 152
   ccttctctgg tctccttgag atgatcgtag acacagggat gattcccacc caaacccacg     60
   tattcattca gtgagttaaa cacgaattga tttaaagtga acacacacaa gggagcttgc    120
   ttgcagatgg tctgagttct tgtgtcctgg taattcctct ccaggccaga ataattggca    180
   tgtctcctca acccacatgg ggttcctggt tgttcctgca tcccgatacc tcagccctgg    240
   ccctgcccag cccatttggg ctctggtttt ctggtggggc tgtcctgctg ccctcccaca    300
   gcctccttct gtttgtcgag catttcttct actcttgaga gctcaggcag cgttagggct    360
   gcttaggtct catggaccag tggctggtct cacccaactg cagtttacta ttgctatctt    420
   ttctggatga tcagaaaaat aattccataa atctattgtc tacttgcgat tttttaaaaa    480
   atgtatattt ttatatatat tgttaaatcc tttgcttcat tcca                     524
<210> 153
   <211> 486
   <212> DNA
   <213> human
<400> 153
   tctcccaacc tctactgtaa actttctggt ccgagaacga gccgaacaca gcgcgacgca     60
   gggactagga cggcccggtg accgcgcgga ttcaggattg cggggacgca gaaaggttaa    120
   ggcactttta aaaactatag caaggctcct gtttatttat tctactttct ttccctaata    180
   atcaaaacac cgcgtaggct cctccgttta tcagtattaa tggtgtaact ttgttggcaa    240
   tatttgccgt gtagaatttt ttttagatat ccattgtaaa tttgaaacaa agaccgatct    300
   gtgtaaaaac aaatttccat atgttttata taaatatata tataatatga aggactaccc    360
   tccttttttt ttttttgtat tttggctgct agagtgcagc atttgtgaca cgtatttgaa    420
   atttgaaatt tccttctgca ctgtataaaa ggaccatttg aggatgtttt gccttttgtg    480
   tatttt                                                               486
<210> 154
   <211> 560
   <212> DNA
   <213> human
<400> 154
   gagccccatt cacaaatttt gacccctcta ctctccttcc ttcatccctg gatttctgga     60
   cctaccctgg ctctctgact catcctcctc tttatgagag tgtaacttgg atcatctgta    120
   aggagagcat cagtgtcagc tcagagcagc tggcacaatt ccgcagcctt ctatcaaatg    180
   ttgaaggtga taacgctgtc cccatgcagc acaacaaccg cccaacccaa cctctgaagg    240
   gcagaacagt gagagcttca ttttgatgat tctgagaaga aacttgtcct tcctcaagaa    300
   cacagccctg cttctgacat aatccagtta aaataataat ttttaagaaa taaatttatt    360
   tcaatattag caagacagca tgccttcaaa tcaatctgta aaactaagaa acttaaattt    420
   tagttcttac tgcttaattc aaataataat tagtaagcta gcaaatagta atctgtaagc    480
   ataagcttat cttaaattca agtttagttt gaggaattct ttaaaattac aactaagtga    540
   tttgtatgtc tatttttttc                                                560
<210> 155
   <211> 521
   <212> DNA
   <213> human
<400> 155
   ggcagctttt ttatcagcct tgctttggat aggacctcca aggactaagc ctccagcccc     60
   atgtgtgacc cttgtcatct ctctgcccca cataattatg ttactttgct atgtgctcct    120
   aatgtatcta gtgtgtcctg tgacaacact catcacactt cattgtaaat cacttgtttt    180
   attgactgtc tttcctatag actgtaagct ccatgagggc aggcacatgt tgttctcatt    240
   gaccgtgctg gccccagtgc ctagatgcat ggctggcaca ttgttggcac tcaacaatgg    300
   ttgaatgaat aaaacaataa atgaatgaat aactaagata tagaaactct catttatatt    360
   gcagattgaa tatatatgat gaaattctta tgttgaatat gttagaatca aatactcatt    420
   tttcattaga tacagtagtg tcatcactct tttaagatct tgttaaagat ttcaaataaa    480
   ggtacttctg gcgagccagg ctgcacagca tttgctttcc t                        521
<210> 156
   <211> 268
   <212> DNA
   <213> human
<400> 156
   tatttgaata tgtatcgtgc cccctccgga gtctttgatc aggatgacat gtgccatttt     60
   tcagaggacg tgcagacagg ctggcattct agattacttt tcttactctg aaacatggcc    120
   tgtttgggag tgcgggattc aaaggtggtc ccacggctgc ccctactgca aatggcagtt    180
   ttaatcttat cttttggctt ctgcagatgg ttgcattgat ccttaaccaa taatggtcag    240
   tcctcatctc tgtcctgact tcataggt                                       268
<210> 157
   <211> 529
   <212> DNA
   <213> human
<400> 157
   ctttctatat tgttatcagt ccaggaaaca ggtaaacaga tgtaattaga gacattggct     60
   ctttgtttag gcctaatctt tctttttact tttttttttc ttttttcttt ttttttaaag    120
   atcatgaatt tgtgacttag ttctgccctt tggagaacaa aagaaagcag tcttccatca    180
   aatcacctta aaatgcacgg ctaaactatt cagagttaac actccagaat tgttaaatta    240
   caagtactat gctttaatgc ttctttcatc ttactagtat ggcctataaa aaaaataata    300
   ccacttgatg ggtgaaggct ttggcaatag aaagaagaat agaattcagg ttttatgtta    360
   ttcctctgtg ttcacttcgc cttgctcttg aaagtgcagt atttttctac atcatgtcga    420
   gaatgattca atgtaaatat ttttcatttt atcatgtata tcctatacac acatctcctt    480
   catcatcata tatgaagttt attttgagaa gtctacattg cttacattt               529
<210> 158
   <211> 494
   <212> DNA
   <213> human
<400> 158
   taacaatcca tgatgctgtt ttgcatattt tgatgaagaa agattacagt acttcaaagt     60
   ttaatcccag tcaggaaaaa gatggaaaaa ttgattttac cataaataca aatggaggat    120
   tacgtaatcg ggtatatgag gtgccagttg aaacaaaatt ctaatcaaca tataattcag    180
   aaggatcttc atctgactat gacataaaaa caactttata cccagaaagt tattgataag    240
   ttcatacatt gtacgaagag tatttttgac agaatatgtt tcaaactttg gaacaagatg    300
   gttctagcat ggcatatttt tcacatatct agtatgaaat tatataagta ttctaaattt    360
   tatatcttgt agctttatca aagggtgaaa attattttgt tcatacatat ttttgtagca    420
   ctgacagatt tccatcctag tcactacctt catgcatagg tttagcagta tagtggcgcc    480
   actgttttga atct                                                      494
<210> 159
   <211> 469
   <212> DNA
   <213> human
<400> 159
   tcatcaggct ccagttattc tccatctccc agctcagctt tttctgtctg taagcctgat     60
   tttcaggaag gctctttcct agtgatggag atgaccacca tcagctccag gcttctatcc    120
   tgctaaccca gtaacccagt gggaagagat ttacttattc caataattcc aagtggagag    180
   tgtcattgac ccgtttgggg tctcatctct acttctaggg gaatgaaaca ctttgagtgg    240
   ccaggcctgt gtcatgtgct aattcctaga gccagggaaa taaggtctga ggattcagga    300
   tggggtgaaa ggtggttgct taaaggaaaa tgaaatacaa ttagcagaat aaggggaaac    360
   gagtggtctg ctctgctcgg gcaaaacaag agatgcccat tactgtgagg gacccttgaa    420
   gtctggactc ttaaatgggt ttttgctgat ttcctgggtg catgctagg                469
<210> 160
   <211> 269
   <212> DNA
   <213> human
<400> 160
   accagcaaca acttcaatcc ggtggagaac aaagatattg tggttttaac ctgtcaacct     60
   gagactcaga acacaaccta cctgtggtgg gtaaacaatc agagcctcct ggtcagtccc    120
   aggctgctgc tctccactga caacaggacc ctcgttctac tcagcgccac aaagaatgac    180
   ataggaccct atgaatgtga aatacagaac ccagtgggtg ccagccgcag tgacccagtc    240
   accctgaatg tccgctatga gtcagtaca                                      269
<210> 161
   <211> 513
   <212> DNA
   <213> human
<400> 161
   atattagtta ccctggtgtg ctgtattctc taaaaccttt aaatgtttgc atgcagccat     60
   tcgtcaaatg tcaaatattc tctctttggc tggaatgaca aaaactcaaa taaatgtatg    120
   attaggagga catcataacc tatgaatgat ggaagtccaa aatgatggta actgacagta    180
   gtgttaatgc cttatgttta gtcaaactct catttaggtg acagcctggt gactccagaa    240
   tggagccagt catgctaaat gccatatact cacactgaaa catgaggaag caggtagatc    300
   ccagaacaga caaaactttc ctaaaaacat gagagtccag gctgtctgag tcagcacagt    360
   aagaaagtcc tttctgcttt aactcttaga aaaaagtaat atgaagtatt ctgaaattaa    420
   ccaatcagtt tatttaaatc aatttattta tattcttctg ttcctggatt cccattttac    480
   aaaacccact gttctactgt tgtattgccc agt                                 513
<210> 162
   <211> 355
   <212> DNA
   <213> human
<400> 162
   cccaggaccc tgaagacgaa attgcggtgc tggcctttct ggtggaggct ggaacccagg     60
   tgaacgaggg cttccagcca ctggtggagg cactgtctaa tatccccaaa cctgagatga    120
   gcactacgat ggcagagagc agcctgttgg acctgctccc caaggaggag aaactgaggc    180
   actacttccg ctacctgggc tcactcacca caccgacctg cgatgagaag gtcgtctgga    240
   ctgtgttccg ggagcccatt cagcttcaca gagaacagat cctggcattc tctcagaagc    300
   tgtactacga caaggaacag acagtgagca tgaaggacaa tgtcaggccc ctgca         355
<210> 163
   <211> 283
   <212> DNA
   <213> human
<400> 163
   accacaccga cctgcgatga gaaggtcgtc tggactgtgt tccgggagcc cattcagctt     60
   cacagagaac agatcctggc attctctcag aagctgtact acgacaagga acagacagtg    120
   agcatgaagg acaatgtcag gcccctgcag cagctggggc agcgcacggt gataaagtcc    180
   ggggccccgg gtcggccgct gccctgggcc ctgcctgccc tgctgggccc catgctggcc    240
   tgcctgctgg ccggcttcct gcgatgatgg ctcacttctg cac                      283
<210> 164
   <211> 337
   <212> DNA
   <213> human
<400> 164
   actgactgga cgcacgtgga aaggagctat ttttggaggc tttaagagta aagaatctgt     60
   cccgaaactt gtggctgact ttatggctaa gaagttttca ctggatgcat taataacaaa    120
   tattttacct tttgaaaaaa taaatgaagg atttgacctg cttcgctctg gaaagagtat    180
   ccgtaccgtc ctgacgtttt gaaacaatac agatgccttc ccttgtagca gttttcagcc    240
   tcctctaccc tacatgatct ggagcaacag ctaggaaata tcattaattc tgctcttcag    300
   agatgttaaa aataaattac acgtgggagc tttccaa                             337
<210> 165
   <211> 498
   <212> DNA
   <213> human
<400> 165
   gttggtcact gttatttgcc tactgctgga agaaggacaa ccgctggcaa ggtagcgttc     60
   cccaatctga atacctgcag gctcccacat gagggagagg gcagactcag gtgggaagat    120
   gtgccatgcg taaggcatca acgtgtatct gtgggatctt cgttgccttc agtaatcagg    180
   gtgtgaaaaa agcagacaag ttgtgtgtgt gtgtgtgtgt ctaagaaaac ttgtgtgctt    240
   ttcaaaaagg cagtgctaag cacaagattt caagaaagcc tcttcttgtt gcctagctga    300
   gtgggagagt cattttcccc agacactaca tttggataca ggtgccaaag aacattatta    360
   aggaattatt tagaaacaat gtgtctagtt taagaaagtg gttttcagta ttgtgacaat    420
   acaacgtttt tacaaggttg ttttctacca ccatatttta aagatatttt tatgaccgtg    480
   tatactcaca ctttgctt                                                  498
<210> 166
   <211> 489
   <212> DNA
   <213> human
<400> 166
   aaagcccttc atctaatatt tgttgctatt gccaattttt caatgaaatg acctaaaaac     60
   aacaaaaaaa aataacctat acggtagttg ctttaggggg tggggggatg ctatctgtta    120
   gtgcttaaaa gggggtaaat gcttgccgct ttagaggtgg atggtgctca taaaaggccc    180
   cagtcggggg tatttaaaaa ggactgaaca gaaatcctta gctagtagaa tggcagcacg    240
   ctgtaaaatt attactgtat tgtgtactgg ctataagatg tagacacctt tcagtaagcc    300
   aatcatttgt aaccattcta gcagtgtcat attaggttaa taaggctgct gtgttttaaa    360
   gggcattttt atttgggttt tggtgaaatt ctttaatttg ttgattatat tcacataaaa    420
   tcagcattca ttgacacata gctctaatga catatgtatg aaaaaccata cactggatga    480
   cctagtcga                                                            489
<210> 167
   <211> 556
   <212> DNA
   <213> human
<400> 167
   ctgatggttc tttctctgat gagatgaaca ccattcttga taatcttgcc gccagggact     60
   ttataaactg gttgattcag accaaaatca ctgacaggaa ataactatat cactattcaa    120
   gatcatcttc acaacatcac ctgctagcca cgtgggatgt ttgaaatgtt aagtcctgta    180
   aatttaagag gtgtattctg aggccacatt gctttgcatg ccaataaata aattttcttt    240
   tagtgttgtg tagccaaaaa ttacaaatgg aataaagttt tatcaaaata ttgctaaaat    300
   atcagcttta aaatatgaaa gtgctagatt ctgttatttt cttcttattt tggatgaagt    360
   accccaacct gtttacattt agcgataaaa ttatttttct atgatataat ttgtaaatgt    420
   aaattattcc gatctgacat atctgcatta taataatagg agaatagaag aactggtagc    480
   cacagtggtg aaattggaaa gagaactttc ttcctgaaac ctttgtctta aaaatactca    540
   gctttcaatg tatcaa                                                    556
<210> 168
   <211> 244
   <212> DNA
   <213> human
<400> 168
   gccggctcct gcaagtgcaa aaagtgcaaa tgcacctcct gcaagaagag ctgctgctcc     60
   tgttgccccc tgggctgtgc caagtgtgcc cagggctgca tctgcaaagg ggcgtcagag    120
   aagtgcagct gctgtgcctg atgtcgggac agccctgctg tcagatgaaa acagaatgac    180
   acgtaaaatc cgaggttttt tttttctaca actccgactc atttgctaca ttcctttttt    240
   tctg                                                                 244
<210> 169
   <211> 557
   <212> DNA
   <213> human
<400> 169
   ttccctgctg gaggatccca agattaagga gattgctgca aagcacaaaa aaaccgcagc     60
   ccaggttctg atccgtttcc atatccagag gaatgtgatt gtcatcccca agtctgtgac    120
   accagcacgc attgttgaga acattcaggt ctttgacttt aaattgagtg atgaggagat    180
   ggcaaccata ctcagcttca acagaaactg gagggcctgt aacgtgttgc aatcctctca    240
   tttggaagac tatcccttca atgcagaata ttgaggttga atctcctggt gagattatac    300
   aggagattct ctttcttcgc tgaagtgtga ctacctccac tcatgtccca ttttagccaa    360
   gcttatttaa gatcacagtg aacttagtcc tgttatagac gagaatcgag gtgctgtttt    420
   agacatttat ttctgtatgt tcaactagga tcagaatatc acagaaaagc atggcttgaa    480
   taaggaaatg acaatttttt ccacttatct gatcagaaca aatgtttatt aagcatcaga    540
   aactctgcca acactga                                                   557
<210> 170
   <211> 268
   <212> DNA
   <213> human
<400> 170
   taagtccagt agtagcaaag ccccaaatca aagccagcaa gaccacagtc acaggagata     60
   aggactctgt gaacctgacc tgctccacaa atgacactgg aatctccatc cgttggttct    120
   tcaaaaacca gagtctcccg tcctcggaga ggatgaagct gtcccagggc aacaccaccc    180
   tcagcataaa ccctgtcaag agggaggatg ctgggacgta ttggtgtgag gtcttcaacc    240
   caatcagtaa gaaccaaagc gaccccat                                       268
<210> 171
   <211> 459
   <212> DNA
   <213> human
<400> 171
   tgagcctggg gttctggtgt tagaatattt ttaagtaggc tttactgaga gaaactaaat     60
   attggcatac gttatcagca acttcccctg ttcaatagta tgggaaaaat aagatgactg    120
   ggaaaaagac acacccacac cgtagaacat atattaatct actggcgaat gggaaaggag    180
   accattttct tagaaagcaa ataaacttga tttttttaaa tctaaaattt acattaatga    240
   gtgcaaaata acacataaaa tgaaaattca cacatcacat ttttctggaa aacagacgga    300
   ttttacttct ggagacatgg catacggtta ctgacttatg agctaccaaa actaaattct    360
   ttctctgcta ttaactggct agaagacatt catctatttt tcaaatgttc tttcaaaaca    420
   tttttataag taatgtttgt atctatttca tgctttact                           459
<210> 172
   <211> 482
   <212> DNA
   <213> human
<400> 172
   atttctttgg cagttttcgt gctaatgttt ttgctaagga agataagctc tgaaccatta     60
   aaggacgagt ttaaaaacac aggatcaggt ctcctgggca tggctaacat tgacctggaa    120
   aagagcagga ctggtgatga aattattctt ccgagaggcc tcgagtacac ggtggaagaa    180
   tgcacctgtg aagactgcat caagagcaaa ccgaaggtcg actctgacca ttgctttcca    240
   ctcccagcta tggaggaagg cgcaaccatt cttgtcacca cgaaaacgaa tgactattgc    300
   aagagcctgc cagctgcttt gagtgctacg gagatagaga aatcaatttc tgctaggtaa    360
   ttaaccattt cgactcgagc agtgccactt taaaaatctt ttgtcagaat agatgatgtg    420
   tcagatctct ttaggatgac tgtatttttc agttgccgat acagcttttt gtcctctaac    480
   tg                                                                   482
<210> 173
   <211> 519
   <212> DNA
   <213> human
<400> 173
   gaagacacta ccaacatgat attacgtatt gatctgacca cacacaatgt tactctagaa     60
   gaaccaatag aaatcaactg gtcatgaaga tcaccatcaa ttttagttgt caatgggaaa    120
   aaacaccagg atttaagttt cacagcactt acaattttcc ctcttcactt ggttcttgta    180
   ctctacaaaa tatagctttc ataacatcga aaagttattt tgtagcgtac atcaatgata    240
   atgctaattt tattatagta atgtgacttg gattcaattt taaggcatat ttaacaaaat    300
   ttgaatagcc ctatttatcc ttgttaagta tcagctacaa ttgtaaacta gttactaaac    360
   atgtatgtaa atagctaaga tataatttaa acgtgatttt taaattaaat aaaattttta    420
   tgtaattata tatactatat ttttctcaat gtttagcaga tttaagatat gtaacaacaa    480
   ttatttgaag atttaattac ttcttagtat gtgcattta                           519
<210> 174
   <211> 227
   <212> DNA
   <213> human
<400> 174
   attgcaattt cttaggtaac cttatattta caataaatga agattaccct caaatgctag     60
   aagctgtcta ggtccgtccg gtgtgtcaga tttcctcaga ttagatgtgc caataaccaa    120
   gtttattcag taaacaactt gtacttgttt catctggttt attactctca cccataaaca    180
   gtaatgactc tctgaccctc tggaaatatg taatgcttcc aatcttg                  227
<210> 175
   <211> 478
   <212> DNA
   <213> human
<400> 175
   ttttggacct gcggtggtgg ccaaccactg gaacttccac tggatctact ggctgggccc     60
   actcctggct ggcctgcttg ttggactgct cattaggtgc ttcattggag atgggaagac    120
   ccgcctcatc ctgaaggctc ggtgagcaga gctcgtggga ttcctgctgc tccaggtgtc    180
   ctcagctcac ctgtcccaga ctgaggacag gggagttcct gcatttcctg ccagggcaga    240
   ggcccagagg agcgaccccc tgcttccact gcttgggcct gctttctcag atagactgac    300
   tgctgaggag gctctaggtt cttggaattc ctttgtgctc atcagagacc ccagcctggg    360
   gaacacgctg cccgcactgc ccagagagca gtgcaaacac cacaacacga gcgtgtttct    420
   tgagaggaat gtccccgagt tggacaagga ggctgtttct gcacatcagc tcatttcc      478
<210> 176
   <211> 436
   <212> DNA
   <213> human
<400> 176
   gaggggtcac cgtgcaggat ggaaatttct ccttttctct ggagtcagtg aagaagctca     60
   aagacctcca ggagccccag gagcccaggg ttgggaaact caggaacttt gcacccatcc    120
   ctggtgaacc tgtggttccc atcctctgta gcaacccgaa ctttccagaa gaactcaagc    180
   ctctctgcaa ggagcccaat gcccaggaga tacttcagag gctggaggaa atcgctgagg    240
   acccgggcac atgtgaaatc tgtgcctacg ctgcctgtac cggatgctag gggggcttgc    300
   ccactgcctg cctcccctcc gcagcaggga agctcttttc tcctgcagaa agggccaccc    360
   atgatactcc actcccagca gctcaaccta ccctggtcca gtcgggagga gcagcccggg    420
   gaggaactgg gtgact                                                    436
<210> 177
   <211> 234
   <212> DNA
   <213> human
<400> 177
   tcgtctgctt cacaagctat cgctatggtg ttcgtgcgca ggccgtggcc cgccttgacc     60
   acagtgcttc tggccctgct cgtctgccta ggggcgctgg tcgacgccta ccccatcaaa    120
   cccgaggctc ccggcgaaga cgcctcgccg gaggagctga accgctacta cgcctccctg    180
   cgccactacc tcaacctggt cacccggcag cggtatggga aaagagacgg cccg          234
<210> 178
   <211> 287
   <212> DNA
   <213> human
<400> 178
   tatttgaata tgtatcgtgc cccctccggt gtctttgatc aggatgacat gtgccatttt     60
   tcagaggacg tgcagacagg ctggcattct agattacttt tcttactctg aaacatggcc    120
   tgtttgggag tgcgggattc aaaggtggtc ccaccgctgc ccctactgca aatggcagtt    180
   ttaatcttat cttttggctt ctgcagatgg ttgcaattga tccttaacca ataatggtca    240
   gtcctcatct ctgtcctgct tcataggtgc caccttgtgt gtttaaa                  287
<210> 179
   <211> 536
   <212> DNA
   <213> human
<400> 179
   tgacgcaaaa taccaccttg gcgcctacac gggagacgac gtccgcatcg tccgtgacga     60
   catgctgtgt gccgggaaca cccggaggga ctcatgccag ggcgactccg gagggcccct    120
   ggtgtgcaag gtgaatggca cctggctgca ggcgggcgtg gtcagctggg gcgagggctg    180
   tgcccagccc aaccggcctg gcatctacac ccgtgtcacc tactacttgg actggatcca    240
   ccactatgtc cccaaaaagc cgtgagtcag gcctgggttg gccacctggg tcactggagg    300
   accaacccct gctgtccaaa acaccactgc ttcctaccca ggtggcgact gccccccaca    360
   ccttccctgc cccgtcctga gtgccccttc ctgtcctaag ccccctgctc tcttctgagc    420
   cccttcccct gtcctgagga cccttcccca tcctgagccc ccttccctgt cctaagcctg    480
   acgcctgcac cgggccctcc ggccctcccc tgcccaggca gctggtggtg ggcgct        536
<210> 180
   <211> 472
   <212> DNA
   <213> human
<400> 180
   atgatcacaa aatgttgcct gttttgtttc cgaaagcttg ccaaaacagg aaagaagaag     60
   aaaagggatt agttatatca aaagcctgaa gtggaatgac caaaagatgg gactcctcct    120
   ttattccagc atggagggtt ttaaatggag gatttccttt ttcctgcgac aaaacgtctt    180
   ttcacaactt accctgttaa gtcaaaattt attttccagg aatttaatat gtactttagt    240
   tggaattatt ctatgtcaat gatttttaag ctatgaaaaa taataatata aaaccttatg    300
   ggcttatatt gaaatttatt attctaatcc aaaagttacc ccacacaaaa gttactgagc    360
   ttccttatgt ttcacacatt gtatttgaac acaaaacatt aacaactcca ctcatagtat    420
   caacattgtt ttgcaaatac tcagaatatt ttggcttcat tttgagcaga at            472
<210> 181
   <211> 334
   <212> DNA
   <213> human
<400> 181
   caagtatcgg ggctctgcta tcaaggtgca aagtccttcg tggatgcaac ctcaaccata     60
   tattctacag caccctggtg ccgtgttaac tccctcaatg gagcacacca tgtcactaca    120
   gcccgcatca atgatcagcc ctctggccca gcagatgagt catctgtcac taggcagcac    180
   cggaacatac atgcctgcaa cgtcagctat gcaaggagcc tacttgccac agtatgcaca    240
   tatgcagacg acagcggttc ctgttgagga ggcaagtggt caacagcagg tggctgtcga    300
   gacgtctaat gaccattctc catatacctt tcaa                                334
<210> 182
   <211> 475
   <212> DNA
   <213> human
<400> 182
   tggactggtg tcgagccaag actcggggcc tacgagcacg tcgacatcca gaacttctcc     60
   tccagctgga gtgatgggat ggccttctgt gccctggtgc acaacttctt ccctgaggcc    120
   ttcgactatg ggcagcttag ccctcagaac cgacgccaga acttcgaggt ggccttctca    180
   tctgcggaga tgctggtgga ctatgtgccc ctggtggagg tggacgacat gatgatcatg    240
   ggcaagaagc ctgaccccaa gtgtgtcttc acctatgtgc agtcgctcta caaccacctg    300
   cgacgccacg aactggcctc gcgcggcaag aatgtctagc ctgcccgccc gcatggccag    360
   ccagtggcaa ctgccgcccc cactctccgg gcaccgtctc ctgcctgtgc gtccgcccac    420
   cgctgccctg tctgttgcga caccctcccc cccacataca cacgcagcgt tttga         475
<210> 183
   <211> 561
   <212> DNA
   <213> human
<400> 183
   ggatgtgata gcattcctgc tggcctgcgt ggcaactgtg atatttatca tcacaaaatt      60
   ttgcctgttt tgtttccgaa agcttgccaa aacaggaaag aagaagaaaa gagattagtt     120
   atatcaaaag cctgaagtgg aatgactgaa agatgggact cctcctttat ttcagcatgg     180
   agggttttaa atggaggatt tcctttttcc tgtgacaaaa catcttttca caacttacct     240
   tgttaagaca aaatttattt tccagggatt taatacgtac tttagttgga attattctat     300
   gtcaatgatt tttaagctat gaaaaataca atggggggaa ggatagcatt tggagatata     360
   cctaatgtta aatgacgagt tactggatgc agcacgcaac atggcacatg tgtatacata     420
   tgtagctaac ccttcgttgt gcacatgtac cctaaaactt aaagtataat ttaaaaaaag     480
   caaaaaaaaa aaataccaac tctttttttt aaaccaggaa ggaaaatgtg aacatggaaa     540
   caacttctag tattggatct g                                               561
<210> 184
   <211> 512
   <212> DNA
   <213> human
<400> 184
   gccccggagt gcagctgcgg ggacccgctg ctcgaccccg gcctgccgga gcccgaggcc      60
   ccgccccctg cgggtcccga accgcttacc ctcatccctg ggcctgtcga gcccttcagc     120
   atcgtgacca tgcccgggcc ccggggtccg gcgccaccct ggctgcccag ccctattggc     180
   gaggaggagg agaatctggc ctgagatctt agagcccagc cccctaagga cagggaacca     240
   ggtccctgca cggcacccac gcaggtgtcc cggtctgcat aagcctcgta tgcctttgta     300
   aagtccacct acacttttga ccagctctcg ctgcccgcat gtgtttggcg ctgtgctagg     360
   ggcgggagtt cttccagact cttggaccag cccgccctga ccaccagctc tacttcccaa     420
   cccccactgc ctgagaggtc tctatcagtg tcctgcctga attctttcct tcaagtgaag     480
   atgtgactga ctacctcctc gagttgtcat ga                                   512
<210> 185
   <211> 450
   <212> DNA
   <213> human
<400> 185
   gaacccaggg agcgcgatgg gctgcagggc tgcgtcaggg ctcctgccag gagtggccgt      60
   ggtcctcctg ctgctgctgc agagcacaca gtcagtctac atccagtacc aaggcttccg     120
   ggtccagctg gaatccatga agaagctgag tgacctggag gcacagtggg cacccagccc     180
   ccgcctgcag gcccagagcc tcctgcccgc cgtgtgccac caccctgctc tgcctcagga     240
   ccttcagcct gtctgcgcct cgcaggaggc ttccagcatc ttcaagaccc tgaggaccat     300
   cgctaacgac gactgtgagc tgtgtgtgaa cgttgcgtgt accggctgcc tctgagatag     360
   ccctgggtac cctgagccca ccagggacac ctcgcccttc agcccaccac cctggcaggc     420
   ttccatcccc gtccatgctc aagatgggtc                                      450
<210> 186
   <211> 474
   <212> DNA
   <213> human
<400> 186
   ttattcttag cgtcactggt ctggctttca gaattaacat acaaggttgc cacacctagt 60
   tctgcccagc tttatgtctt ttattccagt attccaccaa agtttgtttt cctgcattcc 120
   agttctcaag tcttaagata aagattgtac ttgacagttt agtatatcca taaaactatt 180
   tgaggtggtt aaggttcttg ggttcatttt ccttaatact ttgctgaata ttgtagattg 240
   taggcaatga aaaagtctac taaattagga aaaccttgaa taattaggta tcctaggtaa 300
   gagcccctaa acatcaagca atctgtgagt ctgtaaagaa ataaatattt tttggattat 360
   tcttatctaa ttccacccct gttggaagat gatttctttg ttctttgcaa ctatggaagc 420
   tgtgaaaatc atcacaagtg cctctgaaag cgagtgttag gttggttaga gggt 474
<210> 187
   <211> 569
   <212> DNA
   <213> human
<400> 187
   gtgacggtgc tgaagaaggc cctggatgaa gagacgcggt cccatgaggc tcaggtccag 60
   gagatgaggc agaaacacgc acaggcggtg gaggagctca cagagcagct tgagcagttc 120
   aagagggcca aggcgaacct agacaagaat aagcagacgc tggagaaaga gaacgcagac 180
   ctggccgggg agctgcgggt cctgggccag gccaagcagg aggtggaaca taagaagaag 240
   aagctggagg cgcaggtgca gacactgagt ttttagaaaa acatatccac ggtaaccggt 300
   ccctggcaat tctgtttaca tgaaatgggg agaaagtcac cgaaatgggt gccgccggcc 360
   cccactccca attcattccc taacctgcaa acctttccaa cttctcacgt caggcctttg 420
   agaattcttt ccccctctcc tggtttccac acctcagaca cgcacagttc accaagtgcc 480
   ttctgtagtc acatgaattg aaaaggagac gctgctccca cggaggggag caggaatgct 540
   gcactgttta caccctgact gtgcttaaa 569
<210> 188
   <211> 422
   <212> DNA
   <213> human
<400> 188
   tagtggccgt gaggagcatc ttcagtaaga aggaaggttc tgacagacaa tggaactacg 60
   cctgcatgcc cacgccacag agcctcgggg aacccacgga gtgctggtgg gaggagatca 120
   acagggctgg catggaatgg taccagacgt gctccaacaa tgggctggtg gcaggattcc 180
   agagccgcta cttcgagtca gtgctggatc gggagtggca gttttactgt tgtcgctaca 240
   gcaagaggtg cccatattcc tgctggctaa caacagaata tccaggtcac tatggtgagg 300
   aaatggacat gatttcctac aattatgatt actatatccg aggagcaaca accactttct 360
   ctgcagtgga aagggatcgc cagtggaagt tcataatgtg ccggatgact gaatacgact 420
   gt 422
<210> 189
   <211> 183
   <212> DNA
   <213> human
<400> 189
   aatgtcatag acactgattt catcgacgag gaagttctta tgtccttggt gatagaaatg 60
   ggtttggacc gcatcaagga gctgcccgaa ctctggctgg ggcaaaacga gtttgatttt 120
   atgacggact tcgtgtgcaa acagcagccc agcagagtga gctgttgact cgatcgaaac 180
   ccc 183
<210> 190
   <211> 434
   <212> DNA
   <213> human
<220>
   <221> misc_feature
   <222> (158)..(158)
   <223> n is a, c, g, or t
<400> 190
   ccccccaatt gctgccaata gtggataaca tgtatcactc actgccaaaa atagaaagtg 60
   accatgaaaa ataaattcgc tggggaaggg ggctccatgc tggtgtggcc aaggctgaga 120
   gctctctctt ctctgttaca aaacgagata agcaagtntt agaattgcct taaggccaca 180
   ctggcatctc cctgaccttc tccagggaca gaagcaggag taagtttctc atcccatggg 240
   cgaccagggc catctcctcc caccagtggc ccccactcac agggagctgg caatgcccta 300
   cctgcctgtt ctccagatgg agaaacaggc tctgagattt cacaggtctt gcccaaagtc 360
   attgatttta atgattaaaa agaataaaca cagtgtttcc tgagtagcag tgattgttat 420
   gccttgctat ttta 434
<210> 191
   <211> 463
   <212> DNA
   <213> human
<400> 191
   gatgtattaa cctacctgtg aatcatatgt tgtaggaaaa gctgttccca tgtctaacag 60
   gacttgaatt caaagcatgt caagtggata gtagatctgt ggcgatatga gagggatgca 120
   gtgcctttcc ccattcattc ctgatggaat tgttatacta ggttaacatt tgtaattttt 180
   ttctagttgt aatgtgtatg tctggtaaat aggtattata ttttggcctt acaataccgt 240
   aacaatgttt gtcattttga aatacttaat gccaagtaac aatgcatgct ttggaaattt 300
   ggaagatggt tttattcttt gagaagcaaa tatgtttgca ttaaatgctt tgattgttca 360
   tatcaagaaa ttgattgaac gttctcaaac cctgtttacg gtacttggta agagggagcc 420
   ggtttgggag agaccattgc atcgctgtcc aagtgtttct tgt 463
<210> 192
   <211> 468
   <212> DNA
   <213> human
<400> 192
   agggcaagtg ttcccaaatt gacgccacct aataatcatc accacaccgg gagcagatct 60
   gaaggcacac tttgattttt ttaaggataa gaaccacaga acactgggta gtagctaatg 120
   aaattgagaa gggaaatctt agcatgcctc caaaaattca catccaatgc atactttgtt 180
   caaatttaag gttactcagg cattgatctt ttcagtgttt tttcacttag ctatgtggat 240
   tagctagaat gcacaccaaa aagatacttg agctgtatat atatatgtgt gtgtgtgtgt 300
   gtgtgtgtgt gtgtgtgcat gtatgtgcac atgtgtctgt gtgatatttg gtatgtgtat 360
   ttgtatgtac tgttattcaa aatatattta atacctttgg aaaatcttgg gcaagatgac 420
   ctactagttt tccttgaaaa aaagttgctt tgttattaat attgtgct 468
<210> 193
   <211> 531
   <212> DNA
   <213> human
<400> 193
   ccgagccgag cttactgtga gtgtggagat gttatcccac catgtaaagt cgcctgcgca      60
   ggggagggct gcccatctcc ccaacccagt cacagagaga taggaaacgg catttgagtg     120
   ggtgtccagg gccccgtaga gagacattta agatggtgta tgacagagca ttggccttga     180
   ccaaatgtta aatcctctgt gtgtatttca taagttatta caggtataaa agtgatgacc     240
   tatcatgagg aaatgaaagt ggctgatttg ctggtaggat tttgtacagt ttagagaagc     300
   gattatttat tgtgaaactg ttctccactc caactccttt atgtggatct gttcaaagta     360
   gtcactgtat atacgtatag agaggtagat aggtaggtag attttaaatt gcattctgaa     420
   tacaaactca tactccttag agcttgaatt acatttttaa aatgcatatg tgctgtttgg     480
   caccgtggca agatggtatc agagagaaac ccatcaattg ctcaaatact c              531
<210> 194
   <211> 392
   <212> DNA
   <213> human
<400> 194
   ggagctgtca ccatgacggg ggaacttgag gttaagaaca tggacatgaa gccggggtca      60
   accctgaaga tcacaggcag catcgccgat ggcactgatg gctttgtaat taatctgggc     120
   caggggacag acaagctgaa cctgcatttc aaccctcgct tcagcgaatc caccattgtc     180
   tgcaactcat tggacggcag caactggggg caagaacaac gggaagatca cctgtgcttc     240
   agcccagggt cagaggtcaa gttcacagtg acctttgaga gtgacaaatt caaggtgaag     300
   ctgccagatg ggcacgagct gacttttccc aacaggctgg gtcacagcca cctgagctac     360
   ctgagcgtaa ggggcgggtt caacatgtcc tc                                   392
<210> 195
   <211> 233
   <212> DNA
   <213> human
<400> 195
   gcgtgttttc ctcttgatcg ggaactcctg cttctccttg cctcgaaatg gaccccaact      60
   gctcctgctc gcctgttggc tcctgtgcct gtgccggctc ctgcaaatgc aaagagtgca     120
   aatgcacctc ctgcaagaag agctgctgct cctgctgccc tgtcggctgt gccaagtgtg     180
   cccagggctg catctgcaaa gggacgtcag acaagtgcag ctgctgtgcc tga            233
<210> 196
   <211> 578
   <212> DNA
   <213> human
<400> 196
   cccgaccatc gaatcttgcg aacaacacga tacttgttaa gtggctaccc caaaacgatc      60
   tgcttggtca cccgatgacc cgtgccttta tcacccatgc tggttcccat ggtgtttatg     120
   aaagcatatg caatggcgtt cccatggtga tgatgccctt gtttggtgat cagatggaca     180
   atgcaaagcg catggagact aagggagctg gagtgaccct gaatgttctg gaaatgactt     240
   ctgaagattt agaaaatgct ctaaaagcag tcatcaatga caaaagttac aaggagaaca     300
   tcatgcgcct ctccagcctt cacaaggacc gcccggtgga gccgctggac ctggccgtgt     360
   tctgggtgga gtttgtgatg aggcacaagg gcgcgccaca cctgcgcccc gcagcccacg     420
   acctcacctg gtaccagtac cattccttgg acgtgattgg tttcctcttg gccgtcgtgc     480
   tgacagtggc cttcatcacc tttaaatgtt gtgcttatgg ctaccggaaa tgcttgggga     540
   aaaaagggcg agttaagaaa gcccacaaat ccaagacc                             578
<210> 197
   <211> 509
   <212> DNA
   <213> human
<400> 197
   tgcagaggcc tatgttttca ctcctaacat gatctgtgct ggaggagaga agggcatgga     60
   tagctgtaaa ggggacagtg gtggggcctt tgctgtacag gatcccaatg acaagaccaa    120
   attctacgca gctggcctgg tgtcctgggg gccccagtgt gggacctatg ggctctacac    180
   acgggtaaag aactatgttg actggataat gaagactatg caggaaaata gcaccccccg    240
   tgaggactaa tccagataca tcccaccagc ctctccaagg gtggtgacca atgcattacc    300
   ttctgttcct tatgatattc tcattatttc atcatgactg aaagaagaca cgagcgaatg    360
   atttaaatag aacttgattg ttgagacgcc ttgctagagg tagagtttga tcatagaatt    420
   gtgctggtca tacatttgtg gtctgactcc ttggggtcct ttccccggag tacctattgt    480
   agataacact atgggtgggg cactccttt                                    509
<210> 198
   <211> 507
   <212> DNA
   <213> human
<400> 198
   gggcctaacc agccttggga gtattgactg gtcccttacc tcttatggct aagtctttga     60
   ctgtgttcat ttaccaagtt gacccagttt gtcttttagg ttaagtaaga ctcgagagta    120
   aaggcaagga ggggggccag cctctgaatg cggccacgga tgccttgctg ctgcaaccct    180
   ttccccagct gtccactgaa acgtgaagtc ctgttttgaa tgccaaaccc accattcact    240
   ggtgctgact acatagaatg gggttgagag aagatcagtt tgggcttcac agtgtcattt    300
   gaaaacgttt tttgttttgt tttgtaatta ttgtggaaaa ctttcaagtg aacagaagga    360
   tggtgtccta ctgtggatga gggatgaaca aggggatggc tttgatccaa tggagcctgg    420
   gaggtgtgcc cagaaagctt gtctgtagcg ggttttgtga gagtgaacac tttccacttt    480
   ttgacacctt atcctgatgt atggttc                                       507
<210> 199
   <211> 510
   <212> DNA
   <213> human
<400> 199
   agatgtgttt agaacctctt gtttaaaaat aatagactgc ttatcataaa atcacatctc     60
   acacatttga ggcagtggtc aaacaggtaa agcctatgat gtgtgtcatt ttaaagtgtc    120
   ggaatttagc ctctgaatac cttctccatt gggggaaaga tattcttgga accactcatg    180
   acatatctta gaaggtcatt gacaatgtat aaactaattg ttggtttgat atttatgtaa    240
   atatcagttt accatgcttt aattttgcac attcgtacta tagggagcct attggttctc    300
   tattagtctt gtgggttttc tgtttgaaaa ggagtcatgg catctgttta catttacctt    360
   atcaaaccta gaatgtgtat atttataaat gtatgtcttc attgctaggt actaatttgc    420
   agatgtcttt acatatttca atacagaaac tataacattc aatagtgtgc tgtcaaagtg    480
   tgcttagctc acctggatat acctacattg                                    510
<210> 200
   <211> 524
   <212> DNA
   <213> human
<220>
   <221> misc_feature
   <222> (314)..(320)
   <223> n is a, c, g, or t
<400> 200
   gaagggagtg ttgctcccag tccagaggcc tgattctgtt cggactgggt tctcaagaca     60
   cgaccaggtt ctcaagacac gagtcccctt gttcctcccc attaaagggg gtttgtcaga    120
   agcaagaaca gcccctctcc ccagtcacag cctgaaggga ggccccgaga gcttcctcct    180
   tccccccacc tgctccttac cttctctgcc ctgcttttta gaactgcagt tcattgtttt    240
   aagggattgg gggagggagc ctggggacac aaacctttta tacaatacaa agctttgctt    300
   tttttttttt tttnnnnnnn ccttttccct ttctcggttc tcttctctcc tctgaatggc    360
   tgaagacccc tctgccgagg gaggttgggg attgtgggac aaggtccctt ggtgctgatg    420
   gcctgaaggg gcctgagctg tgggcagatg cagttttctg tgggcttggg gaacctctca    480
   cgttgctgtg tcctggtgag cagcccgacc aataaacctg cttt                     524
<210> 201
   <211> 406
   <212> DNA
   <213> human
<400> 201
   ggctgcctgc ggatgaagga ccagtgtgac aagtgccggg agatcttgtc tgtggactgt     60
   tccaccaaca acccctccca ggctaagctg cggcgggagc tcgacgaatc cctccaggtc    120
   gctgagaggt tgaccaggaa atataacgag ctgctaaagt cctaccagtg gaagatgctc    180
   aacacctcct ccttgctgga gcagctgaac gagcagttta actgggtgtc ccggctggca    240
   aacctcacgc aaggcgaaga ccagtactat ctgcgggtca ccacggtggc ttcccacact    300
   tctgactcgg acgttccttc cggtgtcact gaggtggtcg tgaagctctt tgactctgat    360
   cccatcactg tgacggtccc tgtagaagtc tccaggaaga acccta                   406
<210> 202
   <211> 286
   <212> DNA
   <213> human
<400> 202
   agcagctgaa cgagcagttt aactgggtgt cccggctggc aaacctcacg caaggcgaag     60
   accagtacta tctgcgggtc accacggtgg cttcccacac ttctgactcg gacgttcctt    120
   ccggtgtcac tgaggtggtc gtgaagctct ttgactctga tcccatcact gtgacggtcc    180
   ctgtagaagt ctccaggaag aaccctaaat ttatggagac cgtggcggag aaagcgctgc    240
   aggaataccg caaaaagcac cgggaggagt gagatgtgga tgttgc                   286
<210> 203
   <211> 101
   <212> DNA
   <213> human
<400> 203
   cgatcaccaa tgtacctcca gaggtaactg tgctcacgaa cagccctgtg gaactgagag     60
   agcccaacgt cctcatctgt ttcatagaca agttcacccc a                        101
<210> 204
   <211> 530
   <212> DNA
   <213> human
<220>
   <221> misc_feature
   <222> (115)..(115)
   <223> n is a, c, g, or t
<220>
   <221> misc_feature
   <222> (117)..(117)
   <223> n is a, c, g, or t
<220>
   <221> misc_feature
   <222> (121)..(123)
   <223> n is a, c, g, or t
<400> 204
   gatataccat gtattcacac tttaaaaaat acacatagca gagtatcgga aagtatgtac     60
   caaaacattg atctggctac cactgggagt caggatcatg agttcatctt cttcntnctt    120
   nnnactgctt ttccaaacat tctccagtaa gcaggtacta catttataat ggaaggaatt    180
   ttttaaaaaa ttttaagctg tacactttca agtaagatct gaattctaat gctggcttgt    240
   gcctcttact atgtggtttg gtcattatag ataatgccca gtttcagttt ccccatcttt    300
   gaaaaggaga taatatgtta tctcttggga gtagttctga atatgaagtg tgttgataca    360
   agaagcactg acaatgtttc tgtttcttaa atttaaaact ggcctggttt gcctttttta    420
   tcaagagagc ttaacagata aaaaatgaaa ttagtctatt ttctacttgc cagcagagta    480
   tctgtcttat tttaggatgc agtgtgaaac ttaccattca actgaacaac               530
<210> 205
   <211> 480
   <212> DNA
   <213> human
<220>
   <221> misc_feature
   <222> (431)..(431)
   <223> n is a, c, g, or t
<400> 205
   gaaggctgga ttctatctac ataagtcctt tcaattccac cagggccaga gcagctccac     60
   cactgtgcac ttagccatga tggcaacaga aaccaagaga cacaattacg caggtattta    120
   gaagcagagg gacaaccaga aggcccttaa ctatcaccag tgcatcacat ctgcacactc    180
   tcttctccat tccctagcag gaacttctag ctcatttaac agataaagaa actgaggccc    240
   acggtttcag ctagacaatg atttggccag gcctagtaac caaggccctg tctctggcta    300
   ctccctggac cacgaggctg attcctctca tttccagctt ctcagtttct gcctgggcaa    360
   tggccagggg ccaggagtgg ggagagttgt gatggagggg agaggggtca cacccacccc    420
   ctgcctggtt ntaggctgct gcacaccaag gccctgcatc tgtctgctct gcatatatgt    480
<210> 206
   <211> 546
   <212> DNA
   <213> human
<400> 206
   agacagctgt ttagggtctt ctcccctcac ccatgctttc atcatcccct ccgcacagcc     60
   tccccgtcca ggccttctaa ccacacctac ccagggctgc cgcattcctg cactcagaag    120
   tctgcagcgg tgcctcacaa acttgattgt gcataaaaat cactggggat cttgttaata    180
   cagattctaa ctcaatagat ctgggagatc ctgcatttct aacaagctcc caggtaaggc    240
   ggaggctgct ggtgtgagga ccatgctgtg agcagcaggg cgagagtgcc cagggctgat    300
   atatattgga aatatcaccc ctgaagccat cgctggcccc cacctcctgt ggactgatgc    360
   cccagggatt cccaccccac ttctgcaacc ccaggtatcc ttcattatcc accccatccc    420
   agactcccac cccagggatt gcccgtgaag actttggcct agcaaattgt gttggttatg    480
   tgagtgttgt tttaatcaga gatgtacatg attgccaatc tgcatttctt accagtgtga    540
   ccacac                                                               546
<210> 207
   <211> 562
   <212> DNA
   <213> human
<400> 207
   atcatcagat cgactcttat acgagtaata tgcctgctat ttgaagtgta attgagaagg      60
   aaaattttag cgtgctcact gacctgcctg tagccccagt gacagctagg atgtgcattc      120
   tccagccatc aagagactga gtcaagttgt tccttaagtc agaacagcag actcagctct      180
   gacattctga ttcgaatgac actgttcagg aatcggaatc ctgtcgatta gactggacag      240
   cttgtggcaa gtgaatttgc ctgtaacaag ccagattttt taaaatttat attgtaaata      300
   ttgtgtgtgt gtgtgtgtgt gtatatatat atatatatgt acagttatct aagttaattt      360
   aaagttgttt gtgccttttt atttttgttt ttaatgcttt gatatttcaa tgttagcctc      420
   aatttctgaa caccataggt agaatgtaaa gcttgtctga tcgttcaaag catgaaatgg      480
   atacttatat ggaaattctg ctcagataga atgacagtcc gtcaaaacag attgtttgca      540
   aaggggaggc atcagtgtct tg                                              562
<210> 208
   <211> 553
   <212> DNA
   <213> human
<400> 208
   tacaccacaa tggctgagca cttcctgacg ttgctggtag tgcctgccat caagaaagat      60
   tatggttccc aggaagactt cactcaagtg tggaacacca ccatgaaagg gctcaagtgc      120
   tgtggcttca ccaactatac ggattttgag gactcaccct acttcaaaga gaacagtgcc      180
   tttcccccat tctgttgcaa tgacaacgtc accaacacag ccaatgaaac ctgcaccaag      240
   caaaaggctc acgaccaaaa agtagagggt tgcttcaatc agcttttgta tgacatccga      300
   actaatgcag tcaccgtggg tggtgtggca gctggaattg ggggcctcga gctggctgcc      360
   atgattgttt ccatgtatct gtactgcaat ctacaataag tccacttctg cctctgccac      420
   tactgctgcc acatgggaaa ctgtgaagag gcaccctggg caagcagcag tgattggggg      480
   aggggacagg atctaacaat gtcacttggg ccagaatgga cctggccttt ctgctcccag      540
   acttgggggc tag                                                        553
<210> 209
   <211> 220
   <212> DNA
   <213> human
<220>
   <221> misc_feature
   <222> (132)..(132)
   <223> n is a, c, g, or t
<220>
   <221> misc_feature
   <222> (135)..(135)
   <223> n is a, c, g, or t
<220>
   <221> misc_feature
   <222> (159)..(159)
   <223> n is a, c, g, or t
<220>
   <221> misc_feature
   <222> (162)..(162)
   <223> n is a, c, g, or t
<220>
   <221> misc_feature
   <222> (172)..(172)
   <223> n is a, c, g, or t
<220>
   <221> misc_feature
   <222> (174)..(174)
   <223> n is a, c, g, or t
<220>
   <221> misc_feature
   <222> (180)..(180)
   <223> n is a, c, g, or t
<400> 209
   gcagttatgg gcaaccctaa ggtgaaggct catggcaaga aagtgctcgg tgcctttagt     60
   gatggcctgg ctcacctgga caacctcaag ggcacctttg ccacactgag tgagctgcac    120
   tgtgacaagc tncangtgga tcctgagaac ttcaggctnc tnggcaacgt gntngtctgn    180
   gtgctggccc atcactttgg caaagaattc accccaccag                          220
<210> 210
   <211> 417
   <212> DNA
   <213> human
<400> 210
   tctctgggct ccaggctgag gacgaggctg attattactg ctgctcatat gcaggtagtt     60
   acactgtggt tttcggcgga gggaccaaac tgaccgtcct aggtcagccc aaggctgccc    120
   cctcggtcac tctgttcccg ccctcctctg aggagcttca agccaacaag gccacactgg    180
   tgtgtctcat aagtgacttc tacccgggag ccgtgacagt ggcctggaag gcagatagca    240
   gccccgtcaa ggcgggagtg gagaccacca caccctccaa acaaagcaac aacaagtacg    300
   cggccagcag ctatctgagc ctgacgcctg agcagtggaa gtcccacaga agctacagct    360
   gccaggtcac gcatgaaggg agcaccgtgg agaagacagt ggcccctaca gaatgtt       417
<210> 211
   <211> 532
   <212> DNA
   <213> human
<400> 211
   tttggtgcag ctgctagtca gtccctgact gacattgcca agtattcaat aggcagactg     60
   cggcctcact tcttggatgt ttgtgatcca gattggtcaa aaatcaactg cagcgatggt    120
   tacattgaat actacatatg tcgagggaat gcagaaagag ttaaggaagg caggttgtcc    180
   ttctattcag gccactcttc gttttccatg tactgcatgc tgtttgtggc actttatctt    240
   caagccagga tgaagggaga ctgggcaaga ctcttacgcc ccacactgca atttggtctt    300
   gttgccgtat ccatttatgt gggcctttct cgagtttctg attataaaca ccactggagc    360
   gatgtgttga ctggactcat tcagggagct ctggttgcaa tattagttgc tgtatatgta    420
   tcggatttct tcaaagaaag aacttctttt aaagaaagaa aagaggagga ctctcataca    480
   actctgcatg aaacaccaac aactgggaat cactatccga gcaatcacca gc            532
<210> 212
   <211> 361
   <212> DNA
   <213> human
<400> 212
   gcacgccatc aatgccatcg tgcgcagccc gcgtggcggg gcccggaggc acgcagagct     60
   gtccttcgtg ttcctcacgg acggcgtcac gggcaacgac agtctgcacg agtcggcgca    120
   ctccatgcgc aacgagaacg tggtacccac cgtcctggcc ttgggcagcg acgtggacat    180
   ggacgtgctc accacgctca gcctgggtga ccgcgccgcc gtgttccacg agaaggacta    240
   tgacagcctg gcgcaacccg gcttcttcga ccgcttcatc cgctggatct gctagcgccg    300
   ccgcccgggc cccgcagtcg agggtcgtga gcccaccccg tccatggtgc taagcgggcc    360
   c                                                                    361
<210> 213
   <211> 498
   <212> DNA
   <213> human
<400> 213
   tgtttcctca tagctagttc tcaagctgca tgtaagattt taacgggaag agaaaatagg      60
   cctggacctg aaggtctcaa atatgttgag aagaaagtat gaactataag gaacttgaga     120
   tgtagattta ttttgcagga aatacgagga aaataggaaa gaagtgtttg ccgcatcaag     180
   tgtaaagaat gatactgagg atttttacat tttatgaaat gaaataatgg catttacaaa     240
   atgaaaaatg tagtttcaca actaagtttt gttaacagag actgcatgct ttgcttatag     300
   ttcttaattt tggttttgac attcatttaa ttttttccat gttaaatatg tagtttaatt     360
   atttactcaa aataaacatt gttcatgctt ttaggccttt gggggaattg atttttatcc     420
   acaggtagaa aatggtcttt gcacacacta cacttatttc aaatatacaa tgtgctcccg     480
   aactttcgca ttagtctt                                                   498
<210> 214
   <211> 491
   <212> DNA
   <213> human
<400> 214
   gaaatatcac tgacactcca gactaaagca gagtctaggt ttctgcaatt ttgttacagt      60
   aatttgtaaa tagctttagt aaactcacct tgcatggtag attaataaga tgacttactg     120
   tacatgaatt acacaataat gagatctggt ggctatttcc acattttgaa aaggattcag     180
   ttatttactg acagtggtga gcatcctttt taaaataatg ttctgatact taaacattag     240
   agagcagtat ctttaaatga attattaaca ctttggaata cttacatttt ctgttatttt     300
   tgattgcctg ataaccagtt tcaatgatga aaatgaaaac aagtgctgaa gatgaaatgg     360
   aagagaaccg ttttaatctg gattttgttt tgtcacacct ggaaaatact ttgcaaatat     420
   gttctaaatt gaaaacaatt tttttatgat cacatggttc actaccaaat gaccctcaaa     480
   taagccagat g                                                          491
<210> 215
   <211> 389
   <212> DNA
   <213> human
<400> 215
   gatagtcgga atagagccgc cccaactcag atcctacaac acgcaagttc cttcttgaac      60
   cctggtgcct cctaccctat ggccctgaat ggtgcactgg tttaattgtg ttggtgtcgg     120
   cccctcacaa atgcagccaa gtcatgtaat tagtcatctg gaacaaagac taaaaacagc     180
   agagaattgc gggttctacc cagtcagaag atcacaccat ggagactttc tactagagga     240
   cttgaaagag aactgagggg ccacaaaata aacttcacct tccattaagt gttcaagcat     300
   gtctgcaaat taggagggag ttagaaacag tctttttcat cctttgtgat gaagcctgaa     360
   attgtgccgt gttgccttat atgaatatg                                       389
<210> 216
   <211> 515
   <212> DNA
   <213> human
<400> 216
   tgtctccccg ctatctaaaa aagtataaga acaagcagat aacagcgaga atcttggagg      60
   cccatcagaa tgtagctcag atgagtctaa ttgaagccaa gatgagattt attcaagctt     120
   ggcagtcact acctgaattt ggcatcactc acttcattgc aaggttccaa gggggcaaaa     180
   aagaagaact tattggaatt gcatacaaca gactgattcg gatggatgcc agcactggag     240
   atgcaattaa aacatggcgt ttcagcaaca tgaaacagtg gaatgtcaac tgggaaatca     300
   aaatggtcac cgtagagttt gcagatgaag tacgattgtc cttcatttgt actgaagtag     360
   attgcaaagt ggttcatgaa ttcattggtg gctacatatt tctctcaaca cgtgcaaaag     420
   accaaaacga gagtttagat gaagagatgt tctacaaact taccagtggt tgggtgtgaa     480
   tagaaatact gtttaatgaa actccacggc cataa                                515
<210> 217
   <211> 536
   <212> DNA
   <213> human
<220>
   <221> misc_feature
   <222> (26).. (26)
   <223> n is a, c, g, or t
<220>
   <221> misc_feature
   <222> (64)..(64)
   <223> n is a, c, g, or t
<220>
   <221> misc_feature
   <222> (124)..(124)
   <223> n is a, c, g, or t
<400> 217
   aaaatgctat tagtccgtcg tgcttnattt gtttttgtcc ttgaataagc atgttatgta     60
   tatngtctcg tgtttttatt tttacaccat attgtattac acttttagta ttcaccagca    120
   taancactgt ctgcctaaaa tatgcaactc tttgcattac aatatgaagt aaagttctat    180
   gaagtatgca ttttgtgtaa ctaatgtaaa aacacaaatt ttataaaatt gtacagtttt    240
   ttaaaaacta ctcacaacta gcagatggct taaatgtagc aatctctgcg ttaattaaat    300
   gcctttaaga gatataatta acgtgcagtt ttaatatcta ctaaattaag aatgacttca    360
   ttatgatcat gatttgccac aatgtcctta actctaatgc ctggactggc catgttctag    420
   tctgttgcgc tgttacaatc tgtattggtg ctagtcagaa aattcctagc tcacatagcc    480
   caaaagggtg cgagggagag gtggattacc agtattgttc aataatccat ggttca        536
<210> 218
   <211> 514
   <212> DNA
   <213> human
<400> 218
   gaccagttct tcggagagca cctgttggag tctgatcttt tcccgacgtc tacttccctg     60
   agtcccttct accttcggcc accctccttc ctgcgggcac ccagctggtt tgacactgga    120
   ctctcagaga tgcgcctgga gaaggacagg ttctctgtca acctggatgt gaagcacttc    180
   tccccagagg aactcaaagt taaggtgttg ggagatgtga ttgaggtgca tggaaaacat    240
   gaagagcgcc aggatgaaca tggtttcatc tccagggagt tccacaggaa ataccggatc    300
   ccagctgatg tagaccctct caccattact tcatccctgt catctgatgg ggtcctcact    360
   gtgaatggac caaggaaaca ggtctctggc cctgagcgca ccattcccat cacccgtgaa    420
   aagaagcctg ctgtcaccgc agcccccaag aaaaagatgc cctttcttga attgcatttt    480
   ttaaaacaag aaagtttccc caccagtgaa tgaa                                514
<210> 219
   <211> 293
   <212> DNA
   <213> human
<400> 219
   tgaatcttcg ggtgtttccc tttagctaag cacagatcta ccttggtgat ttggaccctg     60
   gttgctttgt gtctagtttt ctagaccctt catctcttac ttgatagact tactaataaa    120
   atgtgaagac tagaccaatt gtcatgcttg acacaactgc tgtggctggt tggtgctttg    180
   tttatggtag tagtttttct gtaacacaga atataggata agaaataaga ataaagtacc    240
   ttgactttgt tcacagcatg tagggtgatg agcactcaca attgttgact aaa           293
<210> 220
   <211> 398
   <212> DNA
   <213> human
<400> 220
   ctgtgtatcc ttcaaagacc cagcccctgc agcaccataa cctcctggtc tgttctgtga    60
   gtggtttcta tccaggcagc attgaagtca ggtggttccg gaatggccag gaagagaaga   120
   ctggggtggt gtccacaggc ctgatccaca atggagactg gaccttccag accctggtga   180
   tgctggaaac agttcctcgg agtggagagg tttacacctg ccaagtggag cacccaagcg   240
   tgacaagccc tctcacagtg gaatggagag cacggtctga atctgcacag agcaagatgc   300
   tgagtggagt cgggggcttt gtgctgggcc tgctcttcct tggggccggg ctgttcatct   360
   acttcaggaa tcagaaagga cactctggac ttcagcca                           398
<210> 221
   <211> 396
   <212> DNA
   <213> human
<220>
   <221> misc_feature
   <222> (148)..(149)
   <223> n is a, c, g, or t
<220>
   <221> misc_feature
   <222> (151)..(154)
   <223> n is a, c, g, or t
<220>
   <221> misc_feature
   <222> (156)..(156)
   <223> n is a, c, g, or t
<400> 221
   agtcaaatgc caaacactag ctctgtatta atccccatca ttactggtaa agcctcattt    60
   gaatgtgtga attcaataca ggctatgtaa aatttttact aatgtcatta ttttgaaaaa   120
   ataaatttaa aaatacattc aaaattanna nnnnanacaa gcttaattgt taatattccc   180
   taaacacaat tttatgaagg gagaagacat tggtttgttg acaataacag tacatctttt   240
   caagttctca gctatttctt ctacctctcc ctatcttaca tttgagtatg gtaacttatg   300
   tcatctatgt tgaatgtaag cttataaagc acaaagcata catttcctga ctggtctaga   360
   gaactgatgt ttcaatttac ccctctgcta aataaa                             396
<210> 222
   <211> 396
   <212> DNA
   <213> human
<400> 222
   ggcaaactgc ttaatcttgt ggattttgta gatggtttca aatgactgaa ctgcattcag    60
   atttacgagt gaaaggaaaa attgcattag ttggttgcat gaactttgaa gggcagatat   120
   tactgcacaa actgccatct cgcttcattt ttttaactat gcatttgagt acagactaat   180
   ttttaaaata tgctaaactg gaagattaaa cagatgtggc ccaaactgtt ctggatcagg   240
   aaagtcatac tgttcacttt caagttggct gtcccccccg ccgccccccc ccacccccat      300
   atgtacagat gataataggg tgtggaatgt cgtcagtggc aaacatttca cagattattt   360
   tgtttctgtc ttcaacattt ttgacactgt gctaat                             396
<210> 223
   <211> 479
   <212> DNA
   <213> human
<400> 223
   ctcctccatg agtctgacat ctcggaaact gagcagctgc cggacgcctg ggtcaggaat     60
   ccaagacccc acctcttaag gactggttcc tcagaaagca ccctcaggga aaaaggtgaa    120
   aacattacat ccgtggattc tcctgccaca accgcattgg aagaaaaggc tgccgcaaca    180
   tctcagcgag gagtgaagga cccatgtccc aggaaccgcg ctgcgccacc tgcactcacc    240
   cccctcacat tctcttaagc acccggtggc cctccgaggc ctggcggaat ggtggtgccc    300
   acggggttgg gcaagggctc accaggacct caacgggcaa agttgtgcac actaaaatat    360
   caaatcaagg tgcttggttt taaagtaaat gtttttctaa agaaagctgt gttcttctgt    420
   tgacccagac gaatagggca cagccctgta actgcacgtg ccttctgtca ttgggaatg     479
<210> 224
   <211> 259
   <212> DNA
   <213> human
<400> 224
   caccatcacg tgcctggtga cgggcttctc tcccgcggac gtcttcgtgc agtggatgca     60
   gagggggcag cccttgtccc cggagaagta tgtgaccagc gccccaatgc ctgagcccca    120
   ggccccaggc cggtacttcg cccacagcat cctgaccgtg tccgaagagg aatggaacac    180
   gggggagacc tacacctgcg tggtggccca tgaggccctg cccaacaggg tcaccgagag    240
   gaccgtggac aagtccacc                                                 259
<210> 225
   <211> 340
   <212> DNA
   <213> human
<220>
   <221> misc_feature
   <222> (208)..(208)
   <223> n is a, c, g, or t
<220>
   <221> misc_feature
   <222> (262)..(262)
   <223> n is a, c, g, or t
<400> 225
   ttctttttta tattgtcctc cacctccatc attttcaata aaagataggg cttttgctcc     60
   cttgttcttg gagggaccat tattacatct ctgaactacc tttgtatcca acatgtttta    120
   aatccttaaa tgaattgctt tctcccaaaa aaagcacaat ataaagaaac acaagattta    180
   attatttttc tacttggggg gaaaaaangt cctcatgtag aagcacccac ttttgcaatg    240
   ttgttctaag ctatctatct anctctcagc ccatgataaa gttccttaag ctggtgattc    300
   ctaatcaagg acaagccacc ctagtgtctc atgtttgtat                          340
<210> 226
   <211> 505
   <212> DNA
   <213> human
<400> 226
   ggaagcacaa tttccacctt attttttgaa ctttggcagt ttcaatgtct gtctctgttg     60
   cttcggggca taagctgatc accgtctagt tgggaaagta accctacagg gtttgtaggg    120
   acatgatcag catcctgatt tgaaccctga aatgttgtgt agacaccctc ttgggtccaa    180
   tgaggtagtt ggttgaagta gcaagatgtt ggcttttctg gatttttttt gccatgggtt    240
   cttcactgac cttggacttt ggcatgattc ttagtcatac ttgaacttgt ctcattccac    300
   ctcttctcag agcaactctt cctttgggaa aagagttctt cagatcatag accaaaaaag    360
   tcataccttc gaggtggtag cagtagattc caggaggaga agggtacttg ctaggtatcc    420
   tgggtcagtg gcggtgcaaa ctggtttcct cagctgcctg tccttctgtg tgcttatgtc    480
   tcttgtgaca attgttttcc tccct                                          505
<210> 227
   <211> 445
   <212> DNA
   <213> human
<400> 227
   agagaaccca ccatggtgct gtctcctgcc gacaagacca acgtcaaggc cgcctggggt     60
   aaggtcggcg cgcacgctgg cgagtatggt gcggaggccc tggagaggat gttcctgtcc    120
   ttccccacca ccaagaccta cttcccgcac ttcgacctga gccacggctc tgcccaggtt    180
   aagggccacg gcaagaaggt ggccgacgcg ctgaccaacg ccgtggcgca cgtggacgac    240
   atgcccaacg cgctgtccgc cctgagcgac ctgcacgcgc acaagcttcg ggtggacccg    300
   gtcaacttca agctcctaag ccactgcctg ctggtgaccc tggccgccca cctccccgcc    360
   gagttcaccc ctgcggtgca cgcctccctg gacaagttcc tggcttctgt gagcaccgtg    420
   ctgacctcca aataccgtta agctg                                          445
<210> 228
   <211> 499
   <212> DNA
   <213> human
<400> 228
   tggaggaatt tcacaagcac ccgcccgtgc agtgggcctt ccaggagacc agtgtggaga     60
   gcgccgtgga cacgcccttc ccagctggaa tatttgtgag gctggaattt aagctgcagc    120
   agacaagctg ccggaagagg gactggaaga aacccgagtg caaagtcagg cccaatggga    180
   ggaaacggaa atgcctggcc tgcatcaaac tgggctctga ggacaaagtt ctgggccggt    240
   tggtccactg ccccatagag acccaagttc tgcgggaggc tgaggagcac caggagaccc    300
   agtgcctcag ggtgcagcgg gctggtgagg acccccacag cttctacttc cctggacagt    360
   tcgccttctc caaggccctg ccccgcagct aagccagcac tgagctgcgt ggtgcctcca    420
   ggaccgctgc cggtggtaac cagtggaaga ccccagcccc cagggagagg accccgttct    480
   atccccagcc atgataata                                                 499
<210> 229
   <211> 465
   <212> DNA
   <213> human
<400> 229
   tttgtcagga aaccttggcc tctgctaagg tgtatttggt ccttgagaag tgggagcacc     60
   ctacagggac actatcactc atgctggtgg cattgtttac agctagaaag ctgcactggt    120
   gctaatgccc cttggggaaa tggggctgtg aggaggagga ttataactta ggcctagcct    180
   cttttaacag cctctgaaat ttatcttttc ttctatgggg tctataaatg tatcttataa    240
   taaaaaggaa ggacaggagg aagacaggca aatgtacttc tcacccagtc ttctacacag    300
   atggaatctc tttggggcta agagaaaggt tttattctat attgcttacc tgatctcatg    360
   ttaggcctaa gaggctttct ccaggaggat tagcttggag ttctctatac tcaggtacct    420
   ctttcagggt tttctaaccc tgacacggac tgtgcatact ttccc                    465
<210> 230
   <211> 526
   <212> DNA
   <213> human
<400> 230
   actggatgcg ttaataaccc atgttttacc ttttgaaaaa ataaatgaag gatttgacct     60
   gcttcactct gggaaaagta tccgtaccgt cctgacgttt tgaggcaata gagatgcctt    120
   cccctgtagc agtcttcagc ctcctctacc ctacaagatc tggagcaaca gctaggaaat    180
   atcattaatt cagctcttca gagatgttat caataaatta cacatggggg ctttccaaag    240
   aaatggaaat tgatgggaaa ttatttttca ggaaaattta aaattcaagt gagaagtaaa    300
   taaagtgttg aacatcagct ggggaattga agccaacaaa ccttccttct taaccattct    360
   actgtgtcac ctttgccatt gaggaaaaat attcctgtga cttcttgcat ttttggtatc    420
   ttcataatct ttagtcatcg aatcccagtg gaggggaccc ttttacttgc cctgaacata    480
   cacatgctgg gccattgtga ttgaagtctt ctaactctgt ctcagt                   526
<210> 231
   <211> 555
   <212> DNA
   <213> human
<400> 231
   gcagatttct tgcttcatat gacaaagcct caattactaa ttgtaaaaac tgaactattc     60
   ccagaatcat gttcaaaaaa tctgtaattt ttgctgatcg aaagtgcttc attgactaaa    120
   cagtattagt ttgtggctat aaatgattat ttagatgatg actgaaaatg tgtataaagt    180
   aattaaaagt aatatggtgg ctttaagtgt agagatggga tggcaaatgc tgtgaatgca    240
   gaatgtaaaa ttggtaacta agaaatggca caaacacctt aagcaatata ttttcctagt    300
   agatatatat atacacatac atatatacac atatacaaat gtatattttt gcaaaattgt    360
   tttcaatcta gaacttttct attaactacc atgtcttaaa atcaagtcta taatcctagc    420
   attagtttaa tattttgaat atgtaaacac ctgtgttaat gctttgttaa tgcttttccc    480
   actctcattt gttaatgctt tcccactctc gggaaggatt tgcattttga gctttatctc    540
   taaatgtgac atgca                                                     555
<210> 232
   <211> 453
   <212> DNA
   <213> human
<400> 232
   ggatgccgct ctgtgacaaa tgtggcagtg gcatagtcgg tgctgtggtg aaggcgcggg     60
   ataagtaccg gcaccctgag tgcttcgtgt gtgccgactg caacctcaac ctcaagcaaa    120
   agggctactt cttcatagaa ggggagctgt actgcgaaac ccacgcaaga gcccgcacaa    180
   aacccccaag aggctatgac acggtcactc tgtatcccaa agcttaagtc tctgcaggcg    240
   tggcacacgc acgcacccac ccacgcgcac ttacacgaga agacattcat ggctttgggc    300
   agaaggattg tgcagattgt caactccaaa tctaaagtca aggctttaga cctttatcct    360
   attgtttatt gaggaaaagg aatgggaggc aaatgcctgc tatgtgaaaa aaacatacac    420
   ttagctatgt tttgcaactc tttttggggc tag                                 453
<210> 233
   <211> 396
   <212> DNA
   <213> human
<400> 233
   ttcctgaagc tcttcggctg acagcccgct cggctcgccc tctcccccgg aggccgcgcc     60
   ctcccggaaa agccgggtcc tccagacccc gaggccttgc tctcagagcg ggaggcccca    120
   cccactggag agccccgccc ctaaggtact atgagttctc aggggtcaag ttcagaaacg    180
   gcccagccag acctaaaccc acacgcccac aaagtggatt gcacacagac aagaactccc    240
   gtgcgggcct ccactctatt cccacccttg agggagcccc cttactgggg gagggtcctt    300
   gcaattccag cgaatcggag gccaggccag gacgtccttg ctccctgcac cctcactgtt    360
   ctgtgcactt tttctaccta cataaacaca cgcatt                               396
<210> 234
   <211> 501
   <212> DNA
   <213> human
<400> 234
   gcagggctag ttattccgat ttcttgcaca attatttagc tttttgtaag ttcaacatgt     60
   aaattttaaa gacataaata tagagagact tatgtgtttg aatataaatg atatatatgg    120
   attagcatgt acctgtatat tattaaacat gcaatgaact gactggtaag tgacgtctaa    180
   ttgtatggct agcaatgtaa tttattcaga ctgtattttt gtacagagca gtgcactcta    240
   acctatgcct ctgtgtcctc tttaatgcct aaagctgtgc ctagaaattt catctgtctt    300
   aaaagtaaaa tatacttcat gctgtttatg ctattagttt ctgtactgct attctatatt    360
   tattattttt aaatatatga catgtttact acttaaacat gaattcatgg tatcctggtt    420
   atttttttta agtcatctgg gggaaaacct gtttatcact ccagtgattt tgagtttgca    480
   gtttcacaat cagttcttca t                                              501
<210> 235
   <211> 94
   <212> DNA
   <213> human
<400> 235
   caggcctttt ccactttgag ggaggtgctt cgaagaatgt tgcccacacc taagtgttag     60
   aagcctatgt ccgttcatcc ctgagaggtc tgaa                                 94
<210> 236
   <211> 508
   <212> DNA
   <213> human
<400> 236
   tggcggactc catgtttgtg atccctgcac tccaagtagc acattttcag tgttcccggg     60
   cccctgtgta cttctacgag ttccagcatc agcccagctg gctcaagaac atcaggccac    120
   cgcacatgaa ggcagaccat ggtgatgagc ttccttttgt tttcagaagt ttctttgggg    180
   gcaactacat taaattcact gaggaagagg agcagctaag caggaagatg atgaagtact    240
   gggccaactt tgcgagaaat gggaacccga atggcgaggg tctgccacac tggccgctgt    300
   tcgaccagga ggagcaatac ctgcagctga acctacagcc tgcggtgggc cgggctctga    360
   aggcccacag gctccagttc tggaagaagg cgctgcccca aaagatccag gagctcgagg    420
   agcctgaaga gagacacaca gagctgtagc tccctgtgcc ggggaggagg gggtgggttc    480
   gctgacaggc gagggtcagc ctgctgtg                                       508
<210> 237
   <211> 475
   <212> DNA
   <213> human
<400> 237
   gagagctcgc tttgagtgac tgggttttgt gattgcctct gaagcctatg tatgccatgg     60
   aggcactaac aaactctgag gtttccgaaa tcagaagcga aaaaatcagt gaataaacca    120
   tcatcttgcc actaccccct cctgaagcca cagcagggtt tcaggttcca atcagaactg    180
   ttggcaaggt gacatttcca tgcataaatg cgatccacag aaggtcctgg tggtatttgt    240
   aactttttgc aaggcatttt tttatatata tttttgtgca catttttttt tacgtttctt    300
   tagaaaacaa atgtatttca aaatatattt atagtcgaac aattcatata tttgaagtgg    360
   agccatatga atgtcagtag tttatacttc tctattatct caaactactg gcaatttgta    420
   aagaaatata tatgatatat aaatgtgatt gcagcttttc aatgttagcc acagt         475
<210> 238
   <211> 491
   <212> DNA
   <213> human
<400> 238
   tgtggtttct gtagcaacac ttctcatgac catctgtttt gtgtttatga tgattttttc      60
   aggtctgttg gtcaatctca caaccattgc atcttggctg tcatggcttc agtacttcag    120
   cattccacga tatggattta cggctttgca gcataatgaa tttttgggac aaaacttctg    180
   cccaggactc aatgcaacag gaaacaatcc ttgtaactat gcaacatgta ctggcgaaga    240
   atatttggta aagcagggca tcgatctctc accctggggc ttgtggaaga atcacgtggc    300
   cttggcttgt atgattgtta ttttcctcac aattgcctac ctgaaattgt tatttcttaa    360
   aaaatattct taaatttccc cttaattcag tatgatttat cctcacataa aaaagaagca    420
   ctttgattga agtattcaat caagtttttt tgttgttttc tgttcccttg ccatcacact    480
   gttgcacagc a                                                          491
<210> 239
   <211> 400
   <212> DNA
   <213> human
<400> 239
   gaacacgcag agagtttccc tagatatact cctgcctcca ggtgctggga cacacctttg     60
   caaaatgctg tgggaagcag gagctgggga gctgtgttaa gtcaaagtag aaaccctcca    120
   gtgtttggtg ttgtgtagag aataggacat agggtaaaga ggccaagctg cctgtagtta    180
   gtagagaaga atggatgtgg ttcttcttgt gtatttattt gtatcataaa cacttggaac    240
   aacaaagacc ataagcatca tttagcagtt gtagccattt tctagttaac tcatgtaaac    300
   aagtaagagt aacataacag tattaccctt tcactgttct cacaggacat gtacctaatt    360
   atggtactta tttatgtagt cactgtattt ctggattttt                           400
<210> 240
   <211> 467
   <212> DNA
   <213> human
<220>
   <221> misc_feature
   <222> (84)..(85)
   <223> n is a, c, g, or t
<220>
   <221> misc_feature
   <222> (136)..(137)
   <223> n is a, c, g, or t
<400> 240
   ctccagcccc agagctgaaa acaccaagtg cctatttgag ggtgtctgtc tggagactta     60
   gagtttgtca tgtgtgtgtg tgtnnttggt taatgtgggt ttatgggttt tctttctttt    120
   tttttttttt tttttnnagt ctacattagg gggaagtgag cgcctcccat gtgcagacag    180
   tgtgtcttta tagatttttc taaggctttc cccaatgatg tcggtaattt ctgatgtttc    240
   tgaagttccc aggactcaca cacccgttcc catctcactt gcccacccag tgtgacaacc    300
   ctcggtgtgg atataccccc gtggactcat ggctcttccc cacccccact ttctataaat    360
   gtaggcctag aatacgcttc tctgttgcaa aactcagcta agttcctgct tccaccttga    420
   tgttgaaata tcttatgtaa gagggcaggg gatgtcgtga agatggc                   467
<210> 241
   <211> 352
   <212> DNA
   <213> human
<400> 241
   ggaatgacac ttacttacga cccaactaca gctgctatac agaacggatt ttatccttca    60
   ccatacagta ttgctacaaa ccgaatgatc actcaaactt ctattacacc ctatattgca   120
   tctcctgtat ctgcctacca ggtgcaaagt ccttcgtgga tgcaacctca accatatatt   180
   ctacagcacc ctggtgccgt gttaactccc tcaatggagc acaccatgtc actacagccc   240
   gcatcaatga tcagccctct ggcccagcag atgagtcatc tgtcactagg cagcaccgga   300
   acatacatgc ctgcaacgtc agctatgcaa ggagcctact tgccacagta tg           352
<210> 242
   <211> 520
   <212> DNA
   <213> human
<400> 242
   ggaacgaaac cagcgtccta ttccagcgcc tctacgggcc ccaggccctc ctcttctccc    60
   tcttctggcc caccttcctg ctgaccggtg gcctcctcat tatcgccatg gtgaagagca   120
   accagtacct gtccatcctg gcggcccaga agtagagcca tccatccatg ccataccact   180
   tgtcagggca caggggactg gctgggcccc cagggctgct ccccacttgc agcacaatgc   240
   cttctccacc tgccctccca ctcttccagt ccaatccacg ctgtcttctg ttgcaggact   300
   aacctttgag aaatcctttt gtgaagtcat tgcctgctca agaatgtaca gtggctcccc   360
   aatgccttgg agccataagg ccagccagtt ctagctctct attacctgtc cccactcaac   420
   tgactcatac ctgtttccgg ctgcatcact atgtgcccca cagagaacga tgatcgtcac   480
   ctctgtgcct gagttctccc tgttgtctca aagcggtacc                         520
<210> 243
   <211> 538
   <212> DNA
   <213> human
<400> 243
   ccggtcagca ggatcatcgt gcacccacag ttctacatca tccagactgg agcggatatc    60
   gccctgctgg agctggagga gcccgtgaac atctccagcc gcgtccacac ggtcatgctg   120
   ccccctgcct cggagacctt ccccccgggg atgccgtgct gggtcactgg ctggggcgat   180
   gtggacaatg atgagcccct cccaccgcca tttcccctga agcaggtgaa ggtccccata   240
   atggaaaacc acatttgtga cgcaaaatac caccttggcg cctacacggg agacgacgtc   300
   cgcatcatcc gtgacgacat gctgtgtgcc gggaacaccc ggagggactc atgccagggc   360
   gactctggag ggcccctggt gtgcaaggtg aatggcacct ggctacaggc gggcgtggtc   420
   agctgggacg agggctgtgc ccagcccaac cggcctggca tctacacccg tgtcacctac   480
   tacttggact ggatccacca ctatgtcccc aaaaagccgt gagtcaggcc tggggtgt     538
<210> 244
   <211> 489
   <212> DNA
   <213> human
<400> 244
   ggccaaatca ccgacctgaa ggcggaaatt cacgggggca gtctcattaa tctgacttgg    60
   acagctcctg gggatgatta tgaccatgga acagctcaca agtatatcat tcgaataagt   120
   acaagtattc ttgatctcag agacaagttc aatgaatctc ttcaagtgaa tactactgct   180
   ctcatcccaa aggaagccaa ctctgaggaa gtctttttgt ttaaaccaga aaacattact   240
   tttgaaaatg gcacagatct tttcattgct attcaggctg ttgataaggt cgatctgaaa   300
   tcagaaatat ccaacattgc acgagtatct ttgtttattc ctccacagac tccgccagag   360
   acacctagtc ctgatgaaac gtctgctcct tgtcctaata ttcatatcaa cagcaccatt   420
   cctggcattc acattttaaa aattatgtgg aagtggatag gagaactgca gctgtcaata   480
   gcctagggc                                                           489
<210> 245
   <211> 461
   <212> DNA
   <213> human
<400> 245
   accccttcag cgactagaga gctacaggag aatcaccagt ggcaaatgtc cccagaaagc     60
   tgtgatcttc aagaccaaac tggccaagga tatctgtgcc gaccccaaga agaagtgggt    120
   gcaggattcc atgaagtatc tggaccaaaa atctccaact ccaaagccat aaataatcac    180
   catttttgaa accaaaccag agcctgagtg ttgcctaatt tgttttccct tcttacaatg    240
   cattctgagg taacctcatt atcagtccaa agggcatggg ttttattata tatatatata    300
   tttttttttt aaaaaaaaac gtattgcatt taatttattg aggctttaaa acttatcctc    360
   catgaatatc agttattttt aaactgtaaa gctttgtgca gattctttac cccctgggag    420
   ccccaattcg atcccctgtc acgtgtgggc aatgttcccc c                        461
<210> 246
   <211> 534
   <212> DNA
   <213> human
<400> 246
   tgtgaagctt tacgcgcaca cggacaaaat gcccaaactg gagcccttgc aaaaacacgg     60
   cttgtggcat tggcatactt gcccttacag gtggagtatc ttcgtcacac atctaaatga    120
   gaaatcagtg acaacaagtc tttgaaatgg tgctatggat ttaccattcc ttattatcac    180
   taatcatcta aacaactcac tggaaatcca attaacaatt ttacaacata agatagaatg    240
   gagacctgaa taattctgtg taatataaat ggtttataac tgcttttgta cctagctagg    300
   ctgctattat tactataatg agtaaatcat aaagccttca tcactcccac atttttctta    360
   cggtcggagc atcagaacaa gcgtctagac tccttgggac cgtgagttcc tagagcttgg    420
   ctgggtctag gctgttctgt gcctccaagg actgtctggc aatgacttgt attggccacc    480
   aactgtagat gtatatatgg tgcccttctg atgctaagac tccagacctt ttgt          534
<210> 247
   <211> 560
   <212> DNA
   <213> human
<400> 247
   tctactgcgt gacttgccat gagaccaagt ttgccaagca ttgcgtgaag tgcaacaagg     60
   ccatcacatc tggaggaatc acttaccagg atcagccctg gcatgccgat tgctttgtgt    120
   gtgttacctg ctctaagaag ctggctgggc agcgtttcac cgctgtggag gaccagtatt    180
   actgcgtgga ttgctacaag aactttgtgg ccaagaagtg tgctggatgc aagaacccca    240
   tcactgggaa aaggactgtg tcaagagtga gccgcccagt ctctaaagct aggaagcccc    300
   cagtgtgcca cgggaaacgc ttgcctctca ccctgtttcc cagcgccaac ctccggggca    360
   ggcatccggg tggagagagg acttgtccct cgtgggtggt ggttctttat agaaaaaatc    420
   gaagcttagc agctcctcgt ggcccgggtt tggtaaaggc tccagtgtgg tggcctatga    480
   aggacaatcc tggcacgact actgcttcca ctgcaaaaaa tgctccgtga atctggccaa    540
   caagcgcttt gttttccacc                                                560
<210> 248
   <211> 445
   <212> DNA
   <213> human
<400> 248
   gcataggaga taaaaccccc actgagatgc tctcatgcct cagctggacc caccgtgtag     60
   acacacgaca tgcaagagtt gcagcggctg ctccaactca ctgctcaccc tctctgtgag    120
   cagaaagaac cctactgaca tgcatggtta acttcctcat cagaactctg cccttccctt    180
   ctgttctttt gtgctttcaa ataactaaca cgaacttcca gaaaattaac atttgaactt    240
   agctgtaatt ctaaactgac ctttccccgt actaacgttt ggtttccccg tgtggcatgc    300
   ttttctgagc cttcctactt taaagcatgg aacatgcagg tgatttggga agtgtagaaa    360
   gacctgagaa aacgagcctg tttcagagga acatcgtcac aacgaatact tctggaagct    420
   taacaaaact aaccctgctg tcctt                                          445
<210> 249
   <211> 185
   <212> DNA
   <213> human
<400> 249
   atttcgtttt gcttttggtt gcctgaatgt tgtcaccaag tgaaaaaatt atttaactat     60
   atgtaaaatt tctcttttaa aaaaaagttt tactgatgtt aaacgttctc agtgccaatg    120
   tcagactgtg ctcctccctc tcctgaacct ctaccctcac cctgagctgt cttgttgaaa    180
   acagt                                                                185
<210> 250
   <211> 551
   <212> DNA
   <213> human
<400> 250
   agccctttgc tctggaggaa gttctccagc ttcagctcaa ctcacagctt ctccaagcat     60
   caccctggga gtttcctgag ggttttctca taaatgaggg ctgcacattg cctgttctgc    120
   ttcgaagtat tcaataccgc tcagtatttt aaatgaagtg attctaagat ttggtttggg    180
   atcaatagga aagcatatgc agccaaccaa gatgcaaatg ttttgaaatg atatgaccaa    240
   aattttaagt aggaaagtca cccaaacact tctgctttca cttaagtgtc tggcccgcaa    300
   tactgtagga acaagcatga tcttgttact gtgatatttt aaatatccac agtactcact    360
   ttttccaaat gatcctagta attgcctaga aatatctttc tcttacctgt tatttatcaa    420
   tttttcccag tatttttata cggaaaaaat tgtattgaaa acacttagta tgcagttgat    480
   aagaggaatt tggtataatt atggtgggtg attatttttt atactgtatg tgccaaagct    540
   ttactactgt g                                                         551
<210> 251
   <211> 518
   <212> DNA
   <213> human
<400> 251
   gtgccatagt gcaggcttgg ggagctttaa gcctcagtta tataacccac gaaaaacaga     60
   gcctcctaga tgtaacattc ctgatcaagg tacaattctt taaaattcac taatgattga    120
   ggtccatatt tagtggtact ctgaaattgg tcactttcct attacacgga gtgtgctaaa    180
   actaaaaagc attttgaaac atacagaatg ttctattgtc attgggaaat ttttctttct    240
   aacccagtgg aggttagaaa gaagttatat tctggtagca aattaacttt acatcctttt    300
   tcctacttgt tatggttgtt tggaccgata agtgtgctta atcctgaggc aaagtagtga    360
   atatgtttta tatgttatga agaaaagaat tgttgtaagt ttttgattct actcttatat    420
   gctggactgc attcacacat ggcatgaaat aagtcaggtt ctttacaaat ggtattttga    480
   tagatactgg attgtgtttg tgccatattt gtgccatt                            518
<210> 252
   <211> 419
   <212> DNA
   <213> human
<400> 252
   gtcaggactg ttatggccct gttgtgctga gttagttgga acagaattca agctccctgc 60
   actagtccac ctgccccact gcttcttcgc ttctctcttg gaaagtccag tctctcctcg 120
   gcttgcaatg gaccccaact gctcctgcgc cgctggtgtc tcctgcacct gcgctggttc 180
   ctgcaagtgc aaagagtgca aatgcacctc ctgcaagaag agctgctgct cctgctgccc 240
   cgtgggctgt agcaagtgtg cccagggctg tgtttgcaaa ggggcgtcag agaagtgcag 300
   ctgctgcgac tgatgccagg acaacctttc tcccagatgt aaacagagag acatgtacaa 360
   acctggattt tttttttata ccaccttgac ccatttgcta cattcctttt cctgtgaaa 419
<210> 253
   <211> 506
   <212> DNA
   <213> human
<400> 253
   caggaaggac cttgctttgg accctacaca cttcggctct ctggacactt gcgacacctc 60
   aaggtgttct ctgtagctca atctgcaaac atgccaggcc tcagggatcc tctgctgggt 120
   gcctccttgc cttgggacca tggccacccc agagccatcc gatcgatgga tgggatgcac 180
   tctcagacca agcagcagga attcaaagct gcttgctgta actgtgtgag attgtgaagt 240
   ggtctgaatt ctggaatcac aaaccaagcc atgctggtgg gccattaatg gttggaaaac 300
   actttcatcc ggggctttgc cagagcgtgc tttcaagtgt cctggaaagt ctgctgcttc 360
   tccaagcttt cagacaagaa tgtgcactct ctgcttaggt tttgcttggg aaactcaact 420
   tctttcctct ggagacgggg catctccctc tgatttcctt ctgctatgac aaaaccttta 480
   atctgcacct tacaactcgg ggacaa 506
<210> 254
   <211> 565
   <212> DNA
   <213> human
<400> 254
   ttaccaatga caaccctgag tgccgccttg tgaaagaaac ccggatttgt gaggtgcggc 60
   cttgtggaca gccagtgtac agcagcctga aaaagggcaa gaaatgcagc aagaccaaga 120
   aatcccccga accagtcagg tttacttacg ctggatgttt gagtgtgaag aaataccggc 180
   ccaagtactg cggttcctgc gtggacggcc gatgctgcac gccccagctg accaggacat 240
   gtgaagatgc ggttccgctg cgaagatggg gagacatttt ccaagaacgt catgatgatc 300
   cagtcctcaa atgcaactac aactgcccgc atgccaatga agcagcgttt cccttctaca 360
   ggctgttcaa tgacattcac aaatttaggg actaaatgct acctgggttt ccagggcaca 420
   cctagacaaa caagggagaa gagtgtcaga atcagaatca tggagaaaat gggcgggggt 480
   ggtgtgggtg atgggactca ttgtagaaag gaagccttgc tcattcttga ggagcattaa 540
   ggtatttcga aactgccaag ggtgc 565
<210> 255
   <211> 413
   <212> DNA
   <213> human
<400> 255
   aaattgtgga gttagcctcc tgtggagtta gcctcctgtg gtaaaggaat tgaagaaaat 60
   ataacacctt acaccctttt tcatcttgac attaaaagtt ctggctaact ttggaatcca 120
   ttagagaaaa atccttgtca ccagattcat tacaattcaa atcgaagagt tgtgaactgt 180
   tatcccattg aaaagaccga gccttgtatg tatgttatgg atacataaaa tgcacgcaag 240
   ccattatctc tccatgggaa gctaagttat aaaaataggt gcttggtgta caaaactttt 300
   tatatcaaaa ggctttgcac atttctatat gagtgggttt actggtaaat tatgttattt 360
   tttacaacta attttgtact ctcagaatgt ttgtcatatg cttcttgcaa tgc 413
<210> 256
   <211> 253
   <212> DNA
   <213> human
<400> 256
   aaggcaagag gatgcatctt tcttcctggt gtacaagcct ttaaagactt ctgctgctga     60
   tatgcctctt ggatgcacac tttgtgtgta catagttacc tttaactcag tggttatcta    120
   atagctctaa actcattaaa aaaactccaa gccttccacc aaaacagtgc cccacctgta    180
   tacattttta ttaaaaaaat gtaatgctta tgtataaaca tgtatgtaat atgctttcta    240
   tgaatgatgt ttg                                                       253
<210> 257
   <211> 445
   <212> DNA
   <213> human
<400> 257
   gaaggagacg gtctggcggc ttgaagaatt tggacgattt gccagctttg aggctcaagg     60
   tgcattggcc aacatagctg tggacaaagc caacttggaa atcatgacaa agcgctccaa    120
   ctatactccg atcaccaatg acaagttcac cccaccagtg gtcaatgtca cgtggcttcg    180
   aaatggaaaa cctgtcacca caggagtgtc agagacagtc ttcctgccca gggaagacca    240
   ccttttccgc aagttccact atctcccctt cctgccctca actgaggacg tttacgactg    300
   cagggtggag cactggggct tggatgagcc tcttctcaag cactgggagt ttgatgctcc    360
   aagccctctc ccagagacta cagagaacgt ggtgtgtgcc ctgggcctga ctgtgggtct    420
   ggtgggcatc attattggga ccatc                                          445
<210> 258
   <211> 370
   <212> DNA
   <213> human
<400> 258
   aatattggat atcaactgct tgtaaaggtg ctcctccttt ttcttgtcat tgctggtcaa     60
   gattactaat atttgggaag gctttaaaga cgcatgttat ggtgctaatg tactttcact    120
   tttaaactct agatcagaat tgttgacttg cattcagaac ataaatgcac aaaatctgta    180
   catgtctccc atcagaaaga ttcattggca tgccacaggg gattctcctc cttcatcctg    240
   taaaggtcaa caataaaaac caaattatgg ggctgctttt gtcacactag catagagaat    300
   gtgttgaaat ttaactttgt aagcttgtat gtggttgttg atcttttttt tccttacaga    360
   cacccataat                                                           370
<210> 259
   <211> 449
   <212> DNA
   <213> human
<400> 259
   atctcatacc cgcaaatttt aaccttgtca acctctgggg tattagtatg ccctgacctg     60
   agtgaattca cccgtgacaa aactgacgtg aagattcaat ggtacaagga ttctcttctt    120
   ttggataaag acaatgagaa atttctaagt gtgaggggga ccactcactt actcgtacac    180
   gatgtggccc tggaagatgc tggctattac cgctgtgtcc tgacatttgc ccatgaaggc    240
   cagcaataca acatcactag gagtattgag ctacgcatca agaaaaaaaa agaagagacc    300
   attcctgtga tcatttcccc cctcaagacc atatcagctt ctctggggtc aagactgaca    360
   atcccgtgta aggtgtttct gggaaccggc acacccttaa ccaccatgct gtggtggacg    420
   gccaatgaca cccacataga gagcgccta                                      449
<210> 260
   <211> 435
   <212> DNA
   <213> human
<400> 260
   agctcgaaag agtttgcaac cccatcatca gcaaacttta ccaaggtggt cctggcggcg     60
   gcagcggcgg cggcggttca ggagcctccg ggggacccac catcgaagaa gtggactaag    120
   cttgcactca agtcagcgta aacctctttg cctttctctc tctctctttt tttttgtttg    180
   tttctttgaa atgtccttgt gccaagtacg agatctattg ttggaagtct ttggtatatg    240
   caaatgaaag gagaggtgca acaacttagt ttaattataa aagttccaaa gtttgttttt    300
   taaaaacatt attcgaggtt tctctttaat gcattttgcg tgtttgctga cttgagcatt    360
   tttgattagt tcgtgcatgg agatttgttt gagatgagaa accttaagtt tgcacacctg    420
   ttctgtagaa gcttg                                                     435
<210> 261
   <211> 476
   <212> DNA
   <213> human
<400> 261
   gctttttttg ttgtcagcta tcttaagaat cattaaatac acctgctttg ggtaaaactc     60
   tttgcaagca gtaattaaca ctagtaacag tgaaagcaca agatttccaa atcagtcgtt    120
   ttctcaaaaa aatatcgtat aagtgactca tcctgtctgc taactccaga cctcccagct    180
   tgaagccaaa tctttccatg tgagattgat atggatttcc tagaagtact ggaatgttgt    240
   catatcttgc cctattttaa ttctgctata gaaaacaatt gccttcactt ttaaggagta    300
   atttgaatat taataactct ggtctagatt ttcatataat gtattaaaga caaagtagtg    360
   aacatcaatg aacatctgat agagataaac tgtaatcagg cataagcttg tttgtatgtt    420
   ctggcagtga ctaatcagta aatgatgtcg gtttgcccag tatcacttat cttctg         476
<210> 262
   <211> 541
   <212> DNA
   <213> human
<400> 262
   cgtgaacggc aaagtggcgc tggtgaccgg cgcggctcag ggcataggca gagcctttgc     60
   agaggcgctg ctgcttaagg gcgccaaggt agcgctggtg gattggaatc ttgaagcagg    120
   tgtacagtgt aaagctgccc tggatgagca atttgaacct cagaagactc tgttcatcca    180
   gtgcgatgtg gctgaccagc aacaactgag agacactttt agaaaagttg tagaccactt    240
   tggaagactg gacattttgg tcaataatgc tggagtgaat aatgagaaaa actgggaaaa    300
   aactctgcaa attaatttgg tttctgttat cagtggaacc tatcttggtt tggattacat    360
   gagtaagcaa aatggaggtg aaggcggcat cattatcaat atgtcatctt tagcagccca    420
   ccattgattg ccaatggatt gataacactc attgaagatg atgctttaaa tggtgctatt    480
   atgaagatca caacttctaa gggaattcat tttcaagact atgatacaac tccatttcaa    540
   g                                                                    541
<210> 263
   <211> 421
   <212> DNA
   <213> human
<400> 263
   gagtattaca ttggccttgg gggacagaaa ggaggaagtt ctgacttttc agggctacct     60
   tatttctact aaggacccag agcaggcctg tccatgccat tccttcgcac agatgaaact    120
   gagctgggac tggaaaggac agcccttgac ctgggttctg ggtataattt gcacttttga    180
   gactggtagc taaccatctt atgagtgcca atgtgtcatt tagtaaaact taaatagaaa    240
   caaggtcctt caaatgttcc tttggccaaa agctgaaggg agttactgag aaaatagtta    300
   acaattactg tcaggtgtca tcactgttca aaaggtaagc acatttagaa ttttgttctt    360
   gacagttaac tgactaatct tacttccaca aaatatgtga atttgctgct tctgagaggc    420
   a                                                                    421
<210> 264
   <211> 281
   <212> DNA
   <213> human
<400> 264
   ggactggtga agccttcgga gaccctgtcc ctcacctgca gtgtctctgg tggctccatc     60
   agtagttact actggagctg gatccggcag cccccaggga agggactgga gtggattggg    120
   tatatcaatt acagtgggag caccaactat aacccctccc tcaagagtcg agtcaccata    180
   tcagtagaca cgtccaagaa ccagttctcc ctgaagctga gctctgtgac cgctgcggac    240
   acggccgtgt attactgtgc gagaacgatt tcggggggtc g                         281
<210> 265
   <211> 293
   <212> DNA
   <213> human
<400> 265
   aatagtgatg acgcagcttc cagccaccct gtctgtgtct ccaggggaaa gagccaccct     60
   ctcctgcagg gccagtcaga gtgttcgcag caacttagcc tggtaccagc aaaaacctgg    120
   ccaggctccc aggctcctca tctacggtgc atccaccagg gccaatggta tcccagccag    180
   gttcagtggc agtgggtctg ggacagagtt cactctcacc atcagcggcc tgcagtctga    240
   cgattttgca gtttattact gtcaacacta taataactgg cctccgtgga cgt           293
<210> 266
   <211> 284
   <212> DNA
   <213> human
<400> 266
   aattgtgttg acgcagtctc caggcaccct gtctttgtct ccaggggaaa gagccaccct     60
   ctcctgcagg gccagtcaga gtattagcag cagtttctta gcctggtacc agcagaaagt    120
   tggccaggct cccaggctcc tcatctatgg tgcatccagc agggccactg gcatcccaga    180
   caggttcagt ggcagtgggt ctgggacaga cttcactctc accatcagca gactggagcc    240
   tgaagatttt gcagtgtatt actgtcagca atatggtagc tcgc                     284
<210> 267
   <211> 263
   <212> DNA
   <213> human
<400> 267
   tcctccctgt ctgcatctgt aggagacaga gtcaccatca cttgccgggc aagtcagggc     60
   attagaaatg atttagcctg gtatcagcag aaaccaggga aagcccctaa gcgcctgatc    120
   tatgctgcat ccagtttgca aagtggggtc ccatcaaggt tcagcggcag tggatctggg    180
   acagaattca ctctcacaat cagcagcctg cagcctgaag attttgcaac ttattactgt    240
   ctacagcata atagttaccc ttg                                            263
<210> 268
   <211> 458
   <212> DNA
   <213> human
<400> 268
   gcagttgagt cgtcatcact tttcagtgat gggagagtag atggtgaaat ttattagtta 60
   atatatccca gaaattagaa accttaatat gtggacgtaa tctccacagt caaagaagga 120
   tggcacctaa accaccagtg cccaaagtct gtgtgatgaa ctttctcttc atactttttt 180
   tcacagttgg ctggatgaaa ttttctagac tttctgttgg tgtatccccc ccctgtatag 240
   ttaggatagc atttttgatt tatgcatgga aacctgaaaa aaagtttaca agtgtatatc 300
   agaaaaggga agttgtgcct tttatagcta ttactgtctg gttttaacaa tttcctttat 360
   atttagtgaa ctacgcttgc tcattttttc ttacataatt ttttattcaa gttattgtac 420
   agctgtttaa gatgggcagc tagttcgtag ctttccca 458
<210> 269
   <211> 494
   <212> DNA
   <213> human
<400> 269
   tgaggagaag tctgccgtta ctgccctgtg gggcaaggtg aacgtggatg aagttggtgg 60
   tgaggccctg ggcaggctgc tggtggtcta cccttggacc cagaggttct ttgagtcctt 120
   tggggatctg tccactcctg atgctgttat gggcaaccct aaggtgaagg ctcatggcaa 180
   gaaagtgctc ggtgccttta gtgatggcct ggctcacctg gacaacctca agggcacctt 240
   tgccacactg agtgagctgc actgtgacaa gctgcacgtg gatcctgaga acttcaggct 300
   cctgggcaac gtgctggtct gtgtgccggc ccatcacttt ggcaaagaat tcacccaacc 360
   agtgcaggct gcctatcaga aagtggtggc tggtgtggct aatgccctgg cccacaagta 420
   tcactaagct cgctttcttg ctgtccaatt tctattaaag gttcctttgt tccctaagtc 480
   caactactaa actg 494
<210> 270
   <211> 445
   <212> DNA
   <213> human
<400> 270
   agagaaccca ccatggtgct gtctcctgac gacaagacca acgtcaaggc cgcctggggt 60
   aaggtcggcg cgcacgctgg cgagtatggt gcggaggccc tggagaggat gttcctgtcc 120
   ttccccacca ccaagaccta cttcccgcac ttcgacctga gccacggctc tgcccaggtt 180
   aagggccacg gcaagaaggt ggccgacgcg ctgaccaacg ccgtggcgca cgtggacgac 240
   atgcccaacg cgctgtccgc cctgagcgac ctgcacgcgc acaagcttcg ggtggacccg 300
   gtcaacttca agctcctaag ccactgcctg ctggtgaccc tggccgccca cctccccgcc 360
   gagttcaccc ctgcggtgca cgcctccctg gacaagttcc tggcttctgt gagcaccgtg 420
   ctgacctcca aataccgtta agctg 445
<210> 271
   <211> 485
   <212> DNA
   <213> human
<400> 271
   gggagtttcc tgagggtttt ctcataaatg agggctgcac attgcctgtt ctgcttcgaa 60
   gtattcaata ccgctcagta ttttaaatga agtgattcta agatttggtt tgggatcaat 120
   aggaaagcat atgcagccaa ccaagatgca aatgttttga aatgatatga ccaaaatttt 180
   aagtaggaaa gtcacccaaa cacttctgct ttcacttaag tgtctggccc gcaatactgt 240
   aggaacaagc atgatcttgt tactgtgata ttttaaatat ccacagtact cactttttcc 300
   aaatgatcct agtaattgcc tagaaatatc tttctcttac ctgttattta tcaatttttc 360
   ccagtatttt tatacggaaa aaattgtatt gaaaacactt agtatgcagt tgataagagg 420
   aatttggtat aattatggtg ggtgattatt ttttatactg tatgtgccaa agctttacta 480
   ctgtg 485
<210> 272
   <211> 435
   <212> DNA
   <213> human
<400> 272
   tcctgccgac aagaccaacg tcaaggccgc ctggggtaag gtcggcgcgc acgctggcga     60
   gtatggtgcg gaggccctgg agaggatgtt cctgtccttc cccaccacca agacctactt     120
   cccgcacttc gacctgagcc acggctctgc ccaggttaag ggccacggca agaaggtggc    180
   cgacgcgctg accaacgccg tggcgcacgt ggacgacatg cccaacgcgc tgtccgccct    240
   gagcgacctg cacgcgcaca agcttcgggt ggacccggtc aacttcaagc tcctaagcca    300
   ctgcctgctg gtgaccctgg ccgcccacct ccccgccgag ttcacccctg cggtgcacgc    360
   ctccctggac aagttcctgg cttctgtgag caccgtgctg acctccaaat accgttaagc    420
   tggagcctcg gtggc                                                      435
<210> 273
   <211> 583
   <212> DNA
   <213> human
<400> 273
   tacgcagact ccgtgaaggg ccgattcacc atctccagag acaattccaa gaacacgctg     60
   tatcttcaaa tgaacagcct gagagccgag gacacggccg tattactgtg caagagagac    120
   ttgtgtgatt ggggccaagg taccctggtc accgtgtcga gaggtggcgg tggctcgggc    180
   ggtggtgggt cgggtggcgg cggatcttct gagctgactc aggaccctgc tgtgtctgtg    240
   gccttgggac agacagtcag gatcacatgc caaggagaca gcctcagaag ctattatgca    300
   agctggtacc agcagaagcc aggacaggcc cctgtacttg tcatctatgg taaaaacaac    360
   cggccctcag ggatcccaga ccgattctct ggctccagct caggaaacac agcttccttg    420
   accatcactg gggctcaggc ggaagatgag gctgactatt actgtaactc ccgggacagc    480
   agtggtaacc atgtggtatt cggcggaggg accaagctga ccgtcctagg tgcggccgca    540
   gaacaaaaac tcatctcaga agaggatctg aatggggccg cat                      583
<210> 274
   <211> 484
   <212> DNA
   <213> human
<400> 274
   ttgcacaagt ttcctgggct ggaccgtttc aacagagagg cttatttgac tttatgctag     60
   aagatgaggc ttctgggata ggcccagaag ttcctgatga ccgcgacttc gagccctccc    120
   taggcccagt gtgccccttc cgctgtcaat gccatcttcg agtggtccag tgttctgatt    180
   tgggtctgga caaagtgcca aaggatcttc cccctgacac aactctgcta gacctgcaaa    240
   acaacaaaat aaccgaaatc aaagatggag actttaagaa cctgaagaac cttcacgttg    300
   tctaccttca taacaacaat atctctgtag ttggatcaag tgacttctgc ccacctggac    360
   acaacaccaa aaaggcttct tattcgggtg tgagtctttt cagcaacccg gtccagtact    420
   gggagataca gccatccacc ttcagatgtg tctacgtgcg ctctgccatt caactcggaa    480
   acta                                                                 484
<210> 275
   <211> 502
   <212> DNA
   <213> human
<400> 275
   tgtccataca gagtgtgcat tccctggcag gggctcctgc tcacagcctc gcttttaacc     60
   ttctggaacc tgccaaacag tgcccagacc aatattgatg gtgtgccgtt caatgtcgca    120
   gaagggaagg aggtccttct agtagtccat aatgagtccc agaatcttta tggctacaac    180
   tggtacaaag ggcaaagggt gcatgccaac tatcgaatta taggatatgt aaaaaatata    240
   agtcaagaaa atgccccagg gcccgcacac aacggtcgag agacaatata ccccaatgga    300
   accctgctga tccagaacgt cacccacaat gacgcaggaa tctataccct acacgttata    360
   aaagaaaatc ttgtgaatga agaagtaacc agacaattct acgtattcta tgagtcagta    420
   caagcaagtt cacctgacct ctcagctggg accgctgtca gcatcatgat tggagtactg    480
   gctgggatgg ctctgatata gc                                             502
<210> 276
   <211> 510
   <212> DNA
   <213> human
<400> 276
   tgcacagtac tcctggctta tcaatggaac attccagcaa agcacacaag agctctttat     60
   ccctaacatc actgtgaata atagtggatc ctatacctgc cacgccaata actcagtcac    120
   tggctgcaac aggaccacag tcaagacgat catagtcact gagagacaga atctcaccat    180
   gttgcccggg ctggactcga actcctgggc tcaagcaatc ctcccatctg tttcccaaag    240
   tgctgagatt acagataatg ctctaccaca agaaaatggc ctctcacctg gggccattgc     300
   tggcattgtg attggagtag tggccctggt tgctctgata gcagtagccc tggcatgttt    360
   tctgcatttc gggaagaccg gcagggcaag cgaccagcgt gatctcacag agcacaaacc    420
   ctcagtgtcc aaccacactc aggaccactc caatgaccca cctaacaaga tgaatgaagt    480
   tacttattct accctgaact ttgaagccca                                     510
<210> 277
   <211> 244
   <212> DNA
   <213> human
<400> 277
   tcctccttcc cttacggaat tacatcttga tggcaacaaa atcagcagag ttgatgcagc     60
   tagcctgaaa ggactgaata atttggctaa gttgggattg agtttcaaca gcatctctgc    120
   tgttgacaat ggctctctgg ccaacacgcc tcatctgagg gagcttcact tggacaacaa    180
   caagcttacc agagtacctg gtgggctggc agagcataag tacatccagg ttgtctacct    240
   tcat                                                                 244
<210> 278
   <211> 198
   <212> DNA
   <213> human
<400> 278
   tcacaccgta gcccacatgg atagcacagt tgtcagacaa gattccttca gattccgagt     60
   tgcctaccgg ttgttttcgt tgttgttgtt gttgtttttc tttttctttt tttttttgaa    120
   gacagcaata accacagtac atattactgt agttctctat agttttacat acattcatac    180
   cataactctg ttctctcc                                                  198
<210> 279
   <211> 430
   <212> DNA
   <213> human
<220>
   <221> misc_feature
   <222> (42)..(42)
   <223> n is a, c, g, or t
<220>
   <221> misc_feature
   <222> (93)..(94)
   <223> n is a, c, g, or t
<220>
   <221> misc_feature
   <222> (107)..(107)
   <223> n is a, c, g, or t
<220>
   <221> misc_feature
   <222> (121)..(121)
   <223> n is a, c, g, or t
<220>
   <221> misc_feature
   <222> (125)..(125)
   <223> n is a, c, g, or t
<220>
   <221> misc_feature
   <222> (144)..(144)
   <223> n is a, c, g, or t
<220>
   <221> misc_feature
   <222> (155)..(155)
   <223> n is a, c, g, or t
<220>
   <221> misc_feature
   <222> (163)..(163)
   <223> n is a, c, g, or t
<220>
   <221> misc_feature
   <222> (167)..(167)
   <223> n is a, c, g, or t
<220>
   <221> misc_feature
   <222> (174)..(174)
   <223> n is a, c, g, or t
<220>
   <221> misc_feature
   <222> (184)..(184)
   <223> n is a, c, g, or t
<220>
   <221> misc_feature
   <222> (188)..(188)
   <223> n is a, c, g, or t
<220>
   <221> misc_feature
   <222> (193)..(193)
   <223> n is a, c, g, or t
<220>
   <221> misc_feature
   <222> (199)..(199)
   <223> n is a, c, g, or t
<220>
   <221> misc_feature
   <222> (254)..(254)
   <223> n is a, c, g, or t
<220>
   <221> misc_feature
   <222> (270)..(270)
   <223> n is a, c, g, or t
<220>
   <221> misc_feature
   <222> (361)..(361)
   <223> n is a, c, g, or t
<220>
   <221> misc_feature
   <222> (364)..(364)
   <223> n is a, c, g, or t
<220>
   <221> misc_feature
   <222> (377)..(377)
   <223> n is a, c, g, or t
<400> 279
   cactttccaa tgccacagac aactcacatt gttcaactcc cnttctcggg gtgggacaga     60
   cgagacaaca gcacacaggc agccagccgt ggnnccagag gctcganggg gctcaggggc    120
   ntcanggcac ccgtccccac acgnagggcc ccgtngggtg ggncctnggc cctngctttc    180
   tacngccnaa tgnttatgnc cagctccatg ttctcccaaa taccgttgat gtgaattatt    240
   ttaaaggcaa aacngtgctc tttattttan aaaacactga taatcacact gcggtaggtc    300
   attcttttgc cacatcccta tagaccactg ggtttggcaa aactcaggca gaagtggaga    360
   nccnttctag acatcantgt cagccttgct acttgaaggt acaccccata gggtcggagg    420
   tgctgtcccc                                                           430
<210> 280
   <211> 135
   <212> DNA
   <213> human
<400> 280
   gaggcaaatg gatctcgata tttcagatgg gcttttgatg cactgttgcc aaggaaggct     60
   ttttctgatt ttttgacaaa tgaatttttg cacactttca ttggtgtctt tcggcaactt    120
   acacacattg aaaat                                                     135
<210> 281
   <211> 260
   <212> DNA
   <213> human
<220>
   <221> misc_feature
   <222> (45)..(45)
   <223> n is a, c, g, or t
<220>
   <221> misc_feature
   <222> (48)..(48)
   <223> n is a, c, g, or t
<400> 281
   gaggacttag gagagatctg aactccagct gccctacaaa ctccntcnca gcttttcttc     60
   tcacttcatg tgaaaactac tccagtggct gactgaattg ctgacccttc aagctctgtc    120
   cttatccatt acctcaaagc agtcattcct tagtaaagtt tccaacaaat agaaattaat    180
   gacactttgg tagcactaat atggagatta tcctttcatt gagcctttta tcctctgttc    240
   tcctttgaag aacccctcac                                                260
<210> 282
   <211> 131
   <212> DNA
   <213> human
<220>
   <221> misc_feature
   <222> (85)..(85)
   <223> n is a, c, g, or t
<400> 282
   gcactgggag gcccaagagc caatccagat gcctgagaca acggagactg tgctctgtgc     60
   cctgggcctg gtgctgggcc tagtnggcat catcgtgggc accgtcctca tcataaagtc    120
   tctgcgttct g                                                         131
<210> 283
   <211> 466
   <212> DNA
   <213> human
<400> 283
   tgcagaagtc aaaccctcat gacaaagtag gcacaagtct acaataagct aaatcagaat     60
   ttacaaatac aagtgtccca ggtagcattg actcccgtca ttggagtgaa atggatcaaa    120
   gtttgaatta aggcctatgg taaggtaaca ttgctttgtt gtacttttga acaagagctc    180
   ctcctgatca ctattacata tttttctaga aaatctaaag ttcagaagag aatgtatcac    240
   tgctgacttt tattccaata tttggatgga gtaagtttta gggtagaatt ttgttcagtt    300
   tggatttaat cttttgaaaa gtaaattcct tgtttactgg tttgactata attctctgtt    360
   atctttacga ggtaaaactg caagctgact agcatgttct gtgaatctgc cattcctaaa    420
   aattttataa acacttgata cttttcactg ataatggatc gctcca                   466
<210> 284
   <211> 496
   <212> DNA
   <213> human
<220>
   <221> misc_feature
   <222> (304)..(304)
   <223> n is a, c, g, or t
<220>
   <221> misc_feature
   <222> (307)..(307)
   <223> n is a, c, g, or t
<400> 284
   tcacagcggg caggacggac cccgcccacg acgtgcgggt ggcggtggtg cagtacagcg     60
   gcacgggcca gcagcgccca gagcgggcgt cgctgcagtt cctgcagaac tacacggccc    120
   tggccagtgc cgtcgatgcc atggacttta tcaacgacgc caccgacgtc aacgatgccc    180
   tgggctatgt gacccgcttc taccgcgagg cctcgtccgg cgctgccaag aagaggctgc    240
   tgctcttctc agatggcaac tcgcagggcg ccacgcccgc tgccatcgag aaggccgtgc    300
   aggnagncca gcgggcaggc atcgagatct tcgtggtggt cgtgggccgc caggtgaatg    360
   agccccacat ccgcgtcctg gtcaccggca agacggccga gtacgacgtg gcctacggcg    420
   agagccacct gttccgtgtc cccagctacc aggccctgct ccgcggtgtc ttccaccaga    480
   cagtctccag gaaggt                                                    496
<210> 285
   <211> 541
   <212> DNA
   <213> human
<220>
   <221> misc_feature
   <222> (54)..(54)
   <223> n is a, c, g, or t
<220>
   <221> misc_feature
   <222> (91)..(91)
   <223> n is a, c, g, or t
<220>
   <221> misc_feature
   <222> (99)..(99)
   <223> n is a, c, g, or t
<220>
   <221> misc_feature
   <222> (106)..(106)
   <223> n is a, c, g, or t
<220>
   <221> misc_feature
   <222> (110)..(110)
   <223> n is a, c, g, or t
<220>
   <221> misc_feature
   <222> (136)..(136)
   <223> n is a, c, g, or t
<220>
   <221> misc_feature
   <222> (151)..(151)
   <223> n is a, c, g, or t
<400> 285
   gaatttcacc tgtaaacctg agtcgtacag aaagctgcct ggtatatcca aaangctttt 60
   tattcctcct gctcatattg tgattctgcc ntttggggna cttttncttn aaaccttcag 120
   ttatgatttt tttttncata cacttattgg naactctgct tgatttttgc ctcttccagt 180
   cttcctgaca ctttaattac caacctgtta cctactttga ctttttgcat ttaaaacaga 240
   cactggcatg gatatagttt tacttttaaa ctgtgtacat aactgaaaat gtgctatact 300
   gcatactttt taaatgtaaa gatattttta tctttatatg aagaaaatca cttaggaaat 360
   ggctttgtga ttcaatctgt aaactgtgta ttccaagaca tgtctgttct acatagatgc 420
   ttagtccctc atgcaaatca attactggtc caaaagattg ctgaaatttt atatgcttac 480
   tgatatattt tacaattttt tatcatgcat gtcctgtaaa ggttacaagc ctgcacaata 540
   a 541
<210> 286
   <211> 557
   <212> DNA
   <213> human
<400> 286
   tcttcccctt gagcttcaga gaggagagtt ggcatggtta aatctgaatg gttacctcac 60
   tgctgaaaac ccagaggggc gtggcacact cgcttgtgtg gaaaagcctc taaatgcatc 120
   ccttcctttc tttcctgctt cctttgcctt acaattgaag cagcccgtgg taccatcaca 180
   gtatgcagag acttcctcac ctttcatatc tagggaccac ccccgatgca ttggtgaggg 240
   tgggcactta taaatgcctg ctattgttaa gccattccag cctcttcctc tgaatagacc 300
   agacgccctt tcacttagtt cagtgccagt ccttttgcct tcccaaccct gctgttaggc 360
   ctgctgttcc ctttgctctt gattaggaga gatggaagga gatgagctcc cataactgaa 420
   ttggcctttg gttcatgttt tctccccata tgtatatatg ccatatgtga atatgccata 480
   tatatgtgcc aacaaatcta tctacgttgt tcttttcaaa ttagcacgca gataggaatt 540
   ttgagtttct tcttctt 557
<210> 287
   <211> 418
   <212> DNA
   <213> human
<400> 287
   tatgtaaact ttaacttcca ctttgtataa atttttaagt gtcagactat ccattttaca     60
   cttgctttat ttttcattac ctgtagcttt gggcagattt gcaacagcaa attaatgtgt    120
   aaaattggat tattactaca aaaccgttta gtcatatcta tctaatcaga tcttcttttg    180
   ggaggatttg atgtaagtta ctgacaagcc tcagcaaacc caaagatgtt aacagtattt    240
   taagaagttg ctgcagattc ctttggccac tgtatttgtt aatttcttgc aatttgaagg    300
   tacgagtaga ggtttaaaga aaaatcagtt tttgttctta aaaatgcatt taagttgtaa    360
   acgtcttttt aagcctttga agtgcctctg attctatgta acttgttgca gactggtg      418
<210> 288
   <211> 199
   <212> DNA
   <213> human
<400> 288
   cgccggctcc tgcaaatgca aagagtgcaa atgcacctcc tgcaagaaaa gctgctgctc     60
   ctgctgccct gtgggctgtg ccaagtgtgc ccagggctgc atctgcaaag gggcgtcgga     120
   caagtgcagc tgctgcgcct gatgctggga cagccccgct cccagatgta aagaacgcga    180
   cttccacaaa cctggattt                                                 199
<210> 289
   <211> 500
   <212> DNA
   <213> human
<400> 289
   caactgtttt tgcgacttta taggcaggta aattttgcta ttactattga atacaaatga     60
   caattcattt atgaccactc aaacagcgtt agtaaccatt tagtgacaaa ggattaaaac    120
   atccatctgg atgttaattt tgaagatgta aattatatgt tgtttaaatt tttccaggca    180
   tctgaaaacc ttatctgcta gacaatgtaa gattcacaca gagttatctg ggattctgat    240
   tttttaaata gtacatatca ttaaaccatt ttctctaaat gtaagaagag cagaaaaaat    300
   cttataagat tatcagattt ttctaatgac acagaaatgt aagaaaaaaa tccctttata    360
   ttgaaaaaag atgcagtcaa agtcttttca gacatgccca aactttgaga atttcttcaa    420
   ccatctaatg ctataaagat ttttgttctt cctgttcaca accagttgta taacagaaat   480
   actagctact gttttccttc                                                500
<210> 290
   <211> 546
   <212> DNA
   <213> human
<400> 290
   gatgggtcgt gtgatgagat gcatttaagg ccgatagtga tagatgtttt ttttatttct     60
   tgaacacagg ctttgtctga atgatgttct tttatctctt gaacacaagc tttgaatgat    120
   aactacaggt tttaagtgct gttacattaa taccataatg tgatgtgtta gaaacaaagg    180
   gatatttcaa aggtagatat ttgaaaattc tctagtctca atatgtatgt gtattgaata    240
   tactctaaaa ataaatgtgc aatttgctag taggacaatg cagtgactga ctagcattag    300
   gtatgtttct tttatatcct agctatgtcc cactttcttc taagtgcaat cctttcatgt    360
   tcacttgctg ttttacccca tctactctaa cttcatttgg aaggcttgtc tagagtatag    420
   catgtatttt tacctttgca gtgaattgca tgtgctaatt gtaaccacag ctatttttat    480
   gttgacataa ctccaaatgt tatattaaat gttctattat atattagctc taatccctta    540
   agtaaa                                                               546
<210> 291
   <211> 419
   <212> DNA
   <213> human
<220>
   <221> misc_feature
   <222> (249)..(249)
   <223> n is a, c, g, or t
<400> 291
   acagtgttct ctaatgttac agatgagatg cgcattgcca aagaggagat ttttggacca     60
   gtgcagcaaa tcatgaagtt taaatcttta gatgacgtga tcaaaagagc aaacaatact    120
   ttctatggct tatcagcagg agtgtttacc aaagacattg ataaagccat aacaatctcc    180
   tctgctctgc aggcaggaac agtgtgggtg aattgctatg gcgtggtaag tgcccagtgc    240
   ccctttggnt gggattcaag atgtctggaa atggaagaga actgggagag tacggtttcc    300
   atgaatatac agaggtcaaa acagtcacag tgaaaatctc tcagaagaac tcataaagaa    360
   aatacaagag tggagagaag ctcttcaata gctaagcatc tccttacagt cactaatat     419
<210> 292
   <211> 538
   <212> DNA
   <213> human
<400> 292
   caaggtccca ctctaggtga tagacaggga ccccttctac tgaacctttg aggaaaggag     60
   gaaggaagaa atgcgtttag atcttggatg cagacctttc aaagggttaa atgtaaccat    120
   atggatcaac cacatgcaca tccttactac agaatccgtc ctttcatttc aacttatagc    180
   aagctatgat ttttatatat aaatattata taaataatgt ataaaacatt aaaagttaac    240
   tatgtaagat attatttctg aaacaattta gctatatcca ctatgattat aaactgtgtc    300
   tcgacctgtg ttatttacat tagctgctta aaaaagcatt gagttaattt ttttaaatat    360
   caactaaaat atcatagttc tgtggtagac attgttttat aatgaaataa ctgcaactag    420
   agaaaactgt ataaaaacat taaattgtca gtatttttgt aaggttccat tttgtaaaga    480
   gaataatatt caaagacttt tgtagcatac aaagtgaaaa cttgtatctg cgaaacta      538
<210> 293
   <211> 431
   <212> DNA
   <213> human
<220>
   <221> misc_feature
   <222> (131)..(131)
   <223> n is a, c, g, or t
<400> 293
   aagcagccta gggatgagca tttctttgaa agcaattagg ttattcacct ggtattaaaa     60
   ctatttactg ttaaaaaatc tgtgacttca tgaagttgat ttttaaaggc agcatcaaaa    120
   actgaaaagg naagggaaaa aataggcagc ttctctgcac ttgtttggag ctccccaaaa    180
   caggagccat ggagaagtgg catcaagacc gggctgccct ttcgagaaca ccctgtggca    240
   gttcagagac acgcttttcc tacactgcat gcagcccctc tttccagcac tggaaagaag    300
   tggtcttgag cccagctgag aagcacttca cactcctctc tcttgttctg aatggtgttt    360
   gtgtcagtct gcagctgtgt atggtattat gtcttataat cctgcatcac ttctatccta    420
   tccagtcata t                                                         431
<210> 294
   <211> 445
   <212> DNA
   <213> human
<400> 294
   gggtagatgg gagtgtcgct tgtatgttat cgtacagctg acatgtattt ttgtctattc     60
   tttattatct tagtttcatg ctatgtatgt accataacca acctattgcc tatgagaaac    120
   atgtaagata atgtatttac agccattgtt acaagtttat aatgtatttt tctatcttgt    180
   tttatatgta tgttatataa cattcaaaaa gaattttttt cttgattgag aaaaggatac    240
   aaaatgcaaa atccacaatt ttgataactg aaaattgcca attgttttgc agtactttat    300
   tatattgggt gtcttgtctt ttttgggctt ttagttagct aatgaatgaa tacattagac    360
   acttttgggt tttagttggg attttacata gcttgcattt taattctttg gttctttgct    420
   gtttctatta acccacagca ttatt                                          445
<210> 295
   <211> 394
   <212> DNA
   <213> human
<400> 295
   gaaggttgtc atgggctgtt ccttgggggt ttttatcctg ctcaccgtgg agataagcct     60
   gcggcttgtc taaccagcgc agcgcaaagg tctcaatgcc ttttggtaac atccgtcatt    120
   gcagaagaaa gtttacacga cgtcaaaaag tgacgttcat gctaagtgtt tttccagaaa    180
   tattggtttc atgtttctta ttggctctgc ctcctgtgct tatatcatcc aaaaactttt    240
   taaaaaggtc cagaattcta ttttaacctg atgttgagca cctttaaaac gttcgtatgt    300
   gtgttgcact aattctaaac tttggaggca ttttgctgtg tgaggccgat cgccactgta    360
   aaggtcctag agttgcctgt ttgtctctgg agat                                394
<210> 296
   <211> 179
   <212> DNA
   <213> human
<400> 296
   gagatttacc atgtatcagt gcctggcttt ttgttataaa gctttgtttg tctagtgctc     60
   ttttgctata aaatagactg tagtacaccc tagtaggaaa aaaaaaaaac taaatttaaa    120
   aataaaaaat atatttggct tatttttcgc aggagcaatc cttttatacc atgaatatt     179
<210> 297
   <211> 540
   <212> DNA
   <213> human
<220>
   <221> misc_feature
   <222> (59)..(59)
   <223> n is a, c, g, or t
<220>
   <221> misc_feature
   <222> (103)..(103)
   <223> n is a, c, g, or t
<400> 297
   gtttatacaa ctgtcagtcc acccagtggt gcaactggtt ctgattcagt cttccgatnc     60
   ctttttattt ttcacttttt cctatttctg aatttttttt ttnatttgtg atcttgattt    120
   tgatgagggg ttggggagtg gggagggagt cgaaccaaga cttggagtta agaggatttt    180
   catcttttgc atccaacagg cagaatatga tctgtgtcca aaagtgaact tgagtcagga    240
   atgaatcaat ttcagcataa acaagcacaa aaatttagtc tgctggctga ctggaagcaa    300
   aaaagtcaag atggaatatg atgaattcca acacaatggg gcaccaaggc ctttaggcct    360
   ctctttttat tttgctttgg ttttgtttgt ttttctttag agacatgctc tttctcatgg    420
   gacttgaagt ggactcatct ttgtgcagtg ctggttttgc catactcatt tcaagtatta    480
   tagacatatg taatggtgaa aatatatgaa ctgtggcctt tttcattctt gttacttgtg    540
<210> 298
   <211> 480
   <212> DNA
   <213> human
<400> 298
   gaagtgactg ttgtaccatg gttgtgcaca tgcttcagaa tcctatggaa gagaatattc     60
   ctacttgcag tacatcaaag gaatggatgg tggaccctac tattcatgtt ttgagacata    120
   aatgttcact ttaaagcaat tgcataatag ataaaaacct gaactttcat tggatttttg    180
   ttaattttcc tcattttgaa ttatgtgcac taccatagct acatcagttt gatacagtat    240
   tgaaaaatta tcagttatat tttgctgttt atgatctatt tgtagattag gattaaaatg    300
   gatttaatcc atttttaagg ctgtgtgaat ttttctaaac aagaaccatt tgcaatatgg    360
   atttcttaga gattaaacca attataactt attagcagtc gcgagcacat gttcatatag    420
   tcaatgtaaa aatacactaa tgagtatttg gtaaatccca gtaggctttt accattagca    480
<210> 299
   <211> 124
   <212> DNA
   <213> human
<400> 299
   tccctcactt ctcttaagcc aagaagtttg ctttccctag ctgcagtgta gatggctctt     60
   gtttttgttt ttttgtttta atcatttggc attcacatgt ggctgttaat atgtgcttgt    120
   tttt                                                                 124
<210> 300
   <211> 547
   <212> DNA
   <213> human
<220>
   <221> misc_feature
   <222> (59)..(59)
   <223> n is a, c, g, or t
<220>
   <221> misc_feature
   <222> (308)..(308)
   <223> n is a, c, g, or t
<400> 300
   tggtgggagg gctctcaatc agcagggccc caggagggaa gaagaagtgg ggcaaagcnt     60
   ggcctcgccg ctcgggagct ttgccatctg agccacgcct cctccaggcc atgctccttg    120
   aacttggaaa tgtcaaccgg agcccttaca ccagccctcc agcatctaat agacttgaat    180
   ctactctaaa cgaatattta atccaacctc actacattgt agctcagtcc aacgactaac    240
   cctgaaatgg gggtgttcca gccttcagcg agatggccaa gcggtcccct gggggctgtg    300
   gcagcggnct tatccttctc tgttgccaac cttgccgtcc gacctcctcc gcccccatgc    360
   ggtgaccccg tccgtgtctg tgtctgtcca tacgtgtgag tccagctaaa aagacaaaac    420
   agaacccgtg ggcccagctc ggaaggtgcg tggagaaggc tccgacgtct ccgaagtgca    480
   gcccttggga tggcattccg ttgtgtgcct tattcctgga gaatctgtat acggctcgcc    540
   tatagaa                                                              547
<210> 301
   <211> 350
   <212> DNA
   <213> human
<220>
   <221> misc_feature
   <222> (90)..(90)
   <223> n is a, c, g, or t
<220>
   <221> misc_feature
   <222> (124)..(124)
   <223> n is a, c, g, or t
<220>
   <221> misc_feature
   <222> (143)..(143)
   <223> n is a, c, g, or t
<220>
   <221> misc_feature
   <222> (150)..(150)
   <223> n is a, c, g, or t
<220>
   <221> misc_feature
   <222> (157)..(158)
   <223> n is a, c, g, or t
<220>
   <221> misc_feature
   <222> (162)..(163)
   <223> n is a, c, g, or t
<220>
   <221> misc_feature
   <222> (168)..(168)
   <223> n is a, c, g, or t
<220>
   <221> misc_feature
   <222> (223)..(223)
   <223> n is a, c, g, or t
<400> 301
   cttctccaag catcaccctg ggagtttcct gagggttttc tcataaatga gggctgcaca     60
   ttgcctgttc tgcttcgaag tattcaatan ccgctcagta ttttaaatga agtgattcta    120
   agantttggt ttgggatcaa tanggaaagn catatgnnca gnnccaancc aagatgcaaa    180
   tgttttgaaa tgatatgacc aaaattttaa gtaggaaagt cancccaaac acttctgctt    240
   tcacttaagt gtctggcccg caatactgta ggaacaagca tgatcttgtt actgtgatat    300
   tttaaatatc cacagtactc actttttcca aatgatccta gtaattgcct               350
<210> 302
   <211> 414
   <212> DNA
   <213> human
<220>
   <221> misc_feature
   <222> (157)..(157)
   <223> n is a, c, g, or t
<400> 302
   gtatcaaaat cttccaatta tcatgctcac ctgaaagagg tatgctctct taggaataca     60
   gtttctagca ttaaacaaat aaacaagggg agaaaataaa actcaaggag tgaaaatcag    120
   gaggtgtaat aaaatgttcc tcgcattccc ccccgcnttt tttttttttt ttgactttgc    180
   cttggagagc cagagcttcc gcattttctt tactattctt tttaaaaaaa gtttcactgt    240
   gtagagaaca tatatgcata aacataggtc aattatatgt ctccattaga aaaataataa    300
   ttggaaaaca tgttctagaa ctagttacaa aaataattta aggtgaaatc tctaatattt    360
   ataaaagtag caaaataaat gcataattaa aatatatttg gacataacag actt          414
<210> 303
   <211> 426
   <212> DNA
   <213> human
<220>
   <221> misc_feature
   <222> (324)..(324)
   <223> n is a, c, g, or t
<400> 303
   caggaggaca cagcattatt ctagtgctgt actgttccgt acggcagcca ctacccacat     60
   gtaacttttt aagatttaaa tttaaattag ttaacattca aaacgcagct ccccaatcac    120
   actagcaaca tttcaagtgc ttgagagcca tgcatgatta gtggttaccc tattgaatag    180
   gtcagaagta gaatcttttc atcatcacag aaagttctat tggacagtgc tcttctagat    240
   catcataaga ctacagagca cttttcaaag ctcatgcatg ttcatcatgt tagtgtcgta    300
   ttttgagctg gggttttgag actnccctta gagatagaga aacagaccca agaaatgtgc    360
   tcaattgcaa tgggccacat acctagatct ccagatgtca tttcccctct cttattttaa    420
   gttatg                                                               426
<210> 304
   <211> 295
   <212> DNA
   <213> human
<220>
   <221> misc_feature
   <222> (114)..(114)
   <223> n is a, c, g, or t
<220>
   <221> misc_feature
   <222> (224)..(224)
   <223> n is a, c, g, or t
<400> 304
   accaatgagg ttcctgaggt cacagtgttt tccaagtctc ccgtgacact gggtcagccc     60
   aacaccctca tctgtcttgt ggacaacatc tttcctcctg tggtcaacat cacntggctg    120
   agcaatgggc actcagtcac agaaggtgtt tctgagacca gcttcctctc caagagtgat    180
   cattccttct tcaagatcag ttacctcacc ttcctccctt ctgntgatga gatttatgac    240
   tgcaaggtgg agcactgggg cctggatgag cctcttctga aacactggga gcctg         295
<210> 305
   <211> 537
   <212> DNA
   <213> human
<220>
   <221> misc_feature
   <222> (368)..(368)
   <223> n is a, c, g, or t
<400> 305
   cacatggttc tagcctggac ctggctgggc tccatgagaa tgagttgcct ccaccctgtc     60
   ccaacagctg acagccagga gccactctcc cagctgcagg cctttgtggt ccatcttgtc    120
   ctgcttcctc actgtggacc cctgtctggg ccaccctagt gtgctaagct gagcagtgca    180
   gtgtgaacag ggcccatggt gtattctagg ccacagccca gcactcctct gggctgctct    240
   caaaccatgt cccatcttca gcatccctcc caccaactta ctcccctgtg gtgagtaccg    300
   tggaacccca gcccacctca ctatcatact cagcttcccc tgatggccca tcccagcccc    360
   tgaagctnta tgccaagaac acagctaccg cacaccaccc tgaaacagcc acagccaagg    420
   taggcatgca tatgaggtct tccccatacc ctctgggtgt tgagaggttt agccacatga    480
   gggagcagag gacaatctct gcagggctgg gagtgggtag ggactgaagg tctcaat       537
<210> 306
   <211> 466
   <212> DNA
   <213> human
<400> 306
   tcagtgtctg ggctcatgac ttgtaaatgg aagctgatgt gaacaggtaa ttaatattat     60
   gacccacttc tatttacttt gggaaatatc ttggatctta attatcatct gcaagtttca    120
   agaagtattc tgccaaaagt atttacaagt atggactcat gagctattgt tggttgctaa    180
   atgtgaatca cgcgggagtg agtgtgccct tcacactgtg acattgtgac attgtgacaa    240
   gctccatgtc ctttaaaatc agtcactctg cacacaagag aaatcaactt cgtggttgga    300
   tggggccgga acacaaccag tctttttgta tttattgtta ctgagacaaa acagtactca    360
   ctgagtgttt ttcagtttcc tactggtggt tttgatattg tttgtttaag atgtatattt    420
   agaatgacat catctaagaa gctgattttg ctaaactcct gttccc                   466
<210> 307
   <211> 474
   <212> DNA
   <213> human
<400> 307
   gaactcatgt gatttaccct tttcaacttt ttggaaaacg atttaattta ttctaattag     60
   attaacccta ttaatctatg gattgggtat caaaatgaat gccagtccag atgtgcctag    120
   acacgaaatt ggagctgagg actctcacga tatgcaagtt catccaacgt gaagatacca    180
   taagcttttt ctctgaacca gagaaatgaa agtcagttta agaggctgat agatcttggc    240
   cctgttaagg catccacttc acagttctga aggctgagtc agccccactc cacagttagg    300
   ccaagaatta gattttaaaa cttcatctgt ctgtcccagt taactgttaa ataaggcctc    360
   atcctccact gaagagtatg gattgaagga ttgtgaacta tgtttagtgt gattgtgaac    420
   ttggtgccta atgttccatg tctgaagttt gccccagtgc tacacgttgg agta           474
<210> 308
   <211> 388
   <212> DNA
   <213> human
<220>
   <221> misc_feature
   <222> (218)..(218)
   <223> n is a, c, g, or t
<400> 308
   gatgcaattg gttctcctgc attgagattt gatttaacag tgttatgtta acatttatac     60
   ttgccttgga ctgtagaaca gaacttaaat gggaatgtat tagttttaca actacaatca    120
   agtcatttta cctttaccca gtttttaata taaaacttaa attttgaaat tcactgtgtg    180
   actaatagca tgatgctctg cagttttatt aagaaatnag cctaaccata caactctcat    240
   ttccttagta agccaaatta ggattaactt ctataaacag tgttgggaac aatgtttaac    300
   attttgtgcc aatttgttcc tgtattcatg tatgtaagtt acagatctga ctcttcattt    360
   ttaagttcct tgttacatca tggtcatt                                       388
<210> 309
   <211> 379
   <212> DNA
   <213> human
<400> 309
   cctatcctac ttgcccatat gagcacggct ccccatggcc acatactcct gcaaagcttt     60
   tatgctgctt cgcttttctc taaacagatc tgatattgct gctcctgtgg ttttctcaaa    120
   attaactttg ccgtggtttt taaaaaggaa tcaaaatgca ttgttgcatt aagctttttc    180
   aataaaggaa aattacggaa ggaaaatagg caacaccagc aaattatatg tggacaggtt    240
   ctaaactcta tatatacata tatatatata tatctatata tctatatacg taatcatcta    300
   gttctgtcat cttactgaaa ggaataacac ttctaaagat caccatttct gagaagttct    360
   tggaaatctt tatgtctaa                                                 379
<210> 310
   <211> 519
   <212> DNA
   <213> human
<220>
   <221> misc_feature
   <222> (130)..(130)
   <223> n is a, c, g, or t
<220>
   <221> misc_feature
   <222> (144)..(144)
   <223> n is a, c, g, or t
<220>
   <221> misc_feature
   <222> (167)..(167)
   <223> n is a, c, g, or t
<220>
   <221> misc_feature
   <222> (169)..(169)
   <223> n is a, c, g, or t
<220>
   <221> misc_feature
   <222> (268)..(268)
   <223> n is a, c, g, or t
<220>
   <221> misc_feature
   <222> (349)..(349)
   <223> n is a, c, g, or t
<220>
   <221> misc_feature
   <222> (358)..(358)
   <223> n is a, c, g, or t
<220>
   <221> misc_feature
   <222> (478)..(478)
   <223> n is a, c, g, or t
<400> 310
   caccgcgcag agctcagggg gtggtgcgcc cggcccttct gcggcgcaca gcccagccca     60
   ggaacgcggg cggtgcggac tcagcgggcc gggtgcaggc gcggagctgg gcctctgcgc    120
   ccggcccgan ctccgtctat aaanagagca gccagttgca gggctcnant ctgctttcca    180
   actgcctgac tgcttgttcg tctcactggt gtgagctcca gcatcccctt tgctcgaaat    240
   ggaccccaac tgctcttgcg ccactggntg gctcctgcac gtgcgccggc tcctgcaagt    300
   gcaaagagtg caaatgcacc tcctgcaaga agagctgctg ttcctgctnc cccgtggnct    360
   gtgccaagtg tgcccagggc tgcgtctgca aaggggcatc ggagaagtgc agctgctgtg    420
   cctgatgtgg gaacagctct tctcccagat gtaaatagaa caacctgcac aacctggnat    480
   ttttttaaaa atacaacact gagccatttg ctgcatttc                           519
<210> 311
   <211> 468
   <212> DNA
   <213> human
<400> 311
   tactgatgct tctgatactg gatgtttagc ttcttactgc aaaaacataa gtaaaacagt     60
   caactttacc atttccgtat tctccataga ttgaagaaat ttataccaca tatcgcatat    120
   gaccatcttt ccatcaaatc aatgtagaga taatgtaaac tgaaaaaaaa tctgcaagat    180
   aatgtaactg aatgttttaa aaacagaact tgtcacttta tataaaagaa tagtatgctc    240
   tatttcctga atggatgtgg aaatgaaagc tagcgcacct gcactttgaa ttcttgcttc    300
   ttttttatta ctgttatgat tttgcttttt acagatgttg gacgattttt tcttctgatt    360
   gttgaattca taatcatggt ctcatttcct ttgcttcttt ggaatatttc tttcaacaca    420
   ttcctttatt ttattataca ttgtgtcctt tttttagcta ttgctgct                 468
<210> 312
   <211> 147
   <212> DNA
   <213> human
<400> 312
   gacaggaggg tgtccacata tgttaacatc agttggatct cctatagaag tttctgctgc     60
   tctctttcct tctccctgag ctggtaactg caatgccaac ttcctgggcc tttctgacta    120
   gtatcacact tctaataaaa tccacaa                                        147
<210> 313
   <211> 560
   <212> DNA
   <213> human
<220>
   <221> misc_feature
   <222> (175)..(177)
   <223> n is a, c, g, or t
<220>
   <221> misc_feature
   <222> (179)..(181)
   <223> n is a, c, g, or t
<220>
   <221> misc_feature
   <222> (190)..(190)
   <223> n is a, c, g, or t
<220>
   <221> misc_feature
   <222> (422)..(422)
   <223> n is a, c, g, or t
<220>
   <221> misc_feature
   <222> (426)..(426)
   <223> n is a, c, g, or t
<220>
   <221> misc_feature
   <222> (429)..(430)
   <223> n is a, c, g, or t
<400> 313
   gctccacatg agccatgcat gcttagcaat ccaagtgcag agctctttgc tccaggagtg     60
   aggagactgg gaggtgaaat ggggaaatgg aagggtttgg aggcagagct gaaaacaggg    120
   ttggaaggat ttcctgaatt agaagacaaa cgttagcata cccagtaagg aaaannngnn    180
   naggggccan ggggaacccg tgaggatcac tctcaaatga gattaaaaac aaggaagcag    240
   agaatggtca gagaatggga ttcagattgg gaacttgtgg ggatgagagt gaccaggttg    300
   aactgggaag tggaaaaagg agtttgagtc actggcacct agaagcctgc ccacgattcc    360
   taggaaggct ggcagacacc ctggaaccct ggggagctac tggcaaactc tcctggattg    420
   gncctnatnn ttttggtggg aaaggctgcc ctggggatca actttccttc tgtgtgtggc    480
   tcaggagttc ttctgcagag atggcgctat ctttcctcct cctgtgatgt cctgctccca    540
   accatttgta ctcttcatta                                                560
<210> 314
   <211> 530
   <212> DNA
   <213> human
<400> 314
   aagtgtgcat aatttcattt aacgttaaag aaatagatcc aattcctttc ttgcaaccaa     60
   aaataaataa aatacgttgc ctcaatataa ggtttgggct attctgtgtt tctatagaag    120
   caatctgttt ttggtaaaat gtacttttaa ggatccagtc atctgaagta ttttatgtag    180
   agttagagat ttcacaatat tgactataca tatatttaaa atataaatta tccagctgat    240
   gtttgaattt gtcttacttt cctggccacc tcgttgtcct attttataag ctggggagtt    300
   aactagctta acaaaagatg cttagctttt gtaaaagaac aagtgtttca ttttacaaag    360
   acactccaaa tgatagttac ttgattttct cgagaccttt aactatggtg atgaataaca    420
   ggacttgctt tcaagcctta ataaatgtaa aatgcctttt aatgaagata cagctgagtg    480
   ttttcctcat gaatctgaac caattaccaa tttgtgttcc agtcttgatt                530
<210> 315
   <211> 417
   <212> DNA
   <213> human
<400> 315
   gaaaccaagg tcaggaggcc gttgcagaca taaatctcgg cgactcggcc ccgtctcctg     60
   agggtcctgc tggtgaccgg cctggacctt ggccctacag ccctggaggc cgctgctgac    120
   cagcactgac cccgacctca gagagtactc gcaggggcgc tggctgcact caagaccctc    180
   gagattaacg gtgctaaccc cgtctgctcc tccctcccgc agagactggg gcctggactg    240
   gacatgagag ccccttggtg ccacagaggg ctgtgtctta ctagaaacaa cgcaaacctc    300
   tccttcctca gaatagtgat gtgttcgacg ttttatcaaa ggcccccttt ctatgttcat    360
   gttagttttg ctccttctgt gtttttttct gaaccatatc catgttgctg acttttc       417
<210> 316
   <211> 544
   <212> DNA
   <213> human
<400> 316
   cacaggcagg tgactactcc atgcgcgtgg acctgcgggc tggggacgag gctgtgttcg     60
   cccagtacga ctccttccac gtagactcgg ctgcggagta ctaccgcctc cacttggagg    120
   gctaccacgg caccgcaggg gactccatga gctaccacag cggcagtgtc ttctctgccc    180
   gtgatcggga ccccaacagc ttgctcatct cctgcgctgt ctcctaccga ggggcctggt    240
   ggtacaggaa ctgccactac gccaacctca acgggctcta cgggagcaca gtggaccatc    300
   agggagtgag ctggtaccac tggaagggct tcgagttctc ggtgcccttc acggaaatga    360
   agctgagacc aagaaacttt cgctccccag cggggggagg ctgagctgct gcccacctct    420
   ctcgcacccc agtatgactg ccgagcactg aggggtcgcc ccgagagaag agccagggtc    480
   cttcaccacc cagccgctgg aggaagcctt ctctgccagc gatctcgcag cactgtgttt    540
   acag                                                                  544
<210> 317
   <211> 437
   <212> DNA
   <213> human
<400> 317
   ccaatggcga gggtctgcca cactggccgc tgttcgacca ggaggagcaa tacctgcagc    60
   tgaacctaca gcctgcggtg ggccgggctc tgaaggccca caggctccag ttctggaaga   120
   aggcgctgcc ccaaaagatc caggagctcg aggagcctga agagagacac acagagctgt   180
   agctccctgt gccggggagg aggggtgggg ttcgctgaca ggcgagggtc agcctgctgt   240
   gcccacacac acccactaag gagaaagaag ttgattcctt cattcacttc gccattcatt   300
   catacttccg tccatccatt cagaaagcat ttattaagaa tttactcagg catgatggcc   360
   catacttgta atcccagcta ttgggaagga tgagatggga ggatggcttg aggccagagg   420
   tttgagaccg accagcc                                                   437
<210> 318
   <211> 557
   <212> DNA
   <213> human
<220>
   <221> misc_feature
   <222> (464)..(464)
   <223> n is a, c, g, or t
<400> 318
   gggagagtcc atctggaagc tggagtatat cctgacccag acctacgaca ttgaagattt    60
   gcagccggaa agtttatatg gattagctaa acaatttaca atcctagaca gtaagcagtt   120
   tataaaatac tacaattact tctttgtgag ttatgacagc agtgtaacat gtgataagac   180
   atgtaaggcc tttcagattt gtgcaattat gaatcttgat aatatttcct atgcagattg   240
   cctcaaacag ctttatataa agcacaatta ctagtatttc acagtttttg ctaatagaaa   300
   atgctgattc tgattctgag atcaatttgt gggaatttta cataaatctt tgttaattac   360
   tgagtgggca agtagacttc ctgtctttgc tttctttttt tttttctttt tgatgcctta   420
   atgtagatat ctttatcatt ctgaattgta ttatatattt aaantgctca ttaatagaat   480
   gatggatgta aattggatgt aaatattcag tttatataat tatatctaat ttgtaccctt   540
   gttgaaattg tcattta                                                   557
<210> 319
   <211> 336
   <212> DNA
   <213> human
<220>
   <221> misc_feature
   <222> (150)..(150)
   <223> n is a, c, g, or t
<220>
   <221> misc_feature
   <222> (154)..(154)
   <223> n is a, c, g, or t
<400> 319
   cttcgcttct ctcttggaaa gtccagtctc tcctcggctt gcaatggacc ccaactgctc    60
   ctgcgccgct ggtgtctcct gcacctgcgc tggttcctgc aagtgcaaag agtgcaaatg   120
   cacctcctgc aagaagagct gctgctcctn gctnccccgt gggctgtagc aagtgtgccc   180
   agggctgtgt ttgcaaaggg gcgtcagaga agtgcagctg ctgcgactga tgccaggaca   240
   acctttctcc cagatgtaaa cagagagaca tgtacaaacc tggatttttt ttttatacca   300
   ccttgaccca tttgctacat tccttttcct gtgaaa                              336
<210> 320
   <211> 208
   <212> DNA
   <213> human
<220>
   <221> misc_feature
   <222> (85)..(85)
   <223> n is a, c, g, or t
<220>
   <221> misc_feature
   <222> (100)..(100)
   <223> n is a, c, g, or t
<220>
   <221> misc_feature
   <222> (102)..(102)
   <223> n is a, c, g, or t
<220>
   <221> misc_feature
   <222> (119)..(123)
   <223> n is a, c, g, or t
<220>
   <221> misc_feature
   <222> (125)..(126)
   <223> n is a, c, g, or t
<220>
   <221> misc_feature
   <222> (128)..(128)
   <223> n is a, c, g, or t
<220>
   <221> misc_feature
   <222> (148)..(148)
   <223> n is a, c, g, or t
<400> 320
   cctggggctg agcaaggcct acgtaggcca gaagagcagc ttcacagtag actgcagcaa     60
   agcaggcaac aacatgctgc tggtnggggt tcatggcccn angaccccct gcgaggagnn    120
   nnngnngnag cacgtgggca gccggctnta cagcgtgtcc tacctgctca aggacaaggg    180
   ggagtacaca ctggtggtca aatggggg                                       208
<210> 321
   <211> 358
   <212> DNA
   <213> human
<220>
   <221> misc_feature
   <222> (146)..(146)
   <223> n is a, c, g, or t
<220>
   <221> misc_feature
   <222> (180)..(181)
   <223> n is a, c, g, or t
<400> 321
   gaaactgtat gggtagcttt tttgtttgtt ttttgttttg tttttgtttt tgtttttgtt     60
   tttagttgta ggtcgcagcg gggaaatttt ttgcgactgt acacatagct gcagcattaa    120
   aaacttaaaa aaattgttaa aaaaanaaaa aaagggaaaa catttcaaaa aaaaaaaaan    180
   ngataaacag ttacaccttg ttttcaatgt gtggctgagt gcctcgattt tttcatgttt   240
   ttggtgtatt tctgatttgt agaagtgtcc aaacaggttg tgtgctggag ttccttcaag    300
   acaaaaacaa acccagcttg gtcaaggcca ttacctgttt cccatctgta gttattcg     358
<210> 322
   <211> 344
   <212> DNA
   <213> human
<400> 322
   gctgctgccg cggggaagca ggaactggcc aagtacttct tggcagagct gctgtctgaa     60
   cccaaccaga cggagaatga tgccctggaa cctgaagatc tgtcccaggc tgctgagcag    120
   gatgaaatga ggcttgagct gcagagatct gctaactcaa acccggctat ggcaccccga    180
   gaacgcaaag ctggctgcaa gaatttcttc tggaagactt tcacatcctg ttagctttct    240
   taactagtat tgtccatatc agacctctga tccctcgccc ccacacccca tctctcttcc    300
   ctaatcctcc aagtcttcag cgagaccctt gcattagaaa ctga                      344
<210> 323
   <211> 486
   <212> DNA
   <213> human
<220>
   <221> misc_feature
   <222> (103)..(103)
   <223> n is a, c, g, or t
<400> 323
   acaggaaatt gctacttacc gccgccttct ggaaggagaa gacgtaaaaa ctacagaata     60
   tcagttaagc accctggaag agagagatat aaagaaaacc agnaagatta agacagtcgt    120
   gcaagaagta gtggatggca aggtcgtgtc atctgaagtc aaagaggtgg aagaaaatat    180
   ctaaatagct accagaagga gatgctgctg aggttttgaa agaaatttgg ctataatctt    240
   atctttgctc cctgcaagaa atcagccata agaaagcact attaatactc tgcagtgatt    300
   agaaggggtg ggtggcgggg aatcctattt atcagactct gtaattgaat ataaatgttt    360
   tactcagagg agctgcaaat tgcctgcaaa aatgaaatcc agtgagcact agaatattta    420
   aaacatcatt actgccatct ttatcatgaa gcacatcaat tacaagctgt agaccaccta    480
   atatca                                                                486
<210> 324
   <211> 299
   <212> DNA
   <213> human
<400> 324
   gagaaagcca ggcaggtagc cagggggact agcccctgtg gagactgggg ggcttgaaat     60
   tgtccccgtg gtctcttact ttcctttccc cagcccaggg tggacttaga aagcaggggc    120
   tacaagaggg aatccccgaa ggtgctggag gtgggagcag gagattgaga aggagagaaa    180
   gtgggtgaga tgctggagaa gagaggagag gagagaggca gagagcggtc tcaggctggt    240
   gggaggggcg cccacctccc cacgccctcc cctcccctgc tgcaggggct ctggagaga     299
<210> 325
   <211> 419
   <212> DNA
   <213> human
<400> 325
   atgagccttc atacatggac tgagagtcag agcttgaaga aaagcttatt tattttcccc     60
   aacctccccc aggtgcagtg tgacattatt ttattataac atccacaaag agattatttt    120
   taaataattt aaagcataat atttcttaaa aagtatttaa ttatatttaa gttgttgatg    180
   ttttaactct atctgtcata catcctagtg aatgtaaaat gcaaaatcct ggtgatgtgt    240
   tttttgtttt tgttttcctg tgagctcaac taagttcacg gcaaaatgtc attgttctcc    300
   ctcctacctg tctgtagtgt tgtggggtcc tcccatggat catcaaggtg aaacactttg    360
   gtattctttg gcaatcagtg ctcctgtaag tcaaatgtgt gctttgtact gctgttgtt     419
<210> 326
   <211> 445
   <212> DNA
   <213> human
<220>
   <221> misc_feature
   <222> (42)..(42)
   <223> n is a, c, g, or t
<220>
   <221> misc_feature
   <222> (51)..(51)
   <223> n is a, c, g, or t
<220>
   <221> misc_feature
   <222> (197)..(197)
   <223> n is a, c, g, or t
<220>
   <221> misc_feature
   <222> (212)..(212)
   <223> n is a, c, g, or t
<220>
   <221> misc_feature
   <222> (220)..(220)
   <223> n is a, c, g, or t
<220>
   <221> misc_feature
   <222> (275)..(275)
   <223> n is a, c, g, or t
<220>
   <221> misc_feature
   <222> (288)..(288)
   <223> n is a, c, g, or t
<220>
   <221> misc_feature
   <222> (293)..(293)
   <223> n is a, c, g, or t
<220>
   <221> misc_feature
   <222> (300)..(300)
   <223> n is a, c, g, or t
<220>
   <221> misc_feature
   <222> (306)..(308)
   <223> n is a, c, g, or t
<220>
   <221> misc_feature
   <222> (336)..(336)
   <223> n is a, c, g, or t
<220>
   <221> misc_feature
   <222> (359)..(359)
   <223> n is a, c, g, or t
<400> 326
   atgcctacag gtatgcgtga ttgtgtgtgt gtgcatgggt gnacagccac ntgtctacct     60
   atgtgtcttt ctgggaatgt gtaccatctg tgtgcctgca gctgtgtagt gctggacagt    120
   gacaaccctt tctctccagt tctccactcc aatgataata gttcacttac acctaaaccc    180
   aaaggaaaaa ccagctntag gtccaattgt tntgctctan actgatacct caaccttggg    240
   gccagcatct cccactgcct ccaaatatta gtaantatga ctgacgtncc canaagtttn    300
   tgggtnnnac cacactcccc aaccccccac tcctanttcc tgaagggccc tcccaaggnt     360
   acatccccac cccacagttc tccctgagag agatcaacct ccctgagatc aaccaaggca    420
   gatgtgacag caagggccac ggacc                                          445
<210> 327
   <211> 524
   <212> DNA
   <213> human
<220>
   <221> misc_feature
   <222> (40)..(40)
   <223> n is a, c, g, or t
<220>
   <221> misc_feature
   <222> (44)..(44)
   <223> n is a, c, g, or t
<220>
   <221> misc_feature
   <222> (48)..(49)
   <223> n is a, c, g, or t
<220>
   <221> misc_feature
   <222> (52)..(56)
   <223> n is a, c, g, or t
<220>
   <221> misc_feature
   <222> (60)..(60)
   <223> n is a, c, g, or t
<220>
   <221> misc_feature
   <222> (98)..(98)
   <223> n is a, c, g, or t
<400> 327
   gcctctggca atgccattca ggccaggtct tcctcctatn gtgnaganna tnnnnntggn     60
   ggtggaaagc gattctctca ttctggcaac cagttggnac ggccccatca ccgccctccg    120
   ggtccgagtc aacacatact acatcgtagg tcttcaggtg cgctatggca aggtgtggag    180
   cgactatgtg ggtggtcgca acggagacct ggaggagatc tttctgcacc ctggggaatc    240
   agtgatccag gtttctggga agtacaagtg gtacctgaag aagctggtat ttgtgacaga    300
   caagggccgc tatctgtctt ttgggaaaga cagtggcaca agtttcaatg ccgtcccctt    360
   gcaccccaac accgtgctcc gcttcatcag tggccggtct ggttctctca tcgatgccat    420
   tggcctgcac tgggatgttt accccactag ctgcagcaga tgctgagcct cctctccttg    480
   gcaggggcac tgtgatgagg agtaagaact cccttatcac taac                      524
<210> 328
   <211> 335
   <212> DNA
   <213> human
<400> 328
   acaaggccca ggctggggcc agggccagag gggaaggccc tggattctca ctcatgtgag     60
   atcttgaatc tctttctttg ttctgtttgt ttagttagta tcatctggta aaatagttaa    120
   aaaacaacaa aaaactctgt atctgtttct agcatgtgct gcattgactc tattaatcac    180
   atttcaaatt caccctacat tcctctcctc ttcactagcc tctctgaagg tgtcctggcc    240
   agccctggag aagcactggt gtctgcagca cccctcagtt cctgtgcctc agcccacagg    300
   ccactgtgat aatggtctgt ttagcacttc tgtat                               335
<210> 329
   <211> 244
   <212> DNA
   <213> human
<220>
   <221> misc_feature
   <222> (74)..(74)
   <223> n is a, c, g, or t
<220>
   <221> misc_feature
   <222> (93)..(93)
   <223> n is a, c, g, or t
<220>
   <221> misc_feature
   <222> (164)..(169)
   <223> n is a, c, g, or t
<220>
   <221> misc_feature
   <222> (171)..(172)
   <223> n is a, c, g, or t
<220>
   <221> misc_feature
   <222> (176)..(176)
   <223> n is a, c, g, or t
<220>
   <221> misc_feature
   <222> (180)..(181)
   <223> n is a, c, g, or t
<400> 329
   tcaacttcaa gctcctaagc cactgcctgc tggtgaccct ggccgcccac ctccccgccg     60
   agttcaaccc ctgnggtgca cgcctccctg ganaagttcc tggcttctgt gagcaccgtg    120
   ctgacctcca aataccgtta agctggagcc tcggtagccg ttcnnnnnnc nngctnggcn    180
   ntccaacggg ccctcctccc ctccttgcac cggcccttcc tggtctttga ataaagtctg    240
   agtg                                                                 244
<210> 330
   <211> 543
   <212> DNA
   <213> human
<220>
   <221> misc_feature
   <222> (324)..(324)
   <223> n is a, c, g, or t
<400> 330
   cttcctcgga ggcagcattg ttaagggagg ccctgtgcaa gtgctggagg acgaggaact 60
   aaagtcccag ccagagcccc tagtggtcaa gggaaaacgg gtggctggag gccctcagat 120
   gatccagctc agcctggatg ggaagcgcct ctacatcacc acgtcgctgt acagtgcctg 180
   ggacaagcag ttttaccctg atctcatcag ggaaggctct gtgatgctgc aggttgatgt 240
   agacacagta aaaggagggc tgaagttgaa ccccaacttc ctggtggact tcgggaagga 300
   gccccttggc ccagcccttg cccnatgagc tccgctaccc tgggggcgat tgtagctctg 360
   acatctggat ttgaaggctc caccctcatc acccacactc cctattttgg gccctcactt 420
   ccttggggac ctggcttcat tctgctctct cttggcaccc gacccttggc agcatgtacc 480
   acacagccaa gctgagactg tggcaatgtg ttgagtcata tacatttact gaccactgtt 540
   gct 543
<210> 331
   <211> 507
   <212> DNA
   <213> human
<400> 331
   ccatcggtgc ggactccaag gaggtgcact ataagaaccg cttctggcat gacacctgct     60
   tccgctgtgc caagtgcctt caccccttgg ccaatgagac ctttgtggcc aaggacaaca    120
   agatcctgtg caacaagtgc accactcggg aggactcccc caagtgcaag gggtgcttca 180
   aggccattgt ggcaggagat caaaacgtgg agtacaaggg gaccgtctgg cacaaagact 240
   gcttcacctg tagtaactgc aagcaagtca tcgggactgg aagcttcttc cctaaagggg 300
   aggacttcta ctgcgtgact tgccatgaga ccaagtttgc caagcattgc gtgaagtgca 360
   acaagggttt ggtaaaggct ccagtgtggt ggcctatgaa ggacaatcct ggcacgacta 420
   ctgcttccac tgcaaaaaat gctccgtgaa tctggccaac aagcgctttg ttttccacca 480
   ggagcaagtg tattgtcccg actgtgc 507
<210> 332
   <211> 492
   <212> DNA
   <213> human
<400> 332
   ttgtcaaggg gctttgcatt caaactgctt ttccagggct atactcagaa gaaagataaa 60
   agtgtgatct aagaaaaagt gatggtttta ggaaagtgaa aatatttttg tttttgtatt 120
   tgaagaagaa tgatgcattt tgacaagaaa tcatatatgt atggatatat tttaataagt 180
   atttgagtac agactttgag gtttcatcaa tataaataaa agagcagaaa aatatgtctt 240
   ggttttcatt tgcttaccaa aaaaacaaca acaaaaaaag ttgtcctttg agaacttcac 300
   ctgctcctat gtgggtacct gagtcaaaat tgtcattttt gttctgtgaa aaataaattt 360
   ccttcttgta ccatttctgt ttagttttac taaaatctgt aaatactgta tttttctgtt 420
   tattccaaat ttgatgaaac tgacaatcca atttgaaagt ttgtgtcgac gtctgtctag 480
   cttaaatgaa tg 492
<210> 333
   <211> 251
   <212> DNA
   <213> human
<400> 333
   tcataagtga cttctacccg ggagccgtga cagtggcctg gaaggcagat agcagccccg 60
   tcaaggcggg agtggagacc accacaccct ccaaacaaag caacaacaag tacgcggcca 120
   gcagctatct gagcctgacg cctgagcagt ggaagtccca cagaagctac agctgccagg 180
   tcacgcatga agggagcacc gtggagaaga cagtggcccc tacagaatgt tcataggttc 240
   tcaaccctca c 251
<210> 334
   <211> 519
   <212> DNA
   <213> human
<220>
   <221> misc_feature
   <222> (118)..(118)
   <223> n is a, c, g, or t
<220>
   <221> misc_feature
   <222> (125)..(125)
   <223> n is a, c, g, or t
<220>
   <221> misc_feature
   <222> (133)..(133)
   <223> n is a, c, g, or t
<220>
   <221> misc_feature
   <222> (136)..(136)
   <223> n is a, c, g, or t
<400> 334
   gagtatacat cggtgcaggc ttcctggatg acagttgggt gatatgtgtc atgtggccta     60
   aaagcctcca tgtcatttga cctacgaatt ctatctttgg gaatttatcc taagaaanta    120
   cttanggatt tanttngtga taagatgttc atcccagcat tgcaatggag aaaaatggga    180
   agcaatggtt tggttgggaa tttattcctt ttctgctgta acgaaagttt gcaatagggg    240
   attgcttaag taaattattg tatctccatc cagatggtgg agtaccgcgc agacattaaa    300
   agtcatgtaa aagaacatct gactgaaaga aaaatgctcc ttgaatatta aaaggttgta    360
   aaaatagtgc atgttatgtg atttcaattt tgttttttaa aatatgggtg tatgcttgta    420
   tacgtagagc agataaaaaa gacggaaggc atactaaaaa atgttgagtg gttatctttg    480
   tatggtggaa caaagtcact gtaattttca tctttggtt                            519
<210> 335
   <211> 492
   <212> DNA
   <213> human
<220>
   <221> misc_feature
   <222> (254)..(254)
   <223> n is a, c, g, or t
<220>
   <221> misc_feature
   <222> (315)..(315)
   <223> n is a, c, g, or t
<220>
   <221> misc_feature
   <222> (388)..(388)
   <223> n is a, c, g, or t
<400> 335
   tggggctgag caaggcctac gtaggccaga agagcagctt cacagtagac tgcagcaaag     60
   caggcaacaa catgctgctg gtgggggttc atggcccaag gaccccctgc gaggagatcc    120
   tggtgaagca cgtgggcagc cggctctaca gcgtgtccta cctgctcaag gacaaggggg    180
   agtacacact ggtggtcaaa tggggggacg agcacatccc aggcagcccc taccgcgttg    240
   tggtgccctg agtntggggc ccgtgccagc cggcagcccc caagcctgcc ccgctaccca    300
   agcagccccg ccctnttccc ctcaaccccg gcccaggccg ccctggccgc ccgcctgtca    360
   ctgcagccgc ccctgccctg tgccgtgntg cgctcacctg cctccccagc cagccgctga    420
   cctctcggct ttcacttggg cagagggagc catttggtgg cgctgcttgt cttctttggt    480
   tctgggaggg gt                                                        492
<210> 336
   <211> 386
   <212> DNA
   <213> human
<220>
   <221> misc_feature
   <222> (30)..(30)
   <223> n is a, c, g, or t
<220>
   <221> misc_feature
   <222> (117)..(117)
   <223> n is a, c, g, or t
<220>
   <221> misc_feature
   <222> (123)..(124)
   <223> n is a, c, g, or t
<220>
   <221> misc_feature
   <222> (221)..(221)
   <223> n is a, c, g, or t
<220>
   <221> misc_feature
   <222> (331)..(331)
   <223> n is a, c, g, or t
<220>
   <221> misc_feature
   <222> (335)..(335)
   <223> n is a, c, g, or t
<220>
   <221> misc_feature
   <222> (338)..(338)
   <223> n is a, c, g, or t
<220>
   <221> misc_feature
   <222> (344)..(344)
   <223> n is a, c, g, or t
<220>
   <221> misc_feature
   <222> (354)..(354)
   <223> n is a, c, g, or t
<220>
   <221> misc_feature
   <222> (359)..(359)
   <223> n is a, c, g, or t
<400> 336
   aatgctctgg gtctctggat ccagtggggn atattgtgat gactcagtct ccactctccc     60
   tgcccgtcac ccctggagag ccggcctcca tctcctgcag gtctagtcag agcctcntgc    120
   atnntaatgg atacaactat ttggattggt acctgcagaa gccagggcag tctccacagc    180
   tcctgatcta tttgggttct aatcgggcct ccggggtccc ngacaggttc agtggcagtg    240
   gatcaggcac agattttaca ctgaaaatca gcagagtgga ggctgaggat gttggggttt    300
   attactgcat gcaagctcta caaactcctc ngacnttngg ccangggacc aagntggana    360
   tcaaacgaac tgtggctgca ccatct                                          386
<210> 337
   <211> 295
   <212> DNA
   <213> human
<220>
   <221> misc_feature
   <222> (136)..(136)
   <223> n is a, c, g, or t
<220>
   <221> misc_feature
   <222> (221)..(221)
   <223> n is a, c, g, or t
<400> 337
   gcaagatggt gttgcagacc caggtcttca tttctctgtt gctctggatc tctggtgcct     60
   acggggacat cgtgatgacc cagtctccag actccctggc tgtgtctctg ggcgagaggg    120
   ccaccatcaa ctgcangtcc agccagagtg ttttatacag ctccaacaat aagaactact    180
   tagcttggta ccagcagaaa ccaggacagc ctcctaagct nctcatttac tgggcatcta    240
   cccgggaatc cggggtccct gaccgattca gtggcagcgg gtctgggaca gattt         295
<210> 338
   <211> 470
   <212> DNA
   <213> human
<220>
   <221> misc_feature
   <222> (92)..(92)
   <223> n is a, c, g, or t
<220>
   <221> misc_feature
   <222> (126)..(126)
   <223> n is a, c, g, or t
<220>
   <221> misc_feature
   <222> (148)..(149)
   <223> n is a, c, g, or t
<220>
   <221> misc_feature
   <222> (166)..(167)
   <223> n is a, c, g, or t
<220>
   <221> misc_feature
   <222> (170)..(170)
   <223> n is a, c, g, or t
<220>
   <221> misc_feature
   <222> (182)..(182)
   <223> n is a, c, g, or t
<220>
   <221> misc_feature
   <222> (239)..(239)
   <223> n is a, c, g, or t
<220>
   <221> misc_feature
   <222> (269)..(269)
   <223> n is a, c, g, or t
<220>
   <221> misc_feature
   <222> (289)..(289)
   <223> n is a, c, g, or t
<220>
   <221> misc_feature
   <222> (291)..(293)
   <223> n is a, c, g, or t
<220>
   <221> misc_feature
   <222> (317)..(318)
   <223> n is a, c, g, or t
<220>
   <221> misc_feature
   <222> (320)..(320)
   <223> n is a, c, g, or t
<220>
   <221> misc_feature
   <222> (327)..(327)
   <223> n is a, c, g, or t
<220>
   <221> misc_feature
   <222> (329)..(330)
   <223> n is a, c, g, or t
<400> 338
   gaggtgcagc tgttggagtc tgggggaggc ttggtacagc ctggggggtc cctgagactc     60
   tcctgtgcag cctctggatt cacctttagc anctatgcca tgagctgggt ccgccaggct    120
   ccaggnaagg ggctggagtg ggtctcannt attagtggta gtggtnntan cacatactac    180
   gnagactccg tgaagggccg gttcaccatc tccagagaca attccaagaa cacgctgtnt    240
   ctgcaaatga acagcctgag agccgaggnc acggccgtat attactgtnc nnnagaaaat    300
   ggtgtcagca gttgganntn ccaaganann accccaacct atgttgacta ctggggccag    360
   gaaccctggt caccgtctcc tcagggagtg catccgcccc aacccttttc cccctcgtct    420
   cctgtgagaa ttccccgtcg gatacgaagc agcgtggccg ttggctgctc                470
<210> 339
   <211> 473
   <212> DNA
   <213> human
<400> 339
   cgtcagtcat cctttattgc agtcgggatc cttggggttg ttctgttggc cattttcgtc     60
   gcattattct tcttgactaa aaagcgaaga cagagacagc ggcttgcagt ttcctcaaga    120
   ggagagaact tagtccacca aattcaatac cgggagatga attcttgcct gaatgcagat    180
   gatctggacc taatgaattc ctcagaaaat tcccatgagt cagctgattt cagtgctgct    240
   gaactaattt ctgtgtctaa atttcttcct atttctggaa tggaaaagga ggccattctg    300
   agccacactg aaaaggaaaa tgggaattta taacccagtg agttcagcct ttaagatacc    360
   ttgatgaaga cctggactat tgaatggagc agaaattcac ctctctcact gactattaca    420
   gttgcatttt tatggagttc ttcttctcct aggattccta agactgctgc tga            473
<210> 340
   <211> 123
   <212> DNA
   <213> human
<400> 340
   ggggaatgga gcaaaacagt ctttgaatat cgaacacgca aggctgtgag actacctatt     60
   gtagatattg caccctatga cattggtggt cctgatcaag aatttggtgt ggacgttggc    120
   cct                                                                   123
<210> 341
   <211> 290
   <212> DNA
   <213> human
<400> 341
   cttgctctgt tgcagattgg cagatgccgc ctccctatgt ggtgctggac ttgccgcagg     60
   agaccctgga ggaggagacc cccggcgcca acctgtggcc caccaccatc accttcctca    120
   ccctcttcct gctgagcctg ttctatagca cagcactgac cgtgaccagc gtccggggcc    180
   catctggcaa cagggagggc ccccagtact gagcgggagc cggcaaggca cagggaggaa    240
   gtgtggagga acctcttgga gaagccagct atgcttgcca gaactcagcc              290
<210> 342
   <211> 276
   <212> DNA
   <213> human
<220>
   <221> misc_feature
   <222> (32)..(35)
   <223> n is a, c, g, or t
<220>
   <221> misc_feature
   <222> (203)..(203)
   <223> n is a, c, g, or t
<220>
   <221> misc_feature
   <222> (240)..(240)
   <223> n is a, c, g, or t
<400> 342
   gcataaatta atcagcccca gagtgcttta annnnttctc ttaaataccg gtgtctttga     60
   tcaggatgac atgtgccatt tttcagagga cgtgcagaca ggctggcatt ctagattact    120
   tttcttactc tgaaacatgg cctgtttggg agtgcgggat tcaaaggtgg tcccaccgct    180
   gcccctactg caaatggcag ttntaatctt atcttttggc ttctgcagat ggttgcaatn    240
   gatccttaac caataatggt cagtcctcat ctctgt                              276
<210> 343
   <211> 314
   <212> DNA
   <213> human
<220>
   <221> misc_feature
   <222> (150)..(150)
   <223> n is a, c, g, or t
<220>
   <221> misc_feature
   <222> (161)..(161)
   <223> n is a, c, g, or t
<220>
   <221> misc_feature
   <222> (185)..(185)
   <223> n is a, c, g, or t
<400> 343
   ctactctggc tccgaggtgc cagatgtgac atccagatga cccagtctcc atcctccctg     60
   tctgcatctg taggagacag agtcaccatc acttgccggg caagtcagag cattagcagc    120
   tatttaaatt ggtatcagca gaaaccaggn aaagccccta ngctcctgat ctatgctgca    180
   tccantttgc aaagtggggt cccatcaagg ttcagtggca gtggatctgg gacagatttc    240
   actctcacca tcagcagtct gcaacctgaa gattttgcaa cttactactg tcaacagagt    300
   tacagtaccc ctca                                                      314
<210> 344
   <211> 448
   <212> DNA
   <213> human
<220>
   <221> misc_feature
   <222> (28)..(28)
   <223> n is a, c, g, or t
<220>
   <221> misc_feature
   <222> (40)..(40)
   <223> n is a, c, g, or t
<220>
   <221> misc_feature
   <222> (49)..(50)
   <223> n is a, c, g, or t
<220>
   <221> misc_feature
   <222> (86)..(87)
   <223> n is a, c, g, or t
<220>
   <221> misc_feature
   <222> (99)..(99)
   <223> n is a, c, g, or t
<220>
   <221> misc_feature
   <222> (121)..(121)
   <223> n is a, c, g, or t
<220>
   <221> misc_feature
   <222> (139)..(139)
   <223> n is a, c, g, or t
<220>
   <221> misc_feature
   <222> (168)..(168)
   <223> n is a, c, g, or t
<220>
   <221> misc_feature
   <222> (180)..(180)
   <223> n is a, c, g, or t
<220>
   <221> misc_feature
   <222> (212)..(212)
   <223> n is a, c, g, or t
<220>
   <221> misc_feature
   <222> (226)..(226)
   <223> n is a, c, g, or t
<220>
   <221> misc_feature
   <222> (232)..(232)
   <223> n is a, c, g, or t
<220>
   <221> misc_feature
   <222> (236)..(236)
   <223> n is a, c, g, or t
<220>
   <221> misc_feature
   <222> (248)..(248)
   <223> n is a, c, g, or t
<220>
   <221> misc_feature
   <222> (255)..(255)
   <223> n is a, c, g, or t
<220>
   <221> misc_feature
   <222> (266)..(266)
   <223> n is a, c, g, or t
<220>
   <221> misc_feature
   <222> (314)..(314)
   <223> n is a, c, g, or t
<220>
   <221> misc_feature
   <222> (369)..(369)
   <223> n is a, c, g, or t
<220>
   <221> misc_feature
   <222> (371)..(371)
   <223> n is a, c, g, or t
<220>
   <221> misc_feature
   <222> (398)..(398)
   <223> n is a, c, g, or t
<400> 344
   gccgagtact ggaacagcca gaaggacntc ctggaagacn agcgggccnn ggtggacacc     60
   tactgcagac acaactacgg ggttgnngag agcttcacng tgcagcggcg agtccatcct    120
   naggtgactg tgtatcctnc aaagacccag cccctgcagc accacaanct cctggtctgn    180
   tctgtgagtg gtttctatcc aggcagcatt gnaagtcagg tggttncgga anggcnagga    240
   agagaagnct ggggntggtg tccacnggcc tgatccagaa tggagactgg accttccaga    300
   ccctggtgat gctngaaaca gttcctcgga gtggagaggt ttacacctgc caagtggagc    360
   acccaagcnt nacgagccct ctcacagtgg aatggagngc acggtctgaa tctgcacaga    420
   gcaagatgct gagtggagtc gggggctt                                       448
<210> 345
   <211> 550
   <212> DNA
   <213> human
<220>
   <221> misc_feature
   <222> (30)..(30)
   <223> n is a, c, g, or t
<220>
   <221> misc_feature
   <222> (42)..(42)
   <223> n is a, c, g, or t
<220>
   <221> misc_feature
   <222> (49)..(49)
   <223> n is a, c, g, or t
<220>
   <221> misc_feature
   <222> (58)..(58)
   <223> n is a, c, g, or t
<220>
   <221> misc_feature
   <222> (116)..(116)
   <223> n is a, c, g, or t
<220>
   <221> misc_feature
   <222> (119)..(119)
   <223> n is a, c, g, or t
<220>
   <221> misc_feature
   <222> (175)..(176)
   <223> n is a, c, g, or t
<220>
   <221> misc_feature
   <222> (178)..(178)
   <223> n is a, c, g, or t
<220>
   <221> misc_feature
   <222> (180)..(181)
   <223> n is a, c, g, or t
<220>
   <221> misc_feature
   <222> (183)..(183)
   <223> n is a, c, g, or t
<220>
   <221> misc_feature
   <222> (188)..(188)
   <223> n is a, c, g, or t
<220>
   <221> misc_feature
   <222> (225)..(225)
   <223> n is a, c, g, or t
<220>
   <221> misc_feature
   <222> (246)..(246)
   <223> n is a, c, g, or t
<220>
   <221> misc_feature
   <222> (248)..(248)
   <223> n is a, c, g, or t
<220>
   <221> misc_feature
   <222> (258)..(258)
   <223> n is a, c, g, or t
<220>
   <221> misc_feature
   <222> (268)..(268)
   <223> n is a, c, g, or t
<220>
   <221> misc_feature
   <222> (274)..(274)
   <223> n is a, c, g, or t
<220>
   <221> misc_feature
   <222> (285)..(287)
   <223> n is a, c, g, or t
<220>
   <221> misc_feature
   <222> (289)..(289)
   <223> n is a, c, g, or t
<220>
   <221> misc_feature
   <222> (299)..(299)
   <223> n is a, c, g, or t
<220>
   <221> misc_feature
   <222> (336)..(336)
   <223> n is a, c, g, or t
<220>
   <221> misc_feature
   <222> (364)..(364)
   <223> n is a, c, g, or t
<400> 345
   aagatcctgg agtttgtggg gcgctccctn ccagaggaga cngtggacnt catggttnag     60
   cacacgtcgt tcaaggagat gaagaagaac cctatgacca actacaccac cgtccnccng    120
   gagttcatgg accacagcat ctcccccttc atgaggaaag gcatggctgg ggacnngngn    180
   ngnccacntt caccgtggcg cagaatgagc gcttcgatgc ggacntatgc ggagaagatg    240
   gcaggncngc agcctcangc ttccgctntg agcngtgaga ggggnnncnt ggagtcacng    300
   cagagggagt gtgcgaatca aacctgacca agcggntcaa gaataaaata tgaattgagg    360
   gccngggacg gtaggtcatg tctgtaatcc cagcaatttg gaggctgagg tgggaggatc    420
   atttgagccc aggagttcga gaccaacctg ggcaacatag tgagattctg ttaaaaaaat    480
   aaaataaaat aaaaccaatt tttaaaaaga gaataaaata tgattgtggg ccaggcagag    540
   tggctcatgc                                                           550
<210> 346
   <211> 496
   <212> DNA
   <213> human
<220>
   <221> misc_feature
   <222> (150)..(151)
   <223> n is a, c, g, or t
<220>
   <221> misc_feature
   <222> (345)..(345)
   <223> n is a, c, g, or t
<220>
   <221> misc_feature
   <222> (347)..(348)
   <223> n is a, c, g, or t
<220>
   <221> misc_feature
   <222> (366)..(366)
   <223> n is a, c, g, or t
<220>
   <221> misc_feature
   <222> (405)..(405)
   <223> n is a, c, g, or t
<220>
   <221> misc_feature
   <222> (428)..(428)
   <223> n is a, c, g, or t
<400> 346
   ccggtcagca ggatcatcgt gcacccacag ttctacatca tccagactgg agcggatatc     60
   gccctgctgg agctggagga gcccgtgaac atctccagcc gcgtccacac ggtcatgctg    120
   ccccctgcct cggagacctt ccccccgggn ntgccgtgct gggtcactgg ctggggcgat    180
   gtggacaatg atgagcccct cccaccgcca tttcccctga agcaggtgaa ggtccccata    240
   atggaaaacc acatttgtga cgcaaaatac caccttggcg cctacacggg agacgacgtc    300
   cgcatcatcc gtgacgacat gctgtgtgcc gggaacaccc ggagngnntc atgccagggc    360
   gactcnggag ggcccctggt gtgcaaggtg aatggcacct ggctncaggc gggcgtggtc    420
   agctgggncg agggctgtgc ccagcccaac cggcctggca tctacacccg tgtcacctac    480
   tacttggact ggatcc                                                    496
<210> 347
   <211> 519
   <212> DNA
   <213> human
<220>
   <221> misc_feature
   <222> (326)..(326)
   <223> n is a, c, g, or t
<220>
   <221> misc_feature
   <222> (374)..(376)
   <223> n is a, c, g, or t
<220>
   <221> misc_feature
   <222> (398)..(401)
   <223> n is a, c, g, or t
<400> 347
   gaaccacctc aatgcaaaga ttctacggga aaatgtgggc cccctccacc tattgacaat     60
   ggggacatta cttcattccc gttgtcagta tatgctccag cttcatcagt tgagtaccaa    120
   tgccagaact tgtatcaact tgagggtaac aagcgaataa catgtagaaa tggacaatgg    180
   tcagaaccac caaaatgctt acatccgtgt gtaatatccc gagaaattat ggaaaattat    240
   aacatagcat taaggtggac agccaaacag aagctttatt tgagaacagg tgaatcagct    300
   gaatttgtgt gtaaacgggg atatcngtct ttcatcacgt tctcacacat tgcgaacaac    360
   atgttgggat gggnnnctgg agtatccaac ttgtgcannn ngatagaatc aatcataaaa    420
   tgcacacctt tattcagaac tttagtatta aatcagttct taatttcatt tttaagtatt    480
   gttttactcc tttttattca tacgtaaaat tttggatta                           519
<210> 348
   <211> 516
   <212> DNA
   <213> human
<220>
   <221> misc_feature
   <222> (161)..(162)
   <223> n is a, c, g, or t
<220>
   <221> misc_feature
   <222> (164)..(167)
   <223> n is a, c, g, or t
<220>
   <221> misc_feature
   <222> (170)..(170)
   <223> n is a, c, g, or t
<220>
   <221> misc_feature
   <222> (174)..(176)
   <223> n is a, c, g, or t
<220>
   <221> misc_feature
   <222> (179)..(179)
   <223> n is a, c, g, or t
<220>
   <221> misc_feature
   <222> (256)..(256)
   <223> n is a, c, g, or t
<220>
   <221> misc_feature
   <222> (364)..(370)
   <223> n is a, c, g, or t
<220>
   <221> misc_feature
   <222> (372)..(376)
   <223> n is a, c, g, or t
<220>
   <221> misc_feature
   <222> (465)..(465)
   <223> n is a, c, g, or t
<400> 348
   tcttccaatt tattatctgc accaattatt ttgggcactt ctcctgtgta tgtaaacttt     60
   ggagtttagg tctacgtcat ttatataaga gcctgtagac ctgagtggat ggacactgcc    120
   tcttagaact agaacttaga actttatctt gaaaatgtac nncnnnngcn gaannnccnc    180
   acagagtatg tgtcaggtag gaaccaaatt tatgattgtt atattcaaac tggttttctg    240
   ttccttgaaa atggtntttt ttctgatcaa agcctattta ggagttatta tgttcttgag    300
   tttattccat gtgtgtgaat attttgaatg aatccactaa taagatatgc atgtatgtct    360
   ggannnnnnn cnnnnnagaa taaatattta agtagacttt aacaagtaaa tggttgtagg    420
   catcattaag tcttaaatag ccttttcaaa gatattagtg ttctngtttt aattattcag    480
   ataattcctt taattctcca ctcaaatctg acttta                              516
<210> 349
   <211> 507
   <212> DNA
   <213> human
<400> 349
   gattcctgtg ggtccagctt tggaactggg aaacctttct tcggatccgc actcattcca     60
   ctgatgccag ctgcccctga aggatgccag tactgtggtg tgtgagtctc agcagccgcc    120
   cacacgctcc taactctgct gcatggcaga tgcctaggtg gaaatagcaa aaacaaggcc    180
   cgggctgggg ccagggccag aggggaaggc cctggattct cactcatgtg agatcttgaa    240
   tctctttctt tgttctgttt gtttagttag tatcatctgg taaaatagtt aaaaaacaac    300
   aaaaaactct gtatctgttt ctagcatgtg ctgcattgac tctattaatc acatttcaaa    360
   ttcaccctac attcctctcc tcttcactag cctctctgaa ggtgtcctgg ccagccctgg    420
   agaagcactg gtgtctgcag cacccctcag ttcctgtgcc tcagcccaca ggccactgtg    480
   ataatggtct gtttagcact tctgtat                                        507
<210> 350
   <211> 275
   <212> DNA
   <213> human
<220>
   <221> misc_feature
   <222> (129)..(129)
   <223> n is a, c, g, or t
<220>
   <221> misc_feature
   <222> (187)..(187)
   <223> n is a, c, g, or t
<220>
   <221> misc_feature
   <222> (191)..(191)
   <223> n is a, c, g, or t
<400> 350
   ctctggctcc caggtgccag atgtgccatc cagttgaccc agtctccatc ctccctgtct     60
   gcatctgtag gagacagagt caccatcact tgccgggcaa gtcagggcat tagcagtgct    120
   ttagcctgnt atcagcagaa accagggaaa gctcctaagc tcctgatcta tgatgcctcc    180
   agtttgnaaa ntggggtccc atcaaggttc agcggcagtg gatctgggac agatttcact    240
   ctcaccatca gcagcctgca gcctgaagat tttgc                               275
<210> 351
   <211> 339
   <212> DNA
   <213> human
<400> 351
   ccaactgcct gactgcttgt tggcctcacc ggtgggagct ccagcatctc ctttgctcga     60
   aatggacccc aactgctcct gtgccactgg tggctcctgc atgtgcgccg gctcctgcaa    120
   gtgcaaagag tgcaaatgca cctcctgcaa gaagagctgc tgctcctgct gccccgtggg    180
   ctgtgccaag tgtgcccagg gctgcatctg caaaggggcg ttggagaagt gcaactgcta    240
   tgcctgacgt ggggacagct ctgctcccag atgtaaatag agtaacctgc acaaacctgg    300
   attttttaaa aaatacaaca ctgagccatt tgcttcatt                          339
<210> 352
   <211> 259
   <212> DNA
   <213> human
<400> 352
   tcctccctgt ctgcatctgt aggagacaga gtcaccatca cttgccgggt gagtcagggc     60
   attagcagtt atttaaattg gtatcggcag aaaccaggga aagttcctaa gctcctgatc    120
   tatagtgcat ccaatttgca atctggagtc ccatctcggt tcagtggcag tggatctggg    180
   acagatttca ctctcactat cagcagcctg cagcctgaag atgttgcaac ttattacggt    240
   caacggactt acaatgccc                                                 259
<210> 353
   <211> 539
   <212> DNA
   <213> human
<400> 353
   tggaggaagt tctccagctt cagctcaact cacagcttct ccaagcatca ccctgggagt     60
   ttcctgaggg ttttctcata aatgagggct gcacattgcc tgttctgctt cgaagtattc    120
   aataccgctc agtattttaa atgaagtgat tctaagattt ggtttgggat caataggaaa    180
   gcatatgcag ccaaccaaga tgcaaatgtt ttgaaatgat atgaccaaaa ttttaagtag    240
   gaaagtcacc caaacacttc tgctttcact taagtgtctg gcccgcaata ctgtaggaac    300
   aagcatgatc ttgttactgt gatattttaa atatccacag tactcacttt ttccaaatga    360
   tcctagtaat tgcctagaaa tatctttctc ttacctgtta tttatcaatt tttcccagta    420
   tttttatacg gaaaaaattg tattgaaaac acttagtatg cagttgataa gaggaatttg    480
   gtataattat ggtgggtgat tattttttat actgtatgtg ccaaagcttt actactgtg     539
<210> 354
   <211> 358
   <212> DNA
   <213> human
<400> 354
   ccgccatttc ctctgaagca ggtgaaggtc cccataatgg aaaaccacat ttgtgacgca     60
   aaataccacc ttggcgccta cacgggagac gacgtccgca tcgtccgtga cgacatgctg    120
   tgtgccggga acacccggag ggactcatgc cagggcgact ccggagggcc cctggtgtgc    180
   aaggtgaatg gcacctggct gcaggcgggc gtggtcagct ggggcgaggg ctgtgcccag    240
   cccaaccggc ctggcatcta cacccgtgtc acctactact tggactggat ccaccactat    300
   gtccccaaaa agccgtgagt caggcctggg ttggccacct gggtcactgg aggaccaa      358
<210> 355
   <211> 273
   <212> DNA
   <213> human
<400> 355
   agtcgggccc aggactggtg aagccttcgg agaccctgtc cctcacctgc actgtctctg     60
   gtggctccat cagtagttac tactggagct ggatccggca gcccgccggg aagggactgg    120
   agtggattgg gcgtatctat accagtggga gcaccaacta caacccctcc ctcaagagtc    180
   gagtcaccat gtcagtagac acgtccaaga accagttctc cctgaagctg agctctgtga    240
   ccgccgcgga cacggccgtg tattactgtg cga                                 273
<210> 356
   <211> 162
   <212> DNA
   <213> human
<220>
   <221> misc_feature
   <222> (114)..(114)
   <223> n is a, c, g, or t
<220>
   <221> misc_feature
   <222> (119)..(119)
   <223> n is a, c, g, or t
<400> 356
   agtgggtctc agctattagt gggagtgcag gtaccacata ctacgcagac tccgtgaagg     60
   gccggttcac cacctccaga gacaattcca agaacacgct gtatctgcaa atgnacagnc    120
   tgagagccga ggacacggcc gtatattact gtgcgaaatc cc                       162
<210> 357
   <211> 288
   <212> DNA
   <213> human
<400> 357
   tcctccctgt ctgcatctgt aggagacaga gtcaccatca cttgccaggc gagtcaggac     60
   attagtaatt ttttaacttg gtatcagaag aagccaggga aagcccctaa agtcctgatc    120
   tacgatgcat ccaatttgga aacaggggtc ccatcaaggt tcagtggaag tggttctggg    180
   acagatttta ctttcaccat cgccagcctg cagcctgacg attttgcaac atattactgt    240
   caacaatata atgatcaccc cctcactttc ggccctggga ccaaagtg                 288
<210> 358
   <211> 356
   <212> DNA
   <213> human
<400> 358
   atgaaactga ttacaacagg ctgtaagaat caaagtcaac tgacatctat gctacatatt 60
   attatatagt ttgtactgag ctattgaagt cccattaact taaagtatat gttttcaaat 120
   tgccattgct actattgctt gtcggtgtat tttattttat tgtttttgac tttggaagag 180
   atgaactgtg tatttaactt aagctattgc tcttaaaacc agggatcaga atatatttgt 240
   aagttaaatc attggtgcta ataataaatg tggattttgt attaaaatat atagaagcaa 300
   tttctgttta catgtccttg ctacttttaa aaacttgcat ttattcctca gatttt 356
<210> 359
   <211> 247
   <212> DNA
   <213> human
<400> 359
   catctcctgc actggaacca gcagtgacgt tggtgcttat aactatgtct cctggtacca 60
   acagcaccca ggcaaagccc ccaaactcat gatttatgag gtcagtaatc ggccctcagg 120
   ggtttctaat cgcttctctg gctccaagtc tggcaacacg gcctccctga ccatctctgg 180
   gctccaggct gacgacgagg gtgattacta ctgcatctca tatacaagta gcaaccctct 240
   cgtggtt 247
<210> 360
   <211> 548
   <212> DNA
   <213> human
<400> 360
   tcaagtggga agagcgctgt tcaaggacca cctgagcgtg acctctgtgg ctgctacagc 60
   gtgttccagt gtcctgccgg gctgtgccga gccatggaac catggggaga ccttcacttg 120
   cactgctgcc caccccgagt tgaagacccc actaaccgcc aacatcacaa aatccggaaa 180
   cacattccgg cccgaggtcc acctgctgcc gccgccgtcg gaggagctgg ccctgaacga 240
   gctggtgacg ctgacgtgcc tggcacgtgg cttcagccca aggatgtgct ggttcgctgg 300
   ctgcaggggt cacaggagct gccccgcgag aagtacctga cttgggcatc ccggcaggag 360
   cccagccagg gcaccaccac cttcgctgtg accagcatac tgcgcgtggc agccgaggac 420
   tggaagaagg gggacacctt ctcctgcatg gtgggccacg aggccctgcc gctggccttc 480
   acacagaaga ccatcgaccg cttggcgggt aaacccaccc atgtcaatgt gtctgttgtc 540
   atggcgga 548
<210> 361
   <211> 257
   <212> DNA
   <213> human
<400> 361
   gctgtcagga ttcttcaagg agaatgaata ctgggaatca agacaggact ataccttatc 60
   cataggcgca ggtgcacagg gggaggccat aaagatcaaa catgcatgga tgggtcctca 120
   cgcagacaca cccacagaag gacactagcc tggcgcgcgt gcacacacac acacacacac 180
   acgagttcat aatgtggtga tggccctaag ttaagcaaaa tgcttctgca cacaaaactc 240
   tctggtttac ttcaaat 257
<210> 362
   <211> 277
   <212> DNA
   <213> human
<400> 362
   gcacctgagc atgaaactcg acgcgttctt cggcatcttc tttggggccc tgggcggcct     60
   cttgctgctg ggggtcggga cgttcgtggt cctgcgcttc tggggttgct ccggggccag    120
   gttctcctat ttcctgaact cagctgaggc cttgccttga aggggcagct gtggcctagg    180
   ctacctcaag actcacctca tccttaccgc acatttaagg cgccattgct tttgggagac    240
   tggaaaaggg aaggtgactg aaggctgtca ggattct                             277
<210> 363
   <211> 140
   <212> DNA
   <213> human
<220>
   <221> misc_feature
   <222> (33)..(33)
   <223> n is a, c, g, or t
<220>
   <221> misc_feature
   <222> (44)..(44)
   <223> n is a, c, g, or t
<220>
   <221> misc_feature
   <222> (64)..(65)
   <223> n is a, c, g, or t
<220>
   <221> misc_feature
   <222> (99)..(100)
   <223> n is a, c, g, or t
<400> 363
   catctcctgc actggaacca gcagtgacgt tgntggttat aacnatgtct cctggtacca     60
   acannaccca ggcaaagccc ccaaactcat gatttatgnn gtcagtaatc ggccctcagg    120
   ggtttctaat cgcttctctg                                                140
<210> 364
   <211> 113
   <212> DNA
   <213> human
<400> 364
   gcgctggttc ctgcaagtgc aaagagtgca aatgcacctc ctgcaagaag agctgctgct     60
   cctgctgccc cgtgggctgt agcaagtgtg cccagggctg tgtttgcaaa ggg          113
<210> 365
   <211> 266
   <212> DNA
   <213> human
<400> 365
   aggacagagg gtcaccatct cttgctctgg aagcagctcc aacattggga agaattatgt     60
   atcctggtac cagcaactcc caggaacagc ccccaaactc ctcatctata aaaataataa    120
   gcgaccctca gggattcctg accgattctc tggctccaag tctggcacgt cagccaccct    180
   gggcatcacc agactccaga ctggggacga ggccgattat tactgcggaa catgggatag    240
   cagcctgagt ggtggccggg ggatat                                         266
<210> 366
   <211> 434
   <212> DNA
   <213> human
<400> 366
   tgaggagaag tctgccgtta ctgccctgtg gggcaaggtg aacgtggatg aagttggtgg     60
   tgaggccctg ggcaggctgc tggtggtcta cccttggacc cagaggttct ttgagtcctt    120
   tggggatctg tccactcctg atgctgttat gggcaaccct aaggtgaagg ctcatggcaa    180
   gaaagtgctc ggttctttga gtcctttggg gatctgtcca ctcctgatgc tgttatgggc    240
   aaccctaagg tgaaggctca tggcaagaaa gtgctcggtg cctttagtga tggcctggct    300
   cacctggaca acctcaaggg cacctttgcc acactgagtg agctgcactg tgacaagctg    360
   cacgtggatc ctgagaactt caggctcctg ggcaacgtgc tggtctgtgt gctggcccat    420
   cactttggca aaga                                                      434
<210> 367
   <211> 273
   <212> DNA
   <213> human
<400> 367
   cagtgtccgg atctcctgga cagtcagtca ccatctcctg caccggaacc agcagtgatg     60
   ttggtggtta taactatgtc tcctggtacc aacagcaccc aggcaaagcc cccacactca    120
   tgatttatga tgtcattaag cggccctcag gggtccctga tcgcttctct ggctccaaat    180
   ctggcaacac ggcctccctg accatctctg ggctccaggc tgaggatgag gctgattatt    240
   attgcagctc aaatgcaggc agtttttatg tct                                 273
<210> 368
   <211> 233
   <212> DNA
   <213> human
<400> 368
   tcctgcactg ggagcagctc ctacatcggg gcaggttatg atgtacactg gtaccggcaa     60
   cttcccggca cagcccccaa actcctcatc tatggtaaca ccgatcggcc ctcaggggtc    120
   cctgaccgat tctctggctc caagtctggc atctcagcct ccctggccat cactgggctc    180
   caggctgagg atgaggctga ttattactgc aagtcctatg acagcagcct gag           233
<210> 369
   <211> 256
   <212> DNA
   <213> human
<400> 369
   gatgtgacat ccaggtgacc cagtctccat cttccctgtc tgcgtctgta ggagacagag     60
   tcaccatcac ctgccgggca agtcagggca ttagcaatgg gttatcctgg tatcagcaga    120
   aaccagggca agcccctacg ctcctgatct atgctgcatc cagtttgcag tcgggggtcc   180
   catctcggtt cagtggcagt ggatctggga cagatttcac tctcaccatc agcagcctgc    240
   agcctgaaga tgttgc                                                    256
<210> 370
   <211> 434
   <212> DNA
   <213> human
<400> 370
   ggtccccaca gactcagaga gaacccacca tggtgctgtc tcctgccgac aagaccaacg    60
   tcaaggccgc ctggggtaag gtcggcgcgc acgctggcga gtatggtgcg gaggccctgg   120
   agaggcactt cgacctgagc cacggctctg cccaagttaa gggccacggc aagaaggtgg   180
   ccgacgcgct gaccaacgcc gtggcgcacg tggacgacat gcccaacgcg ctgtccgccc   240
   tgagcgacct gcacgcgcac aagcttcggg tggacccggt caacttcaag ctaagccact   300
   gcctgctggt gaccctggcc gcccacctcc ccgccgagtt cacccctgcg gtgcacgctt   360
   ccctggacaa gttcctggct tctgtgagca ccgtgctgac ctccaaatac cgttaagctg   420
   gagcctcggt agcc                                                     434
<210> 371
   <211> 270
   <212> DNA
   <213> human
<400> 371
   gctgccaggt gccagatgtg acatccagat gacccagtct ccatcctccc tgtctgcatc    60
   tgtaggagac agagtcacca tcacttgccg ggcgagtcag ggcattagca ataatttaaa   120
   ttggtatcag cagaaaccag ggaaaactcc taagctcctg atctatgctg cacccagtct   180
   gcaaagtggg attccctctc ggttcagtga cagtggatct ggggcagatt acactctcac   240
   catccgcagc ctgcagcctg aagattttgc                                    270
<210> 372
   <211> 319
   <212> DNA
   <213> human
<400> 372
   agtcgctcca tttatcgctt gagatctcca gccttaccgc ggctcgaaat ggaccccaac    60
   tgctcctgca ccactggtgt ctcctgcgcc tgcaccggct cctgcaagtg caaagagtgc   120
   aaatgcacct cctgcaagaa gagctgctgc tcctgctgcc ccgtgggctg tgccaagtgt   180
   gcccacggct gtgtctgcaa agggacgttg gagaactgca gctgctgtgc ctgatgtggg   240
   aacagctctt ctcccagatg ttaatagaac aagctgcaca acctggattt tttttcaata   300
   cgatactgag ccatttgct                                                319
<210> 373
   <211> 434
   <212> DNA
   <213> human
<400> 373
   cctccagacc tccttgaaat acaatattct cccagaaaag gaagagttcc cctttgcttt    60
   aggagtgcag actctgcctc aaacttgtga tgaacccaaa gcccacacca gcttccaaat   120
   ctccctaagt gtcagttaca cagggagccg ctctgcctcc aacatggcga tcgttgatgt   180
   gaagatggtc tctggcttca ttcccctgaa gccaacagtg aaaatgcttg aaagatctaa   240
   ccatgtgagc cggacagaag tcagcagcaa ccatgtcttg atttaccttg ataaggtgtc   300
   aaatcagaca ctgagcttgt tcttcacggt tctgcaagat gtcccagtaa gagatctcaa   360
   accagccata gtgaaagtct atgattacta cgagacggat gagtttgcaa tcgctgagta   420
   caatgctcct tgca                                                     434
<210> 374
   <211> 500
   <212> DNA
   <213> human
<220>
   <221> misc_feature
   <222> (77)..(77)
   <223> n is a, c, g, or t
<220>
   <221> misc_feature
   <222> (181)..(181)
   <223> n is a, c, g, or t
<220>
   <221> misc_feature
   <222> (207)..(207)
   <223> n is a, c, g, or t
<400> 374
   ctattaccat tctaactttc ataaaaagtt gggatcaaga agcagctgat ttcctgccag     60
   ggcttatatt agggggntga ttcttaaagg acattaggat tggtgctcag aaatggttaa    120
   tcatgctgtg tgctagccag ggccagctgg taccttcttt gccatgagca ttcaagggac    180
   ngctaacctt tattgacaat ctatatngca aaagtcagga aagaggttgt gagctgattg    240
   gattaaagac ctggcacttc agtaactcag cacgcttcca cttcactcaa cttaagagag    300
   ttcattgaca gtgttaggat gtgaaggctg ggaaacactt attttgcttc aagagttcca    360
   cttggctctc ccaaataggt acctcaaaaa ctgttagcaa gcggcatttg gatgtcttga    420
   caggggcttt gcagggattt ttagggtttt ttccacattg tccacattaa tggttggcat    480
   gattgtgctt gcaggccaag                                                500
<210> 375
   <211> 542
   <212> DNA
   <213> human
<400> 375
   gaatatgacg ttaccttgca gactaaaagg ttgaaggccc gaaactaact tttagctaac     60
   aataagggct gtgccccaat ggaaactgag ttcattttct gagaaaggtt tggatgactg    120
   aaatatttcc tctacagtca aggactttgg catgcggtgg ctgaaactga gcttttttgt   180
   gtgggctcca gttctcactg ttctgcaatg ctcatggcaa gttgaatggt gagctagctt    240
   ataaattaaa gagctctgaa ctgtattcag accgactggg tatctagctt actgttttaa    300
   catcattgtt gaaaccagac cctgtagtcc agtggtgctg ccctgttgtg caaactgctc    360
   ctttttctcg tgtttttgta aagagcttcc atctgggctg gacccagttc ttgcacatac    420
   aagacaccgc tgcagtcagc taggaccttt ccgccatgta ttctattctg tagtaaagca    480
   tttccatcaa caatgcctaa ttgtatctgt tatttttggt ttaacacaca ctgattcata    540
   ct                                                                   542
<210> 376
   <211> 541
   <212> DNA
   <213> human
<400> 376
   ggtctacgcc tattacaacc tggaggaaag ctgtacccgg ttctaccatc cggaaaagga     60
   ggatggaaag ctgaacaagc tctgccgtga tgaactgtgc cgctgtgctg aggagaattg    120
   cttcatacaa aagtcggatg acaaggtcac cctggaagaa cggctggaca aggcctgtga    180
   gccaggagtg gactatgtgt acaagacccg actggtcaag gttcagctgt ccaatgactt    240
   tgacgagtac atcatggcca ttgagcagac catcaagtca ggctcggatg aggtgcaggt    300
   tggacagcag cgcacgttca tcagccccat caagtgcaga gaagccctga agctggagga    360
   gaagaaacac tacctcatgt ggggtctctc ctccgatttc tggggagaga agcccaacct    420
   cagctacatc atcgggaagg acacttgggt ggagcactgg cctgaggagg acgaatgcca    480
   agacgaagag aaccagaaac aatgccagga cctcggcgcc ttcaccgaga gcatggttgt    540
   c                                                                    541
<210> 377
   <211> 485
   <212> DNA
   <213> human
<400> 377
   cccctgcata tcttctcagc aataactcca tgggctctgg gaccctaccc cttccaacct     60
   tccctgcttc tgagacttca atctacagcc cagctcatcc agatgcagac tacagtccct    120
   gcaattgggt ctctggcagg caatagttga aggactcctg ttccgttggg gccagcacac    180
   cgggatggat ggagggagag cagaggcctt tgcttctctg cctacgtccc cttagatggg    240
   cagcagaggc aactcccgca tcctttgctc tgcctgtcgg tggtcagagc ggtgagcgag    300
   gtgggttgga gactcagcag gctccgtgca gcccttggga acagtgagag gttgaaggtc    360
   ataacgagag tgggaactca acccagatcc cgcccctcct gtcctctgtg ttcccgcgga    420
   aaccaaccaa accgtgcgct gtgacccatt gctgttctct gtatcgtgat ctatcctcaa    480
   caaca                                                                485
<210> 378
   <211> 512
   <212> DNA
   <213> human
<400> 378
   ctcttttact actgaccttt cacagagaaa aaatatttcc cttgaaaaaa actgggcttg     60
   tcattttttc ccttgtagct ttaagcagag acataagtgc cttgcattac acatagtaaa    120
   ctttctttaa aaaaaaaaaa aaaagatttt ggagactacc agggtaagat tccaacttgt    180
   ccaaaagctt tctggcctta catattttat tataaaaatt ctcaagtctg gtaatcttct    240
   atgtcagagc tagtgatttc aaaaggtttc acaattcccc aagacaaaag tgattttcgt    300
   tcattataat aaggttaagt gatatgtgat tcataacaat tttgatgtga agaagggaag    360
   gacatcattg acttaataat agtatcagtc ggtgcaacag ttggcaacat gtgccttcac    420
   actttaccat aaagagacgg gtttgagggt ttgccttcta aagtctgcaa cttcaagaaa    480
   aaaaatcgac actgtggatt gactttcccg gt                                  512
<210> 379
   <211> 520
   <212> DNA
   <213> human
<400> 379
   aactttgtat agcccatgta cctaccttgt atagaaaaat aattttaaaa atttgaatgg     60
   aagggggtaa aggaagtcat gaagtttttt tgcattttta tttaaatgaa ggaattccaa    120
   ataactcacc tacagatttt tagcacaaaa atagccattg taaagtgtta aaatttacga    180
   taagtattct attggggagg aaaggtaact ctgatctcag ttacagtttt tttttccttt    240
   ttaatttcat tattttgggt ttttggtttt tgcagtccta tttatctgca gtcgtattaa    300
   gtcctattgc tagaataggt tactacaaaa aaggttatat tctgaaagaa aaataactga    360
   cattatatat aaccaattaa tttaaagtat tgccatttaa attacacact gagagcatgt    420
   cctatgcaga catagatttt tctgttcatt tatttttctt cattgcagtg gattgatttg    480
   ataaatagat gtgttgaatt actacatttg ctgtacatat                          520
<210> 380
   <211> 448
   <212> DNA
   <213> human
<400> 380
   ggagctagcc ttgttttttg catctttctc actcccatac atttatatta tatccccact    60
   aaatttcttg ttcctcattc ttcaaatgtg ggccagttgt ggctcaaatc ctctatattt    120
   ttagccaatg gcaatcaaat tctttcagct cctttgtttc atacggaact ccagatcctg    180
   agtaatcctt ttagagcccg aagagtcaaa accctcaatg ttccctcctg ctctcctgcc    240
   ccatgtcaac aaatttcagg ctaaggatgc cccagaccca gggctctaac cttgtatgcg    300
   ggcaggccca gggagcaggc agcagtgttc ttcccctcag agtgacttgg ggagggagaa    360
   ataggaggag acgtccagct ctgtcctctc ttcctcactc ctcccttcag tgtcctgagg    420
   aacaggactt tctccacatt gttttgta                                       448
<210> 381
   <211> 547
   <212> DNA
   <213> human
<220>
   <221> misc_feature
   <222> (159)..(159)
   <223> n is a, c, g, or t
<220>
   <221> misc_feature
   <222> (240)..(240)
   <223> n is a, c, g, or t
<220>
   <221> misc_feature
   <222> (299)..(299)
   <223> n is a, c, g, or t
<220>
   <221> misc_feature
   <222> (312)..(312)
   <223> n is a, c, g, or t
<400> 381
   gcgggtcttg ctggctaaaa tgcccaggta aagggttggt tggacacagc gcttagtgca     60
   cgctgtcatc atggacatca taatcagttg tgaaaaacac gcgaacctat gacacttctt    120
   attccacact gaatgtgaaa ttgcatgttc agatgtttna ctacgaggcc tggctcacag    180
   gaagtgttca gtaaaagtat gcactgttag attactgata acgcggatag atttttgttn    240
   accataaatt gttccagatt tatattaatg gaaggaagtg tgcatttatt agctattant    300
   caactttaca angcaaacat cttatttctc atctttaaac atgtcgacca gtttaattga    360
   aaagtattct gagactgcaa aatggggtgt taaaaaatac tgcagttacg gagctgtgta    420
   aaccagtttc tcattgcata agatacagat gtaaattgca tggagaggtt gatatgcacc    480
   tgtacagtaa ttcactcccc catttcactt ctttgtcaga gaatagttct tgttcatact    540
   gagtgtt                                                              547
<210> 382
   <211> 425
   <212> DNA
   <213> human
<400> 382
   gcatccgagg ggagccgccg gatgtggaag aagactcggc tttcctgcag ccatttagtg     60
   ccgccccatg ctaggttatt tgacattgtg cagtgtagag ttgccttaaa gtgcgtgatc    120
   tgccagtgct ttcttcaagt cacccttgcc ccgattcctc atgtttgcgc tccccagggt    180
   tgctcaagtg gaaattttgt cagctgttta gccttttcgt acttggcgtg atgtcaactt    240
   cacttctaat ctgcaaaagc agaagctgtt tcctagttta cctcgcgtgt gtttacctat    300
   atggagtagc tcgcagagat cacagaaatg cttgcagcct aaggcagggt tttcagaccg    360
   tgggtcccag cccatttagt aaaatgggaa atcaattagc aagtggtcac cagcattaca    420
   cagca                                                                425
<210> 383
   <211> 551
   <212> DNA
   <213> human
<400> 383
   tatacagttt aataagcctc ttgcaagtta cttgttctct cacctgaggt atttttttcc     60
   tccccacctt gcccctgttc ctcccttcct cttctccctt tgcaagagga aatatttaac    120
   atatttgggt ccaacttcaa taatgtaata attaatacat taaaagcatt taacttcctt    180
   tctagaaaaa tgcacaggct aaggcataga caaaacaaag agaaatgctg agaaatttgc    240
   cactggagac aagcaatctg aataaatatt tgccaaaagt tctttttatg tcatatagtg    300
   tcaggatttg aaggagctat tttttttaat gttgcaacta gcaactcatc ttcggaagac    360
   acagccagga gaatgaagta gaagtgaaag gtttataaat ccatttgtaa gcatttatcc    420
   catatatttt aaattcaaga aaaattgtgt ttatctttag aattttgtat tcaatacttt    480
   atgtactatg tgactcatgc ttctggataa ataaagcacc aaatatgtat ctgtaaccac    540
   aatcacacat a                                                         551
<210> 384
   <211> 468
   <212> DNA
   <213> human
<400> 384
   ggcttgatgt agactggctt gctttgattt ttagtgaagg gaatgtacgt aaaacaaaat     60
   agggcttggc tggtcaaagg agacaagcag gatggatgga tggatggatg aatagataga    120
   tggtgtttgc atgtaaattg cagagaaaac aaaaccaaag ctgattggaa acaattaatt    180
   gtgggtgtct gagggggaag gtcgcagctt tgggcagctt tgagaagcgg tacaagagct    240
   ctgtgcctgt gtgtccagcc ctggagccag ccagtgcatt tattttaagc tcttagaagc    300
   aactccttgg cccaggaatg cgtgacccct gagatgggtc cacgcatctc tctacacgtc    360
   cttctctccg tgggatactg gactcgtgcc tctgcgccca ttctcttctc acgcatatcc    420
   atgagcttta atttcacttt ctgatcacgg tacgtccata aagccagt                 468
<210> 385
   <211> 355
   <212> DNA
   <213> human
<400> 385
   ggagatgact taagttggca gcagtaatct tcttttagga gcttgtacca cagtcttgca     60
   cataagtgca gatttgcccc aagtaaagag aatttcctca acactaactt cacggggata   120
   atcaccacgt aactaccctt aaagcatatc actagccaaa gaggggaata tctgttcttc    180
   ttactgtgcc tatattaaga ctagtacaaa tgtggtgtgt cttccaactt tcattgaaaa    240
   tgccatatct ataccatatt ttattcgagt cactgatgat gtaatgatat attttttcat    300
   tattatagta gaatattttt atggcaagag atttgtggtc ttgatcatac ctatt         355
<210> 386
   <211> 551
   <212> DNA
   <213> human
<400> 386
   gaagctgaca caccgtatgt tgttagagtc ttttatctgg tcaggggaaa caaaatcttg     60
   acccagctga acatgtcttc ctgagtcagt gcctgaatct ttatttttta aattgaatgt    120
   tccttaaagg ttaacatttc taaagcaata ttaagaaaga ctttaaatgt tattttggaa    180
   gacttacgat gcatgtatac aaacgaatag cagataatga tgactagttc acacataaag    240
   tccttttaag gagaaaatct aaaatgaaaa gtggataaac agaacattta taagtgatca    300
   gttaatgcct aagagtgaaa gtagttctat tgacattcct caagatattt aatatcaact    360
   gcattatgta ttatgtctgc ttaaatcatt taaaaacggc aaagaattat atagactatg    420
   aggtaccttg ctgtgtagga ggatgaaagg ggagttgata gtctcataaa actaatttgg    480
   cttcaagttt catgaatctg taactagaat ttaattttca ccccaataat gttctatata    540
   gcctttgcta a                                                         551
<210> 387
   <211> 433
   <212> DNA
   <213> human
<400> 387
   gttcaaaata tttccaagcc tgagtattgt ctattggtat agatttttag aaatcaataa     60
   ttgattattt atttgcactt attacaatgc ctgaaaaagt gcaccacatg gatgttaagt    120
   agaaattcaa gaaagtaaga tgtcttcagc aactcagtaa aaccttacgc caccttttgg    180
   tttgtaagag gttttttata catttcaaac aggttgcaca aaagttaaaa taatggggtc    240
   ttttataaat ccaaagtact gtgaaaacat tttacatatt ttttaaatct tctgactaat    300
   gctaaaacgt aatctaatta aatttcatac agttactgca gtaagcatta ggaagtgaat    360
   atgatataca aaatagttta taaagactct atagtttcta taatttattt tactggcaaa    420
   tgtcatgcaa caa                                                       433
<210> 388
   <211> 493
   <212> DNA
   <213> human
<400> 388
   ggcagcccac aagtttctcg tggggagatg gaggcagagc ccagggtagg ggacagagct     60
   gctggggcct ttccttgcct gggaatctgt cccaggaaga gcttccccac tcccatcccc    120
   caaattggaa aaaccgtaca ttcaagcctg tttggccctg aaattcttaa gaatctggtt    180
   aagaattaac tcactaatgt caaaagtcaa aacctcctag gggttgtcct gggagtcagg    240
   ttcacgggta cagaagatga atctcagatg tcactcaacc tgagccgtca ttctctgtgg    300
   cagggctgcc ctgggtttct cttactcaat ccctggagtg taagcatttg gattgtgtca    360
   cagattacct ttttaccttt tctttctttt tttttctttt tttcaatatc agtgcccaca    420
   ccttactgag tattgagttt tagagctttc gcttgatgtg cttgaccaag agacttcttt    480
   tgtatccttt tct                                                       493
<210> 389
   <211> 473
   <212> DNA
   <213> human
<400> 389
   tcaagtgcgt tctttagacc aatgcattgc gtttctttcc ctgcttttga gatagtagga     60
   agagttcttg gtggtgtccc cccccttcaa ttcttcagtt gtatagtagt tatagggaag    120
   atatgggtgt ttttctttat tattactttt ttttttctgc aggtcagtaa aaggatttaa    180
   gttgcactga caaaaatacc aaaataaaag tgtattttta agttcccatt tgaaattgct    240
   ggcgctgctg gccggatgca tttttgagtt tgtattagtt gataaattaa cagtaataac    300
   aagattgtat gaaccgcatg gtgcttgcag ttttaaatat tgtggatatt tgtcctgcat    360
   cagaaacgag ctttggtttt tacagattca actgtgttga aatcaaacct gccgcaacag    420
   aaattgtttt tatttcatgt aaaataaggg atcaatttca aaccctgctt atg           473
<210> 390
   <211> 495
   <212> DNA
   <213> human
<400> 390
   cctcctgcct ctggatttta ggtgttgatt tctggatcac gggataccac ttcctgtcca      60
   caccccgacc aggggctaga aaatttgttt gagattttta tatcatcttg tcaaattgct     120
   tcagttgtaa atgtgaaaaa tggctggggg aaaggaggtg gtgtccctaa ttgttttact    180
   tgttaacttg ttcttgtgcc cctgggcact tggcctttgt ctgctctcag tgtcttccct     240
   ttgacatggg aaaggagttg tggccaaaat ccccatcttc ttgcacctca acgtctgtgg     300
   ctcagggctg gggtggcaga gggaggcctt caccttatat ctgtgttgtt atccagggct     360
   ccagacttcc tcctctgcct gccccactgc accctctccc ccttatctat ctccttctcg     420
   gctccccagc ccagtcttgg cttcttgtcc cctcctgggg tcatccctcc actctgactc     480
   tgactatggc agcag                                                      495
<210> 391
   <211> 482
   <212> DNA
   <213> human
<400> 391
   ggcatgtgtg actgtttcag cgactgcgga gtctgtctct gtggcacatt ttgtttcccg      60
   tgccttgggt gtcaagttgc agctgatatg aatgaatgct gtctgtgtgg aacaagcgtc     120
   gcaatgagga ctctctacag gacccgatat ggcatccctg gatctatttg tgatgactat     180
   atggcaactc tttgctgtcc tcattgtact ctttgccaaa tcaagagaga tatcaacaga     240
   aggagagcca tgcgtacttt ctaaaaactg atggtgaaaa gctcttaccg aagcaacaaa     300
   attcagcaga cacctcttca gcttgagttc ttcaccatct tttgcaactg aaatatgatg     360
   gatatgctta agtacaactg atggcatgaa aaaaatcaaa tttttgattt attataaatg     420
   aatgttgtcc ctgaacttag ctaaatggtg caacttagtt tctccttgct ttcatattat     480
   cg                                                                    482
<210> 392
   <211> 566
   <212> DNA
   <213> human
<220>
   <221> misc_feature
   <222> (124)..(124)
   <223> n is a, c, g, or t
<400> 392
   agagaatgcc cttctcctta ttgtaaccct gtctggatcc tatcctccta cctccaaagc      60
   ttcccacggc ctttctagcc tggctatgtc ctaataatat cccactggga gaaaggagtt     120
   ttgncaaagt gcaaggacct aaaacatctc atcagtatcc agtggtaaaa aggcctcctg     180
   gctgtctgag gctaggtggg ttgaaagcca aggagtcact gagaccaagg ctttctctac     240
   tgattccgca gctcagaccc tttcttcagc tctgaaagag aaacacgtat cccacctgac     300
   atgtccttct gagcccggta agagcaaaag aatggcagaa aagtttagcc cctgaaagcc     360
   atggagattc tcataacttg agacctaatc tctgtaaagc taaaataaag aaatagaaca     420
   aggctgagga tacgacagta cactgtcagc agggactgta aacacagaca gggtcaaagt     480
   gttttctctg aacacattga gttggaatca ctgtttagaa cacacacact tactttttct     540
   ggtctctacc actgctgata ttttct                                          566
<210> 393
   <211> 489
   <212> DNA
   <213> human
<400> 393
   attaccttat catgtcttag agcccgtctt tatgtttaaa actaatttct taaaataaag     60
   ccttcagtaa atgttcatta ccaacttgat aaatgctact cataagagct ggtttggggc    120
   tatagcatat gctttttttt ttttaattat tacctgattt aaaaatctct gtaaaaacgt    180
   gtagtgtttc ataaaatctg taactcgcat tttaatgatc cgctattata agcttttaat    240
   agcatgaaaa ttgttaggct atataacatt gccacttcaa ctctaaggaa tatttttgag    300
   atatcccttt ggaagacctt gcttggaaga gcctggacac taacaattct acaccaaatt    360
   gtctcttcaa atacgtatgg actggataac tctgagaaac acatctagta taactgaata    420
   agcagagcat caaattaaac agacagaaac cgaaagctct atataaatgc tcagagttct    480
   ttatgtatt                                                            489
<210> 394
   <211> 506
   <212> DNA
   <213> human
<400> 394
   atggggctgg ggacttgaat tggatgcttc aaaaccatca cctgttggcc aacaagtttg     60
   acccaaaggt agatgataat gctcttcagt gcttagaaga atacctacgt tataaggcca    120
   tctatgggac tgaactttga gacacactat gagagcgttg ctacctgtgg ggcaagagca    180
   tgtacaaaca tgctcagaac ttgctgggac agtgtgggtg ggagaccagg gctttgcaat    240
   tcgtggcatc ctttaggata agagggctgc tattagattg tgggtaagta gatcttttgc     300
   cttgcaaatt gctgcctggg tgaatgctgc ttgttctctc acccctaacc ctagtagttc     360
   ctccactaac tttctcacta agtgagaatg agaactgctg tgatagggag agtgaaggag    420
   ggatatgtgg tagagcactt gatttcagtt gaatgcctgc tggtagcttt tccattctgt    480
   ggagctgccg ttcctaataa ttccag                                         506
<210> 395
   <211> 315
   <212> DNA
   <213> human
<400> 395
   ggaattcttc tcttacttca ataaaatggg ttttaacata actttaaatt cagttaaata     60
   tacaatattg aatacctata gttgactttg ggatggggac tttttcaagt cattaagagt    120
   gtttgtttaa ggtgatctca ttgatggtag ttctcagccg tctcaaaaac tgcaagctaa    180
   tcagtcagac attctttaat gaccccaatt ttttcacttt aattgttacc atgttttcta    240
   tttttactga tttttgctaa agcatgtaag agtgaattta ttatagcagt aatcttgtgt    300
   ttctcctgat gtgca                                                     315
<210> 396
   <211> 331
   <212> DNA
   <213> human
<400> 396
   atcaacacat ttagcttggc gttttattca ttccatcacc cttactgtaa ctactatggc     60
   aactcaaata attgtcatgg gactatgtcc atcttaatgg gtctggatgg catggtgctc    120
   ctcttaagtg tgctggaatt ctgcattgct gtgtccctct ctgcctttgg atgtaaagtg    180
   ctctgttgta cccctggtgg ggttgtgtta attctgccat cacattctca catggcagaa    240
   acagcatctc ccacaccact taatgaggtt tgaggccacc caaagatcaa cagacaaatg    300
   ctccagaaat ctatgctgac tgtgacacaa g                                   331
<210> 397
   <211> 449
   <212> DNA
   <213> human
<400> 397
   catctctcag gaggtgggct gtccaccaaa atgagcattc agggctgcgt ggcccaacct     60
   tccagcttct tgttgaacca caccagacaa atcgggatct tctctgcgcg tgagaagcgt    120
   gatgtgcagc ctcctgcctc tcagcatgag ggaggtgggg ctgagggcct ggagtctctc    180
   acttgggggg tggggctggc actggcccca gcgctgtggt ggggagtggt ttgcccttcc    240
   tgctaactct attaccccca cgattcttca ccgctgctga ccacccacac tcaacctccc    300
   tctgacctca taacctaatg gccttggaca ccagattctt tcccattctg tccatgaatc    360
   atcttcccca cacacaatca ttcatatcta ctcacctaac agcaacactg gggagagcct    420
   ggagcatccg gacttgccct atgggagag                                     449
<210> 398
   <211> 483
   <212> DNA
   <213> human
<400> 398
   tccttgtcca caagcactat ggcccccggc tcaagtgctg ctctggcaaa gctccggagc     60
   cccagcccca aggctttgac aaccaggcgt tcctccctga ccacaaggcc aactgggcgc    120
   ccgtccccag ccccacgcac gaccccaagc ccgcggaggc accgatgccc gcagagcccg    180
   caccccccgg ccctgcctcc ccaggcggtg cccctgagcc ccccgcagcg gcccgagctg    240
   gcggaagccc cacggcggtg aggtccatcc tgaccaagga gcggcggcca gagggcgggt    300
   acaaggctgt ctggtttggc gaggacatcg ggacggaggc agacgtggtc gttctcaacg    360
   cgcccaccct ggacgtggat ggcgccagtg actccggcag cggcgatgag ggcgagggcg    420
   cggggagggg tgggggtccc tacgatgcgc ccggtggtga tgactcctac atctaagtgg    480
   ccc                                                                 483
<210> 399
   <211> 241
   <212> DNA
   <213> human
<400> 399
   accatcgctg gtggtatccc agggtccctg ctcaagtttt ctttgaaaag gagggctgga     60
   atggtacatc acataggcaa gtcctgccct gtatttaggc tttgcctgct tggtgtgatg    120
   taagggaaat tgaaagactt gcccattcaa aatgatcttt accgtggcct gccccatgct    180
   tatggtcccc agcatttaca gtaacttgtg aatgttaagt atcatctctt atctaaatat    240
   t                                                                   241
<210> 400
   <211> 470
   <212> DNA
   <213> human
<400> 400
   gaatactcat aattcttatc tctataatca aaagtataat ttactgtaga aaaataaaga     60
   gatgcttgtt ctgaaagtaa gatcagtgaa ctgcttttca gtctcaatct ttgagaattg    120
   taaattcatc aaataattgc ttacatagta aaaatttaag gtattagaaa acctgcataa    180
   caaatagtat tatatattaa atattttgat atgtaaagct ctacacaaag ctaaatatag    240
   tgtaataatg tttacactaa taagcaaata tgttaatctt ctcatttttt tactgtcata    300
   taatcttagt gatatgccta ttaatagttt taaataaata aattggctca tctggctttt    360
   tgaaaatttt gaaattctta cagatgttga ttaggtatat ctacaaatta atttcaattt    420
   taaaatgatg atataaaaat aaatataagt atttttcttg tgtatgtata              470
<210> 401
   <211> 488
   <212> DNA
   <213> human
<400> 401
   agtaactttg tttatccctc aagcaaatcc tgatgacatt gatcctactc ctactcctac 60
   tcctactcct gataaaagtc ataattctgg agttaatatt tctacgctgg tattgtctgt 120
   gattgggtct gttgtaattg ttaactttat tttaagtacc accatttgaa ccttaacgaa 180
   gaaaaaaatc ttcaagtaga cctagaagag agttttaaaa aacaaaacaa tgtaagtaaa 240
   ggatatttct gaatcttaaa attcatccca tgtgtgatca taaactcata aaaataattt 300
   taagatgtcg gaaaaggata ctttgattaa ataaaaacac tcatggatat gtaaaaactg 360
   tcaagattaa aatttaatag tttcatttat ttgttatttt atttgtaaga aatagtgatg 420
   aacaaagatc ctttttcata ctgatacctg gttgtatatt atttgatgca acagttttct 480
   gaaatgat 488
<210> 402
   <211> 407
   <212> DNA
   <213> human
<400> 402
   ttccacttct attccacgga gaaaaaaatt gatttgtgtc acagaagttt ttatggaaac 60
   tagcaccatg tctacagaaa ctgaaccatt tgttgaaaat aaagcagcat tcaagaatga 120
   agctgctggg tttggaggtg tccccacggc tctgctagtg cttgctctcc tcttctttgg 180
   tgctgcagct ggtcttggat tttgctatgt caaaaggtat gtgaaggcct tcccttttac 240
   aaacaagaat cagcagaagg aaatgatcga aaccaaagta gtaaaggagg agaaggccaa 300
   tgatagcaac cctaatgagg aatcaaagaa aactgataaa aacccagaag agtccaagag 360
   tccaagcaaa actaccgtgc gatgcctgga agctgaagtt tagatga 407
<210> 403
   <211> 120
   <212> DNA
   <213> human
<400> 403
   ctcaactcgg ccatcacata tcgaattacc aaccactcac acttccggat ggagggagag 60
   gttgtgctga ccaccaccac actggcacag gcgggagcct tctacgcaga ggttgaggcc 120
<210> 404
   <211> 321
   <212> DNA
   <213> human
<400> 404
   tccattacca agagctcatg ccacccggtt cctgcatgcc agaggagccc aagccaaaga 60
   ggggaagacg atcgtggccc cggaaaagga ccgccaccca cacttgtgat tacgcgggct 120
   gcggcaaaac ctacacaaag agttcccatc tcaaggcaca cctgcgaacc cacacaggtg 180
   agaaacctta ccactgtgac tgggacggct gtggatggaa attcgcccgc tcagatgaac 240
   tgaccaggca ctaccgtaaa cacacggggc accgcccgtt ccagtgccaa aaatgcgacc 300
   gagcattttc caggtcggac c 321
<210> 405
   <211> 531
   <212> DNA
   <213> human
<400> 405
   aaaacccatt ctttcagtta catctactaa gagctcgtag ttcaataaca ttccaaagaa 60
   cgtagtttgg aaaacactgg cctatcggct gtaataggaa gggtctaaag aaaattattt 120
   gctgagtcct cataatcaat tggctataat cacaatttaa taatttatca atgcaaacag 180
   tacattaaaa actgtagcac aatctataat ttttaccttc atatgcagag aaattaagaa 240
   tttacatctt ttacaaatat gtgtatctct gaaataatga aatatattga tgggtgaaat 300
   aatatttctg gtattttctt cattataaca cgtggaatgg gacagtggct agaggtatgg 360
   atggagcaag gtcaaatatg agctgatgga gctgggcgct aaataagggt ataatatgct 420
   acttctgtgt atgttgtgta tggtatagta tgctatttct gtgtatgttc aaattgttta 480
   tgtatataat aaagttttaa aaatttgtat gtacttgaca attccttaga a 531
<210> 406
   <211> 533
   <212> DNA
   <213> human
<400> 406
   agaggagaca ctttggcgtt cttcaagcag aactgaggct gcgaaaaatc caagtctcta 60
   cagagacact gatgaagttg aaagggtaat tgtttttcca tgccaaatga ggaaatcaaa 120
   ttaatgagtt gacaaacttt tcctgagatg ttatttcatc tacatttagt taaacaactt 180
   agaatgatat ctagaaaata tttattttca gccaggaact ttagcaaact gtggcaatga 240
   tcactgggaa tgaaaacgta taatgttcta caatttttgt tatcacattg gatgactttg 300
   aatatagtag tgacacttgg gaccaaataa attattttat atgactacta gaacaaagtt 360
   ttagtaagct gtctgctaat agagtcagaa ttcttccaga ttgtttccag ctgtaacagg 420
   tattgaattt ccaccgtgct cctatgtaac agctgggtgg tgtaaaaaat gaacttcaaa 480
   ttatggagtg gaagaagcgt aatgttaata tcttgtaaat tcgtattccc tat 533
<210> 407
   <211> 394
   <212> DNA
   <213> human
<400> 407
   cattttccag gatctcagtg tgagtatcat tgatgcctgg gatataacaa ttgcatatgg 60
   cacaaataat gtacacccac ctcaacatgt agtcggaaat cagattaata tattattaaa 120
   ctatatttgt taaataacac aaaagtctga aattcattca cttaagtaaa aaaatttatt 180
   gactgtctac tagcaggcca gatgctgtgt ttggctctga attcccaact agcaagagca 240
   gagaatctac attatggctg atccataagc caccaattca gctaaatgag atgtttctaa 300
   tctgggcttc cacttaaaaa taaaactaaa aatactcatg aaaagagcct acccttcttg 360
   atcagagcca tacccctctt caccaatggg aagt 394
<210> 408
   <211> 478
   <212> DNA
   <213> human
<400> 408
   ggtattttag tctagtttta tatgaacggt tgtatcaggg taaccaactc gatttgggat 60
   gaatcttagg gcaccaaaga ctaagacagt atctttaaga ttgctaggga aaagggccct 120
   atgtgtcagg cctctgagcc caagccaagc atcgcatccc ctgtgatttg cacgtataca 180
   tccagatggc ctaaagtaac tgaagatcca caaaagaagt aaaaatagcc ttaactgatg 240
   acattccacc attgtgattt gttcctgccc caccctaact gatcaatgta ctttgtaatc 300
   tcccccaccc ttaagaaggt actttgtaat cttccccacc cttaagaagg ttctttgtaa 360
   ttctccccac ccttgagaat gtactttgtg agatccaccc tgcccacaaa acattgctct 420
   taacttcacc gcctaaccca aaacctataa gaactaatga taatccatca cccttcgc 478
<210> 409
   <211> 487
   <212> DNA
   <213> human
<400> 409
   gctggccaag actactgggc cgtgctttct ggaaaaggca tttcagccac gctgatgatc     60
   ttctccctct tggagttctt cgtagcttgt gccacagccc attttgccaa ccaagcaaac    120
   accacaacca atatgtctgt cctggttatt ccaaatatgt atgaaagcaa ccctgtgaca    180
   ccagcgtctt cttcagctcc tcccagatgc aacaactact cagctaatgc ccctaaatag    240
   taaaagaaaa aggggtatca gtctaatctc atggagaaaa actacttgca aaaacttctt    300
   aagaagatgt cttttattgt ctacaatgat ttctagtctt taaaaactgt gtttgagatt    360
   tgtttttagg ttggtcgcta atgatggctg tatctccctt cactgtctct tcctacatta    420
   ccactactac atgctggcaa aggtgaagga tcagaggact gaaaaatgat tctgcaactc    480
   tcttaaa                                                              487
<210> 410
   <211> 313
   <212> DNA
   <213> human
<400> 410
   agcccaccag caggagcttg gagtttgggg agtggggatg agtccgtcaa gcacaactgt     60
   tctctgagtg gaaccaaaga agcaaggagc taggaccccc agtcctgccc cccaggagca    120
   caagcagggt cccctcagtc aaggcagtgg gatgggcggc tgaggaacgg ggcaggcaag    180
   gtcactgctc agtcacgtcc acgggggacg agccgtgggt tctgctgagt aggtggagct    240
   cattgctttc tccaagcttg gaactgtttt gaaagataac acagagggaa agggagagcc    300
   acctggtact tgt                                                       313
<210> 411
   <211> 325
   <212> DNA
   <213> human
<400> 411
   gccactatct tgaagaaggt gcacagtgcc ctgctcctct ttcctatgtc cccagaattc     60
   tcttagggtt ctcagatgcc atgactttca aggagagagt acggaaccac atcatgcact    120
   tggaggaaca tttattttgc cagtattttt ccaaaaatgc cctagaaata gcctctgaaa    180
   ttctccaaac acctgtcaca gcatatgatc tctacagcca cacatcaatt tggttgttgc    240
   gaacagactt tgttttggac tatcccaaac ccgtgatgcc caatatgatc ttcattggtg    300
   gtatcaactg ccatcaggga aagcc                                          325
<210> 412
   <211> 284
   <212> DNA
   <213> human
<400> 412
   ctttatcatc cccacaaaca ttttgaaact ggaatatttg tcttcagaaa atggaaacaa     60
   gactataaat gataagccct gtccctagca ccacctctcc tgtgtgtgga atagaggccc    120
   ctcgtgctac caacacttac cctgtgttta aaaagatctt gtaccaagcc aacggcgttc    180
   ctggctctcc tgcccacagg atgaacattt tcggcttcct taggagtttt gccctaccgt    240
   attccaaagc gtgtgctggt ttctcatatt gtctgtaggc tcac                     284
<210> 413
   <211> 539
   <212> DNA
   <213> human
<400> 413
   gtgaggctca gtcagaacct ccaccctccc ccacaccaaa gacaggggca gcgtagtatt     60
   caaaccagta ttgtggtggg gaataattgt atacatgtaa attatcaagc cctatgagtg    120
   gaagaatttt ttcaaattat ttttgtctct ctatatattg atttatatta tgtataacta    180
   tctctttata taaactatat ataattatat atatataact atataattat atatatataa    240
   ctatatatat aactatatat atgtatcccc tagtattgga tcatgaagag ctcttcatgc    300
   attctttgca aaggaggtta taaagttacg ccctcagaac atttataact ataagaatgt    360
   gccagttaaa gtgctcaaca ggaaatatga cagtttaaaa gcattgtaaa actcacatag    420
   cttacttctc tctctaaagt gcaacaagga tgaatagaat gggccaaggt atgacaatta    480
   atggttctgc atgacctagc cactgctggg ggttttcttc tataacgttg tccttgtga     539
<210> 414
   <211> 511
   <212> DNA
   <213> human
<400> 414
   gggacttaac atttcacgtt gtatcttact tgcagtgaat gcaagggtta cttttctctg     60
   gggacctccc ccatcaccca ggttcctact ctgggctccc gattcccatg gctcccaaac    120
   catgccgcat ggtttggtta atgaaaccca gtagctaacc ccactgtgct tccacatgcc    180
   tggcctaaaa tgggtgatat acaggtctta tatccccata tggaatttat ccatcaacca    240
   cataaaaaca aacagtgcct tctgccctct gcccagatgt gtccagcacg ttctcaaagt    300
   ttccacatta gcactcccta aggacgctgg gagcctgtca gtttatgatc tgacctaggt    360
   cccccctttc ttctgtcccc tgtttttaag tccggatttt tacagaagga actgtctcca    420
   gacagctcat caaggaacca agcaaaggcc agatagcctg acagataggc tagtggtaat    480
   tgtgtatatg ggcgggacgt gtgtgtcatt a                                   511
<210> 415
   <211> 316
   <212> DNA
   <213> human
<400> 415
   cctctgtcct caaatgtcca aaatgttgga ggacctctgt tcatatccca cgcctgggct     60
   cttgccagca gtggagttac tgtagaggga tgtcccaagc ttgttttcca atcagtgtta    120
   agctgtttga aactctcctg tgtctgtgtt ttgtttgtgc gtgtgtgtga gagcacatca    180
   gtgtgtgcag gctgtgtttc cccatttctc tcctcccttc agacccatca ttgagaacaa    240
   atgtaagaaa tcccttccca ccaccctccc tgcctcccag gccctctgcg ggggaaacaa    300
   gatcacccag catcct                                                    316
<210> 416
   <211> 149
   <212> DNA
   <213> human
<220>
   <221> misc_feature
   <222> (55)..(55)
   <223> n is a, c, g, or t
<400> 416
   atattttgta tcatcgtgcc tatagccgct gccaccgtgt ataaatcctg gtgtntgctc     60
   cttatcctgg acatgaatgt attgtacact gacgcgtccc cactcctgta cagctgcttt    120
   gtttctttgc aatgcattgt atggcttta                                      149
<210> 417
   <211> 523
   <212> DNA
   <213> human
<220>
   <221> misc_feature
   <222> (29)..(29)
   <223> n is a, c, g, or t
<400> 417
   atccgacttg aatattcctg gacttacana atgccaaggg ggtgactgga agttgtggat     60
   atcagggtat aaattatatc cgtgagttgg gggagggaag accagaattc ccttgaattg    120
   tgtattgatg caatataagc ataaaagatc accttgtatt ctctttacct tctaaaagcc    180
   attattatga tgttagaaga agaggaagaa attcaggtac agaaaacatg tttaaatagc    240
   ctaaatgatg gtgcttggtg agtcttggtt ctaaaggtac caaacaagga agccaaagtt    300
   ttcaaactgc tgcatacttt gacaaggaaa atctatattt gtcttccgat caacatttat    360
   gacctaagtc aggtaatata cctggtttac ttctttagca tttttatgca gacagtctgt    420
   tatgcactgt ggtttcagat gtgcaataat ttgtacaatg gtttattccc aagtatgcct    480
   taagcagaac aaatgtgttt ttctatatag ttccttgcct taa                      523
<210> 418
   <211> 576
   <212> DNA
   <213> human
<400> 418
   ccaatcctga aggtactccc tgtttgctgc agaatgtcag atattttgga tgttgcataa     60
   gagtcctatt tgccccagtt aattcaactt ttgtctgcct gttttgtgga ctggctggct    120
   ctgttagaac tctgtccaaa aagtgcatgg aatataactt gtaaagcttc ccacaattga    180
   caatatatat gcatgtgttt aaaccaaatc cagaaagctt aaacaataga gctgcataat    240
   agtatttatt aaagaatcac aactgtaaac atgagaataa cttaaggatt ctagtttagt    300
   tttttgtaat tgcaaattat atttttgctg ctgatatatt agaataattt ttaaatgtca    360
   tcttgaaata gaaatatgta ttttaagcac tcacgcaaag gtaaatgaac acgttttaaa    420
   tgtgtgtgtt gctaattttt tccataagaa ttgtaaacat tgaactgaac aaattaccta    480
   taatggattt ggttaatgac ttatgagcaa gctggtttgg ccagacagta tacccaaact    540
   tttatataat atacagaagg ctatcacact tgtgaa                              576
<210> 419
   <211> 323
   <212> DNA
   <213> human
<400> 419
   tcgactctgc tgctcatggg aagaacagaa ttgctcctgc atgcaactaa ttcaataaaa     60
   ctgtcttgtg agctgatcgc ttggagggtc ctctttttat gttgagttgc tgcttcccgg    120
   catgccttca ttttgctatg gggggcaggc aggggggatg gaaaataagt agaaacaaaa   180
   aagcagtggc taagatggta tagggactgt cataccagtg aagaataaaa gggtgaagaa    240
   taaaagggat atgatgacaa ggttgatcca cttcaagaat tgcttgcttt caggaagaga    300
   gatgtgtttc aacaagccaa cta                                            323
<210> 420
   <211> 448
   <212> DNA
   <213> human
<400> 420
   aataacgcaa atggcttcct ctttcctttt tttggaccat ctcagtcttt atttgtgtaa     60
   ttcattttga ggaaaaaaca actccatgta tttattcaag tgcattaaag tctacaatgg    120
   aaaaaaagca gtgaagcatt agatgctggt aaaagctaga ggagacacaa tgagcttagt    180
   acctccaatt tcctttcttt cctaccatgt aaccctgctt tgggaatatg gatgtaaaga    240
   agtaacttgt gtctcatgaa aatcagtaca atcacacaag gaggatgaaa cgccggaaca    300
   aaaatgaggt gtgtagaaca gggtcccaca ggtttgggga cattgagatc acttgtcttg    360
   tggtggggag gctgctgagg ggtagcaggt ccatctccag cagctggtcc aacagtcgta    420
   tcctggtgaa tgtctgttca gctcttct                                       448
<210> 421
   <211> 477
   <212> DNA
   <213> human
<400> 421
   tcaaatggaa cctgccctct aaagcacttt ctttccttta cttgcgtggt ttcatgtaag     60
   ctgtgctgtt tagaaacaac atctcagact ttacaaagaa atgacaaaga aggcaattgc    120
   actttttaag ggatatcgac aagcagtttc tgttttctaa aggacaaaat acagagtgtg    180
   tgtcattttt aattagattc tttcccctgc tgagttggaa attccagtgc agcactgatt    240
   gaccacagtt gccaatctaa aagcacaaag acagaagtaa agctttatgc taattttatt    300
   tcaatatgat agaaaattta tcttggtatg tcctttttta gataactcca gcaggaaact    360
   gtaactgcta tgtctttagg aaaacgtaga agaaagaaca ttattattct ttaattccta    420
   caaggtactt gaaaacctta agtgaaaaag atttctatct ttttatcttg gcgcatt       477
<210> 422
   <211> 577
   <212> DNA
   <213> human
<400> 422
   tgccactaat tcattcacac taaggtgtaa atgattgata ataggaatga gttacctctt     60
   cccacagaca tttgttttta agtatgacag agcagggcct taatcccaag ggaaaaggtt    120
   atggaactgg agggggtgag ctttctgggt agaaggagac ttcctgaatt tccttaaaac   180
   ccagtaagag taagacctgt tgttttggaa ggtctgctcc accatctaag agcactgttt    240
   tttttttttt gttgttgttg ttgttttacg gtctctgagg gaatatagta aaaatgcata    300
   tgcacgtgca atttgcacgg cagcatttca ccgattgtgg actgtattgg ctaatgtgtt    360
   tcctggtctt tagatgcaaa ccattaataa cactatctta tctcatagtt ttttcagggg    420
   tgcttcttga ttagtaggga attttgaaca cctctttaaa tacagctaga aaataaaacc    480
   aatttgtaaa gccacatttg catatgatgc cagcctcacg catttgtata tctccagaaa    540
   ttcaggtatg cctcaccaat ttgcccgtct ttaataa                              577
<210> 423
   <211> 544
   <212> DNA
   <213> human
<220>
   <221> misc_feature
   <222> (46)..(46)
   <223> n is a, c, g, or t
<220>
   <221> misc_feature
   <222> (64)..(64)
   <223> n is a, c, g, or t
<220>
   <221> misc_feature
   <222> (66)..(66)
   <223> n is a, c, g, or t
<220>
   <221> misc_feature
   <222> (97)..(97)
   <223> n is a, c, g, or t
<400> 423
   taaactaacc cttacattcc atgtgatgtg atgtaggctt ataagntgct aaaatctata     60
   ggtntnggaa gtgaaagatt ctattttttc ttttttngta cataatggga atttcattcc    120
   agattatatt ttatttacat attaatttca cagaatatta atatttctta acttcttaaa    180
   gcatgctagc gttttatgta tatgtacaca tatattcaga cagggtaatt ttatctgctg    240
   cctaacattg tactaaaata ttgctttatc tgtttttaat tacaaaatgc taatgatttc    300
   ttaaattata gtttaaagac aattggccca ggaagcaaat cccctgcctt tagtatgaac    360
   cactataagt aaccttacaa atagagttaa tccaagacaa tattaacaaa ctgtgctttg    420
   tctttaataa aagggatagg attaacaaac atattgatgg cataacctat tcagctatgt    480
   ccttattttt gcaataatgt aacctcaaat attggattgt tgaaccaaca atggctgtgt    540
   taaa                                                                 544
<210> 424
   <211> 512
   <212> DNA
   <213> human
<220>
   <221> misc_feature
   <222> (79)..(79)
   <223> n is a, c, g, or t
<400> 424
   tttatgatga ctcagtggtg ccagagtaaa gtttctaaaa taacattcct ctcacttgta     60
   ccccactaaa agtattagnc tacacattac attgaagtta aacacaaaat tatcagtgtt    120
   ttagaaacat gagtccggac tgtgtaagta aaagtacaaa cattatttcc accataaagt    180
   atgtattgaa atcaagttgt ctctgtgtac agaatacata cttattccca tttttaagca    240
   tttgcttctg ttttccctac ctagaatgtc agatgttttt cagttatctc cccatttgtc    300
   aaagttgacc tcaagataac atttttcatt aaagcatctg agatctaaga acacaattat    360
   tattctaaca atgattatta gctcattcac ttattttgat aactaatgat cacagctatt    420
   atactacttt ctcgttattt tgtgtgcatg cctcatttcc ctgacttaaa cctcactgag    480
   agcgcaaaat gcagctttat actttttact tt                                  512
<210> 425
   <211> 517
   <212> DNA
   <213> human
<400> 425
   gataacctac atcaaccgag ataccaaaat catcctggag accaagagca agaccattta     60
   caagctgaac ggtgtgtccg aaagggacct gaagaaatcg gtgctgtggc tcaaagacag    120
   cttgcagtgc acctgtgagg agatgaacga catcaacgcg ccctatctgg tcatgggaca    180
   gaaacagggt ggggagctgg tgatcacctc ggtgaagcgg tggcagaagg ggcagagaga    240
   gttcaagcgc atctcccgca gcatccgcaa gctgcagtgc tagtcccggc atcctgatgg    300
   ctccgacagg cctgctccag agcacggctg accatttctg ctccgggatc tcagctcccg    360
   ttccccaagc acactcctag ctgctccagt ctcagcctgg gcagcttccc cctgcctttt    420
   gcacgtttgc atccccagca tttcctgagt tataaggcca caggagtgga tagctgtttt    480
   cacctaaagg aaaagcccac ccgaatcttg tagaaat                              517
<210> 426
   <211> 441
   <212> DNA
   <213> human
<400> 426
   ggtgcaactg tgacttgggt ctggttggtt gttgtttgtt gttttgagtc agctgatttt 60
   cacttcccac tgaggttgtc ataacatgca aattgcttca attttctctg tggcccaaac 120
   ttgtgggtca caaaccctgt tgagataaag ctggctgtta tctcaacatc ttcatcagct 180
   ccagactgag actcagtgtc taagtcttac aacaattcat cattttatac cttcaatggg 240
   aacttaaact gttacatgta tcacattcca gctacaatac ttccatttat tagaagcaca 300
   ttaaccattt ctatagcatg atttcttcaa gtaaaaggca aaagatataa attttataat 360
   tgacttgagt actttaagcc ttgtttaaaa catttcttac ttaacttttg caaattaaac 420
   ccattgtagc ttacctgtaa t 441
<210> 427
   <211> 377
   <212> DNA
   <213> human
<400> 427
   agggtacgag aacttgccaa tgggaaattc atccgagtgg cactggcaga gaaggatagg 60
   agtggaatgc ccacacagtg accaacagaa ctggtctgcg tgcataacca gctgccaccc 120
   tcaggcctgg gccccagagc tcagggcacc cagtgtctta aggaaccatt tggaggacag 180
   tctgagagca ggaacttcaa gctgtgattc tatctcggct cagacttttg gttggaaaaa 240
   gatcttcatg gccccaaatc ccctgagaca tgccttgtag aatgattttg tgatgttgtg 300
   atgcttgtgg agcatcgcgt aaggcttctt gcttatttaa actgtgcaag gtaaaaatca 360
   agcctttgga gccacag 377
<210> 428
   <211> 521
   <212> DNA
   <213> human
<220>
   <221> misc_feature
   <222> (96)..(96)
   <223> n is a, c, g, or t
<220>
   <221> misc_feature
   <222> (102)..(102)
   <223> n is a, c, g, or t
<220>
   <221> misc_feature
   <222> (354)..(354)
   <223> n is a, c, g, or t
<400> 428
   gagtccaaat gtcatcagtg ctcattttga gataccctgc tatcgatggt cgctacaaac 60
   caggaaatac tcaagttatt atgtgtatac attggnttta gntttatgaa acaatttacc 120
   ttcatgatct catagttaaa attgtaataa atttaggaat ataaaggatc aatatgggaa 180
   gcaaaatttc taaaggcagt ttctgttgtt ttaattagta tttgtgtagt tcaaaccagg 240
   aaggatttga ctatcattag attttgctta actttatgaa agctaaaata ttctctgtta 300
   taaaggggca actccatctg gtcctatagc atctttacta ctgatttttt tttngtttaa 360
   tttgaaaatg caaagaattg ttaaatgttc ttaaatgttc tcactacaaa aaaagaaaaa 420
   agataactac gtgaggtgat ggatatgtta attagctgga ttgtggtaat cattttggaa 480
   tgtatatgta tatcaaaaca tgtagtacac cctaaatata t 521
<210> 429
   <211> 423
   <212> DNA
   <213> human
<400> 429
   agatacattt ttacccacca taaatgttac aatatctgaa tatgctttgt caaactatcc      60
   ctttatgcaa tcgtcttcat attgttttta tgattctaat caagctgtat gtagagactg     120
   aatgtgaagt caagtctgag cacaaaaaga taatgcacga tgagattgcc taccatttta     180
   taggatattt actatgtatt tatacgttaa gacctctatg aatgaatgta tcagagaatg     240
   tctttgtaac tgtttaattc aatctgtaat aaaaatctaa ctaactaact catttatttc     300
   tattaaaaag gtattgtcct ttaggcgggg aatgggaatc cttgctgcac tgttgcagtc     360
   attctgaaag gacctttccc tgtacttacc tttcaacatg cttcaatctt atcaacgcta     420
   cat                                                                   423
<210> 430
   <211> 465
   <212> DNA
   <213> human
<400> 430
   gaaaaccgat gtgatccttg atcgaaaaaa aaatccagaa ccttgggaaa ctgtggaccc      60
   tactgtacct caaaagctta taacaatcaa ccaacaatgg aaacccattg aagagttgca     120
   aaatgtccaa agggtgacca aatgacgagc cctcgcctct ttcttctgaa gagtactcta     180
   taaatctagt ggaaacattt ctgcacaaac tagattctgg acaccagtgt gcggaaatgc     240
   ttctgctaca tttttagggt ttgtctacat tttttgggct ctggataagg aattaaagga     300
   gtgcagcaat aactgcactg tctaaaagtt tgtgcttatt ttcttgtaaa tttgaatatt     360
   gcatattgaa atttttgttt atgatctatg aatgtttttc ttaaaattta caaagctttg     420
   taaattagat tttctttaat aaaatgccat ttgtgcaaga tttct                     465
<210> 431
   <211> 548
   <212> DNA
   <213> human
<400> 431
   aattgtgttg tgatgctact cactttgatt gcaatgatga tgtccaaggt aagctattaa      60
   aaggcaggtt acttccaaat cgcactgaag gaaaaggtta agaataatac atgatcacag     120
   aaatgcatac cactgtctgt aaacccaaca aaattcactg ttctcttttg gatttattta     180
   gcctgatgta tttttaattc aatttttatg gtgatgggca aatcattctt ggtaaatgta     240
   aatcaaacat gattgattta aaacttcatg gaatttgtag aaaattatgg acatttttgg     300
   tgagaaagaa caatagtcaa aactcacatg gatagagtgt gtttgttttt tgccaaaaat     360
   gccccagacc ttttcccaaa cctcaaaaac gtcttggaaa aattgtaaaa gtttgataac     420
   agaaacatct ttaggatatt tttgtctgac gtattttgct tctagtatgt gcctactgtg     480
   atttttttca tgtggaaaat gcaaaatttg taacaaaatg gttatatgga acatgcctat     540
   taaacgaa                                                              548
<210> 432
   <211> 494
   <212> DNA
   <213> human
<400> 432
   ggcatctggc acgtgcccaa taagtccccc atgcagcact ggagaaacag ctccctgctg      60
   aggtaccgca cggacactgg cttcctccag acactgggac ataatctgtt tggcatctac     120
   cagaaatatc cagtgaaata tggagaagga aagtgttgga ctgacaacgg cccggtgatc     180
   cctgtggtct atgattttgg cgacgcccag aaaacagcat cttattactc accctatggc     240
   cagcgggaat tcactgcggg atttgttcag ttcagggtat ttaataacga gagagcagcc     300
   aacgccttgt gtgctggaat gagggtcacc ggatgtaaca ctgagcacca ctgcattggt     360
   ggaggaggat actttccaga ggccagtccc cagcagtgtg gagatttttc tggttttgat     420
   tggagtggat atggaactca tgttggttac agcagcagcc gtgagataac tgaggcagct     480
   gtgcttctat tcta                                                       494
<210> 433
   <211> 443
   <212> DNA
   <213> human
<400> 433
   gagaagcacc tgttccaact aagactaaag tggccgttga tgagaataaa gccaaagaat     60
   tccttggcag cctgaagcgc cagaagcggc agctgtggga ccggactcgg cccgaggtgc    120
   agcagtggta ccagcagttt ctctacatgg gctttgacga agcgaaattt gaagatgaca    180
   tcacctattg gcttaacaga gatcgaaatg gacatgaata ctatggcgat tactaccaac    240
   gtcactatga tgaagactct gcaattggtc cccggagccc ctacggcttt aggcatggag    300
   ccagcgtcaa ctacgatgac tactaaccat gacttgccac acgctgtaca agaagcaaat    360
   agcgattctc ttcatgtatc tcctaatgcc ttacactact tggtttctga tttgctctat    420
   ttcagcagat ctttctacct act                                             443
<210> 434
   <211> 507
   <212> DNA
   <213> human
<400> 434
   agctgccggg caacgtgttg tgtaagtgaa catctgggag gtaaacacta cacgtgaaga     60
   gtggtgaaag ggaacattga ttactgaagt gccctggaga gggaaagcac tggtcaacat    120
   cacatggaca aatttcattg ttttctaaag atggcctgga agtagtcttt gccactgctt    180
   cctccacaaa cagctcttca taacatgggc tgcatgaaat caaagcaaac tttcccattt    240
   cctaccatat atgaaggtga gaagcagcat gagagtgaag aaccctttat gccagaagag    300
   agatgtctac ctaggatggc ttctccagtt aatgtcaaag aggaagtgaa ggaacctcca    360
   gggaccaata ttgtgatctt ggaatatgca caccgcctgt ctcaggatat cttgtgtgat    420
   gccttgcagc aatgggcatg caataacatc aagtaccatg acattccata cattgagagt    480
   gaggggcctt gaggctgtag gatgaca                                         507
<210> 435
   <211> 392
   <212> DNA
   <213> human
<400> 435
   ggggctatac tccatccaaa tatgcagtgg aaggtttcaa tgacagctta agacgggaca     60
   tgaaagcttt tggtgtgcac gtctcatgca ttgaacgtct agacaaactg aaaggcaata    120
   aatcctatgt gaacatggac ctctctccgg tggtagagtg catggaccac gctctaacaa    180
   gtctcttccc taagactcat tatgccgctg gaaaagatgc caaaattttc tggatacctc    240
   tgtctcacat gccagcagct ttgcaagact ttttattgtt gaaacagaaa gcagagctgg    300
   ctaatcccaa ggcagtgtga ctcagctaac cacaaatgtc tcctccaggc tatgaaattg    360
   gccgatttca agaacacatc tccttttcaa cc                                  392
<210> 436
   <211> 512
   <212> DNA
   <213> human
<400> 436
   ggggctatac tccatccaaa tatgcagtgg aaggtttcaa tgacagctta agacgggacc     60
   tgaaagcttt tggtgtgcac gtctcatgca ttgaaccagg attgttcaaa acaaacttgg    120
   cagatccagt aaaggtaatt gaaaaaaaac tcgccatttg ggagcagctg tctccagaca    180
   tcaaacaaca atatggagaa ggttacattg aaaaaagtct agacaaactg aaaggcaata    240
   aatcctatgt gaacatggac ctctctccgg tggtagagtg catggaccac gctctaacaa    300
   gtctcttccc taagactcat tatgccgctg gaaaagatgc caaaattttc tggatacctc    360
   tgtctcacat gccagcagct ttgcaagact ttttattgtt gaaacagaaa gcagagctgg    420
   ctaatcccaa ggcagtgtga ctcagctaac cacaaatgtc tcctccaggc tatgaaattg    480
   gccgatttca agaacacatc tccttttcaa cc                                   512
<210> 437
   <211> 262
   <212> DNA
   <213> human
<400> 437
   tatctgcggc cccaggacag aaggtcgcca tctcctgctc tggaagcagc tccaacattg     60
   ggaataatta tgtatcctgg taccaacaac tcccaggaac agcccccaaa ctcctcattt    120
   atgacaataa taagcgaccc tcagggattc ctggccgatt ctctggctcc aagtctggca    180
   cgtcagccac cctgggcatc accggactcc agactgggga cgaggccgat tattactgcg    240
   gaacatggga tagcagcctg ag                                              262
<210> 438
   <211> 544
   <212> DNA
   <213> human
<400> 438
   ttttatttca ggcttccagc tgtccctgtg agttatcctg gacatttcga tggtttttgg     60
   taaggccaaa ctctgataag caaaacagag aatactgacg gtatacttaa ccatatgtgt    120
   aactgatact tggcaccatg gaatttttca ttgagttatt tcctcattct tttaaaaaat    180
   aagggactat aaatcagtta tttagtatct tttgtttttg tagctgattc cttaactttc    240
   ttgtatgcct ctagtaattt cagagattaa atattgcttt aaactgtgat actttgattt    300
   gctagattga caaaactgat actaatataa ttaagttcat ctttgaaata catctttgtg    360
   cgtagagcca aaaaaagaga taaaattaat aatagttcac ttgttatttg agattaattt    420
   ggcatttgaa atgatcattt tattttacaa tcatttataa tgaatcaatg ttccagttag    480
   ctttaaaagg tatacggtgc taattagtaa aatattgaag gcaatatttt actgctagct    540
   tgca                                                                  544
<210> 439
   <211> 380
   <212> DNA
   <213> human
<400> 439
   taatcctgac attagctgac ttgctagtga gcttgcttta aaaatctaca ctcttgcatt     60
   cttaggcata caggggaaat gttgaaaagg aaggtggaaa accaagaatt tagtttgcca    120
   atgattgcct ctgattcttg taagtttgag ttccacaagg gctaatttat tcccctttta     180
   cttgggtttt ggggtggtgg aaagcgggaa atttgggtga tttgttgatt ggcaatgagg    240
   ataaaatgtt aatacttttt tggggactta acaactttat cctattctac aagtcagtaa    300
   aggaacaatt ggtactcacc tcagtgctgc actcaactat ggaaagaggc agagtttgct    360
   tgcccaattg ccaaactaaa                                                 380
<210> 440
   <211> 529
   <212> DNA
   <213> human
<400> 440
   attagtgtca ttagtgtgtt ggaagagaaa tactattcag taagcttcgc caaagaaaag     60
   tgagtcaaag ttaatgtgtg tgtgcattta tatgtaggca gctcgtagac cacattttag    120
   ccagcaactg gtaacaaaga gcttagtttt ccttgtttga atgctgtaga tctgtaccta    180
   gtacccctcc catctactga tttgtttgtt tttgtaacca aacacatttt cagatagaag    240
   gagccttaaa aaaaaaaaaa tcacattgag taacttcagt atgaatgaat gagagtgtgt    300
   ggagctaccc ctcaccctcc acccctttgt gctttttatt cccgaatttt cccagtctct    360
   taaacagaaa aatgactgat ataattatct tttggaaact gagccttaat tttttttaga    420
   gggggaaata agttttcccc aactcacaca gcataagcaa tgtttgacag caatataatg    480
   ccgttgtaaa ctactgagag tattgtatct gttctggtaa ccatgtaca                 529
<210> 441
   <211> 389
   <212> DNA
   <213> human
<220>
   <221> misc_feature
   <222> (74)..(74)
   <223> n is a, c, g, or t
<220>
   <221> misc_feature
   <222> (181)..(181)
   <223> n is a, c, g, or t
<400> 441
   ttgtttttga catcagctgt aatcattcct gtgctgtgtt ttttattacc cttggtaggt     60
   attagacttg cacntttttt aaaaaaaggt ttctgcatcg tggaagcatt tgacccagag    120
   tggaacgcgt ggcctatgca ggtggattcc ttcaggtctt tcctttggtt ctttgagcat    180
   nctttgcttt cattcgtctc ccgtctttgg ttctccagtt caaattattg caaagtaaag    240
   gatctttgag taggttcggt ctgaaaggtg tggcctttat atttgatcca cacacgttgg    300
   tcttttaacc gtgctgagca gaaaacaaaa caggttaaga agagccgggt ggcagctgac    360
   agaggaagcc gctcaaatac cttcacaat                                      389
<210> 442
   <211> 450
   <212> DNA
   <213> human
<400> 442
   ggtcagatgg tcaacttttt tcagtattat ttatagttgg cacttgattg cagttctgtg     60
   aggcttgagc attcatacac ctcacctgcc ttggcaagcc tattttagtg atatggcagc    120
   acggatataa cactatgcat taaaagcact ttttgtaata agtttaatat cctaaaagga    180
   atgccaatta agttttgtta actgtgtcat caacttatcc tagtacctca gtgttcattc    240
   ctgttacctg catatcttct taaaagaaat agctgttatt aatgcctttt tgttttccat    300
   tgagtgtaca ctactgaata agtgtaggag ttttatgttt accatgtgag tcctgcaaca    360
   ctaaagatat tttgaatatc agtcatgatg gcaatttctg tataaaagag ccttaaatgg    420
   aacattgttt tgagatcaaa ctccccaccc                                     450
<210> 443
   <211> 496
   <212> DNA
   <213> human
<400> 443
   gatatctggc catgggtaac ctcattgtaa ctatcatcag aatgggcaga gatgatcttg     60
   aagtgtcaca tacactaaag tccaaacact atgtcagatg ggggtaaaat ccattaaaga    120
   acaggaaaaa ataattataa gatgataagc aaatgtttca gcccaatgtc aacccagtta    180
   aaaaaaaaat taatgctgtg taaaatggtt gaattagttt gcaaactata taaagacata    240
   tgcagtaaaa agtctgttaa tgcacatcct gtgggaatgg agtgttctaa ccaattgcct    300
   tttcttgtta tctgagctct cctatattat catactcaga taaccaaatt aaaagaatta    360
   gaatatgatt tttaatacac ttaacattaa actcttctaa ctttcttctt tctgtgataa    420
   ttcagaagat agttatggat cttcaatgcc tctgagtcat tgttataaaa aatcagttat    480
   cactatacca tgctat                                                    496
<210> 444
   <211> 538
   <212> DNA
   <213> human
<400> 444
   gacctaatac atttcctctg tgtgtgtgtg taacattcca aatacttttt ttttcttttc     60
   cactgtttgt aaggtgcaac aatttaatat ttttaaggga ctttttaaga gttccttaag    120
   aaccaattta aaattacttc agtgcaatcc tacacagtat caacattaga attttgatat    180
   tagtcttatg ttatcttcca ttctattttt atctgctttt tgctgctagt ttcaaactgc    240
   cagtattttt ccttttgctt ttaaaatagt tacaatattt ttcatgatag ccacagtatt    300
   gccacagttt attataataa agggttttta tttgatttag cgcattcaaa gcttttttct    360
   atcacttttg tgttcagaat ataacctttg tgtgcgtgta tgttgtgtgt gtgcatgtgt    420
   ggcgtatatg tgtgttacag gttaatgcct tcttggaatt gtgttaatgt tctcttggtt    480
   tattatgcca tcagaatggt aaatgagaac actacaactg tagtcagctc acaatttt      538
<210> 445
   <211> 474
   <212> DNA
   <213> human
<400> 445
   gagtgagtcc cagttacatc aaacagtgac ttccagttat tccccagtaa gtctgagtgg     60
   ttccttcaag ctgggtgtct ttccagcctt tgccagtcta gccccagcag ggcaccgtgt    120
   atgaatgcag tttggtgctg ttttagagta tgcctgctcc ccagccccct gcctggaacc    180
   ctctgagcaa cttgctctga cctataatgt cttaggtgca acacggaccc caccagagct    240
   cttggatacc cccctagatc catgtggctt tatgtgaggg gactgaatgc agacacacca    300
   tagccccctt ctactacttt ccctctcgcc ctgccaccta gttccacatg gaaccaacaa    360
   gttgagtgca tccctgttgg gtgttttgtg ttgagactgg ctgaaatgag gagactttga    420
   ccatgtgacg tgtcaacaga ctcaaggaga caaccacctc aactgggtca tgtg           474
<210> 446
   <211> 414
   <212> DNA
   <213> human
<400> 446
   ccccagtctc acctacgaaa tatgaagagc aaaagctgat tttgcttact tgctaaactg     60
   ttgggaaagc tctgtagagc atggttccag tgaggccaag attgaaattt gatactaaaa    120
   aggccaccta gctttttgca gataacaaac aagaaagcta ttccaagact cagatgatgc    180
   cagctgtctc ccacgtgtgt attatggttc accaggggga actggcaaaa gtgtgtgtgg    240
   ggaggggaag ggtgtgtgag tggttctgag caaataacta cagggtgccc attaccactc    300
   aagaagacac ttcacgtatt cttgtatcaa attcaataat cttaaacaat ttgtgtagaa    360
   gtccacagac atctttcaac caccttttag gctgcatatg gattgccaag tcag            414
<210> 447
   <211> 524
   <212> DNA
   <213> human
<400> 447
   tcatgcgtca tccctaaaat aataagatac atgggatcaa atagcccttg ccttttcaac     60
   acaaatcagt tggaaaatta tggtttgagt cctgttgctg ccatggcttc tgtttctcag    120
   aaatgagtgt gtatgaacat accaatctat gtaataggct accttttttt gtcttctttg    180
   gaactttgta cacaaaccaa gacaatatca gggtgacagg tgaatgaact taaattctca    240
   gtcttgtcta ttcaccaaaa aagtatactg cctgtttttt ctttaattat tcaaggttga    300
   tgacttttag gaacatgttt tatactgtat tttttaatta aagcaagtgc cttgatgtaa    360
   ttccatgtaa atcattgctt aaccctctta tgggatgagg atgagttatt aatgtattgc    420
   agcctactgg aaaggagggg gagttggtta atagcagata cttttcttct agaagcttat    480
   gttttatgct gtttattatg taagatcctg tatgtgtgtt gaga                    524
<210> 448
   <211> 426
   <212> DNA
   <213> human
<400> 448
   ataatgatcc attcgagttc tgtgatcctt attgttctta attgtgtttc tctacgtatt     60
   gttacagatg agccatacgt ttctttgtat caatgtagac atgacttcag atacctctga    120
   ggacctaccc agcagtctag gaccctgggc caagtgctgg gactatggta ctaaatccag    180
   tagatgggct gtgtagcaac tctcccaggg aacacactag ggtacttagg gaggtgcttt    240
   gtggagcatg ttgaagcttt gagatctgag caggaggcag tgatgtccct ggtctattca    300
   gggaaagatt tcagtgtgaa atggtaaaca tccaattgac aggatttaga ttttgcttag    360
   tttttctgct ttttaatgtt tctatccccc atctcagtgt tttctttatc catcccagtg    420
   atgcct                                                               426
<210> 449
   <211> 470
   <212> DNA
   <213> human
<400> 449
   ataattttac atgatcctca atatcaactc cagtttaaaa agtgttattt ttaaaacatt     60
   tgaaaccaag tactgtttaa tttcaatcag aagatgcaaa tacatacttt gatctatgtt    120
   tgattttgct aataatattt gaaggagatt gcctaccaag gacaaaacaa taaatttaaa    180
   aatcaaacga tttctccata cgctcatagt cacatatgga attttgagaa aataaagcat    240
   gctgtcttta ggaattttta tacttctttg tctttcttcc ttaatatttg cttctagctg    300
   ctcttggcaa tgatgaattg ttatgtatgc attaatgttt tgcagcccaa aagttgttca    360
   catttttcct atataagatc tgtggagtgt gtgtttcaaa gagagaacta cagaaatgtt    420
   aaagcaggaa aacctgaatg tgatgtgcac attttcatcc cacatggaca               470
<210> 450
   <211> 404
   <212> DNA
   <213> human
<400> 450
   catttctgtc acatgcactt agttgacatt acatctacat atattagctt tttcctacat     60
   gagccatcta tttacttagt aaccagtgtt cttaatgaag tatttagtct tgggtttctt    120
   gtaaaatttc tctgcattcc ttagacagtg tactatacat gaaatattct tgttgaccta    180
   gtaatttata ttattccatt taattcttaa acctatggcc tttttattga gcacactctt    240
   aaatcattat ttggcttgta aacattcatc tgaattgtgg ctacaatcct ctttaaataa    300
   tctaggaaaa aagaaagata aagcttacat tttcacagtt ttggctctta aacacattcc    360
   acaaatgcca ttaagaattt attttgtttt aggccagtca tggt                      404
<210> 451
   <211> 522
   <212> DNA
   <213> human
<220>
   <221> misc_feature
   <222> (388)..(388)
   <223> n is a, c, g, or t
<400> 451
   gagcttctag aaatttcact tgcaagttta tttttgcttc ctgtgttact gccattccta     60
   tttacagtat atttgagtga atgattatat ttttaaaaag ttacatgggg cttttttggt    120
   tgtcctaaac ttacaaacat tccactcatt ctgtttgtaa ctgtgattat aatttttgtg    180
   ataatttctg gcctgattga aggaaatttg agaggtctgc atttatatat tttaaataga    240
   tttgataggt ttttaaattg ctttttttca taaggtattt ataaagttat ttggggttgt    300
   ctgggattgt gtgaaagaaa attagaacca cgctgtattt acatttacct tggtagttta    360
   tttgtggatg gcagttttct gtagtttngg ggactgtggt agctcttgga ttgttttgca    420
   aattacagct gaaatctgtg tcatggatta aactggctta tgtggctaga ataggaagag    480
   agaaaaaatg aaatggttgt ttactaattt tatactccca tt                        522
<210> 452
   <211> 500
   <212> DNA
   <213> human
<220>
   <221> misc_feature
   <222> (299)..(299)
   <223> n is a, c, g, or t
<220>
   <221> misc_feature
   <222> (456)..(456)
   <223> n is a, c, g, or t
<400> 452
   ttgtgtgtaa tttcatggtg gcctagtgtt gtggtgcttc tggtaatggt aatagaagct     60
   caactatttt tttgtggatt tcagttttta tcatcagaag tcctagacag tgacatttct    120
   taatggtggg agtccagctc atgcatttct gattatacaa aacagtttgc agtaggttat    180
   ttgtcatttc agttttttac tgaaatttga gctaaacatt tttacatgta aatacttgta    240
   tttaccaaag atttaaatca gttgattaat taattaactc aaatactgtg aactatctnt    300
   aaaacactag aaaaaagaaa tgttagtatc tcaattacac caactgtgca aatgaacttt    360
   gataaaatag aaataatcta cattggcctt tgtgaaatct ggggaagagc tttaggattc    420
   tagtagatgg atactgaata ctcaggccca cttaanttat taatgtatac attgtgtttt    480
   tgtctttatg ctatgtacag                                                 500
<210> 453
   <211> 512
   <212> DNA
   <213> human
<220>
   <221> misc_feature
   <222> (447)..(448)
   <223> n is a, c, g, or t
<220>
   <221> misc_feature
   <222> (450)..(455)
   <223> n is a, c, g, or t
<400> 453
   ggcgtgcaat tttggtctgc gccacataac cattctgaag cttttaggcg ttggagagga     60
   agttggggga gtgttagaac tgttcccaat taatgggagc tctgttgttg agcgagaaga    120
   cgtaccagcc catttggtga aagacattcg taactatttt caagtgagcc cggagtactt    180
   ctccatgctt ctagtcggaa aagacggaaa tgtcaaatcc tggtatcctt ccccaatgtg    240
   gtccatggtg attgtgtacg atttaattga ttcgatgcaa cttcggagac aggaaatggc    300
   gattcagcag tcactgggga tgcgctgccc agaagatgag tatgcaggct atggttacca    360
   tagttaccac caaggatacc aggatggtta ccaggatgac taccgtcatc atgagagtta    420
   tcaccatgga tacccttact gagcagnnan nnnnnacctt agactcagcc agtttcctct    480
   gcagctgcta aaactacatg tggccagctc ca                                 512
<210> 454
   <211> 565
   <212> DNA
   <213> human
<400> 454
   aactgtgtac ttgtctggtc agctgtgtat gatcagttat ctacctcaga gtctattttc     60
   ttttgtgctg ggacaggttg ctggccctcc ctgtttccac agaccaaatc ctcctagctc    120
   aggagctagg gctaagcagt tatttctttc aagtattttt tagttcttaa attttatgct    180
   tgtatttgat gatagatgtc agtgacattt catagtttca aaagtccttg ctgctctgag    240
   aagtgtagat tctagtgaaa attacatagt cataagagaa atgtgttttt gtttttgttt    300
   ttgtttcatt tttttaaagt tgtggtatta ttggttctat gctccctgga atattactgc    360
   tttgtgaaag tccagactga acgcagcacc ctctgtgtac ctagtacagt tataaacctg    420
   ggtctctcac tacttgatat ttttgcatta gttaagacag aaatttgata gctcggttag    480
   aggggagggg aaatctgctg ctagaaatgt ctgaactaag tgccatactc gtctgggtaa    540
   gatttgggaa acataacctc tgtac                                          565
<210> 455
   <211> 575
   <212> DNA
   <213> human
<400> 455
   gatgagagca aaggggtcgc accatatgga aatgttgaaa actattgtaa agtagtatta     60
   tgaagtagct tttgtgtcat tcatgtcgat gacatgaaag tgaagtaaat ttattctatg    120
   taaattcaca ctaaaaccag tacagtacca taagtagaat acatgtaaga atcacctagt    180
   cttcactata ttgagtaaat ataacatgct aattttacaa ttaatgaaac taaactttta    240
   aacatctcca ttatatctac atccttttga aggtatttat catagttgcc aattttaatt     300
   ttaggattga ctttctcttt ctgaatgact tcataaagtt tggtgtgaat tttgaagact    360
   tgggttacta atgattgtat ctttgctagt caacaactta tgaaatatac tcaatgcgtc    420
   tgatgtgtca ttaagtgcag aaataactaa gacacaaata acctttgcaa accttcaagc    480
   tgtgtaatat tccaatgttg tttttttctt tgtatatata cttatatcac gtaggatgta    540
   aaaccagtat gaccttgtct agtctccaaa cttaa                               575
<210> 456
   <211> 411
   <212> DNA
   <213> human
<220>
   <221> misc_feature
   <222> (179)..(179)
   <223> n is a, c, g, or t
<220>
   <221> misc_feature
   <222> (343)..(343)
   <223> n is a, c, g, or t
<400> 456
   gccacacgtc caaaaccaat caggtcaact cgggcggtgt gctgctgagg ttgcaggtgg     60
   gcgaggaggt gtggctggct gtcaatgact actacgacat ggtgggcatc cagggctctg    120
   acagcgtctt ctccggcttc ctgctcttcc ccgactaggg cgggcagatg cgctcgagnc    180
   ccacgggcct tccacctccc tcagcttcct gcatggaccc accttactgg ccagtctgca    240
   tccttgccta gaccattctc cccaccagat ggacttctcc tccagggagc ccaccctgac    300
   ccacccccac tgcaccccct ccccatgggt tctctccttc ctntgaactt ctttaggagt    360
   cactgcttgt gtggttcctg ggacacttaa ccaatgcctt ctggtactgc c              411
<210> 457
   <211> 550
   <212> DNA
   <213> human
<400> 457
   gtatgtttgt aagtgcttct gcacgaatgt ttatacatga ctgtttccat agtacttatg     60
   tttttaaaaa tattcagtca tttcctacta taatcctcat gtatccatgt aactgactca    120
   aaaatacttc agccacagaa agctaaaact gagcaaatct cattcttctt ttccatcccc    180
   tttgcatgtg gctggcattt agtaatgatt aataatatgg ccagctgaat aacagaggtt    240
   tgagacacaa ttctttctca aaggagtcag ctaagctggg tctacttatg gacaaacatc    300
   taaatgtgtg gaagtatctg atatttgaca atggtaaatt tccacttagc tagctagcat    360
   tgtcagactt caatctcctc atggctctgg ccgtcctgtt ttaagcatga taattgttgg    420
   ccacatctca catagttctc attgagtgag ttcataaata aacagggttt tttttttttt    480
   ttaaagagca gccaagcaca aagtgtgact ttgttgacat tttatgtgac tttgtcatat    540
   gttcctaacc                                                            550
<210> 458
   <211> 353
   <212> DNA
   <213> human
<400> 458
   agtgtcaggt gccttgcagg aaaataatct gagtcccaag ctagcctgtg ctcatccaca     60
   atcacaatga acatgtcaag gaagaatttg cagagactca agggaagcac aatgggataa    120
   ggtaatcact ttcagtgaaa aactgttttc ttgaaaacag gcttggacac aattgaaagc    180
   tggcttcctg caaacacacc aagagtctgt aatctagcct atccattata tgtcctttat    240
   tattcatgat atcctattct tctaccttgt tgcctggtaa ctttttctga ggactgagtt    300
   tctgcagcga tgtggtgcac tcttcctgtg atgaggaaac atctgggccc cct           353
<210> 459
   <211> 528
   <212> DNA
   <213> human
<400> 459
   ctggacaccc ggttaccaaa gtcagcaaag aagatgcggt aatcgccgcc tgatctccac     60
   atggtgaaca caacactccc accaacacct ccttgactgg tcggtcttca gcaccggggg    120
   tgggcaggca ggtgttctgt gttgacgaga attgcacagg ctaaacacaa acacggaacc    180
   agagtgagaa cacctcactc acggcagccc aggctgctcc ctaccaggtg acggagcgcg    240
   ccggggctgt gggtgccagg ggctgagtgc tagggactcg tcatgagtgg ggatccccac    300
   gttcctgtca ctgctgtcaa acagaaggta aacagtctta tgaatgtatt tccttaggaa    360
   aacttgtaaa aacttttatt aggatatcta tttaatactg aactttggcc tactttgtga    420
   tagactataa acaaattgag gaaatcacta tttctcactt ctgtattttc tcaaaaataa    480
   ttttgttaca gagttcaata tactgtgtac cattgatctt ctattgtg                 528
<210> 460
   <211> 469
   <212> DNA
   <213> human
<220>
   <221> misc_feature
   <222> (99)..(99)
   <223> n is a, c, g, or t
<400> 460
   gaaactgcgg atcccaaact gttccctttt tcatttcttg aaatgttacc actacagaca     60
   tttttttaag gtgaataaac agttgtgatg tgctgtacnt aaaatcatgt ttaatcgtat    120
   aaggaaacat ttcaatacac ttatacagga agaaaactat agatgaagta catgtgtgtg    180
   attcagtctg attcacagaa ttctgagagt aatatggaat aaaacaactc cacttagatg    240
   ataactgaag catttcctgc cttgtgaaaa tttggatttt aaattgctgt tagaatggga    300
   aatttggaca ctttatatca ttgtataatt tcagaattta gtttctgtat cttttggaaa    360
   acatgattat agcaaaaaca tagaaaataa tctattacta aaacaccata aatgtaaaac    420
   tagtatgctt ggctgttaac tctaaagatg ttacttatgt ctgttttta               469
<210> 461
   <211> 457
   <212> DNA
   <213> human
<220>
   <221> misc_feature
   <222> (151)..(151)
   <223> n is a, c, g, or t
<220>
   <221> misc_feature
   <222> (155)..(155)
   <223> n is a, c, g, or t
<400> 461
   tgagggtgag cttactcagg gcccccagag gaagccctca gcctctgccc tccccccaca      60
   cagggcggga gcccaggcct gttcctggca gctgtggctg cagctgtgct cctgctccct    120
   cctggaatgt gcgacaagcc caaatgttcc ngggnaggcg gccggggcag ggggcttaga    180
   agtgctaata tggttctgtg ttttgcctga aacgatacca ggttcccctg aatagcaact    240
   ttacaaggtc catgtgggag ggaccaaccc agatgccctg ctgagtgtcc ctgaaaccat    300
   ggcagctcca tctgtcaaga tggcaggggc cggagtgagg gggctgctgg cttaacagca    360
   ggcatctggg caggccagtc ctcaaagcag ctcctgaagg tctgtgttgc actgtcacca    420
   gtctcaagct atgcctctaa tttcaccagg gatattg                             457
<210> 462
   <211> 139
   <212> DNA
   <213> human
<400> 462
   gtgtgatgca tgtgagcgtc tctggcacac acacttggac atacagttct gtgtgcgctc 60
   attcttatta caggagtgag caaaggaagc atttaccccg atggttacct agaccacgat 120
   tatttggatt gggggaggg 139
<210> 463
   <211> 430
   <212> DNA
   <213> human
<400> 463
   atccctgtat tcatggcttg actttgtgac tgctctacac tgcatgtctg acattgcaga 60
   gtgagctatg ttgaggtaaa ctggttggtt gtcattattt tgcaatcagc ctggtctctc 120
   ccatgaagat gtcgtgtgca taagcacaat catcactgat tagaagatca cagcagaata 180
   cccttggatt agagagaagt tcgtaccttg catttctctg aattctagtc tctcataagc 240
   actgctttgc tggatgattt tcactgcttt gtgttaatga ctttgagcga tctctcacat 300
   gatggggttc tttagtacat ggtaacagcc atgtcatctt acacacctag cattgtgaat 360
   gctgtagtga catcctttat aggcacctta cagctcaaaa cttttgtttc atttcatgcc 420
   ttacttatca 430
<210> 464
   <211> 486
   <212> DNA
   <213> human
<400> 464
   agtgccattt attctagttt atcatgtttt gcatgtttga aagtatgaat gtgctctttc 60
   ctaaaacatg gcaaatgaat agatgtagag aataacaata ttacttacaa gatgaaatga 120
   ttagattaga agtgtccctt tattaaactt tgtcagcctg actgggtaca attcttttgt 180
   taatttgcag tgtggtttgt atacacgtat acgtgttatc aataataaga ttttgcaact 240
   ggatgacaca agattttact tgaacagtga aggacaaaaa tcatgattgt ggaagatatt 300
   tttaaaatct gattttgcag cgatcacttt taaaccctgt agtgatgtaa gactaaaata 360
   taattgctaa gattttgttg gttaatgtaa agatatgact tttctgcact gtactctctt 420
   cataggattg taaaggtgtt ctaatccaat tgcatgatgt agtaagcctc ttaaatatgt 480
   gtgtta 486
<210> 465
   <211> 550
   <212> DNA
   <213> human
<400> 465
   tcctatgtct tctttcttaa atccagttgc tgattttgta aaatacagtt gtgataaagc 60
   agcattacgg gggggaaaaa gctatattcc aactggtgtt aaatgtattc aacaaaatct 120
   tacatcatac agtatttatt tcttaattaa tagaacttca gtgatatact tggtagatat 180
   ctcaagcctt ttgtctttta cacaatggtg ctctatccta ttgttttctt ttcaaagaag 240
   catctgaaca cttgcatttc tattttccta tccaaaggca tccacatcta agtgtgtttt 300
   taaagttgat taaaattatt tttctgttaa agcattctga aagtgtttgt ctttacctag 360
   aatgatttgt acacactcgt ggtcaactga acatgaatgt cagtagtagt ctaattatgg 420
   gaagggtaaa cgtgttagat taaggctctt aaagctctaa accatataaa ctatggactt 480
   gtatcatgat ttaactgttc ttagatcttt cttacacagt gattcattcc tctatttgta 540
   cagtggcttt 550
<210> 466
   <211> 493
   <212> DNA
   <213> human
<400> 466
   tatcaaaggt ttgccagcca ataaagtgca tcccaagtat acaggggaga aagctagact    60
   cctacagggt cctagagttt aagtaatttt tttgttatta atataggtaa taatttttct   120
   aatttttatt ttttggttcc aaatgtaaag ctccttgtgt ttacctctgt ttatgtcatt   180
   cttgacatgt ttatctaaat tatgtgtgct ctgtgacagg tgaaatgtaa atctgggatc   240
   catagtcaag atatcataag gacctacttc ccagcctacc tttcttcctc tacctgataa   300
   tgataatact caaaataaca acattcaaag gaaacacaaa gaaatcctgc tttcacatct   360
   cctatttctt gggctcctta ataactactg atggtttgtt catgaaaaaa aatttttaaa   420
   tcaaaagatt gtacttggcc ctgagttgaa aaaatttcaa aaatcaaaag tttgtacttg   480
   gccctgagtt gaa                                                      493
<210> 467
   <211> 461
   <212> DNA
   <213> human
<400> 467
   cagaaacatg accctcgctg gtcttgggtc cacatatcat tggactctgg gggacacaaa    60
   gatgcctgtg acactttggt gttgccgagt tagtcaacaa ttattctggg aaaaagcaga   120
   attgaattct tctctagatg tcctaccagg gttggccaag ggccacaaag caggctaata   180
   aattcccaca ggatccagac accaggcaaa attgctctaa gaagccagtt actgtcatcc   240
   ctctatggtt ctagaaaaaa tagtacaaaa atgacaggtc atcctatgag cgtcatgcca   300
   atgaaacccc atcttctgga gaagcccttg aatcagaatt atcttttttc ttgatgtcgt   360
   cagatgcagc cagtttctta atttttttaa aaactgtatg tttctgtggt atgtatattt   420
   gtacacctaa ctacctggca cttggaaatc acagcactac t                       461
<210> 468
   <211> 448
   <212> DNA
   <213> human
<220>
   <221> misc_feature
   <222> (28)..(28)
   <223> n is a, c, g, or t
<400> 468
   ttatcctgcg acaacacttt cagcaganta gcctctcctt atctcacaga tcacaagcac    60
   ccctagatag tgtgattctg tcagatagca tttatgcaaa aatctatgaa gttaaaagat   120
   cgtagaagcc aaatgaaatg tacatatcta ctgactgatg acaagggaat ttcattagga    180
   agaaggtaaa gaaacatcgt tgagtagcct accttgattt ctgtcaagtt cataaccagc   240
   ttcatatttt aaaggcttca ggtttgaaat taagtcaact gcatgcagct ttgctgataa   300
   atgaataatt ctctttgatg ccatttatga gaaaagactt caatatctgt tgcctgtcat   360
   atttaagaaa aattactgtt tctactctct gtatctgatt ttaaaagaaa aaactattca   420
   tacctggctt ccaggtaatt gactttga                                      448
<210> 469
   <211> 427
   <212> DNA
   <213> human
<400> 469
   gaactgagtc aatatggcaa ggtgtatgtg atctgtggga gttatgccat ttaacatagg 60
   aagtgcatgg gactttccct ctctgcactc cagctcttac tgtaccatta gaagatgcag 120
   aattctgttg gtgtgcaaaa agtatagcct tacattcaag cagaatggat ctgaagaaag 180
   cagcaatatc tgttactaga gaacattccc atgtgtttaa actcttcact tcttagatgc 240
   atttaaattc ttaatgcaaa tgacgtagca atttgaaaac ttctccgtat tacttgtgtt 300
   taaaatgtct tgctttaaat acaaaacaaa tggtaaaggg gattatcttt tgtttagatg 360
   gttaaatatt atttttgcct tagatagctt tgtaataatt tttctccaga cagttcaaca 420
   cttttga 427
<210> 470
   <211> 567
   <212> DNA
   <213> human
<220>
   <221> misc_feature
   <222> (246)..(246)
   <223> n is a, c, g, or t
<400> 470
   tactgactga ctacaggggc tgattgtgaa gcacgaggaa ccccatgtgt gtggagactg 60
   tagggtgaga gcacacaatt attagcatca tttctgagtg atctcacaga ttttttttct 120
   tgtgtttgtt ttgctttttg acaactgctt ctcccacgtt ccttgcaatt ctattctctc 180
   accttcactt tactatttgt attcgatgga ccaggataat tcaggcaagg ttaccttgta 240
   aacttnaatt ggccacacac catgttgtca cccagctggc tatgaagtga ataatggtac 300
   tgaaagtaaa cctgaagacc tttctcagat ctattttaag tctgagtctg accaaccatg 360
   gaaaatattc gacatgaatt aatgtagaga actataaagc atttatgaca gctccaagaa 420
   aaatcatcta ctctatgcag gagatatgtt tagagacctc tcagaaaaac ttgcctggtt 480
   tgagggtaca cagtaccatt ttaatcttct gaaaatatct gtattcctgc tctttttctg 540
   ctgtcactgt caatctgcta tattttt 567
<210> 471
   <211> 506
   <212> DNA
   <213> human
<400> 471
   ttcctgacgg gaatgttgtg ctataatgaa tctgcataac gcttgggatt ctaggaggaa 60
   ggaaggttcc atggacatgt aagtacagca tattcccctc agtcttctag gagggcagag 120
   tgaatcccag aactggtaag attgggaatc tgagcattgc cactttaatc ttagaatatt 180
   tatcattttg acacatcctg ttttttagag aggaaaacaa acacagtttc tgcattggta 240
   gtgtaaagca taccttgtta ggaacgtgtt ttgtaagaca catttgggtt gtcattctag 300
   agcatgtcaa actttgtact tcaaaatata tttagtatga ttgttagtgg taacatatat 360
   caaggctttg aattaactgt tttatttaat tttcacaaga agcacttatt ttagccatag 420
   gaaaaccaat ctgagctaca aatagttctt taaaataagc ccaggttatt tagctattct 480
   agaaagtgcc gacttctttc aagaag 506
<210> 472
   <211> 488
   <212> DNA
   <213> human
<400> 472
   ttaaatattc attccattac atctagactc accaagaact acatgttatg atgttaagtt     60
   gaagttgaaa catgatgttt tgcattaaat ttaagatatg caaatttatg tagagaaaat    120
   aaatgttata taccctataa tctttcacct aattagtatt taattatatg gatttgtttt    180
   atattataaa agatgttttg attttgtctt ttgatattga caaaattgtt tggatatcct    240
   tatgttctca agtctgtatc tgcctcccct gccttatttc ttatgttttg ccacagttaa    300
   cccattgtgc ttctttgtaa tcaaacagtt tgtgggagaa tgggcttatt gaatgtctaa    360
   aaaataagtt taaagtgttt gttaccctaa gttttttaca tttttaaact ctaattacat    420
   atgtgaatgt tattactctc agtgaattgt tattgtttgc aaaaatgcac tgggcagtaa    480
   cattttgt                                                              488
<210> 473
   <211> 426
   <212> DNA
   <213> human
<220>
   <221> misc_feature
   <222> (262)..(262)
   <223> n is a, c, g, or t
<220>
   <221> misc_feature
   <222> (271)..(271)
   <223> n is a, c, g, or t
<400> 473
   gccggactgg aaccgtctga gcggcctaac ccgcgcccgg gtagagacct gcgggggatg     60
   acagctgaac cgcctaaagg aggtgaaggc tttcgtcacg caggacattc cattctatca    120
   caacctggtg atgaaacacc tccctggggc cgaccctgag ctcgtgctgc tgggccgccg    180
   ctacgaggaa ctagagcgca tcccactcag tgaaatgacc cgcgaagaga tcaatgcgct    240
   agtgcaggag ctcggcttct ancgcaaggc ngcgcccgac gcgcaggtgc cccccgagta    300
   cgtgtgggcg cccgcgaagc ccccagagga aacttcggac cacgctgacc tgtaggtccg    360
   ggggcgcggc ggagctggga cctacctgcc tgagtcctgg agacagaatg aagcgctcag    420
   catccc                                                                426
<210> 474
   <211> 563
   <212> DNA
   <213> human
<220>
   <221> misc_feature
   <222> (35)..(35)
   <223> n is a, c, g, or t
<220>
   <221> misc_feature
   <222> (55)..(55)
   <223> n is a, c, g, or t
<220>
   <221> misc_feature
   <222> (96)..(96)
   <223> n is a, c, g, or t
<400> 474
   aatggcttct atgatcagaa ctgggaaaac agtgnatctt atggtggaag aggtnctcag 60
   caagtgtaca gtatttacct tcctttgtct tacatnggct ttttaaattt tccattaatt 120
   tcaacataat tatgggaaca agtgtacaga agaatttttt ttttaagata tgtgagaact 180
   tttcatagat gaacttttta acaaatgttt tcatttacag gaaattgcaa agaaaattct 240
   caagtgatag tctttttttt taagtgtttc gtaagacaaa aattgaataa tgtttttgga 300
   agttctggca agattgaagt ctgatattgc agtaatgata tttattaaaa acccataact 360
   accaggaata atgatacctc ccaccccttg attcccataa cataaaagtg ctacttgaga 420
   gtgggggaga atggcatggt aggctacttt tcagggcctt gacaagtaca tcacccagtg 480
   gtatcctaca tacttctttc aagatcttca accatgaggt aaaagagcca agttcaaaga 540
   accctagcac aaatttgctt tgg 563
<210> 475
   <211> 474
   <212> DNA
   <213> human
<220>
   <221> misc_feature
   <222> (48)..(48)
   <223> n is a, c, g, or t
<220>
   <221> misc_feature
   <222> (55)..(55)
   <223> n is a, c, g, or t
<400> 475
   ggaacctatc aatttgagtg gactttttct ttagtagtac accatttngg ttgtngtagt 60
   ttcaaagtct ttctgaagca gatatattgg gattggagcg gggtggggaa aactgtcact 120
   cctttcagag gaaaagggga ggagcatgga gaaaaacaaa aattaaagga cttaaagaat 180
   ggctatacag tgttgagtgt tgaggatatt aaacatgtta tttttcaaac gtatgtaata 240
   tatattaaat ttataaagca aatttatgtt gtgatcttgc ctgaacaaat tatattttaa 300
   tgaaaaaact ttctattaat agttcacgca agagaaaaca ctttcaacat agtcgaaggc 360
   ttcaagatct aagtgtatca gacttaggga aaaagtggca caaccttcga tttaaaattc 420
   tagtctttaa aatgagtttg taaataatta gctattacgt tctattaagt tgtt 474
<210> 476
   <211> 322
   <212> DNA
   <213> human
<400> 476
   agggatctct ctacaagagc ccctgcccct ctgttggagg cacagtttta gaataaggag 60
   gaggagggag aagagaaaat gtaaaggagg gagatctttc ccaggccgca ccatttctgt 120
   cactcacatg gacccaagat aaaagaatgg ccaaaccctc acaacccctg atgtttgaag 180
   agttccaagt tgaagggaaa caaagaagtg tttgatggtg ccagagaggg gctgctctcc 240
   agaaagctaa aatttaattt cttttttcct ctgagttctg tacttcaacc agcctacaag 300
   ctggcacttg ctaacaaatc ag 322
<210> 477
   <211> 409
   <212> DNA
   <213> human
<400> 477
   ggtaattatg gaaactcctc aaagaggaga aagtaatttt tttccagaca tttttctcat      60
   tctgtgtctt tcacacacta gtttccatag ttcgagaatt ctgtttttta ccattgggct     120
   gtgaatgttc acaatatcag tcctgttgaa ttcctatgag gtaatcacaa tgtgtatatg     180
   ttcattttct aggtatgata aaagaatgta tggcttttta ttctgtggaa gtaaaatcct     240
   gaacgtttac aacttttcct taacttgtaa ataaaaaatt gtaagttttt tcttttttta    300
   cagaaaactt agcttgtgta attctgttag tttcagattt ctctcctgtt tttgcaaatt     360
   gtgggaaaga ttgacaatgc aaatgtgtca aagacatact gttgggtgc                 409
<210> 478
   <211> 544
   <212> DNA
   <213> human
<400> 478
   acatgggttt atacaagttc ctcttgagaa ggcaaaaaga ccaccatgtg tgagagctct      60
   ttgacttggc caataggggc ctatcttaat gcacttgttt ggacacattt ctgatcttat     120
   ttgtaaaggc tgcaaaagga gaggatgaaa tgctgtaaaa gtaggaaatg aagtggaagc     180
   tggaagaaaa tgtaattggt ggtacagcta tgggccagat ggtggagggg agggtgggga     240
   cccctgccgg caagcagagt gtcacagctg gctttcctca cttgggaaaa gggtactgcc     300
   ggtctagcag cctcctctgt actcagccag gacacccagc gcgtgggacc tgtttgtgtc     360
   tgttttgctt ccttgggaac ggcacagtca ctcaccctgc catttgcgga aatgacctgg     420
   tgcactttga ctgttaagca atgcgttatt gctgtagtca aggttagtgc aagcaaggaa     480
   acattcccag taaggtattt gtttccattt tctgtctgtg cttctgtcag aaacttgcta     540
   ggac                                                                  544
<210> 479
   <211> 479
   <212> DNA
   <213> human
<400> 479
   caaacgtcct ggactgcaca gacctcccac tccagaccat ccaggcctgg ttcccaagac      60
   ccgatccttc ccctgcaacc agacagtcta caactgcccc ctccagccca ttttctgccg     120
   tgaaacccca gccagccaca ccagactctg gaaccctttt tcgactgccc caactcttgg     180
   acaccaggcc aactagaaca cccaacacca aactgtacag actctcccac cccaacctcc     240
   ccagactctg cacggatgtc ctaggccccc tccccaactc taaccagacc ccatccccct     300
   aagtcccttt gtcttgaccc ccaagtcttc aaccagatat cctcggcaac ccacctccca     360
   ccctcctcct cttctccttc aagacccaac tgagcacccg ctctgattcc ccacagcctt     420
   tctccctgcc accactccct tagtctttcc caggcttact ctcccaataa atgtgctag      479
<210> 480
   <211> 493
   <212> DNA
   <213> human
<400> 480
   ggtggagttg gtggtcttaa tttggagatg caggggcaac ctgtgaccct ttgaggcaag      60
   agccctgcac ccagctgtcc cgtgcagccg tgggcagggg ctgcacacgg aggggcaggc     120
   gggccagttc agggtccgtg ccaggccctc ctcagtgccc tgtgaaggcc tcctgtcctc     180
   cgtgcggctg ggcaccagca ccagggagtt tctatggcaa ccttagtgat tattaaggaa     240
   cactgtcagt tttatgaaca tatgctcaaa tgaaattcta ctttaggagg aaaggattgg     300
   aacagcatgt cacaaggctg ttaattaaca gagagacctt attggatgga gatcacatct     360
   gttaaataga atacctcaac tctacgttgt tttcttggag ataaataata gtttcaagtt     420
   tttgtttgtt tgttttacct aattacctga aagcaaatac caaaggctga tgtctgtata     480
   tggggcaaag ggt                                                        493
<210> 481
   <211> 483
   <212> DNA
   <213> human
<400> 481
   atttttccat cttattcaag tcagagcact tttttttaaa tagcaataca acaggcaaga     60
   gaatactcaa aaatatttaa aactgtattg ataccaagag tatgttttaa atattttcta    120
   ataaatactt gagcggtttt tgtctggcag gcttccaaat ttgccaaaat taagcgttca    180
   gtattttcaa cacatacgct ttttactggt ttatactgaa ctatctgatg agaattcctg    240
   tgttcccaaa gcaactgatg tttacaggtc ttgtgtttct cctcctcctt tctaaggatg    300
   agggaatcca caacagactt tctctagaaa acactaatga tggacaactt tttggtgtca    360
   tcaatgagtt ggctactacc ttgatgtaaa aatttgtaag gaaaattttc accatttcga    420
   gtgtcaagtg tatttttaac tgtctggttt gtacttttat gacttttgta ctaccaaagc    480
   gga                                                                  483
<210> 482
   <211> 408
   <212> DNA
   <213> human
<400> 482
   agtgatctct ggaagcgcta aagctaaaat ttctgttctt gaaacacttc agctttgcaa     60
   ctaaaatatt acagattaat aataaattaa accaaccaat gataaacact actcagtcca    120
   ccaacaacaa acgtgtttga attcacctta ccaatattaa tcccagcgtg tgtaaaacag    180
   aacagtaact ctatgtgacc ccagataaca ttttgtaaca ttgtgcttcc ttgtagtttg    240
   taatgtgagt tcaatcagta tttatgttga aatttctaac attaaatcta gtctctatcc    300
   tgttaattta atttttaaat gctttatcca tttgtgcaaa ggtaaacgca gattgtatct    360
   tttttaatgg tacggcataa aaagtaaccc tcaagtgaag tgtctcta                 408
<210> 483
   <211> 489
   <212> DNA
   <213> human
<400> 483
   tgacgagacc tcttcaaaaa cccacagtaa aactcccctc cctccagttg gccaccagtc     60
   tgccaccaaa catgaacaaa ttctgctgct aatcggtttc ccttgtgatc tggttcctga    120
   ggtcttcgga tctgtgcaat gaattattta ttgttttatt aaaccgacag tggtgtccca    180
   gagaggaacc ataaataaaa tggaaatctg gtgctgtgat aaagtaataa ctagcattaa    240
   tgagacctgg ttttcctttc agaaagtcca gtatacctgt aacaaaggtt aaagcaattt    300
   atatttaatt tgcattctga tgttaacatt taaacagcaa ttctaacaaa aatgcatcga    360
   gtctaattct tacctctatc aaaaaacaac tgtataaatt tatgaccaac attaaaacaa    420
   aaacaaaatg taaattttct ctttagaaat gattaactgg aaatgagtga gacagcacct    480
   tgtgggttt                                                            489
<210> 484
   <211> 460
   <212> DNA
   <213> human
<220>
   <221> misc_feature
   <222> (101)..(101)
   <223> n is a, c, g, or t
<220>
   <221> misc_feature
   <222> (129)..(129)
   <223> n is a, c, g, or t
<220>
   <221> misc_feature
   <222> (131)..(133)
   <223> n is a, c, g, or t
<400> 484
   tgctcgcatt gatcatccta atcatcttat tcagcctatc ccagagaaaa cggcacaggg     60
   aacagtatga tgtgcctcaa gagtggcgaa aggaaggcac ncctggcatc ttccagaaga    120
   cggccatcng nnnagaccag aatctgaggg agagcagatt cggccttgag aacgcctaca    180
   acaacttccg gcccaccctg gagactgttg actctggcac agagctccac atccagaggc    240
   cggagatggt agcatccact gtgtgagcca acgggggcct cccaccctca tctagctctg    300
   ttcaggagag ctgcaaacac agagcccacc acaagcctcc ggggcgggtc aagaggagac    360
   cgaagtcagg ccctgaagcc ggtcctgctc tgagctgaca gacttggcca gtcccctgcc    420
   tgtgctcctg ctggggaagg ctgggggctg taagcctctc                          460
<210> 485
   <211> 560
   <212> DNA
   <213> human
<220>
   <221> misc_feature
   <222> (144)..(144)
   <223> n is a, c, g, or t
<400> 485
   gctgattcta attgtgtgta ggtcttgagg attaagcaca caaatttcac aaacttctgt     60
   ttgagtaaac aaactcagcc ttctgtaaat atacatgcaa gtttggaaac agtaatactg    120
   tacctataaa tatatgctgt ctgntttgtg tacagtatgt aaaaactcct tttctgccac    180
   actaaaaatg caagccattt atgggaatcc taaaactagt attgaactaa aactttgcta    240
   atgatcttta ttagaggatc gtccaacttt tcacttacct tgggttttct tttcaattca    300
   ctcttacact agtctgctta tttccagctg tttattttat tgagtcctga atttaaaaaa    360
   aaaatatttt gattcatttt gtaaatacaa gctgtacaaa aaagagagat ttaatgttgt    420
   cttttaaata ctccaatttt cattctaata tgaatgttgt tatattgtac ttagaaactg    480
   tacctttaat attacattac ctttattaaa agtgcattga acacatcaat tttagatgtg    540
   ctttatgtac tgttatccta                                                560
<210> 486
   <211> 212
   <212> DNA
   <213> human
<400> 486
   taagcattca gtgagctgcc aattttgatt ttgtgttgct ctttacccaa attatttttt     60
   ctttgttttt ctttttttgg gggaggaggg gaaaaaagca gcaatactgt gtttggaaat    120
   tatactctgt atctggtttt cctgtgtatg ttaaccactt aaatgttatt atcctgcttt    180
   ggttttagag tgattgtgag gcattcaatg ca                                  212
<210> 487
   <211> 407
   <212> DNA
   <213> human
<220>
   <221> misc_feature
   <222> (75)..(75)
   <223> n is a, c, g, or t
<400> 487
   agtgcctaaa tcttgtttac ctatcacttt aaaaaaataa ttgaagtgta agctaaataa     60
   aatgcttgga gtttngcctg ggctagtgag agttggtgca aattcttgtg tgtgtttgca    120
   taggaaggtg agatgaccat ctactaaaga ggaagtagct aaatacagat ctgtgggtgt    180
   ttttaaaaaa actcaaccta tctggtgttt tattttaatg gataaaaatg taatttttct    240
   aaggtagcaa cttatttcca aattaatata gatgaaaaat agataccaat tagactaaat    300
   tgaaagcttt ttgttctata tttgcatagc ctttgaaata tttcttagtg cctaggaggt    360
   ctggggattc ctctttcgtg gtggtcacta accttacttg atgcaga                  407
<210> 488
   <211> 553
   <212> DNA
   <213> human
<220>
   <221> misc_feature
   <222> (105)..(105)
   <223> n is a, c, g, or t
<220>
   <221> misc_feature
   <222> (136)..(136)
   <223> n is a, c, g, or t
<220>
   <221> misc_feature
   <222> (142)..(142)
   <223> n is a, c, g, or t
<220>
   <221> misc_feature
   <222> (155)..(156)
   <223> n is a, c, g, or t
<220>
   <221> misc_feature
   <222> (481)..(481)
   <223> n is a, c, g, or t
<400> 488
   aacaccaatt atgcaaacta cttttttttc cagcagaaag ggagctgaca tgatcaaatc     60
   catgttttca aatgaactga aaaaggcatc cagcaccact tatancacat ttatttcagt    120
   tccaggctga cagccntggg gnatctagca ggatnnataa ttgtcatctg gtgagagttg    180
   ggactttgct caattttaat accaatctct cctgattgta attacctcca ctactttcat    240
   gatcccccta caatattttt ttaaatgata tatttattca ctgaatcaaa tgtcattaat    300
   gagttatctt ctgtggagga tgactgttct ctttgttaat gttcacaatc aagatcttgg    360
   gctgagaaga ggcccttcac ccaaggagtt tgaagtatca cagcgtgtgg gaaggtggga    420
   accaggatac ccattcattt ccaaccgaga cacagagaag tgagtcacag aatttgagcc    480
   ngctctcttg actgcccagc cagagacact gatttctgta acctcttcac ttgatcctgc    540
   ctcttaagca tta                                                       553
<210> 489
   <211> 421
   <212> DNA
   <213> human
<400> 489
   tcatttctag agtacctgag ccaaacaaat acacaacgga agctgcagct gtatcatcac     60
   tagcaatttg ctcatcatta tttactacct ttgaacctaa ggtttcctgc ctatgctttt    120
   gaaagcaaaa atcagtctcc tttgcatgaa aaagagcctt agatttttaa acatgttagt    180
   taccagaatg ctaaaatacc agttgattac ccaaattatt ttggaaatct atccataatg    240
   gaagtctaca acaaacacat aaaacagatt acactaagag ctgagaaatt caaaggaact    300
   gaagattctg agagataaac tgttcaagtc ttagcaatga tactgcactt ctctttgaca    360
   ggttctgggc ttaagttaga ggccctactg gttccaaacc atattccact gactttgcaa    420
   g                                                                    421
<210> 490
   <211> 505
   <212> DNA
   <213> human
<400> 490
   cctctgagtg ttttgtgggt tctgggtgtt ttgtacattt tagccaagct aaccacttgt     60
   ctgcaagtac tgactttcct atgaattctt tgaagattat tgagtcagaa aggaaaaata    120
   tagccccaaa ttcccaggct tttaatgcat tacattaact gcctattgaa atgagaagtt    180
   cttcacaaac ttgtataccc actaacaaga ttgcacataa acatgcatta aagtatatac    240
   taagaaaccc tctgtccaac ggctcatgca tatgaagtcc gaacatggga gtttgccaat    300
   tgcattcatc aagtcgtttt gcggagtcag atccctgatg gaagagctca caggctctgc    360
   cttccaagtc ctgggttcct aactggtgac cttagcctgg ggtctgtggg gagaccaacc    420
   ctggcttcca agaaaaccac attccatgga ctatcagaaa tagacacaga tttgggtgac    480
   aaagctggct ctgtatttgc atttt                                          505
<210> 491
   <211> 497
   <212> DNA
   <213> human
<220>
   <221> misc_feature
   <222> (48)..(48)
   <223> n is a, c, g, or t
<220>
   <221> misc_feature
   <222> (84)..(84)
   <223> n is a, c, g, or t
<220>
   <221> misc_feature
   <222> (86)..(87)
   <223> n is a, c, g, or t
<220>
   <221> misc_feature
   <222> (116)..(116)
   <223> n is a, c, g, or t
<220>
   <221> misc_feature
   <222> (348)..(348)
   <223> n is a, c, g, or t
<400> 491
   gcaagcgcgt gctgagcaag ctgcagtctc catcgcgggc ccgcgggnca gggggcagtc      60
   ccggggggct gcagaagcgg cacncnngcg tcaccgtcaa gtatgaccgg cggganctgc     120
   agcggcggct ggacgtggag aagtggatcg acgggcgcct ggaggagctg taccgcggca     180
   tggaggcaga catgcccgat gagatcaaca ttgatgaatt gttggagtta gagagtgaag     240
   aggagagaag ccggaaaatc cagggactcc tgaagtcatg tgggaaacct gtcgaggact     300
   tcatccagga gctgctggca aagcttcaag gcctccacag gcagcccngc ctccgccagc     360
   caagcccctc ccacgacggc agcctcagcc ccctccagga ccgggcccgg actgctcacc     420
   cctgaccctc ttgcactctc cctgcccccc ggacgccgcc cagcttgctt gtgtataagt     480
   tgtatttaat ggttctg                                                    497
<210> 492
   <211> 540
   <212> DNA
   <213> human
<220>
   <221> misc_feature
   <222> (380)..(380)
   <223> n is a, c, g, or t
<400> 492
   cactgttcca tggtcagcaa gtcatatttc ataatgtgga ttttccaaaa taattattga      60
   atacagctat tctatggcta cttttagtgt ttttgtggta tgtggtgtgg gagtgtttat     120
   ggaattacca gtatcttaaa ttttcaaagg aaccttggaa gtctatcact ctaaatgaaa     180
   gtctgtcact ctacatgaat tatgtgctca aatttgacca actcagttta agacacaaaa     240
   cagtaatttg aagaaggaaa aatgaagaga gtttctagtt taatgggtta aatttttgtt     300
   gttgcaatag taagtttagt cttcttataa tatttctaaa tgaaaaatca taggtatttg     360
   ttaccatgtg tgaagattan tttgttaaaa gcaaaagtgg tcgtgtgata tgctaaatgt     420
   taattactga ttttatatgt ttaaatcacg ccaaacaaat tatgtctgtg ccatccaggg     480
   tctgttgtta atctttttct gagtacttgg attgggataa agggcttgta ctatgcactt     540
<210> 493
   <211> 569
   <212> DNA
   <213> human
<220>
   <221> misc_feature
   <222> (79)..(79)
   <223> n is a, c, g, or t
<220>
   <221> misc_feature
   <222> (286)..(286)
   <223> n is a, c, g, or t
<400> 493
   acacttatag tctactgccc atgtaaggat cagctccggc taagaggcca aagatgggtg      60
   acatcgttat gctctgccnt ttattttttc tttcttaccc acttagcttc ctaattggag     120
   gaaggaggcg tggtaaaggt atatgaagac tatggtttaa ttagaccaga aaacactgtc     180
   ataatctctg gggtcatcag aatgtccagt tttgtctttg ggccaagata agggcagtgg      240
   gatttatgat gtgttgttta tagtctgaaa ctactctggt gatcanccag ggtcagtttc     300
   tttaatgatg gtttccaact ggcctaatac attaagtaag actggctgat aacatgacca     360
   gacagacata aagaccctgt tgggaatgac attgaactct caaagtcaag atttcttaca     420
   caaatctatc agctggagaa aatgaaggca gtgtggtata tgtgtgcaaa taaggacatt     480
   atgaagctta aatatggaat gtctcttgga cccccgatgt catctgtatt ctctttttct     540
   tcttgtacta tatcctttgc ctgtaaata                                       569
<210> 494
   <211> 326
   <212> DNA
   <213> human
<400> 494
   tcccagtgcg aactctgctg tgagtgtgtg cggggaggcg cgcccgcgct gagtcggcgg     60
   cgggtagcca ctccatgccc ttgtccgatg gtttgcaact ccgattttgc acaccgctcc    120
   accgtgcccc ccagcgcaca cccattcaca ctcacgccaa cactctcgct gaacactttt    180
   ataattgtta ggcgtggccg ttgggacttt gggcgcagcg cggctgctac tgcgtctgga    240
   ggattgatat ttatttttgc attgcgatgg ctgaaggcat ttatttaacg atctttttac    300
   ctggatatgt ctgtgaggct cctgaa                                         326
<210> 495
   <211> 548
   <212> DNA
   <213> human
<400> 495
   gtgaagtacc atagaccata ttgtctagat gggacaaaat aataaatttt gactaagaac     60
   ttactgttta gagagaaaaa atagtaaata atgttcaaaa tgagaagaca ataactgaca    120
   agtctgtgat ttttgggtta gatgagacca cttttgattt ttgaggaata aaaggagtta    180
   gttttttggt cctgatgtgt aaaatgattc atggggtttc acatacagac aagtagtttt    240
   cttttcagca aatatttaac atatctctca atatattttt caccagtttt tggtaaatat    300
   gcattgctat aggtatacca agtttttttt gttatgttta gtaactaatt tcattttttc    360
   tgtatatgta aatatagcta ttggaccctg cattgaaaac cttcttagta ctaacaacac    420
   tccttttatt attaccttca tagttacttt aaatgacata tttatttcga gttttacttc    480
   tggctgagtt gcaaagtcta tgagaatgtt acctcctgtc tcagaagctt gttagttatt    540
   gtgccacc                                                             548
<210> 496
   <211> 476
   <212> DNA
   <213> human
<400> 496
   acatctgcta gaaccttttg ccttaactat tcaccaatat atgctaatat tcataaatat     60
   ggattgactg tttacaaaca ttagaatctt gtcttggttc cattttgatg gctaatattt    120
   gttatcttaa ttaagactat ttctgaggtc atgattactt gaaaatattg actaaaactg    180
   ggtccttaga aattccaggt ggagctgatt tacctatgac tgaggggaaa aaaaaatcaa    240
   attttactga taatagtaat gctccaaatg aattaatgac acatctgttc aataaataaa    300
   gagcttaaat atacaaaaca taagaaatct gggcaacaaa acttgtggtc tttacttttg    360
   aatagctacc caagaaaagg ttttaaaggt aaaagttatg agtaatgtca tcacaataag    420
   ctcttgttta aaattctttt cttttatgta taattaggtt tatgtttcat gtcttt         476
<210> 497
   <211> 414
   <212> DNA
   <213> human
<400> 497
   atcccatggg caataaagcg cattcaatgt gtttataagc caaacagtca ctttgtttaa     60
   gcaaacacaa gtacaaagta aaatagaacc acaaaataat gaactgcatg ttcataacat    120
   acaaaaatcg ccgcctactc agtaggtaac tacaacattc caactcctga atatatttat    180
   aaatttacat tttcagttaa aaaaatagac ttttgagagt tcagattttg ttttagattt    240
   tgttttctta cattctggag aaccgaagct cagctcagcc ctcttcctta ttttgctccc    300
   aaagcctccc ccaaatcatc actccctgcc ccccttaagg ctagaggtga gcatgtccct    360
   cacaattgca catgtcaagc catcagcaag gcgcatcaca caaaaggcac caag          414
<210> 498
   <211> 309
   <212> DNA
   <213> human
<220>
   <221> misc_feature
   <222> (61)..(61)
   <223> n is a, c, g, or t
<400> 498
   cagtgagtcg aggttgcgct actgcactcc agcctggaca acagagggag actctgtctc     60
   naaaaaaaaa aaaacctaca gctgttcaag gaccagctga caggtcaagt gtggcctttt    120
   ctggtctttg aacacatcat agaaagtgac aaatgctgca aagccatgaa gaacatgaac    180
   tataaacggg tagactaact gcccagctta gacacttatc tatgccacaa aacagctgaa    240
   tttgtcacat ttatatattg caatatggga agtattgaga tcaaaacagg attccattga    300
   cctaattat                                                             309
<210> 499
   <211> 233
   <212> DNA
   <213> human
<400> 499
   gccatcacag ttgcgattcc atgagtagct gctttatgac tgctttttgt actatctgga     60
   tgtgcccaga gttacttctg tacaagctct gtatctatgt ccgttgagaa cattatttta    120
   acaagaagaa caccaacagt agcatgaaat ataatactgt tttataattc taaagctgct    180
   gttaatttat gaagtacata ataatctaat gtaaactgca gaagtcagag caa           233
<210> 500
   <211> 420
   <212> DNA
   <213> human
<220>
   <221> misc_feature
   <222> (71)..(71)
   <223> n is a, c, g, or t
<400> 500
   gcaaccattg gggaatgacc ttttcatttc agaagtggat gaggaaggtg gtgtgagcat     60
   caggtatatt nctggaccat ttcaagtgct ggtgagaaga aaggaactct ttgcctgaac    120
   tgggcttggt tttccaagtg ctgctttgga aatgaagacc cagagatgca gagcttatgg    180
   tagttcataa atcttcatgt tctattatct ttcatctgcc aataaagttc attttcaata    240
   atgtccacca ttgctgtgcc cagaataacc acaggcaaac atcaaaacaa tacgcataag    300
   ttagacaaga ttaaatcttg tctgatatct gcacaaacag atatgcacca tgttggaaac    360
   atgtgttttc ctagtcccat ccaggcttcc cacaagaaag ccatgatgtg ggtctaaacc    420
<210> 501
   <211> 390
   <212> DNA
   <213> human
<220>
   <221> misc_feature
   <222> (96)..(96)
   <223> n is a, c, g, or t
<400> 501
   attgtttccc atagcagaat gtcaatattc acagtacatt tctgtaaaga gcaaaccaat      60
   ataatgtttt gagtgttgaa aaaaattcca gatttntgaa gaattagaca actcttcatc     120
   taccttattt ctagttcaca cagttatctc aaattccact gaaactaatg ggatactgtc     180
   ttgtgtagat gccagttgag tttataatgt gacctagtaa agctgtcttt tttgttgtgt     240
   tgtatgagtg tcggatcatg cttttaggaa tacttttatt aaaatggtgt gcattcatgc     300
   aaaaggccaa ctggcttttg tgaacaatag atcttttctc ccctttattt tgttctcttg     360
   acacttttgt gaaaattacc tagcctgata                                      390
<210> 502
   <211> 460
   <212> DNA
   <213> human
<220>
   <221> misc_feature
   <222> (258)..(258)
   <223> n is a, c, g, or t
<400> 502
   aaagccttcc ctaaaaagag agcatgccag tcatagagac actaatttgg cacttttcct      60
   ccttcttagg tttaaatgtt ataccaatgc atgtgtttga aatatccatt aagatgattt     120
   aaaatttgtc ttgtattttg agtttcttaa atgcgtggat tcttgttcat ttatctctga     180
   atatgtctct tttatatttt ggcacaatta tacacattgg aaagggccaa cctattaggg     240
   ctcaagtatg tatatgcnaa aaaaaaaagt tatatcaaac aggcacagtt attacaacta     300
   gagagaaatt ccagaaatat ttgttttttt aagagaaagt aaattttcac attagatttc     360
   tattcaaagt actaatatct acatggtcca cacttttcta ttttaaaaaa ttgtgttctc     420
   ttgttaaata gattaacatt tccacttctg tttatcacaa                           460
<210> 503
   <211> 514
   <212> DNA
   <213> human
<220>
   <221> misc_feature
   <222> (285)..(285)
   <223> n is a, c, g, or t
<220>
   <221> misc_feature
   <222> (469)..(469)
   <223> n is a, c, g, or t
<400> 503
   atctttccat attgaggaca gcctctgcat ataaaaatgt tcattgggag aaaaattatt      60
   tttaaaacta aatttttata gtagtcctcc attactggca attaaggagc aacccacaaa     120
   atgtcattat gtggttcttt gattaggcat aaaaagtatt gcaaacagct tttctattcc     180
   tttggaatat gaatcttttt aatgtctaaa gttaacctgc aacattttct agtaggacaa     240
   gtcaatgtaa attaggtaat ttttgcctgt tttatacctt ctgtngcatt ttgttcaaat     300
   gaaaacattt tttaaaacat gattgaattt ttacttctca gataattggt ttgagtgtgg     360
   aatttctttt ttaacacttt gatatgtaag ttagtgtaaa ttaatacttt tataacacta     420
   ctaggcttta tatttttatg gaatgtttta aaatttgaaa tttttgaant gttaatttag     480
   cagtacttta tcattatgtg ccaatactga caag                                 514
<210> 504
   <211> 445
   <212> DNA
   <213> human
<400> 504
   tttactccag tcattcgatg ttgctgagat ttacatatga ctcttgtcaa catctcatct      60
   tttgacccaa tcttattcat ttaataagag gtctcattca tttgcatgga aaaatgctca     120
   ttgtatattg caaagtgaaa ataacgagtt gcaaaacagt gtatacatat atgtgtgtat     180
   atatgtacac tttatttgta catttctatg tgacataatg caaaggaaag tgtctgattt     240
   tattatacac caaaggttaa cagtgaatct ctgtgtgatc tctttttttt tctttttgcc     300
   tatctgcatc ttctcacttg ccaaaaaatg aatatatgtt tatgtgtgta tattacttgt     360
   gtcacaaaaa accctaaagt agacagtaaa agaacttgtc aatcgccttt ggaaggcaat     420
   gaaacactta ataaactctc aataa                                           445
<210> 505
   <211> 514
   <212> DNA
   <213> human
<220>
   <221> misc_feature
   <222> (26)..(26)
   <223> n is a, c, g, or t
<220>
   <221> misc_feature
   <222> (128)..(128)
   <223> n is a, c, g, or t
<400> 505
   agccaagaac tgaccggggc cagggnctgc catggtctcc ttgcctgctc caaggcacag      60
   gatacagtgg gaatcttgag actctttggc catttcccat ggctcagact aagctccaag     120
   cccttcanga gttccaaggg aacacttgaa ccatggacaa gactctctca agatggcaaa     180
   tggctaattg aggttctgaa gttcttcagt acattgctgt aggtcctgag gccagggatt     240
   tttaattaaa tggggtgatg ggtggccaat accacaattc ctgctgaaaa acactcttcc     300
   agtccaaaag cttcttgata cagaaaaaag agcctggatt tacagaaaca tatagatctg     360
   gtttgaattc cagatcgagt ttacagttgt gaaatcttga aggtattact taacttcact     420
   acagattgtc tagaagacct ttctaggagt tatctgattc tagaagggtc tatacttgtc     480
   cttgtcttta agctatttga caactctacg tgtt                                 514
<210> 506
   <211> 432
   <212> DNA
   <213> human
<220>
   <221> misc_feature
   <222> (197)..(197)
   <223> n is a, c, g, or t
<220>
   <221> misc_feature
   <222> (268)..(268)
   <223> n is a, c, g, or t
<220>
   <221> misc_feature
   <222> (357)..(357)
   <223> n is a, c, g, or t
<220>
   <221> misc_feature
   <222> (364)..(365)
   <223> n is a, c, g, or t
<400> 506
   gggctgttcc agagattcga tcctcttaag gcattatcag tgagcaaatg tgaaggaaat     60
   ggtgtctgga agaaagttct ggttcacatg ccttgtagct aagtctttct gcaaacaacc    120
   tcccttcccc ccgtcgagtc atttggtgac tttgatgggg ggatttctgg ttatgtcaag    180
   gctctggaga caggaanggc ctttggccgc cttgggtagt tgacctgcct tttctgactc    240
   cgggacgagc cagtcctagg ctgcctcngg gagcacttga ggtatcccgc aggccatgag    300
   gacccactgg gcagctcctg gacagcctct tggctccagc ccccacccga aagtggnaca    360
   ctgnntccgc cctggccacc tggggactgg cactgtggtg cacagtggcc caatgtggcc    420
   aacggaagtt tt                                                        432
<210> 507
   <211> 225
   <212> DNA
   <213> human
<220>
   <221> misc_feature
   <222> (35)..(36)
   <223> n is a, c, g, or t
<220>
   <221> misc_feature
   <222> (38)..(43)
   <223> n is a, c, g, or t
<400> 507
   cacaggtgta gcactcccaa agcaagactc caganngnnn nnnacctcat gcctggcacc     60
   tgaggtaccc agcagcctcc tgtctcccct ttcagccttc acagcagtga gctgcaatgt    120
   tggagggctt catctcgggc tgcaaggacc ctgggaaagt tccagaactc cacgtccttg    180
   tctcaattgt gccatcaact ttcagagcta tcatgagcca acctc                    225
<210> 508
   <211> 224
   <212> DNA
   <213> human
<220>
   <221> misc_feature
   <222> (59)..(59)
   <223> n is a, c, g, or t
<400> 508
   caccatgacc ggtcacagct acaaatccag agaccatcaa tcctgctaga gtgcagggng     60
   gcaagcaccc aagggtggct gaccaagact gcagagtctc ctccatcttc aggtccattc    120
   agcctcctgg catttaacta ccagcatcca gtggtcccca aggaatccct tcctagcctc    180
   ctgacatgag tctgctggaa agagcatcca aacaaacaag taat                     224
<210> 509
   <211> 177
   <212> DNA
   <213> human
<400> 509
   ggttagattc cgttttgact gcttggtatc tcattgtaag catttcccag ggacactaca     60
   cagtctttat aattatgatc actacatctt ttggataact taggcttttg cactattttg    120
   tcacgatgcc tcaaactttt ctatcattgt ctgtaattac tcctataatc ttttatt       177
<210> 510
   <211> 498
   <212> DNA
   <213> human
<400> 510
   aatatctcaa gatttctagg cattttccta tgtctaatag taggatgttc tctctgaatt     60
   tatttattac aaaaataaca ttacaacaat aaagcaaaat aatatttaaa agaaagaaaa    120
   aacaaacagt cgcaatacca gaacaccatt gtagtcgttt gtgtatgtgt gacttcttta    180
   ctgtgttcat gcagagacaa gctttgtaat acctttgtaa ctactgtaca ggtgcgtggt    240
   tagattccgt tttgactgct tggtatctca ttgtaagcat ttcccaggga cactacacag    300
   tctttataat tatgatcact acatcttttg gataacttag gcttttgcac tattttgtca    360
   cgatgcctca aacttttcta tcattgtctg taattactcc tataatcttt tattataaaa    420
   tattgaaaca aagtcacatc actaaaaaca ctgtgagtat aatttattag taagtgtaga    480
   ctgtgtatca ttagcaaa                                                  498
<210> 511
   <211> 453
   <212> DNA
   <213> human
<220>
   <221> misc_feature
   <222> (348)..(348)
   <223> n is a, c, g, or t
<400> 511
   ggagctgctt caagaagaaa cccggcagaa gctcaacgtg tctacgaagc tgcgccagct     60
   ggaggaggag cggaacagcc tgcaagacca gctggacgag gagatggagg ccaagcagaa    120
   cctggagcgc cacatctcca ctctcaacat ccaggtgcct gccccgtgtc cttgcttcct    180
   tcatgggtcc tctcaacttc tctgcgctga gatcccccgc aggcagatcg cggtggagtg    240
   ttggtgcgat ggtgcttgac cccccagctt cccctgctat tgggtttctc caacgaggag    300
   acatggtctt cgcttctcag agtctgtggg gccagggaca ggggccantc atggtccccc    360
   tctcacccta ccctggacgc tgtccttgta gctctccgac tcgaagaaga agctgcagga    420
   ctttgccagc accgtggaag ctctggaaga ggg                                 453
<210> 512
   <211> 447
   <212> DNA
   <213> human
<220>
   <221> misc_feature
   <222> (49).. (49)
   <223> n is a, c, g, or t
<220>
   <221> misc_feature
   <222> (153)..(153)
   <223> n is a, c, g, or t
<220>
   <221> misc_feature
   <222> (177)..(177)
   <223> n is a, c, g, or t
<400> 512
   tgttaccttt cagttacatg cctttgatcc taaaattctc tacttttgnt gccttatcag     60
   ttctttgcaa tctgcctgtg gttatcagca cttaaagcac aattttgaag gggaaaaaaa    120
   tgataatcac cttagtccca aagaaataat ttntgtcaaa ctgccttatt agtattnaaa    180
   acagacacac tgaatgaagt agcatgatac gcatatatcc tactcagtat cattggcctt    240
   ttatcaaatg gggaaactat acttttgtat tacatagttt tagaaatcga aagttagaga    300
   ctctttataa gtaatgtcaa ggaacagtaa tttaaaaaca aagttctaac aaatatattg    360
   tttgcttaat cacaatgccc tcaacttgta tttgaataac taaataggac atgtcttcct    420
   tggagctgtg ggcattagtt cagaagc                                        447
<210> 513
   <211> 469
   <212> DNA
   <213> human
<400> 513
   gtttacacct atactgcata atccaacaat tttaatttca gttgaagaca tgttactaat     60
   ataactatta ttaaaagagt agaggatgtg taattaacca tatcttctaa aacatggtta    120
   ctaaaagaat atgtaacatc aatattgacc ttggtttctt acacaagtgt tgctaactca    180
   atagtgaagg agacactatt aaattttctg aacccatgag agatactaga gatggggagt    240
   ggaaagtgtt tggttcaggg atatctgaag aacagaaggg cagagatttc ttaagtgacg    300
   cctcatctac aagctggaaa ttcctaaaaa caagtagaaa gcttataaac aacaggtgat    360
   acactcacct cactggtttt agtaaattac caatacagaa agtatcccta gtcttaaaaa    420
   caagtggaaa atttgaactg attagtcata ttcctttgat tacactgtt               469
<210> 514
   <211> 236
   <212> DNA
   <213> human
<220>
   <221> misc_feature
   <222> (135)..(135)
   <223> n is a, c, g, or t
<400> 514
   cagtcagagt ggacaaacct ctgtgggagg ctctactgca ctgagatgca gctcttccga     60
   gggggctcct aagccagtgt acaactgggt gcgtcttgga acttttccta caccttctcc    120
   tggcagcatg gttcnagatg aggtgtctgg ccagctcatt ctcaccaacc tctccctgac    180
   ctcctcgggc acctaccgct gtgtggccac caaccagatg ggcagtgcat cctgtg        236
<210> 515
   <211> 488
   <212> DNA
   <213> human
<400> 515
   gcctcctcag acactctcaa gaggatgggg agatgacatc acttgggtac aaacttatga     60
   agaaggtctc ttttatgctc aaaaaagtaa gaagccatta atggttattc atcacctgga    120
   ggattgtcaa tactctcaag cactaaagaa agtatttgcc caaaatgaag aaatacaaga    180
   aatggctcag aataagttca tcatgctaaa ccttatgcat gaaaccactg ataagaattt    240
   atcacctgat gggcaatatg tgcctagaat catgtttgta gacccttctt taacagttag    300
   agctgacata gctggaagat actctaacag attgtacaca tatgagcctc gggatttacc    360
   cctattgata gaaaacatga agaaagcatt aagacttatt cagtcagagc tataagagat    420
   gatagaaaaa agccttcact tcaaagaagt caaatttcat gaagaaaacc tctggcacat    480
   tgacaaat                                                             488
<210> 516
   <211> 452
   <212> DNA
   <213> human
<220>
   <221> misc_feature
   <222> (34)..(34)
   <223> n is a, c, g, or t
<400> 516
   aaggttatag ccacattcaa cttgaatggt cccnaatgtt aaatttacca tgaatactgt      60
   gtcttacaat gctgagctct cgactagact aaccagaata ggaacacagt ctcaaaggat     120
   accataagac aaacagtatt tagacatata aaaaactaca gagatgataa cttgaagaat     180
   aaataacgtg aaaaactact atattcttga atgggagtag aatgtaagct ctttgagggc     240
   agggactttg actgtcttgt tcattgctat atgataatgc ctggcccaac agcaggcatt     300
   ctgtagtgtc tgttgaacaa acatctaaaa tctggcaaag tgaacaatgg ttcctgagga     360
   taagtattta aataacaaaa ttaactttca ctacaaaagc tgccaactta cctttatctt     420
   ctggtgctat cccctgagct ttcttaagat ca                                   452
<210> 517
   <211> 399
   <212> DNA
   <213> human
<220>
   <221> misc_feature
   <222> (119)..(119)
   <223> n is a, c, g, or t
<400> 517
   gaatgcatga tctcaagcct taactactat aatctttttc tgcccctcag aaattgaata      60
   acctaaccaa gatgccttta ggggatgccc taagtaaatg taatttcaga tttcagggnt     120
   tttttttttt tcctctctaa gtgttccttc cctttcttct cctgctctcc atcatgttat     180
   ggagaccagt gaggaaccag tgttaacttg gtgacaatgt gacagctggt gctttatcta     240
   agctccgttt tctatttctt gggaatgctt tattgtggaa actgcttcag atacttaaat     300
   tgaatcataa cttgcttctg taaattgcgt aaagacaaca aactgatttt agtttgaaaa     360
   gtttatcttt tacttgtaaa ccttgtttgc cagttacct                            399
<210> 518
   <211> 529
   <212> DNA
   <213> human
<220>
   <221> misc_feature
   <222> (60)..(64)
   <223> n is a, c, g, or t
<220>
   <221> misc_feature
   <222> (68)..(70)
   <223> n is a, c, g, or t
<220>
   <221> misc_feature
   <222> (74)..(83)
   <223> n is a, c, g, or t
<220>
   <221> misc_feature
   <222> (86)..(87)
   <223> n is a, c, g, or t
<220>
   <221> misc_feature
   <222> (89)..(90)
   <223> n is a, c, g, or t
<220>
   <221> misc_feature
   <222> (92)..(92)
   <223> n is a, c, g, or t
<220>
   <221> misc_feature
   <222> (95)..(95)
   <223> n is a, c, g, or t
<220>
   <221> misc_feature
   <222> (99)..(99)
   <223> n is a, c, g, or t
<220>
   <221> misc_feature
   <222> (101)..(103)
   <223> n is a, c, g, or t
<220>
   <221> misc_feature
   <222> (111)..(112)
   <223> n is a, c, g, or t
<220>
   <221> misc_feature
   <222> (115)..(115)
   <223> n is a, c, g, or t
<220>
   <221> misc_feature
   <222> (121)..(121)
   <223> n is a, c, g, or t
<220>
   <221> misc_feature
   <222> (124)..(124)
   <223> n is a, c, g, or t
<220>
   <221> misc_feature
   <222> (127)..(127)
   <223> n is a, c, g, or t
<220>
   <221> misc_feature
   <222> (129)..(129)
   <223> n is a, c, g, or t
<220>
   <221> misc_feature
   <222> (432)..(432)
   <223> n is a, c, g, or t
<400> 518
   agccccaggt gtatttctga gtgtcaacag tgttaatttc aagcatgcta ataaaatgtn     60
   nnnncggnnn atannnnnnn nnnaannann cnttnaaant nnnggtttcc nntgntaaaa    120
   ncanccncnt ttgcttttgt tagctgtaat attttttgtc atttagataa gacctggttt    180
   ggctctcaat aaaagatgaa gacagtagct ctgtacaggg atatatctat attagtcttc    240
   atctgatgaa tgaagaaatt ttctcatatt atgttcaaga aagtatttac ttcctaaaaa    300
   tagaattccc gattctgtct attttggttg aataccagaa caaatctttc cgttgcaatc    360
   ccagtaaaac gaaagaaaag gaatatctta cagactgttc atattagatg tatgtagact    420
   gttaatttgc antttcccca tatttcctgc ctatcttacc cagataactt tctttgaagg    480
   taaaagctgt gcaaaaggca tgagactcag gcctactctt tgtttaaat                529
<210> 519
   <211> 281
   <212> DNA
   <213> human
<220>
   <221> misc_feature
   <222> (31)..(31)
   <223> n is a, c, g, or t
<400> 519
   aatcttgtga gttgtgacct aggtgaaggt ntaatgataa catctggagg aagcatcatt     60
   tgttcagcta cctagggttt actttcagac ttttcctaca gtttaaagca cttgcgttca    120
   gtgtggtacg atctgtttgt aatgttaatc attgactatt gttctgctac ttggatgttg    180
   atagtgaagc agagaacaat ttatcatcgt ttattatgag tcactatgca cgcaactatg    240
   cttaacatgg cgaaatgtat taaacactcg tccaactatt t                        281
<210> 520
   <211> 352
   <212> DNA
   <213> human
<400> 520
   aaagggtggc atttgcgtca cgtggaccag ggacagtgct gaaatcagca gtgctcagaa     60
   acaatttaac atgttgaaac gacaatattc taaaatactg atgaatcttg catcaatata    120
   attattgggt tttttttctt tttcctgctg tataactcct tgccatgcaa actctcaaga    180
   ggccaatata ttcctggcca tgtttgaatg agcctcttaa aataaactta gagccatgca    240
   aatgccagca gcttaatgga tttcatggaa tgaaataccg tgattaactc atagctacat    300
   atcattgcat aaatgggatt tatctttttt ctcacttatt tttgcggtga aa            352
<210> 521
   <211> 245
   <212> DNA
   <213> human
<400> 521
   agagccatgc aaatgccagc agcttaatgg atttcatgga atgaaatacc gtgattaact     60
   catagctaca tatcattgca taaatgggat ttatcttttt tctcacttat ttttgcggtg    120
   aaagtcgagg gcatgcaaga gtttctcttc cagaagccaa gaggagaaca aaggtcctaa    180
   tgctgtacta ttccaccctt tggacgcctc atccaggacg cagaggactc taggtttaac    240
   atttt                                                                245
<210> 522
   <211> 495
   <212> DNA
   <213> human
<400> 522
   gacccagagt cacctaagag aactcttcca acttaaatga ctagggtgtg taatcagttg     60
   cttcttattc tgtgtctcag atttcctagt ccatgacatt taatagtgag tatagagtag    120
   gatattctat acattttagt atttggccct tggaaacaaa gttaaatacc atgtcactca    180
   gaagactctc aaagtttgtg tataaatggc gaagtatgca ctatgagaga ttctaatcca    240
   ttcacatcat tcttgagttg tgcaaataca cttggctttg atttcacttg gtgatctaaa    300
   tttgtttatt taagctgcaa gaaagttaca ttaatttgga atgtgtcatc acttggaccc    360
   agtatatcag atttttattg agtaaaatgt taaaaataat aagtgtaact cagattctgg    420
   atgttcgagt ttacaataca ttgcctgtaa taaaagctga ttctgaagtg catttttctt   480
   gacttacttt ggcag                                                     495
<210> 523
   <211> 374
   <212> DNA
   <213> human
<400> 523
   atgcacgtac aaatttctta acctgttatc aatgtctgag ctacataatt atctttctag    60
   ttggagtttg ttttaggtgt gtaccaactg acatttcagt ttttctgttt gaagtccaat   120
   gtattagtga ctctgtggct gctctcttca cctgcccctt gtggcctgtc tacaattcta   180
   aatggatttt gaactcaatg tcgtcgcttc tggtttcctg catataccaa tagcattacc   240
   tatgactttt tttttcctga gctattttca ctgagctgag ctaatgaact aaaactgagt   300
   tatgtttaat atttgtatca aatacataaa aggaatactg ctttttcctt ttgtggctca   360
   aaggtagctg catt                                                     374
<210> 524
   <211> 451
   <212> DNA
   <213> human
<220>
   <221> misc_feature
   <222> (271)..(271)
   <223> n is a, c, g, or t
<400> 524
   ggaatctttt ccaataacca gctaaagatt tgcactgaaa tacaacttgt atgccttttg    60
   catttttaaa gcctgcttcc tggatttaag cagagtgata gtgttcaaag agccagttca   120
   gcctgtaaca tatttgaaaa agatatgtct gcactttgag gtcccttttg aatgccattc   180
   actagacctc tcaagcattt tgtttcattg ctacatccaa gcgcctcaca agtccacaat   240
   gcgggacagc atcaaaagct caagactttg naaaaagctt gtgggcttgc actgggggag   300
   ggaagggaac aaaatttgtg tacttctttg tttaatttag aaataaggca tccaagagat   360
   gccattattt tctgtgtttc aattgttgtg cctttgagtt aaactgcatt tttgtctttt   420
   ggttgaaatc tgaaatgtac tgtcccaata t                                  451
<210> 525
   <211> 360
   <212> DNA
   <213> human
<400> 525
   ctccttatct gttctagttc cgaagcagtt tcactcgaag ttgtgcagtc ctggttgcag    60
   ctttccgcat ctgccttcgt ttcgtgtaga ttgacgcgtt tctttgtaat ttcagtgttt   120
   ctgacaagat ttaaaaaaaa aaaaaaggaa aaaaaaagaa aaaatgaatt tactgctgca   180
   ggtttttttc tctctccatg tgtcactaag tgaagtttgt gccttctata gcaaagagaa   240
   tattttttac atcctactaa cagtagattt ttttgtagtg aacatttttt gtatttttat   300
   ttataagtct cataagaaaa atagcaatgt tcagttgtat accttgaatc tgcagttaga   360
<210> 526
   <211> 541
   <212> DNA
   <213> human
<400> 526
   aataggtctt gcaggtttcc ttttgaattt taagtatcat aaatattttt taagtaaata    60
   atacggggtg tcagtaatat ctgcagaatg aatgcagtct ttcatgctaa tgagttagtc   120
   tggaaaaata aagtcttatt ttctatgttt tattcataga aatggagtat taatttttaa   180
   tattttcacc atatgtgata acaaaggatc tttcatgaat gtccaagggt aagtcagtat   240
   taattaatgc tgtattacaa ggcaatgcta ccttctttat tccccctttg aactaccttt   300
   gaagtcacta tgagcacatg gatagaaatt taactttttt ttgtaaagca agcttaaaat   360
   gtttatgtat acatacccag caacttttat aaatgtgtta aacaatttta ctgattttta   420
   taataaatat tttggtaaga ttttgaataa tatgaattca ggcagatata ctaaactgct   480
   tttatttact tgtttagaaa attgtatata tatgtttgtg tatcctaaca gctgctatga   540
   a                                                                   541
<210> 527
   <211> 484
   <212> DNA
   <213> human
<220>
   <221> misc_feature
   <222> (70)..(70)
   <223> n is a, c, g, or t
<400> 527
   gagaacatca gatctaatag agcatagtga tactatttaa tttaaccaaa gtctctagtg     60
   aatatttcan ctttgaatgt aaactaacaa ataaacctga ccaccaagga gattgtttgc    120
   ccagagtttc aaagcacatt gtctacaaat ggaaattgaa ataatttata aaatattgac    180
   gttactatgt tttttaaaaa gttcctaatt ttttcactaa atggaggaaa ctattagttt    240
   tattgttaaa tatggtagat attaatattc ctcttagatg accagtgatt ccaattgtcc    300
   cagtttgaaa taagtaccct gtgagtatga gataaattag tgacaatcag aacaagtttc    360
   agtatcagat gttcaagagg aagttgctat tgcattgatt ttaatatttg tacataaaca    420
   ctgatttttt tgagcattat tttgtatttg ttgtacttta atacctggtg tacagttcca    480
   gaaa                                                                 484
<210> 528
   <211> 399
   <212> DNA
   <213> human
<220>
   <221> misc_feature
   <222> (119)..(120)
   <223> n is a, c, g, or t
<220>
   <221> misc_feature
   <222> (145)..(145)
   <223> n is a, c, g, or t
<400> 528
   gaaggatgct gaggacctag atgagaagtt acctgcaaaa ggcaaaaggg ttacttagtg     60
   tcagaaccaa ggcaatgact tctctctccc agatctccta gctactggtc ctgggccann    120
   tttttttttt taaataatcc caacntttct ttaaaagaca agcatttcag taagctagtt    180
   attttcatgg gttgctcatc catttttttc agtgatctaa aaatgtaggg agatggctac    240
   tactgaagtt gtctgtctac ttgggataat agcaaattaa ttgaagacaa tgggaaagta    300
   agttataaaa aatactggga aatctgtttc tcttctgagc aagcattcag ggcaggtata    360
   aacatcaaac atagtgacat tgtcaaaacc tcttccatt                           399
<210> 529
   <211> 420
   <212> DNA
   <213> human
<220>
   <221> misc_feature
   <222> (78)..(78)
   <223> n is a, c, g, or t
<220>
   <221> misc_feature
   <222> (161)..(161)
   <223> n is a, c, g, or t
<220>
   <221> misc_feature
   <222> (163)..(165)
   <223> n is a, c, g, or t
<400> 529
   ggaaagagaa acgtggctca agtgctggct caccttctag ctgtgtggcc ctgggcaggt     60
   tactgaggct cttccaanct cacttttcac atgtaaaatg gcatttctaa aagtacctac    120
   cctacctcaa aggaattctg aaaaaagtaa gtgaatttgt nannnaagca aagcactcac    180
   aatatgcctg acacacaaac tgcttaataa atgttagtaa tttttaaaac ctcctcataa    240
   gcggcttaga aattattcat gtttggtaca tatttattgc aagtcaagca gatccaactt    300
   ttttgaaagc tggtggtcta ggagtatagt tttaattata tttgtaaata ctcaaaagta    360
   ttagatgcca gtctaattta aatgcagtct gactgtagcg agtaactgac ctgggcaatg    420
<210> 530
   <211> 378
   <212> DNA
   <213> human
<400> 530
   gggactcagt tcttagagaa gtcttttggt cttagagttc atcaagcaga gaaggggaag     60
   gacatggtcc aggaaaataa atcagcacac aaagagatgg ggtcttgagg gtgcatagta    120
   cattctggac agccagttac ctgggatgag ttgggaggga ggagaataag gacaaaagac    180
   catctgggca aaaatcacga aggggtatgt gtgtcatgta aaggtgtgcc atgatagtta    240
   ttcatattgc tattgtaata ttaatatata gtaattaact acacatgaca cagctttaca    300
   tgaccttaag tagttatcaa cattaccata atagtaatat taataactac aataagagcc    360
   attattattc acttgagg                                                  378
<210> 531
   <211> 360
   <212> DNA
   <213> human
<400> 531
   gttagcaagt gccagcttgt aggctggttg aagtacagaa ctcagaggaa aaaagaaatt     60
   aaattttagc tttctggaga gcagcccctc tctggcacca tcaaacactt ctttgtttcc    120
   cttcaacttg gaactcttca aacatcaggg gttgtgaggg tttggccatt cttttatctt    180
   gggtccatgt gagtgacaga aatggtgcgg cctgggaaag atctccctcc tttacatttt    240
   ctcttctccc tcctcctcct tattctaaaa ctgtgcctcc aacagagggg caggggctct    300
   tgtagagaga tccctggccc aggacaggag atgccaaatc taatttatct cactgagggc    360
<210> 532
   <211> 502
   <212> DNA
   <213> human
<220>
   <221> misc_feature
   <222> (371)..(371)
   <223> n is a, c, g, or t
<400> 532
   tttcctacca atttcacatt ttgcagaaac ttgttcacat ttccaacaat atcagaatta     60
   gaaaacagtt cagataacaa gaaagattaa aaattaggga aattctgata tcaccataaa    120
   gcactatttt acatttagag attacattta agataaagtc atcatacaca aaaacaataa    180
   atatttataa ctttctctat aaggtccgca tatactgtat atattgaaac aatctgaatg    240
   actagtagat ttcatatgac cattgttatt tccactttct ccaatacttg tattttatgc    300
   tacatgtaat gaagttggac ctttttatta tttagtaatt cctatatgtt cctatacttt    360
   tcattttcaa natgattgct ctattgtttc atgttgtttc tagcaatata tctccatgag    420
   atatgcactt tgtttcatat tgaaaagtat aaaatttatc tttcaattcc tgtgtgtgta    480
   tcctatggtt atctgtatgt at                                             502
<210> 533
   <211> 258
   <212> DNA
   <213> human
<400> 533
   tcccaacccg gttctggcaa agaatccagt tatcccttcc atgaagacgc acataactct     60
   cttacttggt ctttccatta gggacaacat aagtcttgtt ttacatcaaa taaaaacaat    120
   gttaaaaagt gtgtgaacct taaaaatgga agtctactag tttacatacc tacttcagag    180
   gacatggaaa tgaccatggg cctgcatttc agggaccaaa gcaaattagg cctggcctaa    240
   aatacatcag accttttg                                                  258
<210> 534
   <211> 460
   <212> DNA
   <213> human
<400> 534
   gttgacccca actttggaac tcagatcata ggatctccca gaggtttcag gtgttcctaa     60
   gaagcttcta aaggaagttt tggaagaaag actaataggt gtaaacatat aaacatatat    120
   attatatata gctatatata tttaaagaga tctctacata tatatatata taaatacaac    180
   tactgtactc tctgcatctg tacaaagtat gagttaaact gcccaggtag tgtgagatac    240
   gtaatgcatg aatgtgaata tcctgaaatc tgccttctta gcaacgtgcc gcgtgtactg    300
   gagagaggac agttgctcct gggagttctc tcaatgggca aatgtacaag ttgatgccgt    360
   cttatccacc aacaagccat taaggttggt ttcctgctga gacttttgta ccggaagcga    420
   gaccggcagc actgggggtg tccatatgcc acttatgagg                          460
<210> 535
   <211> 493
   <212> DNA
   <213> human
<220>
   <221> misc_feature
   <222> (280)..(280)
   <223> n is a, c, g, or t
<400> 535
   ggccaaggca catttgactc ctgagatgaa ttttttgtgg tcataatcaa atacttagtt     60
   gtttttgatg ccccaaaata aagtgagaat ggtaatttgc caggaattct tcataacagt    120
   atcttacaaa aaacgtgttg ctctcttcac agtattatgt gtaaagtcat tgtttaaagc    180
   acgaatgttc cctctggggt acttgttaaa gctaaattta ttttgcttcc ctccacttag    240
   aagtgctgca cactttacag cagcttcctt tctttccatn gcactgccta gttaacagaa    300
   gtcttataaa aatttaaaaa gacacatttc ttacaaaaaa gagttgaatg aggtaaaatg    360
   gcattagatg gctctatatt ttttaaagct atgtaattgt tcagcgtcac ttttctaagt    420
   acttatacat atctaaacat gtcttcatgg tttatatttt cacttatata tgctgggctg    480
   gattaagctt tgt                                                       493
<210> 536
   <211> 486
   <212> DNA
   <213> human
<220>
   <221> misc_feature
   <222> (28)..(28)
   <223> n is a, c, g, or t
<220>
   <221> misc_feature
   <222> (31)..(31)
   <223> n is a, c, g, or t
<220>
   <221> misc_feature
   <222> (39)..(39)
   <223> n is a, c, g, or t
<220>
   <221> misc_feature
   <222> (118)..(149)
   <223> n is a, c, g, or t
<400> 536
   ggggcagagg gactatgttg tgagcctngc naaagaagnt tgtgtgggga ctgtgggcag     60
   tgaatgcgtt gggaacaata tggaaaactg ggagctgcct tggaatctac agggccgnnn    120
   nnnnnnnnnn nnnnnnnnnn nnnnnnnnng gctgaagaaa agaagaatgg tattcatgca    180
   gttcccattt tacagatgaa aacaagagga ctttttctgt gaagtcaaga aagtggttac    240
   aatggtactt tcagcctgtc cgaattatgt attgcccctc ccctttttat taataacatt     300
   gaagtgtgat gggacaacca ctgaagccgt ctgttgaaac ctgctgggac tttttagcca    360
   ttctcttcaa cataaagaat gggtgttttt ggagggggtg agaggaatgg ggaaatgttg    420
   tcaaagagta caatgtttta gttgagacag gaggaatata ttttgttgag atctacagca    480
   cagcat                                                               486
<210> 537
   <211> 299
   <212> DNA
   <213> human
<400> 537
   gatagcgaat gcactcaggg tcagcagtgg agtttaaaaa ttgtctcttt tcaacttatt     60
   taaatgacag cacctgagaa gaggaaccgt tttacactgg atgtttctca tgtagaacaa    120
   gaaatctttc tggaattgat gtttacatgt ctgttgttgg tcatctctcc tgtgtcttaa    180
   atactttaat gttggaagag catagtgttt gggctagtgg gtttctgaca gcccatggga    240
   atgccctgaa actactgtat ctgatgtttg ttttcgatga ggttccatgt tttgttttc     299
<210> 538
   <211> 367
   <212> DNA
   <213> human
<220>
   <221> misc_feature
   <222> (27)..(27)
   <223> n is a, c, g, or t
<220>
   <221> misc_feature
   <222> (32)..(32)
   <223> n is a, c, g, or t
<400> 538
   gcagctgtgg tggtcattat aagcatngtc cntcctggga agaagcatcc aggcaagcag     60
   aaaagaaaag atgcagccac cagaaaaaga aactccagaa gtcctgcatt tggatgaggc    120
   caaggatcac aacagcctaa acaacctaag agaaactttg ctctcagaaa agccaaactt    180
   ggcccaggtg gaacttgagt taaaagagag agatgtgctg tcagttttcc ttccggatgt    240
   accagaaact gagagctagt gagggttcag agaagcccca tcctaagcca gacacatgat    300
   gtgggctcag ctcagtggcc tgaaacctct caggttttag agtctctccc aagaagccgc    360
   ttttttc                                                              367
<210> 539
   <211> 394
   <212> DNA
   <213> human
<220>
   <221> misc_feature
   <222> (54)..(54)
   <223> n is a, c, g, or t
<220>
   <221> misc_feature
   <222> (58)..(59)
   <223> n is a, c, g, or t
<220>
   <221> misc_feature
   <222> (61)..(61)
   <223> n is a, c, g, or t
<220>
   <221> misc_feature
   <222> (65)..(65)
   <223> n is a, c, g, or t
<220>
   <221> misc_feature
   <222> (68)..(69)
   <223> n is a, c, g, or t
<220>
   <221> misc_feature
   <222> (71)..(72)
   <223> n is a, c, g, or t
<220>
   <221> misc_feature
   <222> (74)..(74)
   <223> n is a, c, g, or t
<220>
   <221> misc_feature
   <222> (76)..(76)
   <223> n is a, c, g, or t
<220>
   <221> misc_feature
   <222> (78)..(81)
   <223> n is a, c, g, or t
<220>
   <221> misc_feature
   <222> (83)..(84)
   <223> n is a, c, g, or t
<220>
   <221> misc_feature
   <222> (95)..(95)
   <223> n is a, c, g, or t
<220>
   <221> misc_feature
   <222> (115)..(115)
   <223> n is a, c, g, or t
<220>
   <221> misc_feature
   <222> (127)..(127)
   <223> n is a, c, g, or t
<220>
   <221> misc_feature
   <222> (129)..(129)
   <223> n is a, c, g, or t
<220>
   <221> misc_feature
   <222> (219)..(219)
   <223> n is a, c, g, or t
<400> 539
   tactagttgt tttacactct cttttcttat tcttagggct tttgtgtatg tctnactnnt     60
   ntttnaanna nntncncnnn nanncagacc ttaantttta tattttttta aagtngctaa    120
   catagcngna ggcacttaag catttagtca atgatattgg tagaaatagt aaaatacatc    180
   ctttaaatat atatctaagc atatatttta aaaggagcna aaataaaacc aaagtgttag    240
   taaattttga tttattagat attttagaaa aataatagaa ttctgaagtt ttaaaaatgt    300
   cagtaattaa tttattttca ttttcagaaa tatatgcatg cagttatgtt ttatttgatt    360
   gttgacttag gctatgtctg tatacagtaa ccaa                                394
<210> 540
   <211> 452
   <212> DNA
   <213> human
<220>
   <221> misc_feature
   <222> (72)..(72)
   <223> n is a, c, g, or t
<220>
   <221> misc_feature
   <222> (86)..(86)
   <223> n is a, c, g, or t
<220>
   <221> misc_feature
   <222> (93)..(93)
   <223> n is a, c, g, or t
<220>
   <221> misc_feature
   <222> (96)..(96)
   <223> n is a, c, g, or t
<220>
   <221> misc_feature
   <222> (98)..(98)
   <223> n is a, c, g, or t
<220>
   <221> misc_feature
   <222> (107)..(108)
   <223> n is a, c, g, or t
<220>
   <221> misc_feature
   <222> (111)..(111)
   <223> n is a, c, g, or t
<220>
   <221> misc_feature
   <222> (121)..(121)
   <223> n is a, c, g, or t
<220>
   <221> misc_feature
   <222> (124)..(124)
   <223> n is a, c, g, or t
<220>
   <221> misc_feature
   <222> (130)..(130)
   <223> n is a, c, g, or t
<220>
   <221> misc_feature
   <222> (264)..(266)
   <223> n is a, c, g, or t
<220>
   <221> misc_feature
   <222> (269)..(269)
   <223> n is a, c, g, or t
<220>
   <221> misc_feature
   <222> (273)..(274)
   <223> n is a, c, g, or t
<220>
   <221> misc_feature
   <222> (277)..(278)
   <223> n is a, c, g, or t
<220>
   <221> misc_feature
   <222> (281)..(284)
   <223> n is a, c, g, or t
<220>
   <221> misc_feature
   <222> (328)..(328)
   <223> n is a, c, g, or t
<220>
   <221> misc_feature
   <222> (362)..(362)
   <223> n is a, c, g, or t
<220>
   <221> misc_feature
   <222> (373)..(373)
   <223> n is a, c, g, or t
<220>
   <221> misc_feature
   <222> (379)..(379)
   <223> n is a, c, g, or t
<400> 540
   gagtaggttc ggtctgaaag gtgtgggcct ttatatttga tccacacacg ttggtctttt     60
   aaccgtgctg ancagaaaac aaaacnggtt aanaanancc gggtggnnag ntgacagagg    120
   naanccgctn aaataccttc acaataaata gtggcaatat atatatagtt taagaaggct    180
   ccccatttgg catcgtttaa tttaaatgtt atgttataag cacagctctc ttctccaatt    240
   ttcatcctgc aagcaactca aaannnttna aannaanntt nnnntgtagt tattttcaaa    300
   tctttgcttg ataagtatta agaaatantg gacttgctgc cgtaatttaa agctctgttg    360
   antttgtttc cgnttggant tttgggggag gggagcactg tgtttatgct ggaatatgaa    420
   gtctgagacc ttccggtgct gggaacacac aa                                  452
<210> 541
   <211> 457
   <212> DNA
   <213> human
<220>
   <221> misc_feature
   <222> (364)..(366)
   <223> n is a, c, g, or t
<220>
   <221> misc_feature
   <222> (369)..(369)
   <223> n is a, c, g, or t
<220>
   <221> misc_feature
   <222> (379)..(379)
   <223> n is a, c, g, or t
<220>
   <221> misc_feature
   <222> (382)..(383)
   <223> n is a, c, g, or t
<220>
   <221> misc_feature
   <222> (398)..(398)
   <223> n is a, c, g, or t
<220>
   <221> misc_feature
   <222> (407)..(407)
   <223> n is a, c, g, or t
<400> 541
   ggaacctata aatgtagttt catctgtcgt caattactgc agaaaattat gtgtatccac     60
   aacctagtta ttttaaaaat tatgttgact aaatacaaag tttgttttct aaaatgtaaa    120
   tatttgccac aatgtaaagc aaatcttagc tatattttaa atcataaata acatgttcaa    180
   gatacttaac aatttattta aaatctaaga ttgctctaac gtctagtgaa aaaaatattt    240
   ttaaaatttc agccaaataa tgcattttat ttataaaaat acagacagaa aattagggag    300
   aaacctctag ttttgccaat agaaaatgct tcttccattg aataaaagtt atttcaaatt    360
   gaannngtnc ctttcacang tnnatgatta aatctgantt cttaatnata tatcctatgc    420
   tgattttccc aaaacatgac ccatagtatt aaataca                             457
<210> 542
   <211> 344
   <212> DNA
   <213> human
<400> 542
   tctttttgag tgatagtgcc attgaatgag cagtatggaa acagtgttac ttgatatttt     60
   gagctttctc aggtttatct aaatcagtgg tagcttaaca aaacccagac taattgtgtg    120
   taattgtatt tttaataaaa ggaaagtaca tttcctataa tagcatagta ctgtttgcat    180
   gtaagagtat gcaaaacctt gtgtgtgtgt gtgtgtgtgt gtgtgtgtgt gtgtcttagt    240
   gtgtgtaagg catggcagcc aactttgtat ctgctatttt tagtacgagc agagcttcat    300
   aattgtggtc actagaactg tacttaccat ggacagttaa aact                     344
<210> 543
   <211> 414
   <212> DNA
   <213> human
<400> 543
   tctctcagtt taaacatctt aacagagaat ttgttactgt ttattaaaag aattgcattt     60
   ggaagcaaga aaacaaccgg atttgcaatc atgtgaagaa acaataatgc ctttattatc    120
   aagtgttaag cacaattctt ataacaaact gtgataaatt ctttttgttt ttttttcctt    180
   tgccccatta ttttcttatg aacaaaccaa aaattcatgg tgagcagttg cagtgttggc    240
   tgatatatct tttatgtaca gggaatttga aaaggacagt ggattcattt agaagtgtaa    300
   ctggtgctgt gattatagca atacatttgt tagttgtact tcatcttttt catgctagct    360
   ttttaaatgt ttagttttcc tcttgtcatg gtcagctgct gaatttactt gaag          414
<210> 544
   <211> 545
   <212> DNA
   <213> human
<400> 544
   agattctggc cacggctggg aacgttgtca ccctgaccat catccccagt gtgatctacg     60
   agcacatggt caaaaagttg cctccagtcc tgctccacca caccatggac cactccatcc    120
   cagatgcctg aagccactgc agggcagggc aggcaggggg ggcttcccgc cctcctgcag    180
   caaagggcaa ccaccctcgg atgatgggtt gcagccggcc tgctgcttaa ggtgggggct    240
   gccatgaggg gggcgtgtcc aggagggtga ccatgggatg gcttatacac acaggcctcc    300
   ttggagcctc agactccaag ctaggctgag gctcaggcag ggcccacagg cagccgattc    360
   tcttgtgctg atttaaatgc tggacacgga ggcaggctgt ttaaacgctg cttaaagtcg    420
   caactgggcc cctttcaaga aattttgctc taccaggaaa acagttacac attttaagag    480
   aacagagcta cgttctttgt gagagctttt tccttgcctt gacttgctct ttgtcacaga    540
   ctgca                                                                545
<210> 545
   <211> 248
   <212> DNA
   <213> human
<400> 545
   gtcaccatct cttgttctgg aagcagctcc aacatcggaa gtaattatgt atactggtac     60
   cagcagctcc caggaacggc ccccaaactc ctcatctata ggaataatca gcggccctca    120
   ggggtccctg accgattctc tggctccaag tctggcacct cagcctccct ggccatcagt    180
   gggctccggt ccgaggatga ggctgattat tactgtgcag catgggatga cagattgagt    240
   ggttttat                                                             248
<210> 546
   <211> 329
   <212> DNA
   <213> human
<220>
   <221> misc_feature
   <222> (73)..(73)
   <223> n is a, c, g, or t
<220>
   <221> misc_feature
   <222> (298)..(298)
   <223> n is a, c, g, or t
<400> 546
   gtttactctg agattctgta gtcctaatat tgtatcattg tgctgtctgc aaaacaactt     60
   gaatctattt tgnttgcatc ttttgttaca tgtaacgcag ctgtacttta tgttctttgc    120
   aactgtttcc attatgagaa cgctgtgcta tttacaaggt tacatttttc ttggccaggc    180
   gaggtggtca tgcctgtaat cccagcactt tgggaggcca aggtgggcgg atcacttgag    240
   gtaaagagtt gagaccagcc tggctagcat ggcgaaaccc agtctctact aaaaatanca    300
   aaaattagcc gggtgaaatt agccgggcg                                      329
<210> 547
   <211> 488
   <212> DNA
   <213> human
<400> 547
   ggtgttttaa ggttagcaca tgcacaaagg aacgagttgg tttaaaaaga taaatcactg     60
   caaagaatga aattggctta ttcacatcaa aactagataa gatgctaaaa aaaaaagata    120
   tgaaacagaa ctaacctata gtttcctgaa atcagtacag tttaatttat aagaagctag    180
   aaagtaatgc accttgattg ttttaggaat gatttatgtg ttgcaatttt aatttattta    240
   aagcatgtct actgtgtttg tcctaagaga aatatttcaa caaaacgtgc tctgtgttta    300
   agatatgttt aggcagtagt tagcaactct gaaagtagaa actggaaatg tttattgtga    360
   ggcttgttgc agaatttcca ttttgtgagt tactacttag tttcatgtca gcctaaaatt    420
   gtaaattccc tgtagatctt caccccattg tggtgtcatc aatgaatcca aagcaggtgc    480
   cattattt                                                             488
<210> 548
   <211> 373
   <212> DNA
   <213> human
<220>
   <221> misc_feature
   <222> (67)..(67)
   <223> n is a, c, g, or t
<220>
   <221> misc_feature
   <222> (74)..(74)
   <223> n is a, c, g, or t
<400> 548
   gcagggtcat aatcacacag cagtgcctta tatagttgcc ataagacttc agtgcagtac     60
   aacatanttt tacngctaca tatcagggca tattctatat ggtgtatttg tgttagaata    120
   acacattaaa tgtctttaaa cataaaaata agaatgtttg catgtttcag ttttcaagaa    180
   ccaaatgagt aattagctat agattccact ggccttaaac atacaattaa gtgtatacat    240
   gatatagtgc acacacaaaa gccaccttta attattgaaa taacctgtat tctttttgga    300
   aatcatttaa gtttggtatt gaagtactat attttttgtg catcaatgta tttttctatt    360
   tacaagccta tgt                                                       373
<210> 549
   <211> 498
   <212> DNA
   <213> human
<400> 549
   ggagatcgtg ttctgctttt tacatgagga gcagaactgg gccatacacg tgttcaagaa     60
   ctaggggagc tacctggtag caagtgagtg cagacccacc tcaccttggg ggaatctcaa    120
   actcataggc ctcagataca cgatcacctg tcatatcagg tgagcactgg cctgcttggg    180
   gagagacctg ggcccctcca ggtgtaggaa cagcaacact cctggctgac aactaagcca    240
   atatggccct aggtcattct tgcttccaat atgcttgcca ctccttaaat gtcctaatga    300
   tgagaaactc tctttctgac caattgctat gtttacataa cacgcatgta ctcatgcatc    360
   ccttgccaga gcccatatat gtatgcatat ataaacatag cactttttac tacatagctc    420
   agcacattgc aaggtttgca tttaagttaa aaaaaaaaag aaaaaaaacc taagggtgaa    480
   acgatgccac tttgacgc                                                  498
<210> 550
   <211> 551
   <212> DNA
   <213> human
<220>
   <221> misc_feature
   <222> (77)..(77)
   <223> n is a, c, g, or t
<220>
   <221> misc_feature
   <222> (228)..(228)
   <223> n is a, c, g, or t
<220>
   <221> misc_feature
   <222> (340)..(340)
   <223> n is a, c, g, or t
<220>
   <221> misc_feature
   <222> (342)..(346)
   <223> n is a, c, g, or t
<220>
   <221> misc_feature
   <222> (349)..(353)
   <223> n is a, c, g, or t
<220>
   <221> misc_feature
   <222> (355)..(356)
   <223> n is a, c, g, or t
<220>
   <221> misc_feature
   <222> (358)..(359)
   <223> n is a, c, g, or t
<220>
   <221> misc_feature
   <222> (361)..(361)
   <223> n is a, c, g, or t
<400> 550
   tgactttcat acacacccag tacatctcaa aggatgctaa gggacatttt ctgccagtag     60
   agttctcccc ctttttnggt gacagcaata ttattatgtt cacatctaac tccagagctt    120
   acttcctgtg gtgccaatgt atttgttgca atttactaca tttttatatg agcctattta    180
   taggtgccat taaactcagg tctttcaaat gaaagagttt ctagcccnct tagggaaaaa    240
   gataattgtt tagaaaacca taaaatcaat ggtaggaaaa gttggaactg gttacctgga    300
   tgccatggtt ctctgttaaa taaagtaaga gaccaggtgn annnnnaann nnnannanng    360
   ntattttcag catgctaata aatgtctttc cggttatata tctatctaaa attaaccttt    420
   aaaatattgg tttccttgat aaaagcacca ctttggcttt tgttagctgt aatattttgt    480
   catttagatt aagacctggt taggctctca ataaaagatg aagacagtag ctctgtacag    540
   cggataatat c                                                         551
<210> 551
   <211> 537
   <212> DNA
   <213> human
<220>
   <221> misc_feature
   <222> (109)..(109)
   <223> n is a, c, g, or t
<400> 551
   gctagttgca ttatactggg tcatgaaaaa ttatcccttg aaatagatat gaaacatgtt     60
   acttcatttc tggtttaaat aacttgtgga atctttccta atgacaacnt gatattaagg    120
   gaaactaaag aaaatgttat tgtggatccc acagtactat attacactgt ttttttttgt    180
   ttgttttgtt agtttttttt tatttaaagc aaacctcaaa cattattggg tatcaattac    240
   cacctggttg tattaaaata gtaacttatc aatgccatgt aaaaattaat tccattttcg    300
   aagccacctg gcagacaggt ttagctgttt catcagcagc ctaatatata ctgttaaatt    360
   tgttaaggat ttcactttga aggatacatg caaaacatat agttactatt ttcatgagtc    420
   ctgcttctag ctccattgtg gaatacagaa aattaaatat acctgttaag ttcgtatcta    480
   aacctaagac attaccaagg tttgtacaaa ttctactacc tgacatttat tccaaga       537
<210> 552
   <211> 532
   <212> DNA
   <213> human
<220>
   <221> misc_feature
   <222> (69)..(69)
   <223> n is a, c, g, or t
<220>
   <221> misc_feature
   <222> (131)..(131)
   <223> n is a, c, g, or t
<220>
   <221> misc_feature
   <222> (283)..(283)
   <223> n is a, c, g, or t
<220>
   <221> misc_feature
   <222> (412)..(412)
   <223> n is a, c, g, or t
<400> 552
   ctcccctgaa gaacgtgtcc agcaacgcag agtgccctgc ttgttatgaa tctaatggaa     60
   cttcctgtng tgggaagccc tggaaatgct atgaagaaga acagtgtgtc tttctagttg    120
   cagaacttaa naatgacatt gagtctaaga gtctcgtgct gaaaggctgt tccaacgtca    180
   gtaacgccac ctgtcagttc ctgtctggtg aaaacaagac tcttggagga gtcatctttc    240
   gaaagtttga gtgtgcaaat gtaaacagct taacccccac gtntgcacca accacttccc    300
   acaacgtggg ctccaaagct tccctctacc tcttggccct tgccagcctc cttcttcggg    360
   gactgctgcc ctgaggtcct ggggctgcac tttgcccagc accccatttc tncttctctg    420
   aggtccagag caccccctgc ggtgctgaca ccctctttcc ctgctctgcc ccgtttaact    480
   gcccagtaag tgggagtcac aggtctccag gcaatgccga cagctgcctt gt            532
<210> 553
   <211> 521
   <212> DNA
   <213> human
<220>
   <221> misc_feature
   <222> (37)..(37)
   <223> n is a, c, g, or t
<400> 553
   tttgtccata gaggcttcaa gatagatagg taagagncca gtagtgttca taagaagcca     60
   atagagagca ggagccactt tatcaggtgg caggtgtcct gggcctccct gctggctagt    120
   cccaagcggt ggtgttgcca ggatgtcttg gaggtgataa tgggacacac agaggcactg    180
   agtctccata ggttaaaatg ccaccaaaac tggcctttgc ctaatatccc tcattgacta    240
   tttagcattt aatttattta ttttcctgac atttctgcaa gctttgtatt tatatttcca    300
   ctttatagat gaggaaattt gaggctctta gaggtaaaat gacttgccca ggtcacacag    360
   gaagtggcag agacaagctt tttaaataag aaaaaattaa taaaatataa tatgagagta    420
   acttaaaata ttaataaacc acaattttaa attaattaac cgtgataacc aacattaata    480
   aaagttaaga taccaaaaca ctggtgtcta attctttcaa c                        521
<210> 554
   <211> 410
   <212> DNA
   <213> human
<220>
   <221> misc_feature
   <222> (45)..(45)
   <223> n is a, c, g, or t
<400> 554
   gttctgttgt ttatccatga agcctatata tttctagtgc aaaangccca gtgggtctca     60
   aaacattctt ggaggggttt gacacactgc tattagtagt gactcataat tccacgattc    120
   cctgggatac agagaaggca ggggggtagt actgtttgaa caaaaatctt cccaagaccc    180
   tggcaaagag atcttcctta ggaatcagtg gcatatgcgt ttatgttctt taacagggaa    240
   agtaaaagtg agcgcacttt tttgaagctc ataaatactc atgtgactgt aaatttagaa    300
   atgttaatta attgggccta atgtttgatc tggaaaagta cttagcagtc accaggaatt    360
   tgccttgaac tgactcgaac cacaaatcag aaccgtattt ccacaattta               410
<210> 555
   <211> 296
   <212> DNA
   <213> human
<220>
   <221> misc_feature
   <222> (123)..(123)
   <223> n is a, c, g, or t
<400> 555
   gcactgttaa aatccggcta ggccaagatt tcccaatgtc ttgaagccaa tggctaggaa     60
   aacagctgag cctgggactg gactttgtca gtaacctctc ttccctaggg cacgggagga    120
   gtngggtgaa taggacctct tggcttgagg accatgattg tgaatacaga catttaattc    180
   agaatgacac ctacattgat gaattttcat tgtacaagtg tttttccaga tcaaacacaa    240
   aacccatgta attccttgat aaattttcaa tgcagggcag ataaactgtt gctaac        296
<210> 556
   <211> 432
   <212> DNA
   <213> human
<220>
   <221> misc_feature
   <222> (26).. (26)
   <223> n is a, c, g, or t
<220>
   <221> misc_feature
   <222> (332)..(332)
   <223> n is a, c, g, or t
<400> 556
   gtgatcttgg actgtcaatt cccctncctg tgatccattt tactgcaaac ataagggttg     60
   cagtaaaggg ttgtctcacg tcttctgctt taaaagccta taaatatatg acctgaaaac    120
   tccagttaca taaaggatct gcagctatct aaggcttggt tttcttactg tcatatgata    180
   cctgggtcta atgaactctg ctgagatcac ctcaagtttc tgcggttggt aaagagaaca    240
   agggaagaac aaacatccct tttattgctc caaatggtga tttaatccct acatggtgct    300
   gggtggacaa tgtgtcactg tcacatgcct tnactgtata aatccaacct tctgccagag    360
   agaatctgtg gttctggcca tggagggagg atagtggaaa tgatatagtt ggactggtgc    420
   ttgatgtcac ta                                                        432
<210> 557
   <211> 383
   <212> DNA
   <213> human
<220>
   <221> misc_feature
   <222> (42)..(43)
   <223> n is a, c, g, or t
<220>
   <221> misc_feature
   <222> (85)..(85)
   <223> n is a, c, g, or t
<220>
   <221> misc_feature
   <222> (93)..(93)
   <223> n is a, c, g, or t
<220>
   <221> misc_feature
   <222> (99)..(99)
   <223> n is a, c, g, or t
<220>
   <221> misc_feature
   <222> (106)..(106)
   <223> n is a, c, g, or t
<400> 557
   atgtgattat acttctcttt gacttgtcag cttagcttta gnngatacac tctggtgccc     60
   aactattatt gtatcagtga acttnccact ttncttttnc cttttnctct caatttttgt    120
   tgtatcattc ctaccttgtg aggacatata atatttacat tctgttgtca tcctcacatt    180
   tcttagttcc acagtttaaa tgtatttgaa actcaaaaca ttcccattaa tctcttggtc    240
   agctgaaatt aatgatttaa tagtttcctt aaaaaagact catggaacaa tttccctaaa    300
   tttttgccat gtcaaatatg tttatctgta gcctttacac agtaaaaaca atttggctag    360
   ataatacaat tctcagttca tat                                            383
<210> 558
   <211> 391
   <212> DNA
   <213> human
<400> 558
   agggcacttc tcaacattaa ttttggaatt attcaccatt taaaaattgt cactgctctt     60
   tttcattact gtagataact gagagttagc tgttctagga aaaggccatg tttcagttag    120
   tgctatgact tcttttttcg attaaggttg aagcagtgtt ctcttagtag cctgaacact    180
   tctcaaagcg acttatcact agcggcttta atattgagct tctatatcgt tgtgtccacc    240
   tcacttttta aggctgttca tacctggcaa tattcactta ctctgctatt tttaaaaact    300
   attttaaagt tttaagcaca atgttgactc ttctgtaatt tcctaaaatt atattgtttt    360
   cctacagaca gctcaacttt ccttgatatg t                                   391
<210> 559
   <211> 301
   <212> DNA
   <213> human
<400> 559
   ctcattattc ctttacatgc agaatagagg catttatgca aattgaactg cagtttttca     60
   gcatatacac aatgtcttgt gcaacagaaa aacatgttgg ggaaatattc ctcagtggag    120
   agtcgttctc atgctgacgg ggagaacgaa agtgacaggg gtttcctcat aagttttgta    180
   tgaaatatct ctacaaacct caattagttc tactctacac tttcactatc atcaacactg    240
   agactatcct gtctcaccta caaatgtgga aactttacat tgttcgattt ttcagcagac    300
   t                                                                    301
<210> 560
   <211> 463
   <212> DNA
   <213> human
<220>
   <221> misc_feature
   <222> (226)..(226)
   <223> n is a, c, g, or t
<400> 560
   ataaatgacc agactttttc taagaaaaat gttgctttaa tgcatttcat gaatttttac      60
   tcttatatca ttgcttgcta gtaatagcaa atctgctttt ctgcatctgc tttgcgtagc     120
   tattgtaagg ctttgaacta atgtatgtat ttattgcttg aacttctgtg cataccttat     180
   aaagcataat gtctgacaat ttaaatggct catgtattct tgcttntatc ataagctgat     240
   tatggggact atgatctttt gtatacagca aattttaaac tgtagcacaa acatctgttt     300
   atgtattggt ggaatatacc tgttttattt atcttttttg aggtaaacta atttttgata     360
   cttttcatta ctgtgtacta tgttcatact ttgaattctc tgacgttaga agtcatggtt     420
   gagaattgta acagctgtta ttcgttctgt attcatggct ttc                       463
<210> 561
   <211> 541
   <212> DNA
   <213> human
<400> 561
   aaaagcgagg tggccatgtt atgctggtgg ttgccagggc ctccaaccac tgtgccactg      60
   acttgctgtg tgaccctggg caagtcactt aactataagg tgcctcagtt ttccttctgt     120
   taaaatgggg ataataatac tgacctacct caaagggcag ttttgaggca tgactaatgc     180
   tttttagaaa gcattttggg atccttcagc acaggaattc tcaagacctg agtatttttt     240
   ataataggaa tgtccaccat gaacttgata cgtccgtgtg tcccagatgc tgtcattagt     300
   ctatatggtt ctccaagaaa ctgaatgaat ccattggaga agcggtggat aactagccag     360
   acaaaatttg agaatacata aacaacgcat tgccacggaa acatacagag gatgcctttt     420
   ctgtgattgg gtgggatttt ttcccttttt atgtgggata tagtagttac ttgtgacaag     480
   aataattttg gaataatttc tattaatatc aactctgaag ctaattgtac taatctgaga     540
   t                                                                     541
<210> 562
   <211> 419
   <212> DNA
   <213> human
<400> 562
   gagtcggaga tgatgcagca cacacacaat tccccagccc agtgatgctt gtgttgacca      60
   gatgttcctg agtctggagc aagcacccag gccagaataa cagagctttc ttagttggtg     120
   aagacttaaa catctgcctg aggtcaggag gcaatttgcc tgccttgtac aaaagctcag     180
   gtgaaagact gagatgaatg tctttcctct ccctgcctcc caccagactt cctcctggaa     240
   aacgctttgg tagatttggc caggagcttt cttttatgta aattggataa atacacacac     300
   catacactat ccacagatat agccaagtag atttgggtag aggatactat ttccagaata     360
   gtgtttagct cacctagggg gatatgtttg tatacacatt tgcatatacc cacatgggg      419
<210> 563
   <211> 303
   <212> DNA
   <213> human
<400> 563
   ggaatgtcac tggtgcccag gtgtacttga gctgtgaggt catcggaatc ccgacacctg      60
   tcctcatctg gaacaaggta aaaaggggtc actatggagt tcaaaggaca gaactcctgc     120
   ctggtgaccg ggacaacctg gccattcaga cccggggtgg cccagaaaag catgaagtaa     180
   ctggctgggt gctggtatct cctctaagta aggaagatgc tggagaatat gagtgccatg     240
   catccaattc ccaaggacag gcttcagcat cagcaaaaat tacagtggtt gatgccttac     300
   atg                                                                   303
<210> 564
   <211> 192
   <212> DNA
   <213> human
<400> 564
   aggtgaaggt gccgagctat aaacctccag aatattatta gtctgcatgg ttaaaagtag      60
   tcatggataa ctacattacc tgttcttgcc taataagttt cttttaatcc aatccactaa     120
   cactttagtt atattcactg gttttacaca gagaaataca aaataaagat cacacatcaa     180
   gactatctac aa                                                         192
<210> 565
   <211> 449
   <212> DNA
   <213> human
<400> 565
   ctcaaagact ctcccgtgga tgacggatga ggactctggg ctgctggaat aggacactca      60
   agacttttga ctgccatttt gtttgttcag tggagactcc ctggccaaca gaatccttct     120
   tgatagtttg caggcaaaac aaatgtaatg ttgcagatcc gcaggcagaa gctctgccct     180
   tctgtatcct atgtatgcag tgtgcttttt cttgccagct tgggccattc ttgcttagac     240
   agtcagcatt tgtctcctcc tttaactgag tcatcatctt agtccaacta atgcagtcga     300
   tacaatgcgt agatagaaga agccccacgg gagccaggat gggactggtc gtgtttgtgc     360
   ttttctccaa gtcagcaccc aaaggtcaat gcacagagac cccgggtggg tgagcgctgg     420
   cttctcaaac ggccgaagtt gcctctttt                                       449
<210> 566
   <211> 479
   <212> DNA
   <213> human
<400> 566
   tctctctttc tgtgtgtgtg tgtgtgtgtg tgtgtgtgtg tgtgtgtgtc ttgtgcttcc      60
   tcagaccttt ctcgcttctg agcttggtgg cctgttccct ccatctctcc gaacctggct     120
   tcgcctgtcc ctttcactcc acaccctctg gccttctgcc ttgagctggg actgctttct     180
   gtctgtccgg cctgcaccca gcccctgccc acaaaacccc agggacagcg gtctccccag     240
   cctgccctgc tcaggccttg cccccaaacc tgtactgtcc cggaggaggt tgggaggtgg     300
   aggcccagca tcccgcgcag atgacaccat caaccgccag agtcccagac accggttttc      360
   ctagaagccc ctcaccccca ctggcccact ggtggctagg tctcccctta tccttctggt     420
   ccagcgcaag gaggggctgc ttctgaggtc ggtggctgtc tttccattaa agaaacacc      479
<210> 567
   <211> 537
   <212> DNA
   <213> human
<400> 567
   ccaccgctgg ctgggaggag tcggagactg agacctacac agaggtggtg acagagtttg      60
   ggaccgaggt ggagcccgag tttgggacca aggtggagcc cgagtttgag acccagttgg     120
   agcctgagtt cgagacccag ctggaacccg agtttgagga agaggaggag gaggagaaag     180
   aggaggagat agccactggc caggcattcc ccttcacaac agtagagacc tacacagtga     240
   actttgggga cttctgagat cagcgtccta ccaagacccc agcccaactc aagctacagc 300
   agcagcactt cccaagcctg ctgaccacag tcacatcacc catcagcaca tggaaggccc 360
   ctggtatgga cactgaaagg aagggctggt cctgcccctt tgagggggtg caaacatgac 420
   tgggacctaa gagccagagg ctgtgtagag gctcctgctc cacctgccag tctcgtaaga 480
   gatggggttg ctgcagtgtt ggagtagggg cagagggagg gagccaaggt cactcca 537
<210> 568
   <211> 180
   <212> DNA
   <213> human
<400> 568
   ctactacatg attggtgagc agaagttctc cagcctcccc ctgggccggg aggcagtaga 60
   ggctgctgtg aaagaggctg gctacacaat cgaatggttt gaggtgatct cgcaaagtta 120
   ttcttccacc atggccaaca acgaaggact tttctccctg gtggcgagga agctgagcag 180
<210> 569
   <211> 368
   <212> DNA
   <213> human
<400> 569
   tcgtcactga ctactcagac cagaacctgc aggagctgga gaagtggctg aagaaagagc 60
   cagaggcctt tgactggtcc ccagtggtga cctatgtgtg tgatcttgaa gggaacagag 120
   tcaagggtcc agagaaggag gagaagttga gacaggcggt caagcaggtg ctgaagtgtg 180
   atgtgactca gagccagcca ctgggggccg tccccttacc cccggctgac tgcgtgctca 240
   gcacactgtg tctggatgcc gcctgcccag acctccccac ctactgcagg gcgctcagga 300
   acctcggcag cctactgaag ccagggggct tcctggtgat catggatgcg ctcaagagca 360
   gctactac 368
<210> 570
   <211> 473
   <212> DNA
   <213> human
<400> 570
   tggcctacat ggaccagcag actggcaacc tcaagaaggc cctgctcctc cagggctcca 60
   acgagatcga gatccgcgcc gagggcaaca gccgcttcac ctacagcgtc actgtcgatg 120
   gctgcacgag tcacaccgga gcctggggca agacagtgat tgaatacaaa accaccaaga 180
   cctcccgcct gcccatcatc gatgtggccc ccttggacgt tggtgcccca gaccaggaat 240
   tcggcttcga cgttggccct gtctgcttcc tgtaaactcc ctccatccca acctggctcc 300
   ctcccaccca accaactttc cccccaaccc ggaaacagac aagcaaccca aactgaaccc 360
   cctcaaaagc caaaaaatgg gagacaattt cacatggact ttggaaaata tttttttcct 420
   ttgcattcat ctctcaaact tagtttttat ctttgaccaa ccgaacatga cca 473
<210> 571
   <211> 472
   <212> DNA
   <213> human
<220>
   <221> misc_feature
   <222> (149)..(149)
   <223> n is a, c, g, or t
<220>
   <221> misc_feature
   <222> (153)..(153)
   <223> n is a, c, g, or t
<220>
   <221> misc_feature
   <222> (428)..(428)
   <223> n is a, c, g, or t
<400> 571
   gctccccatt tttataccaa aggtgctaca tctatgtgat gggtggggtg gggagggaat     60
   cactggtgct atagaaattg agatgccccc ccaggccagc aaatgttcct ttttgttcaa    120
   agtctatttt tattccttga tattttttnt ttnttttttt ttttttttgt ggatggggac    180
   ttgtgaattt ttctaaaggt gctatttaac atgggaggag agcgtgtgcg gctccagccc    240
   agcccgctgc tcactttcca ccctctctcc acctgcctct ggcttctcag gcctctgctc    300
   tccgacctct ctcctctgaa accctcctcc acagctgcag cccatcctcc cggctccctc    360
   ctagtctgtc ctgcgtcctc tgtccccggg tttcagagac aacttcccaa agcacaaagc    420
   agtttttncc cctaggggtg ggaggaagca aaagactctg tacctatttt gt            472
<210> 572
   <211> 356
   <212> DNA
   <213> human
<400> 572
   actttcccat gagtgtgatc cacattgtta ggtgctgacc tagacagaga tgaactgagg     60
   tccttgtttt gttttgttca taatacaaag gtgctaatta atagtatttc agatacttga    120
   agaatgttga tggtgctaga agaatttgag aagaaatact cctgtattga gttgtatcgt    180
   gtggtgtatt ttttaaaaaa tttgatttag cattcatatt ttccatctta ttcccaatta    240
   aaagtatgca gattatttgc ccaaagttgt cctcttcttc agattcagca tttgttcttt    300
   gccagtctca ttttcatctt cttccatggt tccacagaag ctttgtttct tgggca        356
<210> 573
   <211> 503
   <212> DNA
   <213> human
<400> 573
   gaacaacgcc tgtggcattg ccaacctggc cagcttcccc aagatgtgac tccagccagc     60
   caaatccatc ctgctcttcc atttcttcca cgatggtgca gtgtaacgat gcactttgga    120
   agggagttgg tgtgctattt ttgaagcaga tgtggtgata ctgagattgt ctgttcagtt    180
   tccccatttg tttgtgcttc aaatgatcct tcctactttg cttctctcca cccatgacct    240
   ttttcactgt ggccatcagg actttccctg acagctgtgt actcttaggc taagagatgt    300
   gactacagcc tgcccctgac tgtgttgtcc cagggctgat gctgtacagg tacaggctgg    360
   agattttcac ataggttaga ttctcattca cgggactagt tagctttaag caccctagag    420
   gactagggta atctgacttc tcacttccta agttcccttc tatatcctca aggtagaaat    480
   gtctatgttt tctactccaa ttc                                            503
<210> 574
   <211> 364
   <212> DNA
   <213> human
<400> 574
   gtacccagtt aaattttcat ttcagataaa caacaaataa ttttttagta taagtacatt      60
   attgtttatc tgaaagtttt aattgaacta acaatcctag tttgatactc ccagtcttgt     120
   cattgccagc tgtgttggta gtgctgtgtt gaattacgga ataatgagtt agaactatta     180
   aaacagccaa aactccacag tcaatattag taatttcttg ctggttgaaa cttgtttatt     240
   atgtacaaat agattcttat aatattattt aaatgactgc atttttaaat acaaggcttt     300
   atatttttaa ctttaagatg tttttatgtg ctctccaaat tttttttact gtttctgatt     360
   gtat                                                                  364
<210> 575
   <211> 557
   <212> DNA
   <213> human
<400> 575
   atccgaattc tccatatatt cactaatcaa agacactatt ttcatactag attcctgaga      60
   caaatactca ctgaagggct tgtttaaaaa taaattgtgt tttggtctgt tcttgtagat     120
   aatgcccttc tattttaggt agaagctctg gaatcccttt attgtgctgt tgctcttatc     180
   tgcaaggtgg caagcagttc ttttcagcag attttgccca ctattcctct gagctgaagt     240
   tctttgcata gatttggctt aagcttgaat tagatccctg caaaggcttg ctctgtgatg     300
   tcagatgtaa ttgtaaatgt cagtaatcac ttcatgaatg ctaaatgaga atgtaagtat     360
   ttttaaatgt gtgtatttca aatttgtttg actaattctg gaattacaag atttctatgc     420
   aggatttacc ttcatcctgt gcatgtttcc caaactgtga ggagggaagg ctcagagatc     480
   gagcttctcc tctgagttct aacaaaatgg tgctttgagg gtcagccttt aggaaggtgc     540
   agctttgttg tcctttg                                                    557
<210> 576
   <211> 168
   <212> DNA
   <213> human
<400> 576
   ggcatcatgt tgaccgagct ggagaaagcc ttgaactcta tcatcgacgt ctaccacaag      60
   tactccctga taaaggggaa tttccatgcc gtctacaggg atgacctgaa gaaattgcta     120
   gagaccgagt gtcctcagta tatcaggaaa aagggtgcag acgtctgg                  168
<210> 577
   <211> 557
   <212> DNA
   <213> human
<400> 577
   gccagtcttg accgggatga ggcccacaga caggttgtca tcagcttgtc ccattcaagc      60
   caccgagctc accacagaca cagtggagcc gcgctcttct ccagtgacac gtggacaaat     120
   gcgggctcat cagccccccc agagagggtc aggccgaacc ccatttctcc tcctcttagg     180
   tcattttcag caaacttgaa tatctagacc tctcttccaa tgaaaccctc cagtctatta     240
   tagtcacata gataatggtg ccacgtgttt tctgatttgg tgagctcaga cttggtgctt     300
   ccctctccac aacccccacc ccttgttttt caagatacta ttattatatt ttcacagact     360
   tttgaagcac aaatttattg gcatttaata ttggacatct gggcccttgg aagtacaaat     420
   ctaaggaaaa accaacccac tgtgtaagtg actcatcttc ctgttgttcc aattctgtgg     480
   gtttttgatt caacggtgct ataaccaggg tcctgggtga cagggcgctc actgagcacc     540
   atgtgtcatc acagaca                                                    557
<210> 578
   <211> 546
   <212> DNA
   <213> human
<400> 578
   caggaaatag tcactcatcc cactccacat aaggggttta gtaagagaag tctgtctgtc      60
   tgatgatgga tagggggcaa atctttttcc cctttctgtt aatagtcatc acatttctat     120
   gccaaacagg aacgatccat aactttagtc ttaatgtaca cattgcattt tgataaaatt     180
   aattttgttg tttcctttga ggttgatcgt tgtgttgttt tgctgcactt tttacttttt     240
   tgcgtgtgga gctgtattcc cgagacaacg aagcgttggg atacttcatt aaatgtagcg     300
   actgtcaaca gcgtgcaggt tttctgtttc tgtgttgtgg ggtcaaccgt acaatggtgt     360
   gggaatgacg atgatgtgaa tatttagaat gtaccatatt ttttgtaaat tatttatgtt     420
   tttctaaaca aatttatcgt ataggttgat gaaacgtcat gtgttttgcc aaagactgta     480
   aatatttatt tatgtgttca catggtcaaa atttcaccac tgaaaccctg cacttagcta     540
   gaacct                                                                546
<210> 579
   <211> 361
   <212> DNA
   <213> human
<220>
   <221> misc_feature
   <222> (75)..(75)
   <223> n is a, c, g, or t
<400> 579
   cccacctgga gctgaatcac atgacctagc tgcaccccag cgcctgggcc cgccccacgc      60
   tctgtccaca cccangcgcc ccgggagcgg ggccatgcct ccagcccccc agctcgcccg     120
   acccatcctg ttcgtgaata ggtctcaggg gttgggggag ggactgccag atttggacac     180
   tatatttttt tctaaattca acttgaagat gtgtatttcc cctgaccttc aaaaaatgtt     240
   ccaaggtaag cctcgtaaag gtcatcccac catcaccaaa gcctccgttt ttaacaacct     300
   ccaacacgat ccatttagag gccaaatgtc attctgcagg tgccttcccg atggattaaa     360
   g                                                                     361
<210> 580
   <211> 549
   <212> DNA
   <213> human
<400> 580
   ctggaggaca gcacctgtga cttcggcaac ctcaagcgct atgcatgcac ctctcatacc      60
   cagggcctga gcccaggctg ctatgacacc tacaatgcgg acatcgactg ccagtggatc     120
   gacataaccg acgtgcagcc tgggaactac atcctcaagg tgcacgtgaa cccaaagtat     180
   attgttttgg agtctgactt caccaacaac gtggtgagat gcaacattca ctacacaggt     240
   cgctacgttt ctgcaacaaa ctgcaaaatt gtccaatcct gatctccggg agggacagat     300
   ggccaatctc tccccttcca aagcaggccc tgctccccgg gcagcctccc gccgaggggc     360
   ccagccccca acccacaggc agggaggggc atccctccct gccggcctca gggagcgaac     420
   gtggatgaaa accacaggga ttccggatgc cagaccccat tttatacttc acttttctct     480
   acagtgttgt tttgttgttg ttggttttta ttttttatac tttggccata ccacagagct     540
   agattgccc                                                             549
<210> 581
   <211> 539
   <212> DNA
   <213> human
<400> 581
   tgccagcgac tgtctcagac tgggcaggga ggctttggca tgacttaaga ggaagggcag 60
   tcttgggacc cgctatgcag gtcctggcaa acctggctgc cctgtctcat ccctgtccct 120
   cagggtagca ccatggcagg actgggggaa ctggagtgtc cttgctgtat ccctgttgtg 180
   aggttccttc caggggctgg cactgaagca agggtgctgg ggccccatgg ccttcagccc 240
   tggctgagca actgggctgt agggcagggc cacttcctga ggtcaggtct tggtaggtgc 300
   ctgcatctgt ctgccttctg gctgacaatc ctggaaatct gttctccaga atccaggcca 360
   aaaagttcac agtcaaatgg ggaggggtat tcttcatgca ggagacccca ggccctggag 420
   gctgcaacat acctcaatcc tgtcccaggc cggatcctcc tgaagccctt ttcgcagcac 480
   tgctatcctc caaagccatt gtaaatgtgt gtacagtgtg tataaacctt cttcttctt 539
<210> 582
   <211> 512
   <212> DNA
   <213> human
<400> 582
   gagtacaggt gccagacaaa cctctccacc ctcagtgacc cggtgcagct agaagtccat 60
   atcggctggc tgttgctcca ggcccctcgg tgggtgttca aggaggaaga ccctattcac 120
   ctgaggtgtc acagctggaa gaacactgct ctgcataagg tcacatattt acagaatggc 180
   aaagacagga agtattttca tcataattct gacttccaca ttccaaaagc cacactcaaa 240
   gatagcggct cctacttctg cagggggctt gttgggagta aaaatgtgtc ttcagagact 300
   gtgaacatca ccatcactca aggtttggca gtgtcaacca tctcatcatt ctctccacct 360
   gggtaccaag tctctttctg cttggtgatg gtactccttt ttgcagtgga cacaggacta 420
   tatttctctg tgaagacaaa catttgaagc tcaacaagag actggaagga ccataaactt 480
   aaatggagaa aggaccctca agacaaatga cc 512
<210> 583
   <211> 291
   <212> DNA
   <213> human
<400> 583
   aaccagccag tcccaagaag aacattaaaa ctaggagtgc ccagaagaga acaaacccga 60
   aaagagtgtg agctaactag tttccaaagc ggagacttcc gacttcctta caggatgagg 120
   ctgggcattg cctgggacag cctatgtaag gccatgtgcc ccttgcccta acaactcact 180
   gcagtgctct tcatagacac atcttgcagc atttttctta aggctatgct tcagtttttc 240
   tttgtaagcc atcacaagcc atagtggtag gtttgccctt tggtacagaa g 291
<210> 584
   <211> 507
   <212> DNA
   <213> human
<400> 584
   gaaaatgtac cttggtgcca ccaacccatt ttgtgccaca tgcaagtttt gaataaggat 60
   gtatggaaaa caacgctgca tatacaggta ccatttagga aataccgatg cctttgtggg 120
   ggcagaatca cagacaaaag ctttgaaaat cataaagata taagttggtg tggctaagat 180
   ggaaacaggg ctgattcttg attcccaatt ctcaactctc cttttcctat ttgaatttct 240
   ttggtgctgt agaaaacaaa aaaagaaaaa tatatattca taaaaaatat ggtgctcatt 300
   ctcatccatc caggatgtac taaaacagtg tgtttaataa attgtaatta ttttgtgtac 360
   agttctatac tgttatctgt gtccatttcc aaaacttgca cgtgtccctg aattccgctg 420
   actctaattt atgaggatgc cgaactctga tggcaataat atatgtatta tgaaaatgaa 480
   gttatgattt ccgatgaccc taagtcc 507
<210> 585
   <211> 386
   <212> DNA
   <213> human
<400> 585
   gaagaactgt ctattttctc agtcattttt aacctctaga gtcactgata cacagaatat     60
   aatcttattt atacctcagt ttgcatattt ttttactatt tagaatgtag ccctttttgt    120
   actgatataa tttagttcca caaatggtgg gtacaaaaag tcaagtttgt ggcttatgga    180
   ttcatatagg ccagagttgc aaagatcttt tccagagtat gcaactctga cgttgatccc    240
   agagagcagc ttcagtgaca aacatatcct ttcaagacag aaagagacag gagacatgag    300
   tctttgccgg aggaaaagca gctcaagaac acatgtgcag tcactggtgt caccctggat    360
   aggcaaggga taactcttct aacaca                                          386
<210> 586
   <211> 541
   <212> DNA
   <213> human
<400> 586
   tgccgtgctc ccaaaacatt ttaaatgaaa gtattggcat tcaaaaagac agcagacaaa     60
   atgaaagaaa atgagagcag aaagtaagca tttccagcct atctaatttc tttagttttc    120
   tatttgcctc cagtgcagtc catttcctaa tgtataccag cctactgtac tatttaaaat    180
   gctcaatttc agcaccgatg gccatgtaaa taagatgatt taatgttgat tttaatcctg    240
   tatataaaat aaaaagtcac aatgagtttg ggcatattta atgatgatta tggagcctta    300
   gaggtcttta atcattggtt cggctgcttt tatgtagttt aggctggaaa tggtttcact    360
   tgctctttga ctgtcagcaa gactgaagat ggcttttcct ggacagctag aaaacacaaa    420
   atcttgtagg tcattgcacc tatctcagcc ataggtgcag tttgcttcta catgatgcta    480
   aaggctgcga atgggatcct gatggaacta aggactccaa tgtcgaactc ttctttgctg    540
   c                                                                    541
<210> 587
   <211> 524
   <212> DNA
   <213> human
<400> 587
   ggggaactga gggcaagggg atatagtgat ggggctcaga tggactggga ggagggggag     60
   ggtgatgcat taattaatgg cttcgttaat taatgtcatg ttgcttgtcg ctttctcagt    120
   gtgtgtgtgt ggtccatgcc cactgctggt gccagggtgg gtgtccatgt gcacccggcc    180
   tggatgccag ctgtgtcctt cgggggcgtg cgtgtaactg tagtgtagtc aggtgctcaa    240
   tggagaatat aaacatatac agaaaaatat atattttaag tttaaaaaac agaaaaacag    300
   acaaaacaat ccccatcagg tagctgtcta acccccagct gggtctaatc cttctcatta    360
   cccacccgac ctggctgccc ctcaccttgg gctgggggac tgggggccca tttccttttc    420
   tctgcccttt ttttgttgtt ctattttgta cagacaagtt ggaaaaacaa cagcgacaaa    480
   aaagtcaaga aactttgtaa aatatcgtgt gtgtgattcc ttgt                      524
<210> 588
   <211> 502
   <212> DNA
   <213> human
<400> 588
   cccgaccggt gggcatttgt gaggcccatg gttgagaaat gaataatttc ccaattagga     60
   agtgtaagca gctgaggtct cttgagggag cttagccaat gtgggagcag cggtttgggg    120
   agcagagaca ctaacgactt cagggcaggg ctctgatatt ccatgaatgt atcaggaaat    180
   atatatgtgt gtgtatgttt gcacacttgt tgtgtgggct gtgagtgtaa gtgtgagtaa    240
   gagctggtgt ctgattgtta agtctaaata tttccttaaa ctgtgtggac tgtgatgcca    300
   cacagagtgg tctttctgga gaggttatag gtcactcctg gggcctcttg ggtcccccac     360
   gtgacagtgc ctgggaatgt acttattctg cagcatgacc tgtgaccagc actgtctcag    420
   tttcactttc acatagatgt ccctttcttg gccagttatc ccttcctttt agcctagttc    480
   atccaatcct cactgggtgg gg 502
<210> 589
   <211> 555
   <212> DNA
   <213> human
<400> 589
   caccacctac ctatgatgcc gtggtacaga tggagtacct tgacatggtg gtgaatgaaa 60
   cactcagatt attcccagtt gctattagac ttgagaggac ttgcaagaaa gatgttgaaa 120
   tcaatggggt attcattccc aaagggtcaa tggtggtgat tccaacttat gctcttcacc 180
   atgacccaaa gtactggaca gagcctgagg agttccgccc tgaaaggttc agtaagaaga 240
   aggacagcat agatccttac atatacacac cctttggaac tggacccaga aactgcattg 300
   gcatgaggtt tgctctcatg aacatgaaac ttgctctaat cagagtcctt cagaacttct 360
   ccttcaaacc ttgtaaagaa acacagatcc ccttgaaatt agacacgcaa ggacttcttc 420
   aaccagaaaa acccattgtt ctaaaggtgg attcaagaga tggaacccta agtggagaat 480
   gagttattct aaggacttct actttggtct tcaagaaagc tgtgccccag aacaccagag 540
   atttcaactt agtca 555
<210> 590
   <211> 540
   <212> DNA
   <213> human
<400> 590
   gaaaatcgat gcagccattt ctgataagga aaagaacaaa acatatttct ttgtagagga 60
   caaatactgg agatttgatg agaagagaaa ttccatggag ccaggctttc ccaagcaaat 120
   agctgaagac tttccaggga ttgactcaaa gattgatgct gtttttgaag aatttgggtt 180
   cttttatttc tttactggat cttcacagtt ggagtttgac ccaaatgcaa agaaagtgac 240
   acacactttg aagagtaaca gctggcttaa ttgttgaaag agatatgtag aaggcacaat 300
   atgggcactt taaatgaagc taataattct tcacctaagt ctctgtgaat tgaaatgttc 360
   gttttctcct gcctgtgctg tgactcgagt cacactcaag ggaacttgag cgtgaatctg 420
   tatcttgccg gtcattttta tgttattaca gggcattcaa atgggctgct gcttagcttg 480
   caccttgtca catagagtga tctttcccaa gagaagggga agcactcgtg tgcaacagac 540
<210> 591
   <211> 511
   <212> DNA
   <213> human
<400> 591
   tccacacacg gccaggcctg tttatctaca ctgctgccca ctcctctctc cagctccaca 60
   tgctgtacct ggatcattct gaagcaaatt ccgagcatta catcattttg tccataaata 120
   tttctaacat ccttaaatat acaatcggaa ttcaagcatc tcccattgtc ccacaaatgt 180
   ttggctgttt ttgtagttgg attgtttgta ttaggattca agcaaggccc atatattgca 240
   tttatttgaa atgtctgtaa gtctctttcc atctacagag tttagcacat ttgaacgttg 300
   ctggttgaaa tcccgaggtg tcatttgaca tggttctctg aacttatctt tcctataaaa 360
   tggtagttag atctggaggt ctgattttgt ggcaaaaata cttcctaggt ggtgctgggt 420
   acttcttgtt gcatcctgtc aggaggcaga taatgctggt gcctctctat tggtaatgtt 480
   aagactgctg ggtgggtttg gagttcttgg c 511
<210> 592
   <211> 428
   <212> DNA
   <213> human
<400> 592
   gaacatccaa gtctttcttc ttttttaagt tgtcaaagaa gcttccacaa aattagaaag    60
   gacaacagtt ctgagctgta atttcgcctt aaactctgga cactctatat gtagtgcatt   120
   tttaaacttg aaatatataa tattcagcca gcttaaaccc atacaatgta tgtacaatac   180
   aatgtacaat tatgtctctt gagcatcaat cttgttactg ctgattcttg taaatctttt   240
   tgcttctact ttcatcttaa actaatacgt gccagatata actgtcttgt ttcagtgaga   300
   gacgccctat ttctatgtca tttttaatgt atctatttgt acaattttaa agttcttatt   360
   ttagtataca tataaatatc agtattctga catgtaagaa aatgttacgg catcacactt   420
   atatttta                                                            428
<210> 593
   <211> 541
   <212> DNA
   <213> human
<400> 593
   aatctcttct agagcacctg ctatccccaa cttctagacc tgctccaaac tagtgactag    60
   gatagaattt gatcccctaa ctcactgtct gcggtgctca ttgctgctaa cagcattgcc   120
   tgtgctctcc tctcaggggc agcatgctaa cggggcgacg tcctaatcca actgggagaa   180
   gcctcagtgg tggaattcca ggcactgtga ctgtcaagct ggcaagggcc aggattgggg   240
   gaatggagct ggggcttagc tgggaggtgg tctgaagcag acagggaatg ggagaggagg   300
   atgggaagta gacagtggct ggtatggctc tgaggctccc tggggcctgc tcaagctcct   360
   cctgctcctt gctgttttct gatgatttgg gggcttggga gtccctttgt cctcatctga   420
   gactgaaatg tggggatcca ggatggcttc cttcctctta cccttcctcc ctcagcctgc   480
   aacctctatc ctggaacctg tcctcccttt ctccccaact atgcatctgt tgtctgctcc   540
   t                                                                   541
<210> 594
   <211> 502
   <212> DNA
   <213> human
<400> 594
   aagaagctaa gcctgatctc ctgtaaaaac atcatttccc tgatggacac cagcggcaat    60
   gggaagctgg agtttgatga attcaaagtg ttctgggaca agctgaagca gtggattaac   120
   cttttccttc ggtttgatgc tgacaagtcc ggcaccatgt ctacctatga actacggact   180
   gcactgaaag ctgcaggctt tcagctgagc agccacctcc tgcagctgat tgtgctcagg   240
   tatgcggatg aggagctcca gctggacttc gatgacttcc tcaactgcct ggtccggctg   300
   gagaatgcga gccgggtgtt ccaggctctc agtacaaaga acaaggagtt cattcatctc   360
   aatataaatg agttcatcca tttgacaatg aacatctgag gctgccttgt agagatgcag   420
   cctgcccagc tgaatcttgg cttctggacc ttgaccttca gaacttctct tggtgtggaa   480
   ccattacgcc cagggttcac tc                                            502
<210> 595
   <211> 542
   <212> DNA
   <213> human
<400> 595
   actcccaaga gcaaatccac attcctcttg agttctgcag cttctgtgta aatagggcag    60
   ctgtcgtcta tgccgtagaa tcacatgatc tgaggaccat tcatggaagc tgctaaatag   120
   cctagtctgg ggagtcttcc ataaagtttt gcatggagca aacaaacagg attaaactag   180
   gtttggttcc ttcagccctc taaaagcata gggcttagcc tgcaggcttc cttgggcttt   240
   ctctgtgtgt gtagttttgt aaacactata gcatctgtta agatccagtg tccatggaaa   300
   cattcccaca tgccgtgact ctggactata tcagtttttg gaaagcaggg ttcctctgcc   360
   tgctaacaag cccacgtgga ccagtctgaa tgtctttcct ttacacctat gtttttaagt   420
   agtcaaactt caagaaacaa tctaaacaag tttctgttgc atatgtgttt gtgaacttgt   480
   atttgtattt agtaggcttc tatattgcat ttaacttgtt tttgtaactc ctgattcttc   540
   ct                                                                  542
<210> 596
   <211> 473
   <212> DNA
   <213> human
<400> 596
   gaagcctcaa gttccagtgc agagatccta cttctctgag tcagctgacc ccctccccgc     60
   aatccctcaa accttgagga gaagtgggga ccccacccct catcaggagt tccagtgctg    120
   catgcgatta tctacccacg tccacgcggc cacctcaccc tctccgcaca cctctggctg    180
   tctttttgta ctttttgttc cagagctgct tctgtctggt ttatttaggt tttatccttc    240
   cttttctttg agagttcgtg aagagggaag ccaggattgg ggacctgatg gagagtgaga    300
   gcatgtgagg ggtagtggga tggtggggta ccagccactg gaggggtcat ccttgcccat    360
   cgggaccaga aacctgggag agacttggat gaggagtggt tgggctgtgc ctgggcctag    420
   cacggacatg gtctgtcctg acagcactcc tcggcaggca tggctggtgc ctg           473
<210> 597
   <211> 477
   <212> DNA
   <213> human
<400> 597
   aaagcagcgc tagtggaggg ttggagaagg aggtaaggat gagggttcat catccctccc     60
   tgcctaagga agctaaaagc atggccctgc tgcccctccc tgcctccacc cacagtggag    120
   agggctacaa aggaggacaa gaccctctca ggctgtccca agctcccaag agcttccaga    180
   gctctgaccc acagcctcca agtcaggtgg ggtggagtcc cagagctgca cagggtttgg    240
   cccaagtttc taagggaggc acttcctccc ctcgcccatc agtgccagcc cctgctggct    300
   ggtgcctgag cccctcagac agccccctgc cccgcaggcc tgccttctca gggacttctg    360
   cggggcctga ggcaagccat ggagtgagac ccaggagccg gacacttctc aggaaatggc    420
   ttttcccaac ccccagcccc cacccggtgg ttcttcctgt tctgtgactg tgtatag        477
<210> 598
   <211> 432
   <212> DNA
   <213> human
<400> 598
   ttcagccagt cgccaagaat catgaaagtc gccagtggca gcaccgccac cgccgccgcg     60
   ggccccacgt gcgcgctgaa ggccggcaag acagcgagcg gtgcgggcga ggtggtgcgc    120
   tgtctgtctg agcagagcgt ggccatctcg cgctgccggg gcgccggggc gcgcctgcct    180
   gccctgctgg acgagcagca ggtaaacgtg ctgctctacg acatgaacgg ctgttactca    240
   cgcctcaagg agctggtgcc caccctgccc cagaaccgca aggtgagcaa ggtggagatt    300
   ctccagcacg tcatcgacta catcagggac cttcagttgg agctgaactc ggaatccgaa    360
   gttggaaccc ccgggggccg agggctgccg gtccgggctc cgctcagcac cctcaacggc    420
   gagatcagcg cc                                                        432
<210> 599
   <211> 382
   <212> DNA
   <213> human
<400> 599
   gattccctgc atcaactaag aaaagcctgt tttctttatt tcaaacttgg tggcgaatgt     60
   gttgcgggtc ctgttgggct gctttctgta ttgtctccta accctctagt tttaattgga    120
   cacttctttg ctgttgcaat ctatgccgtg tatttttgct ttaagtcaga accttggatt    180
   acaaaacctc gagcccttct cagtagtggt gctgtattgt acaaagcgtg ttctgtaata    240
   tttcctctaa tttactcaga aatgaagtat atggttcatt aagcttaaag gggaaccatt    300
   tgtgaatgaa tatttggaac ttaccaagtc ctaagagact tttggaagag gatatatata    360
   gcatagtacc ataccactta ta                                             382
<210> 600
   <211> 269
   <212> DNA
   <213> human
<400> 600
   gctgtgggga tagtgaggca tcgcaatgta agactcggga ttagtacaca cttgttgatg     60
   attaatggaa atgtttacag atccccaagc ctggcaaggg aatttcttca actccctgcc    120
   ccctagccct ccttatcaaa ggacaccatt ttggcaagct ctatcaccaa ggagccaaac    180
   atcctacaag acacagtgac catactaatt ataccccctg caaagccagc ttgaaacctt   240
   cacttaggaa cgtaatcgtg tcccctatc                                     269
<210> 601
   <211> 473
   <212> DNA
   <213> human
<400> 601
   tcaccaatgc catcaaggat gcactcgctg caacgtagcc ctctgttctg cacacagcac     60
   gggggccaag gatgccccgt ccccctctgg ctccagctgg ccgggagcct gatcacctgc    120
   cctgctgagt cccaggctga gcctcagtct ccctcccttg gggcctatgc agaggtccac     180
   aacacacaga tttgagctca gccctggtgg gcagagaggt agggatgggg ctgtggggat    240
   agtgaggcat cgcaatgtaa gactcgggat tagtacacac ttgttgatga ttaatggaaa    300
   tgtttacaga tccccaagcc tggcaaggga atttcttcaa ctccctgccc cctagccctc    360
   cttatcaaag gacaccattt tggcaagctc tatcaccaag gagccaaaca tcctacaaga    420
   cacagtgacc atactaatta taccccctgc aaagccagct tgaaaccttc act          473
<210> 602
   <211> 329
   <212> DNA
   <213> human
<400> 602
   tccaacctcc ttcaaattca gtcaccactg ttatattacc ttctccagga accctccagt     60
   ggggaaggct gcgatattag atttccttgt atgcaaagtt tttgttgaaa gctgtgctca    120
   gaggaggtga gaggagagga aggagaaaac tgcatcataa ctttacagaa ttgaatctag    180
   agtcttcccc gaaaagccca gaaacttctc tgcagtatct ggcttgtcca tctggtctaa    240
   ggtggctgct tcttccccag ccatgagtca gtttgtgccc atgaataata cacgacctgt    300
   tatttccatg actgctttac tgtattttt                                      329
<210> 603
   <211> 529
   <212> DNA
   <213> human
<400> 603
   gaatggtgca tacaaggcca tccccgttgc ccaggacctg aacgcgcctt ctgattggga     60
   cagccgtggg aaggacagtt atgaaacgag tcagctggat gaccagagtg ctgaaaccca    120
   cagccacaag cagtccagat tatataagcg gaaagctaat gatgagagca atgagcattc    180
   cgatgtgatt gatagtcagg aactttccaa agtcagccgt gaattccaca gccatgaatt    240
   tcacagccat gaagatatgc tggttgtaga ccccaaaagt aaggaagaag ataaacacct    300
   gaaatttcgt atttctcatg aattagatag tgcatcttct gaggtcaatt aaaaggagaa    360
   aaaatacaat ttctcacttt gcatttagtc aaaagaaaaa atgctttata gcaaaatgaa    420
   agagaacatg aaatgcttct ttctcagttt attggttgaa tgtgtatcta tttgagtctg    480
   gaaataactg atgtgtttga taattagttt agtttgtggc ttcatggaa                529
<210> 604
   <211> 536
   <212> DNA
   <213> human
<400> 604
   acagctttcc aaggtgacaa actcctctat gcagtgtatc gaaagctggg tgtttatgaa     60
   gttgaagacc agattacagc tgtcagaaaa ttcatagaaa tgggtttcat tgatgaaaaa    120
   agaatagcca tatggggctg gtcctatgga ggatacgttt catcactggc ccttgcatct    180
   ggaactggtc ttttcaaatg tggtatagca gtggctccag tctccagctg ggaatattac    240
   gcgtctgtct acacagagag attcatgggt ctcccaacaa aggatgataa tcttgagcac    300
   tataagaatt caactgtgat ggcaagagca gaatatttca gaaatgtaga ctatcttctc    360
   atccacggaa cagcagatga taatgtgcac tttcagaact cagcacagat tgctaaagct    420
   ctggttaatg cacaagtgga tttccaggca atgtggtact ctgaccagaa ccacggctta    480
   tccggcctgt ccacgaacca cttatacacc cacatgaccc acttcctaaa gcagtg        536
<210> 605
   <211> 470
   <212> DNA
   <213> human
<400> 605
   tcttttgtcc tccttagcac aatgtaaaaa agaatagtaa tatcagaaca ggaaggagga     60
   atggcttgct ggggagccca tccaggacac tgggagcaca tagagattca cccatgtttg    120
   ttgaacttag agtcattctc atgcttttct ttataattca cacatatatg cagagaagat    180
   atgttcttgt taacattgta tacaacatag ccccaaatat agtaagatct atactagata    240
   atcctagatg aaatgttaga gatgctatat gatacaactg tggccatgac tgaggaaagg    300
   agctcacgcc cagagactgg gctgctctcc cggaggccaa acccaagaag gtctggcaaa    360
   gtcaggctca gggagactct gccctgctgc agacctcggt gtggacacac gctgcataga    420
   gctctccttg aaaacagagg ggtctcaaga cattctgcct acctattagc              470
<210> 606
   <211> 441
   <212> DNA
   <213> human
<400> 606
   aaaggagcag tcgcacagac ctttcctcat gctgcaggcc cggcagtctg aagaccaccc     60
   tcatcgccgg cgtcggcggg gcttggagtg tgatggcaag gtcaacatct gctgtaagaa    120
   acagttcttt gtcagtttca aggacatcgg ctggaatgac tggatcattg ctccctctgg    180
   ctatcatgcc aactactgcg agggtgagtg cccgagccat atagcaggca cgtccgggtc    240
   ctcactgtcc ttccactcaa cagtcatcaa ccactaccgc atgcggggcc atagcccctt    300
   tgccaacctc aaatcgtgct gtgtgcccac caagctgaga cccatgtcca tgttgtacta    360
   tgatgatggt caaaacatca tcaaaaagga cattcagaac atgatcgtgg aggagtgtgg    420
   gtgctcatag agttgcccag c                                              441
<210> 607
   <211> 561
   <212> DNA
   <213> human
<400> 607
   acaagcatcc tgtctcacga agaacaaatg tttgttaatc gtgtgggcca tgattatcag      60
   tggataggcc tcaatgacaa gatgtttgag catgacttcc gttggactga tggcagcaca     120
   ctgcaatacg agaattggag acccaaccag ccagacagct tcttttctgc tggagaagac     180
   tgtgttgtaa tcatttggca tgagaatggc cagtggaatg atgttccctg caattaccat     240
   ctcacctata cgtgcaagaa aggaacagtt gcttgcggcc agccccctgt tgtagaaaat     300
   gccaagacct ttggaaagat gaaacctcgt tatgaaatca actccctgat tagataccac     360
   tgcaaagatg gtttcattca acgtcacctt ccaactatcc ggtgcttagg aaatggaaga     420
   tgggctatac ctaaaattac ctgcatgaac ccatctgcat accaaaggac ttattctatg     480
   aaatacttta aaaattcctc atcagcaaag gacaattcaa taaatacatc caaacatgat     540
   catcgttgga gccggaggtg g                                               561
<210> 608
   <211> 557
   <212> DNA
   <213> human
<400> 608
   tggtgatgtc tgctactatg ccagccggaa cgacaagtcc tactggctct ctaccactgc      60
   gccgctgccc atgatgcccg tggccgagga cgagatcaag ccctacatca gccgctgttc     120
   tgtgtgtgag gccccggcca tcgccatcgc ggtccacagt caggatgtct ccatcccaca     180
   ctgcccagct gggtggcgga gtttgtggat cggatattcc ttcctcatgc acacggcggc     240
   gggagacgaa ggcggtggcc aatcactggt gtcaccgggc agctgtctag aggacttccg     300
   cgccacacca ttcatcgaat gcaatggagg ccgcggcacc tgccactact acgccaacaa     360
   gtacagcttc tggctgacca ccattcccga gcagagcttc cagggctcgc cctccgccga     420
   cacgctcaag gccggcctca tccgcacaca catcagccgc tgccaggtgt gcatgaagaa     480
   cctgtgagcc ggcgcgtgcc aggaagggcc attttggtgc ttattcttaa cttattacct     540
   caggtgccaa cccaaaa                                                    557
<210> 609
   <211> 436
   <212> DNA
   <213> human
<400> 609
   gaaagactgt gctgtccttt aacataggtt tttaaagact aggatattga atgtgaaaca      60
   tccgttttca ttgttcactt ctaaaccaaa aattatgtgt tgccaaaacc aaacccaggt     120
   tcatgaatat ggtgtctatt atagtgaaac atgtactttg agcttattgt ttttattctg     180
   tattaaatat tttcagggtt ttaaacacta atcacaaact gaatgacttg acttcaaaag     240
   caacaacctt aaaggccgtc atttcattag tattcctcat tctgcatcct ggcttgaaaa     300
   acagctctgt tgaatcacag tatcagtatt ttcacacgta agcacattcg ggccatttcc     360
   gtggtttctc atgagctgtg ttcacagacc tcagcagggc atcgcatgga ccgcaggagg     420
   gcagattcgg accact                                                     436
<210> 610
   <211> 429
   <212> DNA
   <213> human
<400> 610
   tcggctactc ttttgtgatg cacaccagcg ctggtgcaga aggctctggc caagccctgg      60
   cgtcccccgg ctcctgcctg gaggagttta gaagtgcgcc attcatcgag tgtcacggcc   120
   gtgggacctg caattactac gcaaacgctt acagcttttg gctcgccacc atagagagga     180
   gcgagatgtt caagaagcct acgccgtcca ccttgaaggc aggggagctg cgcacgcacg     240
   tcagccgctg ccaagtctgt atgagaagaa cataatgaag cctgactcag ctaatgtcac     300
   aacatggtgc tacttcttct tctttttgtt aacagcaacg aaccctagaa atatatcctg    360
   tgtacctcac tgtccaatat gaaaaccgta aagtgcctta taggaatttg cgtaactaac    420
   acaccctgc                                                            429
<210> 611
   <211> 517
   <212> DNA
   <213> human
<220>
   <221> misc_feature
   <222> (181)..(181)
   <223> n is a, c, g, or t
<400> 611
   ggaaaacacc tcatttgacc ttgccagctg accttcaaac cctgcatttg aaccgaccaa     60
   cattaagtcc agagagtaaa cttgaatgga ataacgacat tccagaagtt aatcatttga    120
   attctgaaca ctggagaaaa accgaaaaat ggacggggca tgaagagact aatcatctgg    180
   naaaccgatt tcagtggcga tggcatgaca gagctagagc tcgggcccag ccccaggctg    240
   cagcccattc gcaggcaccc gaaagaactt ccccagtatg gtggtcctgg aaaggacatt    300
   tttgaagatc aactatatct tcctgtgcat tccgatggaa tttcagttca tcagatgttc    360
   accatggcca ccgcagaaca ccgaagtaat tccagcatag cggggaagat gttgaccaag    420
   gtggagaaga atcacgaaaa ggagaagtca cagcacctag aaggcagcgc ctcctcttca    480
   ctctcctctg attagatgaa actgttacct tacccta                             517
<210> 612
   <211> 397
   <212> DNA
   <213> human
<400> 612
   aatatccttg ttgtgtatta ggtttttaaa taccagctaa aggattacct cactgagtca     60
   tcagtaccct cctattcagc tccccaagat gatgtgtttt tgcttaccct aagagaggtt    120
   ttcttcttat ttttagataa ttcaagtgct tagataaatt atgttttctt taagtgttta    180
   tggtaaactc ttttaaagaa aatttaatat gttatagctg aatctttttg gtaactttaa    240
   atctttatca tagactctgt acatatgttc aaattagctg cttgcctgat gtgtgtatca    300
   tcggtgggat gacagaacaa acatatttat gatcatgaat aatgtgcttt gtaaaaagat    360
   ttcaagttat taggaagcat actctgtttt ttaatca                              397
<210> 613
   <211> 518
   <212> DNA
   <213> human
<400> 613
   gtgtgcacac ggagactcat cgttataatt tactatctgc caagagtaga aagaaaggct     60
   ggggatattt gggttggctt ggttttgatt ttttgcttgt ttgtttgttt tgtactaaaa    120
   cagtattatc ttttgaatat cgtagggaca taagtatata catgttatcc aatcaagatg    180
   gctagaatgg tgcctttctg agtgtctaaa acttgacacc cctggtaaat ctttcaacac    240
   acttccactg cctgcgtaat gaagttttga ttcattttta accactggaa tttttcaatg    300
   ccgtcatttt cagttagatg attttgcact ttgagattaa aatgccatgt ctatttgatt    360
   agtcttattt ttttattttt acaggcttat cagtctcact gttggctgtc attgtgacaa    420
   agtcaaataa acccccaagg acgacacaca gtatggatca catattgttt gacattaagc    480
   ttttgccaga aaatgttgca tgtgttttac ctcgactt                             518
<210> 614
   <211> 523
   <212> DNA
   <213> human
<400> 614
   gaaaggcgat ctcttcactg tgaaaagttg cccgggtgca gcgccttttc cttctaccat     60
   gggaaatgca ggctgggccc ttggggtgag cctgcggggc tctggtgctg tccccgaccc    120
   ccaccaccac cagaatgcag ttccagctta ggaagccaca aacaagccac ccaggaggaa    180
   caaaacaccg ccagcgtgga ttttccaaat ttccctggaa agtaagtctc gctcttgcca    240
   aagaaaagtc tggcttggag agtctctgga gcccaggatg ccagcatgtg ccaatgactg    300
   tcaccttcat ctcttcaaaa gaaaagccat agccgaggac tgtcccgcga cccccgtgga    360
   ctgcgtctag gtcatgtgat tctgttttca tttctcatcc catccaattt gtccttttct    420
   cctgtcattt tcttcctctg tggtcccttc aaagttgtta taatttgtac tgaacttcaa    480
   aatgtgtccc gttctcccca gaccactcta gccacagtat att                      523
<210> 615
   <211> 542
   <212> DNA
   <213> human
<220>
   <221> misc_feature
   <222> (56)..(56)
   <223> n is a, c, g, or t
<220>
   <221> misc_feature
   <222> (63)..(63)
   <223> n is a, c, g, or t
<220>
   <221> misc_feature
   <222> (102)..(102)
   <223> n is a, c, g, or t
<400> 615
   attgaaaagc aggtaccagt gccccttttc agacagtttt tgattcgctc tagacntttt     60
   ttntttttta atagggaggg aaaaaatttg ataattttct tntttctaca tgcacttaag    120
   actaaaacac aggtttggat taattttatt tgcttccttt ttccgctttt cttcccgcag    180
   agcctgatgg gagaatgtcc agggcaggga aaccacattt tttgtaggtg ataactcaat    240
   gaaaattggt gcttattttt tacacttctc tcttgtggct ctcttgtggt gctatctgtt    300
   ttaaggtctc cttgaaggcg cactggggtc cctggccatg cctcgttctc cctgctttct    360
   ttatcctgtt attgcctcca cagtctgttg ccaaggactc taagatcaat gcacgtcact    420
   ttcctttcca ctgggcagga tagccaagca cactccctcc tgcgctctcc cgccccggtg    480
   cgtccactcc cgagggctgt tatgaggact gggttgtgcc tacttgattt gaaaacacac    540
   ac                                                                   542
<210> 616
   <211> 559
   <212> DNA
   <213> human
<220>
   <221> misc_feature
   <222> (36)..(36)
   <223> n is a, c, g, or t
<220>
   <221> misc_feature
   <222> (51)..(51)
   <223> n is a, c, g, or t
<220>
   <221> misc_feature
   <222> (56)..(56)
   <223> n is a, c, g, or t
<400> 616
   acttctcagc aaataaatct cccttaagta ggaaanctag atttcatatt ngcttncttt    60
   gaattaacag caactttcca caggtaaatc tgttcttgca aagatgtgag cagaatagtt   120
   aaaaataata tttttatgtt tcatggttct aaatggaagc cataaatgca gtaaatacta   180
   tctgttgttt aactacttta atcgtcattt tttacatttt caagtttatt aggttaagaa   240
   aaacagggca gccttggaag gcagctacta cagaaaactg cagttttgcg ttaaagataa   300
   agtagtattt tcagctccct gaaaaaccat tcctgctgaa actgctgtag aaattgtgaa   360
   gctgcatgag tggagagtat tgaatctgtg gttatagtag ttttctcagg tttgtttatc   420
   ttgatgtttg atgcactgtg ttttatagtt attaaaattg agtaatatta tttctatgca   480
   gtgttatgtg tcattggcct tttgtgaatg tgcatgtttt aaactgcaaa ttttaaacat   540
   tttgtcctct aattgttat                                                559
<210> 617
   <211> 462
   <212> DNA
   <213> human
<400> 617
   gaatgagtat tcctatacga cccagataga ctacaacccc aaggaccgcc tgctctatgc    60
   ctgggacaat ggccaccagg tcacttacca tgtcatcttt gcctactgac acccttgtcc   120
   ccacaagcag aagcacagag gggtcactag caccttgtgt gtatgtgtgt gcgcgcacgt   180
   gtgtgtaggt gggtatgtgt tgtttaaaaa tatatattat tttgtataat attgcaaatg   240
   taaaatgaca atttgggtct atttttttat atggattgta gatcaatcca tacgtgtatg   300
   tgctggtctc atcctcccca gtttatattt ttgtgcaaat gaacttctcc ttttgaccag   360
   taaccacctt ccttcaagcc ttcagcccct ccagctccaa gtctcagatc tcgaccattg   420
   aaaaggtttc ttcatctggg tcttgcagga ggcaggcaac ac                      462
<210> 618
   <211> 503
   <212> DNA
   <213> human
<220>
   <221> misc_feature
   <222> (419)..(419)
   <223> n is a, c, g, or t
<400> 618
   gtgctcggcc tgatggagac tgtgtgcagc cccttcacgg ccgccagacg tctgcgggac    60
   caggaggcag ccgtggcgga gctgcaggcc gccctggagc gacaggctct ccagaagcaa   120
   gccctgcagg agaaaggcaa gcagcaggac acggtcctcg gcggccgggc cctgtccaac   180
   cggcagcacg cctcctagga actgtgggag accagcggag tgggagggag acgcagtaga   240
   cagagacaga ccgagaagga agggagagac agagggggcg cgcgcacagg agcctgactc   300
   cgctgggaga gtgcaggagc acgtgctgtt ttttatttgg acttaacttc agagaaaccg   360
   ctgacatcta gaactgacct accacaagca tccaccaaag gagtttggga ttgagtttnt   420
   gctgctgtgc agcactgcat tgtcatgaca tttccaacac tgtgtgaatt atctaaatgc   480
   gtctaccatt ttgcactagg gag                                           503
<210> 619
   <211> 568
   <212> DNA
   <213> human
<220>
   <221> misc_feature
   <222> (82)..(82)
   <223> n is a, c, g, or t
<220>
   <221> misc_feature
   <222> (251)..(251)
   <223> n is a, c, g, or t
<220>
   <221> misc_feature
   <222> (393)..(393)
   <223> n is a, c, g, or t
<220>
   <221> misc_feature
   <222> (407)..(407)
   <223> n is a, c, g, or t
<220>
   <221> misc_feature
   <222> (412)..(412)
   <223> n is a, c, g, or t
<400> 619
   cacagcctcc aagtcaggtg gggtggagtc ccagagctgc acagggtttg gcccaagttt     60
   ctaagggagg cacttcctcc cntcgcccat cagtgccagc ccctgctggc tggtgcctga    120
   gcccctcaga cagccccctg ccccgcaggc ctgccttctc agggacttct gcggggcctg    180
   aggcaagcca tggagtgaga cccaggagcc ggacacttct caggaaatgg cttttcccaa    240
   cccccagccc nccacccggt ggttcttcct gttctgtgac tgtgtatagt gccaccacag    300
   cttatggcat ctcattgagg acaaagaaaa ctgcacaata aaaccaagcc tctggaatct    360
   gtcctcgtgt ccacctggcc ttcgctcctc cancagtgcc tgcctgnccc cncttcgctg    420
   gggtctccac gggtgaggct ggggaacgcc acctcttcct cttccctgac ttctccccaa    480
   ccacttagta gcaacgctac cccaggggct aatgactgca cactgggctt cttttcagaa    540
   tgaccctaac gagacacatt tgcccaaa                                       568
<210> 620
   <211> 248
   <212> DNA
   <213> human
<220>
   <221> misc_feature
   <222> (45)..(45)
   <223> n is a, c, g, or t
<400> 620
   cacaaaaccc cagggacagc ggtctcccca gcctgccctg ctcangcctt gcccccaaac     60
   ctgtactgtc ccggaggagg ttgggaggtg gaggcccagc atcccgcgca gatgacaccg    120
   gttttcctag aagcccctca cccccactgg cccactggtg gctaggtctc cccttatcct    180
   tctggtccag cgcaaggagg ggctgcttct gaggtcggtg gctgtctttc cattaaagaa    240
   acaccgtg                                                             248
<210> 621
   <211> 261
   <212> DNA
   <213> human
<400> 621
   gaatgaagaa aagtcgcctc aacgacaaac aaaagcaccg actagatttc cttcagctga     60
   tgattgactc ccagaattcg aaagaaactg agtcccacaa agctctgtct gatctggagc    120
   tcgcagccca gtcaataatc ttcatttttg ctggctatga aaccaccagc agtgttcttt    180
   ccttcacttt atatgaactg gccactcacc ctgatgtcca gcagaaactg caaaaggaga    240
   ttgatgcagt tttgcccaat a                                              261
<210> 622
   <211> 245
   <212> DNA
   <213> human
<400> 622
   ggtgagggga tgacccctgg agatgaaggg aagaggtgaa gccttagcaa aaatgcctcc     60
   tcaccactcc ccaggagaat ttttataaaa agcataatca ctgattcctt cactgacata    120
   atgtaggaag cctctgagga gaaaaacaaa gggagaaaca tagagaacgg ttgctactgg    180
   cagaagcata agatctttgt acaatattgc tggccctggt tcacctgttt actgttatca    240
   caata                                                                245
<210> 623
   <211> 273
   <212> DNA
   <213> human
<400> 623
   acttaggtaa ttgtagggcg aggattataa atgaaatttg caaaatcact tagcagcaac     60
   tgaagacaat tatcaaccac gtggagaaaa tcaaaccgag cagggctgtg tgaaacatgg    120
   ttgtaatatg cgactgcgaa cactgaactc tacgccactc cacaaatgat gttttcaggt    180
   gtcatggact gttgccacca tgtattcatc cagagttctt aaagtttaaa gttgcacatg    240
   attgtataag catgctttct ttgagtttta aat                                 273
<210> 624
   <211> 212
   <212> DNA
   <213> human
<220>
   <221> misc_feature
   <222> (43)..(43)
   <223> n is a, c, g, or t
<220>
   <221> misc_feature
   <222> (121)..(121)
   <223> n is a, c, g, or t
<400> 624
   tctcacctat acgtgcaaga aaggaacagt tgcttgcggc cancccctgt tgtagaaaat     60
   gccaagacct ttggaaagat gaaacctcgt tatgaaatca actccctgat tagataccac    120
   ntgcaaagat ggtttcattc aacgtcacct tccaactatc cggtgcttag gaaatggaag    180
   atgggctata cctaaaatta cctgcatgaa cc                                  212
<210> 625
   <211> 157
   <212> DNA
   <213> human
<400> 625
   agggcctaag ggtgacagag gtgatgctgg tcccaaaggt gctgatggct ctcctggcaa     60
   agatggcgtc cgtggtctga ccggccccat tggtcctcct ggccctgctg gtgcccctgg    120
   tgacaagggg gaccccattc ccgaggagct ttatgag                             157
<210> 626
   <211> 481
   <212> DNA
   <213> human
<400> 626
   aacattgtaa tggccatcgc tggaaacaag tgcgacctct cagatattag ggaggttccc     60
   ctgaaggatg ctaaggaata cgctgaatcc ataggtgcca tcgtggttga gacaagtgca    120
   aaaaatgcta ttaatatcga agagctcttt caaggaatca gccgccagat cccacccttg    180
   gacccccatg aaaatggaaa caatggaaca atcaaagttg agaagccaac catgcaagcc    240
   agccgccggt gctgttgacc caagggcgtg gtccacggta cttgaagaag ccagagccca    300
   catcctgtgc actgctgaag gaccctacgc tcggtggcct ggcacctcac tttgagaaga    360
   gtgagcacac tggctttgca tcctggaagg cctgcagggg gcggggcagg aaatgtacct    420
   gaaaaggatt ttagaaaacc ctgggaaacc caccacacca ccacaaaatg gcctttagtg    480
   t                                                                    481
<210> 627
   <211> 399
   <212> DNA
   <213> human
<400> 627
   gccacacctt cgcgaaacct gtggtggccc accagtccta acgggacagg acagagagac     60
   agagcagccc tgcactgttt tccctccacc acagccatcc tgtccctcat tggctctgtg    120
   ctttccacta tacacagtca ccgtcccaat gagaaacaag aaggagcacc ctccacatgg    180
   actcccacct gcaagtggac agcgacattc agtcctgcac tgctcacctg ggtttactga    240
   tgactcctgg ctgccccacc atcctctctg atctgtgaga aacagctaag ctgctgtgac    300
   ttccctttag gacaatgttg tgtaaatctt tgaaggacac accgaagacc tttatactgt    360
   gatcttttac ccctttcact cttggctttc ttatgttgc                            399
<210> 628
   <211> 548
   <212> DNA
   <213> human
<400> 628
   ctgccccgag ctcgaagaag aggctgagtg cgtccctgat aactgcgtct aagaccagag     60
   ccccgcagcc cctggggccc ccggagccat ggggtgtcgg gggctcctgt gcaggctcat    120
   gctgcaggcg gccgaggcac agggggtttc gcgctgctcc tgaccgcggt gaggccgcgc    180
   cgaccatctc tgcactgaag ggccctctgg tggccggcac gggcattggg aaacagcctc    240
   ctcctttccc aaccttgctt cttaggggcc cccgtgtccc gtctgctctc agcctcctcc    300
   tcctgcagga taaagtcatc cccaaggctc cagctactct aaattatggt ctccttataa    360
   gttattgctg ctccaggaga ttgtccttca tcgtccaggg gcctggctcc cacgtggttg    420
   cagatacctc agacctggtg ctctaggctg tgctgagccc actctcccga gggcgcatcc    480
   aagcgggggc cacttgagaa gtgaataaat ggggcggttt cggaagcgtc agtgtttcca    540
   tgttatgg                                                             548
<210> 629
   <211> 524
   <212> DNA
   <213> human
<400> 629
   gaagttgcaa cattcgtttg ataggaattc cagaaaagga gagttatgag aatagggcag      60
   aggacataat taaagaaata attgatgaaa actttgcaga actaaagaaa ggttcaagtc     120
   ttgagattgt cagtgcttgt cgagtaccta gtaaaattga tgaaaagaga ctgactccta     180
   gacacatctt ggtgaaattt tggaattcta gtgataaaga gaaaataata agggcttcta     240
   gagagagaag agaaattacc taccaaggaa caagaatcag gttgacagca gacttatcac     300
   tggacacact ggatgctaga agtaaatgga gcaatgtctt caaagttctg ctggaaaaag     360
   gctttaatcc tagaatccta tatccagcca aaatggcatt tgattttagg ggcaaaacaa     420
   aggtatttct tagtattgaa gaatttagag attatgtttt gcatatgccc accttgagag     480
   aattactggg gaataatata ccttagcacg ccagggtgac taca                      524
<210> 630
   <211> 488
   <212> DNA
   <213> human
<400> 630
   gagtggttca tccatactct cattccctcg cctccccttg tggacggggg tcttgccttt      60
   tcaattcctg tgttttggtg tcttccctta tctgctaccc tgaatcacct gtcctggtct     120
   tgctgtgtga tgggaacatg cttgtaaact gcgtaacaaa tctactttgt gtatgtgtct     180
   gtttatgggg gtggtttatt atttttgctg gtccctagac cactttgtat gaccgtttgc     240
   agtctgagca ggccaggggc tgacagctaa tgtcaggacc ctcagcggtg gagcctgctg     300
   gggggaccca gctgctcttg gacaagtggc tgagctccta tctggcctcc tctttttttt     360
   ttttttcaag taatttgtgt gtatttctaa ctgattgtat tgaaaaaatt cctagtattt      420
   cagtaaaaat gcctgttgtg agatgaacct cctgtaactt ctatctgttc ttttttgagg     480
   ctcaggga                                                              488
<210> 631
   <211> 460
   <212> DNA
   <213> human
<400> 631
   tggaattaga ccatttggcc tttgaacttt cataggaaaa atgacccaac atttcttagc      60
   atgagctacc tcatctctag aagctgggat ggacttacta ttcttgttta tattttagat     120
   actgaaaggt gctatgcttc tgttattatt ccaagactgg agataggcag ggctaaaaag     180
   gtattattat ttttccttta atgatggtgc taaaattctt cctataaaat tccttaaaaa     240
   taaagatggt ttaatcacta ccattgtgaa aacataactg ttagacttcc cgtttctgaa     300
   agaaagagca tcgttccaat gcttgttcac tgttcctctg tcatactgta tctggaatgc     360
   tttgtaatac ttgcatgctt cttagaccag aacatgtagg tccccttgtg tctcaatact     420
   ttttttttct taattgcatt tgttggctct attttaattt                            460
<210> 632
   <211> 446
   <212> DNA
   <213> human
<400> 632
   tagattccgg tatatcgttc ttcaagacac ttgctctaag cggaatggaa atgtgggcaa      60
   gactgtcttt gaatatagaa cacagaatgt ggcacgcttg cccatcatag atcttgctcc     120
   tgtggatgtt ggcggcacag accaggaatt cggcgttgaa attgggccag tttgttttgt     180
   gtaaagtaag ccaagacaca tcgacaatga gcaccaccat caatgaccac cgccattcac     240
   aagaactttg actgtttgaa gttgatcctg agactcttga agtaatggct gatcctgcat     300
   cagcattgta tatatggtct taagtgcctg gcctccttat ccttcagaat atttatttta     360
   cttacaatcc tcaagtttta attgatttta aatatttttc aatacaacag tttaggttta     420
   agatgaccaa tgacaatgac cacctt                                          446
<210> 633
   <211> 552
   <212> DNA
   <213> human
<220>
   <221> misc_feature
   <222> (63)..(63)
   <223> n is a, c, g, or t
<220>
   <221> misc_feature
   <222> (72)..(72)
   <223> n is a, c, g, or t
<220>
   <221> misc_feature
   <222> (74)..(74)
   <223> n is a, c, g, or t
<220>
   <221> misc_feature
   <222> (85)..(85)
   <223> n is a, c, g, or t
<220>
   <221> misc_feature
   <222> (138)..(138)
   <223> n is a, c, g, or t
<400> 633
   tttcagcacc gatggccatg taaataagat gatttaatgt tgattttaat cctgtatata     60
   aantaaaaag tncncaatga gtttngggca tatttaatga tgattatgga gccttagagg    120
   tctttaatca ttggttcngg ctgcttttat gtagtttagg ctggaaatgg tttcacttgc    180
   tctttgactg tcagcaagac tgaagatggc ttttcctgga cagctagaaa acacaaaatc    240
   ttgtaggtca ttgcacctat ctcagccata ggtgcagttt gcttctacat gatgctaaag    300
   gctgcgaatg ggatcctgat ggaactaagg actccaatgt cgaactcttc tttgctgcat    360
   tcctttttct tcacttacaa gaaaggcctg aatggaggac ttttctgtaa ccaggaacat    420
   tttttagggg tcaaagtgct aataattaac tcaaccaggt ctacttttta atggctttca    480
   taacactaac tcataaggtt accgatcaat gcatttcata cggatataga cctagggctc    540
   tggagggtgg gg                                                        552
<210> 634
   <211> 440
   <212> DNA
   <213> human
<220>
   <221> misc_feature
   <222> (165)..(165)
   <223> n is a, c, g, or t
<400> 634
   ctggcctacg tgttcaattt tctatgaaca aaggctttag tccttgaccc agggctaaag     60
   tggtctgtcc aagctgttgt tggtagaggg agtatgataa aatgtttaaa tctcatttgg    120
   ttaccttgag tcctggaaca cgcagtaact gtcatgctat agtcntcatc tgtatttggc    180
   tgggaataca aatgaagatt gtggtgtatt caagcagtag ggtttttgct tttgtttttg    240
   ttttagtgcc aacaaaactt ttttttgtct gactacatta aagataagac tgactatatt    300
   tatacaacag aaactttgta atagattttt tcagctttgt gaaatcgaat tttttttcat    360
   cagggctggt tggatttcct ttttaccctg taatccaagc gttaatagtt tgttagaaga    420
   tgggttattg catgtcactt                                                440
<210> 635
   <211> 360
   <212> DNA
   <213> human
<400> 635
   gaagatcaag gatgttctca acagtctaga gtacagtccc tctcctataa gcaagaagct     60
   ctcgtgtgct agtgtcaaaa gccaaggcag accgtcctcc tgccctgctg ggatggctgt    120
   cactggctgt gcttgtggct atggctgtgg ttcgtgggat gttcagctgg aaaccacctg    180
   ccactgccag tgcagtgtgg tggactggac cactgcccgc tgctgccacc tgacctgaca    240
   gggaggaggc tgagaactca gttttgtgac catgacagta atgaaaccag ggtcccaacc    300
   aagaaatcta actcaaacgt cccacttcat ttgttccatt cctgattctt gggtaataaa    360
<210> 636
   <211> 490
   <212> DNA
   <213> human
<400> 636
   ggagctcaga gatctaagct gctttccatc ttttctccca gccccaggac actgactctg     60
   tacaggatgg ggccgtcctc ttgcctcctt ctcatcctaa tcccccttct ccagctgatc    120
   aacccgggga gtactcagtg ttccttagac tccgttatgg ataagaagat caaggatgtt    180
   ctcaacagtc tagagtacag tccctctcct ataagcaaga agctctcgtg tgctagtgtc    240
   aaaagccaag gcagaccgtc ctcctgccct gctgggatgg ctgtcactgg ctgtgcttgt    300
   ggctatggct gtggttcgtg ggatgttcag ctggaaacca cctgccactg ccagtgcagt    360
   gtggtggact ggaccactgc ccgctgctgc cacctgacct gacagggagg aggctgagaa    420
   ctcagttttg tgaccatgac agtaatgaaa ccagggtccc aaccaagaaa tctaactcaa    480
   acgtcccact                                                           490
<210> 637
   <211> 577
   <212> DNA
   <213> human
<400> 637
   gcctgagaaa gcaagcacgc actctcagtc aacatgacag attctggagg ataaccagca     60
   ggagcagaga taacttcagg aagtccattt ttgcccctgc ttttgctttg gattatacct    120
   caccagctgc acaaaatgca ttttttcgta tcaaaaagtc accactaacc ctcccccaga    180
   agctcacaaa ggaaaacgga gagagcgagc gagagagatt tccttggaaa tttctcccaa    240
   gggcgaaagt cattggaatt tttaaatcat aggggaaaag cagtcctgtt ctaaatcctc    300
   ttattctttt ggtttgtcac aaagaaggaa ctaagaagca ggacagaggc aacgtggaga    360
   ggctgaaaac agtgcagaga cgtttgacaa tgagtcagta gcacaaaaga gatgacattt    420
   acctagcact ataaaccctg gttgcctctg aagaaactgc cttcattgta tatatgtgac    480
   tatttacatg taatcaacat gggaactttt aggggaacct aataagaaat cccaattttc     540
   aggagtggtg gtgtcaataa acgctctgtg gccagtg                             577
<210> 638
   <211> 351
   <212> DNA
   <213> human
<400> 638
   ggaacccaga gctgctgtgt atttcgagcg ggcagtttat cttttgctat acttattttc     60
   aattcaatta caccacgatt caaataattc ccctcctaaa accaaaaagg agggaaacgt    120
   caactccatt gcaattactt atcttcctct tctatctctg ttatacgccg gggcatagaa    180
   tgctcgtata catctcttta acaaccacaa accttaagcc atgtagatga agttagtgca    240
   tcaacgggat acagttccat attgccttaa acctccttgt tttagacaca ctaacattta    300
   taccaaattg cagattattc tgcagagagg gaattgcatg tttgtgttgt a             351
<210> 639
   <211> 506
   <212> DNA
   <213> human
<400> 639
   aattaatatt catcgcactt cttctgtgga aggactttgt gaaggaattg gtgctggatt     60
   agtggatgtt gctatctggg ttggcacttg ttcagattac ccaaaaggag atgcttctac    120
   tggatggaat tcagtttctc gcatcattat tgaagaacta ccaaaataaa tgctttaatt    180
   ttcatttgct acctcttttt ttattatgcc ttggaatggt tcacttaaat gacattttaa    240
   ataagtttat gtatacatct gaatgaaaag caaagctaaa tatgtttaca gaccaaagtg    300
   tgatttcaca ctgtttttaa atctagcatt attcattttg cttcaatcaa aagtggtttc     360
   aatatttttt ttagttggtt agaatacttt cttcatagtc acattctctc aacctataat    420
   ttggaatatt gttgtggtct tttgtttttt ctcttagtat agcattttta aaaaaatata    480
   aaagctacca atctttgtac aatttg                                         506
<210> 640
   <211> 360
   <212> DNA
   <213> human
<220>
   <221> misc_feature
   <222> (128)..(128)
   <223> n is a, c, g, or t
<220>
   <221> misc_feature
   <222> (190)..(190)
   <223> n is a, c, g, or t
<220>
   <221> misc_feature
   <222> (206)..(206)
   <223> n is a, c, g, or t
<400> 640
   aaatgactgg atggtcgctg ctttttaagt ttcaaattga cattccagac aagcggtgcc     60
   tgagcccgtg cctgtcttca gatcttcaca gcacagttcc tgggaaggtg gagccaccag    120
   cctctccntg aataactggg agatgaaaca ggaagctcta tgacacactt gatcgaatat    180
   gacagacacn gaaaatcacg actcancccc ctccagcacc tctacctgtt gcccgccgat   240
   cacagccgga atgcagctga aagattccct ggggcctggt tccaaccgcc cactgtggac    300
   tctgaggcct ctgcatttgc gggtggtctg cctgtgatat tttggtcatg ggctggtctg    360
<210> 641
   <211> 427
   <212> DNA
   <213> human
<400> 641
   gaagaggagc aacatctatg ccaaatactg tgcattctac aatggtgcta atctcagacc     60
   taaatgatac tccatttaat ttaaaaaaga gttttaaata attatctatg tgcctgtatt    120
   tcccttttga gtgctgcaca acatgttaac atattagtgt aaaagcagat gaaacaacca    180
   cgtgttctaa agtctaggga ttgtgctata atccctattt agttcaaaat taaccagaat    240
   tcttccatgt gaaatggacc aaactcatat tattgttatg taaatacaga gttttaatgc    300
   agtatgacat cccacagggg aaaagaatgt ctgtagtggg tgactgttat caaatatttt    360
   atagaataca atgaacggtg aacagactgg taacttgttt gagttcccat gacagatttg    420
   agacttg                                                              427
<210> 642
   <211> 574
   <212> DNA
   <213> human
<220>
   <221> misc_feature
   <222> (83)..(83)
   <223> n is a, c, g, or t
<220>
   <221> misc_feature
   <222> (94)..(94)
   <223> n is a, c, g, or t
<220>
   <221> misc_feature
   <222> (103)..(103)
   <223> n is a, c, g, or t
<220>
   <221> misc_feature
   <222> (134)..(138)
   <223> n is a, c, g, or t
<220>
   <221> misc_feature
   <222> (140)..(141)
   <223> n is a, c, g, or t
<220>
   <221> misc_feature
   <222> (143)..(143)
   <223> n is a, c, g, or t
<220>
   <221> misc_feature
   <222> (145)..(146)
   <223> n is a, c, g, or t
<220>
   <221> misc_feature
   <222> (169)..(169)
   <223> n is a, c, g, or t
<220>
   <221> misc_feature
   <222> (171)..(172)
   <223> n is a, c, g, or t
<220>
   <221> misc_feature
   <222> (174)..(177)
   <223> n is a, c, g, or t
<220>
   <221> misc_feature
   <222> (179)..(179)
   <223> n is a, c, g, or t
<220>
   <221> misc_feature
   <222> (181)..(181)
   <223> n is a, c, g, or t
<220>
   <221> misc_feature
   <222> (183)..(184)
   <223> n is a, c, g, or t
<220>
   <221> misc_feature
   <222> (186)..(193)
   <223> n is a, c, g, or t
<220>
   <221> misc_feature
   <222> (196)..(196)
   <223> n is a, c, g, or t
<220>
   <221> misc_feature
   <222> (198)..(203)
   <223> n is a, c, g, or t
<220>
   <221> misc_feature
   <222> (206)..(207)
   <223> n is a, c, g, or t
<400> 642
   tgaaagtggg catctctgca ggcacctgta ctgccatcct gctcaccgtc ttgacctgct     60
   acttttggaa aaagaatcaa aanctagagt acangtactc cangctggtg atgaatgcta    120
   ctctcaagga ctgnnnnntn ncngnngctg acagctgcgc catcatggna nncnnnnang    180
   nanngnnnnn nnncancnnn nnncanntgc gccatcatgg aaggcgagga tgtagaggac    240
   gacctcatct ttaccagcaa gaagtcactc tttgggaaga tcaaatcatt tacctccaag    300
   aggactcctg atggatttga ctcagtgccg ctgaagacat cctcaggagg cccagacatg    360
   gacctgtgag aggcactgcc tgcctcacct gcctcctcac cttgcatagc acctttgcaa    420
   gcctgcggcg atttgggtgc cagcatcctg caacacccac tgctggaaat ctcttcattg    480
   tggccttatc agatgtttga atttcagatc tttttttata gagtacccaa accctccttt    540
   ctgcttgcct caaacctgcc aaatataccc acac                                574
<210> 643
   <211> 485
   <212> DNA
   <213> human
<400> 643
   aaacgacgca aatctctgag ctggggacca cttggagaac cggcttagta acagtcctga     60
   tcttcgcaag ccagcttctt ctgcatctga ggggctcctg gcgcccagag gaggcagaca    120
   gatgtcttct agctgagttt ctaaccgcat gatgagactc agaccttccg ctgcactaga    180
   aaatctgcaa cagtgtccct gagtcacttc tccttagtgg gcagactcgt gttagatttg    240
   tggaacccag ctctctgatt tactcctttt ggaaaaccca tggaatttca tgtataaggc    300
   tttcatttgt attttaaggt ttttctgttt gttttgagta tatacatggt gctcaatagc    360
   aacatcttag cagatgaagc agtttatgat tccactccct cctgtatgac aggtagccac    420
   tatactgaat caaggtgctg aactcaaatc acaaaattct ggcttaccga tacaacaacc    480
   aatac                                                                485
<210> 644
   <211> 487
   <212> DNA
   <213> human
<220>
   <221> misc_feature
   <222> (296)..(296)
   <223> n is a, c, g, or t
<400> 644
   tataaggtaa ctctttagtc ctccatttag cacattttaa atcctccaaa gaataagtat     60
   catgtgatta ttttagcttt acaaaaaaaa agttgaatgg cgttttattt tcatggccta    120
   taagcaggta ccttagtagg gcagatatag gaaaaacaaa ttagagcaaa acaaatcctc    180
   tacaaatcca aggcaggaaa agtggtggca gagtgactca ttctcctgtc cctcccatca    240
   ggtcaaatca ggaggctgca gtgaatgcct gttctttgaa tgtgtagcag ttgttncctg    300
   taactcttta aaacttggct ataggctgtt tagcacagta cagattaaag atacagttac    360
   gtaaacagca aagtaatttt atagtgcttc atccatttat catgctttgg tttgctaatt    420
   ttttcacata cctttttcta tcacagtctg ttgcttttgt acacatttct catattgggg    480
   ttcgaca                                                              487
<210> 645
   <211> 520
   <212> DNA
   <213> human
<400> 645
   ggtgccagga aggtcacaga tggacactgg ccattctggt catctcagtc tggaactcag     60
   tcccacttct tggcctggac aatgaacagg attcagtttt gctgttaact ttgcttctct    120
   actttttttt gtttgtttgt aatagcacat cccagagaca tcagaaacca gcaactgatt    180
   cagtgtgatt tccagacttt ttaggcatga aattcggaca cttcagtatt tccaggaata    240
   gcatatgcac gctgttcttg cttcatggaa tgctacatgc tttctgtttt tctcattttg    300
   gatttctcca aaactaactg aatttaagct tcaggtccct ttgtatgcag tagaaaggaa    360
   ttattaaaaa caccaccaaa gaaaataaat atatcctact tgaaatttac tctatggact    420
   tacccactgc tagaataaat gtatcaaatc ttatttgtaa attctcaatt ttgatatata    480
   tatgtatata tgcatataca tatccacact tgtctgcaag                          520
<210> 646
   <211> 516
   <212> DNA
   <213> human
<400> 646
   ttaccctcta tttaaatgct ttgaaaaaca gtgcattgac aatgggttga tatttttctt     60
   taaaagaaaa atataattat gaaagccaag ataatctgaa gcctgtttta ttttaaaact    120
   ttttatgttc tgtggttgat gttgtttgtt tgtttgtttc tattttgttg gttttttact    180
   ttgttttttg ttttgttttg ttttgttttg catactacat gcagttcttt aaccaatgtc    240
   tgtttggcta atgtaattaa agttgttaat ttatatgagt gcatttcaac tatgtcaatg    300
   gtttcttaat atttattgtg tagaagtact ggtaattttt ttatttacaa tatgtttaaa    360
   gagataacag tttgatatgt tttcatgtgt ttatagcaga agttatttat ttctatggca    420
   ttccagcgga tattttggtg tttgcgaggc atgcagtcaa tattttgtac agttagtgga    480
   cagtattcag caacgcctga tagcttcttt ggcctt                               516
<210> 647
   <211> 390
   <212> DNA
   <213> human
<400> 647
   ccacacacca ggcactaatc acctggtgag gatttggcat atccaccaaa aaatgcatcc     60
   gatttaacca acatctccac cagcgctacg gactcctccc aattctgaca tctcttgcag    120
   acaatactat gctctctaca cactgtttag aaatggaaag gtgatctgca ctgtatcttg    180
   ggtttgttgg ctatgcttcc tttgatgaca tatattatac agtatatata tacatatatt    240
   ttttttgtta gagttctagc cattttattt ctccgcaggg tcctttctca gacattactg    300
   catgctgtat atggcgttag ctgtgtgttg atcttctaaa agatgataga gtttactggt    360
   aattgtgtaa tcagctcctg cctttttatt                                     390
<210> 648
   <211> 505
   <212> DNA
   <213> human
<220>
   <221> misc_feature
   <222> (39)..(39)
   <223> n is a, c, g, or t
<400> 648
   caggaaactc ttctctgact tggggagttc ctacgcgana caactgggct tccgggacag     60
   ctgggtcttc ataggagcca aagacctcag gggtaaaagc ccctttgagc agttcttaaa    120
   gaacagccca gacacaaaca aatacgaggg atggccagag ctgctggaga tggagggctg    180
   catgcccccg aagccatttt agggtggctg tggctcttcc tcagccaggg gcctgaagaa    240
   gctcctgcct gacttaggag tcagagcccg gcaggggctg aggaggagga gcagggggtg    300
   ctgcgtggaa ggtgctgcag gtccttgcac gctgtgtcgc gcctctcctc ctcggaaaca    360
   gaaccctccc acagcacatc ctacccggaa gaccagcctc agagggtcct tctggaacca    420
   gctgtctgtg gagagaatgg ggtgctttcg tcagggactg ctgacggctg gtcctgagga    480
   aggacaaact gcccagactt gagcc                                          505
<210> 649
   <211> 331
   <212> DNA
   <213> human
<220>
   <221> misc_feature
   <222> (73)..(74)
   <223> n is a, c, g, or t
<220>
   <221> misc_feature
   <222> (76).. (76)
   <223> n is a, c, g, or t
<400> 649
   cacttgatag actgtaagca cctgcttaac tttgtgtccc aaatatttag tgtgtatata      60
   tatatatata tanncncaca cacacacata tatattcaac aaataaagca aaatataaca     120
   tgcatttcac attttgtctt tccctgttac gattttaata gcagaactgt atgacaagtt    180
   taggtgatcc tagcatatgt taaattcaaa ttaatgtaaa acagattaac aacaacaaag    240
   aaactgtcta tttgagtgaa gtcatgcttt ctattataat aacttggctt cggttatcca    300
   tcaaatgcac acttatactg ttatctgatt g                                   331
<210> 650
   <211> 582
   <212> DNA
   <213> human
<220>
   <221> misc_feature
   <222> (179)..(180)
   <223> n is a, c, g, or t
<220>
   <221> misc_feature
   <222> (218)..(218)
   <223> n is a, c, g, or t
<220>
   <221> misc_feature
   <222> (226)..(227)
   <223> n is a, c, g, or t
<400> 650
   gccaaaaggc ctggcttctg ataggaaact ggtaagaaac tcttcatgaa aacacatcac     60
   taatattcgc tattactctc ctggtctgaa gtcagctttt ctgaaccatt aaggtatttc    120
   atcacaagtt atattttata atatcagttt aagaggcttt tattcatgtg aacaccagnn    180
   ccctttcagg ggcatggtct ttttgaaaaa aaaaaaanaa aaaaannaac agttttagcc    240
   acatatcaga tatttctata tctaattatc ctttatggct aacattctgc ctccattgtt    300
   aaggtataat tgttcctgaa tttaaaggtg gtttggcctc taatttaatt ctgattcaga    360
   ctctcctgtc aggactcaag aaaatttaat taattaccaa ggattaagtc ttctggttaa    420
   ggtttctggg aaaaaaaaat agcaaagatg ttgatttctt ggaatccttt tacaggttca    480
   taacagaaaa atcttcattc cctgtaggca tttaattaaa cctagttgag aagtgtgtgg    540
   gattcctcaa ttatgaacaa aacacgtata ttggctttct tt                       582
<210> 651
   <211> 433
   <212> DNA
   <213> human
<400> 651
   gacatgattg tctataatct cgctagcctt gtactgtgtg tgcatagcaa ttacagggaa     60
   gtaatctagc tcctgactat tatgttgaac tatgtcgctg ctttttacaa acttgtcttg    120
   atccaaagca gtcacaatga taaccctgca tatctgggaa tcataagtca actatgtatc    180
   tctgtgtgtg tatatatatg tatgtatgta tctattttca aactgtgatt taatatttaa    240
   atattcctac tgccattttt gtgactgaaa aactacacat gaggaaacgt cttagaattt    300
   tccaatagag gaaaaataac acttgggcaa tctgtcatgt ttcacaacag ttctcatttt    360
   tctcatgatt tgtgtagcgt ggaatgtgtt tgctcaatgt gaagggtttt cattgctcaa    420
   tttctctgtg taa                                                       433
<210> 652
   <211> 361
   <212> DNA
   <213> human
<220>
   <221> misc_feature
   <222> (71)..(71)
   <223> n is a, c, g, or t
<220>
   <221> misc_feature
   <222> (295)..(304)
   <223> n is a, c, g, or t
<400> 652
   ggttccggct aacacatttt ctaagtcgcc agtgctgctt acagtttgaa tacatgaaaa     60
   tcctgtttct nagatgtttg cgcacgtgct tattaggaaa tgagtctgta tggaaatctc    120
   accacagata atggttaacg aaccgggtcg acatcacaaa ggagggtgga gactcttttt    180
   actaacttga atgagacaaa agcagtggtg tcagtttata atcctgatgc atttcagtaa    240
   taatgtagaa aaacattatt ttaaaaaagt tccaacacac agccatgagg agccnnnnnn    300
   nnnntcagtt ttgaaagagg tgcataataa aactactaac cagaggagtc tatgccattt    360
   t                                                                    361
<210> 653
   <211> 415
   <212> DNA
   <213> human
<220>
   <221> misc_feature
   <222> (42)..(42)
   <223> n is a, c, g, or t
<220>
   <221> misc_feature
   <222> (95)..(95)
   <223> n is a, c, g, or t
<220>
   <221> misc_feature
   <222> (353)..(353)
   <223> n is a, c, g, or t
<400> 653
   gagtttcaac tttaaatgtt cactatgtca tttagtgtcc anctttacgg ataggttgac      60
   tatctaaata ggcattttta gtcattaaaa aaaantctag tcaccaggag gatccctata     120
   actcaaaata acttgtttgt aaaagaaaat ttgtttactt acccattagt aagttcctgc     180
   atattcatta taagatggca aatcaaactt ttctaggatg aagacagctt atttttaagt     240
   tgtatagtct tagttggttt agggtctcaa ttttaattaa taaaatactt ggtttttatt     300
   tgcttgtcct tttgaattcc tgttttaata attttaaaat gagcacaaag aangttgaag     360
   ttcagattaa tctcttctga atgatgtttt tttcctctgt gatgagttgt ttctg          415
<210> 654
   <211> 364
   <212> DNA
   <213> human
<220>
   <221> misc_feature
   <222> (282)..(282)
   <223> n is a, c, g, or t
<220>
   <221> misc_feature
   <222> (294)..(294)
   <223> n is a, c, g, or t
<220>
   <221> misc_feature
   <222> (336)..(337)
   <223> n is a, c, g, or t
<400> 654
   tgctgtagtc actggtgttc ctcacccacc agctgtaact cagtttgtgt gaggtacagc      60
   cacagaagat gtcatgtact gtatattacc tggtgatagt tgcttttcac cccccgagtt     120
   cagtttctag gagccaatga aacttcccct cacctcctca tctttccaag ttgttctttg     180
   aattgaggag tttgaaggca taaacagtta cttggggatt tgcgaaaatc ctacttagtt     240
   actgcgttta cagttctttg gcccagtctc tgacccttcc cnagtatttg tgcnatgatt     300
   gtgtttactg ctggattttt gaaggttttt tttttnnaag aaagtgccat ttcattattt     360
   gatt                                                                  364
<210> 655
   <211> 436
   <212> DNA
   <213> human
<400> 655
   gggatcactg ggagaagcca tggcattatc ttcaggcaat ttagtctgtc ccaaataaaa      60
   taaatccttg catgtaaatc attcaagggt tatagtaata tttcatatac tgaaaagtgt     120
   ctcataggag tcctcttgca catctaaaaa ggctgaacat ttaagtatcc cgaattttct     180
   tgaattgctt tccctataga ttaattacaa ttggatttca tcatttaaaa accatacttg     240
   tatatgtagt tataatatgt aaggaataca ttgtttataa ccagtatgta cttcaaaaat     300
   gtgtattgtc aaacatacct aactttcttg caataaatgc aaaagaaact ggaacttgac     360
   aattataaat agtaatagtg aagaaaaaat agaaaggttg caattatata ggccatgggt     420
   ggctcaaaac tttgaa                                                     436
<210> 656
   <211> 427
   <212> DNA
   <213> human
<220>
   <221> misc_feature
   <222> (45)..(51)
   <223> n is a, c, g, or t
<220>
   <221> misc_feature
   <222> (402)..(402)
   <223> n is a, c, g, or t
<400> 656
   gattcctgga ttgatgtctt atctacaggc ttctttttaa aaatnnnnnn ngattggctg     60
   tgagtttacc aggattatag ttgaggctaa aggacagctc ctcaggaaag cccctgttct    120
   acatctacgg tcacatgctg gaccttgagt tgtcactcag agaaaagagt gccatctacc    180
   gaaactccac agtttccatt gtgaatggct tctttggtgc agagttccaa aaattatgta    240
   gcccagctct ttaattttgt aacatctaat gatatcaccg ccttgaagtg attaaagtag    300
   attgcttaaa gaattaaagc tttaaagatg aaagatgtta ttgcttttgc tggacatgag    360
   gaacagttgt aaagtttcca ggtctacaat aactttctgg anccctctca gtgaactgtt    420
   tcttgta                                                             427
<210> 657
   <211> 559
   <212> DNA
   <213> human
<400> 657
   gaagtccatc ctttggtcca aagcatctgg aagaggaaga agagaggaat gagaaagaag     60
   gaagtgatgc aaaacatctc caaagaagtc ttttggaaca ggaaaatcat tcaccactca    120
   cagggtcaaa tatgaaatac aaaaccacga accaatcaac agaattttta tccttccaag    180
   atgccagctc attgtacaga aacattttag aaaaagaaag ggaacttcag caactgggaa    240
   tcacagaata cctaaggaaa aacattgctc agctccagcc tgatatggag gcacattatc    300
   ctggagccca cgaagagctg aagttaatgg aaacattaat gtactcacgt ccaaggaagg    360
   tattagtgga acagacaaaa aatgagtatt ttgaacttaa agctaattta catgctgaac    420
   ctgactattt agaagtcctg gagcagcaaa catagatgga gagtttgagg gctttcgcag    480
   aaatgctgtg attctgtttt aagtccatac cttgtaaata agtgccttac gtgagtgtgt    540
   catcaatcag aacctaagc                                                559
<210> 658
   <211> 368
   <212> DNA
   <213> human
<400> 658
   gctgctcaag cgcctgcaga acaacgacac gtgcagcatg ccaggcctca cgtgcttcac     60
   ccacgacaac cagcactggc agacggcgcc tttctggaca ctggggcctt tctgtgcctg    120
   caccagcgcc aacaataaca cgtactggtg catgaggacc atcaatgaga ctcacaattt    180
   cctcttctgt gaatttgcaa ctggcttcct agagtacttt gatctcaaca cagaccccta    240
   ccagctgatg aatgcagtga acacactgga cagggatgtc ctcaaccagc tacacgtaca    300
   gctcatggag ctgaggagct gcaagggtta caagcagtgt aacccccgga ctcgaaacat    360
   ggacctgg                                                            368
<210> 659
   <211> 436
   <212> DNA
   <213> human
<220>
   <221> misc_feature
   <222> (300)..(300)
   <223> n is a, c, g, or t
<400> 659
   acaatcggct aaccttgaca tttcttttta ccttcatatg ccactatctc ggtagttcaa     60
   aaaaatttag ttcttgataa attgccttga agtttacctt gtgctggaga gccttatgat    120
   aactccaaag actttcttac ggtataatac atgttgttta ggattgtgtt tcttagtcac    180
   tgaagataat aaatattaaa atggatgttt tcatcagaaa attttcatgt tttcctttaa    240
   ggtaacataa ttgtaagaat tgtttaataa aatactcagg aaattctaaa ggtttctccn    300
   aatacctaaa catttctgaa catcagtatt gcagttgtgg aagagcagaa ggaggataca    360
   tttgtttgtg ttgctcccca aaattccacc ttgcatttgc atcacaaact tccctcaatt    420
   gaggcagttt tctttg                                                    436
<210> 660
   <211> 431
   <212> DNA
   <213> human
<220>
   <221> misc_feature
   <222> (311)..(311)
   <223> n is a, c, g, or t
<220>
   <221> misc_feature
   <222> (391)..(391)
   <223> n is a, c, g, or t
<400> 660
   cagtgctgct gtgaactaaa gtatgtcatt tatgctcaaa gtttaattct tcttcttggg     60
   atattttaaa aatgctactg agattctgct gtaaatatga ctagagaata tattgggttt    120
   gctttatttc ataggcttaa ttctttgtaa atctgaatga ccataataga aatacatttc    180
   ttgtggcaag taattcacag ttgtaaagta aataggaaaa attattttat ttttattgat    240
   gtacattgat agatgccata aatcagtagc aaaaggcact tctaaaggta agtggtttaa    300
   gttgcctcaa nagagggaca atgtagcttt attttacaag aaggcatagt tagatttcta    360
   tgaaatattt attctgtaca gttttatata nttttggttc acaaaagtaa ttattcttgg    420
   gtgcctttca a                                                         431
<210> 661
   <211> 322
   <212> DNA
   <213> human
<220>
   <221> misc_feature
   <222> (37)..(38)
   <223> n is a, c, g, or t
<400> 661
   aaagtatatt gtgctagctt gtctaagaat aaacttnnat actgttgggg gagggctgca     60
   cctgtcaaga taacctgtca atgtagtagg aaaacaggag gggacagtaa cagaaaagca    120
   cgggaaaaga tggcaaggtt agttaaaata gaaaagtgct cagttcctca tacctgtaat    180
   cccagcagtt taggggccca aggaaggtgg gtcacttgag cccaagagtt caaggccatc   240
   ctgggcaatg tggcgaaagt gtctacaaaa aaatacaaaa agaggaagaa atgatatttc    300
   acaagtttgt atcatttgtc at                                             322
<210> 662
   <211> 282
   <212> DNA
   <213> human
<220>
   <221> misc_feature
   <222> (43)..(43)
   <223> n is a, c, g, or t
<220>
   <221> misc_feature
   <222> (50)..(50)
   <223> n is a, c, g, or t
<220>
   <221> misc_feature
   <222> (99)..(99)
   <223> n is a, c, g, or t
<220>
   <221> misc_feature
   <222> (115)..(115)
   <223> n is a, c, g, or t
<400> 662
   cagtccaaaa tccatagaca cagacagtag aaaggtggtt atncaagggn ctggtaggag     60
   agggagttag tatttagtgg tatagagttt tagtgtttnc cgggtaaaaa tgttnctaga    120
   gatctgttgc aaccatgtga atatacttaa cactaccgta ctgtacactg aaaaaatggt    180
   taagaggata aatttaaatg gttaagagga aaaatggtta agaggataat gttgttttgt    240
   taccacagta gtaactttta aaaaccctca tgtatgttct tt                       282
<210> 663
   <211> 398
   <212> DNA
   <213> human
<220>
   <221> misc_feature
   <222> (80)..(80)
   <223> n is a, c, g, or t
<220>
   <221> misc_feature
   <222> (82)..(83)
   <223> n is a, c, g, or t
<220>
   <221> misc_feature
   <222> (85)..(85)
   <223> n is a, c, g, or t
<220>
   <221> misc_feature
   <222> (87)..(87)
   <223> n is a, c, g, or t
<220>
   <221> misc_feature
   <222> (97)..(97)
   <223> n is a, c, g, or t
<400> 663
   caacccattt tgtgccacat gcaagttttg aataaggatg gtatagaaaa caacgctgca     60
   tatacaggta ccatttaggn annancngat gcctttntgg gggcagaatc acatggcaaa    120
   agctttgaaa atcataaaga tataagttgg tgtggctaag atggaaacag ggctgattct    180
   tgattcccaa ttctcaactc tccttttcct atttgaattt ctttggtgct gtagaaaaca    240
   aaaaaagaaa aatatatatt cataaaaaat atggtgctca ttctcatcca tccaggatgt    300
   actaaaacag tgtgtttaat aaattgtaat tattttgtgt acagttctat actgttatct    360
   gtgtccattt ccaaaacttg cacgtgtccc tgaattcc                           398
<210> 664
   <211> 22
   <212> DNA
   <213> synthetic
<400> 664
   tcgtttttcg cgtttagttg tc 22
<210> 665
   <211> 17
   <212> DNA
   <213> synthetic
<400> 665
   gcgcaccgaa aaaaccg 17
<210> 666
   <211> 24
   <212> DNA
   <213> synthetic
<400> 666
   cggttacggt tagtattcgg tttc 24
<210> 667
   <211> 23
   <212> DNA
   <213> synthetic
<400> 667
   aaaatacgac gataaaaaac gcg 23
<210> 668
   <211> 23
   <212> DNA
   <213> synthetic
<400> 668
   cgagggtttt ttagcgattt gtc 23
<210> 669
   <211> 17
   <212> DNA
   <213> synthetic
<400> 669
   aaacgaaccg ctccccg 17
<210> 670
   <211> 21
   <212> DNA
   <213> synthetic
<400> 670
   ttcggcgttt tcgtttttta c 21
<210> 671
   <211> 21
   <212> DNA
   <213> synthetic
<400> 671
   ccccttaaca acataatcgc g 21
<210> 672
   <211> 23
   <212> DNA
   <213> synthetic
<400> 672
   gatggtgcgt tcggtattta tgt 23
<210> 673
   <211> 19
   <212> DNA
   <213> synthetic
<400> 673
   gcttacaccc gcccgacta 19
<210> 674
   <211> 28
   <212> DNA
   <213> synthetic
<400> 674
   gttttgtgat tggttaggtt attaaagt 28
<210> 675
   <211> 25
   <212> DNA
   <213> synthetic
<400> 675
   ccrtcacctt ctacaccaaa aaata 25
<210> 676
   <211> 30
   <212> DNA
   <213> synthetic
<400> 676
   tgtggattta tttttataag gatattgaat 30
<210> 677
   <211> 32
   <212> DNA
   <213> synthetic
<400> 677
   ataactcatt aaaaactcac atctaaacac ta 32
<210> 678
   <211> 28
   <212> DNA
   <213> synthetic
<400> 678
   ttaagaaaga gagtaaggag gaagagtt 28
<210> 679
   <211> 24
   <212> DNA
   <213> synthetic
<400> 679
   aaaatcccaa ttaaaaaaca acca 24
<210> 680
   <211> 33
   <212> DNA
   <213> synthetic
<400> 680
   caaaataata aatccctcta ctactattat caa 33
<210> 681
   <211> 30
   <212> DNA
   <213> synthetic
<400> 681
   ggaggaaagg aattagttta gaagttagtt 30